(19) Europäisches Patentamt
European Patent Office
Office européen des brevets

(11) EP 3 677 680 A1

(12) **EUROPEAN PATENT APPLICATION**
published in accordance with Art. 153(4) EPC

(43) Date of publication:
**08.07.2020 Bulletin 2020/28**

(21) Application number: **18850444.3**

(22) Date of filing: **30.08.2018**

(51) Int Cl.:
***C12N 15/113** (2010.01)* ***A61K 31/7088** (2006.01)*
***A61P 35/00** (2006.01)* ***A61P 43/00** (2006.01)*

(86) International application number:
**PCT/JP2018/032122**

(87) International publication number:
**WO 2019/044974 (07.03.2019 Gazette 2019/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.08.2017 JP 2017166641**

(71) Applicant: **Veritas In Silico Inc.**
**Tokyo 141-0031 (JP)**

(72) Inventors:
- **KAMIMURA, Takashi**
**Tokyo 141-0031 (JP)**
- **NASHIMOTO, Masayuki**
**Niigata-shi**
**Niigata 950-2015 (JP)**
- **NAKAMURA, Shingo**
**Tokyo 141-0031 (JP)**
- **JIN, Ling**
**Tokyo 141-0031 (JP)**

(74) Representative: **Plasseraud IP**
**66, rue de la Chaussée d'Antin**
**75440 Paris Cedex 09 (FR)**

# (54) SMALL GUIDE ANTISENSE NUCLEIC ACID AND USE THEREOF

(57) The present invention relates to providing a nucleic acid medicine for managing a disease or disorder related to unwanted gene expression. More specifically, the inventors of the present invention developed a technique for efficiently regulating gene expression in a cell using a relatively short oligonucleotide, by establishing a method for designing a nucleic acid medicine, comprising performing structural analysis based on sequence information of a target RNA sequence as an object for gene expression regulation, calculating the probability of existence of each structure from the obtained results of the structural analysis and the energy of each structure, and calculating the existence probability in unified fashion of an endogenous stem-loop substructure, and thereby specifying a more favorable endogenous stem-loop substructure. This technique can theoretically be applied to regulation of any gene expression, and is therefore useful for treatment or prevention of various diseases and disorders.

Fig. 1

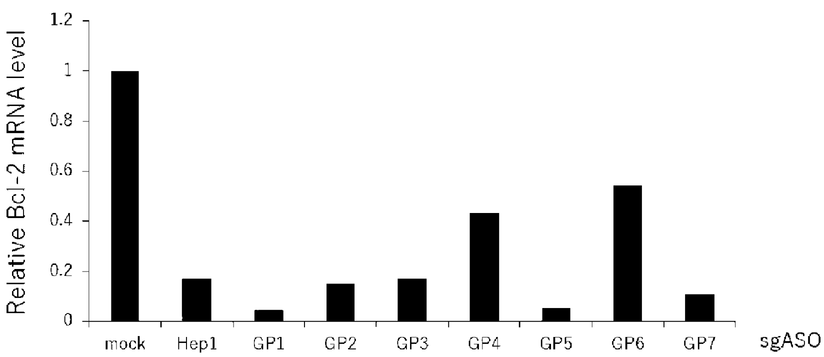




Printed by Jouve, 75001 PARIS (FR)

## Description

### Cross-Reference to Related Applications

The present application is an application claiming priority to JP 2017-166641 (filing date: August 31, 2017), which is incorporated herein by reference in its entirety.

### Technical Field

The present invention relates to regulation of gene expression using oligonucleotides. More specifically, it relates to pharmaceutical compositions comprising relatively short oligonucleotides for regulating gene expression and use thereof in disease treatment. The present invention also relates to a method of designing relatively short oligonucleotides for regulation of gene expression.

### Background Art

In the drug discovery technology in drug development, conventionally most efforts have been made to use proteins as a drug target in drug discovery, and the focus of drug discovery was medicine including small-molecule medicine and antibody, and peptide medicine. Since the causes of diseases have been elucidated at the gene level in recent years in genome analysis and personalized medicine, efforts have been made to develop nucleic acids as targets of drug discovery. Nucleic acid medicine is thought to be able to deal with tough therapeutic targets by small-molecule drugs and antibody drugs and is thus expected as a new modality (drug discovery method).

As basic research, research on antisense nucleic acids and RNAi (RNA interference) (Non-patent Document 1) was conducted, and drug development based on the technology was carried out, and market products are also beginning to be released. In addition, functions of nucleic acids that do not carry protein-coding genetic information are being elucidated, and application thereof to the drug discovery technology as gene expression regulation technology is progressing for miRNA (microRNA) (Non-patent Document 2).

In antisense nucleic acid technology, Nashimoto et al. have developed a gene expression suppression technique named TRUE gene silencing (tRNase $Z^L$-utilizing efficacious gene silencing) (Patent Documents 1-3, Non-patent Documents 3-10). This TRUE gene silencing is based on the unique enzymatic properties of mammalian tRNase $Z^L$ that any RNA can be cleaved at any point by recognizing pre-tRNA-like or micro pre-tRNA- like complexes formed by target RNAs and synthetic small guide antisense nucleic acids. In particular, TRUE gene silencing has been reported for human BCL-2, WT-1, and CCND-1 when the synthetic small guide antisense nucleic acid has seven bases (Non-patent Documents 5-8). The effect of TRUE gene silencing is comparable to RNA interference (RNA interference: RNAi) (Non-patent Document 9) and has also been confirmed to outperform the effect of RNAi in some cases (Non-patent Document 10).

### Citation List

### Patent Documents

Patent Document 1: Japanese Patent No. 3660718
Patent Document 2: Japanese Patent No. 5959522
Patent Document 3: Japanese Patent No. 5995849


### Non-patent Documents

Non-patent Document 1: Chery, J. (2016) RNA therapeutics: RNAi and antisense mechanisms and clinical applications. Postdoc J. 4(7): 35-50

Non-patent Document 2: Rupaimoole, R. & Slack,F.J. (2017) MicroRNA therapeutics: towards a new era for the management of cancer and other diseases. Nature Review Drug Discovery, 16, 203-221

Non-patent Document 3: Tamura, M., Nashimoto, C., Miyake, N., Daikuhara,Y., Ochi,K. and Nashimoto,M.(2003) Intracellular mRNA cleavage by 3’ tRNase under the direction of 2’-O-methylRNA heptamers. Nucleic Acids Res., 31, 4354-4360.

Non-patent Document 4: Habu,Y., Miyano-Kurosaki,N., Kitano,M., Endo,Y., Yukita,M., Ohira,S., Takaku,H., Nashimoto,M. and Takaku,H. (2005) Inhibition of HIV-1 gene expression by retroviral vector-mediated small-guide RNAs that direct specific RNA cleavage by tRNase ZL. Nucleic Acids Res., 33, 235-243.

Non-patent Document 5: Takako Sano, Masayuki Takahashi, Tadasuke Nozaki, Yoshiaki Takahashi, Masato Tamura, and Masayuki Nashimoto. (2011) Expanding the utility of heptamer-type sgRNA for TRUE gene silencing. Biochem. and Biophys. Res. Commun. 416, 427-432.

Non-patent Document 6: Masayuki Takahashi, Reyad A. Elbarbary, Aiko Nakashima, Mayumi Abe, Norihiro Watanabe, Miwako Narita, Masuhiro Takahashi, Masato Tamura, Tetsuo Yoshida, and Masayuki Nashimoto. (2013) A naked RNA heptamer targeting the human Bcl-2 mRNA induces apoptosis of HL60 leukemia cells. Cancer Letters 328, 362-368.

Non-patent Document 7: Norihiro Watanabe, Miwako Narita, Akie Yamahira, Tomoyo Taniguchi, Tatsuo Furukawa, Tetsuo Yoshida, Tatsuya Miyazawa, Masayuki Nashimoto, and Masuhiro Takahashi. (2013) Induction of apoptosis of leukemic cells by TRUE gene silencing using small guide RNAs targeting the WT1 mRNA. Leukemia Research 37, 580-585.

Non-patent Document 8: Satoshi Iizuka, Nobuhiko Oridate, Masayuki Nashimoto, Satoshi Fukuda, and Masato Tamura. (2014) Growth inhibition of head and neck squamous cell carcinoma cells by sgRNA targeting the cyclin D1 mRNA based on TRUE gene silencing. PLoS One 9, e114121.

Non-patent Document 9: Nakashima,A., Takaku,H., Shibata,H.S., Negishi,Y., Takagi,M., Tamura,M. and Nashimoto,M. (2007) Gene silencing by the tRNA maturase tRNase ZL under the direction of small guide RNA. Gene Therapy, 14, 78-85.

Non-patent Document 10: Elbarbary,R.A., Takaku,H., Tamura,M. and Nashimoto,M. (2009) Inhibition of vascular endothelial growth factor expression by TRUE gene silencing.Biochem.and Biophys. Res. Commun., 379, 924-927.

## Summary of the Invention

### Problems to be Solved by the Invention

One of the objectives of the present invention is to provide pharmaceutical compositions comprising relatively short oligonucleotides (small guide antisense oligonucleotides; hereafter also called sgASO) for regulating gene expression and methods of disease treatment using thereof. Also, for the objectives of the present invention, the present invention includes providing a method of designing relatively short oligonucleotides for modulating gene expression.

In TRUE gene silencing, in order for the small guide antisense oligonucleotide (sgASO) used as an active agent to bind target RNA and form a tRNase$Z^L$-substrate structure, it is necessary that at least one intrinsic stem-loop structure should be formed in the target RNA sequence, and it requires a step of identifying such sequences in the target RNA sequence. However, to date, no technique has been developed to comprehensively analyze secondary structures such as stem-loop structures or their stability from the primary sequence of the target RNA, with a level of precision to utilize in such drug discovery technology. In the present invention, one of the challenges is to provide a method of identifying sequences in the target RNA sequence, so that the sgASO binds to the target RNA, and at least one stem-loop structure is formed to become a substrate for tRNase$Z^L$.

### Means for Solving the Problems

The present inventors performed a structural analysis of the target RNA sequences subject to gene expression regulation based on the sequence information. Then, each of the existence probabilities was calculated from the obtained structural analysis results and their respective energy levels. By establishing a way of identifying a more favorable intrinsic stem-loop structure and designing sgASO by uniformly calculating the existence probabilities of intrinsic stem-loop substructures, the present inventors have developed a method to efficiently regulate intracellular gene expression by using relatively short oligonucleotides. The present invention is based on such a technique developed by the present inventors and encompasses the embodiments below.

Embodiment 1

A method for cleaving a target RNA in a eukaryotic cell, comprising:

i) identifying a sequence in the target RNA sequence where at least one stem-loop structure is formed by hybridization of a complementary 6-mer to 10-mer oligonucleotide (sgASO) to the target RNA, and

ii) preparing the sgASO and contacting it with the target RNA in the eukaryotic cell,

wherein the stem-loop structure formed through sgASO hybridization is recognized by tRNaseZ$^L$ within the eukaryotic cell to cleave the target RNA.

Embodiment 2

The method according to embodiment 1, wherein the number of base pairs in the stem portion of the stem-loop structure formed through sgASO hybridization is 11 to 14.

Embodiment 3

The method according to embodiment 1 or 2, wherein the number of base pairs in the stem portion of the stem-loop structure formed through sgASO hybridization is 12.

Embodiment 4

The method according to any of embodiments 1-3, wherein the sgASO is a 7-mer.

Embodiment 5

The method according to any of embodiments 1-4, wherein in the stem-loop structure formed through sgASO hybridization, the number of base pairs in the stem portion formed by the target RNA is 5.

Embodiment 6

The method according to any of embodiments 1-5, wherein in the stem-loop structure formed through sgASO hybridization, the loop portion is formed by the target RNA.

Embodiment 7

The method according to any of embodiments 1-6, wherein the number of bases in the loop portion of the stem-loop structure formed through sgASO hybridization is 3 to 10.

Embodiment 8

The method according to any of embodiments 1-7, wherein the number of bases in the loop portion of the stem-loop structure formed through sgASO hybridization is 4 to 8.

Embodiment 9

The method according to any of embodiments 1-8, wherein the number of bases in the loop portion of the stem-loop structure formed through sgASO hybridization is 7.

Embodiment 10

The method according to any of embodiments 1-9, wherein the sgASO is fully complementary to the target RNA.

Embodiment 11

The method according to any of embodiments 1-10, wherein the stem portion of the stem-loop structure formed through sgASO hybridization does not contain a mismatch or bulges.

Embodiment 12

The method according to any of embodiments 1-10, wherein the stem portion of the stem-loop structure formed through sgASO hybridization contains a mismatch or bulges.

Embodiment 13

The method according to embodiment 12, wherein in the counting of the number of base pairs in the stem portion, when the number of base pairs in the mismatch or bulges of the stem is 2 or less, the mismatch or bulges is considered to form a base pair and counted; and when the number of base pairs in the mismatch or bulges of the stem is 3 or more, half of the number of bases in the mismatch or bulges is counted as base pairs.

Embodiment 14

The method according to any of embodiments 1-13, wherein the target RNA is mRNA or ncRNA.

Embodiment 15

The method according to any of embodiments 1-14, wherein the target RNA is RNA from the nuclear genome DNA.

Embodiment 16

The method according to any of embodiments 1-14, wherein the target RNA is RNA from the mitochondrial genome DNA.

Embodiment 17

The method according to any of embodiments 1-14, wherein the target RNA is RNA from a viral or bacterial genome DNA and RNA.

Embodiment 18

The method according to any of embodiments 1-17, wherein the sgASO comprises a modified nucleoside and/or a modified internucleoside linkage.

Embodiment 19

The method according to any of embodiments 1-18, wherein the difference in the number of bases between the 5' terminal sequence and 3' terminal sequence of the stem portion formed by the target RNA is 1 or less.

Embodiment 20

The method according to any of embodiments 1-19, wherein one or both of the terminal hydroxyl groups of the sgASO are modified.

Embodiment 21

The method according to any of embodiments 1-20, wherein a phosphate group is added to one or both of the terminal hydroxyl groups of the sgASO.

Embodiment 22

The method according to any of embodiments 1-21, wherein when the bases constituting the sgASO-bound region on the target RNA from the 5' terminal are N1, N2, N3, N4, N5, N6 and N7, and further the first base adjacent to the 3' terminal of the region to which the sgASO binds is N8, at least one of the conditions 1-3 below is met:

- condition 1: N8 is A or G;
- condition 2: N7 is C; and
- condition 3: N6 is A, C or G.

Embodiment 23

The method according to any of embodiments 1-22, wherein the sgASO is an oligonucleotide consisting of any one sequence from SEQ ID NO.: 1 to SEQ ID NO.: 16384.

Embodiment 24

A pharmaceutical composition for use in the treatment or prevention of a disease or disorder in a patient, comprising an oligonucleotide of about 7 mers, wherein the oligonucleotide of about 7 mers is complementary to the target RNA associated with the disease or disorder, the target RNA can form at least one stem-loop structure, the oligonucleotide of about 7 mers can hybridize to the 3' side region of the stem loop structure to form at least one larger stem loop structure in conjunction with the stem loop formed by the target RNA, and wherein the formed structures are recognized by tRNaseZ$^L$ in the patient's body and the target mRNA is cleaved.

Embodiment 25

A pharmaceutical composition for the treatment or prevention of a cancer, which comprising a sgASO comprising any of the sequences selected from:

5'-GAAACUU-3';
5'-CUGUCAA-3';
5'-UCUUCAA-3';
5'-UUAUCGU-3';
5'-CUUAUAA-3';
5'-GCGGGGG-3'; and
5'-ACUCAAA-3'.

Embodiment 26

A method for designing an oligonucleotide (sgASO) to cleave a target RNA in a eukaryotic cell, comprising:

i) identifying a sequence in the target RNA sequence in which at least one stem-loop structure is formed,
ii) identifying a sequence of 6 to 10 bases on the 3' terminal adjacent to the stem-loop structure as a sgASO-binding sequence, and
iii) identifying a sequence complementary to the binding sequence as a sgASO sequence.

Embodiment 27

The method for designing an oligonucleotide (sgASO) according to embodiment 25, wherein

i) the number of base pairs in the stem portion of the stem-loop structure formed by the target RNA is 4 to 8;
ii) the number of bases in the loop portion of the stem-loop structure is 3 to 10;
iii) in the case where the stem portion of the stem-loop structure contains a mismatch or bulges, in the counting of the number of base pairs in the stem portion, when the number of base pairs in the mismatch or bulges of the stem is 2 or less, the mismatch or bulges is considered to form a base pair and counted; and when the number of base pairs in the mismatch or bulges of the stem is 3 or more, half of the number of bases in the mismatch or bulges is counted as base pairs; and
iv) the difference in the number of bases between the 5'-terminal sequence and 3'-terminal sequence of the stem portion is 1 or less.

Embodiment 28

The method according to embodiment 27, wherein when the bases constituting the sgASO-bound region on the target RNA from the 5' terminal are N1, N2, N3, N4, N5, N6 and N7, and further the first base adjacent to the 3' terminal of the region to which the sgASO binds is N8, at least one of the conditions 1-3 below is met:

- condition 1: N8 is A or G;
- condition 2: N7 is C; and

- condition 3: N6 is A, C or G.

Embodiment 29

A method of designing a small guide antisense oligonucleotide (sgASO), comprising

(1) identifying a stem loop with a high existence probability on the target RNA,
(2) evaluating the stem loop, and
(3) setting the seven bases sequence complimentary to 3' terminal sequence to the stem loop-forming base pair as the target sequence (sense strand) and identifying its antisense strand to be a sgASO sequence,

wherein the step of (1) identifying a stem loop with a high existence probability on the target RNA comprises:

i) a step of predicting structure, comprising setting frame n having the width of W bases by an increment of R bases starting from the 5' terminal, wherein the number of the resulting frames is nmax, computing the base-pair pattern which can be obtained by pattern matching for the constituent base sequence of the W bases in each frame n, applying known thermodynamic stability calculations to the result, and giving $\Delta G$ for each base-pair pattern;
ii) a step of analyzing structure, comprising hypothesizing based on the resulting mmax(n) structures predicted in frame n and respective energy level, that the state inside the cell within which the RNA is placed is in equilibrium, calculating the existence probability of each resulting predicted structure according to the Maxwell-Boltzmann statistics, wherein the existence probability of each predicted structure result is j(n,m) for the mth predicted result from the most stable structure among the resulting predicted structures in frame n;
iii) a step of calculating local existence probability, comprising setting p as a property profile of a loop and stem (characteristics of the stem loop defined by the position of base in the stem-constituting base pair) formed beginning from the absolute position x on the sequence rather than in the frame, and defining the stem-loop as motif(x, p), and defining the existence probability in frame n of the motif(x,p) as partial existence probability P_local(x,p,n), and calculating the value as sum $\sum$j(n,m) of the j values for the prediction results of structures in which the stem loop exists among all the resulting predicted structures obtained in the frame n, wherein the local existence probability P_local(x,p,n) of motif(x,p) in the frame n is represented below:

$$\mathrm{P_local(x,p,n)} = \sum_{m=1}^{\mathrm{mmax(n)}} \begin{cases} 0 & \text{... if motif (x, p)is not found in the m} - \text{th structure of frame n} \\ \mathrm{j(n,m)} & \text{... if motif (x, p)is found in the m} - \text{th structure of frame n} \end{cases}$$

iv) a step of calculating existence probability, comprising giving the existence probability P_global(x,p) of motif(x,p) among the entirety as $\sum$P_local(x,p,n)/n_all(x,p) when $\sum$P_local(x,p,n) is the result of sum of P_local(x,p,n) from frame 1 to nmax, and the number of frames in which the full length of the sequence constituting the stem-loop is contained within the frame is n_all(x,p), wherein the existence probability of motif(x,p) among the entirety is represented as below, and

$$\mathrm{P_global(x,p)} = \sum_{n=1}^{nmax} \frac{P_local(x,p,n)}{n_all}$$

v) a step of analyzing, comprising selecting a stem-loop based on the existence probability and property p, with respect to the obtained existence probability P_global(x,p) of motif(x, p).

## Brief Description of the Drawings

Figure 1 is a graphical representation of the result of testing the inhibition of Bcl-2 mRNA expression by the sgASO (VIS-1 to VIS-7) designed according to the present invention using the human leukemic cell line HL60. As a result, VIS-1 and VIS-5 were obtained as sgASO with a 2-fold activity relative to that of a previously reported sgASO, Hep1. The vertical axis shows the relative Bcl-2 mRNA levels with mock (sample with only water as medium) set as 1.

**Mode for Carrying Out the Invention**

The present inventors have developed a method for efficiently regulating gene expression in cells using relatively short oligonucleotides. The present invention is described in detail below.

TRUE gene silencing

The inventors of the present invention have developed a new gene expression repression technology named TRUE gene silencing (tRNase $Z^L$-utilizing efficacious gene silencing). tRNase $Z^L$ is a large molecular-weight type enzyme of endoribonuclease (tRNase Z or 3' tRNase) that processes the 3' end portion of tRNA, and it excises the 3' end portion of the precursor tRNA. This TRUE gene silencing is based on the unique enzymatic properties of mammalian tRNase $Z^L$ that any RNA can be cleaved at any point by recognizing pre-tRNA- or micro-pre-tRNA-like complexes formed by target RNAs and synthetic small guide antisense nucleic acids. Small guide antisense oligonucleotides (in particular, small guide RNAs) are categorized into four types: 5'-half tRNA (Nashimoto, M. (1996) Specific cleavage of target RNAs from HIV-1 with 5' half tRNA by mammalian tRNA 3' processing endoribonuclease. RNA, 2, 2523-2524.), 12-16 nt linear RNA (Shibata, H.S., Takaku, H., Takagi, M. and Nashimoto, M. (2005) The T loops structure is dispensable for substrate recognition by tRNase ZL. J. Biol. Chem., 280, 22326-22334.), heptameric RNA (Nashimoto, M., Geary, S., Tamura, M. and Kasper, R. (1998) RNA heptamers that directs RNA cleavage by mammalian tRNA 3' processing endoribonuclease. Nucleic Acids Res., 26, 2565-2571.), and hook-type RNA (Takaku, H., Minagawa, A., Takagi, M. and Nashimoto, M. (2004) A novel four-base-recognizing RNA cutter that can remove the single 3' terminal nucleotides from RNA molecules. Nucleic Acids Res., 32, e91.).

Previously, the efficacy of TRUE gene silencing in various mammalian cells has been demonstrated by introducing expression plasmids of small guide antisense oligonucleotides (sgASO) targeting various mRNAs or 2'-O-methylated RNAs. For example, the HIV-1 expression in Jurkat cells was suppressed by 5' half tRNA-type sgRNA for more than 18 days (Habu et al. (2005) above), and the luciferase expression in mouse liver was suppressed by heptamer-type sgASO (Nakashima et al. (2007) above). Moreover, the effects of TRUE gene silencing are comparable to those of RNA interference (RNA interference: RNAi), and in some cases have also been confirmed to outweigh the effects of RNAi.

It has been found that tRNase $Z^L$ is present not only in the nucleus but also in the cytosol, and that the same 5'-half tRNA used as sgASO is present in the cytosol. It has also been found that human cytosolic tRNase $Z^L$ regulates gene expression by cleaving mRNA under the guidance of 5'-half tRNA and that PPM1F mRNA is one of the bona fide targets of tRNase $Z^L$ (Elbarbary, R.A., Takaku, H., Uchiumi, N., Tamiya, H., Abe, M., Takahashi, M., Nishida, H. and Nashimoto, M. (2009) Modulation of gene expression by human cytosolic tRNase ZL through 5'-half-tRNA. PLoS ONE, 4, e5908.). Moreover, it has been proved that a portion of microRNAs including miR-103 can act as a hook-type sgASO and down-regulate gene expression through mRNA cleavage by cytosolic tRNase $Z^L$ (Elbarbary, R.A., Takaku, H., Uchiumi, N., Tamiya, H., Abe, M., Nishida, H. and Nashimoto, M. (2009) Human cytosolic tRNase ZL can downregulate gene expression through miRNA. FEBS Lett. 583, 3241-3246.). Thus, it is clear that TRUE gene silencing functions on the basis of the newly elucidated physiological roles of cytosolic tRNase $Z^L$ operated by small non-coding RNAs in cells.

One of the ultimate purposes of TRUE gene silencing is the use of sgASO as an agent for treating or preventing a disease caused by the expression of a specific gene. For example, the present inventors have previously found that the intracellular levels of mRNAs encoding Bcl-2 and VEGF, which have shown promise as molecular targets for cancer therapy, are suppressed by 5'-type half tRNA type sgASO, 14-nt linear sgASO or heptamer-type sgASO (Tamura et al. (2003) and Elbarbary et al. (2009) above). One embodiment of the present invention relates to a method for cleaving a target RNA within a eukaryotic cell, comprising: (1) identifying in the target RNA sequence a sequence in which at least one stem-loop structure is formed by hybridization of a 6mer to 10mer oligonucleotide (sgASO) complementary to the target RNA, and (2) preparing the sgASO and contacting it with the target RNA in a eukaryotic cell, wherein tRNase$Z^L$ in the eukaryotic cell recognizes the stem-loop structure formed by sgASO hybridization and cleaves the target RNA. The method can be applied both *in vitro* and *in vivo.* When applied *in vivo* to humans or non-human animals, such methods for cleaving target RNAs within eukaryotic cells may be used for the treatment of diseases or disorders related to the expression of undesirable genes. Thus, from another perspective, one of the embodiments of the present invention relates to a method for treating a disease or disorder related to the expression of a undesirable gene in a human or a non-human animal, comprising (1) identifying a sequence in the target RNA sequence in which at least one stem-loop structure is formed by hybridization of a 6-mer to 10-mer oligonucleotide (sgASO) complementary to a target RNA transcribed from a disease-related gene, and (2) preparing the sgASO and administering it to a human or a non-human animal to contact it with the target RNA in the cell, wherein tRNase$Z^L$ in the cell recognizes the stem-loop structure formed by sgASO hybridization and cleaves the target RNA. The disease of interest can be, for example, cancer.

Small guide antisense nucleic acid (sgASO).

In one embodiment, the small guide antisense nucleic acid is a relatively short oligonucleotide, for example, 6-mer to 10-mer, i.e., 6-mer, 7-mer, 8-mer, 9-mer, or 10-mer, preferably 7-mer oligonucleotide (heptamer-type sgASO). When sgASO is used as a pharmaceutical ingredient, a shorter sgASO is preferred from the pharmacological and CMC (Chemistry, Manufacturing and Control) perspectives. The reason is that a shorter sgASO can be synthesized more conveniently and less expensively than a long chain sgASO. Also, shorter sgASO can be easily internalized into cells without the use of cell transfection agents (transfection reagents, etc.) and DDS bases (Loke, S.L., Stein, C.A., Zhang X. H., Mori, K., Nakanishi, M., Subasinghe, C., Cohen, J.S. and Neckers, L.M. (1989) Characterization of oligonucleotide transport into living cells. Proc. Natl. Acad. Sci. USA, 86, 3474-3478.). In practical applications, 7-mer oligonucleotides may be preferred in terms of the advantage in synthesis and from the perspective of sequence recognition specificity.

The sgASO may be prepared by methods using known chemical syntheses, enzymatic transcription methods, or such. Methods using known chemical syntheses can include phosphoroamidite method, phosphorothioate method, phosphotriester method, and such; and synthesis can be done in ABI3900 high-throughput nucleic acid synthesizers (manufactured by Applied Biosystems, Inc.), NTS H-6 nucleic acid synthesizers (manufactured by Nippon Technology Services), and Oligoilot10 nucleic acid synthesizers (manufactured by GE Healthcare Inc.). Enzymatic transcription methods can include transcription using RNA polymerases such as T7, T3, and SP6RNA polymerases, and plasmids or DNAs with the sequence of interest as template. Heptamer-type sgASO produced by synthetic or transcriptional methods are then purified by HPLC and such. For example, at the time of HPLC purification, sgASO is eluted from the columns using a mixed solution of triethylammonium acetate (TEAA) or hexylammonium acetate (HAA) and acetonitrile. After the eluted solution is dialyzed with 1000 times the eluted volume of distilled water for 10 hours and the dialysis solution is lyophilized, it is cryopreserved until use. At the time of use, it is dissolved in distilled water, for example, so that the final concentration becomes about 100 $\mu$M.

The nucleic acids used in the sgASO of the present invention may be any nucleosides or molecules whose function is equivalent to the nucleosides, which are polymerized via inter-nucleoside bonds. Nucleosides are a class of compounds in which bases (nucleobases) and sugars are attached. Bases include purine bases such as adenine and guanine, pyrimidine bases such as thymine, cytosine and uracil, and nicotinamide and dimethylisoalloxazine. Adenosine, thymidine, guanosine, cytidine, uridine and such are representative nucleosides. A nucleotide is a substance in which a phosphate group is attached to a nucleoside. Oligonucleotides (also called polynucleotides) include, for example, RNA ribonucleotide polymers, DNA deoxyribonucleotide polymers, polymers of mixed RNA and DNA, and nucleotide polymers containing modified nucleosides. Natural DNA and RNA have phosphodiester bonds as internucleoside bonds. The nucleic acids used for the sgASO in the present invention may include modifications. The location of nucleic acid modifications includes sugar moieties, backbone (ligation) moieties, nucleobase (base) moieties, and 3' or 5' end moieties. Also, the sgASO used in the present invention may include a morpholino nucleic acid or a peptide nucleic acid.

Modified nucleosides

Modified nucleosides can include molecules that have been modified to ribonucleosides, deoxyribonucleosides, RNA or DNA to enhance or stabilize nuclease resistance, to enhance affinity with complementary strand nucleic acids, to increase cell permeability, or to be visualized, for example, in comparison with RNA or DNA. Examples include sugar-modified nucleosides. The sgASO of the present invention may include, for example, modified nucleic acid molecules disclosed in Khvorova & Watts (Nature Biotechnology 35, 238-248 (2017) doi:10.1038/nbt.3765).

Modified sugars refer to sugars with substitutions and/or any changes from natural sugar moieties (i.e., sugar moieties found in DNA (2'-H) or RNA (2'-OH)), whereas sugar-modified nucleosides refer to modified nucleosides containing modified sugars. Sugar-modified nucleosides can be any nucleosides whose sugar chemical structure is partially or entirely modified by addition of or substitution with any arbitrary chemical structure substance, for example, modified nucleosides by substitution with 2'-O-methylribose, modified nucleosides by substitution with 2'-methoxyethoxyribose, modified nucleosides by substitution with 2'-O-methoxyethylribose, modified nucleosides by substitution with 2'-O-[2-(guanidium)ethyl]ribose, modified nucleosides by substitution with 2'-O-fluororibose, bridged structure-type artificial nucleic acids (Bridged Nucleic Acid) with two cyclic structures (BNA) by introduction of a cross-linking structure into the sugar moiety, more specifically, locked artificial nucleic acids (Locked Nucleic Acid: LNA) with oxygen atoms at the 2' position and carbon atoms at the 4' position being cross-linked via methylene, ethylene bridged structure-type nucleic acids (Ethylene bridged nucleic acid: ENA) [Nucleic Acid Research, 32, e175 (2004)] and such; and may further include peptide nucleic acids (PNAs) [Acc. Chem. Res., 32, 624 (1999)], oxypeptide nucleic acids (OPNA) [J. Am. Chem. Soc., 123, 4653 (2001)], and peptide ribonucleic acids (PRNA) [J. Am. Chem. Soc., 122, 6900 (2000)].

RNA 2'-O-methyl (2'-OMe) modification (2'-OMe-RNA) is a naturally occurring modification that improves the binding affinity and nuclease resistance of modified oligonucleotides, as well as reduces immunostimulatory properties. 2'-O-Methoxyethyl (2'-MOE) increases the nuclease resistance more than the 2'-OMe modification, and the binding affinity (ΔTm) of the modified nucleotides is also greatly elevated. The sgASO containing 2'-OMe is also compatible with recognition and cleavage by tRNase$Z^L$. The 2'-fluoro (2'-F) modification of RNA (2'-F-RNA) can also be used to increase the affinity of oligonucleotides. Other 2'-modified nucleic acids may include 2'-F-ANA and 2'-modified derivatives of Kane et al. (Japanese Patent No. 5194256, Japanese Patent Application Kokai Publication No. (JP-A) 2015-020994 (unexamined, published Japanese patent application)).

LNA (Locked nucleic acid), which links the 2' oxygen and 4' carbon of ribose, leads to substantial increases in binding affinity. In LNA, the 2' oxygen and 4' carbon of the RNA ribose sugar are anchored in the ring structure. This modification increases specificity, affinity, and half-life, allowing effective delivery to the tissue of interest with lower toxicity. It is known that there is a tendency for oligomers longer than ~8 nucleotides that have been fully modified with LNA to aggregate, and it is commonly used in mixture with DNA and other sugar-modified nucleic acids. LNA-containing sgASO is also compatible with recognition and cleavage by tRNase$Z^L$.

A methylation analog of LNA, cEt, is as useful as LNA. Tricyclo-DNA (tcDNA) is a restricted form of nucleotides based on the three-ring scaffold.

As modified nucleosides, in addition, those with atoms (e.g., hydrogen atoms, oxygen atoms) or functional groups (e.g., hydroxyl groups, amino groups) in the base moiety of a nucleic acid being substituted with other atoms (e.g., hydrogen atoms, sulfur atoms), functional groups (e.g., amino groups), or alkyl groups of carbon numbers 1-6, or protected with protecting groups (e.g., methyl or acyl groups); and nucleosides added with phospholipid, phenazine, folate, phenanthridine, anthraquinone, acridine, fluorescein, rhodamine, coumarin, dye, or another chemical substance may be used.

Modified nucleobases (or modified bases) include any nucleobases other than adenine, cytosine, guanine, thymine, and uracil, for example, 5-methylcytosine, 5-fluorocytosine, 5-bromocytosine, 5-iodocytosine, N4-methylcytosine, 5-fluorouracil, 5-bromouracil, 5-iodouracil, 2-thiothymine, N6-methyladenine, 8-bromoadenine, N2-methylguanine, 8-bromoguanine, and inosine.

## Internucleoside linkages

Natural DNA and RNA have phosphodiester bonds as internucleoside bonds. In one embodiment of the present invention, the internucleoside linkage may comprise a modification. Modified internucleoside linkages refer to internucleoside linkages with substitutions or any change from naturally occurring internucleoside linkages (i.e., phosphodiester linkages); and modified internucleoside linkages include internucleoside linkages containing phosphorus atoms, and internucleoside linkages without phosphorus atoms. Modified nucleoside-to-nucleoside bonds may be those in which the chemical structure of a nucleotide's phosphate diester bond is partially or entirely added or substituted with any arbitrary chemical substance, for example, modified internucleoside bonds by substitution with phosphorothioate bonds, modified internucleoside bonds by substitution with N3'-P5' phosphoamidate bonds, and such. Other modified internucleoside linkages include ($S_{C5'}$,R)-α, β-CNA, and PMOs.

Representative phosphorus-containing internucleoside bonds are, for example, phosphodiester bonds, phosphorothioate bonds, phosphorodithioate bonds, phosphotriester bonds, and methylphosphonate bonds, methylthiophosphonate bonds, boranophosphate bonds, phosphoroamidate bonds, and such.

Phosphorothioate (PS) linkage, which is one of the major modified internucleoside linkages, serves to protect oligonucleotides from degradation by nucleases. The phosphorodithioate (PS) modification was originally incorporated into the oligonucleotide to confer nuclease resistance, but this modification also greatly influences oligonucleotide transportation and uptake. PS enhances binding to receptor sites and plasma proteins by altering the charge of ASOs, increasing the amount of ASOs that reach target tissues. Heparin-binding proteins are among the highest affinity targets of phosphorothioate-modified oligonucleotides. Proper binding by plasma proteins suppresses rapid elimination from the blood by the kidney system and facilitates optimal delivery. A sgASO with a phosphothioate bond is also compatible with recognition and cleavage by tRNase$Z^L$.

Phosphorothioate bonds have a stereocenter in phosphorus atomic moieties, and fully modified oligonucleotides usually form a mixture of diastereomers of $2^{n-1}$ species (e.g., 7-mer phosphorothioate oligonucleotides form a mixture of $2^6$ species of diastereomers). $S_p$ and $R_p$ diastereomeric binding are known to exhibit distinct properties. $R_p$ diastereomers are less nuclease resistant than $S_p$ diastereomers, but they bind complementary strands with higher affinities. In the sgASO of the present invention, synthesis may be regulated so that a particular phosphorothioate bond becomes a particular diastereomer.

## Ligand-linked oligonucleotides/terminally modified oligonucleotides

Molecules in which another chemical substance is added to a nucleic acid are, for example, 5'-polyamine-conjugated derivatives, cholesterol-conjugated derivatives, corticosteroid- conjugated derivatives, bile acid-conjugated derivatives, vitamin- conjugated derivatives, Cy5- conjugated derivatives, Cy3- conjugated derivatives, 6-FAM- conjugated derivatives, biotin- conjugated derivatives, Kitaeji's derivatives (PCT/JP2007/000087, PCT/JP2016/59398) and such. Sites at which a ligand or such is added may be terminus of an oligonucleotide (5' end or 3' end) and/or within an oligonucleotide.

As examples of terminal modifications, GalNAc-linked oligonucleotides and PUFA-linked oligonucleotides are known. Ligating GalNAc to the end of an oligonucleotide can enhance its delivery efficiency to the liver. The sgASO used in the present invention may be linked to a ligand such as GalNAc.

Short oligonucleotides are more likely to be delivered to the kidney, and long oligomers are more likely to be delivered to the liver. Short oligonucleotides tend to bind poorly to plasma proteins, resulting in a shorter half-life in plasma, but multimers can be constructed using cleavable linkers or such. The sgASO used in the present invention may be linked to another sgASO using a cleavable linker or such.

A phosphate group may be added to the 5' end and/or 3' end of the sgASO in the present invention. Other terminal modifications include E-VP, methylphosphonates, phosphorothioates, and C-methyl analogs, which are known to enhance the stability of oligonucleotides. The sgASO used in the present invention may include these terminal modifications.

SgASO sequence design

In order for the sgASO in the present invention to enable specific cleavage of the target RNA by tRNase $Z^L$ through formation of a tRNA acceptor stem-like duplex with the target RNA via base pairing, the target site of the sgASO is directly below a stable hairpin structural region similar to the tRNA T arm. SgASO effectively cleaves RNA not only with its seven bases, but also by specificity of the sequence region that forms a stable hairpin structure. Therefore, the sgASO sequence can be designed based on the mRNA sequence data of the target gene.

One embodiment of the present invention relates to a method for cleaving a target RNA in a eukaryotic cell, comprising: (1) identifying in the target RNA sequence at least one stem-loop structure formed by hybridizing a 6-mer to 10-mer oligonucleotide (sgASO) complementary to the target RNA, and (2) preparing the sgASO and contacting it with the target RNA in a eukaryotic cell, wherein tRNase$Z^L$ in the eukaryotic cell recognizes the stem-loop structure formed by sgASO hybridization to cleave the target RNA.

Conformationally, the acceptor stem and T arm of the tRNA adopt a single stem-loop structure in tandem, and tRNase $Z^L$ is thought to recognize this stem-loop conformation and perform RNA cleavage. Thus, hybridization of the sgASO with a target RNA allows cleavage of the target RNA if it reproduces a single stem-loop structure similar to the structure formed by the acceptor stem and T arm.

For example, for human glutamate tRNA, the acceptor stem is 7 bases long, the stem portion of T arm is 5 bases long, and the loop portion of T arm is 7 bases long. Thus, the sgASO corresponding to the acceptor stem may be, for example, 7 bases long, but it is not necessarily limited thereto. The length of the sgASO may be, for example, 6-10 mers. Also, the T-arm-like stem-loop structure formed by the target RNA may be five bases long in the stem portion and seven bases in the loop portion, but it is not necessarily limited thereto. The length of the stem portion of the T-arm-like stem-loop structure formed by the target RNA may be, for example, 4-8 bases long. The number of bases in the loop portion can be, for example, 3-10 bases, preferably 4-8 bases. Moreover, the length of the sgASO and the length of the stem portion in the T-arm-like stem-loop structure formed by the target RNA can be a length of 12 bases in sum, but it is not necessarily limited thereto. The sum of the lengths can be, for example, 11-14 bases. However, in real applications, it may be preferable that the sgASO is a 7-mer oligonucleotide in terms of the advantage in synthesis and from the perspective of sequence recognition specificity. For the T-arm-like stem-loop structure formed by target RNA, it may be preferable to have a stem portion length of 5 bases in terms of stability and existence probability. From a similar point of view, for a T-arm-like stem-loop structure formed by target RNA, it may be preferable that the two strands of the stem portion have equal lengths (within one difference in the number of bases between the stem's 5' side sequence and 3' side sequence flanking the loop) and that the length of the loop portion does not exceed 10.

In designing the sgASO sequence, based on the results of the sequencing analysis of the tRNA gene performed by the present inventors, the bases constituting the sgASO-bound region on the target RNA from the 5' side are defined as N1, N2, N3, N4, N5, N6 and N7, and further the first base adjacent to the 3' side of the region to which the sgASO binds is defined as N8. The sequence may be designed to satisfy at least one of the conditions 1 to 3 below, or the sequence may be designed to satisfy at least two of the conditions 1 to 3 or all of the conditions 1 to 3:

- condition 1: N8 is A or G;
- condition 2: N7 is C; and
- condition 3: N6 is A, C or G.

The T-arm-like stem-loop structure formed by the target RNA and/or the acceptor stem-like structure formed by the target RNA and the sgASO may include non-Watson-Crick base pairs besides Watson-Crick base pairs (e.g., G-U bonds). The stem may also contain a mismatch or bulge.

If the sgASO is a 7-mer, the sgASO may be an oligonucleotide consisting of any one sequence from Sequence Number 1 to Sequence Number 16384. In addition, the term "Sequence Number n" may be referred to as "SEQ ID NO: n" in the present specification. Sequences from SEQ ID NO: 1 to SEQ ID NO: 16384 are shown in the table below.

Table 1

| | | | | | | | | | | | |
|---|---|---|---|---|---|---|---|---|---|---|---|
| SEQ ID NO: 1 | AAAAAAA | SEQ ID NO: 52 | AAAAUAU | SEQ ID NO: 103 | AAACGCG | SEQ ID NO: 154 | AAAGCGC | SEQ ID NO: 205 | AAAUAUA | SEQ ID NO: 256 | AAAUUUU |
| SEQ ID NO: 2 | AAAAAAC | SEQ ID NO: 53 | AAAAUCA | SEQ ID NO: 104 | AAACGCU | SEQ ID NO: 155 | AAAGCGG | SEQ ID NO: 206 | AAAUAUC | SEQ ID NO: 257 | AACAAAA |
| SEQ ID NO: 3 | AAAAAAG | SEQ ID NO: 54 | AAAAUCC | SEQ ID NO: 105 | AAACGGA | SEQ ID NO: 156 | AAAGCGU | SEQ ID NO: 207 | AAAUAUG | SEQ ID NO: 258 | AACAAAC |
| SEQ ID NO: 4 | AAAAAAU | SEQ ID NO: 55 | AAAAUCG | SEQ ID NO: 106 | AAACGGC | SEQ ID NO: 157 | AAAGCUA | SEQ ID NO: 208 | AAAUAUU | SEQ ID NO: 259 | AACAAAG |
| SEQ ID NO: 5 | AAAAACA | SEQ ID NO: 56 | AAAAUCU | SEQ ID NO: 107 | AAACGGG | SEQ ID NO: 158 | AAAGCUC | SEQ ID NO: 209 | AAAUCAA | SEQ ID NO: 260 | AACAAAU |
| SEQ ID NO: 6 | AAAAACC | SEQ ID NO: 57 | AAAAUGA | SEQ ID NO: 108 | AAACGGU | SEQ ID NO: 159 | AAAGCUG | SEQ ID NO: 210 | AAAUCAC | SEQ ID NO: 261 | AACAACA |
| SEQ ID NO: 7 | AAAAACG | SEQ ID NO: 58 | AAAAUGC | SEQ ID NO: 109 | AAACGUA | SEQ ID NO: 160 | AAAGCUU | SEQ ID NO: 211 | AAAUCAG | SEQ ID NO: 262 | AACAACC |
| SEQ ID NO: 8 | AAAAACU | SEQ ID NO: 59 | AAAAUGG | SEQ ID NO: 110 | AAACGUC | SEQ ID NO: 161 | AAAGGAA | SEQ ID NO: 212 | AAAUCAU | SEQ ID NO: 263 | AACAACG |
| SEQ ID NO: 9 | AAAAAGA | SEQ ID NO: 60 | AAAAUGU | SEQ ID NO: 111 | AAACGUG | SEQ ID NO: 162 | AAAGGAC | SEQ ID NO: 213 | AAAUCCA | SEQ ID NO: 264 | AACAACU |
| SEQ ID NO: 10 | AAAAAGC | SEQ ID NO: 61 | AAAAUUA | SEQ ID NO: 112 | AAACGUU | SEQ ID NO: 163 | AAAGGAG | SEQ ID NO: 214 | AAAUCCC | SEQ ID NO: 265 | AACAAGA |
| SEQ ID NO: 11 | AAAAAGG | SEQ ID NO: 62 | AAAAUUC | SEQ ID NO: 113 | AAACUAA | SEQ ID NO: 164 | AAAGGAU | SEQ ID NO: 215 | AAAUCCG | SEQ ID NO: 266 | AACAAGC |
| SEQ ID NO: 12 | AAAAAGU | SEQ ID NO: 63 | AAAAUUG | SEQ ID NO: 114 | AAACUAC | SEQ ID NO: 165 | AAAGGCA | SEQ ID NO: 216 | AAAUCCU | SEQ ID NO: 267 | AACAAGG |
| SEQ ID NO: 13 | AAAAAUA | SEQ ID NO: 64 | AAAAUUU | SEQ ID NO: 115 | AAACUAG | SEQ ID NO: 166 | AAAGGCC | SEQ ID NO: 217 | AAAUCGA | SEQ ID NO: 268 | AACAAGU |
| SEQ ID NO: 14 | AAAAAUC | SEQ ID NO: 65 | AAACAAA | SEQ ID NO: 116 | AAACUAU | SEQ ID NO: 167 | AAAGGCG | SEQ ID NO: 218 | AAAUCGC | SEQ ID NO: 269 | AACAAUA |
| SEQ ID NO: 15 | AAAAAUG | SEQ ID NO: 66 | AAACAAC | SEQ ID NO: 117 | AAACUCA | SEQ ID NO: 168 | AAAGGCU | SEQ ID NO: 219 | AAAUCGG | SEQ ID NO: 270 | AACAAUC |
| SEQ ID NO: 16 | AAAAAUU | SEQ ID NO: 67 | AAACAAG | SEQ ID NO: 118 | AAACUCC | SEQ ID NO: 169 | AAAGGGA | SEQ ID NO: 220 | AAAUCGU | SEQ ID NO: 271 | AACAAUG |
| SEQ ID NO: 17 | AAAACAA | SEQ ID NO: 68 | AAACAAU | SEQ ID NO: 119 | AAACUCG | SEQ ID NO: 170 | AAAGGGC | SEQ ID NO: 221 | AAAUCUA | SEQ ID NO: 272 | AACAAUU |
| SEQ ID NO: 18 | AAAACAC | SEQ ID NO: 69 | AAACACA | SEQ ID NO: 120 | AAACUCU | SEQ ID NO: 171 | AAAGGGG | SEQ ID NO: 222 | AAAUCUC | SEQ ID NO: 273 | AACACAA |
| SEQ ID NO: 19 | AAAACAG | SEQ ID NO: 70 | AAACACC | SEQ ID NO: 121 | AAACUGA | SEQ ID NO: 172 | AAAGGGU | SEQ ID NO: 223 | AAAUCUG | SEQ ID NO: 274 | AACACAC |
| SEQ ID NO: 20 | AAAACAU | SEQ ID NO: 71 | AAACACG | SEQ ID NO: 122 | AAACUGC | SEQ ID NO: 173 | AAAGGUA | SEQ ID NO: 224 | AAAUCUU | SEQ ID NO: 275 | AACACAG |
| SEQ ID NO: 21 | AAAACCA | SEQ ID NO: 72 | AAACACU | SEQ ID NO: 123 | AAACUGG | SEQ ID NO: 174 | AAAGGUC | SEQ ID NO: 225 | AAAUGAA | SEQ ID NO: 276 | AACACAU |
| SEQ ID NO: 22 | AAAACCC | SEQ ID NO: 73 | AAACAGA | SEQ ID NO: 124 | AAACUGU | SEQ ID NO: 175 | AAAGGUG | SEQ ID NO: 226 | AAAUGAC | SEQ ID NO: 277 | AACACCA |
| SEQ ID NO: 23 | AAAACCG | SEQ ID NO: 74 | AAACAGC | SEQ ID NO: 125 | AAACUUA | SEQ ID NO: 176 | AAAGGUU | SEQ ID NO: 227 | AAAUGAG | SEQ ID NO: 278 | AACACCC |
| SEQ ID NO: 24 | AAAACCU | SEQ ID NO: 75 | AAACAGG | SEQ ID NO: 126 | AAACUUC | SEQ ID NO: 177 | AAAGUAA | SEQ ID NO: 228 | AAAUGAU | SEQ ID NO: 279 | AACACCG |
| SEQ ID NO: 25 | AAAACGA | SEQ ID NO: 76 | AAACAGU | SEQ ID NO: 127 | AAACUUG | SEQ ID NO: 178 | AAAGUAC | SEQ ID NO: 229 | AAAUGCA | SEQ ID NO: 280 | AACACCU |
| SEQ ID NO: 26 | AAAACGC | SEQ ID NO: 77 | AAACAUA | SEQ ID NO: 128 | AAACUUU | SEQ ID NO: 179 | AAAGUAG | SEQ ID NO: 230 | AAAUGCC | SEQ ID NO: 281 | AACACGA |
| SEQ ID NO: 27 | AAAACGG | SEQ ID NO: 78 | AAACAUC | SEQ ID NO: 129 | AAAGAAA | SEQ ID NO: 180 | AAAGUAU | SEQ ID NO: 231 | AAAUGCG | SEQ ID NO: 282 | AACACGC |
| SEQ ID NO: 28 | AAAACGU | SEQ ID NO: 79 | AAACAUG | SEQ ID NO: 130 | AAAGAAC | SEQ ID NO: 181 | AAAGUCA | SEQ ID NO: 232 | AAAUGCU | SEQ ID NO: 283 | AACACGG |
| SEQ ID NO: 29 | AAAACUA | SEQ ID NO: 80 | AAACAUU | SEQ ID NO: 131 | AAAGAAG | SEQ ID NO: 182 | AAAGUCC | SEQ ID NO: 233 | AAAUGGA | SEQ ID NO: 284 | AACACGU |
| SEQ ID NO: 30 | AAAACUC | SEQ ID NO: 81 | AAACCAA | SEQ ID NO: 132 | AAAGAAU | SEQ ID NO: 183 | AAAGUCG | SEQ ID NO: 234 | AAAUGGC | SEQ ID NO: 285 | AACACUA |
| SEQ ID NO: 31 | AAAACUG | SEQ ID NO: 82 | AAACCAC | SEQ ID NO: 133 | AAAGACA | SEQ ID NO: 184 | AAAGUCU | SEQ ID NO: 235 | AAAUGGG | SEQ ID NO: 286 | AACACUC |
| SEQ ID NO: 32 | AAAACUU | SEQ ID NO: 83 | AAACCAG | SEQ ID NO: 134 | AAAGACC | SEQ ID NO: 185 | AAAGUGA | SEQ ID NO: 236 | AAAUGGU | SEQ ID NO: 287 | AACACUG |
| SEQ ID NO: 33 | AAAAGAA | SEQ ID NO: 84 | AAACCAU | SEQ ID NO: 135 | AAAGACG | SEQ ID NO: 186 | AAAGUGC | SEQ ID NO: 237 | AAAUGUA | SEQ ID NO: 288 | AACACUU |
| SEQ ID NO: 34 | AAAAGAC | SEQ ID NO: 85 | AAACCCA | SEQ ID NO: 136 | AAAGACU | SEQ ID NO: 187 | AAAGUGG | SEQ ID NO: 238 | AAAUGUC | SEQ ID NO: 289 | AACAGAA |
| SEQ ID NO: 35 | AAAAGAG | SEQ ID NO: 86 | AAACCCC | SEQ ID NO: 137 | AAAGAGA | SEQ ID NO: 188 | AAAGUGU | SEQ ID NO: 239 | AAAUGUG | SEQ ID NO: 290 | AACAGAC |
| SEQ ID NO: 36 | AAAAGAU | SEQ ID NO: 87 | AAACCCG | SEQ ID NO: 138 | AAAGAGC | SEQ ID NO: 189 | AAAGUUA | SEQ ID NO: 240 | AAAUGUU | SEQ ID NO: 291 | AACAGAG |
| SEQ ID NO: 37 | AAAAGCA | SEQ ID NO: 88 | AAACCCU | SEQ ID NO: 139 | AAAGAGG | SEQ ID NO: 190 | AAAGUUC | SEQ ID NO: 241 | AAAUUAA | SEQ ID NO: 292 | AACAGAU |
| SEQ ID NO: 38 | AAAAGCC | SEQ ID NO: 89 | AAACCGA | SEQ ID NO: 140 | AAAGAGU | SEQ ID NO: 191 | AAAGUUG | SEQ ID NO: 242 | AAAUUAC | SEQ ID NO: 293 | AACAGCA |
| SEQ ID NO: 39 | AAAAGCG | SEQ ID NO: 90 | AAACCGC | SEQ ID NO: 141 | AAAGAUA | SEQ ID NO: 192 | AAAGUUU | SEQ ID NO: 243 | AAAUUAG | SEQ ID NO: 294 | AACAGCC |
| SEQ ID NO: 40 | AAAAGCU | SEQ ID NO: 91 | AAACCGG | SEQ ID NO: 142 | AAAGAUC | SEQ ID NO: 193 | AAAUAAA | SEQ ID NO: 244 | AAAUUAU | SEQ ID NO: 295 | AACAGCG |
| SEQ ID NO: 41 | AAAAGGA | SEQ ID NO: 92 | AAACCGU | SEQ ID NO: 143 | AAAGAUG | SEQ ID NO: 194 | AAAUAAC | SEQ ID NO: 245 | AAAUUCA | SEQ ID NO: 296 | AACAGCU |
| SEQ ID NO: 42 | AAAAGGC | SEQ ID NO: 93 | AAACCUA | SEQ ID NO: 144 | AAAGAUU | SEQ ID NO: 195 | AAAUAAG | SEQ ID NO: 246 | AAAUUCC | SEQ ID NO: 297 | AACAGGA |
| SEQ ID NO: 43 | AAAAGGG | SEQ ID NO: 94 | AAACCUC | SEQ ID NO: 145 | AAAGCAA | SEQ ID NO: 196 | AAAUAAU | SEQ ID NO: 247 | AAAUUCG | SEQ ID NO: 298 | AACAGGC |
| SEQ ID NO: 44 | AAAAGGU | SEQ ID NO: 95 | AAACCUG | SEQ ID NO: 146 | AAAGCAC | SEQ ID NO: 197 | AAAUACA | SEQ ID NO: 248 | AAAUUCU | SEQ ID NO: 299 | AACAGGG |
| SEQ ID NO: 45 | AAAAGUA | SEQ ID NO: 96 | AAACCUU | SEQ ID NO: 147 | AAAGCAG | SEQ ID NO: 198 | AAAUACC | SEQ ID NO: 249 | AAAUUGA | SEQ ID NO: 300 | AACAGGU |
| SEQ ID NO: 46 | AAAAGUC | SEQ ID NO: 97 | AAACGAA | SEQ ID NO: 148 | AAAGCAU | SEQ ID NO: 199 | AAAUACG | SEQ ID NO: 250 | AAAUUGC | SEQ ID NO: 301 | AACAGUA |
| SEQ ID NO: 47 | AAAAGUG | SEQ ID NO: 98 | AAACGAC | SEQ ID NO: 149 | AAAGCCA | SEQ ID NO: 200 | AAAUACU | SEQ ID NO: 251 | AAAUUGG | SEQ ID NO: 302 | AACAGUC |
| SEQ ID NO: 48 | AAAAGUU | SEQ ID NO: 99 | AAACGAG | SEQ ID NO: 150 | AAAGCCC | SEQ ID NO: 201 | AAAUAGA | SEQ ID NO: 252 | AAAUUGU | SEQ ID NO: 303 | AACAGUG |
| SEQ ID NO: 49 | AAAAUAA | SEQ ID NO: 100 | AAACGAU | SEQ ID NO: 151 | AAAGCCG | SEQ ID NO: 202 | AAAUAGC | SEQ ID NO: 253 | AAAUUUA | SEQ ID NO: 304 | AACAGUU |
| SEQ ID NO: 50 | AAAAUAC | SEQ ID NO: 101 | AAACGCA | SEQ ID NO: 152 | AAAGCCU | SEQ ID NO: 203 | AAAUAGG | SEQ ID NO: 254 | AAAUUUC | SEQ ID NO: 305 | AACAUAA |
| SEQ ID NO: 51 | AAAAUAG | SEQ ID NO: 102 | AAACGCC | SEQ ID NO: 153 | AAAGCGA | SEQ ID NO: 204 | AAAUAGU | SEQ ID NO: 255 | AAAUUUG | SEQ ID NO: 306 | AACAUAC |

Table 2

| | | | | | | | | | | | |
|---|---|---|---|---|---|---|---|---|---|---|---|
| SEQ ID NO: 307 | AACAUAG | SEQ ID NO: 358 | AACCGCC | SEQ ID NO: 409 | AACGCGA | SEQ ID NO: 460 | AACUAGU | SEQ ID NO: 511 | AACUUUG | SEQ ID NO: 562 | AAGAUAC |
| SEQ ID NO: 308 | AACAUAU | SEQ ID NO: 359 | AACCGCG | SEQ ID NO: 410 | AACGCGC | SEQ ID NO: 461 | AACUAUA | SEQ ID NO: 512 | AACUUUU | SEQ ID NO: 563 | AAGAUAG |
| SEQ ID NO: 309 | AACAUCA | SEQ ID NO: 360 | AACCGCU | SEQ ID NO: 411 | AACGCGG | SEQ ID NO: 462 | AACUAUC | SEQ ID NO: 513 | AAGAAAA | SEQ ID NO: 564 | AAGAUAU |
| SEQ ID NO: 310 | AACAUCC | SEQ ID NO: 361 | AACCGGA | SEQ ID NO: 412 | AACGCGU | SEQ ID NO: 463 | AACUAUG | SEQ ID NO: 514 | AAGAAAC | SEQ ID NO: 565 | AAGAUCA |
| SEQ ID NO: 311 | AACAUCG | SEQ ID NO: 362 | AACCGGC | SEQ ID NO: 413 | AACGCUA | SEQ ID NO: 464 | AACUAUU | SEQ ID NO: 515 | AAGAAAG | SEQ ID NO: 566 | AAGAUCC |
| SEQ ID NO: 312 | AACAUCU | SEQ ID NO: 363 | AACCGGG | SEQ ID NO: 414 | AACGCUC | SEQ ID NO: 465 | AACUCAA | SEQ ID NO: 516 | AAGAAAU | SEQ ID NO: 567 | AAGAUCG |
| SEQ ID NO: 313 | AACAUGA | SEQ ID NO: 364 | AACCGGU | SEQ ID NO: 415 | AACGCUG | SEQ ID NO: 466 | AACUCAC | SEQ ID NO: 517 | AAGAACA | SEQ ID NO: 568 | AAGAUCU |
| SEQ ID NO: 314 | AACAUGC | SEQ ID NO: 365 | AACCGUA | SEQ ID NO: 416 | AACGCUU | SEQ ID NO: 467 | AACUCAG | SEQ ID NO: 518 | AAGAACC | SEQ ID NO: 569 | AAGAUGA |
| SEQ ID NO: 315 | AACAUGG | SEQ ID NO: 366 | AACCGUC | SEQ ID NO: 417 | AACGGAA | SEQ ID NO: 468 | AACUCAU | SEQ ID NO: 519 | AAGAACG | SEQ ID NO: 570 | AAGAUGC |
| SEQ ID NO: 316 | AACAUGU | SEQ ID NO: 367 | AACCGUG | SEQ ID NO: 418 | AACGGAC | SEQ ID NO: 469 | AACUCCA | SEQ ID NO: 520 | AAGAACU | SEQ ID NO: 571 | AAGAUGG |
| SEQ ID NO: 317 | AACAUUA | SEQ ID NO: 368 | AACCGUU | SEQ ID NO: 419 | AACGGAG | SEQ ID NO: 470 | AACUCCC | SEQ ID NO: 521 | AAGAAGA | SEQ ID NO: 572 | AAGAUGU |
| SEQ ID NO: 318 | AACAUUC | SEQ ID NO: 369 | AACCUAA | SEQ ID NO: 420 | AACGGAU | SEQ ID NO: 471 | AACUCCG | SEQ ID NO: 522 | AAGAAGC | SEQ ID NO: 573 | AAGAUUA |
| SEQ ID NO: 319 | AACAUUG | SEQ ID NO: 370 | AACCUAC | SEQ ID NO: 421 | AACGGCA | SEQ ID NO: 472 | AACUCCU | SEQ ID NO: 523 | AAGAAGG | SEQ ID NO: 574 | AAGAUUC |
| SEQ ID NO: 320 | AACAUUU | SEQ ID NO: 371 | AACCUAG | SEQ ID NO: 422 | AACGGCC | SEQ ID NO: 473 | AACUCGA | SEQ ID NO: 524 | AAGAAGU | SEQ ID NO: 575 | AAGAUUG |
| SEQ ID NO: 321 | AACCAAA | SEQ ID NO: 372 | AACCUAU | SEQ ID NO: 423 | AACGGCG | SEQ ID NO: 474 | AACUCGC | SEQ ID NO: 525 | AAGAAUA | SEQ ID NO: 576 | AAGAUUU |
| SEQ ID NO: 322 | AACCAAC | SEQ ID NO: 373 | AACCUCA | SEQ ID NO: 424 | AACGGCU | SEQ ID NO: 475 | AACUCGG | SEQ ID NO: 526 | AAGAAUC | SEQ ID NO: 577 | AAGCAAA |
| SEQ ID NO: 323 | AACCAAG | SEQ ID NO: 374 | AACCUCC | SEQ ID NO: 425 | AACGGGA | SEQ ID NO: 476 | AACUCGU | SEQ ID NO: 527 | AAGAAUG | SEQ ID NO: 578 | AAGCAAC |
| SEQ ID NO: 324 | AACCAAU | SEQ ID NO: 375 | AACCUCG | SEQ ID NO: 426 | AACGGGC | SEQ ID NO: 477 | AACUCUA | SEQ ID NO: 528 | AAGAAUU | SEQ ID NO: 579 | AAGCAAG |
| SEQ ID NO: 325 | AACCACA | SEQ ID NO: 376 | AACCUCU | SEQ ID NO: 427 | AACGGGG | SEQ ID NO: 478 | AACUCUC | SEQ ID NO: 529 | AAGACAA | SEQ ID NO: 580 | AAGCAAU |
| SEQ ID NO: 326 | AACCACC | SEQ ID NO: 377 | AACCUGA | SEQ ID NO: 428 | AACGGGU | SEQ ID NO: 479 | AACUCUG | SEQ ID NO: 530 | AAGACAC | SEQ ID NO: 581 | AAGCACA |
| SEQ ID NO: 327 | AACCACG | SEQ ID NO: 378 | AACCUGC | SEQ ID NO: 429 | AACGGUA | SEQ ID NO: 480 | AACUCUU | SEQ ID NO: 531 | AAGACAG | SEQ ID NO: 582 | AAGCACC |
| SEQ ID NO: 328 | AACCACU | SEQ ID NO: 379 | AACCUGG | SEQ ID NO: 430 | AACGGUC | SEQ ID NO: 481 | AACUGAA | SEQ ID NO: 532 | AAGACAU | SEQ ID NO: 583 | AAGCACG |
| SEQ ID NO: 329 | AACCAGA | SEQ ID NO: 380 | AACCUGU | SEQ ID NO: 431 | AACGGUG | SEQ ID NO: 482 | AACUGAC | SEQ ID NO: 533 | AAGACCA | SEQ ID NO: 584 | AAGCACU |
| SEQ ID NO: 330 | AACCAGC | SEQ ID NO: 381 | AACCUUA | SEQ ID NO: 432 | AACGGUU | SEQ ID NO: 483 | AACUGAG | SEQ ID NO: 534 | AAGACCC | SEQ ID NO: 585 | AAGCAGA |
| SEQ ID NO: 331 | AACCAGG | SEQ ID NO: 382 | AACCUUC | SEQ ID NO: 433 | AACGUAA | SEQ ID NO: 484 | AACUGAU | SEQ ID NO: 535 | AAGACCG | SEQ ID NO: 586 | AAGCAGC |
| SEQ ID NO: 332 | AACCAGU | SEQ ID NO: 383 | AACCUUG | SEQ ID NO: 434 | AACGUAC | SEQ ID NO: 485 | AACUGCA | SEQ ID NO: 536 | AAGACCU | SEQ ID NO: 587 | AAGCAGG |
| SEQ ID NO: 333 | AACCAUA | SEQ ID NO: 384 | AACCUUU | SEQ ID NO: 435 | AACGUAG | SEQ ID NO: 486 | AACUGCC | SEQ ID NO: 537 | AAGACGA | SEQ ID NO: 588 | AAGCAGU |
| SEQ ID NO: 334 | AACCAUC | SEQ ID NO: 385 | AACGAAA | SEQ ID NO: 436 | AACGUAU | SEQ ID NO: 487 | AACUGCG | SEQ ID NO: 538 | AAGACGC | SEQ ID NO: 589 | AAGCAUA |
| SEQ ID NO: 335 | AACCAUG | SEQ ID NO: 386 | AACGAAC | SEQ ID NO: 437 | AACGUCA | SEQ ID NO: 488 | AACUGCU | SEQ ID NO: 539 | AAGACGG | SEQ ID NO: 590 | AAGCAUC |
| SEQ ID NO: 336 | AACCAUU | SEQ ID NO: 387 | AACGAAG | SEQ ID NO: 438 | AACGUCC | SEQ ID NO: 489 | AACUGGA | SEQ ID NO: 540 | AAGACGU | SEQ ID NO: 591 | AAGCAUG |
| SEQ ID NO: 337 | AACCCAA | SEQ ID NO: 388 | AACGAAU | SEQ ID NO: 439 | AACGUCG | SEQ ID NO: 490 | AACUGGC | SEQ ID NO: 541 | AAGACUA | SEQ ID NO: 592 | AAGCAUU |
| SEQ ID NO: 338 | AACCCAC | SEQ ID NO: 389 | AACGACA | SEQ ID NO: 440 | AACGUCU | SEQ ID NO: 491 | AACUGGG | SEQ ID NO: 542 | AAGACUC | SEQ ID NO: 593 | AAGCCAA |
| SEQ ID NO: 339 | AACCCAG | SEQ ID NO: 390 | AACGACC | SEQ ID NO: 441 | AACGUGA | SEQ ID NO: 492 | AACUGGU | SEQ ID NO: 543 | AAGACUG | SEQ ID NO: 594 | AAGCCAC |
| SEQ ID NO: 340 | AACCCAU | SEQ ID NO: 391 | AACGACG | SEQ ID NO: 442 | AACGUGC | SEQ ID NO: 493 | AACUGUA | SEQ ID NO: 544 | AAGACUU | SEQ ID NO: 595 | AAGCCAG |
| SEQ ID NO: 341 | AACCCCA | SEQ ID NO: 392 | AACGACU | SEQ ID NO: 443 | AACGUGG | SEQ ID NO: 494 | AACUGUC | SEQ ID NO: 545 | AAGAGAA | SEQ ID NO: 596 | AAGCCAU |
| SEQ ID NO: 342 | AACCCCC | SEQ ID NO: 393 | AACGAGA | SEQ ID NO: 444 | AACGUGU | SEQ ID NO: 495 | AACUGUG | SEQ ID NO: 546 | AAGAGAC | SEQ ID NO: 597 | AAGCCCA |
| SEQ ID NO: 343 | AACCCCG | SEQ ID NO: 394 | AACGAGC | SEQ ID NO: 445 | AACGUUA | SEQ ID NO: 496 | AACUGUU | SEQ ID NO: 547 | AAGAGAG | SEQ ID NO: 598 | AAGCCCC |
| SEQ ID NO: 344 | AACCCCU | SEQ ID NO: 395 | AACGAGG | SEQ ID NO: 446 | AACGUUC | SEQ ID NO: 497 | AACUUAA | SEQ ID NO: 548 | AAGAGAU | SEQ ID NO: 599 | AAGCCCG |
| SEQ ID NO: 345 | AACCCGA | SEQ ID NO: 396 | AACGAGU | SEQ ID NO: 447 | AACGUUG | SEQ ID NO: 498 | AACUUAC | SEQ ID NO: 549 | AAGAGCA | SEQ ID NO: 600 | AAGCCCU |
| SEQ ID NO: 346 | AACCCGC | SEQ ID NO: 397 | AACGAUA | SEQ ID NO: 448 | AACGUUU | SEQ ID NO: 499 | AACUUAG | SEQ ID NO: 550 | AAGAGCC | SEQ ID NO: 601 | AAGCCGA |
| SEQ ID NO: 347 | AACCCGG | SEQ ID NO: 398 | AACGAUC | SEQ ID NO: 449 | AACUAAA | SEQ ID NO: 500 | AACUUAU | SEQ ID NO: 551 | AAGAGCG | SEQ ID NO: 602 | AAGCCGC |
| SEQ ID NO: 348 | AACCCGU | SEQ ID NO: 399 | AACGAUG | SEQ ID NO: 450 | AACUAAC | SEQ ID NO: 501 | AACUUCA | SEQ ID NO: 552 | AAGAGCU | SEQ ID NO: 603 | AAGCCGG |
| SEQ ID NO: 349 | AACCCUA | SEQ ID NO: 400 | AACGAUU | SEQ ID NO: 451 | AACUAAG | SEQ ID NO: 502 | AACUUCC | SEQ ID NO: 553 | AAGAGGA | SEQ ID NO: 604 | AAGCCGU |
| SEQ ID NO: 350 | AACCCUC | SEQ ID NO: 401 | AACGCAA | SEQ ID NO: 452 | AACUAAU | SEQ ID NO: 503 | AACUUCG | SEQ ID NO: 554 | AAGAGGC | SEQ ID NO: 605 | AAGCCUA |
| SEQ ID NO: 351 | AACCCUG | SEQ ID NO: 402 | AACGCAC | SEQ ID NO: 453 | AACUACA | SEQ ID NO: 504 | AACUUCU | SEQ ID NO: 555 | AAGAGGG | SEQ ID NO: 606 | AAGCCUC |
| SEQ ID NO: 352 | AACCCUU | SEQ ID NO: 403 | AACGCAG | SEQ ID NO: 454 | AACUACC | SEQ ID NO: 505 | AACUUGA | SEQ ID NO: 556 | AAGAGGU | SEQ ID NO: 607 | AAGCCUG |
| SEQ ID NO: 353 | AACCGAA | SEQ ID NO: 404 | AACGCAU | SEQ ID NO: 455 | AACUACG | SEQ ID NO: 506 | AACUUGC | SEQ ID NO: 557 | AAGAGUA | SEQ ID NO: 608 | AAGCCUU |
| SEQ ID NO: 354 | AACCGAC | SEQ ID NO: 405 | AACGCCA | SEQ ID NO: 456 | AACUACU | SEQ ID NO: 507 | AACUUGG | SEQ ID NO: 558 | AAGAGUC | SEQ ID NO: 609 | AAGCGAA |
| SEQ ID NO: 355 | AACCGAG | SEQ ID NO: 406 | AACGCCC | SEQ ID NO: 457 | AACUAGA | SEQ ID NO: 508 | AACUUGU | SEQ ID NO: 559 | AAGAGUG | SEQ ID NO: 610 | AAGCGAC |
| SEQ ID NO: 356 | AACCGAU | SEQ ID NO: 407 | AACGCCG | SEQ ID NO: 458 | AACUAGC | SEQ ID NO: 509 | AACUUUA | SEQ ID NO: 560 | AAGAGUU | SEQ ID NO: 611 | AAGCGAG |
| SEQ ID NO: 357 | AACCGCA | SEQ ID NO: 408 | AACGCCU | SEQ ID NO: 459 | AACUAGG | SEQ ID NO: 510 | AACUUUC | SEQ ID NO: 561 | AAGAUAA | SEQ ID NO: 612 | AAGCGAU |

Table 3

| | |
|---|---|
| SEQ ID NO: 613 | AAGCGCA |
| SEQ ID NO: 614 | AAGCGCC |
| SEQ ID NO: 615 | AAGCGCG |
| SEQ ID NO: 616 | AAGCGCU |
| SEQ ID NO: 617 | AAGCGGA |
| SEQ ID NO: 618 | AAGCGGC |
| SEQ ID NO: 619 | AAGCGGG |
| SEQ ID NO: 620 | AAGCGGU |
| SEQ ID NO: 621 | AAGCGUA |
| SEQ ID NO: 622 | AAGCGUC |
| SEQ ID NO: 623 | AAGCGUG |
| SEQ ID NO: 624 | AAGCGUU |
| SEQ ID NO: 625 | AAGCUAA |
| SEQ ID NO: 626 | AAGCUAC |
| SEQ ID NO: 627 | AAGCUAG |
| SEQ ID NO: 628 | AAGCUAU |
| SEQ ID NO: 629 | AAGCUCA |
| SEQ ID NO: 630 | AAGCUCC |
| SEQ ID NO: 631 | AAGCUCG |
| SEQ ID NO: 632 | AAGCUCU |
| SEQ ID NO: 633 | AAGCUGA |
| SEQ ID NO: 634 | AAGCUGC |
| SEQ ID NO: 635 | AAGCUGG |
| SEQ ID NO: 636 | AAGCUGU |
| SEQ ID NO: 637 | AAGCUUA |
| SEQ ID NO: 638 | AAGCUUC |
| SEQ ID NO: 639 | AAGCUUG |
| SEQ ID NO: 640 | AAGCUUU |
| SEQ ID NO: 641 | AAGGAAA |
| SEQ ID NO: 642 | AAGGAAC |
| SEQ ID NO: 643 | AAGGAAG |
| SEQ ID NO: 644 | AAGGAAU |
| SEQ ID NO: 645 | AAGGACA |
| SEQ ID NO: 646 | AAGGACC |
| SEQ ID NO: 647 | AAGGACG |
| SEQ ID NO: 648 | AAGGACU |
| SEQ ID NO: 649 | AAGGAGA |
| SEQ ID NO: 650 | AAGGAGC |
| SEQ ID NO: 651 | AAGGAGG |
| SEQ ID NO: 652 | AAGGAGU |
| SEQ ID NO: 653 | AAGGAUA |
| SEQ ID NO: 654 | AAGGAUC |
| SEQ ID NO: 655 | AAGGAUG |
| SEQ ID NO: 656 | AAGGAUU |
| SEQ ID NO: 657 | AAGGCAA |
| SEQ ID NO: 658 | AAGGCAC |
| SEQ ID NO: 659 | AAGGCAG |
| SEQ ID NO: 660 | AAGGCAU |
| SEQ ID NO: 661 | AAGGCCA |
| SEQ ID NO: 662 | AAGGCCC |
| SEQ ID NO: 663 | AAGGCCG |
| SEQ ID NO: 664 | AAGGCCU |
| SEQ ID NO: 665 | AAGGCGA |
| SEQ ID NO: 666 | AAGGCGC |
| SEQ ID NO: 667 | AAGGCGG |
| SEQ ID NO: 668 | AAGGCGU |
| SEQ ID NO: 669 | AAGGCUA |
| SEQ ID NO: 670 | AAGGCUC |
| SEQ ID NO: 671 | AAGGCUG |
| SEQ ID NO: 672 | AAGGCUU |
| SEQ ID NO: 673 | AAGGGAA |
| SEQ ID NO: 674 | AAGGGAC |
| SEQ ID NO: 675 | AAGGGAG |
| SEQ ID NO: 676 | AAGGGAU |
| SEQ ID NO: 677 | AAGGGCA |
| SEQ ID NO: 678 | AAGGGCC |
| SEQ ID NO: 679 | AAGGGCG |
| SEQ ID NO: 680 | AAGGGCU |
| SEQ ID NO: 681 | AAGGGGA |
| SEQ ID NO: 682 | AAGGGGC |
| SEQ ID NO: 683 | AAGGGGG |
| SEQ ID NO: 684 | AAGGGGU |
| SEQ ID NO: 685 | AAGGGUA |
| SEQ ID NO: 686 | AAGGGUC |
| SEQ ID NO: 687 | AAGGGUG |
| SEQ ID NO: 688 | AAGGGUU |
| SEQ ID NO: 689 | AAGGUAA |
| SEQ ID NO: 690 | AAGGUAC |
| SEQ ID NO: 691 | AAGGUAG |
| SEQ ID NO: 692 | AAGGUAU |
| SEQ ID NO: 693 | AAGGUCA |
| SEQ ID NO: 694 | AAGGUCC |
| SEQ ID NO: 695 | AAGGUCG |
| SEQ ID NO: 696 | AAGGUCU |
| SEQ ID NO: 697 | AAGGUGA |
| SEQ ID NO: 698 | AAGGUGC |
| SEQ ID NO: 699 | AAGGUGG |
| SEQ ID NO: 700 | AAGGUGU |
| SEQ ID NO: 701 | AAGGUUA |
| SEQ ID NO: 702 | AAGGUUC |
| SEQ ID NO: 703 | AAGGUUG |
| SEQ ID NO: 704 | AAGGUUU |
| SEQ ID NO: 705 | AAGUAAA |
| SEQ ID NO: 706 | AAGUAAC |
| SEQ ID NO: 707 | AAGUAAG |
| SEQ ID NO: 708 | AAGUAAU |
| SEQ ID NO: 709 | AAGUACA |
| SEQ ID NO: 710 | AAGUACC |
| SEQ ID NO: 711 | AAGUACG |
| SEQ ID NO: 712 | AAGUACU |
| SEQ ID NO: 713 | AAGUAGA |
| SEQ ID NO: 714 | AAGUAGC |
| SEQ ID NO: 715 | AAGUAGG |
| SEQ ID NO: 716 | AAGUAGU |
| SEQ ID NO: 717 | AAGUAUA |
| SEQ ID NO: 718 | AAGUAUC |
| SEQ ID NO: 719 | AAGUAUG |
| SEQ ID NO: 720 | AAGUAUU |
| SEQ ID NO: 721 | AAGUCAA |
| SEQ ID NO: 722 | AAGUCAC |
| SEQ ID NO: 723 | AAGUCAG |
| SEQ ID NO: 724 | AAGUCAU |
| SEQ ID NO: 725 | AAGUCCA |
| SEQ ID NO: 726 | AAGUCCC |
| SEQ ID NO: 727 | AAGUCCG |
| SEQ ID NO: 728 | AAGUCCU |
| SEQ ID NO: 729 | AAGUCGA |
| SEQ ID NO: 730 | AAGUCGC |
| SEQ ID NO: 731 | AAGUCGG |
| SEQ ID NO: 732 | AAGUCGU |
| SEQ ID NO: 733 | AAGUCUA |
| SEQ ID NO: 734 | AAGUCUC |
| SEQ ID NO: 735 | AAGUCUG |
| SEQ ID NO: 736 | AAGUCUU |
| SEQ ID NO: 737 | AAGUGAA |
| SEQ ID NO: 738 | AAGUGAC |
| SEQ ID NO: 739 | AAGUGAG |
| SEQ ID NO: 740 | AAGUGAU |
| SEQ ID NO: 741 | AAGUGCA |
| SEQ ID NO: 742 | AAGUGCC |
| SEQ ID NO: 743 | AAGUGCG |
| SEQ ID NO: 744 | AAGUGCU |
| SEQ ID NO: 745 | AAGUGGA |
| SEQ ID NO: 746 | AAGUGGC |
| SEQ ID NO: 747 | AAGUGGG |
| SEQ ID NO: 748 | AAGUGGU |
| SEQ ID NO: 749 | AAGUGUA |
| SEQ ID NO: 750 | AAGUGUC |
| SEQ ID NO: 751 | AAGUGUG |
| SEQ ID NO: 752 | AAGUGUU |
| SEQ ID NO: 753 | AAGUUAA |
| SEQ ID NO: 754 | AAGUUAC |
| SEQ ID NO: 755 | AAGUUAG |
| SEQ ID NO: 756 | AAGUUAU |
| SEQ ID NO: 757 | AAGUUCA |
| SEQ ID NO: 758 | AAGUUCC |
| SEQ ID NO: 759 | AAGUUCG |
| SEQ ID NO: 760 | AAGUUCU |
| SEQ ID NO: 761 | AAGUUGA |
| SEQ ID NO: 762 | AAGUUGC |
| SEQ ID NO: 763 | AAGUUGG |
| SEQ ID NO: 764 | AAGUUGU |
| SEQ ID NO: 765 | AAGUUUA |
| SEQ ID NO: 766 | AAGUUUC |
| SEQ ID NO: 767 | AAGUUUG |
| SEQ ID NO: 768 | AAGUUUU |
| SEQ ID NO: 769 | AAUAAAA |
| SEQ ID NO: 770 | AAUAAAC |
| SEQ ID NO: 771 | AAUAAAG |
| SEQ ID NO: 772 | AAUAAAU |
| SEQ ID NO: 773 | AAUAACA |
| SEQ ID NO: 774 | AAUAACC |
| SEQ ID NO: 775 | AAUAACG |
| SEQ ID NO: 776 | AAUAACU |
| SEQ ID NO: 777 | AAUAAGA |
| SEQ ID NO: 778 | AAUAAGC |
| SEQ ID NO: 779 | AAUAAGG |
| SEQ ID NO: 780 | AAUAAGU |
| SEQ ID NO: 781 | AAUAAUA |
| SEQ ID NO: 782 | AAUAAUC |
| SEQ ID NO: 783 | AAUAAUG |
| SEQ ID NO: 784 | AAUAAUU |
| SEQ ID NO: 785 | AAUACAA |
| SEQ ID NO: 786 | AAUACAC |
| SEQ ID NO: 787 | AAUACAG |
| SEQ ID NO: 788 | AAUACAU |
| SEQ ID NO: 789 | AAUACCA |
| SEQ ID NO: 790 | AAUACCC |
| SEQ ID NO: 791 | AAUACCG |
| SEQ ID NO: 792 | AAUACCU |
| SEQ ID NO: 793 | AAUACGA |
| SEQ ID NO: 794 | AAUACGC |
| SEQ ID NO: 795 | AAUACGG |
| SEQ ID NO: 796 | AAUACGU |
| SEQ ID NO: 797 | AAUACUA |
| SEQ ID NO: 798 | AAUACUC |
| SEQ ID NO: 799 | AAUACUG |
| SEQ ID NO: 800 | AAUACUU |
| SEQ ID NO: 801 | AAUAGAA |
| SEQ ID NO: 802 | AAUAGAC |
| SEQ ID NO: 803 | AAUAGAG |
| SEQ ID NO: 804 | AAUAGAU |
| SEQ ID NO: 805 | AAUAGCA |
| SEQ ID NO: 806 | AAUAGCC |
| SEQ ID NO: 807 | AAUAGCG |
| SEQ ID NO: 808 | AAUAGCU |
| SEQ ID NO: 809 | AAUAGGA |
| SEQ ID NO: 810 | AAUAGGC |
| SEQ ID NO: 811 | AAUAGGG |
| SEQ ID NO: 812 | AAUAGGU |
| SEQ ID NO: 813 | AAUAGUA |
| SEQ ID NO: 814 | AAUAGUC |
| SEQ ID NO: 815 | AAUAGUG |
| SEQ ID NO: 816 | AAUAGUU |
| SEQ ID NO: 817 | AAUAUAA |
| SEQ ID NO: 818 | AAUAUAC |
| SEQ ID NO: 819 | AAUAUAG |
| SEQ ID NO: 820 | AAUAUAU |
| SEQ ID NO: 821 | AAUAUCA |
| SEQ ID NO: 822 | AAUAUCC |
| SEQ ID NO: 823 | AAUAUCG |
| SEQ ID NO: 824 | AAUAUCU |
| SEQ ID NO: 825 | AAUAUGA |
| SEQ ID NO: 826 | AAUAUGC |
| SEQ ID NO: 827 | AAUAUGG |
| SEQ ID NO: 828 | AAUAUGU |
| SEQ ID NO: 829 | AAUAUUA |
| SEQ ID NO: 830 | AAUAUUC |
| SEQ ID NO: 831 | AAUAUUG |
| SEQ ID NO: 832 | AAUAUUU |
| SEQ ID NO: 833 | AAUCAAA |
| SEQ ID NO: 834 | AAUCAAC |
| SEQ ID NO: 835 | AAUCAAG |
| SEQ ID NO: 836 | AAUCAAU |
| SEQ ID NO: 837 | AAUCACA |
| SEQ ID NO: 838 | AAUCACC |
| SEQ ID NO: 839 | AAUCACG |
| SEQ ID NO: 840 | AAUCACU |
| SEQ ID NO: 841 | AAUCAGA |
| SEQ ID NO: 842 | AAUCAGC |
| SEQ ID NO: 843 | AAUCAGG |
| SEQ ID NO: 844 | AAUCAGU |
| SEQ ID NO: 845 | AAUCAUA |
| SEQ ID NO: 846 | AAUCAUC |
| SEQ ID NO: 847 | AAUCAUG |
| SEQ ID NO: 848 | AAUCAUU |
| SEQ ID NO: 849 | AAUCCAA |
| SEQ ID NO: 850 | AAUCCAC |
| SEQ ID NO: 851 | AAUCCAG |
| SEQ ID NO: 852 | AAUCCAU |
| SEQ ID NO: 853 | AAUCCCA |
| SEQ ID NO: 854 | AAUCCCC |
| SEQ ID NO: 855 | AAUCCCG |
| SEQ ID NO: 856 | AAUCCCU |
| SEQ ID NO: 857 | AAUCCGA |
| SEQ ID NO: 858 | AAUCCGC |
| SEQ ID NO: 859 | AAUCCGG |
| SEQ ID NO: 860 | AAUCCGU |
| SEQ ID NO: 861 | AAUCCUA |
| SEQ ID NO: 862 | AAUCCUC |
| SEQ ID NO: 863 | AAUCCUG |
| SEQ ID NO: 864 | AAUCCUU |
| SEQ ID NO: 865 | AAUCGAA |
| SEQ ID NO: 866 | AAUCGAC |
| SEQ ID NO: 867 | AAUCGAG |
| SEQ ID NO: 868 | AAUCGAU |
| SEQ ID NO: 869 | AAUCGCA |
| SEQ ID NO: 870 | AAUCGCC |
| SEQ ID NO: 871 | AAUCGCG |
| SEQ ID NO: 872 | AAUCGCU |
| SEQ ID NO: 873 | AAUCGGA |
| SEQ ID NO: 874 | AAUCGGC |
| SEQ ID NO: 875 | AAUCGGG |
| SEQ ID NO: 876 | AAUCGGU |
| SEQ ID NO: 877 | AAUCGUA |
| SEQ ID NO: 878 | AAUCGUC |
| SEQ ID NO: 879 | AAUCGUG |
| SEQ ID NO: 880 | AAUCGUU |
| SEQ ID NO: 881 | AAUCUAA |
| SEQ ID NO: 882 | AAUCUAC |
| SEQ ID NO: 883 | AAUCUAG |
| SEQ ID NO: 884 | AAUCUAU |
| SEQ ID NO: 885 | AAUCUCA |
| SEQ ID NO: 886 | AAUCUCC |
| SEQ ID NO: 887 | AAUCUCG |
| SEQ ID NO: 888 | AAUCUCU |
| SEQ ID NO: 889 | AAUCUGA |
| SEQ ID NO: 890 | AAUCUGC |
| SEQ ID NO: 891 | AAUCUGG |
| SEQ ID NO: 892 | AAUCUGU |
| SEQ ID NO: 893 | AAUCUUA |
| SEQ ID NO: 894 | AAUCUUC |
| SEQ ID NO: 895 | AAUCUUG |
| SEQ ID NO: 896 | AAUCUUU |
| SEQ ID NO: 897 | AAUGAAA |
| SEQ ID NO: 898 | AAUGAAC |
| SEQ ID NO: 899 | AAUGAAG |
| SEQ ID NO: 900 | AAUGAAU |
| SEQ ID NO: 901 | AAUGACA |
| SEQ ID NO: 902 | AAUGACC |
| SEQ ID NO: 903 | AAUGACG |
| SEQ ID NO: 904 | AAUGACU |
| SEQ ID NO: 905 | AAUGAGA |
| SEQ ID NO: 906 | AAUGAGC |
| SEQ ID NO: 907 | AAUGAGG |
| SEQ ID NO: 908 | AAUGAGU |
| SEQ ID NO: 909 | AAUGAUA |
| SEQ ID NO: 910 | AAUGAUC |
| SEQ ID NO: 911 | AAUGAUG |
| SEQ ID NO: 912 | AAUGAUU |
| SEQ ID NO: 913 | AAUGCAA |
| SEQ ID NO: 914 | AAUGCAC |
| SEQ ID NO: 915 | AAUGCAG |
| SEQ ID NO: 916 | AAUGCAU |
| SEQ ID NO: 917 | AAUGCCA |
| SEQ ID NO: 918 | AAUGCCC |

Table 4

| | | | | | | | | | | | |
|---|---|---|---|---|---|---|---|---|---|---|---|
| SEQ ID NO: 919 | AAUGCCG | SEQ ID NO: 970 | AAUUAGC | SEQ ID NO: 1021 | AAUUUUA | SEQ ID NO: 1072 | ACAAGUU | SEQ ID NO: 1123 | ACACGAG | SEQ ID NO: 1174 | ACAGCCC |
| SEQ ID NO: 920 | AAUGCCU | SEQ ID NO: 971 | AAUUAGG | SEQ ID NO: 1022 | AAUUUUC | SEQ ID NO: 1073 | ACAAUAA | SEQ ID NO: 1124 | ACACGAU | SEQ ID NO: 1175 | ACAGCCG |
| SEQ ID NO: 921 | AAUGCGA | SEQ ID NO: 972 | AAUUAGU | SEQ ID NO: 1023 | AAUUUUG | SEQ ID NO: 1074 | ACAAUAC | SEQ ID NO: 1125 | ACACGCA | SEQ ID NO: 1176 | ACAGCCU |
| SEQ ID NO: 922 | AAUGCGC | SEQ ID NO: 973 | AAUUAUA | SEQ ID NO: 1024 | AAUUUUU | SEQ ID NO: 1075 | ACAAUAG | SEQ ID NO: 1126 | ACACGCC | SEQ ID NO: 1177 | ACAGCGA |
| SEQ ID NO: 923 | AAUGCGG | SEQ ID NO: 974 | AAUUAUC | SEQ ID NO: 1025 | ACAAAAA | SEQ ID NO: 1076 | ACAAUAU | SEQ ID NO: 1127 | ACACGCG | SEQ ID NO: 1178 | ACAGCGC |
| SEQ ID NO: 924 | AAUGCGU | SEQ ID NO: 975 | AAUUAUG | SEQ ID NO: 1026 | ACAAAAC | SEQ ID NO: 1077 | ACAAUCA | SEQ ID NO: 1128 | ACACGCU | SEQ ID NO: 1179 | ACAGCGG |
| SEQ ID NO: 925 | AAUGCUA | SEQ ID NO: 976 | AAUUAUU | SEQ ID NO: 1027 | ACAAAAG | SEQ ID NO: 1078 | ACAAUCC | SEQ ID NO: 1129 | ACACGGA | SEQ ID NO: 1180 | ACAGCGU |
| SEQ ID NO: 926 | AAUGCUC | SEQ ID NO: 977 | AAUUCAA | SEQ ID NO: 1028 | ACAAAAU | SEQ ID NO: 1079 | ACAAUCG | SEQ ID NO: 1130 | ACACGGC | SEQ ID NO: 1181 | ACAGCUA |
| SEQ ID NO: 927 | AAUGCUG | SEQ ID NO: 978 | AAUUCAC | SEQ ID NO: 1029 | ACAAACA | SEQ ID NO: 1080 | ACAAUCU | SEQ ID NO: 1131 | ACACGGG | SEQ ID NO: 1182 | ACAGCUC |
| SEQ ID NO: 928 | AAUGCUU | SEQ ID NO: 979 | AAUUCAG | SEQ ID NO: 1030 | ACAAACC | SEQ ID NO: 1081 | ACAAUGA | SEQ ID NO: 1132 | ACACGGU | SEQ ID NO: 1183 | ACAGCUG |
| SEQ ID NO: 929 | AAUGGAA | SEQ ID NO: 980 | AAUUCAU | SEQ ID NO: 1031 | ACAAACG | SEQ ID NO: 1082 | ACAAUGC | SEQ ID NO: 1133 | ACACGUA | SEQ ID NO: 1184 | ACAGCUU |
| SEQ ID NO: 930 | AAUGGAC | SEQ ID NO: 981 | AAUUCCA | SEQ ID NO: 1032 | ACAAACU | SEQ ID NO: 1083 | ACAAUGG | SEQ ID NO: 1134 | ACACGUC | SEQ ID NO: 1185 | ACAGGAA |
| SEQ ID NO: 931 | AAUGGAG | SEQ ID NO: 982 | AAUUCCC | SEQ ID NO: 1033 | ACAAAGA | SEQ ID NO: 1084 | ACAAUGU | SEQ ID NO: 1135 | ACACGUG | SEQ ID NO: 1186 | ACAGGAC |
| SEQ ID NO: 932 | AAUGGAU | SEQ ID NO: 983 | AAUUCCG | SEQ ID NO: 1034 | ACAAAGC | SEQ ID NO: 1085 | ACAAUUA | SEQ ID NO: 1136 | ACACGUU | SEQ ID NO: 1187 | ACAGGAG |
| SEQ ID NO: 933 | AAUGGCA | SEQ ID NO: 984 | AAUUCCU | SEQ ID NO: 1035 | ACAAAGG | SEQ ID NO: 1086 | ACAAUUC | SEQ ID NO: 1137 | ACACUAA | SEQ ID NO: 1188 | ACAGGAU |
| SEQ ID NO: 934 | AAUGGCC | SEQ ID NO: 985 | AAUUCGA | SEQ ID NO: 1036 | ACAAAGU | SEQ ID NO: 1087 | ACAAUUG | SEQ ID NO: 1138 | ACACUAC | SEQ ID NO: 1189 | ACAGGCA |
| SEQ ID NO: 935 | AAUGGCG | SEQ ID NO: 986 | AAUUCGC | SEQ ID NO: 1037 | ACAAAUA | SEQ ID NO: 1088 | ACAAUUU | SEQ ID NO: 1139 | ACACUAG | SEQ ID NO: 1190 | ACAGGCC |
| SEQ ID NO: 936 | AAUGGCU | SEQ ID NO: 987 | AAUUCGG | SEQ ID NO: 1038 | ACAAAUC | SEQ ID NO: 1089 | ACACAAA | SEQ ID NO: 1140 | ACACUAU | SEQ ID NO: 1191 | ACAGGCG |
| SEQ ID NO: 937 | AAUGGGA | SEQ ID NO: 988 | AAUUCGU | SEQ ID NO: 1039 | ACAAAUG | SEQ ID NO: 1090 | ACACAAC | SEQ ID NO: 1141 | ACACUCA | SEQ ID NO: 1192 | ACAGGCU |
| SEQ ID NO: 938 | AAUGGGC | SEQ ID NO: 989 | AAUUCUA | SEQ ID NO: 1040 | ACAAAUU | SEQ ID NO: 1091 | ACACAAG | SEQ ID NO: 1142 | ACACUCC | SEQ ID NO: 1193 | ACAGGGA |
| SEQ ID NO: 939 | AAUGGGG | SEQ ID NO: 990 | AAUUCUC | SEQ ID NO: 1041 | ACAACAA | SEQ ID NO: 1092 | ACACAAU | SEQ ID NO: 1143 | ACACUCG | SEQ ID NO: 1194 | ACAGGGC |
| SEQ ID NO: 940 | AAUGGGU | SEQ ID NO: 991 | AAUUCUG | SEQ ID NO: 1042 | ACAACAC | SEQ ID NO: 1093 | ACACACA | SEQ ID NO: 1144 | ACACUCU | SEQ ID NO: 1195 | ACAGGGG |
| SEQ ID NO: 941 | AAUGGUA | SEQ ID NO: 992 | AAUUCUU | SEQ ID NO: 1043 | ACAACAG | SEQ ID NO: 1094 | ACACACC | SEQ ID NO: 1145 | ACACUGA | SEQ ID NO: 1196 | ACAGGGU |
| SEQ ID NO: 942 | AAUGGUC | SEQ ID NO: 993 | AAUUGAA | SEQ ID NO: 1044 | ACAACAU | SEQ ID NO: 1095 | ACACACG | SEQ ID NO: 1146 | ACACUGC | SEQ ID NO: 1197 | ACAGGUA |
| SEQ ID NO: 943 | AAUGGUG | SEQ ID NO: 994 | AAUUGAC | SEQ ID NO: 1045 | ACAACCA | SEQ ID NO: 1096 | ACACACU | SEQ ID NO: 1147 | ACACUGG | SEQ ID NO: 1198 | ACAGGUC |
| SEQ ID NO: 944 | AAUGGUU | SEQ ID NO: 995 | AAUUGAG | SEQ ID NO: 1046 | ACAACCC | SEQ ID NO: 1097 | ACACAGA | SEQ ID NO: 1148 | ACACUGU | SEQ ID NO: 1199 | ACAGGUG |
| SEQ ID NO: 945 | AAUGUAA | SEQ ID NO: 996 | AAUUGAU | SEQ ID NO: 1047 | ACAACCG | SEQ ID NO: 1098 | ACACAGC | SEQ ID NO: 1149 | ACACUUA | SEQ ID NO: 1200 | ACAGGUU |
| SEQ ID NO: 946 | AAUGUAC | SEQ ID NO: 997 | AAUUGCA | SEQ ID NO: 1048 | ACAACCU | SEQ ID NO: 1099 | ACACAGG | SEQ ID NO: 1150 | ACACUUC | SEQ ID NO: 1201 | ACAGUAA |
| SEQ ID NO: 947 | AAUGUAG | SEQ ID NO: 998 | AAUUGCC | SEQ ID NO: 1049 | ACAACGA | SEQ ID NO: 1100 | ACACAGU | SEQ ID NO: 1151 | ACACUUG | SEQ ID NO: 1202 | ACAGUAC |
| SEQ ID NO: 948 | AAUGUAU | SEQ ID NO: 999 | AAUUGCG | SEQ ID NO: 1050 | ACAACGC | SEQ ID NO: 1101 | ACACAUA | SEQ ID NO: 1152 | ACACUUU | SEQ ID NO: 1203 | ACAGUAG |
| SEQ ID NO: 949 | AAUGUCA | SEQ ID NO: 1000 | AAUUGCU | SEQ ID NO: 1051 | ACAACGG | SEQ ID NO: 1102 | ACACAUC | SEQ ID NO: 1153 | ACAGAAA | SEQ ID NO: 1204 | ACAGUAU |
| SEQ ID NO: 950 | AAUGUCC | SEQ ID NO: 1001 | AAUUGGA | SEQ ID NO: 1052 | ACAACGU | SEQ ID NO: 1103 | ACACAUG | SEQ ID NO: 1154 | ACAGAAC | SEQ ID NO: 1205 | ACAGUCA |
| SEQ ID NO: 951 | AAUGUCG | SEQ ID NO: 1002 | AAUUGGC | SEQ ID NO: 1053 | ACAACUA | SEQ ID NO: 1104 | ACACAUU | SEQ ID NO: 1155 | ACAGAAG | SEQ ID NO: 1206 | ACAGUCC |
| SEQ ID NO: 952 | AAUGUCU | SEQ ID NO: 1003 | AAUUGGG | SEQ ID NO: 1054 | ACAACUC | SEQ ID NO: 1105 | ACACCAA | SEQ ID NO: 1156 | ACAGAAU | SEQ ID NO: 1207 | ACAGUCG |
| SEQ ID NO: 953 | AAUGUGA | SEQ ID NO: 1004 | AAUUGGU | SEQ ID NO: 1055 | ACAACUG | SEQ ID NO: 1106 | ACACCAC | SEQ ID NO: 1157 | ACAGACA | SEQ ID NO: 1208 | ACAGUCU |
| SEQ ID NO: 954 | AAUGUGC | SEQ ID NO: 1005 | AAUUGUA | SEQ ID NO: 1056 | ACAACUU | SEQ ID NO: 1107 | ACACCAG | SEQ ID NO: 1158 | ACAGACC | SEQ ID NO: 1209 | ACAGUGA |
| SEQ ID NO: 955 | AAUGUGG | SEQ ID NO: 1006 | AAUUGUC | SEQ ID NO: 1057 | ACAAGAA | SEQ ID NO: 1108 | ACACCAU | SEQ ID NO: 1159 | ACAGACG | SEQ ID NO: 1210 | ACAGUGC |
| SEQ ID NO: 956 | AAUGUGU | SEQ ID NO: 1007 | AAUUGUG | SEQ ID NO: 1058 | ACAAGAC | SEQ ID NO: 1109 | ACACCCA | SEQ ID NO: 1160 | ACAGACU | SEQ ID NO: 1211 | ACAGUGG |
| SEQ ID NO: 957 | AAUGUUA | SEQ ID NO: 1008 | AAUUGUU | SEQ ID NO: 1059 | ACAAGAG | SEQ ID NO: 1110 | ACACCCC | SEQ ID NO: 1161 | ACAGAGA | SEQ ID NO: 1212 | ACAGUGU |
| SEQ ID NO: 958 | AAUGUUC | SEQ ID NO: 1009 | AAUUUAA | SEQ ID NO: 1060 | ACAAGAU | SEQ ID NO: 1111 | ACACCCG | SEQ ID NO: 1162 | ACAGAGC | SEQ ID NO: 1213 | ACAGUUA |
| SEQ ID NO: 959 | AAUGUUG | SEQ ID NO: 1010 | AAUUUAC | SEQ ID NO: 1061 | ACAAGCA | SEQ ID NO: 1112 | ACACCCU | SEQ ID NO: 1163 | ACAGAGG | SEQ ID NO: 1214 | ACAGUUC |
| SEQ ID NO: 960 | AAUGUUU | SEQ ID NO: 1011 | AAUUUAG | SEQ ID NO: 1062 | ACAAGCC | SEQ ID NO: 1113 | ACACCGA | SEQ ID NO: 1164 | ACAGAGU | SEQ ID NO: 1215 | ACAGUUG |
| SEQ ID NO: 961 | AAUUAAA | SEQ ID NO: 1012 | AAUUUAU | SEQ ID NO: 1063 | ACAAGCG | SEQ ID NO: 1114 | ACACCGC | SEQ ID NO: 1165 | ACAGAUA | SEQ ID NO: 1216 | ACAGUUU |
| SEQ ID NO: 962 | AAUUAAC | SEQ ID NO: 1013 | AAUUUCA | SEQ ID NO: 1064 | ACAAGCU | SEQ ID NO: 1115 | ACACCGG | SEQ ID NO: 1166 | ACAGAUC | SEQ ID NO: 1217 | ACAUAAA |
| SEQ ID NO: 963 | AAUUAAG | SEQ ID NO: 1014 | AAUUUCC | SEQ ID NO: 1065 | ACAAGGA | SEQ ID NO: 1116 | ACACCGU | SEQ ID NO: 1167 | ACAGAUG | SEQ ID NO: 1218 | ACAUAAC |
| SEQ ID NO: 964 | AAUUAAU | SEQ ID NO: 1015 | AAUUUCG | SEQ ID NO: 1066 | ACAAGGC | SEQ ID NO: 1117 | ACACCUA | SEQ ID NO: 1168 | ACAGAUU | SEQ ID NO: 1219 | ACAUAAG |
| SEQ ID NO: 965 | AAUUACA | SEQ ID NO: 1016 | AAUUUCU | SEQ ID NO: 1067 | ACAAGGG | SEQ ID NO: 1118 | ACACCUC | SEQ ID NO: 1169 | ACAGCAA | SEQ ID NO: 1220 | ACAUAAU |
| SEQ ID NO: 966 | AAUUACC | SEQ ID NO: 1017 | AAUUUGA | SEQ ID NO: 1068 | ACAAGGU | SEQ ID NO: 1119 | ACACCUG | SEQ ID NO: 1170 | ACAGCAC | SEQ ID NO: 1221 | ACAUACA |
| SEQ ID NO: 967 | AAUUACG | SEQ ID NO: 1018 | AAUUUGC | SEQ ID NO: 1069 | ACAAGUA | SEQ ID NO: 1120 | ACACCUU | SEQ ID NO: 1171 | ACAGCAG | SEQ ID NO: 1222 | ACAUACC |
| SEQ ID NO: 968 | AAUUACU | SEQ ID NO: 1019 | AAUUUGG | SEQ ID NO: 1070 | ACAAGUC | SEQ ID NO: 1121 | ACACGAA | SEQ ID NO: 1172 | ACAGCAU | SEQ ID NO: 1223 | ACAUACG |
| SEQ ID NO: 969 | AAUUAGA | SEQ ID NO: 1020 | AAUUUGU | SEQ ID NO: 1071 | ACAAGUG | SEQ ID NO: 1122 | ACACGAC | SEQ ID NO: 1173 | ACAGCCA | SEQ ID NO: 1224 | ACAUACU |

Table 5

| | |
|---|---|
| SEQ ID NO: 1225 | ACAUAGA |
| SEQ ID NO: 1226 | ACAUAGC |
| SEQ ID NO: 1227 | ACAUAGG |
| SEQ ID NO: 1228 | ACAUAGU |
| SEQ ID NO: 1229 | ACAUAUA |
| SEQ ID NO: 1230 | ACAUAUC |
| SEQ ID NO: 1231 | ACAUAUG |
| SEQ ID NO: 1232 | ACAUAUU |
| SEQ ID NO: 1233 | ACAUCAA |
| SEQ ID NO: 1234 | ACAUCAC |
| SEQ ID NO: 1235 | ACAUCAG |
| SEQ ID NO: 1236 | ACAUCAU |
| SEQ ID NO: 1237 | ACAUCCA |
| SEQ ID NO: 1238 | ACAUCCC |
| SEQ ID NO: 1239 | ACAUCCG |
| SEQ ID NO: 1240 | ACAUCCU |
| SEQ ID NO: 1241 | ACAUCGA |
| SEQ ID NO: 1242 | ACAUCGC |
| SEQ ID NO: 1243 | ACAUCGG |
| SEQ ID NO: 1244 | ACAUCGU |
| SEQ ID NO: 1245 | ACAUCUA |
| SEQ ID NO: 1246 | ACAUCUC |
| SEQ ID NO: 1247 | ACAUCUG |
| SEQ ID NO: 1248 | ACAUCUU |
| SEQ ID NO: 1249 | ACAUGAA |
| SEQ ID NO: 1250 | ACAUGAC |
| SEQ ID NO: 1251 | ACAUGAG |
| SEQ ID NO: 1252 | ACAUGAU |
| SEQ ID NO: 1253 | ACAUGCA |
| SEQ ID NO: 1254 | ACAUGCC |
| SEQ ID NO: 1255 | ACAUGCG |
| SEQ ID NO: 1256 | ACAUGCU |
| SEQ ID NO: 1257 | ACAUGGA |
| SEQ ID NO: 1258 | ACAUGGC |
| SEQ ID NO: 1259 | ACAUGGG |
| SEQ ID NO: 1260 | ACAUGGU |
| SEQ ID NO: 1261 | ACAUGUA |
| SEQ ID NO: 1262 | ACAUGUC |
| SEQ ID NO: 1263 | ACAUGUG |
| SEQ ID NO: 1264 | ACAUGUU |
| SEQ ID NO: 1265 | ACAUUAA |
| SEQ ID NO: 1266 | ACAUUAC |
| SEQ ID NO: 1267 | ACAUUAG |
| SEQ ID NO: 1268 | ACAUUAU |
| SEQ ID NO: 1269 | ACAUUCA |
| SEQ ID NO: 1270 | ACAUUCC |
| SEQ ID NO: 1271 | ACAUUCG |
| SEQ ID NO: 1272 | ACAUUCU |
| SEQ ID NO: 1273 | ACAUUGA |
| SEQ ID NO: 1274 | ACAUUGC |
| SEQ ID NO: 1275 | ACAUUGG |
| SEQ ID NO: 1276 | ACAUUGU |
| SEQ ID NO: 1277 | ACAUUUA |
| SEQ ID NO: 1278 | ACAUUUC |
| SEQ ID NO: 1279 | ACAUUUG |
| SEQ ID NO: 1280 | ACAUUUU |
| SEQ ID NO: 1281 | ACCAAAA |
| SEQ ID NO: 1282 | ACCAAAC |
| SEQ ID NO: 1283 | ACCAAAG |
| SEQ ID NO: 1284 | ACCAAAU |
| SEQ ID NO: 1285 | ACCAACA |
| SEQ ID NO: 1286 | ACCAACC |
| SEQ ID NO: 1287 | ACCAACG |
| SEQ ID NO: 1288 | ACCAACU |
| SEQ ID NO: 1289 | ACCAAGA |
| SEQ ID NO: 1290 | ACCAAGC |
| SEQ ID NO: 1291 | ACCAAGG |
| SEQ ID NO: 1292 | ACCAAGU |
| SEQ ID NO: 1293 | ACCAAUA |
| SEQ ID NO: 1294 | ACCAAUC |
| SEQ ID NO: 1295 | ACCAAUG |
| SEQ ID NO: 1296 | ACCAAUU |
| SEQ ID NO: 1297 | ACCACAA |
| SEQ ID NO: 1298 | ACCACAC |
| SEQ ID NO: 1299 | ACCACAG |
| SEQ ID NO: 1300 | ACCACAU |
| SEQ ID NO: 1301 | ACCACCA |
| SEQ ID NO: 1302 | ACCACCC |
| SEQ ID NO: 1303 | ACCACCG |
| SEQ ID NO: 1304 | ACCACCU |
| SEQ ID NO: 1305 | ACCACGA |
| SEQ ID NO: 1306 | ACCACGC |
| SEQ ID NO: 1307 | ACCACGG |
| SEQ ID NO: 1308 | ACCACGU |
| SEQ ID NO: 1309 | ACCACUA |
| SEQ ID NO: 1310 | ACCACUC |
| SEQ ID NO: 1311 | ACCACUG |
| SEQ ID NO: 1312 | ACCACUU |
| SEQ ID NO: 1313 | ACCAGAA |
| SEQ ID NO: 1314 | ACCAGAC |
| SEQ ID NO: 1315 | ACCAGAG |
| SEQ ID NO: 1316 | ACCAGAU |
| SEQ ID NO: 1317 | ACCAGCA |
| SEQ ID NO: 1318 | ACCAGCC |
| SEQ ID NO: 1319 | ACCAGCG |
| SEQ ID NO: 1320 | ACCAGCU |
| SEQ ID NO: 1321 | ACCAGGA |
| SEQ ID NO: 1322 | ACCAGGC |
| SEQ ID NO: 1323 | ACCAGGG |
| SEQ ID NO: 1324 | ACCAGGU |
| SEQ ID NO: 1325 | ACCAGUA |
| SEQ ID NO: 1326 | ACCAGUC |
| SEQ ID NO: 1327 | ACCAGUG |
| SEQ ID NO: 1328 | ACCAGUU |
| SEQ ID NO: 1329 | ACCAUAA |
| SEQ ID NO: 1330 | ACCAUAC |
| SEQ ID NO: 1331 | ACCAUAG |
| SEQ ID NO: 1332 | ACCAUAU |
| SEQ ID NO: 1333 | ACCAUCA |
| SEQ ID NO: 1334 | ACCAUCC |
| SEQ ID NO: 1335 | ACCAUCG |
| SEQ ID NO: 1336 | ACCAUCU |
| SEQ ID NO: 1337 | ACCAUGA |
| SEQ ID NO: 1338 | ACCAUGC |
| SEQ ID NO: 1339 | ACCAUGG |
| SEQ ID NO: 1340 | ACCAUGU |
| SEQ ID NO: 1341 | ACCAUUA |
| SEQ ID NO: 1342 | ACCAUUC |
| SEQ ID NO: 1343 | ACCAUUG |
| SEQ ID NO: 1344 | ACCAUUU |
| SEQ ID NO: 1345 | ACCCAAA |
| SEQ ID NO: 1346 | ACCCAAC |
| SEQ ID NO: 1347 | ACCCAAG |
| SEQ ID NO: 1348 | ACCCAAU |
| SEQ ID NO: 1349 | ACCCACA |
| SEQ ID NO: 1350 | ACCCACC |
| SEQ ID NO: 1351 | ACCCACG |
| SEQ ID NO: 1352 | ACCCACU |
| SEQ ID NO: 1353 | ACCCAGA |
| SEQ ID NO: 1354 | ACCCAGC |
| SEQ ID NO: 1355 | ACCCAGG |
| SEQ ID NO: 1356 | ACCCAGU |
| SEQ ID NO: 1357 | ACCCAUA |
| SEQ ID NO: 1358 | ACCCAUC |
| SEQ ID NO: 1359 | ACCCAUG |
| SEQ ID NO: 1360 | ACCCAUU |
| SEQ ID NO: 1361 | ACCCCAA |
| SEQ ID NO: 1362 | ACCCCAC |
| SEQ ID NO: 1363 | ACCCCAG |
| SEQ ID NO: 1364 | ACCCCAU |
| SEQ ID NO: 1365 | ACCCCCA |
| SEQ ID NO: 1366 | ACCCCCC |
| SEQ ID NO: 1367 | ACCCCCG |
| SEQ ID NO: 1368 | ACCCCCU |
| SEQ ID NO: 1369 | ACCCCGA |
| SEQ ID NO: 1370 | ACCCCGC |
| SEQ ID NO: 1371 | ACCCCGG |
| SEQ ID NO: 1372 | ACCCCGU |
| SEQ ID NO: 1373 | ACCCCUA |
| SEQ ID NO: 1374 | ACCCCUC |
| SEQ ID NO: 1375 | ACCCCUG |
| SEQ ID NO: 1376 | ACCCCUU |
| SEQ ID NO: 1377 | ACCCGAA |
| SEQ ID NO: 1378 | ACCCGAC |
| SEQ ID NO: 1379 | ACCCGAG |
| SEQ ID NO: 1380 | ACCCGAU |
| SEQ ID NO: 1381 | ACCCGCA |
| SEQ ID NO: 1382 | ACCCGCC |
| SEQ ID NO: 1383 | ACCCGCG |
| SEQ ID NO: 1384 | ACCCGCU |
| SEQ ID NO: 1385 | ACCCGGA |
| SEQ ID NO: 1386 | ACCCGGC |
| SEQ ID NO: 1387 | ACCCGGG |
| SEQ ID NO: 1388 | ACCCGGU |
| SEQ ID NO: 1389 | ACCCGUA |
| SEQ ID NO: 1390 | ACCCGUC |
| SEQ ID NO: 1391 | ACCCGUG |
| SEQ ID NO: 1392 | ACCCGUU |
| SEQ ID NO: 1393 | ACCCUAA |
| SEQ ID NO: 1394 | ACCCUAC |
| SEQ ID NO: 1395 | ACCCUAG |
| SEQ ID NO: 1396 | ACCCUAU |
| SEQ ID NO: 1397 | ACCCUCA |
| SEQ ID NO: 1398 | ACCCUCC |
| SEQ ID NO: 1399 | ACCCUCG |
| SEQ ID NO: 1400 | ACCCUCU |
| SEQ ID NO: 1401 | ACCCUGA |
| SEQ ID NO: 1402 | ACCCUGC |
| SEQ ID NO: 1403 | ACCCUGG |
| SEQ ID NO: 1404 | ACCCUGU |
| SEQ ID NO: 1405 | ACCCUUA |
| SEQ ID NO: 1406 | ACCCUUC |
| SEQ ID NO: 1407 | ACCCUUG |
| SEQ ID NO: 1408 | ACCCUUU |
| SEQ ID NO: 1409 | ACCGAAA |
| SEQ ID NO: 1410 | ACCGAAC |
| SEQ ID NO: 1411 | ACCGAAG |
| SEQ ID NO: 1412 | ACCGAAU |
| SEQ ID NO: 1413 | ACCGACA |
| SEQ ID NO: 1414 | ACCGACC |
| SEQ ID NO: 1415 | ACCGACG |
| SEQ ID NO: 1416 | ACCGACU |
| SEQ ID NO: 1417 | ACCGAGA |
| SEQ ID NO: 1418 | ACCGAGC |
| SEQ ID NO: 1419 | ACCGAGG |
| SEQ ID NO: 1420 | ACCGAGU |
| SEQ ID NO: 1421 | ACCGAUA |
| SEQ ID NO: 1422 | ACCGAUC |
| SEQ ID NO: 1423 | ACCGAUG |
| SEQ ID NO: 1424 | ACCGAUU |
| SEQ ID NO: 1425 | ACCGCAA |
| SEQ ID NO: 1426 | ACCGCAC |
| SEQ ID NO: 1427 | ACCGCAG |
| SEQ ID NO: 1428 | ACCGCAU |
| SEQ ID NO: 1429 | ACCGCCA |
| SEQ ID NO: 1430 | ACCGCCC |
| SEQ ID NO: 1431 | ACCGCCG |
| SEQ ID NO: 1432 | ACCGCCU |
| SEQ ID NO: 1433 | ACCGCGA |
| SEQ ID NO: 1434 | ACCGCGC |
| SEQ ID NO: 1435 | ACCGCGG |
| SEQ ID NO: 1436 | ACCGCGU |
| SEQ ID NO: 1437 | ACCGCUA |
| SEQ ID NO: 1438 | ACCGCUC |
| SEQ ID NO: 1439 | ACCGCUG |
| SEQ ID NO: 1440 | ACCGCUU |
| SEQ ID NO: 1441 | ACCGGAA |
| SEQ ID NO: 1442 | ACCGGAC |
| SEQ ID NO: 1443 | ACCGGAG |
| SEQ ID NO: 1444 | ACCGGAU |
| SEQ ID NO: 1445 | ACCGGCA |
| SEQ ID NO: 1446 | ACCGGCC |
| SEQ ID NO: 1447 | ACCGGCG |
| SEQ ID NO: 1448 | ACCGGCU |
| SEQ ID NO: 1449 | ACCGGGA |
| SEQ ID NO: 1450 | ACCGGGC |
| SEQ ID NO: 1451 | ACCGGGG |
| SEQ ID NO: 1452 | ACCGGGU |
| SEQ ID NO: 1453 | ACCGGUA |
| SEQ ID NO: 1454 | ACCGGUC |
| SEQ ID NO: 1455 | ACCGGUG |
| SEQ ID NO: 1456 | ACCGGUU |
| SEQ ID NO: 1457 | ACCGUAA |
| SEQ ID NO: 1458 | ACCGUAC |
| SEQ ID NO: 1459 | ACCGUAG |
| SEQ ID NO: 1460 | ACCGUAU |
| SEQ ID NO: 1461 | ACCGUCA |
| SEQ ID NO: 1462 | ACCGUCC |
| SEQ ID NO: 1463 | ACCGUCG |
| SEQ ID NO: 1464 | ACCGUCU |
| SEQ ID NO: 1465 | ACCGUGA |
| SEQ ID NO: 1466 | ACCGUGC |
| SEQ ID NO: 1467 | ACCGUGG |
| SEQ ID NO: 1468 | ACCGUGU |
| SEQ ID NO: 1469 | ACCGUUA |
| SEQ ID NO: 1470 | ACCGUUC |
| SEQ ID NO: 1471 | ACCGUUG |
| SEQ ID NO: 1472 | ACCGUUU |
| SEQ ID NO: 1473 | ACCUAAA |
| SEQ ID NO: 1474 | ACCUAAC |
| SEQ ID NO: 1475 | ACCUAAG |
| SEQ ID NO: 1476 | ACCUAAU |
| SEQ ID NO: 1477 | ACCUACA |
| SEQ ID NO: 1478 | ACCUACC |
| SEQ ID NO: 1479 | ACCUACG |
| SEQ ID NO: 1480 | ACCUACU |
| SEQ ID NO: 1481 | ACCUAGA |
| SEQ ID NO: 1482 | ACCUAGC |
| SEQ ID NO: 1483 | ACCUAGG |
| SEQ ID NO: 1484 | ACCUAGU |
| SEQ ID NO: 1485 | ACCUAUA |
| SEQ ID NO: 1486 | ACCUAUC |
| SEQ ID NO: 1487 | ACCUAUG |
| SEQ ID NO: 1488 | ACCUAUU |
| SEQ ID NO: 1489 | ACCUCAA |
| SEQ ID NO: 1490 | ACCUCAC |
| SEQ ID NO: 1491 | ACCUCAG |
| SEQ ID NO: 1492 | ACCUCAU |
| SEQ ID NO: 1493 | ACCUCCA |
| SEQ ID NO: 1494 | ACCUCCC |
| SEQ ID NO: 1495 | ACCUCCG |
| SEQ ID NO: 1496 | ACCUCCU |
| SEQ ID NO: 1497 | ACCUCGA |
| SEQ ID NO: 1498 | ACCUCGC |
| SEQ ID NO: 1499 | ACCUCGG |
| SEQ ID NO: 1500 | ACCUCGU |
| SEQ ID NO: 1501 | ACCUCUA |
| SEQ ID NO: 1502 | ACCUCUC |
| SEQ ID NO: 1503 | ACCUCUG |
| SEQ ID NO: 1504 | ACCUCUU |
| SEQ ID NO: 1505 | ACCUGAA |
| SEQ ID NO: 1506 | ACCUGAC |
| SEQ ID NO: 1507 | ACCUGAG |
| SEQ ID NO: 1508 | ACCUGAU |
| SEQ ID NO: 1509 | ACCUGCA |
| SEQ ID NO: 1510 | ACCUGCC |
| SEQ ID NO: 1511 | ACCUGCG |
| SEQ ID NO: 1512 | ACCUGCU |
| SEQ ID NO: 1513 | ACCUGGA |
| SEQ ID NO: 1514 | ACCUGGC |
| SEQ ID NO: 1515 | ACCUGGG |
| SEQ ID NO: 1516 | ACCUGGU |
| SEQ ID NO: 1517 | ACCUGUA |
| SEQ ID NO: 1518 | ACCUGUC |
| SEQ ID NO: 1519 | ACCUGUG |
| SEQ ID NO: 1520 | ACCUGUU |
| SEQ ID NO: 1521 | ACCUUAA |
| SEQ ID NO: 1522 | ACCUUAC |
| SEQ ID NO: 1523 | ACCUUAG |
| SEQ ID NO: 1524 | ACCUUAU |
| SEQ ID NO: 1525 | ACCUUCA |
| SEQ ID NO: 1526 | ACCUUCC |
| SEQ ID NO: 1527 | ACCUUCG |
| SEQ ID NO: 1528 | ACCUUCU |
| SEQ ID NO: 1529 | ACCUUGA |
| SEQ ID NO: 1530 | ACCUUGC |

Table 6

| | | | | | | | | | | | |
|---|---|---|---|---|---|---|---|---|---|---|---|
| SEQ ID NO: 1531 | ACCUUGG | SEQ ID NO: 1582 | ACGAGUC | SEQ ID NO: 1633 | ACGCGAA | SEQ ID NO: 1684 | ACGGCAU | SEQ ID NO: 1735 | ACGUACG | SEQ ID NO: 1786 | ACGUUGC |
| SEQ ID NO: 1532 | ACCUUGU | SEQ ID NO: 1583 | ACGAGUG | SEQ ID NO: 1634 | ACGCGAC | SEQ ID NO: 1685 | ACGGCCA | SEQ ID NO: 1736 | ACGUACU | SEQ ID NO: 1787 | ACGUUGG |
| SEQ ID NO: 1533 | ACCUUUA | SEQ ID NO: 1584 | ACGAGUU | SEQ ID NO: 1635 | ACGCGAG | SEQ ID NO: 1686 | ACGGCCC | SEQ ID NO: 1737 | ACGUAGA | SEQ ID NO: 1788 | ACGUUGU |
| SEQ ID NO: 1534 | ACCUUUC | SEQ ID NO: 1585 | ACGAUAA | SEQ ID NO: 1636 | ACGCGAU | SEQ ID NO: 1687 | ACGGCCG | SEQ ID NO: 1738 | ACGUAGC | SEQ ID NO: 1789 | ACGUUUA |
| SEQ ID NO: 1535 | ACCUUUG | SEQ ID NO: 1586 | ACGAUAC | SEQ ID NO: 1637 | ACGCGCA | SEQ ID NO: 1688 | ACGGCCU | SEQ ID NO: 1739 | ACGUAGG | SEQ ID NO: 1790 | ACGUUUC |
| SEQ ID NO: 1536 | ACCUUUU | SEQ ID NO: 1587 | ACGAUAG | SEQ ID NO: 1638 | ACGCGCC | SEQ ID NO: 1689 | ACGGCGA | SEQ ID NO: 1740 | ACGUAGU | SEQ ID NO: 1791 | ACGUUUG |
| SEQ ID NO: 1537 | ACGAAAA | SEQ ID NO: 1588 | ACGAUAU | SEQ ID NO: 1639 | ACGCGCG | SEQ ID NO: 1690 | ACGGCGC | SEQ ID NO: 1741 | ACGUAUA | SEQ ID NO: 1792 | ACGUUUU |
| SEQ ID NO: 1538 | ACGAAAC | SEQ ID NO: 1589 | ACGAUCA | SEQ ID NO: 1640 | ACGCGCU | SEQ ID NO: 1691 | ACGGCGG | SEQ ID NO: 1742 | ACGUAUC | SEQ ID NO: 1793 | ACUAAAA |
| SEQ ID NO: 1539 | ACGAAAG | SEQ ID NO: 1590 | ACGAUCC | SEQ ID NO: 1641 | ACGCGGA | SEQ ID NO: 1692 | ACGGCGU | SEQ ID NO: 1743 | ACGUAUG | SEQ ID NO: 1794 | ACUAAAC |
| SEQ ID NO: 1540 | ACGAAAU | SEQ ID NO: 1591 | ACGAUCG | SEQ ID NO: 1642 | ACGCGGC | SEQ ID NO: 1693 | ACGGCUA | SEQ ID NO: 1744 | ACGUAUU | SEQ ID NO: 1795 | ACUAAAG |
| SEQ ID NO: 1541 | ACGAACA | SEQ ID NO: 1592 | ACGAUCU | SEQ ID NO: 1643 | ACGCGGG | SEQ ID NO: 1694 | ACGGCUC | SEQ ID NO: 1745 | ACGUCAA | SEQ ID NO: 1796 | ACUAAAU |
| SEQ ID NO: 1542 | ACGAACC | SEQ ID NO: 1593 | ACGAUGA | SEQ ID NO: 1644 | ACGCGGU | SEQ ID NO: 1695 | ACGGCUG | SEQ ID NO: 1746 | ACGUCAC | SEQ ID NO: 1797 | ACUAACA |
| SEQ ID NO: 1543 | ACGAACG | SEQ ID NO: 1594 | ACGAUGC | SEQ ID NO: 1645 | ACGCGUA | SEQ ID NO: 1696 | ACGGCUU | SEQ ID NO: 1747 | ACGUCAG | SEQ ID NO: 1798 | ACUAACC |
| SEQ ID NO: 1544 | ACGAACU | SEQ ID NO: 1595 | ACGAUGG | SEQ ID NO: 1646 | ACGCGUC | SEQ ID NO: 1697 | ACGGGAA | SEQ ID NO: 1748 | ACGUCAU | SEQ ID NO: 1799 | ACUAACG |
| SEQ ID NO: 1545 | ACGAAGA | SEQ ID NO: 1596 | ACGAUGU | SEQ ID NO: 1647 | ACGCGUG | SEQ ID NO: 1698 | ACGGGAC | SEQ ID NO: 1749 | ACGUCCA | SEQ ID NO: 1800 | ACUAACU |
| SEQ ID NO: 1546 | ACGAAGC | SEQ ID NO: 1597 | ACGAUUA | SEQ ID NO: 1648 | ACGCGUU | SEQ ID NO: 1699 | ACGGGAG | SEQ ID NO: 1750 | ACGUCCC | SEQ ID NO: 1801 | ACUAAGA |
| SEQ ID NO: 1547 | ACGAAGG | SEQ ID NO: 1598 | ACGAUUC | SEQ ID NO: 1649 | ACGCUAA | SEQ ID NO: 1700 | ACGGGAU | SEQ ID NO: 1751 | ACGUCCG | SEQ ID NO: 1802 | ACUAAGC |
| SEQ ID NO: 1548 | ACGAAGU | SEQ ID NO: 1599 | ACGAUUG | SEQ ID NO: 1650 | ACGCUAC | SEQ ID NO: 1701 | ACGGGCA | SEQ ID NO: 1752 | ACGUCCU | SEQ ID NO: 1803 | ACUAAGG |
| SEQ ID NO: 1549 | ACGAAUA | SEQ ID NO: 1600 | ACGAUUU | SEQ ID NO: 1651 | ACGCUAG | SEQ ID NO: 1702 | ACGGGCC | SEQ ID NO: 1753 | ACGUCGA | SEQ ID NO: 1804 | ACUAAGU |
| SEQ ID NO: 1550 | ACGAAUC | SEQ ID NO: 1601 | ACGCAAA | SEQ ID NO: 1652 | ACGCUAU | SEQ ID NO: 1703 | ACGGGCG | SEQ ID NO: 1754 | ACGUCGC | SEQ ID NO: 1805 | ACUAAUA |
| SEQ ID NO: 1551 | ACGAAUG | SEQ ID NO: 1602 | ACGCAAC | SEQ ID NO: 1653 | ACGCUCA | SEQ ID NO: 1704 | ACGGGCU | SEQ ID NO: 1755 | ACGUCGG | SEQ ID NO: 1806 | ACUAAUC |
| SEQ ID NO: 1552 | ACGAAUU | SEQ ID NO: 1603 | ACGCAAG | SEQ ID NO: 1654 | ACGCUCC | SEQ ID NO: 1705 | ACGGGGA | SEQ ID NO: 1756 | ACGUCGU | SEQ ID NO: 1807 | ACUAAUG |
| SEQ ID NO: 1553 | ACGACAA | SEQ ID NO: 1604 | ACGCAAU | SEQ ID NO: 1655 | ACGCUCG | SEQ ID NO: 1706 | ACGGGGC | SEQ ID NO: 1757 | ACGUCUA | SEQ ID NO: 1808 | ACUAAUU |
| SEQ ID NO: 1554 | ACGACAC | SEQ ID NO: 1605 | ACGCACA | SEQ ID NO: 1656 | ACGCUCU | SEQ ID NO: 1707 | ACGGGGG | SEQ ID NO: 1758 | ACGUCUC | SEQ ID NO: 1809 | ACUACAA |
| SEQ ID NO: 1555 | ACGACAG | SEQ ID NO: 1606 | ACGCACC | SEQ ID NO: 1657 | ACGCUGA | SEQ ID NO: 1708 | ACGGGGU | SEQ ID NO: 1759 | ACGUCUG | SEQ ID NO: 1810 | ACUACAC |
| SEQ ID NO: 1556 | ACGACAU | SEQ ID NO: 1607 | ACGCACG | SEQ ID NO: 1658 | ACGCUGC | SEQ ID NO: 1709 | ACGGGUA | SEQ ID NO: 1760 | ACGUCUU | SEQ ID NO: 1811 | ACUACAG |
| SEQ ID NO: 1557 | ACGACCA | SEQ ID NO: 1608 | ACGCACU | SEQ ID NO: 1659 | ACGCUGG | SEQ ID NO: 1710 | ACGGGUC | SEQ ID NO: 1761 | ACGUGAA | SEQ ID NO: 1812 | ACUACAU |
| SEQ ID NO: 1558 | ACGACCC | SEQ ID NO: 1609 | ACGCAGA | SEQ ID NO: 1660 | ACGCUGU | SEQ ID NO: 1711 | ACGGGUG | SEQ ID NO: 1762 | ACGUGAC | SEQ ID NO: 1813 | ACUACCA |
| SEQ ID NO: 1559 | ACGACCG | SEQ ID NO: 1610 | ACGCAGC | SEQ ID NO: 1661 | ACGCUUA | SEQ ID NO: 1712 | ACGGGUU | SEQ ID NO: 1763 | ACGUGAG | SEQ ID NO: 1814 | ACUACCC |
| SEQ ID NO: 1560 | ACGACCU | SEQ ID NO: 1611 | ACGCAGG | SEQ ID NO: 1662 | ACGCUUC | SEQ ID NO: 1713 | ACGGUAA | SEQ ID NO: 1764 | ACGUGAU | SEQ ID NO: 1815 | ACUACCG |
| SEQ ID NO: 1561 | ACGACGA | SEQ ID NO: 1612 | ACGCAGU | SEQ ID NO: 1663 | ACGCUUG | SEQ ID NO: 1714 | ACGGUAC | SEQ ID NO: 1765 | ACGUGCA | SEQ ID NO: 1816 | ACUACCU |
| SEQ ID NO: 1562 | ACGACGC | SEQ ID NO: 1613 | ACGCAUA | SEQ ID NO: 1664 | ACGCUUU | SEQ ID NO: 1715 | ACGGUAG | SEQ ID NO: 1766 | ACGUGCC | SEQ ID NO: 1817 | ACUACGA |
| SEQ ID NO: 1563 | ACGACGG | SEQ ID NO: 1614 | ACGCAUC | SEQ ID NO: 1665 | ACGGAAA | SEQ ID NO: 1716 | ACGGUAU | SEQ ID NO: 1767 | ACGUGCG | SEQ ID NO: 1818 | ACUACGC |
| SEQ ID NO: 1564 | ACGACGU | SEQ ID NO: 1615 | ACGCAUG | SEQ ID NO: 1666 | ACGGAAC | SEQ ID NO: 1717 | ACGGUCA | SEQ ID NO: 1768 | ACGUGCU | SEQ ID NO: 1819 | ACUACGG |
| SEQ ID NO: 1565 | ACGACUA | SEQ ID NO: 1616 | ACGCAUU | SEQ ID NO: 1667 | ACGGAAG | SEQ ID NO: 1718 | ACGGUCC | SEQ ID NO: 1769 | ACGUGGA | SEQ ID NO: 1820 | ACUACGU |
| SEQ ID NO: 1566 | ACGACUC | SEQ ID NO: 1617 | ACGCCAA | SEQ ID NO: 1668 | ACGGAAU | SEQ ID NO: 1719 | ACGGUCG | SEQ ID NO: 1770 | ACGUGGC | SEQ ID NO: 1821 | ACUACUA |
| SEQ ID NO: 1567 | ACGACUG | SEQ ID NO: 1618 | ACGCCAC | SEQ ID NO: 1669 | ACGGACA | SEQ ID NO: 1720 | ACGGUCU | SEQ ID NO: 1771 | ACGUGGG | SEQ ID NO: 1822 | ACUACUC |
| SEQ ID NO: 1568 | ACGACUU | SEQ ID NO: 1619 | ACGCCAG | SEQ ID NO: 1670 | ACGGACC | SEQ ID NO: 1721 | ACGGUGA | SEQ ID NO: 1772 | ACGUGGU | SEQ ID NO: 1823 | ACUACUG |
| SEQ ID NO: 1569 | ACGAGAA | SEQ ID NO: 1620 | ACGCCAU | SEQ ID NO: 1671 | ACGGACG | SEQ ID NO: 1722 | ACGGUGC | SEQ ID NO: 1773 | ACGUGUA | SEQ ID NO: 1824 | ACUACUU |
| SEQ ID NO: 1570 | ACGAGAC | SEQ ID NO: 1621 | ACGCCCA | SEQ ID NO: 1672 | ACGGACU | SEQ ID NO: 1723 | ACGGUGG | SEQ ID NO: 1774 | ACGUGUC | SEQ ID NO: 1825 | ACUAGAA |
| SEQ ID NO: 1571 | ACGAGAG | SEQ ID NO: 1622 | ACGCCCC | SEQ ID NO: 1673 | ACGGAGA | SEQ ID NO: 1724 | ACGGUGU | SEQ ID NO: 1775 | ACGUGUG | SEQ ID NO: 1826 | ACUAGAC |
| SEQ ID NO: 1572 | ACGAGAU | SEQ ID NO: 1623 | ACGCCCG | SEQ ID NO: 1674 | ACGGAGC | SEQ ID NO: 1725 | ACGGUUA | SEQ ID NO: 1776 | ACGUGUU | SEQ ID NO: 1827 | ACUAGAG |
| SEQ ID NO: 1573 | ACGAGCA | SEQ ID NO: 1624 | ACGCCCU | SEQ ID NO: 1675 | ACGGAGG | SEQ ID NO: 1726 | ACGGUUC | SEQ ID NO: 1777 | ACGUUAA | SEQ ID NO: 1828 | ACUAGAU |
| SEQ ID NO: 1574 | ACGAGCC | SEQ ID NO: 1625 | ACGCCGA | SEQ ID NO: 1676 | ACGGAGU | SEQ ID NO: 1727 | ACGGUUG | SEQ ID NO: 1778 | ACGUUAC | SEQ ID NO: 1829 | ACUAGCA |
| SEQ ID NO: 1575 | ACGAGCG | SEQ ID NO: 1626 | ACGCCGC | SEQ ID NO: 1677 | ACGGAUA | SEQ ID NO: 1728 | ACGGUUU | SEQ ID NO: 1779 | ACGUUAG | SEQ ID NO: 1830 | ACUAGCC |
| SEQ ID NO: 1576 | ACGAGCU | SEQ ID NO: 1627 | ACGCCGG | SEQ ID NO: 1678 | ACGGAUC | SEQ ID NO: 1729 | ACGUAAA | SEQ ID NO: 1780 | ACGUUAU | SEQ ID NO: 1831 | ACUAGCG |
| SEQ ID NO: 1577 | ACGAGGA | SEQ ID NO: 1628 | ACGCCGU | SEQ ID NO: 1679 | ACGGAUG | SEQ ID NO: 1730 | ACGUAAC | SEQ ID NO: 1781 | ACGUUCA | SEQ ID NO: 1832 | ACUAGCU |
| SEQ ID NO: 1578 | ACGAGGC | SEQ ID NO: 1629 | ACGCCUA | SEQ ID NO: 1680 | ACGGAUU | SEQ ID NO: 1731 | ACGUAAG | SEQ ID NO: 1782 | ACGUUCC | SEQ ID NO: 1833 | ACUAGGA |
| SEQ ID NO: 1579 | ACGAGGG | SEQ ID NO: 1630 | ACGCCUC | SEQ ID NO: 1681 | ACGGCAA | SEQ ID NO: 1732 | ACGUAAU | SEQ ID NO: 1783 | ACGUUCG | SEQ ID NO: 1834 | ACUAGGC |
| SEQ ID NO: 1580 | ACGAGGU | SEQ ID NO: 1631 | ACGCCUG | SEQ ID NO: 1682 | ACGGCAC | SEQ ID NO: 1733 | ACGUACA | SEQ ID NO: 1784 | ACGUUCU | SEQ ID NO: 1835 | ACUAGGG |
| SEQ ID NO: 1581 | ACGAGUA | SEQ ID NO: 1632 | ACGCCUU | SEQ ID NO: 1683 | ACGGCAG | SEQ ID NO: 1734 | ACGUACC | SEQ ID NO: 1785 | ACGUUGA | SEQ ID NO: 1836 | ACUAGGU |

Table 7

| | | | | | | | | | | | |
|---|---|---|---|---|---|---|---|---|---|---|---|
| SEQ ID NO: 1837 | ACUAGUA | SEQ ID NO: 1888 | ACUCCUU | SEQ ID NO: 1939 | ACUGCAG | SEQ ID NO: 1990 | ACUUACC | SEQ ID NO: 2041 | ACUUUGA | SEQ ID NO: 2092 | AGAAGGU |
| SEQ ID NO: 1838 | ACUAGUC | SEQ ID NO: 1889 | ACUCGAA | SEQ ID NO: 1940 | ACUGCAU | SEQ ID NO: 1991 | ACUUACG | SEQ ID NO: 2042 | ACUUUGC | SEQ ID NO: 2093 | AGAAGUA |
| SEQ ID NO: 1839 | ACUAGUG | SEQ ID NO: 1890 | ACUCGAC | SEQ ID NO: 1941 | ACUGCCA | SEQ ID NO: 1992 | ACUUACU | SEQ ID NO: 2043 | ACUUUGG | SEQ ID NO: 2094 | AGAAGUC |
| SEQ ID NO: 1840 | ACUAGUU | SEQ ID NO: 1891 | ACUCGAG | SEQ ID NO: 1942 | ACUGCCC | SEQ ID NO: 1993 | ACUUAGA | SEQ ID NO: 2044 | ACUUUGU | SEQ ID NO: 2095 | AGAAGUG |
| SEQ ID NO: 1841 | ACUAUAA | SEQ ID NO: 1892 | ACUCGAU | SEQ ID NO: 1943 | ACUGCCG | SEQ ID NO: 1994 | ACUUAGC | SEQ ID NO: 2045 | ACUUUUA | SEQ ID NO: 2096 | AGAAGUU |
| SEQ ID NO: 1842 | ACUAUAC | SEQ ID NO: 1893 | ACUCGCA | SEQ ID NO: 1944 | ACUGCCU | SEQ ID NO: 1995 | ACUUAGG | SEQ ID NO: 2046 | ACUUUUC | SEQ ID NO: 2097 | AGAAUAA |
| SEQ ID NO: 1843 | ACUAUAG | SEQ ID NO: 1894 | ACUCGCC | SEQ ID NO: 1945 | ACUGCGA | SEQ ID NO: 1996 | ACUUAGU | SEQ ID NO: 2047 | ACUUUUG | SEQ ID NO: 2098 | AGAAUAC |
| SEQ ID NO: 1844 | ACUAUAU | SEQ ID NO: 1895 | ACUCGCG | SEQ ID NO: 1946 | ACUGCGC | SEQ ID NO: 1997 | ACUUAUA | SEQ ID NO: 2048 | ACUUUUU | SEQ ID NO: 2099 | AGAAUAG |
| SEQ ID NO: 1845 | ACUAUCA | SEQ ID NO: 1896 | ACUCGCU | SEQ ID NO: 1947 | ACUGCGG | SEQ ID NO: 1998 | ACUUAUC | SEQ ID NO: 2049 | AGAAAAA | SEQ ID NO: 2100 | AGAAUAU |
| SEQ ID NO: 1846 | ACUAUCC | SEQ ID NO: 1897 | ACUCGGA | SEQ ID NO: 1948 | ACUGCGU | SEQ ID NO: 1999 | ACUUAUG | SEQ ID NO: 2050 | AGAAAAC | SEQ ID NO: 2101 | AGAAUCA |
| SEQ ID NO: 1847 | ACUAUCG | SEQ ID NO: 1898 | ACUCGGC | SEQ ID NO: 1949 | ACUGCUA | SEQ ID NO: 2000 | ACUUAUU | SEQ ID NO: 2051 | AGAAAAG | SEQ ID NO: 2102 | AGAAUCC |
| SEQ ID NO: 1848 | ACUAUCU | SEQ ID NO: 1899 | ACUCGGG | SEQ ID NO: 1950 | ACUGCUC | SEQ ID NO: 2001 | ACUUCAA | SEQ ID NO: 2052 | AGAAAAU | SEQ ID NO: 2103 | AGAAUCG |
| SEQ ID NO: 1849 | ACUAUGA | SEQ ID NO: 1900 | ACUCGGU | SEQ ID NO: 1951 | ACUGCUG | SEQ ID NO: 2002 | ACUUCAC | SEQ ID NO: 2053 | AGAAACA | SEQ ID NO: 2104 | AGAAUCU |
| SEQ ID NO: 1850 | ACUAUGC | SEQ ID NO: 1901 | ACUCGUA | SEQ ID NO: 1952 | ACUGCUU | SEQ ID NO: 2003 | ACUUCAG | SEQ ID NO: 2054 | AGAAACC | SEQ ID NO: 2105 | AGAAUGA |
| SEQ ID NO: 1851 | ACUAUGG | SEQ ID NO: 1902 | ACUCGUC | SEQ ID NO: 1953 | ACUGGAA | SEQ ID NO: 2004 | ACUUCAU | SEQ ID NO: 2055 | AGAAACG | SEQ ID NO: 2106 | AGAAUGC |
| SEQ ID NO: 1852 | ACUAUGU | SEQ ID NO: 1903 | ACUCGUG | SEQ ID NO: 1954 | ACUGGAC | SEQ ID NO: 2005 | ACUUCCA | SEQ ID NO: 2056 | AGAAACU | SEQ ID NO: 2107 | AGAAUGG |
| SEQ ID NO: 1853 | ACUAUUA | SEQ ID NO: 1904 | ACUCGUU | SEQ ID NO: 1955 | ACUGGAG | SEQ ID NO: 2006 | ACUUCCC | SEQ ID NO: 2057 | AGAAAGA | SEQ ID NO: 2108 | AGAAUGU |
| SEQ ID NO: 1854 | ACUAUUC | SEQ ID NO: 1905 | ACUCUAA | SEQ ID NO: 1956 | ACUGGAU | SEQ ID NO: 2007 | ACUUCCG | SEQ ID NO: 2058 | AGAAAGC | SEQ ID NO: 2109 | AGAAUUA |
| SEQ ID NO: 1855 | ACUAUUG | SEQ ID NO: 1906 | ACUCUAC | SEQ ID NO: 1957 | ACUGGCA | SEQ ID NO: 2008 | ACUUCCU | SEQ ID NO: 2059 | AGAAAGG | SEQ ID NO: 2110 | AGAAUUC |
| SEQ ID NO: 1856 | ACUAUUU | SEQ ID NO: 1907 | ACUCUAG | SEQ ID NO: 1958 | ACUGGCC | SEQ ID NO: 2009 | ACUUCGA | SEQ ID NO: 2060 | AGAAAGU | SEQ ID NO: 2111 | AGAAUUG |
| SEQ ID NO: 1857 | ACUCAAA | SEQ ID NO: 1908 | ACUCUAU | SEQ ID NO: 1959 | ACUGGCG | SEQ ID NO: 2010 | ACUUCGC | SEQ ID NO: 2061 | AGAAAUA | SEQ ID NO: 2112 | AGAAUUU |
| SEQ ID NO: 1858 | ACUCAAC | SEQ ID NO: 1909 | ACUCUCA | SEQ ID NO: 1960 | ACUGGCU | SEQ ID NO: 2011 | ACUUCGG | SEQ ID NO: 2062 | AGAAAUC | SEQ ID NO: 2113 | AGACAAA |
| SEQ ID NO: 1859 | ACUCAAG | SEQ ID NO: 1910 | ACUCUCC | SEQ ID NO: 1961 | ACUGGGA | SEQ ID NO: 2012 | ACUUCGU | SEQ ID NO: 2063 | AGAAAUG | SEQ ID NO: 2114 | AGACAAC |
| SEQ ID NO: 1860 | ACUCAAU | SEQ ID NO: 1911 | ACUCUCG | SEQ ID NO: 1962 | ACUGGGC | SEQ ID NO: 2013 | ACUUCUA | SEQ ID NO: 2064 | AGAAAUU | SEQ ID NO: 2115 | AGACAAG |
| SEQ ID NO: 1861 | ACUCACA | SEQ ID NO: 1912 | ACUCUCU | SEQ ID NO: 1963 | ACUGGGG | SEQ ID NO: 2014 | ACUUCUC | SEQ ID NO: 2065 | AGAACAA | SEQ ID NO: 2116 | AGACAAU |
| SEQ ID NO: 1862 | ACUCACC | SEQ ID NO: 1913 | ACUCUGA | SEQ ID NO: 1964 | ACUGGGU | SEQ ID NO: 2015 | ACUUCUG | SEQ ID NO: 2066 | AGAACAC | SEQ ID NO: 2117 | AGACACA |
| SEQ ID NO: 1863 | ACUCACG | SEQ ID NO: 1914 | ACUCUGC | SEQ ID NO: 1965 | ACUGGUA | SEQ ID NO: 2016 | ACUUCUU | SEQ ID NO: 2067 | AGAACAG | SEQ ID NO: 2118 | AGACACC |
| SEQ ID NO: 1864 | ACUCACU | SEQ ID NO: 1915 | ACUCUGG | SEQ ID NO: 1966 | ACUGGUC | SEQ ID NO: 2017 | ACUUGAA | SEQ ID NO: 2068 | AGAACAU | SEQ ID NO: 2119 | AGACACG |
| SEQ ID NO: 1865 | ACUCAGA | SEQ ID NO: 1916 | ACUCUGU | SEQ ID NO: 1967 | ACUGGUG | SEQ ID NO: 2018 | ACUUGAC | SEQ ID NO: 2069 | AGAACCA | SEQ ID NO: 2120 | AGACACU |
| SEQ ID NO: 1866 | ACUCAGC | SEQ ID NO: 1917 | ACUCUUA | SEQ ID NO: 1968 | ACUGGUU | SEQ ID NO: 2019 | ACUUGAG | SEQ ID NO: 2070 | AGAACCC | SEQ ID NO: 2121 | AGACAGA |
| SEQ ID NO: 1867 | ACUCAGG | SEQ ID NO: 1918 | ACUCUUC | SEQ ID NO: 1969 | ACUGUAA | SEQ ID NO: 2020 | ACUUGAU | SEQ ID NO: 2071 | AGAACCG | SEQ ID NO: 2122 | AGACAGC |
| SEQ ID NO: 1868 | ACUCAGU | SEQ ID NO: 1919 | ACUCUUG | SEQ ID NO: 1970 | ACUGUAC | SEQ ID NO: 2021 | ACUUGCA | SEQ ID NO: 2072 | AGAACCU | SEQ ID NO: 2123 | AGACAGG |
| SEQ ID NO: 1869 | ACUCAUA | SEQ ID NO: 1920 | ACUCUUU | SEQ ID NO: 1971 | ACUGUAG | SEQ ID NO: 2022 | ACUUGCC | SEQ ID NO: 2073 | AGAACGA | SEQ ID NO: 2124 | AGACAGU |
| SEQ ID NO: 1870 | ACUCAUC | SEQ ID NO: 1921 | ACUGAAA | SEQ ID NO: 1972 | ACUGUAU | SEQ ID NO: 2023 | ACUUGCG | SEQ ID NO: 2074 | AGAACGC | SEQ ID NO: 2125 | AGACAUA |
| SEQ ID NO: 1871 | ACUCAUG | SEQ ID NO: 1922 | ACUGAAC | SEQ ID NO: 1973 | ACUGUCA | SEQ ID NO: 2024 | ACUUGCU | SEQ ID NO: 2075 | AGAACGG | SEQ ID NO: 2126 | AGACAUC |
| SEQ ID NO: 1872 | ACUCAUU | SEQ ID NO: 1923 | ACUGAAG | SEQ ID NO: 1974 | ACUGUCC | SEQ ID NO: 2025 | ACUUGGA | SEQ ID NO: 2076 | AGAACGU | SEQ ID NO: 2127 | AGACAUG |
| SEQ ID NO: 1873 | ACUCCAA | SEQ ID NO: 1924 | ACUGAAU | SEQ ID NO: 1975 | ACUGUCG | SEQ ID NO: 2026 | ACUUGGC | SEQ ID NO: 2077 | AGAACUA | SEQ ID NO: 2128 | AGACAUU |
| SEQ ID NO: 1874 | ACUCCAC | SEQ ID NO: 1925 | ACUGACA | SEQ ID NO: 1976 | ACUGUCU | SEQ ID NO: 2027 | ACUUGGG | SEQ ID NO: 2078 | AGAACUC | SEQ ID NO: 2129 | AGACCAA |
| SEQ ID NO: 1875 | ACUCCAG | SEQ ID NO: 1926 | ACUGACC | SEQ ID NO: 1977 | ACUGUGA | SEQ ID NO: 2028 | ACUUGGU | SEQ ID NO: 2079 | AGAACUG | SEQ ID NO: 2130 | AGACCAC |
| SEQ ID NO: 1876 | ACUCCAU | SEQ ID NO: 1927 | ACUGACG | SEQ ID NO: 1978 | ACUGUGC | SEQ ID NO: 2029 | ACUUGUA | SEQ ID NO: 2080 | AGAACUU | SEQ ID NO: 2131 | AGACCAG |
| SEQ ID NO: 1877 | ACUCCCA | SEQ ID NO: 1928 | ACUGACU | SEQ ID NO: 1979 | ACUGUGG | SEQ ID NO: 2030 | ACUUGUC | SEQ ID NO: 2081 | AGAAGAA | SEQ ID NO: 2132 | AGACCAU |
| SEQ ID NO: 1878 | ACUCCCC | SEQ ID NO: 1929 | ACUGAGA | SEQ ID NO: 1980 | ACUGUGU | SEQ ID NO: 2031 | ACUUGUG | SEQ ID NO: 2082 | AGAAGAC | SEQ ID NO: 2133 | AGACCCA |
| SEQ ID NO: 1879 | ACUCCCG | SEQ ID NO: 1930 | ACUGAGC | SEQ ID NO: 1981 | ACUGUUA | SEQ ID NO: 2032 | ACUUGUU | SEQ ID NO: 2083 | AGAAGAG | SEQ ID NO: 2134 | AGACCCC |
| SEQ ID NO: 1880 | ACUCCCU | SEQ ID NO: 1931 | ACUGAGG | SEQ ID NO: 1982 | ACUGUUC | SEQ ID NO: 2033 | ACUUUAA | SEQ ID NO: 2084 | AGAAGAU | SEQ ID NO: 2135 | AGACCCG |
| SEQ ID NO: 1881 | ACUCCGA | SEQ ID NO: 1932 | ACUGAGU | SEQ ID NO: 1983 | ACUGUUG | SEQ ID NO: 2034 | ACUUUAC | SEQ ID NO: 2085 | AGAAGCA | SEQ ID NO: 2136 | AGACCCU |
| SEQ ID NO: 1882 | ACUCCGC | SEQ ID NO: 1933 | ACUGAUA | SEQ ID NO: 1984 | ACUGUUU | SEQ ID NO: 2035 | ACUUUAG | SEQ ID NO: 2086 | AGAAGCC | SEQ ID NO: 2137 | AGACCGA |
| SEQ ID NO: 1883 | ACUCCGG | SEQ ID NO: 1934 | ACUGAUC | SEQ ID NO: 1985 | ACUUAAA | SEQ ID NO: 2036 | ACUUUAU | SEQ ID NO: 2087 | AGAAGCG | SEQ ID NO: 2138 | AGACCGC |
| SEQ ID NO: 1884 | ACUCCGU | SEQ ID NO: 1935 | ACUGAUG | SEQ ID NO: 1986 | ACUUAAC | SEQ ID NO: 2037 | ACUUUCA | SEQ ID NO: 2088 | AGAAGCU | SEQ ID NO: 2139 | AGACCGG |
| SEQ ID NO: 1885 | ACUCCUA | SEQ ID NO: 1936 | ACUGAUU | SEQ ID NO: 1987 | ACUUAAG | SEQ ID NO: 2038 | ACUUUCC | SEQ ID NO: 2089 | AGAAGGA | SEQ ID NO: 2140 | AGACCGU |
| SEQ ID NO: 1886 | ACUCCUC | SEQ ID NO: 1937 | ACUGCAA | SEQ ID NO: 1988 | ACUUAAU | SEQ ID NO: 2039 | ACUUUCG | SEQ ID NO: 2090 | AGAAGGC | SEQ ID NO: 2141 | AGACCUA |
| SEQ ID NO: 1887 | ACUCCUG | SEQ ID NO: 1938 | ACUGCAC | SEQ ID NO: 1989 | ACUUACA | SEQ ID NO: 2040 | ACUUUCU | SEQ ID NO: 2091 | AGAAGGG | SEQ ID NO: 2142 | AGACCUC |

Table 8

| | | | | | | | | | | | |
|---|---|---|---|---|---|---|---|---|---|---|---|
| SEQ ID NO: 2143 | AGACCUG | SEQ ID NO: 2194 | AGAGCAC | SEQ ID NO: 2245 | AGAUACA | SEQ ID NO: 2296 | AGAUUCU | SEQ ID NO: 2347 | AGCAGGG | SEQ ID NO: 2398 | AGCCCUC |
| SEQ ID NO: 2144 | AGACCUU | SEQ ID NO: 2195 | AGAGCAG | SEQ ID NO: 2246 | AGAUACC | SEQ ID NO: 2297 | AGAUUGA | SEQ ID NO: 2348 | AGCAGGU | SEQ ID NO: 2399 | AGCCCUG |
| SEQ ID NO: 2145 | AGACGAA | SEQ ID NO: 2196 | AGAGCAU | SEQ ID NO: 2247 | AGAUACG | SEQ ID NO: 2298 | AGAUUGC | SEQ ID NO: 2349 | AGCAGUA | SEQ ID NO: 2400 | AGCCCUU |
| SEQ ID NO: 2146 | AGACGAC | SEQ ID NO: 2197 | AGAGCCA | SEQ ID NO: 2248 | AGAUACU | SEQ ID NO: 2299 | AGAUUGG | SEQ ID NO: 2350 | AGCAGUC | SEQ ID NO: 2401 | AGCCGAA |
| SEQ ID NO: 2147 | AGACGAG | SEQ ID NO: 2198 | AGAGCCC | SEQ ID NO: 2249 | AGAUAGA | SEQ ID NO: 2300 | AGAUUGU | SEQ ID NO: 2351 | AGCAGUG | SEQ ID NO: 2402 | AGCCGAC |
| SEQ ID NO: 2148 | AGACGAU | SEQ ID NO: 2199 | AGAGCCG | SEQ ID NO: 2250 | AGAUAGC | SEQ ID NO: 2301 | AGAUUUA | SEQ ID NO: 2352 | AGCAGUU | SEQ ID NO: 2403 | AGCCGAG |
| SEQ ID NO: 2149 | AGACGCA | SEQ ID NO: 2200 | AGAGCCU | SEQ ID NO: 2251 | AGAUAGG | SEQ ID NO: 2302 | AGAUUUC | SEQ ID NO: 2353 | AGCAUAA | SEQ ID NO: 2404 | AGCCGAU |
| SEQ ID NO: 2150 | AGACGCC | SEQ ID NO: 2201 | AGAGCGA | SEQ ID NO: 2252 | AGAUAGU | SEQ ID NO: 2303 | AGAUUUG | SEQ ID NO: 2354 | AGCAUAC | SEQ ID NO: 2405 | AGCCGCA |
| SEQ ID NO: 2151 | AGACGCG | SEQ ID NO: 2202 | AGAGCGC | SEQ ID NO: 2253 | AGAUAUA | SEQ ID NO: 2304 | AGAUUUU | SEQ ID NO: 2355 | AGCAUAG | SEQ ID NO: 2406 | AGCCGCC |
| SEQ ID NO: 2152 | AGACGCU | SEQ ID NO: 2203 | AGAGCGG | SEQ ID NO: 2254 | AGAUAUC | SEQ ID NO: 2305 | AGCAAAA | SEQ ID NO: 2356 | AGCAUAU | SEQ ID NO: 2407 | AGCCGCG |
| SEQ ID NO: 2153 | AGACGGA | SEQ ID NO: 2204 | AGAGCGU | SEQ ID NO: 2255 | AGAUAUG | SEQ ID NO: 2306 | AGCAAAC | SEQ ID NO: 2357 | AGCAUCA | SEQ ID NO: 2408 | AGCCGCU |
| SEQ ID NO: 2154 | AGACGGC | SEQ ID NO: 2205 | AGAGCUA | SEQ ID NO: 2256 | AGAUAUU | SEQ ID NO: 2307 | AGCAAAG | SEQ ID NO: 2358 | AGCAUCC | SEQ ID NO: 2409 | AGCCGGA |
| SEQ ID NO: 2155 | AGACGGG | SEQ ID NO: 2206 | AGAGCUC | SEQ ID NO: 2257 | AGAUCAA | SEQ ID NO: 2308 | AGCAAAU | SEQ ID NO: 2359 | AGCAUCG | SEQ ID NO: 2410 | AGCCGGC |
| SEQ ID NO: 2156 | AGACGGU | SEQ ID NO: 2207 | AGAGCUG | SEQ ID NO: 2258 | AGAUCAC | SEQ ID NO: 2309 | AGCAACA | SEQ ID NO: 2360 | AGCAUCU | SEQ ID NO: 2411 | AGCCGGG |
| SEQ ID NO: 2157 | AGACGUA | SEQ ID NO: 2208 | AGAGCUU | SEQ ID NO: 2259 | AGAUCAG | SEQ ID NO: 2310 | AGCAACC | SEQ ID NO: 2361 | AGCAUGA | SEQ ID NO: 2412 | AGCCGGU |
| SEQ ID NO: 2158 | AGACGUC | SEQ ID NO: 2209 | AGAGGAA | SEQ ID NO: 2260 | AGAUCAU | SEQ ID NO: 2311 | AGCAACG | SEQ ID NO: 2362 | AGCAUGC | SEQ ID NO: 2413 | AGCCGUA |
| SEQ ID NO: 2159 | AGACGUG | SEQ ID NO: 2210 | AGAGGAC | SEQ ID NO: 2261 | AGAUCCA | SEQ ID NO: 2312 | AGCAACU | SEQ ID NO: 2363 | AGCAUGG | SEQ ID NO: 2414 | AGCCGUC |
| SEQ ID NO: 2160 | AGACGUU | SEQ ID NO: 2211 | AGAGGAG | SEQ ID NO: 2262 | AGAUCCC | SEQ ID NO: 2313 | AGCAAGA | SEQ ID NO: 2364 | AGCAUGU | SEQ ID NO: 2415 | AGCCGUG |
| SEQ ID NO: 2161 | AGACUAA | SEQ ID NO: 2212 | AGAGGAU | SEQ ID NO: 2263 | AGAUCCG | SEQ ID NO: 2314 | AGCAAGC | SEQ ID NO: 2365 | AGCAUUA | SEQ ID NO: 2416 | AGCCGUU |
| SEQ ID NO: 2162 | AGACUAC | SEQ ID NO: 2213 | AGAGGCA | SEQ ID NO: 2264 | AGAUCCU | SEQ ID NO: 2315 | AGCAAGG | SEQ ID NO: 2366 | AGCAUUC | SEQ ID NO: 2417 | AGCCUAA |
| SEQ ID NO: 2163 | AGACUAG | SEQ ID NO: 2214 | AGAGGCC | SEQ ID NO: 2265 | AGAUCGA | SEQ ID NO: 2316 | AGCAAGU | SEQ ID NO: 2367 | AGCAUUG | SEQ ID NO: 2418 | AGCCUAC |
| SEQ ID NO: 2164 | AGACUAU | SEQ ID NO: 2215 | AGAGGCG | SEQ ID NO: 2266 | AGAUCGC | SEQ ID NO: 2317 | AGCAAUA | SEQ ID NO: 2368 | AGCAUUU | SEQ ID NO: 2419 | AGCCUAG |
| SEQ ID NO: 2165 | AGACUCA | SEQ ID NO: 2216 | AGAGGCU | SEQ ID NO: 2267 | AGAUCGG | SEQ ID NO: 2318 | AGCAAUC | SEQ ID NO: 2369 | AGCCAAA | SEQ ID NO: 2420 | AGCCUAU |
| SEQ ID NO: 2166 | AGACUCC | SEQ ID NO: 2217 | AGAGGGA | SEQ ID NO: 2268 | AGAUCGU | SEQ ID NO: 2319 | AGCAAUG | SEQ ID NO: 2370 | AGCCAAC | SEQ ID NO: 2421 | AGCCUCA |
| SEQ ID NO: 2167 | AGACUCG | SEQ ID NO: 2218 | AGAGGGC | SEQ ID NO: 2269 | AGAUCUA | SEQ ID NO: 2320 | AGCAAUU | SEQ ID NO: 2371 | AGCCAAG | SEQ ID NO: 2422 | AGCCUCC |
| SEQ ID NO: 2168 | AGACUCU | SEQ ID NO: 2219 | AGAGGGG | SEQ ID NO: 2270 | AGAUCUC | SEQ ID NO: 2321 | AGCACAA | SEQ ID NO: 2372 | AGCCAAU | SEQ ID NO: 2423 | AGCCUCG |
| SEQ ID NO: 2169 | AGACUGA | SEQ ID NO: 2220 | AGAGGGU | SEQ ID NO: 2271 | AGAUCUG | SEQ ID NO: 2322 | AGCACAC | SEQ ID NO: 2373 | AGCCACA | SEQ ID NO: 2424 | AGCCUCU |
| SEQ ID NO: 2170 | AGACUGC | SEQ ID NO: 2221 | AGAGGUA | SEQ ID NO: 2272 | AGAUCUU | SEQ ID NO: 2323 | AGCACAG | SEQ ID NO: 2374 | AGCCACC | SEQ ID NO: 2425 | AGCCUGA |
| SEQ ID NO: 2171 | AGACUGG | SEQ ID NO: 2222 | AGAGGUC | SEQ ID NO: 2273 | AGAUGAA | SEQ ID NO: 2324 | AGCACAU | SEQ ID NO: 2375 | AGCCACG | SEQ ID NO: 2426 | AGCCUGC |
| SEQ ID NO: 2172 | AGACUGU | SEQ ID NO: 2223 | AGAGGUG | SEQ ID NO: 2274 | AGAUGAC | SEQ ID NO: 2325 | AGCACCA | SEQ ID NO: 2376 | AGCCACU | SEQ ID NO: 2427 | AGCCUGG |
| SEQ ID NO: 2173 | AGACUUA | SEQ ID NO: 2224 | AGAGGUU | SEQ ID NO: 2275 | AGAUGAG | SEQ ID NO: 2326 | AGCACCC | SEQ ID NO: 2377 | AGCCAGA | SEQ ID NO: 2428 | AGCCUGU |
| SEQ ID NO: 2174 | AGACUUC | SEQ ID NO: 2225 | AGAGUAA | SEQ ID NO: 2276 | AGAUGAU | SEQ ID NO: 2327 | AGCACCG | SEQ ID NO: 2378 | AGCCAGC | SEQ ID NO: 2429 | AGCCUUA |
| SEQ ID NO: 2175 | AGACUUG | SEQ ID NO: 2226 | AGAGUAC | SEQ ID NO: 2277 | AGAUGCA | SEQ ID NO: 2328 | AGCACCU | SEQ ID NO: 2379 | AGCCAGG | SEQ ID NO: 2430 | AGCCUUC |
| SEQ ID NO: 2176 | AGACUUU | SEQ ID NO: 2227 | AGAGUAG | SEQ ID NO: 2278 | AGAUGCC | SEQ ID NO: 2329 | AGCACGA | SEQ ID NO: 2380 | AGCCAGU | SEQ ID NO: 2431 | AGCCUUG |
| SEQ ID NO: 2177 | AGAGAAA | SEQ ID NO: 2228 | AGAGUAU | SEQ ID NO: 2279 | AGAUGCG | SEQ ID NO: 2330 | AGCACGC | SEQ ID NO: 2381 | AGCCAUA | SEQ ID NO: 2432 | AGCCUUU |
| SEQ ID NO: 2178 | AGAGAAC | SEQ ID NO: 2229 | AGAGUCA | SEQ ID NO: 2280 | AGAUGCU | SEQ ID NO: 2331 | AGCACGG | SEQ ID NO: 2382 | AGCCAUC | SEQ ID NO: 2433 | AGCGAAA |
| SEQ ID NO: 2179 | AGAGAAG | SEQ ID NO: 2230 | AGAGUCC | SEQ ID NO: 2281 | AGAUGGA | SEQ ID NO: 2332 | AGCACGU | SEQ ID NO: 2383 | AGCCAUG | SEQ ID NO: 2434 | AGCGAAC |
| SEQ ID NO: 2180 | AGAGAAU | SEQ ID NO: 2231 | AGAGUCG | SEQ ID NO: 2282 | AGAUGGC | SEQ ID NO: 2333 | AGCACUA | SEQ ID NO: 2384 | AGCCAUU | SEQ ID NO: 2435 | AGCGAAG |
| SEQ ID NO: 2181 | AGAGACA | SEQ ID NO: 2232 | AGAGUCU | SEQ ID NO: 2283 | AGAUGGG | SEQ ID NO: 2334 | AGCACUC | SEQ ID NO: 2385 | AGCCCAA | SEQ ID NO: 2436 | AGCGAAU |
| SEQ ID NO: 2182 | AGAGACC | SEQ ID NO: 2233 | AGAGUGA | SEQ ID NO: 2284 | AGAUGGU | SEQ ID NO: 2335 | AGCACUG | SEQ ID NO: 2386 | AGCCCAC | SEQ ID NO: 2437 | AGCGACA |
| SEQ ID NO: 2183 | AGAGACG | SEQ ID NO: 2234 | AGAGUGC | SEQ ID NO: 2285 | AGAUGUA | SEQ ID NO: 2336 | AGCACUU | SEQ ID NO: 2387 | AGCCCAG | SEQ ID NO: 2438 | AGCGACC |
| SEQ ID NO: 2184 | AGAGACU | SEQ ID NO: 2235 | AGAGUGG | SEQ ID NO: 2286 | AGAUGUC | SEQ ID NO: 2337 | AGCAGAA | SEQ ID NO: 2388 | AGCCCAU | SEQ ID NO: 2439 | AGCGACG |
| SEQ ID NO: 2185 | AGAGAGA | SEQ ID NO: 2236 | AGAGUGU | SEQ ID NO: 2287 | AGAUGUG | SEQ ID NO: 2338 | AGCAGAC | SEQ ID NO: 2389 | AGCCCCA | SEQ ID NO: 2440 | AGCGACU |
| SEQ ID NO: 2186 | AGAGAGC | SEQ ID NO: 2237 | AGAGUUA | SEQ ID NO: 2288 | AGAUGUU | SEQ ID NO: 2339 | AGCAGAG | SEQ ID NO: 2390 | AGCCCCC | SEQ ID NO: 2441 | AGCGAGA |
| SEQ ID NO: 2187 | AGAGAGG | SEQ ID NO: 2238 | AGAGUUC | SEQ ID NO: 2289 | AGAUUAA | SEQ ID NO: 2340 | AGCAGAU | SEQ ID NO: 2391 | AGCCCCG | SEQ ID NO: 2442 | AGCGAGC |
| SEQ ID NO: 2188 | AGAGAGU | SEQ ID NO: 2239 | AGAGUUG | SEQ ID NO: 2290 | AGAUUAC | SEQ ID NO: 2341 | AGCAGCA | SEQ ID NO: 2392 | AGCCCCU | SEQ ID NO: 2443 | AGCGAGG |
| SEQ ID NO: 2189 | AGAGAUA | SEQ ID NO: 2240 | AGAGUUU | SEQ ID NO: 2291 | AGAUUAG | SEQ ID NO: 2342 | AGCAGCC | SEQ ID NO: 2393 | AGCCCGA | SEQ ID NO: 2444 | AGCGAGU |
| SEQ ID NO: 2190 | AGAGAUC | SEQ ID NO: 2241 | AGAUAAA | SEQ ID NO: 2292 | AGAUUAU | SEQ ID NO: 2343 | AGCAGCG | SEQ ID NO: 2394 | AGCCCGC | SEQ ID NO: 2445 | AGCGAUA |
| SEQ ID NO: 2191 | AGAGAUG | SEQ ID NO: 2242 | AGAUAAC | SEQ ID NO: 2293 | AGAUUCA | SEQ ID NO: 2344 | AGCAGCU | SEQ ID NO: 2395 | AGCCCGG | SEQ ID NO: 2446 | AGCGAUC |
| SEQ ID NO: 2192 | AGAGAUU | SEQ ID NO: 2243 | AGAUAAG | SEQ ID NO: 2294 | AGAUUCC | SEQ ID NO: 2345 | AGCAGGA | SEQ ID NO: 2396 | AGCCCGU | SEQ ID NO: 2447 | AGCGAUG |
| SEQ ID NO: 2193 | AGAGCAA | SEQ ID NO: 2244 | AGAUAAU | SEQ ID NO: 2295 | AGAUUCG | SEQ ID NO: 2346 | AGCAGGC | SEQ ID NO: 2397 | AGCCCUA | SEQ ID NO: 2448 | AGCGAUU |

Table 9

| | |
|---|---|
| SEQ ID NO: 2449 | AGCGCAA |
| SEQ ID NO: 2450 | AGCGCAC |
| SEQ ID NO: 2451 | AGCGCAG |
| SEQ ID NO: 2452 | AGCGCAU |
| SEQ ID NO: 2453 | AGCGCCA |
| SEQ ID NO: 2454 | AGCGCCC |
| SEQ ID NO: 2455 | AGCGCCG |
| SEQ ID NO: 2456 | AGCGCCU |
| SEQ ID NO: 2457 | AGCGCGA |
| SEQ ID NO: 2458 | AGCGCGC |
| SEQ ID NO: 2459 | AGCGCGG |
| SEQ ID NO: 2460 | AGCGCGU |
| SEQ ID NO: 2461 | AGCGCUA |
| SEQ ID NO: 2462 | AGCGCUC |
| SEQ ID NO: 2463 | AGCGCUG |
| SEQ ID NO: 2464 | AGCGCUU |
| SEQ ID NO: 2465 | AGCGGAA |
| SEQ ID NO: 2466 | AGCGGAC |
| SEQ ID NO: 2467 | AGCGGAG |
| SEQ ID NO: 2468 | AGCGGAU |
| SEQ ID NO: 2469 | AGCGGCA |
| SEQ ID NO: 2470 | AGCGGCC |
| SEQ ID NO: 2471 | AGCGGCG |
| SEQ ID NO: 2472 | AGCGGCU |
| SEQ ID NO: 2473 | AGCGGGA |
| SEQ ID NO: 2474 | AGCGGGC |
| SEQ ID NO: 2475 | AGCGGGG |
| SEQ ID NO: 2476 | AGCGGGU |
| SEQ ID NO: 2477 | AGCGGUA |
| SEQ ID NO: 2478 | AGCGGUC |
| SEQ ID NO: 2479 | AGCGGUG |
| SEQ ID NO: 2480 | AGCGGUU |
| SEQ ID NO: 2481 | AGCGUAA |
| SEQ ID NO: 2482 | AGCGUAC |
| SEQ ID NO: 2483 | AGCGUAG |
| SEQ ID NO: 2484 | AGCGUAU |
| SEQ ID NO: 2485 | AGCGUCA |
| SEQ ID NO: 2486 | AGCGUCC |
| SEQ ID NO: 2487 | AGCGUCG |
| SEQ ID NO: 2488 | AGCGUCU |
| SEQ ID NO: 2489 | AGCGUGA |
| SEQ ID NO: 2490 | AGCGUGC |
| SEQ ID NO: 2491 | AGCGUGG |
| SEQ ID NO: 2492 | AGCGUGU |
| SEQ ID NO: 2493 | AGCGUUA |
| SEQ ID NO: 2494 | AGCGUUC |
| SEQ ID NO: 2495 | AGCGUUG |
| SEQ ID NO: 2496 | AGCGUUU |
| SEQ ID NO: 2497 | AGCUAAA |
| SEQ ID NO: 2498 | AGCUAAC |
| SEQ ID NO: 2499 | AGCUAAG |
| SEQ ID NO: 2500 | AGCUAAU |
| SEQ ID NO: 2501 | AGCUACA |
| SEQ ID NO: 2502 | AGCUACC |
| SEQ ID NO: 2503 | AGCUACG |
| SEQ ID NO: 2504 | AGCUACU |
| SEQ ID NO: 2505 | AGCUAGA |
| SEQ ID NO: 2506 | AGCUAGC |
| SEQ ID NO: 2507 | AGCUAGG |
| SEQ ID NO: 2508 | AGCUAGU |
| SEQ ID NO: 2509 | AGCUAUA |
| SEQ ID NO: 2510 | AGCUAUC |
| SEQ ID NO: 2511 | AGCUAUG |
| SEQ ID NO: 2512 | AGCUAUU |
| SEQ ID NO: 2513 | AGCUCAA |
| SEQ ID NO: 2514 | AGCUCAC |
| SEQ ID NO: 2515 | AGCUCAG |
| SEQ ID NO: 2516 | AGCUCAU |
| SEQ ID NO: 2517 | AGCUCCA |
| SEQ ID NO: 2518 | AGCUCCC |
| SEQ ID NO: 2519 | AGCUCCG |
| SEQ ID NO: 2520 | AGCUCCU |
| SEQ ID NO: 2521 | AGCUCGA |
| SEQ ID NO: 2522 | AGCUCGC |
| SEQ ID NO: 2523 | AGCUCGG |
| SEQ ID NO: 2524 | AGCUCGU |
| SEQ ID NO: 2525 | AGCUCUA |
| SEQ ID NO: 2526 | AGCUCUC |
| SEQ ID NO: 2527 | AGCUCUG |
| SEQ ID NO: 2528 | AGCUCUU |
| SEQ ID NO: 2529 | AGCUGAA |
| SEQ ID NO: 2530 | AGCUGAC |
| SEQ ID NO: 2531 | AGCUGAG |
| SEQ ID NO: 2532 | AGCUGAU |
| SEQ ID NO: 2533 | AGCUGCA |
| SEQ ID NO: 2534 | AGCUGCC |
| SEQ ID NO: 2535 | AGCUGCG |
| SEQ ID NO: 2536 | AGCUGCU |
| SEQ ID NO: 2537 | AGCUGGA |
| SEQ ID NO: 2538 | AGCUGGC |
| SEQ ID NO: 2539 | AGCUGGG |
| SEQ ID NO: 2540 | AGCUGGU |
| SEQ ID NO: 2541 | AGCUGUA |
| SEQ ID NO: 2542 | AGCUGUC |
| SEQ ID NO: 2543 | AGCUGUG |
| SEQ ID NO: 2544 | AGCUGUU |
| SEQ ID NO: 2545 | AGCUUAA |
| SEQ ID NO: 2546 | AGCUUAC |
| SEQ ID NO: 2547 | AGCUUAG |
| SEQ ID NO: 2548 | AGCUUAU |
| SEQ ID NO: 2549 | AGCUUCA |
| SEQ ID NO: 2550 | AGCUUCC |
| SEQ ID NO: 2551 | AGCUUCG |
| SEQ ID NO: 2552 | AGCUUCU |
| SEQ ID NO: 2553 | AGCUUGA |
| SEQ ID NO: 2554 | AGCUUGC |
| SEQ ID NO: 2555 | AGCUUGG |
| SEQ ID NO: 2556 | AGCUUGU |
| SEQ ID NO: 2557 | AGCUUUA |
| SEQ ID NO: 2558 | AGCUUUC |
| SEQ ID NO: 2559 | AGCUUUG |
| SEQ ID NO: 2560 | AGCUUUU |
| SEQ ID NO: 2561 | AGGAAAA |
| SEQ ID NO: 2562 | AGGAAAC |
| SEQ ID NO: 2563 | AGGAAAG |
| SEQ ID NO: 2564 | AGGAAAU |
| SEQ ID NO: 2565 | AGGAACA |
| SEQ ID NO: 2566 | AGGAACC |
| SEQ ID NO: 2567 | AGGAACG |
| SEQ ID NO: 2568 | AGGAACU |
| SEQ ID NO: 2569 | AGGAAGA |
| SEQ ID NO: 2570 | AGGAAGC |
| SEQ ID NO: 2571 | AGGAAGG |
| SEQ ID NO: 2572 | AGGAAGU |
| SEQ ID NO: 2573 | AGGAAUA |
| SEQ ID NO: 2574 | AGGAAUC |
| SEQ ID NO: 2575 | AGGAAUG |
| SEQ ID NO: 2576 | AGGAAUU |
| SEQ ID NO: 2577 | AGGACAA |
| SEQ ID NO: 2578 | AGGACAC |
| SEQ ID NO: 2579 | AGGACAG |
| SEQ ID NO: 2580 | AGGACAU |
| SEQ ID NO: 2581 | AGGACCA |
| SEQ ID NO: 2582 | AGGACCC |
| SEQ ID NO: 2583 | AGGACCG |
| SEQ ID NO: 2584 | AGGACCU |
| SEQ ID NO: 2585 | AGGACGA |
| SEQ ID NO: 2586 | AGGACGC |
| SEQ ID NO: 2587 | AGGACGG |
| SEQ ID NO: 2588 | AGGACGU |
| SEQ ID NO: 2589 | AGGACUA |
| SEQ ID NO: 2590 | AGGACUC |
| SEQ ID NO: 2591 | AGGACUG |
| SEQ ID NO: 2592 | AGGACUU |
| SEQ ID NO: 2593 | AGGAGAA |
| SEQ ID NO: 2594 | AGGAGAC |
| SEQ ID NO: 2595 | AGGAGAG |
| SEQ ID NO: 2596 | AGGAGAU |
| SEQ ID NO: 2597 | AGGAGCA |
| SEQ ID NO: 2598 | AGGAGCC |
| SEQ ID NO: 2599 | AGGAGCG |
| SEQ ID NO: 2600 | AGGAGCU |
| SEQ ID NO: 2601 | AGGAGGA |
| SEQ ID NO: 2602 | AGGAGGC |
| SEQ ID NO: 2603 | AGGAGGG |
| SEQ ID NO: 2604 | AGGAGGU |
| SEQ ID NO: 2605 | AGGAGUA |
| SEQ ID NO: 2606 | AGGAGUC |
| SEQ ID NO: 2607 | AGGAGUG |
| SEQ ID NO: 2608 | AGGAGUU |
| SEQ ID NO: 2609 | AGGAUAA |
| SEQ ID NO: 2610 | AGGAUAC |
| SEQ ID NO: 2611 | AGGAUAG |
| SEQ ID NO: 2612 | AGGAUAU |
| SEQ ID NO: 2613 | AGGAUCA |
| SEQ ID NO: 2614 | AGGAUCC |
| SEQ ID NO: 2615 | AGGAUCG |
| SEQ ID NO: 2616 | AGGAUCU |
| SEQ ID NO: 2617 | AGGAUGA |
| SEQ ID NO: 2618 | AGGAUGC |
| SEQ ID NO: 2619 | AGGAUGG |
| SEQ ID NO: 2620 | AGGAUGU |
| SEQ ID NO: 2621 | AGGAUUA |
| SEQ ID NO: 2622 | AGGAUUC |
| SEQ ID NO: 2623 | AGGAUUG |
| SEQ ID NO: 2624 | AGGAUUU |
| SEQ ID NO: 2625 | AGGCAAA |
| SEQ ID NO: 2626 | AGGCAAC |
| SEQ ID NO: 2627 | AGGCAAG |
| SEQ ID NO: 2628 | AGGCAAU |
| SEQ ID NO: 2629 | AGGCACA |
| SEQ ID NO: 2630 | AGGCACC |
| SEQ ID NO: 2631 | AGGCACG |
| SEQ ID NO: 2632 | AGGCACU |
| SEQ ID NO: 2633 | AGGCAGA |
| SEQ ID NO: 2634 | AGGCAGC |
| SEQ ID NO: 2635 | AGGCAGG |
| SEQ ID NO: 2636 | AGGCAGU |
| SEQ ID NO: 2637 | AGGCAUA |
| SEQ ID NO: 2638 | AGGCAUC |
| SEQ ID NO: 2639 | AGGCAUG |
| SEQ ID NO: 2640 | AGGCAUU |
| SEQ ID NO: 2641 | AGGCCAA |
| SEQ ID NO: 2642 | AGGCCAC |
| SEQ ID NO: 2643 | AGGCCAG |
| SEQ ID NO: 2644 | AGGCCAU |
| SEQ ID NO: 2645 | AGGCCCA |
| SEQ ID NO: 2646 | AGGCCCC |
| SEQ ID NO: 2647 | AGGCCCG |
| SEQ ID NO: 2648 | AGGCCCU |
| SEQ ID NO: 2649 | AGGCCGA |
| SEQ ID NO: 2650 | AGGCCGC |
| SEQ ID NO: 2651 | AGGCCGG |
| SEQ ID NO: 2652 | AGGCCGU |
| SEQ ID NO: 2653 | AGGCCUA |
| SEQ ID NO: 2654 | AGGCCUC |
| SEQ ID NO: 2655 | AGGCCUG |
| SEQ ID NO: 2656 | AGGCCUU |
| SEQ ID NO: 2657 | AGGCGAA |
| SEQ ID NO: 2658 | AGGCGAC |
| SEQ ID NO: 2659 | AGGCGAG |
| SEQ ID NO: 2660 | AGGCGAU |
| SEQ ID NO: 2661 | AGGCGCA |
| SEQ ID NO: 2662 | AGGCGCC |
| SEQ ID NO: 2663 | AGGCGCG |
| SEQ ID NO: 2664 | AGGCGCU |
| SEQ ID NO: 2665 | AGGCGGA |
| SEQ ID NO: 2666 | AGGCGGC |
| SEQ ID NO: 2667 | AGGCGGG |
| SEQ ID NO: 2668 | AGGCGGU |
| SEQ ID NO: 2669 | AGGCGUA |
| SEQ ID NO: 2670 | AGGCGUC |
| SEQ ID NO: 2671 | AGGCGUG |
| SEQ ID NO: 2672 | AGGCGUU |
| SEQ ID NO: 2673 | AGGCUAA |
| SEQ ID NO: 2674 | AGGCUAC |
| SEQ ID NO: 2675 | AGGCUAG |
| SEQ ID NO: 2676 | AGGCUAU |
| SEQ ID NO: 2677 | AGGCUCA |
| SEQ ID NO: 2678 | AGGCUCC |
| SEQ ID NO: 2679 | AGGCUCG |
| SEQ ID NO: 2680 | AGGCUCU |
| SEQ ID NO: 2681 | AGGCUGA |
| SEQ ID NO: 2682 | AGGCUGC |
| SEQ ID NO: 2683 | AGGCUGG |
| SEQ ID NO: 2684 | AGGCUGU |
| SEQ ID NO: 2685 | AGGCUUA |
| SEQ ID NO: 2686 | AGGCUUC |
| SEQ ID NO: 2687 | AGGCUUG |
| SEQ ID NO: 2688 | AGGCUUU |
| SEQ ID NO: 2689 | AGGGAAA |
| SEQ ID NO: 2690 | AGGGAAC |
| SEQ ID NO: 2691 | AGGGAAG |
| SEQ ID NO: 2692 | AGGGAAU |
| SEQ ID NO: 2693 | AGGGACA |
| SEQ ID NO: 2694 | AGGGACC |
| SEQ ID NO: 2695 | AGGGACG |
| SEQ ID NO: 2696 | AGGGACU |
| SEQ ID NO: 2697 | AGGGAGA |
| SEQ ID NO: 2698 | AGGGAGC |
| SEQ ID NO: 2699 | AGGGAGG |
| SEQ ID NO: 2700 | AGGGAGU |
| SEQ ID NO: 2701 | AGGGAUA |
| SEQ ID NO: 2702 | AGGGAUC |
| SEQ ID NO: 2703 | AGGGAUG |
| SEQ ID NO: 2704 | AGGGAUU |
| SEQ ID NO: 2705 | AGGGCAA |
| SEQ ID NO: 2706 | AGGGCAC |
| SEQ ID NO: 2707 | AGGGCAG |
| SEQ ID NO: 2708 | AGGGCAU |
| SEQ ID NO: 2709 | AGGGCCA |
| SEQ ID NO: 2710 | AGGGCCC |
| SEQ ID NO: 2711 | AGGGCCG |
| SEQ ID NO: 2712 | AGGGCCU |
| SEQ ID NO: 2713 | AGGGCGA |
| SEQ ID NO: 2714 | AGGGCGC |
| SEQ ID NO: 2715 | AGGGCGG |
| SEQ ID NO: 2716 | AGGGCGU |
| SEQ ID NO: 2717 | AGGGCUA |
| SEQ ID NO: 2718 | AGGGCUC |
| SEQ ID NO: 2719 | AGGGCUG |
| SEQ ID NO: 2720 | AGGGCUU |
| SEQ ID NO: 2721 | AGGGGAA |
| SEQ ID NO: 2722 | AGGGGAC |
| SEQ ID NO: 2723 | AGGGGAG |
| SEQ ID NO: 2724 | AGGGGAU |
| SEQ ID NO: 2725 | AGGGGCA |
| SEQ ID NO: 2726 | AGGGGCC |
| SEQ ID NO: 2727 | AGGGGCG |
| SEQ ID NO: 2728 | AGGGGCU |
| SEQ ID NO: 2729 | AGGGGGA |
| SEQ ID NO: 2730 | AGGGGGC |
| SEQ ID NO: 2731 | AGGGGGG |
| SEQ ID NO: 2732 | AGGGGGU |
| SEQ ID NO: 2733 | AGGGGUA |
| SEQ ID NO: 2734 | AGGGGUC |
| SEQ ID NO: 2735 | AGGGGUG |
| SEQ ID NO: 2736 | AGGGGUU |
| SEQ ID NO: 2737 | AGGGUAA |
| SEQ ID NO: 2738 | AGGGUAC |
| SEQ ID NO: 2739 | AGGGUAG |
| SEQ ID NO: 2740 | AGGGUAU |
| SEQ ID NO: 2741 | AGGGUCA |
| SEQ ID NO: 2742 | AGGGUCC |
| SEQ ID NO: 2743 | AGGGUCG |
| SEQ ID NO: 2744 | AGGGUCU |
| SEQ ID NO: 2745 | AGGGUGA |
| SEQ ID NO: 2746 | AGGGUGC |
| SEQ ID NO: 2747 | AGGGUGG |
| SEQ ID NO: 2748 | AGGGUGU |
| SEQ ID NO: 2749 | AGGGUUA |
| SEQ ID NO: 2750 | AGGGUUC |
| SEQ ID NO: 2751 | AGGGUUG |
| SEQ ID NO: 2752 | AGGGUUU |
| SEQ ID NO: 2753 | AGGUAAA |
| SEQ ID NO: 2754 | AGGUAAC |

Table 10

| | | | | | | | | | | | |
|---|---|---|---|---|---|---|---|---|---|---|---|
| SEQ ID NO: 2755 | AGGUAAG | SEQ ID NO: 2806 | AGGUUCC | SEQ ID NO: 2857 | AGUAGGA | SEQ ID NO: 2908 | AGUCCGU | SEQ ID NO: 2959 | AGUGAUG | SEQ ID NO: 3010 | AGUUAAC |
| SEQ ID NO: 2756 | AGGUAAU | SEQ ID NO: 2807 | AGGUUCG | SEQ ID NO: 2858 | AGUAGGC | SEQ ID NO: 2909 | AGUCCUA | SEQ ID NO: 2960 | AGUGAUU | SEQ ID NO: 3011 | AGUUAAG |
| SEQ ID NO: 2757 | AGGUACA | SEQ ID NO: 2808 | AGGUUCU | SEQ ID NO: 2859 | AGUAGGG | SEQ ID NO: 2910 | AGUCCUC | SEQ ID NO: 2961 | AGUGCAA | SEQ ID NO: 3012 | AGUUAAU |
| SEQ ID NO: 2758 | AGGUACC | SEQ ID NO: 2809 | AGGUUGA | SEQ ID NO: 2860 | AGUAGGU | SEQ ID NO: 2911 | AGUCCUG | SEQ ID NO: 2962 | AGUGCAC | SEQ ID NO: 3013 | AGUUACA |
| SEQ ID NO: 2759 | AGGUACG | SEQ ID NO: 2810 | AGGUUGC | SEQ ID NO: 2861 | AGUAGUA | SEQ ID NO: 2912 | AGUCCUU | SEQ ID NO: 2963 | AGUGCAG | SEQ ID NO: 3014 | AGUUACC |
| SEQ ID NO: 2760 | AGGUACU | SEQ ID NO: 2811 | AGGUUGG | SEQ ID NO: 2862 | AGUAGUC | SEQ ID NO: 2913 | AGUCGAA | SEQ ID NO: 2964 | AGUGCAU | SEQ ID NO: 3015 | AGUUACG |
| SEQ ID NO: 2761 | AGGUAGA | SEQ ID NO: 2812 | AGGUUGU | SEQ ID NO: 2863 | AGUAGUG | SEQ ID NO: 2914 | AGUCGAC | SEQ ID NO: 2965 | AGUGCCA | SEQ ID NO: 3016 | AGUUACU |
| SEQ ID NO: 2762 | AGGUAGC | SEQ ID NO: 2813 | AGGUUUA | SEQ ID NO: 2864 | AGUAGUU | SEQ ID NO: 2915 | AGUCGAG | SEQ ID NO: 2966 | AGUGCCC | SEQ ID NO: 3017 | AGUUAGA |
| SEQ ID NO: 2763 | AGGUAGG | SEQ ID NO: 2814 | AGGUUUC | SEQ ID NO: 2865 | AGUAUAA | SEQ ID NO: 2916 | AGUCGAU | SEQ ID NO: 2967 | AGUGCCG | SEQ ID NO: 3018 | AGUUAGC |
| SEQ ID NO: 2764 | AGGUAGU | SEQ ID NO: 2815 | AGGUUUG | SEQ ID NO: 2866 | AGUAUAC | SEQ ID NO: 2917 | AGUCGCA | SEQ ID NO: 2968 | AGUGCCU | SEQ ID NO: 3019 | AGUUAGG |
| SEQ ID NO: 2765 | AGGUAUA | SEQ ID NO: 2816 | AGGUUUU | SEQ ID NO: 2867 | AGUAUAG | SEQ ID NO: 2918 | AGUCGCC | SEQ ID NO: 2969 | AGUGCGA | SEQ ID NO: 3020 | AGUUAGU |
| SEQ ID NO: 2766 | AGGUAUC | SEQ ID NO: 2817 | AGUAAAA | SEQ ID NO: 2868 | AGUAUAU | SEQ ID NO: 2919 | AGUCGCG | SEQ ID NO: 2970 | AGUGCGC | SEQ ID NO: 3021 | AGUUAUA |
| SEQ ID NO: 2767 | AGGUAUG | SEQ ID NO: 2818 | AGUAAAC | SEQ ID NO: 2869 | AGUAUCA | SEQ ID NO: 2920 | AGUCGCU | SEQ ID NO: 2971 | AGUGCGG | SEQ ID NO: 3022 | AGUUAUC |
| SEQ ID NO: 2768 | AGGUAUU | SEQ ID NO: 2819 | AGUAAAG | SEQ ID NO: 2870 | AGUAUCC | SEQ ID NO: 2921 | AGUCGGA | SEQ ID NO: 2972 | AGUGCGU | SEQ ID NO: 3023 | AGUUAUG |
| SEQ ID NO: 2769 | AGGUCAA | SEQ ID NO: 2820 | AGUAAAU | SEQ ID NO: 2871 | AGUAUCG | SEQ ID NO: 2922 | AGUCGGC | SEQ ID NO: 2973 | AGUGCUA | SEQ ID NO: 3024 | AGUUAUU |
| SEQ ID NO: 2770 | AGGUCAC | SEQ ID NO: 2821 | AGUAACA | SEQ ID NO: 2872 | AGUAUCU | SEQ ID NO: 2923 | AGUCGGG | SEQ ID NO: 2974 | AGUGCUC | SEQ ID NO: 3025 | AGUUCAA |
| SEQ ID NO: 2771 | AGGUCAG | SEQ ID NO: 2822 | AGUAACC | SEQ ID NO: 2873 | AGUAUGA | SEQ ID NO: 2924 | AGUCGGU | SEQ ID NO: 2975 | AGUGCUG | SEQ ID NO: 3026 | AGUUCAC |
| SEQ ID NO: 2772 | AGGUCAU | SEQ ID NO: 2823 | AGUAACG | SEQ ID NO: 2874 | AGUAUGC | SEQ ID NO: 2925 | AGUCGUA | SEQ ID NO: 2976 | AGUGCUU | SEQ ID NO: 3027 | AGUUCAG |
| SEQ ID NO: 2773 | AGGUCCA | SEQ ID NO: 2824 | AGUAACU | SEQ ID NO: 2875 | AGUAUGG | SEQ ID NO: 2926 | AGUCGUC | SEQ ID NO: 2977 | AGUGGAA | SEQ ID NO: 3028 | AGUUCAU |
| SEQ ID NO: 2774 | AGGUCCC | SEQ ID NO: 2825 | AGUAAGA | SEQ ID NO: 2876 | AGUAUGU | SEQ ID NO: 2927 | AGUCGUG | SEQ ID NO: 2978 | AGUGGAC | SEQ ID NO: 3029 | AGUUCCA |
| SEQ ID NO: 2775 | AGGUCCG | SEQ ID NO: 2826 | AGUAAGC | SEQ ID NO: 2877 | AGUAUUA | SEQ ID NO: 2928 | AGUCGUU | SEQ ID NO: 2979 | AGUGGAG | SEQ ID NO: 3030 | AGUUCCC |
| SEQ ID NO: 2776 | AGGUCCU | SEQ ID NO: 2827 | AGUAAGG | SEQ ID NO: 2878 | AGUAUUC | SEQ ID NO: 2929 | AGUCUAA | SEQ ID NO: 2980 | AGUGGAU | SEQ ID NO: 3031 | AGUUCCG |
| SEQ ID NO: 2777 | AGGUCGA | SEQ ID NO: 2828 | AGUAAGU | SEQ ID NO: 2879 | AGUAUUG | SEQ ID NO: 2930 | AGUCUAC | SEQ ID NO: 2981 | AGUGGCA | SEQ ID NO: 3032 | AGUUCCU |
| SEQ ID NO: 2778 | AGGUCGC | SEQ ID NO: 2829 | AGUAAUA | SEQ ID NO: 2880 | AGUAUUU | SEQ ID NO: 2931 | AGUCUAG | SEQ ID NO: 2982 | AGUGGCC | SEQ ID NO: 3033 | AGUUCGA |
| SEQ ID NO: 2779 | AGGUCGG | SEQ ID NO: 2830 | AGUAAUC | SEQ ID NO: 2881 | AGUCAAA | SEQ ID NO: 2932 | AGUCUAU | SEQ ID NO: 2983 | AGUGGCG | SEQ ID NO: 3034 | AGUUCGC |
| SEQ ID NO: 2780 | AGGUCGU | SEQ ID NO: 2831 | AGUAAUG | SEQ ID NO: 2882 | AGUCAAC | SEQ ID NO: 2933 | AGUCUCA | SEQ ID NO: 2984 | AGUGGCU | SEQ ID NO: 3035 | AGUUCGG |
| SEQ ID NO: 2781 | AGGUCUA | SEQ ID NO: 2832 | AGUAAUU | SEQ ID NO: 2883 | AGUCAAG | SEQ ID NO: 2934 | AGUCUCC | SEQ ID NO: 2985 | AGUGGGA | SEQ ID NO: 3036 | AGUUCGU |
| SEQ ID NO: 2782 | AGGUCUC | SEQ ID NO: 2833 | AGUACAA | SEQ ID NO: 2884 | AGUCAAU | SEQ ID NO: 2935 | AGUCUCG | SEQ ID NO: 2986 | AGUGGGC | SEQ ID NO: 3037 | AGUUCUA |
| SEQ ID NO: 2783 | AGGUCUG | SEQ ID NO: 2834 | AGUACAC | SEQ ID NO: 2885 | AGUCACA | SEQ ID NO: 2936 | AGUCUCU | SEQ ID NO: 2987 | AGUGGGG | SEQ ID NO: 3038 | AGUUCUC |
| SEQ ID NO: 2784 | AGGUCUU | SEQ ID NO: 2835 | AGUACAG | SEQ ID NO: 2886 | AGUCACC | SEQ ID NO: 2937 | AGUCUGA | SEQ ID NO: 2988 | AGUGGGU | SEQ ID NO: 3039 | AGUUCUG |
| SEQ ID NO: 2785 | AGGUGAA | SEQ ID NO: 2836 | AGUACAU | SEQ ID NO: 2887 | AGUCACG | SEQ ID NO: 2938 | AGUCUGC | SEQ ID NO: 2989 | AGUGGUA | SEQ ID NO: 3040 | AGUUCUU |
| SEQ ID NO: 2786 | AGGUGAC | SEQ ID NO: 2837 | AGUACCA | SEQ ID NO: 2888 | AGUCACU | SEQ ID NO: 2939 | AGUCUGG | SEQ ID NO: 2990 | AGUGGUC | SEQ ID NO: 3041 | AGUUGAA |
| SEQ ID NO: 2787 | AGGUGAG | SEQ ID NO: 2838 | AGUACCC | SEQ ID NO: 2889 | AGUCAGA | SEQ ID NO: 2940 | AGUCUGU | SEQ ID NO: 2991 | AGUGGUG | SEQ ID NO: 3042 | AGUUGAC |
| SEQ ID NO: 2788 | AGGUGAU | SEQ ID NO: 2839 | AGUACCG | SEQ ID NO: 2890 | AGUCAGC | SEQ ID NO: 2941 | AGUCUUA | SEQ ID NO: 2992 | AGUGGUU | SEQ ID NO: 3043 | AGUUGAG |
| SEQ ID NO: 2789 | AGGUGCA | SEQ ID NO: 2840 | AGUACCU | SEQ ID NO: 2891 | AGUCAGG | SEQ ID NO: 2942 | AGUCUUC | SEQ ID NO: 2993 | AGUGUAA | SEQ ID NO: 3044 | AGUUGAU |
| SEQ ID NO: 2790 | AGGUGCC | SEQ ID NO: 2841 | AGUACGA | SEQ ID NO: 2892 | AGUCAGU | SEQ ID NO: 2943 | AGUCUUG | SEQ ID NO: 2994 | AGUGUAC | SEQ ID NO: 3045 | AGUUGCA |
| SEQ ID NO: 2791 | AGGUGCG | SEQ ID NO: 2842 | AGUACGC | SEQ ID NO: 2893 | AGUCAUA | SEQ ID NO: 2944 | AGUCUUU | SEQ ID NO: 2995 | AGUGUAG | SEQ ID NO: 3046 | AGUUGCC |
| SEQ ID NO: 2792 | AGGUGCU | SEQ ID NO: 2843 | AGUACGG | SEQ ID NO: 2894 | AGUCAUC | SEQ ID NO: 2945 | AGUGAAA | SEQ ID NO: 2996 | AGUGUAU | SEQ ID NO: 3047 | AGUUGCG |
| SEQ ID NO: 2793 | AGGUGGA | SEQ ID NO: 2844 | AGUACGU | SEQ ID NO: 2895 | AGUCAUG | SEQ ID NO: 2946 | AGUGAAC | SEQ ID NO: 2997 | AGUGUCA | SEQ ID NO: 3048 | AGUUGCU |
| SEQ ID NO: 2794 | AGGUGGC | SEQ ID NO: 2845 | AGUACUA | SEQ ID NO: 2896 | AGUCAUU | SEQ ID NO: 2947 | AGUGAAG | SEQ ID NO: 2998 | AGUGUCC | SEQ ID NO: 3049 | AGUUGGA |
| SEQ ID NO: 2795 | AGGUGGG | SEQ ID NO: 2846 | AGUACUC | SEQ ID NO: 2897 | AGUCCAA | SEQ ID NO: 2948 | AGUGAAU | SEQ ID NO: 2999 | AGUGUCG | SEQ ID NO: 3050 | AGUUGGC |
| SEQ ID NO: 2796 | AGGUGGU | SEQ ID NO: 2847 | AGUACUG | SEQ ID NO: 2898 | AGUCCAC | SEQ ID NO: 2949 | AGUGACA | SEQ ID NO: 3000 | AGUGUCU | SEQ ID NO: 3051 | AGUUGGG |
| SEQ ID NO: 2797 | AGGUGUA | SEQ ID NO: 2848 | AGUACUU | SEQ ID NO: 2899 | AGUCCAG | SEQ ID NO: 2950 | AGUGACC | SEQ ID NO: 3001 | AGUGUGA | SEQ ID NO: 3052 | AGUUGGU |
| SEQ ID NO: 2798 | AGGUGUC | SEQ ID NO: 2849 | AGUAGAA | SEQ ID NO: 2900 | AGUCCAU | SEQ ID NO: 2951 | AGUGACG | SEQ ID NO: 3002 | AGUGUGC | SEQ ID NO: 3053 | AGUUGUA |
| SEQ ID NO: 2799 | AGGUGUG | SEQ ID NO: 2850 | AGUAGAC | SEQ ID NO: 2901 | AGUCCCA | SEQ ID NO: 2952 | AGUGACU | SEQ ID NO: 3003 | AGUGUGG | SEQ ID NO: 3054 | AGUUGUC |
| SEQ ID NO: 2800 | AGGUGUU | SEQ ID NO: 2851 | AGUAGAG | SEQ ID NO: 2902 | AGUCCCC | SEQ ID NO: 2953 | AGUGAGA | SEQ ID NO: 3004 | AGUGUGU | SEQ ID NO: 3055 | AGUUGUG |
| SEQ ID NO: 2801 | AGGUUAA | SEQ ID NO: 2852 | AGUAGAU | SEQ ID NO: 2903 | AGUCCCG | SEQ ID NO: 2954 | AGUGAGC | SEQ ID NO: 3005 | AGUGUUA | SEQ ID NO: 3056 | AGUUGUU |
| SEQ ID NO: 2802 | AGGUUAC | SEQ ID NO: 2853 | AGUAGCA | SEQ ID NO: 2904 | AGUCCCU | SEQ ID NO: 2955 | AGUGAGG | SEQ ID NO: 3006 | AGUGUUC | SEQ ID NO: 3057 | AGUUUAA |
| SEQ ID NO: 2803 | AGGUUAG | SEQ ID NO: 2854 | AGUAGCC | SEQ ID NO: 2905 | AGUCCGA | SEQ ID NO: 2956 | AGUGAGU | SEQ ID NO: 3007 | AGUGUUG | SEQ ID NO: 3058 | AGUUUAC |
| SEQ ID NO: 2804 | AGGUUAU | SEQ ID NO: 2855 | AGUAGCG | SEQ ID NO: 2906 | AGUCCGC | SEQ ID NO: 2957 | AGUGAUA | SEQ ID NO: 3008 | AGUGUUU | SEQ ID NO: 3059 | AGUUUAG |
| SEQ ID NO: 2805 | AGGUUCA | SEQ ID NO: 2856 | AGUAGCU | SEQ ID NO: 2907 | AGUCCGG | SEQ ID NO: 2958 | AGUGAUC | SEQ ID NO: 3009 | AGUUAAA | SEQ ID NO: 3060 | AGUUUAU |

Table 11

| | | | | | | | | | | | |
|---|---|---|---|---|---|---|---|---|---|---|---|
| SEQ ID NO: 3061 | AGUUUCA | SEQ ID NO: 3112 | AUAAGCU | SEQ ID NO: 3163 | AUACCGG | SEQ ID NO: 3214 | AUAGAUC | SEQ ID NO: 3265 | AUAUAAA | SEQ ID NO: 3316 | AUAUUAU |
| SEQ ID NO: 3062 | AGUUUCC | SEQ ID NO: 3113 | AUAAGGA | SEQ ID NO: 3164 | AUACCGU | SEQ ID NO: 3215 | AUAGAUG | SEQ ID NO: 3266 | AUAUAAC | SEQ ID NO: 3317 | AUAUUCA |
| SEQ ID NO: 3063 | AGUUUCG | SEQ ID NO: 3114 | AUAAGGC | SEQ ID NO: 3165 | AUACCUA | SEQ ID NO: 3216 | AUAGAUU | SEQ ID NO: 3267 | AUAUAAG | SEQ ID NO: 3318 | AUAUUCC |
| SEQ ID NO: 3064 | AGUUUCU | SEQ ID NO: 3115 | AUAAGGG | SEQ ID NO: 3166 | AUACCUC | SEQ ID NO: 3217 | AUAGCAA | SEQ ID NO: 3268 | AUAUAAU | SEQ ID NO: 3319 | AUAUUCG |
| SEQ ID NO: 3065 | AGUUUGA | SEQ ID NO: 3116 | AUAAGGU | SEQ ID NO: 3167 | AUACCUG | SEQ ID NO: 3218 | AUAGCAC | SEQ ID NO: 3269 | AUAUACA | SEQ ID NO: 3320 | AUAUUCU |
| SEQ ID NO: 3066 | AGUUUGC | SEQ ID NO: 3117 | AUAAGUA | SEQ ID NO: 3168 | AUACCUU | SEQ ID NO: 3219 | AUAGCAG | SEQ ID NO: 3270 | AUAUACC | SEQ ID NO: 3321 | AUAUUGA |
| SEQ ID NO: 3067 | AGUUUGG | SEQ ID NO: 3118 | AUAAGUC | SEQ ID NO: 3169 | AUACGAA | SEQ ID NO: 3220 | AUAGCAU | SEQ ID NO: 3271 | AUAUACG | SEQ ID NO: 3322 | AUAUUGC |
| SEQ ID NO: 3068 | AGUUUGU | SEQ ID NO: 3119 | AUAAGUG | SEQ ID NO: 3170 | AUACGAC | SEQ ID NO: 3221 | AUAGCCA | SEQ ID NO: 3272 | AUAUACU | SEQ ID NO: 3323 | AUAUUGG |
| SEQ ID NO: 3069 | AGUUUUA | SEQ ID NO: 3120 | AUAAGUU | SEQ ID NO: 3171 | AUACGAG | SEQ ID NO: 3222 | AUAGCCC | SEQ ID NO: 3273 | AUAUAGA | SEQ ID NO: 3324 | AUAUUGU |
| SEQ ID NO: 3070 | AGUUUUC | SEQ ID NO: 3121 | AUAAUAA | SEQ ID NO: 3172 | AUACGAU | SEQ ID NO: 3223 | AUAGCCG | SEQ ID NO: 3274 | AUAUAGC | SEQ ID NO: 3325 | AUAUUUA |
| SEQ ID NO: 3071 | AGUUUUG | SEQ ID NO: 3122 | AUAAUAC | SEQ ID NO: 3173 | AUACGCA | SEQ ID NO: 3224 | AUAGCCU | SEQ ID NO: 3275 | AUAUAGG | SEQ ID NO: 3326 | AUAUUUC |
| SEQ ID NO: 3072 | AGUUUUU | SEQ ID NO: 3123 | AUAAUAG | SEQ ID NO: 3174 | AUACGCC | SEQ ID NO: 3225 | AUAGCGA | SEQ ID NO: 3276 | AUAUAGU | SEQ ID NO: 3327 | AUAUUUG |
| SEQ ID NO: 3073 | AUAAAAA | SEQ ID NO: 3124 | AUAAUAU | SEQ ID NO: 3175 | AUACGCG | SEQ ID NO: 3226 | AUAGCGC | SEQ ID NO: 3277 | AUAUAUA | SEQ ID NO: 3328 | AUAUUUU |
| SEQ ID NO: 3074 | AUAAAAC | SEQ ID NO: 3125 | AUAAUCA | SEQ ID NO: 3176 | AUACGCU | SEQ ID NO: 3227 | AUAGCGG | SEQ ID NO: 3278 | AUAUAUC | SEQ ID NO: 3329 | AUCAAAA |
| SEQ ID NO: 3075 | AUAAAAG | SEQ ID NO: 3126 | AUAAUCC | SEQ ID NO: 3177 | AUACGGA | SEQ ID NO: 3228 | AUAGCGU | SEQ ID NO: 3279 | AUAUAUG | SEQ ID NO: 3330 | AUCAAAC |
| SEQ ID NO: 3076 | AUAAAAU | SEQ ID NO: 3127 | AUAAUCG | SEQ ID NO: 3178 | AUACGGC | SEQ ID NO: 3229 | AUAGCUA | SEQ ID NO: 3280 | AUAUAUU | SEQ ID NO: 3331 | AUCAAAG |
| SEQ ID NO: 3077 | AUAAACA | SEQ ID NO: 3128 | AUAAUCU | SEQ ID NO: 3179 | AUACGGG | SEQ ID NO: 3230 | AUAGCUC | SEQ ID NO: 3281 | AUAUCAA | SEQ ID NO: 3332 | AUCAAAU |
| SEQ ID NO: 3078 | AUAAACC | SEQ ID NO: 3129 | AUAAUGA | SEQ ID NO: 3180 | AUACGGU | SEQ ID NO: 3231 | AUAGCUG | SEQ ID NO: 3282 | AUAUCAC | SEQ ID NO: 3333 | AUCAACA |
| SEQ ID NO: 3079 | AUAAACG | SEQ ID NO: 3130 | AUAAUGC | SEQ ID NO: 3181 | AUACGUA | SEQ ID NO: 3232 | AUAGCUU | SEQ ID NO: 3283 | AUAUCAG | SEQ ID NO: 3334 | AUCAACC |
| SEQ ID NO: 3080 | AUAAACU | SEQ ID NO: 3131 | AUAAUGG | SEQ ID NO: 3182 | AUACGUC | SEQ ID NO: 3233 | AUAGGAA | SEQ ID NO: 3284 | AUAUCAU | SEQ ID NO: 3335 | AUCAACG |
| SEQ ID NO: 3081 | AUAAAGA | SEQ ID NO: 3132 | AUAAUGU | SEQ ID NO: 3183 | AUACGUG | SEQ ID NO: 3234 | AUAGGAC | SEQ ID NO: 3285 | AUAUCCA | SEQ ID NO: 3336 | AUCAACU |
| SEQ ID NO: 3082 | AUAAAGC | SEQ ID NO: 3133 | AUAAUUA | SEQ ID NO: 3184 | AUACGUU | SEQ ID NO: 3235 | AUAGGAG | SEQ ID NO: 3286 | AUAUCCC | SEQ ID NO: 3337 | AUCAAGA |
| SEQ ID NO: 3083 | AUAAAGG | SEQ ID NO: 3134 | AUAAUUC | SEQ ID NO: 3185 | AUACUAA | SEQ ID NO: 3236 | AUAGGAU | SEQ ID NO: 3287 | AUAUCCG | SEQ ID NO: 3338 | AUCAAGC |
| SEQ ID NO: 3084 | AUAAAGU | SEQ ID NO: 3135 | AUAAUUG | SEQ ID NO: 3186 | AUACUAC | SEQ ID NO: 3237 | AUAGGCA | SEQ ID NO: 3288 | AUAUCCU | SEQ ID NO: 3339 | AUCAAGG |
| SEQ ID NO: 3085 | AUAAAUA | SEQ ID NO: 3136 | AUAAUUU | SEQ ID NO: 3187 | AUACUAG | SEQ ID NO: 3238 | AUAGGCC | SEQ ID NO: 3289 | AUAUCGA | SEQ ID NO: 3340 | AUCAAGU |
| SEQ ID NO: 3086 | AUAAAUC | SEQ ID NO: 3137 | AUACAAA | SEQ ID NO: 3188 | AUACUAU | SEQ ID NO: 3239 | AUAGGCG | SEQ ID NO: 3290 | AUAUCGC | SEQ ID NO: 3341 | AUCAAUA |
| SEQ ID NO: 3087 | AUAAAUG | SEQ ID NO: 3138 | AUACAAC | SEQ ID NO: 3189 | AUACUCA | SEQ ID NO: 3240 | AUAGGCU | SEQ ID NO: 3291 | AUAUCGG | SEQ ID NO: 3342 | AUCAAUC |
| SEQ ID NO: 3088 | AUAAAUU | SEQ ID NO: 3139 | AUACAAG | SEQ ID NO: 3190 | AUACUCC | SEQ ID NO: 3241 | AUAGGGA | SEQ ID NO: 3292 | AUAUCGU | SEQ ID NO: 3343 | AUCAAUG |
| SEQ ID NO: 3089 | AUAACAA | SEQ ID NO: 3140 | AUACAAU | SEQ ID NO: 3191 | AUACUCG | SEQ ID NO: 3242 | AUAGGGC | SEQ ID NO: 3293 | AUAUCUA | SEQ ID NO: 3344 | AUCAAUU |
| SEQ ID NO: 3090 | AUAACAC | SEQ ID NO: 3141 | AUACACA | SEQ ID NO: 3192 | AUACUCU | SEQ ID NO: 3243 | AUAGGGG | SEQ ID NO: 3294 | AUAUCUC | SEQ ID NO: 3345 | AUCACAA |
| SEQ ID NO: 3091 | AUAACAG | SEQ ID NO: 3142 | AUACACC | SEQ ID NO: 3193 | AUACUGA | SEQ ID NO: 3244 | AUAGGGU | SEQ ID NO: 3295 | AUAUCUG | SEQ ID NO: 3346 | AUCACAC |
| SEQ ID NO: 3092 | AUAACAU | SEQ ID NO: 3143 | AUACACG | SEQ ID NO: 3194 | AUACUGC | SEQ ID NO: 3245 | AUAGGUA | SEQ ID NO: 3296 | AUAUCUU | SEQ ID NO: 3347 | AUCACAG |
| SEQ ID NO: 3093 | AUAACCA | SEQ ID NO: 3144 | AUACACU | SEQ ID NO: 3195 | AUACUGG | SEQ ID NO: 3246 | AUAGGUC | SEQ ID NO: 3297 | AUAUGAA | SEQ ID NO: 3348 | AUCACAU |
| SEQ ID NO: 3094 | AUAACCC | SEQ ID NO: 3145 | AUACAGA | SEQ ID NO: 3196 | AUACUGU | SEQ ID NO: 3247 | AUAGGUG | SEQ ID NO: 3298 | AUAUGAC | SEQ ID NO: 3349 | AUCACCA |
| SEQ ID NO: 3095 | AUAACCG | SEQ ID NO: 3146 | AUACAGC | SEQ ID NO: 3197 | AUACUUA | SEQ ID NO: 3248 | AUAGGUU | SEQ ID NO: 3299 | AUAUGAG | SEQ ID NO: 3350 | AUCACCC |
| SEQ ID NO: 3096 | AUAACCU | SEQ ID NO: 3147 | AUACAGG | SEQ ID NO: 3198 | AUACUUC | SEQ ID NO: 3249 | AUAGUAA | SEQ ID NO: 3300 | AUAUGAU | SEQ ID NO: 3351 | AUCACCG |
| SEQ ID NO: 3097 | AUAACGA | SEQ ID NO: 3148 | AUACAGU | SEQ ID NO: 3199 | AUACUUG | SEQ ID NO: 3250 | AUAGUAC | SEQ ID NO: 3301 | AUAUGCA | SEQ ID NO: 3352 | AUCACCU |
| SEQ ID NO: 3098 | AUAACGC | SEQ ID NO: 3149 | AUACAUA | SEQ ID NO: 3200 | AUACUUU | SEQ ID NO: 3251 | AUAGUAG | SEQ ID NO: 3302 | AUAUGCC | SEQ ID NO: 3353 | AUCACGA |
| SEQ ID NO: 3099 | AUAACGG | SEQ ID NO: 3150 | AUACAUC | SEQ ID NO: 3201 | AUAGAAA | SEQ ID NO: 3252 | AUAGUAU | SEQ ID NO: 3303 | AUAUGCG | SEQ ID NO: 3354 | AUCACGC |
| SEQ ID NO: 3100 | AUAACGU | SEQ ID NO: 3151 | AUACAUG | SEQ ID NO: 3202 | AUAGAAC | SEQ ID NO: 3253 | AUAGUCA | SEQ ID NO: 3304 | AUAUGCU | SEQ ID NO: 3355 | AUCACGG |
| SEQ ID NO: 3101 | AUAACUA | SEQ ID NO: 3152 | AUACAUU | SEQ ID NO: 3203 | AUAGAAG | SEQ ID NO: 3254 | AUAGUCC | SEQ ID NO: 3305 | AUAUGGA | SEQ ID NO: 3356 | AUCACGU |
| SEQ ID NO: 3102 | AUAACUC | SEQ ID NO: 3153 | AUACCAA | SEQ ID NO: 3204 | AUAGAAU | SEQ ID NO: 3255 | AUAGUCG | SEQ ID NO: 3306 | AUAUGGC | SEQ ID NO: 3357 | AUCACUA |
| SEQ ID NO: 3103 | AUAACUG | SEQ ID NO: 3154 | AUACCAC | SEQ ID NO: 3205 | AUAGACA | SEQ ID NO: 3256 | AUAGUCU | SEQ ID NO: 3307 | AUAUGGG | SEQ ID NO: 3358 | AUCACUC |
| SEQ ID NO: 3104 | AUAACUU | SEQ ID NO: 3155 | AUACCAG | SEQ ID NO: 3206 | AUAGACC | SEQ ID NO: 3257 | AUAGUGA | SEQ ID NO: 3308 | AUAUGGU | SEQ ID NO: 3359 | AUCACUG |
| SEQ ID NO: 3105 | AUAAGAA | SEQ ID NO: 3156 | AUACCAU | SEQ ID NO: 3207 | AUAGACG | SEQ ID NO: 3258 | AUAGUGC | SEQ ID NO: 3309 | AUAUGUA | SEQ ID NO: 3360 | AUCACUU |
| SEQ ID NO: 3106 | AUAAGAC | SEQ ID NO: 3157 | AUACCCA | SEQ ID NO: 3208 | AUAGACU | SEQ ID NO: 3259 | AUAGUGG | SEQ ID NO: 3310 | AUAUGUC | SEQ ID NO: 3361 | AUCAGAA |
| SEQ ID NO: 3107 | AUAAGAG | SEQ ID NO: 3158 | AUACCCC | SEQ ID NO: 3209 | AUAGAGA | SEQ ID NO: 3260 | AUAGUGU | SEQ ID NO: 3311 | AUAUGUG | SEQ ID NO: 3362 | AUCAGAC |
| SEQ ID NO: 3108 | AUAAGAU | SEQ ID NO: 3159 | AUACCCG | SEQ ID NO: 3210 | AUAGAGC | SEQ ID NO: 3261 | AUAGUUA | SEQ ID NO: 3312 | AUAUGUU | SEQ ID NO: 3363 | AUCAGAG |
| SEQ ID NO: 3109 | AUAAGCA | SEQ ID NO: 3160 | AUACCCU | SEQ ID NO: 3211 | AUAGAGG | SEQ ID NO: 3262 | AUAGUUC | SEQ ID NO: 3313 | AUAUUAA | SEQ ID NO: 3364 | AUCAGAU |
| SEQ ID NO: 3110 | AUAAGCC | SEQ ID NO: 3161 | AUACCGA | SEQ ID NO: 3212 | AUAGAGU | SEQ ID NO: 3263 | AUAGUUG | SEQ ID NO: 3314 | AUAUUAC | SEQ ID NO: 3365 | AUCAGCA |
| SEQ ID NO: 3111 | AUAAGCG | SEQ ID NO: 3162 | AUACCGC | SEQ ID NO: 3213 | AUAGAUA | SEQ ID NO: 3264 | AUAGUUU | SEQ ID NO: 3315 | AUAUUAG | SEQ ID NO: 3366 | AUCAGCC |

Table 12

| | | | | | | | | | | | |
|---|---|---|---|---|---|---|---|---|---|---|---|
| SEQ ID NO: 3367 | AUCAGCG | SEQ ID NO: 3418 | AUCCCGC | SEQ ID NO: 3469 | AUCGAUA | SEQ ID NO: 3520 | AUCGUUU | SEQ ID NO: 3571 | AUCUUAG | SEQ ID NO: 3622 | AUGAGCC |
| SEQ ID NO: 3368 | AUCAGCU | SEQ ID NO: 3419 | AUCCCGG | SEQ ID NO: 3470 | AUCGAUC | SEQ ID NO: 3521 | AUCUAAA | SEQ ID NO: 3572 | AUCUUAU | SEQ ID NO: 3623 | AUGAGCG |
| SEQ ID NO: 3369 | AUCAGGA | SEQ ID NO: 3420 | AUCCCGU | SEQ ID NO: 3471 | AUCGAUG | SEQ ID NO: 3522 | AUCUAAC | SEQ ID NO: 3573 | AUCUUCA | SEQ ID NO: 3624 | AUGAGCU |
| SEQ ID NO: 3370 | AUCAGGC | SEQ ID NO: 3421 | AUCCCUA | SEQ ID NO: 3472 | AUCGAUU | SEQ ID NO: 3523 | AUCUAAG | SEQ ID NO: 3574 | AUCUUCC | SEQ ID NO: 3625 | AUGAGGA |
| SEQ ID NO: 3371 | AUCAGGG | SEQ ID NO: 3422 | AUCCCUC | SEQ ID NO: 3473 | AUCGCAA | SEQ ID NO: 3524 | AUCUAAU | SEQ ID NO: 3575 | AUCUUCG | SEQ ID NO: 3626 | AUGAGGC |
| SEQ ID NO: 3372 | AUCAGGU | SEQ ID NO: 3423 | AUCCCUG | SEQ ID NO: 3474 | AUCGCAC | SEQ ID NO: 3525 | AUCUACA | SEQ ID NO: 3576 | AUCUUCU | SEQ ID NO: 3627 | AUGAGGG |
| SEQ ID NO: 3373 | AUCAGUA | SEQ ID NO: 3424 | AUCCCUU | SEQ ID NO: 3475 | AUCGCAG | SEQ ID NO: 3526 | AUCUACC | SEQ ID NO: 3577 | AUCUUGA | SEQ ID NO: 3628 | AUGAGGU |
| SEQ ID NO: 3374 | AUCAGUC | SEQ ID NO: 3425 | AUCCGAA | SEQ ID NO: 3476 | AUCGCAU | SEQ ID NO: 3527 | AUCUACG | SEQ ID NO: 3578 | AUCUUGC | SEQ ID NO: 3629 | AUGAGUA |
| SEQ ID NO: 3375 | AUCAGUG | SEQ ID NO: 3426 | AUCCGAC | SEQ ID NO: 3477 | AUCGCCA | SEQ ID NO: 3528 | AUCUACU | SEQ ID NO: 3579 | AUCUUGG | SEQ ID NO: 3630 | AUGAGUC |
| SEQ ID NO: 3376 | AUCAGUU | SEQ ID NO: 3427 | AUCCGAG | SEQ ID NO: 3478 | AUCGCCC | SEQ ID NO: 3529 | AUCUAGA | SEQ ID NO: 3580 | AUCUUGU | SEQ ID NO: 3631 | AUGAGUG |
| SEQ ID NO: 3377 | AUCAUAA | SEQ ID NO: 3428 | AUCCGAU | SEQ ID NO: 3479 | AUCGCCG | SEQ ID NO: 3530 | AUCUAGC | SEQ ID NO: 3581 | AUCUUUA | SEQ ID NO: 3632 | AUGAGUU |
| SEQ ID NO: 3378 | AUCAUAC | SEQ ID NO: 3429 | AUCCGCA | SEQ ID NO: 3480 | AUCGCCU | SEQ ID NO: 3531 | AUCUAGG | SEQ ID NO: 3582 | AUCUUUC | SEQ ID NO: 3633 | AUGAUAA |
| SEQ ID NO: 3379 | AUCAUAG | SEQ ID NO: 3430 | AUCCGCC | SEQ ID NO: 3481 | AUCGCGA | SEQ ID NO: 3532 | AUCUAGU | SEQ ID NO: 3583 | AUCUUUG | SEQ ID NO: 3634 | AUGAUAC |
| SEQ ID NO: 3380 | AUCAUAU | SEQ ID NO: 3431 | AUCCGCG | SEQ ID NO: 3482 | AUCGCGC | SEQ ID NO: 3533 | AUCUAUA | SEQ ID NO: 3584 | AUCUUUU | SEQ ID NO: 3635 | AUGAUAG |
| SEQ ID NO: 3381 | AUCAUCA | SEQ ID NO: 3432 | AUCCGCU | SEQ ID NO: 3483 | AUCGCGG | SEQ ID NO: 3534 | AUCUAUC | SEQ ID NO: 3585 | AUGAAAA | SEQ ID NO: 3636 | AUGAUAU |
| SEQ ID NO: 3382 | AUCAUCC | SEQ ID NO: 3433 | AUCCGGA | SEQ ID NO: 3484 | AUCGCGU | SEQ ID NO: 3535 | AUCUAUG | SEQ ID NO: 3586 | AUGAAAC | SEQ ID NO: 3637 | AUGAUCA |
| SEQ ID NO: 3383 | AUCAUCG | SEQ ID NO: 3434 | AUCCGGC | SEQ ID NO: 3485 | AUCGCUA | SEQ ID NO: 3536 | AUCUAUU | SEQ ID NO: 3587 | AUGAAAG | SEQ ID NO: 3638 | AUGAUCC |
| SEQ ID NO: 3384 | AUCAUCU | SEQ ID NO: 3435 | AUCCGGG | SEQ ID NO: 3486 | AUCGCUC | SEQ ID NO: 3537 | AUCUCAA | SEQ ID NO: 3588 | AUGAAAU | SEQ ID NO: 3639 | AUGAUCG |
| SEQ ID NO: 3385 | AUCAUGA | SEQ ID NO: 3436 | AUCCGGU | SEQ ID NO: 3487 | AUCGCUG | SEQ ID NO: 3538 | AUCUCAC | SEQ ID NO: 3589 | AUGAACA | SEQ ID NO: 3640 | AUGAUCU |
| SEQ ID NO: 3386 | AUCAUGC | SEQ ID NO: 3437 | AUCCGUA | SEQ ID NO: 3488 | AUCGCUU | SEQ ID NO: 3539 | AUCUCAG | SEQ ID NO: 3590 | AUGAACC | SEQ ID NO: 3641 | AUGAUGA |
| SEQ ID NO: 3387 | AUCAUGG | SEQ ID NO: 3438 | AUCCGUC | SEQ ID NO: 3489 | AUCGGAA | SEQ ID NO: 3540 | AUCUCAU | SEQ ID NO: 3591 | AUGAACG | SEQ ID NO: 3642 | AUGAUGC |
| SEQ ID NO: 3388 | AUCAUGU | SEQ ID NO: 3439 | AUCCGUG | SEQ ID NO: 3490 | AUCGGAC | SEQ ID NO: 3541 | AUCUCCA | SEQ ID NO: 3592 | AUGAACU | SEQ ID NO: 3643 | AUGAUGG |
| SEQ ID NO: 3389 | AUCAUUA | SEQ ID NO: 3440 | AUCCGUU | SEQ ID NO: 3491 | AUCGGAG | SEQ ID NO: 3542 | AUCUCCC | SEQ ID NO: 3593 | AUGAAGA | SEQ ID NO: 3644 | AUGAUGU |
| SEQ ID NO: 3390 | AUCAUUC | SEQ ID NO: 3441 | AUCCUAA | SEQ ID NO: 3492 | AUCGGAU | SEQ ID NO: 3543 | AUCUCCG | SEQ ID NO: 3594 | AUGAAGC | SEQ ID NO: 3645 | AUGAUUA |
| SEQ ID NO: 3391 | AUCAUUG | SEQ ID NO: 3442 | AUCCUAC | SEQ ID NO: 3493 | AUCGGCA | SEQ ID NO: 3544 | AUCUCCU | SEQ ID NO: 3595 | AUGAAGG | SEQ ID NO: 3646 | AUGAUUC |
| SEQ ID NO: 3392 | AUCAUUU | SEQ ID NO: 3443 | AUCCUAG | SEQ ID NO: 3494 | AUCGGCC | SEQ ID NO: 3545 | AUCUCGA | SEQ ID NO: 3596 | AUGAAGU | SEQ ID NO: 3647 | AUGAUUG |
| SEQ ID NO: 3393 | AUCCAAA | SEQ ID NO: 3444 | AUCCUAU | SEQ ID NO: 3495 | AUCGGCG | SEQ ID NO: 3546 | AUCUCGC | SEQ ID NO: 3597 | AUGAAUA | SEQ ID NO: 3648 | AUGAUUU |
| SEQ ID NO: 3394 | AUCCAAC | SEQ ID NO: 3445 | AUCCUCA | SEQ ID NO: 3496 | AUCGGCU | SEQ ID NO: 3547 | AUCUCGG | SEQ ID NO: 3598 | AUGAAUC | SEQ ID NO: 3649 | AUGCAAA |
| SEQ ID NO: 3395 | AUCCAAG | SEQ ID NO: 3446 | AUCCUCC | SEQ ID NO: 3497 | AUCGGGA | SEQ ID NO: 3548 | AUCUCGU | SEQ ID NO: 3599 | AUGAAUG | SEQ ID NO: 3650 | AUGCAAC |
| SEQ ID NO: 3396 | AUCCAAU | SEQ ID NO: 3447 | AUCCUCG | SEQ ID NO: 3498 | AUCGGGC | SEQ ID NO: 3549 | AUCUCUA | SEQ ID NO: 3600 | AUGAAUU | SEQ ID NO: 3651 | AUGCAAG |
| SEQ ID NO: 3397 | AUCCACA | SEQ ID NO: 3448 | AUCCUCU | SEQ ID NO: 3499 | AUCGGGG | SEQ ID NO: 3550 | AUCUCUC | SEQ ID NO: 3601 | AUGACAA | SEQ ID NO: 3652 | AUGCAAU |
| SEQ ID NO: 3398 | AUCCACC | SEQ ID NO: 3449 | AUCCUGA | SEQ ID NO: 3500 | AUCGGGU | SEQ ID NO: 3551 | AUCUCUG | SEQ ID NO: 3602 | AUGACAC | SEQ ID NO: 3653 | AUGCACA |
| SEQ ID NO: 3399 | AUCCACG | SEQ ID NO: 3450 | AUCCUGC | SEQ ID NO: 3501 | AUCGGUA | SEQ ID NO: 3552 | AUCUCUU | SEQ ID NO: 3603 | AUGACAG | SEQ ID NO: 3654 | AUGCACC |
| SEQ ID NO: 3400 | AUCCACU | SEQ ID NO: 3451 | AUCCUGG | SEQ ID NO: 3502 | AUCGGUC | SEQ ID NO: 3553 | AUCUGAA | SEQ ID NO: 3604 | AUGACAU | SEQ ID NO: 3655 | AUGCACG |
| SEQ ID NO: 3401 | AUCCAGA | SEQ ID NO: 3452 | AUCCUGU | SEQ ID NO: 3503 | AUCGGUG | SEQ ID NO: 3554 | AUCUGAC | SEQ ID NO: 3605 | AUGACCA | SEQ ID NO: 3656 | AUGCACU |
| SEQ ID NO: 3402 | AUCCAGC | SEQ ID NO: 3453 | AUCCUUA | SEQ ID NO: 3504 | AUCGGUU | SEQ ID NO: 3555 | AUCUGAG | SEQ ID NO: 3606 | AUGACCC | SEQ ID NO: 3657 | AUGCAGA |
| SEQ ID NO: 3403 | AUCCAGG | SEQ ID NO: 3454 | AUCCUUC | SEQ ID NO: 3505 | AUCGUAA | SEQ ID NO: 3556 | AUCUGAU | SEQ ID NO: 3607 | AUGACCG | SEQ ID NO: 3658 | AUGCAGC |
| SEQ ID NO: 3404 | AUCCAGU | SEQ ID NO: 3455 | AUCCUUG | SEQ ID NO: 3506 | AUCGUAC | SEQ ID NO: 3557 | AUCUGCA | SEQ ID NO: 3608 | AUGACCU | SEQ ID NO: 3659 | AUGCAGG |
| SEQ ID NO: 3405 | AUCCAUA | SEQ ID NO: 3456 | AUCCUUU | SEQ ID NO: 3507 | AUCGUAG | SEQ ID NO: 3558 | AUCUGCC | SEQ ID NO: 3609 | AUGACGA | SEQ ID NO: 3660 | AUGCAGU |
| SEQ ID NO: 3406 | AUCCAUC | SEQ ID NO: 3457 | AUCGAAA | SEQ ID NO: 3508 | AUCGUAU | SEQ ID NO: 3559 | AUCUGCG | SEQ ID NO: 3610 | AUGACGC | SEQ ID NO: 3661 | AUGCAUA |
| SEQ ID NO: 3407 | AUCCAUG | SEQ ID NO: 3458 | AUCGAAC | SEQ ID NO: 3509 | AUCGUCA | SEQ ID NO: 3560 | AUCUGCU | SEQ ID NO: 3611 | AUGACGG | SEQ ID NO: 3662 | AUGCAUC |
| SEQ ID NO: 3408 | AUCCAUU | SEQ ID NO: 3459 | AUCGAAG | SEQ ID NO: 3510 | AUCGUCC | SEQ ID NO: 3561 | AUCUGGA | SEQ ID NO: 3612 | AUGACGU | SEQ ID NO: 3663 | AUGCAUG |
| SEQ ID NO: 3409 | AUCCCAA | SEQ ID NO: 3460 | AUCGAAU | SEQ ID NO: 3511 | AUCGUCG | SEQ ID NO: 3562 | AUCUGGC | SEQ ID NO: 3613 | AUGACUA | SEQ ID NO: 3664 | AUGCAUU |
| SEQ ID NO: 3410 | AUCCCAC | SEQ ID NO: 3461 | AUCGACA | SEQ ID NO: 3512 | AUCGUCU | SEQ ID NO: 3563 | AUCUGGG | SEQ ID NO: 3614 | AUGACUC | SEQ ID NO: 3665 | AUGCCAA |
| SEQ ID NO: 3411 | AUCCCAG | SEQ ID NO: 3462 | AUCGACC | SEQ ID NO: 3513 | AUCGUGA | SEQ ID NO: 3564 | AUCUGGU | SEQ ID NO: 3615 | AUGACUG | SEQ ID NO: 3666 | AUGCCAC |
| SEQ ID NO: 3412 | AUCCCAU | SEQ ID NO: 3463 | AUCGACG | SEQ ID NO: 3514 | AUCGUGC | SEQ ID NO: 3565 | AUCUGUA | SEQ ID NO: 3616 | AUGACUU | SEQ ID NO: 3667 | AUGCCAG |
| SEQ ID NO: 3413 | AUCCCCA | SEQ ID NO: 3464 | AUCGACU | SEQ ID NO: 3515 | AUCGUGG | SEQ ID NO: 3566 | AUCUGUC | SEQ ID NO: 3617 | AUGAGAA | SEQ ID NO: 3668 | AUGCCAU |
| SEQ ID NO: 3414 | AUCCCCC | SEQ ID NO: 3465 | AUCGAGA | SEQ ID NO: 3516 | AUCGUGU | SEQ ID NO: 3567 | AUCUGUG | SEQ ID NO: 3618 | AUGAGAC | SEQ ID NO: 3669 | AUGCCCA |
| SEQ ID NO: 3415 | AUCCCCG | SEQ ID NO: 3466 | AUCGAGC | SEQ ID NO: 3517 | AUCGUUA | SEQ ID NO: 3568 | AUCUGUU | SEQ ID NO: 3619 | AUGAGAG | SEQ ID NO: 3670 | AUGCCCC |
| SEQ ID NO: 3416 | AUCCCCU | SEQ ID NO: 3467 | AUCGAGG | SEQ ID NO: 3518 | AUCGUUC | SEQ ID NO: 3569 | AUCUUAA | SEQ ID NO: 3620 | AUGAGAU | SEQ ID NO: 3671 | AUGCCCG |
| SEQ ID NO: 3417 | AUCCCGA | SEQ ID NO: 3468 | AUCGAGU | SEQ ID NO: 3519 | AUCGUUG | SEQ ID NO: 3570 | AUCUUAC | SEQ ID NO: 3621 | AUGAGCA | SEQ ID NO: 3672 | AUGCCCU |

Table 13

| | | | | | | | | | | | |
|---|---|---|---|---|---|---|---|---|---|---|---|
| SEQ ID NO: 3673 | AUGCCGA | SEQ ID NO: 3724 | AUGGAGU | SEQ ID NO: 3775 | AUGGUUG | SEQ ID NO: 3826 | AUGUUAC | SEQ ID NO: 3877 | AUUAGCA | SEQ ID NO: 3928 | AUUCCCU |
| SEQ ID NO: 3674 | AUGCCGC | SEQ ID NO: 3725 | AUGGAUA | SEQ ID NO: 3776 | AUGGUUU | SEQ ID NO: 3827 | AUGUUAG | SEQ ID NO: 3878 | AUUAGCC | SEQ ID NO: 3929 | AUUCCGA |
| SEQ ID NO: 3675 | AUGCCGG | SEQ ID NO: 3726 | AUGGAUC | SEQ ID NO: 3777 | AUGUAAA | SEQ ID NO: 3828 | AUGUUAU | SEQ ID NO: 3879 | AUUAGCG | SEQ ID NO: 3930 | AUUCCGC |
| SEQ ID NO: 3676 | AUGCCGU | SEQ ID NO: 3727 | AUGGAUG | SEQ ID NO: 3778 | AUGUAAC | SEQ ID NO: 3829 | AUGUUCA | SEQ ID NO: 3880 | AUUAGCU | SEQ ID NO: 3931 | AUUCCGG |
| SEQ ID NO: 3677 | AUGCCUA | SEQ ID NO: 3728 | AUGGAUU | SEQ ID NO: 3779 | AUGUAAG | SEQ ID NO: 3830 | AUGUUCC | SEQ ID NO: 3881 | AUUAGGA | SEQ ID NO: 3932 | AUUCCGU |
| SEQ ID NO: 3678 | AUGCCUC | SEQ ID NO: 3729 | AUGGCAA | SEQ ID NO: 3780 | AUGUAAU | SEQ ID NO: 3831 | AUGUUCG | SEQ ID NO: 3882 | AUUAGGC | SEQ ID NO: 3933 | AUUCCUA |
| SEQ ID NO: 3679 | AUGCCUG | SEQ ID NO: 3730 | AUGGCAC | SEQ ID NO: 3781 | AUGUACA | SEQ ID NO: 3832 | AUGUUCU | SEQ ID NO: 3883 | AUUAGGG | SEQ ID NO: 3934 | AUUCCUC |
| SEQ ID NO: 3680 | AUGCCUU | SEQ ID NO: 3731 | AUGGCAG | SEQ ID NO: 3782 | AUGUACC | SEQ ID NO: 3833 | AUGUUGA | SEQ ID NO: 3884 | AUUAGGU | SEQ ID NO: 3935 | AUUCCUG |
| SEQ ID NO: 3681 | AUGCGAA | SEQ ID NO: 3732 | AUGGCAU | SEQ ID NO: 3783 | AUGUACG | SEQ ID NO: 3834 | AUGUUGC | SEQ ID NO: 3885 | AUUAGUA | SEQ ID NO: 3936 | AUUCCUU |
| SEQ ID NO: 3682 | AUGCGAC | SEQ ID NO: 3733 | AUGGCCA | SEQ ID NO: 3784 | AUGUACU | SEQ ID NO: 3835 | AUGUUGG | SEQ ID NO: 3886 | AUUAGUC | SEQ ID NO: 3937 | AUUCGAA |
| SEQ ID NO: 3683 | AUGCGAG | SEQ ID NO: 3734 | AUGGCCC | SEQ ID NO: 3785 | AUGUAGA | SEQ ID NO: 3836 | AUGUUGU | SEQ ID NO: 3887 | AUUAGUG | SEQ ID NO: 3938 | AUUCGAC |
| SEQ ID NO: 3684 | AUGCGAU | SEQ ID NO: 3735 | AUGGCCG | SEQ ID NO: 3786 | AUGUAGC | SEQ ID NO: 3837 | AUGUUUA | SEQ ID NO: 3888 | AUUAGUU | SEQ ID NO: 3939 | AUUCGAG |
| SEQ ID NO: 3685 | AUGCGCA | SEQ ID NO: 3736 | AUGGCCU | SEQ ID NO: 3787 | AUGUAGG | SEQ ID NO: 3838 | AUGUUUC | SEQ ID NO: 3889 | AUUAUAA | SEQ ID NO: 3940 | AUUCGAU |
| SEQ ID NO: 3686 | AUGCGCC | SEQ ID NO: 3737 | AUGGCGA | SEQ ID NO: 3788 | AUGUAGU | SEQ ID NO: 3839 | AUGUUUG | SEQ ID NO: 3890 | AUUAUAC | SEQ ID NO: 3941 | AUUCGCA |
| SEQ ID NO: 3687 | AUGCGCG | SEQ ID NO: 3738 | AUGGCGC | SEQ ID NO: 3789 | AUGUAUA | SEQ ID NO: 3840 | AUGUUUU | SEQ ID NO: 3891 | AUUAUAG | SEQ ID NO: 3942 | AUUCGCC |
| SEQ ID NO: 3688 | AUGCGCU | SEQ ID NO: 3739 | AUGGCGG | SEQ ID NO: 3790 | AUGUAUC | SEQ ID NO: 3841 | AUUAAAA | SEQ ID NO: 3892 | AUUAUAU | SEQ ID NO: 3943 | AUUCGCG |
| SEQ ID NO: 3689 | AUGCGGA | SEQ ID NO: 3740 | AUGGCGU | SEQ ID NO: 3791 | AUGUAUG | SEQ ID NO: 3842 | AUUAAAC | SEQ ID NO: 3893 | AUUAUCA | SEQ ID NO: 3944 | AUUCGCU |
| SEQ ID NO: 3690 | AUGCGGC | SEQ ID NO: 3741 | AUGGCUA | SEQ ID NO: 3792 | AUGUAUU | SEQ ID NO: 3843 | AUUAAAG | SEQ ID NO: 3894 | AUUAUCC | SEQ ID NO: 3945 | AUUCGGA |
| SEQ ID NO: 3691 | AUGCGGG | SEQ ID NO: 3742 | AUGGCUC | SEQ ID NO: 3793 | AUGUCAA | SEQ ID NO: 3844 | AUUAAAU | SEQ ID NO: 3895 | AUUAUCG | SEQ ID NO: 3946 | AUUCGGC |
| SEQ ID NO: 3692 | AUGCGGU | SEQ ID NO: 3743 | AUGGCUG | SEQ ID NO: 3794 | AUGUCAC | SEQ ID NO: 3845 | AUUAACA | SEQ ID NO: 3896 | AUUAUCU | SEQ ID NO: 3947 | AUUCGGG |
| SEQ ID NO: 3693 | AUGCGUA | SEQ ID NO: 3744 | AUGGCUU | SEQ ID NO: 3795 | AUGUCAG | SEQ ID NO: 3846 | AUUAACC | SEQ ID NO: 3897 | AUUAUGA | SEQ ID NO: 3948 | AUUCGGU |
| SEQ ID NO: 3694 | AUGCGUC | SEQ ID NO: 3745 | AUGGGAA | SEQ ID NO: 3796 | AUGUCAU | SEQ ID NO: 3847 | AUUAACG | SEQ ID NO: 3898 | AUUAUGC | SEQ ID NO: 3949 | AUUCGUA |
| SEQ ID NO: 3695 | AUGCGUG | SEQ ID NO: 3746 | AUGGGAC | SEQ ID NO: 3797 | AUGUCCA | SEQ ID NO: 3848 | AUUAACU | SEQ ID NO: 3899 | AUUAUGG | SEQ ID NO: 3950 | AUUCGUC |
| SEQ ID NO: 3696 | AUGCGUU | SEQ ID NO: 3747 | AUGGGAG | SEQ ID NO: 3798 | AUGUCCC | SEQ ID NO: 3849 | AUUAAGA | SEQ ID NO: 3900 | AUUAUGU | SEQ ID NO: 3951 | AUUCGUG |
| SEQ ID NO: 3697 | AUGCUAA | SEQ ID NO: 3748 | AUGGGAU | SEQ ID NO: 3799 | AUGUCCG | SEQ ID NO: 3850 | AUUAAGC | SEQ ID NO: 3901 | AUUAUUA | SEQ ID NO: 3952 | AUUCGUU |
| SEQ ID NO: 3698 | AUGCUAC | SEQ ID NO: 3749 | AUGGGCA | SEQ ID NO: 3800 | AUGUCCU | SEQ ID NO: 3851 | AUUAAGG | SEQ ID NO: 3902 | AUUAUUC | SEQ ID NO: 3953 | AUUCUAA |
| SEQ ID NO: 3699 | AUGCUAG | SEQ ID NO: 3750 | AUGGGCC | SEQ ID NO: 3801 | AUGUCGA | SEQ ID NO: 3852 | AUUAAGU | SEQ ID NO: 3903 | AUUAUUG | SEQ ID NO: 3954 | AUUCUAC |
| SEQ ID NO: 3700 | AUGCUAU | SEQ ID NO: 3751 | AUGGGCG | SEQ ID NO: 3802 | AUGUCGC | SEQ ID NO: 3853 | AUUAAUA | SEQ ID NO: 3904 | AUUAUUU | SEQ ID NO: 3955 | AUUCUAG |
| SEQ ID NO: 3701 | AUGCUCA | SEQ ID NO: 3752 | AUGGGCU | SEQ ID NO: 3803 | AUGUCGG | SEQ ID NO: 3854 | AUUAAUC | SEQ ID NO: 3905 | AUUCAAA | SEQ ID NO: 3956 | AUUCUAU |
| SEQ ID NO: 3702 | AUGCUCC | SEQ ID NO: 3753 | AUGGGGA | SEQ ID NO: 3804 | AUGUCGU | SEQ ID NO: 3855 | AUUAAUG | SEQ ID NO: 3906 | AUUCAAC | SEQ ID NO: 3957 | AUUCUCA |
| SEQ ID NO: 3703 | AUGCUCG | SEQ ID NO: 3754 | AUGGGGC | SEQ ID NO: 3805 | AUGUCUA | SEQ ID NO: 3856 | AUUAAUU | SEQ ID NO: 3907 | AUUCAAG | SEQ ID NO: 3958 | AUUCUCC |
| SEQ ID NO: 3704 | AUGCUCU | SEQ ID NO: 3755 | AUGGGGG | SEQ ID NO: 3806 | AUGUCUC | SEQ ID NO: 3857 | AUUACAA | SEQ ID NO: 3908 | AUUCAAU | SEQ ID NO: 3959 | AUUCUCG |
| SEQ ID NO: 3705 | AUGCUGA | SEQ ID NO: 3756 | AUGGGGU | SEQ ID NO: 3807 | AUGUCUG | SEQ ID NO: 3858 | AUUACAC | SEQ ID NO: 3909 | AUUCACA | SEQ ID NO: 3960 | AUUCUCU |
| SEQ ID NO: 3706 | AUGCUGC | SEQ ID NO: 3757 | AUGGGUA | SEQ ID NO: 3808 | AUGUCUU | SEQ ID NO: 3859 | AUUACAG | SEQ ID NO: 3910 | AUUCACC | SEQ ID NO: 3961 | AUUCUGA |
| SEQ ID NO: 3707 | AUGCUGG | SEQ ID NO: 3758 | AUGGGUC | SEQ ID NO: 3809 | AUGUGAA | SEQ ID NO: 3860 | AUUACAU | SEQ ID NO: 3911 | AUUCACG | SEQ ID NO: 3962 | AUUCUGC |
| SEQ ID NO: 3708 | AUGCUGU | SEQ ID NO: 3759 | AUGGGUG | SEQ ID NO: 3810 | AUGUGAC | SEQ ID NO: 3861 | AUUACCA | SEQ ID NO: 3912 | AUUCACU | SEQ ID NO: 3963 | AUUCUGG |
| SEQ ID NO: 3709 | AUGCUUA | SEQ ID NO: 3760 | AUGGGUU | SEQ ID NO: 3811 | AUGUGAG | SEQ ID NO: 3862 | AUUACCC | SEQ ID NO: 3913 | AUUCAGA | SEQ ID NO: 3964 | AUUCUGU |
| SEQ ID NO: 3710 | AUGCUUC | SEQ ID NO: 3761 | AUGGUAA | SEQ ID NO: 3812 | AUGUGAU | SEQ ID NO: 3863 | AUUACCG | SEQ ID NO: 3914 | AUUCAGC | SEQ ID NO: 3965 | AUUCUUA |
| SEQ ID NO: 3711 | AUGCUUG | SEQ ID NO: 3762 | AUGGUAC | SEQ ID NO: 3813 | AUGUGCA | SEQ ID NO: 3864 | AUUACCU | SEQ ID NO: 3915 | AUUCAGG | SEQ ID NO: 3966 | AUUCUUC |
| SEQ ID NO: 3712 | AUGCUUU | SEQ ID NO: 3763 | AUGGUAG | SEQ ID NO: 3814 | AUGUGCC | SEQ ID NO: 3865 | AUUACGA | SEQ ID NO: 3916 | AUUCAGU | SEQ ID NO: 3967 | AUUCUUG |
| SEQ ID NO: 3713 | AUGGAAA | SEQ ID NO: 3764 | AUGGUAU | SEQ ID NO: 3815 | AUGUGCG | SEQ ID NO: 3866 | AUUACGC | SEQ ID NO: 3917 | AUUCAUA | SEQ ID NO: 3968 | AUUCUUU |
| SEQ ID NO: 3714 | AUGGAAC | SEQ ID NO: 3765 | AUGGUCA | SEQ ID NO: 3816 | AUGUGCU | SEQ ID NO: 3867 | AUUACGG | SEQ ID NO: 3918 | AUUCAUC | SEQ ID NO: 3969 | AUUGAAA |
| SEQ ID NO: 3715 | AUGGAAG | SEQ ID NO: 3766 | AUGGUCC | SEQ ID NO: 3817 | AUGUGGA | SEQ ID NO: 3868 | AUUACGU | SEQ ID NO: 3919 | AUUCAUG | SEQ ID NO: 3970 | AUUGAAC |
| SEQ ID NO: 3716 | AUGGAAU | SEQ ID NO: 3767 | AUGGUCG | SEQ ID NO: 3818 | AUGUGGC | SEQ ID NO: 3869 | AUUACUA | SEQ ID NO: 3920 | AUUCAUU | SEQ ID NO: 3971 | AUUGAAG |
| SEQ ID NO: 3717 | AUGGACA | SEQ ID NO: 3768 | AUGGUCU | SEQ ID NO: 3819 | AUGUGGG | SEQ ID NO: 3870 | AUUACUC | SEQ ID NO: 3921 | AUUCCAA | SEQ ID NO: 3972 | AUUGAAU |
| SEQ ID NO: 3718 | AUGGACC | SEQ ID NO: 3769 | AUGGUGA | SEQ ID NO: 3820 | AUGUGGU | SEQ ID NO: 3871 | AUUACUG | SEQ ID NO: 3922 | AUUCCAC | SEQ ID NO: 3973 | AUUGACA |
| SEQ ID NO: 3719 | AUGGACG | SEQ ID NO: 3770 | AUGGUGC | SEQ ID NO: 3821 | AUGUGUA | SEQ ID NO: 3872 | AUUACUU | SEQ ID NO: 3923 | AUUCCAG | SEQ ID NO: 3974 | AUUGACC |
| SEQ ID NO: 3720 | AUGGACU | SEQ ID NO: 3771 | AUGGUGG | SEQ ID NO: 3822 | AUGUGUC | SEQ ID NO: 3873 | AUUAGAA | SEQ ID NO: 3924 | AUUCCAU | SEQ ID NO: 3975 | AUUGACG |
| SEQ ID NO: 3721 | AUGGAGA | SEQ ID NO: 3772 | AUGGUGU | SEQ ID NO: 3823 | AUGUGUG | SEQ ID NO: 3874 | AUUAGAC | SEQ ID NO: 3925 | AUUCCCA | SEQ ID NO: 3976 | AUUGACU |
| SEQ ID NO: 3722 | AUGGAGC | SEQ ID NO: 3773 | AUGGUUA | SEQ ID NO: 3824 | AUGUGUU | SEQ ID NO: 3875 | AUUAGAG | SEQ ID NO: 3926 | AUUCCCC | SEQ ID NO: 3977 | AUUGAGA |
| SEQ ID NO: 3723 | AUGGAGG | SEQ ID NO: 3774 | AUGGUUC | SEQ ID NO: 3825 | AUGUUAA | SEQ ID NO: 3876 | AUUAGAU | SEQ ID NO: 3927 | AUUCCCG | SEQ ID NO: 3978 | AUUGAGC |

Table 14

| | | | | | | | | | | | |
|---|---|---|---|---|---|---|---|---|---|---|---|
| SEQ ID NO: 3979 | AUUGAGG | SEQ ID NO: 4030 | AUUGUUC | SEQ ID NO: 4081 | AUUUUAA | SEQ ID NO: 4132 | CAAAGAU | SEQ ID NO: 4183 | CAACCCG | SEQ ID NO: 4234 | CAAGAGC |
| SEQ ID NO: 3980 | AUUGAGU | SEQ ID NO: 4031 | AUUGUUG | SEQ ID NO: 4082 | AUUUUAC | SEQ ID NO: 4133 | CAAAGCA | SEQ ID NO: 4184 | CAACCCU | SEQ ID NO: 4235 | CAAGAGG |
| SEQ ID NO: 3981 | AUUGAUA | SEQ ID NO: 4032 | AUUGUUU | SEQ ID NO: 4083 | AUUUUAG | SEQ ID NO: 4134 | CAAAGCC | SEQ ID NO: 4185 | CAACCGA | SEQ ID NO: 4236 | CAAGAGU |
| SEQ ID NO: 3982 | AUUGAUC | SEQ ID NO: 4033 | AUUUAAA | SEQ ID NO: 4084 | AUUUUAU | SEQ ID NO: 4135 | CAAAGCG | SEQ ID NO: 4186 | CAACCGC | SEQ ID NO: 4237 | CAAGAUA |
| SEQ ID NO: 3983 | AUUGAUG | SEQ ID NO: 4034 | AUUUAAC | SEQ ID NO: 4085 | AUUUUCA | SEQ ID NO: 4136 | CAAAGCU | SEQ ID NO: 4187 | CAACCGG | SEQ ID NO: 4238 | CAAGAUC |
| SEQ ID NO: 3984 | AUUGAUU | SEQ ID NO: 4035 | AUUUAAG | SEQ ID NO: 4086 | AUUUUCC | SEQ ID NO: 4137 | CAAAGGA | SEQ ID NO: 4188 | CAACCGU | SEQ ID NO: 4239 | CAAGAUG |
| SEQ ID NO: 3985 | AUUGCAA | SEQ ID NO: 4036 | AUUUAAU | SEQ ID NO: 4087 | AUUUUCG | SEQ ID NO: 4138 | CAAAGGC | SEQ ID NO: 4189 | CAACCUA | SEQ ID NO: 4240 | CAAGAUU |
| SEQ ID NO: 3986 | AUUGCAC | SEQ ID NO: 4037 | AUUUACA | SEQ ID NO: 4088 | AUUUUCU | SEQ ID NO: 4139 | CAAAGGG | SEQ ID NO: 4190 | CAACCUC | SEQ ID NO: 4241 | CAAGCAA |
| SEQ ID NO: 3987 | AUUGCAG | SEQ ID NO: 4038 | AUUUACC | SEQ ID NO: 4089 | AUUUUGA | SEQ ID NO: 4140 | CAAAGGU | SEQ ID NO: 4191 | CAACCUG | SEQ ID NO: 4242 | CAAGCAC |
| SEQ ID NO: 3988 | AUUGCAU | SEQ ID NO: 4039 | AUUUACG | SEQ ID NO: 4090 | AUUUUGC | SEQ ID NO: 4141 | CAAAGUA | SEQ ID NO: 4192 | CAACCUU | SEQ ID NO: 4243 | CAAGCAG |
| SEQ ID NO: 3989 | AUUGCCA | SEQ ID NO: 4040 | AUUUACU | SEQ ID NO: 4091 | AUUUUGG | SEQ ID NO: 4142 | CAAAGUC | SEQ ID NO: 4193 | CAACGAA | SEQ ID NO: 4244 | CAAGCAU |
| SEQ ID NO: 3990 | AUUGCCC | SEQ ID NO: 4041 | AUUUAGA | SEQ ID NO: 4092 | AUUUUGU | SEQ ID NO: 4143 | CAAAGUG | SEQ ID NO: 4194 | CAACGAC | SEQ ID NO: 4245 | CAAGCCA |
| SEQ ID NO: 3991 | AUUGCCG | SEQ ID NO: 4042 | AUUUAGC | SEQ ID NO: 4093 | AUUUUUA | SEQ ID NO: 4144 | CAAAGUU | SEQ ID NO: 4195 | CAACGAG | SEQ ID NO: 4246 | CAAGCCC |
| SEQ ID NO: 3992 | AUUGCCU | SEQ ID NO: 4043 | AUUUAGG | SEQ ID NO: 4094 | AUUUUUC | SEQ ID NO: 4145 | CAAAUAA | SEQ ID NO: 4196 | CAACGAU | SEQ ID NO: 4247 | CAAGCCG |
| SEQ ID NO: 3993 | AUUGCGA | SEQ ID NO: 4044 | AUUUAGU | SEQ ID NO: 4095 | AUUUUUG | SEQ ID NO: 4146 | CAAAUAC | SEQ ID NO: 4197 | CAACGCA | SEQ ID NO: 4248 | CAAGCCU |
| SEQ ID NO: 3994 | AUUGCGC | SEQ ID NO: 4045 | AUUUAUA | SEQ ID NO: 4096 | AUUUUUU | SEQ ID NO: 4147 | CAAAUAG | SEQ ID NO: 4198 | CAACGCC | SEQ ID NO: 4249 | CAAGCGA |
| SEQ ID NO: 3995 | AUUGCGG | SEQ ID NO: 4046 | AUUUAUC | SEQ ID NO: 4097 | CAAAAAA | SEQ ID NO: 4148 | CAAAUAU | SEQ ID NO: 4199 | CAACGCG | SEQ ID NO: 4250 | CAAGCGC |
| SEQ ID NO: 3996 | AUUGCGU | SEQ ID NO: 4047 | AUUUAUG | SEQ ID NO: 4098 | CAAAAAC | SEQ ID NO: 4149 | CAAAUCA | SEQ ID NO: 4200 | CAACGCU | SEQ ID NO: 4251 | CAAGCGG |
| SEQ ID NO: 3997 | AUUGCUA | SEQ ID NO: 4048 | AUUUAUU | SEQ ID NO: 4099 | CAAAAAG | SEQ ID NO: 4150 | CAAAUCC | SEQ ID NO: 4201 | CAACGGA | SEQ ID NO: 4252 | CAAGCGU |
| SEQ ID NO: 3998 | AUUGCUC | SEQ ID NO: 4049 | AUUUCAA | SEQ ID NO: 4100 | CAAAAAU | SEQ ID NO: 4151 | CAAAUCG | SEQ ID NO: 4202 | CAACGGC | SEQ ID NO: 4253 | CAAGCUA |
| SEQ ID NO: 3999 | AUUGCUG | SEQ ID NO: 4050 | AUUUCAC | SEQ ID NO: 4101 | CAAAACA | SEQ ID NO: 4152 | CAAAUCU | SEQ ID NO: 4203 | CAACGGG | SEQ ID NO: 4254 | CAAGCUC |
| SEQ ID NO: 4000 | AUUGCUU | SEQ ID NO: 4051 | AUUUCAG | SEQ ID NO: 4102 | CAAAACC | SEQ ID NO: 4153 | CAAAUGA | SEQ ID NO: 4204 | CAACGGU | SEQ ID NO: 4255 | CAAGCUG |
| SEQ ID NO: 4001 | AUUGGAA | SEQ ID NO: 4052 | AUUUCAU | SEQ ID NO: 4103 | CAAAACG | SEQ ID NO: 4154 | CAAAUGC | SEQ ID NO: 4205 | CAACGUA | SEQ ID NO: 4256 | CAAGCUU |
| SEQ ID NO: 4002 | AUUGGAC | SEQ ID NO: 4053 | AUUUCCA | SEQ ID NO: 4104 | CAAAACU | SEQ ID NO: 4155 | CAAAUGG | SEQ ID NO: 4206 | CAACGUC | SEQ ID NO: 4257 | CAAGGAA |
| SEQ ID NO: 4003 | AUUGGAG | SEQ ID NO: 4054 | AUUUCCC | SEQ ID NO: 4105 | CAAAAGA | SEQ ID NO: 4156 | CAAAUGU | SEQ ID NO: 4207 | CAACGUG | SEQ ID NO: 4258 | CAAGGAC |
| SEQ ID NO: 4004 | AUUGGAU | SEQ ID NO: 4055 | AUUUCCG | SEQ ID NO: 4106 | CAAAAGC | SEQ ID NO: 4157 | CAAAUUA | SEQ ID NO: 4208 | CAACGUU | SEQ ID NO: 4259 | CAAGGAG |
| SEQ ID NO: 4005 | AUUGGCA | SEQ ID NO: 4056 | AUUUCCU | SEQ ID NO: 4107 | CAAAAGG | SEQ ID NO: 4158 | CAAAUUC | SEQ ID NO: 4209 | CAACUAA | SEQ ID NO: 4260 | CAAGGAU |
| SEQ ID NO: 4006 | AUUGGCC | SEQ ID NO: 4057 | AUUUCGA | SEQ ID NO: 4108 | CAAAAGU | SEQ ID NO: 4159 | CAAAUUG | SEQ ID NO: 4210 | CAACUAC | SEQ ID NO: 4261 | CAAGGCA |
| SEQ ID NO: 4007 | AUUGGCG | SEQ ID NO: 4058 | AUUUCGC | SEQ ID NO: 4109 | CAAAAUA | SEQ ID NO: 4160 | CAAAUUU | SEQ ID NO: 4211 | CAACUAG | SEQ ID NO: 4262 | CAAGGCC |
| SEQ ID NO: 4008 | AUUGGCU | SEQ ID NO: 4059 | AUUUCGG | SEQ ID NO: 4110 | CAAAAUC | SEQ ID NO: 4161 | CAACAAA | SEQ ID NO: 4212 | CAACUAU | SEQ ID NO: 4263 | CAAGGCG |
| SEQ ID NO: 4009 | AUUGGGA | SEQ ID NO: 4060 | AUUUCGU | SEQ ID NO: 4111 | CAAAAUG | SEQ ID NO: 4162 | CAACAAC | SEQ ID NO: 4213 | CAACUCA | SEQ ID NO: 4264 | CAAGGCU |
| SEQ ID NO: 4010 | AUUGGGC | SEQ ID NO: 4061 | AUUUCUA | SEQ ID NO: 4112 | CAAAAUU | SEQ ID NO: 4163 | CAACAAG | SEQ ID NO: 4214 | CAACUCC | SEQ ID NO: 4265 | CAAGGGA |
| SEQ ID NO: 4011 | AUUGGGG | SEQ ID NO: 4062 | AUUUCUC | SEQ ID NO: 4113 | CAAACAA | SEQ ID NO: 4164 | CAACAAU | SEQ ID NO: 4215 | CAACUCG | SEQ ID NO: 4266 | CAAGGGC |
| SEQ ID NO: 4012 | AUUGGGU | SEQ ID NO: 4063 | AUUUCUG | SEQ ID NO: 4114 | CAAACAC | SEQ ID NO: 4165 | CAACACA | SEQ ID NO: 4216 | CAACUCU | SEQ ID NO: 4267 | CAAGGGG |
| SEQ ID NO: 4013 | AUUGGUA | SEQ ID NO: 4064 | AUUUCUU | SEQ ID NO: 4115 | CAAACAG | SEQ ID NO: 4166 | CAACACC | SEQ ID NO: 4217 | CAACUGA | SEQ ID NO: 4268 | CAAGGGU |
| SEQ ID NO: 4014 | AUUGGUC | SEQ ID NO: 4065 | AUUUGAA | SEQ ID NO: 4116 | CAAACAU | SEQ ID NO: 4167 | CAACACG | SEQ ID NO: 4218 | CAACUGC | SEQ ID NO: 4269 | CAAGGUA |
| SEQ ID NO: 4015 | AUUGGUG | SEQ ID NO: 4066 | AUUUGAC | SEQ ID NO: 4117 | CAAACCA | SEQ ID NO: 4168 | CAACACU | SEQ ID NO: 4219 | CAACUGG | SEQ ID NO: 4270 | CAAGGUC |
| SEQ ID NO: 4016 | AUUGGUU | SEQ ID NO: 4067 | AUUUGAG | SEQ ID NO: 4118 | CAAACCC | SEQ ID NO: 4169 | CAACAGA | SEQ ID NO: 4220 | CAACUGU | SEQ ID NO: 4271 | CAAGGUG |
| SEQ ID NO: 4017 | AUUGUAA | SEQ ID NO: 4068 | AUUUGAU | SEQ ID NO: 4119 | CAAACCG | SEQ ID NO: 4170 | CAACAGC | SEQ ID NO: 4221 | CAACUUA | SEQ ID NO: 4272 | CAAGGUU |
| SEQ ID NO: 4018 | AUUGUAC | SEQ ID NO: 4069 | AUUUGCA | SEQ ID NO: 4120 | CAAACCU | SEQ ID NO: 4171 | CAACAGG | SEQ ID NO: 4222 | CAACUUC | SEQ ID NO: 4273 | CAAGUAA |
| SEQ ID NO: 4019 | AUUGUAG | SEQ ID NO: 4070 | AUUUGCC | SEQ ID NO: 4121 | CAAACGA | SEQ ID NO: 4172 | CAACAGU | SEQ ID NO: 4223 | CAACUUG | SEQ ID NO: 4274 | CAAGUAC |
| SEQ ID NO: 4020 | AUUGUAU | SEQ ID NO: 4071 | AUUUGCG | SEQ ID NO: 4122 | CAAACGC | SEQ ID NO: 4173 | CAACAUA | SEQ ID NO: 4224 | CAACUUU | SEQ ID NO: 4275 | CAAGUAG |
| SEQ ID NO: 4021 | AUUGUCA | SEQ ID NO: 4072 | AUUUGCU | SEQ ID NO: 4123 | CAAACGG | SEQ ID NO: 4174 | CAACAUC | SEQ ID NO: 4225 | CAAGAAA | SEQ ID NO: 4276 | CAAGUAU |
| SEQ ID NO: 4022 | AUUGUCC | SEQ ID NO: 4073 | AUUUGGA | SEQ ID NO: 4124 | CAAACGU | SEQ ID NO: 4175 | CAACAUG | SEQ ID NO: 4226 | CAAGAAC | SEQ ID NO: 4277 | CAAGUCA |
| SEQ ID NO: 4023 | AUUGUCG | SEQ ID NO: 4074 | AUUUGGC | SEQ ID NO: 4125 | CAAACUA | SEQ ID NO: 4176 | CAACAUU | SEQ ID NO: 4227 | CAAGAAG | SEQ ID NO: 4278 | CAAGUCC |
| SEQ ID NO: 4024 | AUUGUCU | SEQ ID NO: 4075 | AUUUGGG | SEQ ID NO: 4126 | CAAACUC | SEQ ID NO: 4177 | CAACCAA | SEQ ID NO: 4228 | CAAGAAU | SEQ ID NO: 4279 | CAAGUCG |
| SEQ ID NO: 4025 | AUUGUGA | SEQ ID NO: 4076 | AUUUGGU | SEQ ID NO: 4127 | CAAACUG | SEQ ID NO: 4178 | CAACCAC | SEQ ID NO: 4229 | CAAGACA | SEQ ID NO: 4280 | CAAGUCU |
| SEQ ID NO: 4026 | AUUGUGC | SEQ ID NO: 4077 | AUUUGUA | SEQ ID NO: 4128 | CAAACUU | SEQ ID NO: 4179 | CAACCAG | SEQ ID NO: 4230 | CAAGACC | SEQ ID NO: 4281 | CAAGUGA |
| SEQ ID NO: 4027 | AUUGUGG | SEQ ID NO: 4078 | AUUUGUC | SEQ ID NO: 4129 | CAAAGAA | SEQ ID NO: 4180 | CAACCAU | SEQ ID NO: 4231 | CAAGACG | SEQ ID NO: 4282 | CAAGUGC |
| SEQ ID NO: 4028 | AUUGUGU | SEQ ID NO: 4079 | AUUUGUG | SEQ ID NO: 4130 | CAAAGAC | SEQ ID NO: 4181 | CAACCCA | SEQ ID NO: 4232 | CAAGACU | SEQ ID NO: 4283 | CAAGUGG |
| SEQ ID NO: 4029 | AUUGUUA | SEQ ID NO: 4080 | AUUUGUU | SEQ ID NO: 4131 | CAAAGAG | SEQ ID NO: 4182 | CAACCCC | SEQ ID NO: 4233 | CAAGAGA | SEQ ID NO: 4284 | CAAGUGU |

Table 15

| | | | | | | | | | | | |
|---|---|---|---|---|---|---|---|---|---|---|---|
| SEQ ID NO: 4285 | CAAGUUA | SEQ ID NO: 4336 | CAAUGUU | SEQ ID NO: 4387 | CACAGAG | SEQ ID NO: 4438 | CACCCCC | SEQ ID NO: 4489 | CACGAGA | SEQ ID NO: 4540 | CACGUGU |
| SEQ ID NO: 4286 | CAAGUUC | SEQ ID NO: 4337 | CAAUUAA | SEQ ID NO: 4388 | CACAGAU | SEQ ID NO: 4439 | CACCCCG | SEQ ID NO: 4490 | CACGAGC | SEQ ID NO: 4541 | CACGUUA |
| SEQ ID NO: 4287 | CAAGUUG | SEQ ID NO: 4338 | CAAUUAC | SEQ ID NO: 4389 | CACAGCA | SEQ ID NO: 4440 | CACCCCU | SEQ ID NO: 4491 | CACGAGG | SEQ ID NO: 4542 | CACGUUC |
| SEQ ID NO: 4288 | CAAGUUU | SEQ ID NO: 4339 | CAAUUAG | SEQ ID NO: 4390 | CACAGCC | SEQ ID NO: 4441 | CACCCGA | SEQ ID NO: 4492 | CACGAGU | SEQ ID NO: 4543 | CACGUUG |
| SEQ ID NO: 4289 | CAAUAAA | SEQ ID NO: 4340 | CAAUUAU | SEQ ID NO: 4391 | CACAGCG | SEQ ID NO: 4442 | CACCCGC | SEQ ID NO: 4493 | CACGAUA | SEQ ID NO: 4544 | CACGUUU |
| SEQ ID NO: 4290 | CAAUAAC | SEQ ID NO: 4341 | CAAUUCA | SEQ ID NO: 4392 | CACAGCU | SEQ ID NO: 4443 | CACCCGG | SEQ ID NO: 4494 | CACGAUC | SEQ ID NO: 4545 | CACUAAA |
| SEQ ID NO: 4291 | CAAUAAG | SEQ ID NO: 4342 | CAAUUCC | SEQ ID NO: 4393 | CACAGGA | SEQ ID NO: 4444 | CACCCGU | SEQ ID NO: 4495 | CACGAUG | SEQ ID NO: 4546 | CACUAAC |
| SEQ ID NO: 4292 | CAAUAAU | SEQ ID NO: 4343 | CAAUUCG | SEQ ID NO: 4394 | CACAGGC | SEQ ID NO: 4445 | CACCCUA | SEQ ID NO: 4496 | CACGAUU | SEQ ID NO: 4547 | CACUAAG |
| SEQ ID NO: 4293 | CAAUACA | SEQ ID NO: 4344 | CAAUUCU | SEQ ID NO: 4395 | CACAGGG | SEQ ID NO: 4446 | CACCCUC | SEQ ID NO: 4497 | CACGCAA | SEQ ID NO: 4548 | CACUAAU |
| SEQ ID NO: 4294 | CAAUACC | SEQ ID NO: 4345 | CAAUUGA | SEQ ID NO: 4396 | CACAGGU | SEQ ID NO: 4447 | CACCCUG | SEQ ID NO: 4498 | CACGCAC | SEQ ID NO: 4549 | CACUACA |
| SEQ ID NO: 4295 | CAAUACG | SEQ ID NO: 4346 | CAAUUGC | SEQ ID NO: 4397 | CACAGUA | SEQ ID NO: 4448 | CACCCUU | SEQ ID NO: 4499 | CACGCAG | SEQ ID NO: 4550 | CACUACC |
| SEQ ID NO: 4296 | CAAUACU | SEQ ID NO: 4347 | CAAUUGG | SEQ ID NO: 4398 | CACAGUC | SEQ ID NO: 4449 | CACCGAA | SEQ ID NO: 4500 | CACGCAU | SEQ ID NO: 4551 | CACUACG |
| SEQ ID NO: 4297 | CAAUAGA | SEQ ID NO: 4348 | CAAUUGU | SEQ ID NO: 4399 | CACAGUG | SEQ ID NO: 4450 | CACCGAC | SEQ ID NO: 4501 | CACGCCA | SEQ ID NO: 4552 | CACUACU |
| SEQ ID NO: 4298 | CAAUAGC | SEQ ID NO: 4349 | CAAUUUA | SEQ ID NO: 4400 | CACAGUU | SEQ ID NO: 4451 | CACCGAG | SEQ ID NO: 4502 | CACGCCC | SEQ ID NO: 4553 | CACUAGA |
| SEQ ID NO: 4299 | CAAUAGG | SEQ ID NO: 4350 | CAAUUUC | SEQ ID NO: 4401 | CACAUAA | SEQ ID NO: 4452 | CACCGAU | SEQ ID NO: 4503 | CACGCCG | SEQ ID NO: 4554 | CACUAGC |
| SEQ ID NO: 4300 | CAAUAGU | SEQ ID NO: 4351 | CAAUUUG | SEQ ID NO: 4402 | CACAUAC | SEQ ID NO: 4453 | CACCGCA | SEQ ID NO: 4504 | CACGCCU | SEQ ID NO: 4555 | CACUAGG |
| SEQ ID NO: 4301 | CAAUAUA | SEQ ID NO: 4352 | CAAUUUU | SEQ ID NO: 4403 | CACAUAG | SEQ ID NO: 4454 | CACCGCC | SEQ ID NO: 4505 | CACGCGA | SEQ ID NO: 4556 | CACUAGU |
| SEQ ID NO: 4302 | CAAUAUC | SEQ ID NO: 4353 | CACAAAA | SEQ ID NO: 4404 | CACAUAU | SEQ ID NO: 4455 | CACCGCG | SEQ ID NO: 4506 | CACGCGC | SEQ ID NO: 4557 | CACUAUA |
| SEQ ID NO: 4303 | CAAUAUG | SEQ ID NO: 4354 | CACAAAC | SEQ ID NO: 4405 | CACAUCA | SEQ ID NO: 4456 | CACCGCU | SEQ ID NO: 4507 | CACGCGG | SEQ ID NO: 4558 | CACUAUC |
| SEQ ID NO: 4304 | CAAUAUU | SEQ ID NO: 4355 | CACAAAG | SEQ ID NO: 4406 | CACAUCC | SEQ ID NO: 4457 | CACCGGA | SEQ ID NO: 4508 | CACGCGU | SEQ ID NO: 4559 | CACUAUG |
| SEQ ID NO: 4305 | CAAUCAA | SEQ ID NO: 4356 | CACAAAU | SEQ ID NO: 4407 | CACAUCG | SEQ ID NO: 4458 | CACCGGC | SEQ ID NO: 4509 | CACGCUA | SEQ ID NO: 4560 | CACUAUU |
| SEQ ID NO: 4306 | CAAUCAC | SEQ ID NO: 4357 | CACAACA | SEQ ID NO: 4408 | CACAUCU | SEQ ID NO: 4459 | CACCGGG | SEQ ID NO: 4510 | CACGCUC | SEQ ID NO: 4561 | CACUCAA |
| SEQ ID NO: 4307 | CAAUCAG | SEQ ID NO: 4358 | CACAACC | SEQ ID NO: 4409 | CACAUGA | SEQ ID NO: 4460 | CACCGGU | SEQ ID NO: 4511 | CACGCUG | SEQ ID NO: 4562 | CACUCAC |
| SEQ ID NO: 4308 | CAAUCAU | SEQ ID NO: 4359 | CACAACG | SEQ ID NO: 4410 | CACAUGC | SEQ ID NO: 4461 | CACCGUA | SEQ ID NO: 4512 | CACGCUU | SEQ ID NO: 4563 | CACUCAG |
| SEQ ID NO: 4309 | CAAUCCA | SEQ ID NO: 4360 | CACAACU | SEQ ID NO: 4411 | CACAUGG | SEQ ID NO: 4462 | CACCGUC | SEQ ID NO: 4513 | CACGGAA | SEQ ID NO: 4564 | CACUCAU |
| SEQ ID NO: 4310 | CAAUCCC | SEQ ID NO: 4361 | CACAAGA | SEQ ID NO: 4412 | CACAUGU | SEQ ID NO: 4463 | CACCGUG | SEQ ID NO: 4514 | CACGGAC | SEQ ID NO: 4565 | CACUCCA |
| SEQ ID NO: 4311 | CAAUCCG | SEQ ID NO: 4362 | CACAAGC | SEQ ID NO: 4413 | CACAUUA | SEQ ID NO: 4464 | CACCGUU | SEQ ID NO: 4515 | CACGGAG | SEQ ID NO: 4566 | CACUCCC |
| SEQ ID NO: 4312 | CAAUCCU | SEQ ID NO: 4363 | CACAAGG | SEQ ID NO: 4414 | CACAUUC | SEQ ID NO: 4465 | CACCUAA | SEQ ID NO: 4516 | CACGGAU | SEQ ID NO: 4567 | CACUCCG |
| SEQ ID NO: 4313 | CAAUCGA | SEQ ID NO: 4364 | CACAAGU | SEQ ID NO: 4415 | CACAUUG | SEQ ID NO: 4466 | CACCUAC | SEQ ID NO: 4517 | CACGGCA | SEQ ID NO: 4568 | CACUCCU |
| SEQ ID NO: 4314 | CAAUCGC | SEQ ID NO: 4365 | CACAAUA | SEQ ID NO: 4416 | CACAUUU | SEQ ID NO: 4467 | CACCUAG | SEQ ID NO: 4518 | CACGGCC | SEQ ID NO: 4569 | CACUCGA |
| SEQ ID NO: 4315 | CAAUCGG | SEQ ID NO: 4366 | CACAAUC | SEQ ID NO: 4417 | CACCAAA | SEQ ID NO: 4468 | CACCUAU | SEQ ID NO: 4519 | CACGGCG | SEQ ID NO: 4570 | CACUCGC |
| SEQ ID NO: 4316 | CAAUCGU | SEQ ID NO: 4367 | CACAAUG | SEQ ID NO: 4418 | CACCAAC | SEQ ID NO: 4469 | CACCUCA | SEQ ID NO: 4520 | CACGGCU | SEQ ID NO: 4571 | CACUCGG |
| SEQ ID NO: 4317 | CAAUCUA | SEQ ID NO: 4368 | CACAAUU | SEQ ID NO: 4419 | CACCAAG | SEQ ID NO: 4470 | CACCUCC | SEQ ID NO: 4521 | CACGGGA | SEQ ID NO: 4572 | CACUCGU |
| SEQ ID NO: 4318 | CAAUCUC | SEQ ID NO: 4369 | CACACAA | SEQ ID NO: 4420 | CACCAAU | SEQ ID NO: 4471 | CACCUCG | SEQ ID NO: 4522 | CACGGGC | SEQ ID NO: 4573 | CACUCUA |
| SEQ ID NO: 4319 | CAAUCUG | SEQ ID NO: 4370 | CACACAC | SEQ ID NO: 4421 | CACCACA | SEQ ID NO: 4472 | CACCUCU | SEQ ID NO: 4523 | CACGGGG | SEQ ID NO: 4574 | CACUCUC |
| SEQ ID NO: 4320 | CAAUCUU | SEQ ID NO: 4371 | CACACAG | SEQ ID NO: 4422 | CACCACC | SEQ ID NO: 4473 | CACCUGA | SEQ ID NO: 4524 | CACGGGU | SEQ ID NO: 4575 | CACUCUG |
| SEQ ID NO: 4321 | CAAUGAA | SEQ ID NO: 4372 | CACACAU | SEQ ID NO: 4423 | CACCACG | SEQ ID NO: 4474 | CACCUGC | SEQ ID NO: 4525 | CACGGUA | SEQ ID NO: 4576 | CACUCUU |
| SEQ ID NO: 4322 | CAAUGAC | SEQ ID NO: 4373 | CACACCA | SEQ ID NO: 4424 | CACCACU | SEQ ID NO: 4475 | CACCUGG | SEQ ID NO: 4526 | CACGGUC | SEQ ID NO: 4577 | CACUGAA |
| SEQ ID NO: 4323 | CAAUGAG | SEQ ID NO: 4374 | CACACCC | SEQ ID NO: 4425 | CACCAGA | SEQ ID NO: 4476 | CACCUGU | SEQ ID NO: 4527 | CACGGUG | SEQ ID NO: 4578 | CACUGAC |
| SEQ ID NO: 4324 | CAAUGAU | SEQ ID NO: 4375 | CACACCG | SEQ ID NO: 4426 | CACCAGC | SEQ ID NO: 4477 | CACCUUA | SEQ ID NO: 4528 | CACGGUU | SEQ ID NO: 4579 | CACUGAG |
| SEQ ID NO: 4325 | CAAUGCA | SEQ ID NO: 4376 | CACACCU | SEQ ID NO: 4427 | CACCAGG | SEQ ID NO: 4478 | CACCUUC | SEQ ID NO: 4529 | CACGUAA | SEQ ID NO: 4580 | CACUGAU |
| SEQ ID NO: 4326 | CAAUGCC | SEQ ID NO: 4377 | CACACGA | SEQ ID NO: 4428 | CACCAGU | SEQ ID NO: 4479 | CACCUUG | SEQ ID NO: 4530 | CACGUAC | SEQ ID NO: 4581 | CACUGCA |
| SEQ ID NO: 4327 | CAAUGCG | SEQ ID NO: 4378 | CACACGC | SEQ ID NO: 4429 | CACCAUA | SEQ ID NO: 4480 | CACCUUU | SEQ ID NO: 4531 | CACGUAG | SEQ ID NO: 4582 | CACUGCC |
| SEQ ID NO: 4328 | CAAUGCU | SEQ ID NO: 4379 | CACACGG | SEQ ID NO: 4430 | CACCAUC | SEQ ID NO: 4481 | CACGAAA | SEQ ID NO: 4532 | CACGUAU | SEQ ID NO: 4583 | CACUGCG |
| SEQ ID NO: 4329 | CAAUGGA | SEQ ID NO: 4380 | CACACGU | SEQ ID NO: 4431 | CACCAUG | SEQ ID NO: 4482 | CACGAAC | SEQ ID NO: 4533 | CACGUCA | SEQ ID NO: 4584 | CACUGCU |
| SEQ ID NO: 4330 | CAAUGGC | SEQ ID NO: 4381 | CACACUA | SEQ ID NO: 4432 | CACCAUU | SEQ ID NO: 4483 | CACGAAG | SEQ ID NO: 4534 | CACGUCC | SEQ ID NO: 4585 | CACUGGA |
| SEQ ID NO: 4331 | CAAUGGG | SEQ ID NO: 4382 | CACACUC | SEQ ID NO: 4433 | CACCCAA | SEQ ID NO: 4484 | CACGAAU | SEQ ID NO: 4535 | CACGUCG | SEQ ID NO: 4586 | CACUGGC |
| SEQ ID NO: 4332 | CAAUGGU | SEQ ID NO: 4383 | CACACUG | SEQ ID NO: 4434 | CACCCAC | SEQ ID NO: 4485 | CACGACA | SEQ ID NO: 4536 | CACGUCU | SEQ ID NO: 4587 | CACUGGG |
| SEQ ID NO: 4333 | CAAUGUA | SEQ ID NO: 4384 | CACACUU | SEQ ID NO: 4435 | CACCCAG | SEQ ID NO: 4486 | CACGACC | SEQ ID NO: 4537 | CACGUGA | SEQ ID NO: 4588 | CACUGGU |
| SEQ ID NO: 4334 | CAAUGUC | SEQ ID NO: 4385 | CACAGAA | SEQ ID NO: 4436 | CACCCAU | SEQ ID NO: 4487 | CACGACG | SEQ ID NO: 4538 | CACGUGC | SEQ ID NO: 4589 | CACUGUA |
| SEQ ID NO: 4335 | CAAUGUG | SEQ ID NO: 4386 | CACAGAC | SEQ ID NO: 4437 | CACCCCA | SEQ ID NO: 4488 | CACGACU | SEQ ID NO: 4539 | CACGUGG | SEQ ID NO: 4590 | CACUGUC |

Table 16

| | | | | | | | | | | | |
|---|---|---|---|---|---|---|---|---|---|---|---|
| SEQ ID NO: 4591 | CACUGUG | SEQ ID NO: 4642 | CAGAGAC | SEQ ID NO: 4693 | CAGCCCA | SEQ ID NO: 4744 | CAGGACU | SEQ ID NO: 4795 | CAGGUGG | SEQ ID NO: 4846 | CAGUGUC |
| SEQ ID NO: 4592 | CACUGUU | SEQ ID NO: 4643 | CAGAGAG | SEQ ID NO: 4694 | CAGCCCC | SEQ ID NO: 4745 | CAGGAGA | SEQ ID NO: 4796 | CAGGUGU | SEQ ID NO: 4847 | CAGUGUG |
| SEQ ID NO: 4593 | CACUUAA | SEQ ID NO: 4644 | CAGAGAU | SEQ ID NO: 4695 | CAGCCCG | SEQ ID NO: 4746 | CAGGAGC | SEQ ID NO: 4797 | CAGGUUA | SEQ ID NO: 4848 | CAGUGUU |
| SEQ ID NO: 4594 | CACUUAC | SEQ ID NO: 4645 | CAGAGCA | SEQ ID NO: 4696 | CAGCCCU | SEQ ID NO: 4747 | CAGGAGG | SEQ ID NO: 4798 | CAGGUUC | SEQ ID NO: 4849 | CAGUUAA |
| SEQ ID NO: 4595 | CACUUAG | SEQ ID NO: 4646 | CAGAGCC | SEQ ID NO: 4697 | CAGCCGA | SEQ ID NO: 4748 | CAGGAGU | SEQ ID NO: 4799 | CAGGUUG | SEQ ID NO: 4850 | CAGUUAC |
| SEQ ID NO: 4596 | CACUUAU | SEQ ID NO: 4647 | CAGAGCG | SEQ ID NO: 4698 | CAGCCGC | SEQ ID NO: 4749 | CAGGAUA | SEQ ID NO: 4800 | CAGGUUU | SEQ ID NO: 4851 | CAGUUAG |
| SEQ ID NO: 4597 | CACUUCA | SEQ ID NO: 4648 | CAGAGCU | SEQ ID NO: 4699 | CAGCCGG | SEQ ID NO: 4750 | CAGGAUC | SEQ ID NO: 4801 | CAGUAAA | SEQ ID NO: 4852 | CAGUUAU |
| SEQ ID NO: 4598 | CACUUCC | SEQ ID NO: 4649 | CAGAGGA | SEQ ID NO: 4700 | CAGCCGU | SEQ ID NO: 4751 | CAGGAUG | SEQ ID NO: 4802 | CAGUAAC | SEQ ID NO: 4853 | CAGUUCA |
| SEQ ID NO: 4599 | CACUUCG | SEQ ID NO: 4650 | CAGAGGC | SEQ ID NO: 4701 | CAGCCUA | SEQ ID NO: 4752 | CAGGAUU | SEQ ID NO: 4803 | CAGUAAG | SEQ ID NO: 4854 | CAGUUCC |
| SEQ ID NO: 4600 | CACUUCU | SEQ ID NO: 4651 | CAGAGGG | SEQ ID NO: 4702 | CAGCCUC | SEQ ID NO: 4753 | CAGGCAA | SEQ ID NO: 4804 | CAGUAAU | SEQ ID NO: 4855 | CAGUUCG |
| SEQ ID NO: 4601 | CACUUGA | SEQ ID NO: 4652 | CAGAGGU | SEQ ID NO: 4703 | CAGCCUG | SEQ ID NO: 4754 | CAGGCAC | SEQ ID NO: 4805 | CAGUACA | SEQ ID NO: 4856 | CAGUUCU |
| SEQ ID NO: 4602 | CACUUGC | SEQ ID NO: 4653 | CAGAGUA | SEQ ID NO: 4704 | CAGCCUU | SEQ ID NO: 4755 | CAGGCAG | SEQ ID NO: 4806 | CAGUACC | SEQ ID NO: 4857 | CAGUUGA |
| SEQ ID NO: 4603 | CACUUGG | SEQ ID NO: 4654 | CAGAGUC | SEQ ID NO: 4705 | CAGCGAA | SEQ ID NO: 4756 | CAGGCAU | SEQ ID NO: 4807 | CAGUACG | SEQ ID NO: 4858 | CAGUUGC |
| SEQ ID NO: 4604 | CACUUGU | SEQ ID NO: 4655 | CAGAGUG | SEQ ID NO: 4706 | CAGCGAC | SEQ ID NO: 4757 | CAGGCCA | SEQ ID NO: 4808 | CAGUACU | SEQ ID NO: 4859 | CAGUUGG |
| SEQ ID NO: 4605 | CACUUUA | SEQ ID NO: 4656 | CAGAGUU | SEQ ID NO: 4707 | CAGCGAG | SEQ ID NO: 4758 | CAGGCCC | SEQ ID NO: 4809 | CAGUAGA | SEQ ID NO: 4860 | CAGUUGU |
| SEQ ID NO: 4606 | CACUUUC | SEQ ID NO: 4657 | CAGAUAA | SEQ ID NO: 4708 | CAGCGAU | SEQ ID NO: 4759 | CAGGCCG | SEQ ID NO: 4810 | CAGUAGC | SEQ ID NO: 4861 | CAGUUUA |
| SEQ ID NO: 4607 | CACUUUG | SEQ ID NO: 4658 | CAGAUAC | SEQ ID NO: 4709 | CAGCGCA | SEQ ID NO: 4760 | CAGGCCU | SEQ ID NO: 4811 | CAGUAGG | SEQ ID NO: 4862 | CAGUUUC |
| SEQ ID NO: 4608 | CACUUUU | SEQ ID NO: 4659 | CAGAUAG | SEQ ID NO: 4710 | CAGCGCC | SEQ ID NO: 4761 | CAGGCGA | SEQ ID NO: 4812 | CAGUAGU | SEQ ID NO: 4863 | CAGUUUG |
| SEQ ID NO: 4609 | CAGAAAA | SEQ ID NO: 4660 | CAGAUAU | SEQ ID NO: 4711 | CAGCGCG | SEQ ID NO: 4762 | CAGGCGC | SEQ ID NO: 4813 | CAGUAUA | SEQ ID NO: 4864 | CAGUUUU |
| SEQ ID NO: 4610 | CAGAAAC | SEQ ID NO: 4661 | CAGAUCA | SEQ ID NO: 4712 | CAGCGCU | SEQ ID NO: 4763 | CAGGCGG | SEQ ID NO: 4814 | CAGUAUC | SEQ ID NO: 4865 | CAUAAAA |
| SEQ ID NO: 4611 | CAGAAAG | SEQ ID NO: 4662 | CAGAUCC | SEQ ID NO: 4713 | CAGCGGA | SEQ ID NO: 4764 | CAGGCGU | SEQ ID NO: 4815 | CAGUAUG | SEQ ID NO: 4866 | CAUAAAC |
| SEQ ID NO: 4612 | CAGAAAU | SEQ ID NO: 4663 | CAGAUCG | SEQ ID NO: 4714 | CAGCGGC | SEQ ID NO: 4765 | CAGGCUA | SEQ ID NO: 4816 | CAGUAUU | SEQ ID NO: 4867 | CAUAAAG |
| SEQ ID NO: 4613 | CAGAACA | SEQ ID NO: 4664 | CAGAUCU | SEQ ID NO: 4715 | CAGCGGG | SEQ ID NO: 4766 | CAGGCUC | SEQ ID NO: 4817 | CAGUCAA | SEQ ID NO: 4868 | CAUAAAU |
| SEQ ID NO: 4614 | CAGAACC | SEQ ID NO: 4665 | CAGAUGA | SEQ ID NO: 4716 | CAGCGGU | SEQ ID NO: 4767 | CAGGCUG | SEQ ID NO: 4818 | CAGUCAC | SEQ ID NO: 4869 | CAUAACA |
| SEQ ID NO: 4615 | CAGAACG | SEQ ID NO: 4666 | CAGAUGC | SEQ ID NO: 4717 | CAGCGUA | SEQ ID NO: 4768 | CAGGCUU | SEQ ID NO: 4819 | CAGUCAG | SEQ ID NO: 4870 | CAUAACC |
| SEQ ID NO: 4616 | CAGAACU | SEQ ID NO: 4667 | CAGAUGG | SEQ ID NO: 4718 | CAGCGUC | SEQ ID NO: 4769 | CAGGGAA | SEQ ID NO: 4820 | CAGUCAU | SEQ ID NO: 4871 | CAUAACG |
| SEQ ID NO: 4617 | CAGAAGA | SEQ ID NO: 4668 | CAGAUGU | SEQ ID NO: 4719 | CAGCGUG | SEQ ID NO: 4770 | CAGGGAC | SEQ ID NO: 4821 | CAGUCCA | SEQ ID NO: 4872 | CAUAACU |
| SEQ ID NO: 4618 | CAGAAGC | SEQ ID NO: 4669 | CAGAUUA | SEQ ID NO: 4720 | CAGCGUU | SEQ ID NO: 4771 | CAGGGAG | SEQ ID NO: 4822 | CAGUCCC | SEQ ID NO: 4873 | CAUAAGA |
| SEQ ID NO: 4619 | CAGAAGG | SEQ ID NO: 4670 | CAGAUUC | SEQ ID NO: 4721 | CAGCUAA | SEQ ID NO: 4772 | CAGGGAU | SEQ ID NO: 4823 | CAGUCCG | SEQ ID NO: 4874 | CAUAAGC |
| SEQ ID NO: 4620 | CAGAAGU | SEQ ID NO: 4671 | CAGAUUG | SEQ ID NO: 4722 | CAGCUAC | SEQ ID NO: 4773 | CAGGGCA | SEQ ID NO: 4824 | CAGUCCU | SEQ ID NO: 4875 | CAUAAGG |
| SEQ ID NO: 4621 | CAGAAUA | SEQ ID NO: 4672 | CAGAUUU | SEQ ID NO: 4723 | CAGCUAG | SEQ ID NO: 4774 | CAGGGCC | SEQ ID NO: 4825 | CAGUCGA | SEQ ID NO: 4876 | CAUAAGU |
| SEQ ID NO: 4622 | CAGAAUC | SEQ ID NO: 4673 | CAGCAAA | SEQ ID NO: 4724 | CAGCUAU | SEQ ID NO: 4775 | CAGGGCG | SEQ ID NO: 4826 | CAGUCGC | SEQ ID NO: 4877 | CAUAAUA |
| SEQ ID NO: 4623 | CAGAAUG | SEQ ID NO: 4674 | CAGCAAC | SEQ ID NO: 4725 | CAGCUCA | SEQ ID NO: 4776 | CAGGGCU | SEQ ID NO: 4827 | CAGUCGG | SEQ ID NO: 4878 | CAUAAUC |
| SEQ ID NO: 4624 | CAGAAUU | SEQ ID NO: 4675 | CAGCAAG | SEQ ID NO: 4726 | CAGCUCC | SEQ ID NO: 4777 | CAGGGGA | SEQ ID NO: 4828 | CAGUCGU | SEQ ID NO: 4879 | CAUAAUG |
| SEQ ID NO: 4625 | CAGACAA | SEQ ID NO: 4676 | CAGCAAU | SEQ ID NO: 4727 | CAGCUCG | SEQ ID NO: 4778 | CAGGGGC | SEQ ID NO: 4829 | CAGUCUA | SEQ ID NO: 4880 | CAUAAUU |
| SEQ ID NO: 4626 | CAGACAC | SEQ ID NO: 4677 | CAGCACA | SEQ ID NO: 4728 | CAGCUCU | SEQ ID NO: 4779 | CAGGGGG | SEQ ID NO: 4830 | CAGUCUC | SEQ ID NO: 4881 | CAUACAA |
| SEQ ID NO: 4627 | CAGACAG | SEQ ID NO: 4678 | CAGCACC | SEQ ID NO: 4729 | CAGCUGA | SEQ ID NO: 4780 | CAGGGGU | SEQ ID NO: 4831 | CAGUCUG | SEQ ID NO: 4882 | CAUACAC |
| SEQ ID NO: 4628 | CAGACAU | SEQ ID NO: 4679 | CAGCACG | SEQ ID NO: 4730 | CAGCUGC | SEQ ID NO: 4781 | CAGGGUA | SEQ ID NO: 4832 | CAGUCUU | SEQ ID NO: 4883 | CAUACAG |
| SEQ ID NO: 4629 | CAGACCA | SEQ ID NO: 4680 | CAGCACU | SEQ ID NO: 4731 | CAGCUGG | SEQ ID NO: 4782 | CAGGGUC | SEQ ID NO: 4833 | CAGUGAA | SEQ ID NO: 4884 | CAUACAU |
| SEQ ID NO: 4630 | CAGACCC | SEQ ID NO: 4681 | CAGCAGA | SEQ ID NO: 4732 | CAGCUGU | SEQ ID NO: 4783 | CAGGGUG | SEQ ID NO: 4834 | CAGUGAC | SEQ ID NO: 4885 | CAUACCA |
| SEQ ID NO: 4631 | CAGACCG | SEQ ID NO: 4682 | CAGCAGC | SEQ ID NO: 4733 | CAGCUUA | SEQ ID NO: 4784 | CAGGGUU | SEQ ID NO: 4835 | CAGUGAG | SEQ ID NO: 4886 | CAUACCC |
| SEQ ID NO: 4632 | CAGACCU | SEQ ID NO: 4683 | CAGCAGG | SEQ ID NO: 4734 | CAGCUUC | SEQ ID NO: 4785 | CAGGUAA | SEQ ID NO: 4836 | CAGUGAU | SEQ ID NO: 4887 | CAUACCG |
| SEQ ID NO: 4633 | CAGACGA | SEQ ID NO: 4684 | CAGCAGU | SEQ ID NO: 4735 | CAGCUUG | SEQ ID NO: 4786 | CAGGUAC | SEQ ID NO: 4837 | CAGUGCA | SEQ ID NO: 4888 | CAUACCU |
| SEQ ID NO: 4634 | CAGACGC | SEQ ID NO: 4685 | CAGCAUA | SEQ ID NO: 4736 | CAGCUUU | SEQ ID NO: 4787 | CAGGUAG | SEQ ID NO: 4838 | CAGUGCC | SEQ ID NO: 4889 | CAUACGA |
| SEQ ID NO: 4635 | CAGACGG | SEQ ID NO: 4686 | CAGCAUC | SEQ ID NO: 4737 | CAGGAAA | SEQ ID NO: 4788 | CAGGUAU | SEQ ID NO: 4839 | CAGUGCG | SEQ ID NO: 4890 | CAUACGC |
| SEQ ID NO: 4636 | CAGACGU | SEQ ID NO: 4687 | CAGCAUG | SEQ ID NO: 4738 | CAGGAAC | SEQ ID NO: 4789 | CAGGUCA | SEQ ID NO: 4840 | CAGUGCU | SEQ ID NO: 4891 | CAUACGG |
| SEQ ID NO: 4637 | CAGACUA | SEQ ID NO: 4688 | CAGCAUU | SEQ ID NO: 4739 | CAGGAAG | SEQ ID NO: 4790 | CAGGUCC | SEQ ID NO: 4841 | CAGUGGA | SEQ ID NO: 4892 | CAUACGU |
| SEQ ID NO: 4638 | CAGACUC | SEQ ID NO: 4689 | CAGCCAA | SEQ ID NO: 4740 | CAGGAAU | SEQ ID NO: 4791 | CAGGUCG | SEQ ID NO: 4842 | CAGUGGC | SEQ ID NO: 4893 | CAUACUA |
| SEQ ID NO: 4639 | CAGACUG | SEQ ID NO: 4690 | CAGCCAC | SEQ ID NO: 4741 | CAGGACA | SEQ ID NO: 4792 | CAGGUCU | SEQ ID NO: 4843 | CAGUGGG | SEQ ID NO: 4894 | CAUACUC |
| SEQ ID NO: 4640 | CAGACUU | SEQ ID NO: 4691 | CAGCCAG | SEQ ID NO: 4742 | CAGGACC | SEQ ID NO: 4793 | CAGGUGA | SEQ ID NO: 4844 | CAGUGGU | SEQ ID NO: 4895 | CAUACUG |
| SEQ ID NO: 4641 | CAGAGAA | SEQ ID NO: 4692 | CAGCCAU | SEQ ID NO: 4743 | CAGGACG | SEQ ID NO: 4794 | CAGGUGC | SEQ ID NO: 4845 | CAGUGUA | SEQ ID NO: 4896 | CAUACUU |

Table 17

| | |
|---|---|
| SEQ ID NO: 4897 | CAUAGAA |
| SEQ ID NO: 4898 | CAUAGAC |
| SEQ ID NO: 4899 | CAUAGAG |
| SEQ ID NO: 4900 | CAUAGAU |
| SEQ ID NO: 4901 | CAUAGCA |
| SEQ ID NO: 4902 | CAUAGCC |
| SEQ ID NO: 4903 | CAUAGCG |
| SEQ ID NO: 4904 | CAUAGCU |
| SEQ ID NO: 4905 | CAUAGGA |
| SEQ ID NO: 4906 | CAUAGGC |
| SEQ ID NO: 4907 | CAUAGGG |
| SEQ ID NO: 4908 | CAUAGGU |
| SEQ ID NO: 4909 | CAUAGUA |
| SEQ ID NO: 4910 | CAUAGUC |
| SEQ ID NO: 4911 | CAUAGUG |
| SEQ ID NO: 4912 | CAUAGUU |
| SEQ ID NO: 4913 | CAUAUAA |
| SEQ ID NO: 4914 | CAUAUAC |
| SEQ ID NO: 4915 | CAUAUAG |
| SEQ ID NO: 4916 | CAUAUAU |
| SEQ ID NO: 4917 | CAUAUCA |
| SEQ ID NO: 4918 | CAUAUCC |
| SEQ ID NO: 4919 | CAUAUCG |
| SEQ ID NO: 4920 | CAUAUCU |
| SEQ ID NO: 4921 | CAUAUGA |
| SEQ ID NO: 4922 | CAUAUGC |
| SEQ ID NO: 4923 | CAUAUGG |
| SEQ ID NO: 4924 | CAUAUGU |
| SEQ ID NO: 4925 | CAUAUUA |
| SEQ ID NO: 4926 | CAUAUUC |
| SEQ ID NO: 4927 | CAUAUUG |
| SEQ ID NO: 4928 | CAUAUUU |
| SEQ ID NO: 4929 | CAUCAAA |
| SEQ ID NO: 4930 | CAUCAAC |
| SEQ ID NO: 4931 | CAUCAAG |
| SEQ ID NO: 4932 | CAUCAAU |
| SEQ ID NO: 4933 | CAUCACA |
| SEQ ID NO: 4934 | CAUCACC |
| SEQ ID NO: 4935 | CAUCACG |
| SEQ ID NO: 4936 | CAUCACU |
| SEQ ID NO: 4937 | CAUCAGA |
| SEQ ID NO: 4938 | CAUCAGC |
| SEQ ID NO: 4939 | CAUCAGG |
| SEQ ID NO: 4940 | CAUCAGU |
| SEQ ID NO: 4941 | CAUCAUA |
| SEQ ID NO: 4942 | CAUCAUC |
| SEQ ID NO: 4943 | CAUCAUG |
| SEQ ID NO: 4944 | CAUCAUU |
| SEQ ID NO: 4945 | CAUCCAA |
| SEQ ID NO: 4946 | CAUCCAC |
| SEQ ID NO: 4947 | CAUCCAG |
| SEQ ID NO: 4948 | CAUCCAU |
| SEQ ID NO: 4949 | CAUCCCA |
| SEQ ID NO: 4950 | CAUCCCC |
| SEQ ID NO: 4951 | CAUCCCG |
| SEQ ID NO: 4952 | CAUCCCU |
| SEQ ID NO: 4953 | CAUCCGA |
| SEQ ID NO: 4954 | CAUCCGC |
| SEQ ID NO: 4955 | CAUCCGG |
| SEQ ID NO: 4956 | CAUCCGU |
| SEQ ID NO: 4957 | CAUCCUA |
| SEQ ID NO: 4958 | CAUCCUC |
| SEQ ID NO: 4959 | CAUCCUG |
| SEQ ID NO: 4960 | CAUCCUU |
| SEQ ID NO: 4961 | CAUCGAA |
| SEQ ID NO: 4962 | CAUCGAC |
| SEQ ID NO: 4963 | CAUCGAG |
| SEQ ID NO: 4964 | CAUCGAU |
| SEQ ID NO: 4965 | CAUCGCA |
| SEQ ID NO: 4966 | CAUCGCC |
| SEQ ID NO: 4967 | CAUCGCG |
| SEQ ID NO: 4968 | CAUCGCU |
| SEQ ID NO: 4969 | CAUCGGA |
| SEQ ID NO: 4970 | CAUCGGC |
| SEQ ID NO: 4971 | CAUCGGG |
| SEQ ID NO: 4972 | CAUCGGU |
| SEQ ID NO: 4973 | CAUCGUA |
| SEQ ID NO: 4974 | CAUCGUC |
| SEQ ID NO: 4975 | CAUCGUG |
| SEQ ID NO: 4976 | CAUCGUU |
| SEQ ID NO: 4977 | CAUCUAA |
| SEQ ID NO: 4978 | CAUCUAC |
| SEQ ID NO: 4979 | CAUCUAG |
| SEQ ID NO: 4980 | CAUCUAU |
| SEQ ID NO: 4981 | CAUCUCA |
| SEQ ID NO: 4982 | CAUCUCC |
| SEQ ID NO: 4983 | CAUCUCG |
| SEQ ID NO: 4984 | CAUCUCU |
| SEQ ID NO: 4985 | CAUCUGA |
| SEQ ID NO: 4986 | CAUCUGC |
| SEQ ID NO: 4987 | CAUCUGG |
| SEQ ID NO: 4988 | CAUCUGU |
| SEQ ID NO: 4989 | CAUCUUA |
| SEQ ID NO: 4990 | CAUCUUC |
| SEQ ID NO: 4991 | CAUCUUG |
| SEQ ID NO: 4992 | CAUCUUU |
| SEQ ID NO: 4993 | CAUGAAA |
| SEQ ID NO: 4994 | CAUGAAC |
| SEQ ID NO: 4995 | CAUGAAG |
| SEQ ID NO: 4996 | CAUGAAU |
| SEQ ID NO: 4997 | CAUGACA |
| SEQ ID NO: 4998 | CAUGACC |
| SEQ ID NO: 4999 | CAUGACG |
| SEQ ID NO: 5000 | CAUGACU |
| SEQ ID NO: 5001 | CAUGAGA |
| SEQ ID NO: 5002 | CAUGAGC |
| SEQ ID NO: 5003 | CAUGAGG |
| SEQ ID NO: 5004 | CAUGAGU |
| SEQ ID NO: 5005 | CAUGAUA |
| SEQ ID NO: 5006 | CAUGAUC |
| SEQ ID NO: 5007 | CAUGAUG |
| SEQ ID NO: 5008 | CAUGAUU |
| SEQ ID NO: 5009 | CAUGCAA |
| SEQ ID NO: 5010 | CAUGCAC |
| SEQ ID NO: 5011 | CAUGCAG |
| SEQ ID NO: 5012 | CAUGCAU |
| SEQ ID NO: 5013 | CAUGCCA |
| SEQ ID NO: 5014 | CAUGCCC |
| SEQ ID NO: 5015 | CAUGCCG |
| SEQ ID NO: 5016 | CAUGCCU |
| SEQ ID NO: 5017 | CAUGCGA |
| SEQ ID NO: 5018 | CAUGCGC |
| SEQ ID NO: 5019 | CAUGCGG |
| SEQ ID NO: 5020 | CAUGCGU |
| SEQ ID NO: 5021 | CAUGCUA |
| SEQ ID NO: 5022 | CAUGCUC |
| SEQ ID NO: 5023 | CAUGCUG |
| SEQ ID NO: 5024 | CAUGCUU |
| SEQ ID NO: 5025 | CAUGGAA |
| SEQ ID NO: 5026 | CAUGGAC |
| SEQ ID NO: 5027 | CAUGGAG |
| SEQ ID NO: 5028 | CAUGGAU |
| SEQ ID NO: 5029 | CAUGGCA |
| SEQ ID NO: 5030 | CAUGGCC |
| SEQ ID NO: 5031 | CAUGGCG |
| SEQ ID NO: 5032 | CAUGGCU |
| SEQ ID NO: 5033 | CAUGGGA |
| SEQ ID NO: 5034 | CAUGGGC |
| SEQ ID NO: 5035 | CAUGGGG |
| SEQ ID NO: 5036 | CAUGGGU |
| SEQ ID NO: 5037 | CAUGGUA |
| SEQ ID NO: 5038 | CAUGGUC |
| SEQ ID NO: 5039 | CAUGGUG |
| SEQ ID NO: 5040 | CAUGGUU |
| SEQ ID NO: 5041 | CAUGUAA |
| SEQ ID NO: 5042 | CAUGUAC |
| SEQ ID NO: 5043 | CAUGUAG |
| SEQ ID NO: 5044 | CAUGUAU |
| SEQ ID NO: 5045 | CAUGUCA |
| SEQ ID NO: 5046 | CAUGUCC |
| SEQ ID NO: 5047 | CAUGUCG |
| SEQ ID NO: 5048 | CAUGUCU |
| SEQ ID NO: 5049 | CAUGUGA |
| SEQ ID NO: 5050 | CAUGUGC |
| SEQ ID NO: 5051 | CAUGUGG |
| SEQ ID NO: 5052 | CAUGUGU |
| SEQ ID NO: 5053 | CAUGUUA |
| SEQ ID NO: 5054 | CAUGUUC |
| SEQ ID NO: 5055 | CAUGUUG |
| SEQ ID NO: 5056 | CAUGUUU |
| SEQ ID NO: 5057 | CAUUAAA |
| SEQ ID NO: 5058 | CAUUAAC |
| SEQ ID NO: 5059 | CAUUAAG |
| SEQ ID NO: 5060 | CAUUAAU |
| SEQ ID NO: 5061 | CAUUACA |
| SEQ ID NO: 5062 | CAUUACC |
| SEQ ID NO: 5063 | CAUUACG |
| SEQ ID NO: 5064 | CAUUACU |
| SEQ ID NO: 5065 | CAUUAGA |
| SEQ ID NO: 5066 | CAUUAGC |
| SEQ ID NO: 5067 | CAUUAGG |
| SEQ ID NO: 5068 | CAUUAGU |
| SEQ ID NO: 5069 | CAUUAUA |
| SEQ ID NO: 5070 | CAUUAUC |
| SEQ ID NO: 5071 | CAUUAUG |
| SEQ ID NO: 5072 | CAUUAUU |
| SEQ ID NO: 5073 | CAUUCAA |
| SEQ ID NO: 5074 | CAUUCAC |
| SEQ ID NO: 5075 | CAUUCAG |
| SEQ ID NO: 5076 | CAUUCAU |
| SEQ ID NO: 5077 | CAUUCCA |
| SEQ ID NO: 5078 | CAUUCCC |
| SEQ ID NO: 5079 | CAUUCCG |
| SEQ ID NO: 5080 | CAUUCCU |
| SEQ ID NO: 5081 | CAUUCGA |
| SEQ ID NO: 5082 | CAUUCGC |
| SEQ ID NO: 5083 | CAUUCGG |
| SEQ ID NO: 5084 | CAUUCGU |
| SEQ ID NO: 5085 | CAUUCUA |
| SEQ ID NO: 5086 | CAUUCUC |
| SEQ ID NO: 5087 | CAUUCUG |
| SEQ ID NO: 5088 | CAUUCUU |
| SEQ ID NO: 5089 | CAUUGAA |
| SEQ ID NO: 5090 | CAUUGAC |
| SEQ ID NO: 5091 | CAUUGAG |
| SEQ ID NO: 5092 | CAUUGAU |
| SEQ ID NO: 5093 | CAUUGCA |
| SEQ ID NO: 5094 | CAUUGCC |
| SEQ ID NO: 5095 | CAUUGCG |
| SEQ ID NO: 5096 | CAUUGCU |
| SEQ ID NO: 5097 | CAUUGGA |
| SEQ ID NO: 5098 | CAUUGGC |
| SEQ ID NO: 5099 | CAUUGGG |
| SEQ ID NO: 5100 | CAUUGGU |
| SEQ ID NO: 5101 | CAUUGUA |
| SEQ ID NO: 5102 | CAUUGUC |
| SEQ ID NO: 5103 | CAUUGUG |
| SEQ ID NO: 5104 | CAUUGUU |
| SEQ ID NO: 5105 | CAUUUAA |
| SEQ ID NO: 5106 | CAUUUAC |
| SEQ ID NO: 5107 | CAUUUAG |
| SEQ ID NO: 5108 | CAUUUAU |
| SEQ ID NO: 5109 | CAUUUCA |
| SEQ ID NO: 5110 | CAUUUCC |
| SEQ ID NO: 5111 | CAUUUCG |
| SEQ ID NO: 5112 | CAUUUCU |
| SEQ ID NO: 5113 | CAUUUGA |
| SEQ ID NO: 5114 | CAUUUGC |
| SEQ ID NO: 5115 | CAUUUGG |
| SEQ ID NO: 5116 | CAUUUGU |
| SEQ ID NO: 5117 | CAUUUUA |
| SEQ ID NO: 5118 | CAUUUUC |
| SEQ ID NO: 5119 | CAUUUUG |
| SEQ ID NO: 5120 | CAUUUUU |
| SEQ ID NO: 5121 | CCAAAAA |
| SEQ ID NO: 5122 | CCAAAAC |
| SEQ ID NO: 5123 | CCAAAAG |
| SEQ ID NO: 5124 | CCAAAAU |
| SEQ ID NO: 5125 | CCAAACA |
| SEQ ID NO: 5126 | CCAAACC |
| SEQ ID NO: 5127 | CCAAACG |
| SEQ ID NO: 5128 | CCAAACU |
| SEQ ID NO: 5129 | CCAAAGA |
| SEQ ID NO: 5130 | CCAAAGC |
| SEQ ID NO: 5131 | CCAAAGG |
| SEQ ID NO: 5132 | CCAAAGU |
| SEQ ID NO: 5133 | CCAAAUA |
| SEQ ID NO: 5134 | CCAAAUC |
| SEQ ID NO: 5135 | CCAAAUG |
| SEQ ID NO: 5136 | CCAAAUU |
| SEQ ID NO: 5137 | CCAACAA |
| SEQ ID NO: 5138 | CCAACAC |
| SEQ ID NO: 5139 | CCAACAG |
| SEQ ID NO: 5140 | CCAACAU |
| SEQ ID NO: 5141 | CCAACCA |
| SEQ ID NO: 5142 | CCAACCC |
| SEQ ID NO: 5143 | CCAACCG |
| SEQ ID NO: 5144 | CCAACCU |
| SEQ ID NO: 5145 | CCAACGA |
| SEQ ID NO: 5146 | CCAACGC |
| SEQ ID NO: 5147 | CCAACGG |
| SEQ ID NO: 5148 | CCAACGU |
| SEQ ID NO: 5149 | CCAACUA |
| SEQ ID NO: 5150 | CCAACUC |
| SEQ ID NO: 5151 | CCAACUG |
| SEQ ID NO: 5152 | CCAACUU |
| SEQ ID NO: 5153 | CCAAGAA |
| SEQ ID NO: 5154 | CCAAGAC |
| SEQ ID NO: 5155 | CCAAGAG |
| SEQ ID NO: 5156 | CCAAGAU |
| SEQ ID NO: 5157 | CCAAGCA |
| SEQ ID NO: 5158 | CCAAGCC |
| SEQ ID NO: 5159 | CCAAGCG |
| SEQ ID NO: 5160 | CCAAGCU |
| SEQ ID NO: 5161 | CCAAGGA |
| SEQ ID NO: 5162 | CCAAGGC |
| SEQ ID NO: 5163 | CCAAGGG |
| SEQ ID NO: 5164 | CCAAGGU |
| SEQ ID NO: 5165 | CCAAGUA |
| SEQ ID NO: 5166 | CCAAGUC |
| SEQ ID NO: 5167 | CCAAGUG |
| SEQ ID NO: 5168 | CCAAGUU |
| SEQ ID NO: 5169 | CCAAUAA |
| SEQ ID NO: 5170 | CCAAUAC |
| SEQ ID NO: 5171 | CCAAUAG |
| SEQ ID NO: 5172 | CCAAUAU |
| SEQ ID NO: 5173 | CCAAUCA |
| SEQ ID NO: 5174 | CCAAUCC |
| SEQ ID NO: 5175 | CCAAUCG |
| SEQ ID NO: 5176 | CCAAUCU |
| SEQ ID NO: 5177 | CCAAUGA |
| SEQ ID NO: 5178 | CCAAUGC |
| SEQ ID NO: 5179 | CCAAUGG |
| SEQ ID NO: 5180 | CCAAUGU |
| SEQ ID NO: 5181 | CCAAUUA |
| SEQ ID NO: 5182 | CCAAUUC |
| SEQ ID NO: 5183 | CCAAUUG |
| SEQ ID NO: 5184 | CCAAUUU |
| SEQ ID NO: 5185 | CCACAAA |
| SEQ ID NO: 5186 | CCACAAC |
| SEQ ID NO: 5187 | CCACAAG |
| SEQ ID NO: 5188 | CCACAAU |
| SEQ ID NO: 5189 | CCACACA |
| SEQ ID NO: 5190 | CCACACC |
| SEQ ID NO: 5191 | CCACACG |
| SEQ ID NO: 5192 | CCACACU |
| SEQ ID NO: 5193 | CCACAGA |
| SEQ ID NO: 5194 | CCACAGC |
| SEQ ID NO: 5195 | CCACAGG |
| SEQ ID NO: 5196 | CCACAGU |
| SEQ ID NO: 5197 | CCACAUA |
| SEQ ID NO: 5198 | CCACAUC |
| SEQ ID NO: 5199 | CCACAUG |
| SEQ ID NO: 5200 | CCACAUU |
| SEQ ID NO: 5201 | CCACCAA |
| SEQ ID NO: 5202 | CCACCAC |

Table 18

| | | | | | | | | | | | |
|---|---|---|---|---|---|---|---|---|---|---|---|
| SEQ ID NO: 5203 | CCACCAG | SEQ ID NO: 5254 | CCAGACC | SEQ ID NO: 5305 | CCAGUGA | SEQ ID NO: 5356 | CCAUGGU | SEQ ID NO: 5407 | CCCACUG | SEQ ID NO: 5458 | CCCCCAC |
| SEQ ID NO: 5204 | CCACCAU | SEQ ID NO: 5255 | CCAGACG | SEQ ID NO: 5306 | CCAGUGC | SEQ ID NO: 5357 | CCAUGUA | SEQ ID NO: 5408 | CCCACUU | SEQ ID NO: 5459 | CCCCCAG |
| SEQ ID NO: 5205 | CCACCCA | SEQ ID NO: 5256 | CCAGACU | SEQ ID NO: 5307 | CCAGUGG | SEQ ID NO: 5358 | CCAUGUC | SEQ ID NO: 5409 | CCCAGAA | SEQ ID NO: 5460 | CCCCCAU |
| SEQ ID NO: 5206 | CCACCCC | SEQ ID NO: 5257 | CCAGAGA | SEQ ID NO: 5308 | CCAGUGU | SEQ ID NO: 5359 | CCAUGUG | SEQ ID NO: 5410 | CCCAGAC | SEQ ID NO: 5461 | CCCCCCA |
| SEQ ID NO: 5207 | CCACCCG | SEQ ID NO: 5258 | CCAGAGC | SEQ ID NO: 5309 | CCAGUUA | SEQ ID NO: 5360 | CCAUGUU | SEQ ID NO: 5411 | CCCAGAG | SEQ ID NO: 5462 | CCCCCCC |
| SEQ ID NO: 5208 | CCACCCU | SEQ ID NO: 5259 | CCAGAGG | SEQ ID NO: 5310 | CCAGUUC | SEQ ID NO: 5361 | CCAUUAA | SEQ ID NO: 5412 | CCCAGAU | SEQ ID NO: 5463 | CCCCCCG |
| SEQ ID NO: 5209 | CCACCGA | SEQ ID NO: 5260 | CCAGAGU | SEQ ID NO: 5311 | CCAGUUG | SEQ ID NO: 5362 | CCAUUAC | SEQ ID NO: 5413 | CCCAGCA | SEQ ID NO: 5464 | CCCCCCU |
| SEQ ID NO: 5210 | CCACCGC | SEQ ID NO: 5261 | CCAGAUA | SEQ ID NO: 5312 | CCAGUUU | SEQ ID NO: 5363 | CCAUUAG | SEQ ID NO: 5414 | CCCAGCC | SEQ ID NO: 5465 | CCCCCGA |
| SEQ ID NO: 5211 | CCACCGG | SEQ ID NO: 5262 | CCAGAUC | SEQ ID NO: 5313 | CCAUAAA | SEQ ID NO: 5364 | CCAUUAU | SEQ ID NO: 5415 | CCCAGCG | SEQ ID NO: 5466 | CCCCCGC |
| SEQ ID NO: 5212 | CCACCGU | SEQ ID NO: 5263 | CCAGAUG | SEQ ID NO: 5314 | CCAUAAC | SEQ ID NO: 5365 | CCAUUCA | SEQ ID NO: 5416 | CCCAGCU | SEQ ID NO: 5467 | CCCCCGG |
| SEQ ID NO: 5213 | CCACCUA | SEQ ID NO: 5264 | CCAGAUU | SEQ ID NO: 5315 | CCAUAAG | SEQ ID NO: 5366 | CCAUUCC | SEQ ID NO: 5417 | CCCAGGA | SEQ ID NO: 5468 | CCCCCGU |
| SEQ ID NO: 5214 | CCACCUC | SEQ ID NO: 5265 | CCAGCAA | SEQ ID NO: 5316 | CCAUAAU | SEQ ID NO: 5367 | CCAUUCG | SEQ ID NO: 5418 | CCCAGGC | SEQ ID NO: 5469 | CCCCCUA |
| SEQ ID NO: 5215 | CCACCUG | SEQ ID NO: 5266 | CCAGCAC | SEQ ID NO: 5317 | CCAUACA | SEQ ID NO: 5368 | CCAUUCU | SEQ ID NO: 5419 | CCCAGGG | SEQ ID NO: 5470 | CCCCCUC |
| SEQ ID NO: 5216 | CCACCUU | SEQ ID NO: 5267 | CCAGCAG | SEQ ID NO: 5318 | CCAUACC | SEQ ID NO: 5369 | CCAUUGA | SEQ ID NO: 5420 | CCCAGGU | SEQ ID NO: 5471 | CCCCCUG |
| SEQ ID NO: 5217 | CCACGAA | SEQ ID NO: 5268 | CCAGCAU | SEQ ID NO: 5319 | CCAUACG | SEQ ID NO: 5370 | CCAUUGC | SEQ ID NO: 5421 | CCCAGUA | SEQ ID NO: 5472 | CCCCCUU |
| SEQ ID NO: 5218 | CCACGAC | SEQ ID NO: 5269 | CCAGCCA | SEQ ID NO: 5320 | CCAUACU | SEQ ID NO: 5371 | CCAUUGG | SEQ ID NO: 5422 | CCCAGUC | SEQ ID NO: 5473 | CCCCGAA |
| SEQ ID NO: 5219 | CCACGAG | SEQ ID NO: 5270 | CCAGCCC | SEQ ID NO: 5321 | CCAUAGA | SEQ ID NO: 5372 | CCAUUGU | SEQ ID NO: 5423 | CCCAGUG | SEQ ID NO: 5474 | CCCCGAC |
| SEQ ID NO: 5220 | CCACGAU | SEQ ID NO: 5271 | CCAGCCG | SEQ ID NO: 5322 | CCAUAGC | SEQ ID NO: 5373 | CCAUUUA | SEQ ID NO: 5424 | CCCAGUU | SEQ ID NO: 5475 | CCCCGAG |
| SEQ ID NO: 5221 | CCACGCA | SEQ ID NO: 5272 | CCAGCCU | SEQ ID NO: 5323 | CCAUAGG | SEQ ID NO: 5374 | CCAUUUC | SEQ ID NO: 5425 | CCCAUAA | SEQ ID NO: 5476 | CCCCGAU |
| SEQ ID NO: 5222 | CCACGCC | SEQ ID NO: 5273 | CCAGCGA | SEQ ID NO: 5324 | CCAUAGU | SEQ ID NO: 5375 | CCAUUUG | SEQ ID NO: 5426 | CCCAUAC | SEQ ID NO: 5477 | CCCCGCA |
| SEQ ID NO: 5223 | CCACGCG | SEQ ID NO: 5274 | CCAGCGC | SEQ ID NO: 5325 | CCAUAUA | SEQ ID NO: 5376 | CCAUUUU | SEQ ID NO: 5427 | CCCAUAG | SEQ ID NO: 5478 | CCCCGCC |
| SEQ ID NO: 5224 | CCACGCU | SEQ ID NO: 5275 | CCAGCGG | SEQ ID NO: 5326 | CCAUAUC | SEQ ID NO: 5377 | CCCAAAA | SEQ ID NO: 5428 | CCCAUAU | SEQ ID NO: 5479 | CCCCGCG |
| SEQ ID NO: 5225 | CCACGGA | SEQ ID NO: 5276 | CCAGCGU | SEQ ID NO: 5327 | CCAUAUG | SEQ ID NO: 5378 | CCCAAAC | SEQ ID NO: 5429 | CCCAUCA | SEQ ID NO: 5480 | CCCCGCU |
| SEQ ID NO: 5226 | CCACGGC | SEQ ID NO: 5277 | CCAGCUA | SEQ ID NO: 5328 | CCAUAUU | SEQ ID NO: 5379 | CCCAAAG | SEQ ID NO: 5430 | CCCAUCC | SEQ ID NO: 5481 | CCCCGGA |
| SEQ ID NO: 5227 | CCACGGG | SEQ ID NO: 5278 | CCAGCUC | SEQ ID NO: 5329 | CCAUCAA | SEQ ID NO: 5380 | CCCAAAU | SEQ ID NO: 5431 | CCCAUCG | SEQ ID NO: 5482 | CCCCGGC |
| SEQ ID NO: 5228 | CCACGGU | SEQ ID NO: 5279 | CCAGCUG | SEQ ID NO: 5330 | CCAUCAC | SEQ ID NO: 5381 | CCCAACA | SEQ ID NO: 5432 | CCCAUCU | SEQ ID NO: 5483 | CCCCGGG |
| SEQ ID NO: 5229 | CCACGUA | SEQ ID NO: 5280 | CCAGCUU | SEQ ID NO: 5331 | CCAUCAG | SEQ ID NO: 5382 | CCCAACC | SEQ ID NO: 5433 | CCCAUGA | SEQ ID NO: 5484 | CCCCGGU |
| SEQ ID NO: 5230 | CCACGUC | SEQ ID NO: 5281 | CCAGGAA | SEQ ID NO: 5332 | CCAUCAU | SEQ ID NO: 5383 | CCCAACG | SEQ ID NO: 5434 | CCCAUGC | SEQ ID NO: 5485 | CCCCGUA |
| SEQ ID NO: 5231 | CCACGUG | SEQ ID NO: 5282 | CCAGGAC | SEQ ID NO: 5333 | CCAUCCA | SEQ ID NO: 5384 | CCCAACU | SEQ ID NO: 5435 | CCCAUGG | SEQ ID NO: 5486 | CCCCGUC |
| SEQ ID NO: 5232 | CCACGUU | SEQ ID NO: 5283 | CCAGGAG | SEQ ID NO: 5334 | CCAUCCC | SEQ ID NO: 5385 | CCCAAGA | SEQ ID NO: 5436 | CCCAUGU | SEQ ID NO: 5487 | CCCCGUG |
| SEQ ID NO: 5233 | CCACUAA | SEQ ID NO: 5284 | CCAGGAU | SEQ ID NO: 5335 | CCAUCCG | SEQ ID NO: 5386 | CCCAAGC | SEQ ID NO: 5437 | CCCAUUA | SEQ ID NO: 5488 | CCCCGUU |
| SEQ ID NO: 5234 | CCACUAC | SEQ ID NO: 5285 | CCAGGCA | SEQ ID NO: 5336 | CCAUCCU | SEQ ID NO: 5387 | CCCAAGG | SEQ ID NO: 5438 | CCCAUUC | SEQ ID NO: 5489 | CCCCUAA |
| SEQ ID NO: 5235 | CCACUAG | SEQ ID NO: 5286 | CCAGGCC | SEQ ID NO: 5337 | CCAUCGA | SEQ ID NO: 5388 | CCCAAGU | SEQ ID NO: 5439 | CCCAUUG | SEQ ID NO: 5490 | CCCCUAC |
| SEQ ID NO: 5236 | CCACUAU | SEQ ID NO: 5287 | CCAGGCG | SEQ ID NO: 5338 | CCAUCGC | SEQ ID NO: 5389 | CCCAAUA | SEQ ID NO: 5440 | CCCAUUU | SEQ ID NO: 5491 | CCCCUAG |
| SEQ ID NO: 5237 | CCACUCA | SEQ ID NO: 5288 | CCAGGCU | SEQ ID NO: 5339 | CCAUCGG | SEQ ID NO: 5390 | CCCAAUC | SEQ ID NO: 5441 | CCCCAAA | SEQ ID NO: 5492 | CCCCUAU |
| SEQ ID NO: 5238 | CCACUCC | SEQ ID NO: 5289 | CCAGGGA | SEQ ID NO: 5340 | CCAUCGU | SEQ ID NO: 5391 | CCCAAUG | SEQ ID NO: 5442 | CCCCAAC | SEQ ID NO: 5493 | CCCCUCA |
| SEQ ID NO: 5239 | CCACUCG | SEQ ID NO: 5290 | CCAGGGC | SEQ ID NO: 5341 | CCAUCUA | SEQ ID NO: 5392 | CCCAAUU | SEQ ID NO: 5443 | CCCCAAG | SEQ ID NO: 5494 | CCCCUCC |
| SEQ ID NO: 5240 | CCACUCU | SEQ ID NO: 5291 | CCAGGGG | SEQ ID NO: 5342 | CCAUCUC | SEQ ID NO: 5393 | CCCACAA | SEQ ID NO: 5444 | CCCCAAU | SEQ ID NO: 5495 | CCCCUCG |
| SEQ ID NO: 5241 | CCACUGA | SEQ ID NO: 5292 | CCAGGGU | SEQ ID NO: 5343 | CCAUCUG | SEQ ID NO: 5394 | CCCACAC | SEQ ID NO: 5445 | CCCCACA | SEQ ID NO: 5496 | CCCCUCU |
| SEQ ID NO: 5242 | CCACUGC | SEQ ID NO: 5293 | CCAGGUA | SEQ ID NO: 5344 | CCAUCUU | SEQ ID NO: 5395 | CCCACAG | SEQ ID NO: 5446 | CCCCACC | SEQ ID NO: 5497 | CCCCUGA |
| SEQ ID NO: 5243 | CCACUGG | SEQ ID NO: 5294 | CCAGGUC | SEQ ID NO: 5345 | CCAUGAA | SEQ ID NO: 5396 | CCCACAU | SEQ ID NO: 5447 | CCCCACG | SEQ ID NO: 5498 | CCCCUGC |
| SEQ ID NO: 5244 | CCACUGU | SEQ ID NO: 5295 | CCAGGUG | SEQ ID NO: 5346 | CCAUGAC | SEQ ID NO: 5397 | CCCACCA | SEQ ID NO: 5448 | CCCCACU | SEQ ID NO: 5499 | CCCCUGG |
| SEQ ID NO: 5245 | CCACUUA | SEQ ID NO: 5296 | CCAGGUU | SEQ ID NO: 5347 | CCAUGAG | SEQ ID NO: 5398 | CCCACCC | SEQ ID NO: 5449 | CCCCAGA | SEQ ID NO: 5500 | CCCCUGU |
| SEQ ID NO: 5246 | CCACUUC | SEQ ID NO: 5297 | CCAGUAA | SEQ ID NO: 5348 | CCAUGAU | SEQ ID NO: 5399 | CCCACCG | SEQ ID NO: 5450 | CCCCAGC | SEQ ID NO: 5501 | CCCCUUA |
| SEQ ID NO: 5247 | CCACUUG | SEQ ID NO: 5298 | CCAGUAC | SEQ ID NO: 5349 | CCAUGCA | SEQ ID NO: 5400 | CCCACCU | SEQ ID NO: 5451 | CCCCAGG | SEQ ID NO: 5502 | CCCCUUC |
| SEQ ID NO: 5248 | CCACUUU | SEQ ID NO: 5299 | CCAGUAG | SEQ ID NO: 5350 | CCAUGCC | SEQ ID NO: 5401 | CCCACGA | SEQ ID NO: 5452 | CCCCAGU | SEQ ID NO: 5503 | CCCCUUG |
| SEQ ID NO: 5249 | CCAGAAA | SEQ ID NO: 5300 | CCAGUAU | SEQ ID NO: 5351 | CCAUGCG | SEQ ID NO: 5402 | CCCACGC | SEQ ID NO: 5453 | CCCCAUA | SEQ ID NO: 5504 | CCCCUUU |
| SEQ ID NO: 5250 | CCAGAAC | SEQ ID NO: 5301 | CCAGUCA | SEQ ID NO: 5352 | CCAUGCU | SEQ ID NO: 5403 | CCCACGG | SEQ ID NO: 5454 | CCCCAUC | SEQ ID NO: 5505 | CCCGAAA |
| SEQ ID NO: 5251 | CCAGAAG | SEQ ID NO: 5302 | CCAGUCC | SEQ ID NO: 5353 | CCAUGGA | SEQ ID NO: 5404 | CCCACGU | SEQ ID NO: 5455 | CCCCAUG | SEQ ID NO: 5506 | CCCGAAC |
| SEQ ID NO: 5252 | CCAGAAU | SEQ ID NO: 5303 | CCAGUCG | SEQ ID NO: 5354 | CCAUGGC | SEQ ID NO: 5405 | CCCACUA | SEQ ID NO: 5456 | CCCCAUU | SEQ ID NO: 5507 | CCCGAAG |
| SEQ ID NO: 5253 | CCAGACA | SEQ ID NO: 5304 | CCAGUCU | SEQ ID NO: 5355 | CCAUGGG | SEQ ID NO: 5406 | CCCACUC | SEQ ID NO: 5457 | CCCCCAA | SEQ ID NO: 5508 | CCCGAAU |

Table 19

| | | | | | | | | | | | |
|---|---|---|---|---|---|---|---|---|---|---|---|
| SEQ ID NO: 5509 | CCCGACA | SEQ ID NO: 5560 | CCCGUCU | SEQ ID NO: 5611 | CCCUGGG | SEQ ID NO: 5662 | CCGACUC | SEQ ID NO: 5713 | CCGCCAA | SEQ ID NO: 5764 | CCGGAAU |
| SEQ ID NO: 5510 | CCCGACC | SEQ ID NO: 5561 | CCCGUGA | SEQ ID NO: 5612 | CCCUGGU | SEQ ID NO: 5663 | CCGACUG | SEQ ID NO: 5714 | CCGCCAC | SEQ ID NO: 5765 | CCGGACA |
| SEQ ID NO: 5511 | CCCGACG | SEQ ID NO: 5562 | CCCGUGC | SEQ ID NO: 5613 | CCCUGUA | SEQ ID NO: 5664 | CCGACUU | SEQ ID NO: 5715 | CCGCCAG | SEQ ID NO: 5766 | CCGGACC |
| SEQ ID NO: 5512 | CCCGACU | SEQ ID NO: 5563 | CCCGUGG | SEQ ID NO: 5614 | CCCUGUC | SEQ ID NO: 5665 | CCGAGAA | SEQ ID NO: 5716 | CCGCCAU | SEQ ID NO: 5767 | CCGGACG |
| SEQ ID NO: 5513 | CCCGAGA | SEQ ID NO: 5564 | CCCGUGU | SEQ ID NO: 5615 | CCCUGUG | SEQ ID NO: 5666 | CCGAGAC | SEQ ID NO: 5717 | CCGCCCA | SEQ ID NO: 5768 | CCGGACU |
| SEQ ID NO: 5514 | CCCGAGC | SEQ ID NO: 5565 | CCCGUUA | SEQ ID NO: 5616 | CCCUGUU | SEQ ID NO: 5667 | CCGAGAG | SEQ ID NO: 5718 | CCGCCCC | SEQ ID NO: 5769 | CCGGAGA |
| SEQ ID NO: 5515 | CCCGAGG | SEQ ID NO: 5566 | CCCGUUC | SEQ ID NO: 5617 | CCCUUAA | SEQ ID NO: 5668 | CCGAGAU | SEQ ID NO: 5719 | CCGCCCG | SEQ ID NO: 5770 | CCGGAGC |
| SEQ ID NO: 5516 | CCCGAGU | SEQ ID NO: 5567 | CCCGUUG | SEQ ID NO: 5618 | CCCUUAC | SEQ ID NO: 5669 | CCGAGCA | SEQ ID NO: 5720 | CCGCCCU | SEQ ID NO: 5771 | CCGGAGG |
| SEQ ID NO: 5517 | CCCGAUA | SEQ ID NO: 5568 | CCCGUUU | SEQ ID NO: 5619 | CCCUUAG | SEQ ID NO: 5670 | CCGAGCC | SEQ ID NO: 5721 | CCGCCGA | SEQ ID NO: 5772 | CCGGAGU |
| SEQ ID NO: 5518 | CCCGAUC | SEQ ID NO: 5569 | CCCUAAA | SEQ ID NO: 5620 | CCCUUAU | SEQ ID NO: 5671 | CCGAGCG | SEQ ID NO: 5722 | CCGCCGC | SEQ ID NO: 5773 | CCGGAUA |
| SEQ ID NO: 5519 | CCCGAUG | SEQ ID NO: 5570 | CCCUAAC | SEQ ID NO: 5621 | CCCUUCA | SEQ ID NO: 5672 | CCGAGCU | SEQ ID NO: 5723 | CCGCCGG | SEQ ID NO: 5774 | CCGGAUC |
| SEQ ID NO: 5520 | CCCGAUU | SEQ ID NO: 5571 | CCCUAAG | SEQ ID NO: 5622 | CCCUUCC | SEQ ID NO: 5673 | CCGAGGA | SEQ ID NO: 5724 | CCGCCGU | SEQ ID NO: 5775 | CCGGAUG |
| SEQ ID NO: 5521 | CCCGCAA | SEQ ID NO: 5572 | CCCUAAU | SEQ ID NO: 5623 | CCCUUCG | SEQ ID NO: 5674 | CCGAGGC | SEQ ID NO: 5725 | CCGCCUA | SEQ ID NO: 5776 | CCGGAUU |
| SEQ ID NO: 5522 | CCCGCAC | SEQ ID NO: 5573 | CCCUACA | SEQ ID NO: 5624 | CCCUUCU | SEQ ID NO: 5675 | CCGAGGG | SEQ ID NO: 5726 | CCGCCUC | SEQ ID NO: 5777 | CCGGCAA |
| SEQ ID NO: 5523 | CCCGCAG | SEQ ID NO: 5574 | CCCUACC | SEQ ID NO: 5625 | CCCUUGA | SEQ ID NO: 5676 | CCGAGGU | SEQ ID NO: 5727 | CCGCCUG | SEQ ID NO: 5778 | CCGGCAC |
| SEQ ID NO: 5524 | CCCGCAU | SEQ ID NO: 5575 | CCCUACG | SEQ ID NO: 5626 | CCCUUGC | SEQ ID NO: 5677 | CCGAGUA | SEQ ID NO: 5728 | CCGCCUU | SEQ ID NO: 5779 | CCGGCAG |
| SEQ ID NO: 5525 | CCCGCCA | SEQ ID NO: 5576 | CCCUACU | SEQ ID NO: 5627 | CCCUUGG | SEQ ID NO: 5678 | CCGAGUC | SEQ ID NO: 5729 | CCGCGAA | SEQ ID NO: 5780 | CCGGCAU |
| SEQ ID NO: 5526 | CCCGCCC | SEQ ID NO: 5577 | CCCUAGA | SEQ ID NO: 5628 | CCCUUGU | SEQ ID NO: 5679 | CCGAGUG | SEQ ID NO: 5730 | CCGCGAC | SEQ ID NO: 5781 | CCGGCCA |
| SEQ ID NO: 5527 | CCCGCCG | SEQ ID NO: 5578 | CCCUAGC | SEQ ID NO: 5629 | CCCUUUA | SEQ ID NO: 5680 | CCGAGUU | SEQ ID NO: 5731 | CCGCGAG | SEQ ID NO: 5782 | CCGGCCC |
| SEQ ID NO: 5528 | CCCGCCU | SEQ ID NO: 5579 | CCCUAGG | SEQ ID NO: 5630 | CCCUUUC | SEQ ID NO: 5681 | CCGAUAA | SEQ ID NO: 5732 | CCGCGAU | SEQ ID NO: 5783 | CCGGCCG |
| SEQ ID NO: 5529 | CCCGCGA | SEQ ID NO: 5580 | CCCUAGU | SEQ ID NO: 5631 | CCCUUUG | SEQ ID NO: 5682 | CCGAUAC | SEQ ID NO: 5733 | CCGCGCA | SEQ ID NO: 5784 | CCGGCCU |
| SEQ ID NO: 5530 | CCCGCGC | SEQ ID NO: 5581 | CCCUAUA | SEQ ID NO: 5632 | CCCUUUU | SEQ ID NO: 5683 | CCGAUAG | SEQ ID NO: 5734 | CCGCGCC | SEQ ID NO: 5785 | CCGGCGA |
| SEQ ID NO: 5531 | CCCGCGG | SEQ ID NO: 5582 | CCCUAUC | SEQ ID NO: 5633 | CCGAAAA | SEQ ID NO: 5684 | CCGAUAU | SEQ ID NO: 5735 | CCGCGCG | SEQ ID NO: 5786 | CCGGCGC |
| SEQ ID NO: 5532 | CCCGCGU | SEQ ID NO: 5583 | CCCUAUG | SEQ ID NO: 5634 | CCGAAAC | SEQ ID NO: 5685 | CCGAUCA | SEQ ID NO: 5736 | CCGCGCU | SEQ ID NO: 5787 | CCGGCGG |
| SEQ ID NO: 5533 | CCCGCUA | SEQ ID NO: 5584 | CCCUAUU | SEQ ID NO: 5635 | CCGAAAG | SEQ ID NO: 5686 | CCGAUCC | SEQ ID NO: 5737 | CCGCGGA | SEQ ID NO: 5788 | CCGGCGU |
| SEQ ID NO: 5534 | CCCGCUC | SEQ ID NO: 5585 | CCCUCAA | SEQ ID NO: 5636 | CCGAAAU | SEQ ID NO: 5687 | CCGAUCG | SEQ ID NO: 5738 | CCGCGGC | SEQ ID NO: 5789 | CCGGCUA |
| SEQ ID NO: 5535 | CCCGCUG | SEQ ID NO: 5586 | CCCUCAC | SEQ ID NO: 5637 | CCGAACA | SEQ ID NO: 5688 | CCGAUCU | SEQ ID NO: 5739 | CCGCGGG | SEQ ID NO: 5790 | CCGGCUC |
| SEQ ID NO: 5536 | CCCGCUU | SEQ ID NO: 5587 | CCCUCAG | SEQ ID NO: 5638 | CCGAACC | SEQ ID NO: 5689 | CCGAUGA | SEQ ID NO: 5740 | CCGCGGU | SEQ ID NO: 5791 | CCGGCUG |
| SEQ ID NO: 5537 | CCCGGAA | SEQ ID NO: 5588 | CCCUCAU | SEQ ID NO: 5639 | CCGAACG | SEQ ID NO: 5690 | CCGAUGC | SEQ ID NO: 5741 | CCGCGUA | SEQ ID NO: 5792 | CCGGCUU |
| SEQ ID NO: 5538 | CCCGGAC | SEQ ID NO: 5589 | CCCUCCA | SEQ ID NO: 5640 | CCGAACU | SEQ ID NO: 5691 | CCGAUGG | SEQ ID NO: 5742 | CCGCGUC | SEQ ID NO: 5793 | CCGGGAA |
| SEQ ID NO: 5539 | CCCGGAG | SEQ ID NO: 5590 | CCCUCCC | SEQ ID NO: 5641 | CCGAAGA | SEQ ID NO: 5692 | CCGAUGU | SEQ ID NO: 5743 | CCGCGUG | SEQ ID NO: 5794 | CCGGGAC |
| SEQ ID NO: 5540 | CCCGGAU | SEQ ID NO: 5591 | CCCUCCG | SEQ ID NO: 5642 | CCGAAGC | SEQ ID NO: 5693 | CCGAUUA | SEQ ID NO: 5744 | CCGCGUU | SEQ ID NO: 5795 | CCGGGAG |
| SEQ ID NO: 5541 | CCCGGCA | SEQ ID NO: 5592 | CCCUCCU | SEQ ID NO: 5643 | CCGAAGG | SEQ ID NO: 5694 | CCGAUUC | SEQ ID NO: 5745 | CCGCUAA | SEQ ID NO: 5796 | CCGGGAU |
| SEQ ID NO: 5542 | CCCGGCC | SEQ ID NO: 5593 | CCCUCGA | SEQ ID NO: 5644 | CCGAAGU | SEQ ID NO: 5695 | CCGAUUG | SEQ ID NO: 5746 | CCGCUAC | SEQ ID NO: 5797 | CCGGGCA |
| SEQ ID NO: 5543 | CCCGGCG | SEQ ID NO: 5594 | CCCUCGC | SEQ ID NO: 5645 | CCGAAUA | SEQ ID NO: 5696 | CCGAUUU | SEQ ID NO: 5747 | CCGCUAG | SEQ ID NO: 5798 | CCGGGCC |
| SEQ ID NO: 5544 | CCCGGCU | SEQ ID NO: 5595 | CCCUCGG | SEQ ID NO: 5646 | CCGAAUC | SEQ ID NO: 5697 | CCGCAAA | SEQ ID NO: 5748 | CCGCUAU | SEQ ID NO: 5799 | CCGGGCG |
| SEQ ID NO: 5545 | CCCGGGA | SEQ ID NO: 5596 | CCCUCGU | SEQ ID NO: 5647 | CCGAAUG | SEQ ID NO: 5698 | CCGCAAC | SEQ ID NO: 5749 | CCGCUCA | SEQ ID NO: 5800 | CCGGGCU |
| SEQ ID NO: 5546 | CCCGGGC | SEQ ID NO: 5597 | CCCUCUA | SEQ ID NO: 5648 | CCGAAUU | SEQ ID NO: 5699 | CCGCAAG | SEQ ID NO: 5750 | CCGCUCC | SEQ ID NO: 5801 | CCGGGGA |
| SEQ ID NO: 5547 | CCCGGGG | SEQ ID NO: 5598 | CCCUCUC | SEQ ID NO: 5649 | CCGACAA | SEQ ID NO: 5700 | CCGCAAU | SEQ ID NO: 5751 | CCGCUCG | SEQ ID NO: 5802 | CCGGGGC |
| SEQ ID NO: 5548 | CCCGGGU | SEQ ID NO: 5599 | CCCUCUG | SEQ ID NO: 5650 | CCGACAC | SEQ ID NO: 5701 | CCGCACA | SEQ ID NO: 5752 | CCGCUCU | SEQ ID NO: 5803 | CCGGGGG |
| SEQ ID NO: 5549 | CCCGGUA | SEQ ID NO: 5600 | CCCUCUU | SEQ ID NO: 5651 | CCGACAG | SEQ ID NO: 5702 | CCGCACC | SEQ ID NO: 5753 | CCGCUGA | SEQ ID NO: 5804 | CCGGGGU |
| SEQ ID NO: 5550 | CCCGGUC | SEQ ID NO: 5601 | CCCUGAA | SEQ ID NO: 5652 | CCGACAU | SEQ ID NO: 5703 | CCGCACG | SEQ ID NO: 5754 | CCGCUGC | SEQ ID NO: 5805 | CCGGGUA |
| SEQ ID NO: 5551 | CCCGGUG | SEQ ID NO: 5602 | CCCUGAC | SEQ ID NO: 5653 | CCGACCA | SEQ ID NO: 5704 | CCGCACU | SEQ ID NO: 5755 | CCGCUGG | SEQ ID NO: 5806 | CCGGGUC |
| SEQ ID NO: 5552 | CCCGGUU | SEQ ID NO: 5603 | CCCUGAG | SEQ ID NO: 5654 | CCGACCC | SEQ ID NO: 5705 | CCGCAGA | SEQ ID NO: 5756 | CCGCUGU | SEQ ID NO: 5807 | CCGGGUG |
| SEQ ID NO: 5553 | CCCGUAA | SEQ ID NO: 5604 | CCCUGAU | SEQ ID NO: 5655 | CCGACCG | SEQ ID NO: 5706 | CCGCAGC | SEQ ID NO: 5757 | CCGCUUA | SEQ ID NO: 5808 | CCGGGUU |
| SEQ ID NO: 5554 | CCCGUAC | SEQ ID NO: 5605 | CCCUGCA | SEQ ID NO: 5656 | CCGACCU | SEQ ID NO: 5707 | CCGCAGG | SEQ ID NO: 5758 | CCGCUUC | SEQ ID NO: 5809 | CCGGUAA |
| SEQ ID NO: 5555 | CCCGUAG | SEQ ID NO: 5606 | CCCUGCC | SEQ ID NO: 5657 | CCGACGA | SEQ ID NO: 5708 | CCGCAGU | SEQ ID NO: 5759 | CCGCUUG | SEQ ID NO: 5810 | CCGGUAC |
| SEQ ID NO: 5556 | CCCGUAU | SEQ ID NO: 5607 | CCCUGCG | SEQ ID NO: 5658 | CCGACGC | SEQ ID NO: 5709 | CCGCAUA | SEQ ID NO: 5760 | CCGCUUU | SEQ ID NO: 5811 | CCGGUAG |
| SEQ ID NO: 5557 | CCCGUCA | SEQ ID NO: 5608 | CCCUGCU | SEQ ID NO: 5659 | CCGACGG | SEQ ID NO: 5710 | CCGCAUC | SEQ ID NO: 5761 | CCGGAAA | SEQ ID NO: 5812 | CCGGUAU |
| SEQ ID NO: 5558 | CCCGUCC | SEQ ID NO: 5609 | CCCUGGA | SEQ ID NO: 5660 | CCGACGU | SEQ ID NO: 5711 | CCGCAUG | SEQ ID NO: 5762 | CCGGAAC | SEQ ID NO: 5813 | CCGGUCA |
| SEQ ID NO: 5559 | CCCGUCG | SEQ ID NO: 5610 | CCCUGGC | SEQ ID NO: 5661 | CCGACUA | SEQ ID NO: 5712 | CCGCAUU | SEQ ID NO: 5763 | CCGGAAG | SEQ ID NO: 5814 | CCGGUCC |

Table 20

| | | | | | | | | | | | |
|---|---|---|---|---|---|---|---|---|---|---|---|
| SEQ ID NO: 5815 | CCGGUCG | SEQ ID NO: 5866 | CCGUGGC | SEQ ID NO: 5917 | CCUACUA | SEQ ID NO: 5968 | CCUCAUU | SEQ ID NO: 6019 | CCUGAAG | SEQ ID NO: 6070 | CCUGUCC |
| SEQ ID NO: 5816 | CCGGUCU | SEQ ID NO: 5867 | CCGUGGG | SEQ ID NO: 5918 | CCUACUC | SEQ ID NO: 5969 | CCUCCAA | SEQ ID NO: 6020 | CCUGAAU | SEQ ID NO: 6071 | CCUGUCG |
| SEQ ID NO: 5817 | CCGGUGA | SEQ ID NO: 5868 | CCGUGGU | SEQ ID NO: 5919 | CCUACUG | SEQ ID NO: 5970 | CCUCCAC | SEQ ID NO: 6021 | CCUGACA | SEQ ID NO: 6072 | CCUGUCU |
| SEQ ID NO: 5818 | CCGGUGC | SEQ ID NO: 5869 | CCGUGUA | SEQ ID NO: 5920 | CCUACUU | SEQ ID NO: 5971 | CCUCCAG | SEQ ID NO: 6022 | CCUGACC | SEQ ID NO: 6073 | CCUGUGA |
| SEQ ID NO: 5819 | CCGGUGG | SEQ ID NO: 5870 | CCGUGUC | SEQ ID NO: 5921 | CCUAGAA | SEQ ID NO: 5972 | CCUCCAU | SEQ ID NO: 6023 | CCUGACG | SEQ ID NO: 6074 | CCUGUGC |
| SEQ ID NO: 5820 | CCGGUGU | SEQ ID NO: 5871 | CCGUGUG | SEQ ID NO: 5922 | CCUAGAC | SEQ ID NO: 5973 | CCUCCCA | SEQ ID NO: 6024 | CCUGACU | SEQ ID NO: 6075 | CCUGUGG |
| SEQ ID NO: 5821 | CCGGUUA | SEQ ID NO: 5872 | CCGUGUU | SEQ ID NO: 5923 | CCUAGAG | SEQ ID NO: 5974 | CCUCCCC | SEQ ID NO: 6025 | CCUGAGA | SEQ ID NO: 6076 | CCUGUGU |
| SEQ ID NO: 5822 | CCGGUUC | SEQ ID NO: 5873 | CCGUUAA | SEQ ID NO: 5924 | CCUAGAU | SEQ ID NO: 5975 | CCUCCCG | SEQ ID NO: 6026 | CCUGAGC | SEQ ID NO: 6077 | CCUGUUA |
| SEQ ID NO: 5823 | CCGGUUG | SEQ ID NO: 5874 | CCGUUAC | SEQ ID NO: 5925 | CCUAGCA | SEQ ID NO: 5976 | CCUCCCU | SEQ ID NO: 6027 | CCUGAGG | SEQ ID NO: 6078 | CCUGUUC |
| SEQ ID NO: 5824 | CCGGUUU | SEQ ID NO: 5875 | CCGUUAG | SEQ ID NO: 5926 | CCUAGCC | SEQ ID NO: 5977 | CCUCCGA | SEQ ID NO: 6028 | CCUGAGU | SEQ ID NO: 6079 | CCUGUUG |
| SEQ ID NO: 5825 | CCGUAAA | SEQ ID NO: 5876 | CCGUUAU | SEQ ID NO: 5927 | CCUAGCG | SEQ ID NO: 5978 | CCUCCGC | SEQ ID NO: 6029 | CCUGAUA | SEQ ID NO: 6080 | CCUGUUU |
| SEQ ID NO: 5826 | CCGUAAC | SEQ ID NO: 5877 | CCGUUCA | SEQ ID NO: 5928 | CCUAGCU | SEQ ID NO: 5979 | CCUCCGG | SEQ ID NO: 6030 | CCUGAUC | SEQ ID NO: 6081 | CCUUAAA |
| SEQ ID NO: 5827 | CCGUAAG | SEQ ID NO: 5878 | CCGUUCC | SEQ ID NO: 5929 | CCUAGGA | SEQ ID NO: 5980 | CCUCCGU | SEQ ID NO: 6031 | CCUGAUG | SEQ ID NO: 6082 | CCUUAAC |
| SEQ ID NO: 5828 | CCGUAAU | SEQ ID NO: 5879 | CCGUUCG | SEQ ID NO: 5930 | CCUAGGC | SEQ ID NO: 5981 | CCUCCUA | SEQ ID NO: 6032 | CCUGAUU | SEQ ID NO: 6083 | CCUUAAG |
| SEQ ID NO: 5829 | CCGUACA | SEQ ID NO: 5880 | CCGUUCU | SEQ ID NO: 5931 | CCUAGGG | SEQ ID NO: 5982 | CCUCCUC | SEQ ID NO: 6033 | CCUGCAA | SEQ ID NO: 6084 | CCUUAAU |
| SEQ ID NO: 5830 | CCGUACC | SEQ ID NO: 5881 | CCGUUGA | SEQ ID NO: 5932 | CCUAGGU | SEQ ID NO: 5983 | CCUCCUG | SEQ ID NO: 6034 | CCUGCAC | SEQ ID NO: 6085 | CCUUACA |
| SEQ ID NO: 5831 | CCGUACG | SEQ ID NO: 5882 | CCGUUGC | SEQ ID NO: 5933 | CCUAGUA | SEQ ID NO: 5984 | CCUCCUU | SEQ ID NO: 6035 | CCUGCAG | SEQ ID NO: 6086 | CCUUACC |
| SEQ ID NO: 5832 | CCGUACU | SEQ ID NO: 5883 | CCGUUGG | SEQ ID NO: 5934 | CCUAGUC | SEQ ID NO: 5985 | CCUCGAA | SEQ ID NO: 6036 | CCUGCAU | SEQ ID NO: 6087 | CCUUACG |
| SEQ ID NO: 5833 | CCGUAGA | SEQ ID NO: 5884 | CCGUUGU | SEQ ID NO: 5935 | CCUAGUG | SEQ ID NO: 5986 | CCUCGAC | SEQ ID NO: 6037 | CCUGCCA | SEQ ID NO: 6088 | CCUUACU |
| SEQ ID NO: 5834 | CCGUAGC | SEQ ID NO: 5885 | CCGUUUA | SEQ ID NO: 5936 | CCUAGUU | SEQ ID NO: 5987 | CCUCGAG | SEQ ID NO: 6038 | CCUGCCC | SEQ ID NO: 6089 | CCUUAGA |
| SEQ ID NO: 5835 | CCGUAGG | SEQ ID NO: 5886 | CCGUUUC | SEQ ID NO: 5937 | CCUAUAA | SEQ ID NO: 5988 | CCUCGAU | SEQ ID NO: 6039 | CCUGCCG | SEQ ID NO: 6090 | CCUUAGC |
| SEQ ID NO: 5836 | CCGUAGU | SEQ ID NO: 5887 | CCGUUUG | SEQ ID NO: 5938 | CCUAUAC | SEQ ID NO: 5989 | CCUCGCA | SEQ ID NO: 6040 | CCUGCCU | SEQ ID NO: 6091 | CCUUAGG |
| SEQ ID NO: 5837 | CCGUAUA | SEQ ID NO: 5888 | CCGUUUU | SEQ ID NO: 5939 | CCUAUAG | SEQ ID NO: 5990 | CCUCGCC | SEQ ID NO: 6041 | CCUGCGA | SEQ ID NO: 6092 | CCUUAGU |
| SEQ ID NO: 5838 | CCGUAUC | SEQ ID NO: 5889 | CCUAAAA | SEQ ID NO: 5940 | CCUAUAU | SEQ ID NO: 5991 | CCUCGCG | SEQ ID NO: 6042 | CCUGCGC | SEQ ID NO: 6093 | CCUUAUA |
| SEQ ID NO: 5839 | CCGUAUG | SEQ ID NO: 5890 | CCUAAAC | SEQ ID NO: 5941 | CCUAUCA | SEQ ID NO: 5992 | CCUCGCU | SEQ ID NO: 6043 | CCUGCGG | SEQ ID NO: 6094 | CCUUAUC |
| SEQ ID NO: 5840 | CCGUAUU | SEQ ID NO: 5891 | CCUAAAG | SEQ ID NO: 5942 | CCUAUCC | SEQ ID NO: 5993 | CCUCGGA | SEQ ID NO: 6044 | CCUGCGU | SEQ ID NO: 6095 | CCUUAUG |
| SEQ ID NO: 5841 | CCGUCAA | SEQ ID NO: 5892 | CCUAAAU | SEQ ID NO: 5943 | CCUAUCG | SEQ ID NO: 5994 | CCUCGGC | SEQ ID NO: 6045 | CCUGCUA | SEQ ID NO: 6096 | CCUUAUU |
| SEQ ID NO: 5842 | CCGUCAC | SEQ ID NO: 5893 | CCUAACA | SEQ ID NO: 5944 | CCUAUCU | SEQ ID NO: 5995 | CCUCGGG | SEQ ID NO: 6046 | CCUGCUC | SEQ ID NO: 6097 | CCUUCAA |
| SEQ ID NO: 5843 | CCGUCAG | SEQ ID NO: 5894 | CCUAACC | SEQ ID NO: 5945 | CCUAUGA | SEQ ID NO: 5996 | CCUCGGU | SEQ ID NO: 6047 | CCUGCUG | SEQ ID NO: 6098 | CCUUCAC |
| SEQ ID NO: 5844 | CCGUCAU | SEQ ID NO: 5895 | CCUAACG | SEQ ID NO: 5946 | CCUAUGC | SEQ ID NO: 5997 | CCUCGUA | SEQ ID NO: 6048 | CCUGCUU | SEQ ID NO: 6099 | CCUUCAG |
| SEQ ID NO: 5845 | CCGUCCA | SEQ ID NO: 5896 | CCUAACU | SEQ ID NO: 5947 | CCUAUGG | SEQ ID NO: 5998 | CCUCGUC | SEQ ID NO: 6049 | CCUGGAA | SEQ ID NO: 6100 | CCUUCAU |
| SEQ ID NO: 5846 | CCGUCCC | SEQ ID NO: 5897 | CCUAAGA | SEQ ID NO: 5948 | CCUAUGU | SEQ ID NO: 5999 | CCUCGUG | SEQ ID NO: 6050 | CCUGGAC | SEQ ID NO: 6101 | CCUUCCA |
| SEQ ID NO: 5847 | CCGUCCG | SEQ ID NO: 5898 | CCUAAGC | SEQ ID NO: 5949 | CCUAUUA | SEQ ID NO: 6000 | CCUCGUU | SEQ ID NO: 6051 | CCUGGAG | SEQ ID NO: 6102 | CCUUCCC |
| SEQ ID NO: 5848 | CCGUCCU | SEQ ID NO: 5899 | CCUAAGG | SEQ ID NO: 5950 | CCUAUUC | SEQ ID NO: 6001 | CCUCUAA | SEQ ID NO: 6052 | CCUGGAU | SEQ ID NO: 6103 | CCUUCCG |
| SEQ ID NO: 5849 | CCGUCGA | SEQ ID NO: 5900 | CCUAAGU | SEQ ID NO: 5951 | CCUAUUG | SEQ ID NO: 6002 | CCUCUAC | SEQ ID NO: 6053 | CCUGGCA | SEQ ID NO: 6104 | CCUUCCU |
| SEQ ID NO: 5850 | CCGUCGC | SEQ ID NO: 5901 | CCUAAUA | SEQ ID NO: 5952 | CCUAUUU | SEQ ID NO: 6003 | CCUCUAG | SEQ ID NO: 6054 | CCUGGCC | SEQ ID NO: 6105 | CCUUCGA |
| SEQ ID NO: 5851 | CCGUCGG | SEQ ID NO: 5902 | CCUAAUC | SEQ ID NO: 5953 | CCUCAAA | SEQ ID NO: 6004 | CCUCUAU | SEQ ID NO: 6055 | CCUGGCG | SEQ ID NO: 6106 | CCUUCGC |
| SEQ ID NO: 5852 | CCGUCGU | SEQ ID NO: 5903 | CCUAAUG | SEQ ID NO: 5954 | CCUCAAC | SEQ ID NO: 6005 | CCUCUCA | SEQ ID NO: 6056 | CCUGGCU | SEQ ID NO: 6107 | CCUUCGG |
| SEQ ID NO: 5853 | CCGUCUA | SEQ ID NO: 5904 | CCUAAUU | SEQ ID NO: 5955 | CCUCAAG | SEQ ID NO: 6006 | CCUCUCC | SEQ ID NO: 6057 | CCUGGGA | SEQ ID NO: 6108 | CCUUCGU |
| SEQ ID NO: 5854 | CCGUCUC | SEQ ID NO: 5905 | CCUACAA | SEQ ID NO: 5956 | CCUCAAU | SEQ ID NO: 6007 | CCUCUCG | SEQ ID NO: 6058 | CCUGGGC | SEQ ID NO: 6109 | CCUUCUA |
| SEQ ID NO: 5855 | CCGUCUG | SEQ ID NO: 5906 | CCUACAC | SEQ ID NO: 5957 | CCUCACA | SEQ ID NO: 6008 | CCUCUCU | SEQ ID NO: 6059 | CCUGGGG | SEQ ID NO: 6110 | CCUUCUC |
| SEQ ID NO: 5856 | CCGUCUU | SEQ ID NO: 5907 | CCUACAG | SEQ ID NO: 5958 | CCUCACC | SEQ ID NO: 6009 | CCUCUGA | SEQ ID NO: 6060 | CCUGGGU | SEQ ID NO: 6111 | CCUUCUG |
| SEQ ID NO: 5857 | CCGUGAA | SEQ ID NO: 5908 | CCUACAU | SEQ ID NO: 5959 | CCUCACG | SEQ ID NO: 6010 | CCUCUGC | SEQ ID NO: 6061 | CCUGGUA | SEQ ID NO: 6112 | CCUUCUU |
| SEQ ID NO: 5858 | CCGUGAC | SEQ ID NO: 5909 | CCUACCA | SEQ ID NO: 5960 | CCUCACU | SEQ ID NO: 6011 | CCUCUGG | SEQ ID NO: 6062 | CCUGGUC | SEQ ID NO: 6113 | CCUUGAA |
| SEQ ID NO: 5859 | CCGUGAG | SEQ ID NO: 5910 | CCUACCC | SEQ ID NO: 5961 | CCUCAGA | SEQ ID NO: 6012 | CCUCUGU | SEQ ID NO: 6063 | CCUGGUG | SEQ ID NO: 6114 | CCUUGAC |
| SEQ ID NO: 5860 | CCGUGAU | SEQ ID NO: 5911 | CCUACCG | SEQ ID NO: 5962 | CCUCAGC | SEQ ID NO: 6013 | CCUCUUA | SEQ ID NO: 6064 | CCUGGUU | SEQ ID NO: 6115 | CCUUGAG |
| SEQ ID NO: 5861 | CCGUGCA | SEQ ID NO: 5912 | CCUACCU | SEQ ID NO: 5963 | CCUCAGG | SEQ ID NO: 6014 | CCUCUUC | SEQ ID NO: 6065 | CCUGUAA | SEQ ID NO: 6116 | CCUUGAU |
| SEQ ID NO: 5862 | CCGUGCC | SEQ ID NO: 5913 | CCUACGA | SEQ ID NO: 5964 | CCUCAGU | SEQ ID NO: 6015 | CCUCUUG | SEQ ID NO: 6066 | CCUGUAC | SEQ ID NO: 6117 | CCUUGCA |
| SEQ ID NO: 5863 | CCGUGCG | SEQ ID NO: 5914 | CCUACGC | SEQ ID NO: 5965 | CCUCAUA | SEQ ID NO: 6016 | CCUCUUU | SEQ ID NO: 6067 | CCUGUAG | SEQ ID NO: 6118 | CCUUGCC |
| SEQ ID NO: 5864 | CCGUGCU | SEQ ID NO: 5915 | CCUACGG | SEQ ID NO: 5966 | CCUCAUC | SEQ ID NO: 6017 | CCUGAAA | SEQ ID NO: 6068 | CCUGUAU | SEQ ID NO: 6119 | CCUUGCG |
| SEQ ID NO: 5865 | CCGUGGA | SEQ ID NO: 5916 | CCUACGU | SEQ ID NO: 5967 | CCUCAUG | SEQ ID NO: 6018 | CCUGAAC | SEQ ID NO: 6069 | CCUGUCA | SEQ ID NO: 6120 | CCUUGCU |

Table 21

| | | | | | | | | | | | |
|---|---|---|---|---|---|---|---|---|---|---|---|
| SEQ ID NO: 6121 | CCUUGGA | SEQ ID NO: 6172 | CGAACGU | SEQ ID NO: 6223 | CGACAUG | SEQ ID NO: 6274 | CGAGAAC | SEQ ID NO: 6325 | CGAGUCA | SEQ ID NO: 6376 | CGAUGCU |
| SEQ ID NO: 6122 | CCUUGGC | SEQ ID NO: 6173 | CGAACUA | SEQ ID NO: 6224 | CGACAUU | SEQ ID NO: 6275 | CGAGAAG | SEQ ID NO: 6326 | CGAGUCC | SEQ ID NO: 6377 | CGAUGGA |
| SEQ ID NO: 6123 | CCUUGGG | SEQ ID NO: 6174 | CGAACUC | SEQ ID NO: 6225 | CGACCAA | SEQ ID NO: 6276 | CGAGAAU | SEQ ID NO: 6327 | CGAGUCG | SEQ ID NO: 6378 | CGAUGGC |
| SEQ ID NO: 6124 | CCUUGGU | SEQ ID NO: 6175 | CGAACUG | SEQ ID NO: 6226 | CGACCAC | SEQ ID NO: 6277 | CGAGACA | SEQ ID NO: 6328 | CGAGUCU | SEQ ID NO: 6379 | CGAUGGG |
| SEQ ID NO: 6125 | CCUUGUA | SEQ ID NO: 6176 | CGAACUU | SEQ ID NO: 6227 | CGACCAG | SEQ ID NO: 6278 | CGAGACC | SEQ ID NO: 6329 | CGAGUGA | SEQ ID NO: 6380 | CGAUGGU |
| SEQ ID NO: 6126 | CCUUGUC | SEQ ID NO: 6177 | CGAAGAA | SEQ ID NO: 6228 | CGACCAU | SEQ ID NO: 6279 | CGAGACG | SEQ ID NO: 6330 | CGAGUGC | SEQ ID NO: 6381 | CGAUGUA |
| SEQ ID NO: 6127 | CCUUGUG | SEQ ID NO: 6178 | CGAAGAC | SEQ ID NO: 6229 | CGACCCA | SEQ ID NO: 6280 | CGAGACU | SEQ ID NO: 6331 | CGAGUGG | SEQ ID NO: 6382 | CGAUGUC |
| SEQ ID NO: 6128 | CCUUGUU | SEQ ID NO: 6179 | CGAAGAG | SEQ ID NO: 6230 | CGACCCC | SEQ ID NO: 6281 | CGAGAGA | SEQ ID NO: 6332 | CGAGUGU | SEQ ID NO: 6383 | CGAUGUG |
| SEQ ID NO: 6129 | CCUUUAA | SEQ ID NO: 6180 | CGAAGAU | SEQ ID NO: 6231 | CGACCCG | SEQ ID NO: 6282 | CGAGAGC | SEQ ID NO: 6333 | CGAGUUA | SEQ ID NO: 6384 | CGAUGUU |
| SEQ ID NO: 6130 | CCUUUAC | SEQ ID NO: 6181 | CGAAGCA | SEQ ID NO: 6232 | CGACCCU | SEQ ID NO: 6283 | CGAGAGG | SEQ ID NO: 6334 | CGAGUUC | SEQ ID NO: 6385 | CGAUUAA |
| SEQ ID NO: 6131 | CCUUUAG | SEQ ID NO: 6182 | CGAAGCC | SEQ ID NO: 6233 | CGACCGA | SEQ ID NO: 6284 | CGAGAGU | SEQ ID NO: 6335 | CGAGUUG | SEQ ID NO: 6386 | CGAUUAC |
| SEQ ID NO: 6132 | CCUUUAU | SEQ ID NO: 6183 | CGAAGCG | SEQ ID NO: 6234 | CGACCGC | SEQ ID NO: 6285 | CGAGAUA | SEQ ID NO: 6336 | CGAGUUU | SEQ ID NO: 6387 | CGAUUAG |
| SEQ ID NO: 6133 | CCUUUCA | SEQ ID NO: 6184 | CGAAGCU | SEQ ID NO: 6235 | CGACCGG | SEQ ID NO: 6286 | CGAGAUC | SEQ ID NO: 6337 | CGAUAAA | SEQ ID NO: 6388 | CGAUUAU |
| SEQ ID NO: 6134 | CCUUUCC | SEQ ID NO: 6185 | CGAAGGA | SEQ ID NO: 6236 | CGACCGU | SEQ ID NO: 6287 | CGAGAUG | SEQ ID NO: 6338 | CGAUAAC | SEQ ID NO: 6389 | CGAUUCA |
| SEQ ID NO: 6135 | CCUUUCG | SEQ ID NO: 6186 | CGAAGGC | SEQ ID NO: 6237 | CGACCUA | SEQ ID NO: 6288 | CGAGAUU | SEQ ID NO: 6339 | CGAUAAG | SEQ ID NO: 6390 | CGAUUCC |
| SEQ ID NO: 6136 | CCUUUCU | SEQ ID NO: 6187 | CGAAGGG | SEQ ID NO: 6238 | CGACCUC | SEQ ID NO: 6289 | CGAGCAA | SEQ ID NO: 6340 | CGAUAAU | SEQ ID NO: 6391 | CGAUUCG |
| SEQ ID NO: 6137 | CCUUUGA | SEQ ID NO: 6188 | CGAAGGU | SEQ ID NO: 6239 | CGACCUG | SEQ ID NO: 6290 | CGAGCAC | SEQ ID NO: 6341 | CGAUACA | SEQ ID NO: 6392 | CGAUUCU |
| SEQ ID NO: 6138 | CCUUUGC | SEQ ID NO: 6189 | CGAAGUA | SEQ ID NO: 6240 | CGACCUU | SEQ ID NO: 6291 | CGAGCAG | SEQ ID NO: 6342 | CGAUACC | SEQ ID NO: 6393 | CGAUUGA |
| SEQ ID NO: 6139 | CCUUUGG | SEQ ID NO: 6190 | CGAAGUC | SEQ ID NO: 6241 | CGACGAA | SEQ ID NO: 6292 | CGAGCAU | SEQ ID NO: 6343 | CGAUACG | SEQ ID NO: 6394 | CGAUUGC |
| SEQ ID NO: 6140 | CCUUUGU | SEQ ID NO: 6191 | CGAAGUG | SEQ ID NO: 6242 | CGACGAC | SEQ ID NO: 6293 | CGAGCCA | SEQ ID NO: 6344 | CGAUACU | SEQ ID NO: 6395 | CGAUUGG |
| SEQ ID NO: 6141 | CCUUUUA | SEQ ID NO: 6192 | CGAAGUU | SEQ ID NO: 6243 | CGACGAG | SEQ ID NO: 6294 | CGAGCCC | SEQ ID NO: 6345 | CGAUAGA | SEQ ID NO: 6396 | CGAUUGU |
| SEQ ID NO: 6142 | CCUUUUC | SEQ ID NO: 6193 | CGAAUAA | SEQ ID NO: 6244 | CGACGAU | SEQ ID NO: 6295 | CGAGCCG | SEQ ID NO: 6346 | CGAUAGC | SEQ ID NO: 6397 | CGAUUUA |
| SEQ ID NO: 6143 | CCUUUUG | SEQ ID NO: 6194 | CGAAUAC | SEQ ID NO: 6245 | CGACGCA | SEQ ID NO: 6296 | CGAGCCU | SEQ ID NO: 6347 | CGAUAGG | SEQ ID NO: 6398 | CGAUUUC |
| SEQ ID NO: 6144 | CCUUUUU | SEQ ID NO: 6195 | CGAAUAG | SEQ ID NO: 6246 | CGACGCC | SEQ ID NO: 6297 | CGAGCGA | SEQ ID NO: 6348 | CGAUAGU | SEQ ID NO: 6399 | CGAUUUG |
| SEQ ID NO: 6145 | CGAAAAA | SEQ ID NO: 6196 | CGAAUAU | SEQ ID NO: 6247 | CGACGCG | SEQ ID NO: 6298 | CGAGCGC | SEQ ID NO: 6349 | CGAUAUA | SEQ ID NO: 6400 | CGAUUUU |
| SEQ ID NO: 6146 | CGAAAAC | SEQ ID NO: 6197 | CGAAUCA | SEQ ID NO: 6248 | CGACGCU | SEQ ID NO: 6299 | CGAGCGG | SEQ ID NO: 6350 | CGAUAUC | SEQ ID NO: 6401 | CGCAAAA |
| SEQ ID NO: 6147 | CGAAAAG | SEQ ID NO: 6198 | CGAAUCC | SEQ ID NO: 6249 | CGACGGA | SEQ ID NO: 6300 | CGAGCGU | SEQ ID NO: 6351 | CGAUAUG | SEQ ID NO: 6402 | CGCAAAC |
| SEQ ID NO: 6148 | CGAAAAU | SEQ ID NO: 6199 | CGAAUCG | SEQ ID NO: 6250 | CGACGGC | SEQ ID NO: 6301 | CGAGCUA | SEQ ID NO: 6352 | CGAUAUU | SEQ ID NO: 6403 | CGCAAAG |
| SEQ ID NO: 6149 | CGAAACA | SEQ ID NO: 6200 | CGAAUCU | SEQ ID NO: 6251 | CGACGGG | SEQ ID NO: 6302 | CGAGCUC | SEQ ID NO: 6353 | CGAUCAA | SEQ ID NO: 6404 | CGCAAAU |
| SEQ ID NO: 6150 | CGAAACC | SEQ ID NO: 6201 | CGAAUGA | SEQ ID NO: 6252 | CGACGGU | SEQ ID NO: 6303 | CGAGCUG | SEQ ID NO: 6354 | CGAUCAC | SEQ ID NO: 6405 | CGCAACA |
| SEQ ID NO: 6151 | CGAAACG | SEQ ID NO: 6202 | CGAAUGC | SEQ ID NO: 6253 | CGACGUA | SEQ ID NO: 6304 | CGAGCUU | SEQ ID NO: 6355 | CGAUCAG | SEQ ID NO: 6406 | CGCAACC |
| SEQ ID NO: 6152 | CGAAACU | SEQ ID NO: 6203 | CGAAUGG | SEQ ID NO: 6254 | CGACGUC | SEQ ID NO: 6305 | CGAGGAA | SEQ ID NO: 6356 | CGAUCAU | SEQ ID NO: 6407 | CGCAACG |
| SEQ ID NO: 6153 | CGAAAGA | SEQ ID NO: 6204 | CGAAUGU | SEQ ID NO: 6255 | CGACGUG | SEQ ID NO: 6306 | CGAGGAC | SEQ ID NO: 6357 | CGAUCCA | SEQ ID NO: 6408 | CGCAACU |
| SEQ ID NO: 6154 | CGAAAGC | SEQ ID NO: 6205 | CGAAUUA | SEQ ID NO: 6256 | CGACGUU | SEQ ID NO: 6307 | CGAGGAG | SEQ ID NO: 6358 | CGAUCCC | SEQ ID NO: 6409 | CGCAAGA |
| SEQ ID NO: 6155 | CGAAAGG | SEQ ID NO: 6206 | CGAAUUC | SEQ ID NO: 6257 | CGACUAA | SEQ ID NO: 6308 | CGAGGAU | SEQ ID NO: 6359 | CGAUCCG | SEQ ID NO: 6410 | CGCAAGC |
| SEQ ID NO: 6156 | CGAAAGU | SEQ ID NO: 6207 | CGAAUUG | SEQ ID NO: 6258 | CGACUAC | SEQ ID NO: 6309 | CGAGGCA | SEQ ID NO: 6360 | CGAUCCU | SEQ ID NO: 6411 | CGCAAGG |
| SEQ ID NO: 6157 | CGAAAUA | SEQ ID NO: 6208 | CGAAUUU | SEQ ID NO: 6259 | CGACUAG | SEQ ID NO: 6310 | CGAGGCC | SEQ ID NO: 6361 | CGAUCGA | SEQ ID NO: 6412 | CGCAAGU |
| SEQ ID NO: 6158 | CGAAAUC | SEQ ID NO: 6209 | CGACAAA | SEQ ID NO: 6260 | CGACUAU | SEQ ID NO: 6311 | CGAGGCG | SEQ ID NO: 6362 | CGAUCGC | SEQ ID NO: 6413 | CGCAAUA |
| SEQ ID NO: 6159 | CGAAAUG | SEQ ID NO: 6210 | CGACAAC | SEQ ID NO: 6261 | CGACUCA | SEQ ID NO: 6312 | CGAGGCU | SEQ ID NO: 6363 | CGAUCGG | SEQ ID NO: 6414 | CGCAAUC |
| SEQ ID NO: 6160 | CGAAAUU | SEQ ID NO: 6211 | CGACAAG | SEQ ID NO: 6262 | CGACUCC | SEQ ID NO: 6313 | CGAGGGA | SEQ ID NO: 6364 | CGAUCGU | SEQ ID NO: 6415 | CGCAAUG |
| SEQ ID NO: 6161 | CGAACAA | SEQ ID NO: 6212 | CGACAAU | SEQ ID NO: 6263 | CGACUCG | SEQ ID NO: 6314 | CGAGGGC | SEQ ID NO: 6365 | CGAUCUA | SEQ ID NO: 6416 | CGCAAUU |
| SEQ ID NO: 6162 | CGAACAC | SEQ ID NO: 6213 | CGACACA | SEQ ID NO: 6264 | CGACUCU | SEQ ID NO: 6315 | CGAGGGG | SEQ ID NO: 6366 | CGAUCUC | SEQ ID NO: 6417 | CGCACAA |
| SEQ ID NO: 6163 | CGAACAG | SEQ ID NO: 6214 | CGACACC | SEQ ID NO: 6265 | CGACUGA | SEQ ID NO: 6316 | CGAGGGU | SEQ ID NO: 6367 | CGAUCUG | SEQ ID NO: 6418 | CGCACAC |
| SEQ ID NO: 6164 | CGAACAU | SEQ ID NO: 6215 | CGACACG | SEQ ID NO: 6266 | CGACUGC | SEQ ID NO: 6317 | CGAGGUA | SEQ ID NO: 6368 | CGAUCUU | SEQ ID NO: 6419 | CGCACAG |
| SEQ ID NO: 6165 | CGAACCA | SEQ ID NO: 6216 | CGACACU | SEQ ID NO: 6267 | CGACUGG | SEQ ID NO: 6318 | CGAGGUC | SEQ ID NO: 6369 | CGAUGAA | SEQ ID NO: 6420 | CGCACAU |
| SEQ ID NO: 6166 | CGAACCC | SEQ ID NO: 6217 | CGACAGA | SEQ ID NO: 6268 | CGACUGU | SEQ ID NO: 6319 | CGAGGUG | SEQ ID NO: 6370 | CGAUGAC | SEQ ID NO: 6421 | CGCACCA |
| SEQ ID NO: 6167 | CGAACCG | SEQ ID NO: 6218 | CGACAGC | SEQ ID NO: 6269 | CGACUUA | SEQ ID NO: 6320 | CGAGGUU | SEQ ID NO: 6371 | CGAUGAG | SEQ ID NO: 6422 | CGCACCC |
| SEQ ID NO: 6168 | CGAACCU | SEQ ID NO: 6219 | CGACAGG | SEQ ID NO: 6270 | CGACUUC | SEQ ID NO: 6321 | CGAGUAA | SEQ ID NO: 6372 | CGAUGAU | SEQ ID NO: 6423 | CGCACCG |
| SEQ ID NO: 6169 | CGAACGA | SEQ ID NO: 6220 | CGACAGU | SEQ ID NO: 6271 | CGACUUG | SEQ ID NO: 6322 | CGAGUAC | SEQ ID NO: 6373 | CGAUGCA | SEQ ID NO: 6424 | CGCACCU |
| SEQ ID NO: 6170 | CGAACGC | SEQ ID NO: 6221 | CGACAUA | SEQ ID NO: 6272 | CGACUUU | SEQ ID NO: 6323 | CGAGUAG | SEQ ID NO: 6374 | CGAUGCC | SEQ ID NO: 6425 | CGCACGA |
| SEQ ID NO: 6171 | CGAACGG | SEQ ID NO: 6222 | CGACAUC | SEQ ID NO: 6273 | CGAGAAA | SEQ ID NO: 6324 | CGAGUAU | SEQ ID NO: 6375 | CGAUGCG | SEQ ID NO: 6426 | CGCACGC |

Table 22

| | | | | | | | | | | | |
|---|---|---|---|---|---|---|---|---|---|---|---|
| SEQ ID NO: 6427 | CGCACGG | SEQ ID NO: 6478 | CGCCAUC | SEQ ID NO: 6529 | CGCGAAA | SEQ ID NO: 6580 | CGCGUAU | SEQ ID NO: 6631 | CGCUGCG | SEQ ID NO: 6682 | CGGACGC |
| SEQ ID NO: 6428 | CGCACGU | SEQ ID NO: 6479 | CGCCAUG | SEQ ID NO: 6530 | CGCGAAC | SEQ ID NO: 6581 | CGCGUCA | SEQ ID NO: 6632 | CGCUGCU | SEQ ID NO: 6683 | CGGACGG |
| SEQ ID NO: 6429 | CGCACUA | SEQ ID NO: 6480 | CGCCAUU | SEQ ID NO: 6531 | CGCGAAG | SEQ ID NO: 6582 | CGCGUCC | SEQ ID NO: 6633 | CGCUGGA | SEQ ID NO: 6684 | CGGACGU |
| SEQ ID NO: 6430 | CGCACUC | SEQ ID NO: 6481 | CGCCCAA | SEQ ID NO: 6532 | CGCGAAU | SEQ ID NO: 6583 | CGCGUCG | SEQ ID NO: 6634 | CGCUGGC | SEQ ID NO: 6685 | CGGACUA |
| SEQ ID NO: 6431 | CGCACUG | SEQ ID NO: 6482 | CGCCCAC | SEQ ID NO: 6533 | CGCGACA | SEQ ID NO: 6584 | CGCGUCU | SEQ ID NO: 6635 | CGCUGGG | SEQ ID NO: 6686 | CGGACUC |
| SEQ ID NO: 6432 | CGCACUU | SEQ ID NO: 6483 | CGCCCAG | SEQ ID NO: 6534 | CGCGACC | SEQ ID NO: 6585 | CGCGUGA | SEQ ID NO: 6636 | CGCUGGU | SEQ ID NO: 6687 | CGGACUG |
| SEQ ID NO: 6433 | CGCAGAA | SEQ ID NO: 6484 | CGCCCAU | SEQ ID NO: 6535 | CGCGACG | SEQ ID NO: 6586 | CGCGUGC | SEQ ID NO: 6637 | CGCUGUA | SEQ ID NO: 6688 | CGGACUU |
| SEQ ID NO: 6434 | CGCAGAC | SEQ ID NO: 6485 | CGCCCCA | SEQ ID NO: 6536 | CGCGACU | SEQ ID NO: 6587 | CGCGUGG | SEQ ID NO: 6638 | CGCUGUC | SEQ ID NO: 6689 | CGGAGAA |
| SEQ ID NO: 6435 | CGCAGAG | SEQ ID NO: 6486 | CGCCCCC | SEQ ID NO: 6537 | CGCGAGA | SEQ ID NO: 6588 | CGCGUGU | SEQ ID NO: 6639 | CGCUGUG | SEQ ID NO: 6690 | CGGAGAC |
| SEQ ID NO: 6436 | CGCAGAU | SEQ ID NO: 6487 | CGCCCCG | SEQ ID NO: 6538 | CGCGAGC | SEQ ID NO: 6589 | CGCGUUA | SEQ ID NO: 6640 | CGCUGUU | SEQ ID NO: 6691 | CGGAGAG |
| SEQ ID NO: 6437 | CGCAGCA | SEQ ID NO: 6488 | CGCCCCU | SEQ ID NO: 6539 | CGCGAGG | SEQ ID NO: 6590 | CGCGUUC | SEQ ID NO: 6641 | CGCUUAA | SEQ ID NO: 6692 | CGGAGAU |
| SEQ ID NO: 6438 | CGCAGCC | SEQ ID NO: 6489 | CGCCCGA | SEQ ID NO: 6540 | CGCGAGU | SEQ ID NO: 6591 | CGCGUUG | SEQ ID NO: 6642 | CGCUUAC | SEQ ID NO: 6693 | CGGAGCA |
| SEQ ID NO: 6439 | CGCAGCG | SEQ ID NO: 6490 | CGCCCGC | SEQ ID NO: 6541 | CGCGAUA | SEQ ID NO: 6592 | CGCGUUU | SEQ ID NO: 6643 | CGCUUAG | SEQ ID NO: 6694 | CGGAGCC |
| SEQ ID NO: 6440 | CGCAGCU | SEQ ID NO: 6491 | CGCCCGG | SEQ ID NO: 6542 | CGCGAUC | SEQ ID NO: 6593 | CGCUAAA | SEQ ID NO: 6644 | CGCUUAU | SEQ ID NO: 6695 | CGGAGCG |
| SEQ ID NO: 6441 | CGCAGGA | SEQ ID NO: 6492 | CGCCCGU | SEQ ID NO: 6543 | CGCGAUG | SEQ ID NO: 6594 | CGCUAAC | SEQ ID NO: 6645 | CGCUUCA | SEQ ID NO: 6696 | CGGAGCU |
| SEQ ID NO: 6442 | CGCAGGC | SEQ ID NO: 6493 | CGCCCUA | SEQ ID NO: 6544 | CGCGAUU | SEQ ID NO: 6595 | CGCUAAG | SEQ ID NO: 6646 | CGCUUCC | SEQ ID NO: 6697 | CGGAGGA |
| SEQ ID NO: 6443 | CGCAGGG | SEQ ID NO: 6494 | CGCCCUC | SEQ ID NO: 6545 | CGCGCAA | SEQ ID NO: 6596 | CGCUAAU | SEQ ID NO: 6647 | CGCUUCG | SEQ ID NO: 6698 | CGGAGGC |
| SEQ ID NO: 6444 | CGCAGGU | SEQ ID NO: 6495 | CGCCCUG | SEQ ID NO: 6546 | CGCGCAC | SEQ ID NO: 6597 | CGCUACA | SEQ ID NO: 6648 | CGCUUCU | SEQ ID NO: 6699 | CGGAGGG |
| SEQ ID NO: 6445 | CGCAGUA | SEQ ID NO: 6496 | CGCCCUU | SEQ ID NO: 6547 | CGCGCAG | SEQ ID NO: 6598 | CGCUACC | SEQ ID NO: 6649 | CGCUUGA | SEQ ID NO: 6700 | CGGAGGU |
| SEQ ID NO: 6446 | CGCAGUC | SEQ ID NO: 6497 | CGCCGAA | SEQ ID NO: 6548 | CGCGCAU | SEQ ID NO: 6599 | CGCUACG | SEQ ID NO: 6650 | CGCUUGC | SEQ ID NO: 6701 | CGGAGUA |
| SEQ ID NO: 6447 | CGCAGUG | SEQ ID NO: 6498 | CGCCGAC | SEQ ID NO: 6549 | CGCGCCA | SEQ ID NO: 6600 | CGCUACU | SEQ ID NO: 6651 | CGCUUGG | SEQ ID NO: 6702 | CGGAGUC |
| SEQ ID NO: 6448 | CGCAGUU | SEQ ID NO: 6499 | CGCCGAG | SEQ ID NO: 6550 | CGCGCCC | SEQ ID NO: 6601 | CGCUAGA | SEQ ID NO: 6652 | CGCUUGU | SEQ ID NO: 6703 | CGGAGUG |
| SEQ ID NO: 6449 | CGCAUAA | SEQ ID NO: 6500 | CGCCGAU | SEQ ID NO: 6551 | CGCGCCG | SEQ ID NO: 6602 | CGCUAGC | SEQ ID NO: 6653 | CGCUUUA | SEQ ID NO: 6704 | CGGAGUU |
| SEQ ID NO: 6450 | CGCAUAC | SEQ ID NO: 6501 | CGCCGCA | SEQ ID NO: 6552 | CGCGCCU | SEQ ID NO: 6603 | CGCUAGG | SEQ ID NO: 6654 | CGCUUUC | SEQ ID NO: 6705 | CGGAUAA |
| SEQ ID NO: 6451 | CGCAUAG | SEQ ID NO: 6502 | CGCCGCC | SEQ ID NO: 6553 | CGCGCGA | SEQ ID NO: 6604 | CGCUAGU | SEQ ID NO: 6655 | CGCUUUG | SEQ ID NO: 6706 | CGGAUAC |
| SEQ ID NO: 6452 | CGCAUAU | SEQ ID NO: 6503 | CGCCGCG | SEQ ID NO: 6554 | CGCGCGC | SEQ ID NO: 6605 | CGCUAUA | SEQ ID NO: 6656 | CGCUUUU | SEQ ID NO: 6707 | CGGAUAG |
| SEQ ID NO: 6453 | CGCAUCA | SEQ ID NO: 6504 | CGCCGCU | SEQ ID NO: 6555 | CGCGCGG | SEQ ID NO: 6606 | CGCUAUC | SEQ ID NO: 6657 | CGGAAAA | SEQ ID NO: 6708 | CGGAUAU |
| SEQ ID NO: 6454 | CGCAUCC | SEQ ID NO: 6505 | CGCCGGA | SEQ ID NO: 6556 | CGCGCGU | SEQ ID NO: 6607 | CGCUAUG | SEQ ID NO: 6658 | CGGAAAC | SEQ ID NO: 6709 | CGGAUCA |
| SEQ ID NO: 6455 | CGCAUCG | SEQ ID NO: 6506 | CGCCGGC | SEQ ID NO: 6557 | CGCGCUA | SEQ ID NO: 6608 | CGCUAUU | SEQ ID NO: 6659 | CGGAAAG | SEQ ID NO: 6710 | CGGAUCC |
| SEQ ID NO: 6456 | CGCAUCU | SEQ ID NO: 6507 | CGCCGGG | SEQ ID NO: 6558 | CGCGCUC | SEQ ID NO: 6609 | CGCUCAA | SEQ ID NO: 6660 | CGGAAAU | SEQ ID NO: 6711 | CGGAUCG |
| SEQ ID NO: 6457 | CGCAUGA | SEQ ID NO: 6508 | CGCCGGU | SEQ ID NO: 6559 | CGCGCUG | SEQ ID NO: 6610 | CGCUCAC | SEQ ID NO: 6661 | CGGAACA | SEQ ID NO: 6712 | CGGAUCU |
| SEQ ID NO: 6458 | CGCAUGC | SEQ ID NO: 6509 | CGCCGUA | SEQ ID NO: 6560 | CGCGCUU | SEQ ID NO: 6611 | CGCUCAG | SEQ ID NO: 6662 | CGGAACC | SEQ ID NO: 6713 | CGGAUGA |
| SEQ ID NO: 6459 | CGCAUGG | SEQ ID NO: 6510 | CGCCGUC | SEQ ID NO: 6561 | CGCGGAA | SEQ ID NO: 6612 | CGCUCAU | SEQ ID NO: 6663 | CGGAACG | SEQ ID NO: 6714 | CGGAUGC |
| SEQ ID NO: 6460 | CGCAUGU | SEQ ID NO: 6511 | CGCCGUG | SEQ ID NO: 6562 | CGCGGAC | SEQ ID NO: 6613 | CGCUCCA | SEQ ID NO: 6664 | CGGAACU | SEQ ID NO: 6715 | CGGAUGG |
| SEQ ID NO: 6461 | CGCAUUA | SEQ ID NO: 6512 | CGCCGUU | SEQ ID NO: 6563 | CGCGGAG | SEQ ID NO: 6614 | CGCUCCC | SEQ ID NO: 6665 | CGGAAGA | SEQ ID NO: 6716 | CGGAUGU |
| SEQ ID NO: 6462 | CGCAUUC | SEQ ID NO: 6513 | CGCCUAA | SEQ ID NO: 6564 | CGCGGAU | SEQ ID NO: 6615 | CGCUCCG | SEQ ID NO: 6666 | CGGAAGC | SEQ ID NO: 6717 | CGGAUUA |
| SEQ ID NO: 6463 | CGCAUUG | SEQ ID NO: 6514 | CGCCUAC | SEQ ID NO: 6565 | CGCGGCA | SEQ ID NO: 6616 | CGCUCCU | SEQ ID NO: 6667 | CGGAAGG | SEQ ID NO: 6718 | CGGAUUC |
| SEQ ID NO: 6464 | CGCAUUU | SEQ ID NO: 6515 | CGCCUAG | SEQ ID NO: 6566 | CGCGGCC | SEQ ID NO: 6617 | CGCUCGA | SEQ ID NO: 6668 | CGGAAGU | SEQ ID NO: 6719 | CGGAUUG |
| SEQ ID NO: 6465 | CGCCAAA | SEQ ID NO: 6516 | CGCCUAU | SEQ ID NO: 6567 | CGCGGCG | SEQ ID NO: 6618 | CGCUCGC | SEQ ID NO: 6669 | CGGAAUA | SEQ ID NO: 6720 | CGGAUUU |
| SEQ ID NO: 6466 | CGCCAAC | SEQ ID NO: 6517 | CGCCUCA | SEQ ID NO: 6568 | CGCGGCU | SEQ ID NO: 6619 | CGCUCGG | SEQ ID NO: 6670 | CGGAAUC | SEQ ID NO: 6721 | CGGCAAA |
| SEQ ID NO: 6467 | CGCCAAG | SEQ ID NO: 6518 | CGCCUCC | SEQ ID NO: 6569 | CGCGGGA | SEQ ID NO: 6620 | CGCUCGU | SEQ ID NO: 6671 | CGGAAUG | SEQ ID NO: 6722 | CGGCAAC |
| SEQ ID NO: 6468 | CGCCAAU | SEQ ID NO: 6519 | CGCCUCG | SEQ ID NO: 6570 | CGCGGGC | SEQ ID NO: 6621 | CGCUCUA | SEQ ID NO: 6672 | CGGAAUU | SEQ ID NO: 6723 | CGGCAAG |
| SEQ ID NO: 6469 | CGCCACA | SEQ ID NO: 6520 | CGCCUCU | SEQ ID NO: 6571 | CGCGGGG | SEQ ID NO: 6622 | CGCUCUC | SEQ ID NO: 6673 | CGGACAA | SEQ ID NO: 6724 | CGGCAAU |
| SEQ ID NO: 6470 | CGCCACC | SEQ ID NO: 6521 | CGCCUGA | SEQ ID NO: 6572 | CGCGGGU | SEQ ID NO: 6623 | CGCUCUG | SEQ ID NO: 6674 | CGGACAC | SEQ ID NO: 6725 | CGGCACA |
| SEQ ID NO: 6471 | CGCCACG | SEQ ID NO: 6522 | CGCCUGC | SEQ ID NO: 6573 | CGCGGUA | SEQ ID NO: 6624 | CGCUCUU | SEQ ID NO: 6675 | CGGACAG | SEQ ID NO: 6726 | CGGCACC |
| SEQ ID NO: 6472 | CGCCACU | SEQ ID NO: 6523 | CGCCUGG | SEQ ID NO: 6574 | CGCGGUC | SEQ ID NO: 6625 | CGCUGAA | SEQ ID NO: 6676 | CGGACAU | SEQ ID NO: 6727 | CGGCACG |
| SEQ ID NO: 6473 | CGCCAGA | SEQ ID NO: 6524 | CGCCUGU | SEQ ID NO: 6575 | CGCGGUG | SEQ ID NO: 6626 | CGCUGAC | SEQ ID NO: 6677 | CGGACCA | SEQ ID NO: 6728 | CGGCACU |
| SEQ ID NO: 6474 | CGCCAGC | SEQ ID NO: 6525 | CGCCUUA | SEQ ID NO: 6576 | CGCGGUU | SEQ ID NO: 6627 | CGCUGAG | SEQ ID NO: 6678 | CGGACCC | SEQ ID NO: 6729 | CGGCAGA |
| SEQ ID NO: 6475 | CGCCAGG | SEQ ID NO: 6526 | CGCCUUC | SEQ ID NO: 6577 | CGCGUAA | SEQ ID NO: 6628 | CGCUGAU | SEQ ID NO: 6679 | CGGACCG | SEQ ID NO: 6730 | CGGCAGC |
| SEQ ID NO: 6476 | CGCCAGU | SEQ ID NO: 6527 | CGCCUUG | SEQ ID NO: 6578 | CGCGUAC | SEQ ID NO: 6629 | CGCUGCA | SEQ ID NO: 6680 | CGGACCU | SEQ ID NO: 6731 | CGGCAGG |
| SEQ ID NO: 6477 | CGCCAUA | SEQ ID NO: 6528 | CGCCUUU | SEQ ID NO: 6579 | CGCGUAG | SEQ ID NO: 6630 | CGCUGCC | SEQ ID NO: 6681 | CGGACGA | SEQ ID NO: 6732 | CGGCAGU |

Table 23

| | |
|---|---|
| SEQ ID NO: 6733 | CGGCAUA |
| SEQ ID NO: 6734 | CGGCAUC |
| SEQ ID NO: 6735 | CGGCAUG |
| SEQ ID NO: 6736 | CGGCAUU |
| SEQ ID NO: 6737 | CGGCCAA |
| SEQ ID NO: 6738 | CGGCCAC |
| SEQ ID NO: 6739 | CGGCCAG |
| SEQ ID NO: 6740 | CGGCCAU |
| SEQ ID NO: 6741 | CGGCCCA |
| SEQ ID NO: 6742 | CGGCCCC |
| SEQ ID NO: 6743 | CGGCCCG |
| SEQ ID NO: 6744 | CGGCCCU |
| SEQ ID NO: 6745 | CGGCCGA |
| SEQ ID NO: 6746 | CGGCCGC |
| SEQ ID NO: 6747 | CGGCCGG |
| SEQ ID NO: 6748 | CGGCCGU |
| SEQ ID NO: 6749 | CGGCCUA |
| SEQ ID NO: 6750 | CGGCCUC |
| SEQ ID NO: 6751 | CGGCCUG |
| SEQ ID NO: 6752 | CGGCCUU |
| SEQ ID NO: 6753 | CGGCGAA |
| SEQ ID NO: 6754 | CGGCGAC |
| SEQ ID NO: 6755 | CGGCGAG |
| SEQ ID NO: 6756 | CGGCGAU |
| SEQ ID NO: 6757 | CGGCGCA |
| SEQ ID NO: 6758 | CGGCGCC |
| SEQ ID NO: 6759 | CGGCGCG |
| SEQ ID NO: 6760 | CGGCGCU |
| SEQ ID NO: 6761 | CGGCGGA |
| SEQ ID NO: 6762 | CGGCGGC |
| SEQ ID NO: 6763 | CGGCGGG |
| SEQ ID NO: 6764 | CGGCGGU |
| SEQ ID NO: 6765 | CGGCGUA |
| SEQ ID NO: 6766 | CGGCGUC |
| SEQ ID NO: 6767 | CGGCGUG |
| SEQ ID NO: 6768 | CGGCGUU |
| SEQ ID NO: 6769 | CGGCUAA |
| SEQ ID NO: 6770 | CGGCUAC |
| SEQ ID NO: 6771 | CGGCUAG |
| SEQ ID NO: 6772 | CGGCUAU |
| SEQ ID NO: 6773 | CGGCUCA |
| SEQ ID NO: 6774 | CGGCUCC |
| SEQ ID NO: 6775 | CGGCUCG |
| SEQ ID NO: 6776 | CGGCUCU |
| SEQ ID NO: 6777 | CGGCUGA |
| SEQ ID NO: 6778 | CGGCUGC |
| SEQ ID NO: 6779 | CGGCUGG |
| SEQ ID NO: 6780 | CGGCUGU |
| SEQ ID NO: 6781 | CGGCUUA |
| SEQ ID NO: 6782 | CGGCUUC |
| SEQ ID NO: 6783 | CGGCUUG |
| SEQ ID NO: 6784 | CGGCUUU |
| SEQ ID NO: 6785 | CGGGAAA |
| SEQ ID NO: 6786 | CGGGAAC |
| SEQ ID NO: 6787 | CGGGAAG |
| SEQ ID NO: 6788 | CGGGAAU |
| SEQ ID NO: 6789 | CGGGACA |
| SEQ ID NO: 6790 | CGGGACC |
| SEQ ID NO: 6791 | CGGGACG |
| SEQ ID NO: 6792 | CGGGACU |
| SEQ ID NO: 6793 | CGGGAGA |
| SEQ ID NO: 6794 | CGGGAGC |
| SEQ ID NO: 6795 | CGGGAGG |
| SEQ ID NO: 6796 | CGGGAGU |
| SEQ ID NO: 6797 | CGGGAUA |
| SEQ ID NO: 6798 | CGGGAUC |
| SEQ ID NO: 6799 | CGGGAUG |
| SEQ ID NO: 6800 | CGGGAUU |
| SEQ ID NO: 6801 | CGGGCAA |
| SEQ ID NO: 6802 | CGGGCAC |
| SEQ ID NO: 6803 | CGGGCAG |
| SEQ ID NO: 6804 | CGGGCAU |
| SEQ ID NO: 6805 | CGGGCCA |
| SEQ ID NO: 6806 | CGGGCCC |
| SEQ ID NO: 6807 | CGGGCCG |
| SEQ ID NO: 6808 | CGGGCCU |
| SEQ ID NO: 6809 | CGGGCGA |
| SEQ ID NO: 6810 | CGGGCGC |
| SEQ ID NO: 6811 | CGGGCGG |
| SEQ ID NO: 6812 | CGGGCGU |
| SEQ ID NO: 6813 | CGGGCUA |
| SEQ ID NO: 6814 | CGGGCUC |
| SEQ ID NO: 6815 | CGGGCUG |
| SEQ ID NO: 6816 | CGGGCUU |
| SEQ ID NO: 6817 | CGGGGAA |
| SEQ ID NO: 6818 | CGGGGAC |
| SEQ ID NO: 6819 | CGGGGAG |
| SEQ ID NO: 6820 | CGGGGAU |
| SEQ ID NO: 6821 | CGGGGCA |
| SEQ ID NO: 6822 | CGGGGCC |
| SEQ ID NO: 6823 | CGGGGCG |
| SEQ ID NO: 6824 | CGGGGCU |
| SEQ ID NO: 6825 | CGGGGGA |
| SEQ ID NO: 6826 | CGGGGGC |
| SEQ ID NO: 6827 | CGGGGGG |
| SEQ ID NO: 6828 | CGGGGGU |
| SEQ ID NO: 6829 | CGGGGUA |
| SEQ ID NO: 6830 | CGGGGUC |
| SEQ ID NO: 6831 | CGGGGUG |
| SEQ ID NO: 6832 | CGGGGUU |
| SEQ ID NO: 6833 | CGGGUAA |
| SEQ ID NO: 6834 | CGGGUAC |
| SEQ ID NO: 6835 | CGGGUAG |
| SEQ ID NO: 6836 | CGGGUAU |
| SEQ ID NO: 6837 | CGGGUCA |
| SEQ ID NO: 6838 | CGGGUCC |
| SEQ ID NO: 6839 | CGGGUCG |
| SEQ ID NO: 6840 | CGGGUCU |
| SEQ ID NO: 6841 | CGGGUGA |
| SEQ ID NO: 6842 | CGGGUGC |
| SEQ ID NO: 6843 | CGGGUGG |
| SEQ ID NO: 6844 | CGGGUGU |
| SEQ ID NO: 6845 | CGGGUUA |
| SEQ ID NO: 6846 | CGGGUUC |
| SEQ ID NO: 6847 | CGGGUUG |
| SEQ ID NO: 6848 | CGGGUUU |
| SEQ ID NO: 6849 | CGGUAAA |
| SEQ ID NO: 6850 | CGGUAAC |
| SEQ ID NO: 6851 | CGGUAAG |
| SEQ ID NO: 6852 | CGGUAAU |
| SEQ ID NO: 6853 | CGGUACA |
| SEQ ID NO: 6854 | CGGUACC |
| SEQ ID NO: 6855 | CGGUACG |
| SEQ ID NO: 6856 | CGGUACU |
| SEQ ID NO: 6857 | CGGUAGA |
| SEQ ID NO: 6858 | CGGUAGC |
| SEQ ID NO: 6859 | CGGUAGG |
| SEQ ID NO: 6860 | CGGUAGU |
| SEQ ID NO: 6861 | CGGUAUA |
| SEQ ID NO: 6862 | CGGUAUC |
| SEQ ID NO: 6863 | CGGUAUG |
| SEQ ID NO: 6864 | CGGUAUU |
| SEQ ID NO: 6865 | CGGUCAA |
| SEQ ID NO: 6866 | CGGUCAC |
| SEQ ID NO: 6867 | CGGUCAG |
| SEQ ID NO: 6868 | CGGUCAU |
| SEQ ID NO: 6869 | CGGUCCA |
| SEQ ID NO: 6870 | CGGUCCC |
| SEQ ID NO: 6871 | CGGUCCG |
| SEQ ID NO: 6872 | CGGUCCU |
| SEQ ID NO: 6873 | CGGUCGA |
| SEQ ID NO: 6874 | CGGUCGC |
| SEQ ID NO: 6875 | CGGUCGG |
| SEQ ID NO: 6876 | CGGUCGU |
| SEQ ID NO: 6877 | CGGUCUA |
| SEQ ID NO: 6878 | CGGUCUC |
| SEQ ID NO: 6879 | CGGUCUG |
| SEQ ID NO: 6880 | CGGUCUU |
| SEQ ID NO: 6881 | CGGUGAA |
| SEQ ID NO: 6882 | CGGUGAC |
| SEQ ID NO: 6883 | CGGUGAG |
| SEQ ID NO: 6884 | CGGUGAU |
| SEQ ID NO: 6885 | CGGUGCA |
| SEQ ID NO: 6886 | CGGUGCC |
| SEQ ID NO: 6887 | CGGUGCG |
| SEQ ID NO: 6888 | CGGUGCU |
| SEQ ID NO: 6889 | CGGUGGA |
| SEQ ID NO: 6890 | CGGUGGC |
| SEQ ID NO: 6891 | CGGUGGG |
| SEQ ID NO: 6892 | CGGUGGU |
| SEQ ID NO: 6893 | CGGUGUA |
| SEQ ID NO: 6894 | CGGUGUC |
| SEQ ID NO: 6895 | CGGUGUG |
| SEQ ID NO: 6896 | CGGUGUU |
| SEQ ID NO: 6897 | CGGUUAA |
| SEQ ID NO: 6898 | CGGUUAC |
| SEQ ID NO: 6899 | CGGUUAG |
| SEQ ID NO: 6900 | CGGUUAU |
| SEQ ID NO: 6901 | CGGUUCA |
| SEQ ID NO: 6902 | CGGUUCC |
| SEQ ID NO: 6903 | CGGUUCG |
| SEQ ID NO: 6904 | CGGUUCU |
| SEQ ID NO: 6905 | CGGUUGA |
| SEQ ID NO: 6906 | CGGUUGC |
| SEQ ID NO: 6907 | CGGUUGG |
| SEQ ID NO: 6908 | CGGUUGU |
| SEQ ID NO: 6909 | CGGUUUA |
| SEQ ID NO: 6910 | CGGUUUC |
| SEQ ID NO: 6911 | CGGUUUG |
| SEQ ID NO: 6912 | CGGUUUU |
| SEQ ID NO: 6913 | CGUAAAA |
| SEQ ID NO: 6914 | CGUAAAC |
| SEQ ID NO: 6915 | CGUAAAG |
| SEQ ID NO: 6916 | CGUAAAU |
| SEQ ID NO: 6917 | CGUAACA |
| SEQ ID NO: 6918 | CGUAACC |
| SEQ ID NO: 6919 | CGUAACG |
| SEQ ID NO: 6920 | CGUAACU |
| SEQ ID NO: 6921 | CGUAAGA |
| SEQ ID NO: 6922 | CGUAAGC |
| SEQ ID NO: 6923 | CGUAAGG |
| SEQ ID NO: 6924 | CGUAAGU |
| SEQ ID NO: 6925 | CGUAAUA |
| SEQ ID NO: 6926 | CGUAAUC |
| SEQ ID NO: 6927 | CGUAAUG |
| SEQ ID NO: 6928 | CGUAAUU |
| SEQ ID NO: 6929 | CGUACAA |
| SEQ ID NO: 6930 | CGUACAC |
| SEQ ID NO: 6931 | CGUACAG |
| SEQ ID NO: 6932 | CGUACAU |
| SEQ ID NO: 6933 | CGUACCA |
| SEQ ID NO: 6934 | CGUACCC |
| SEQ ID NO: 6935 | CGUACCG |
| SEQ ID NO: 6936 | CGUACCU |
| SEQ ID NO: 6937 | CGUACGA |
| SEQ ID NO: 6938 | CGUACGC |
| SEQ ID NO: 6939 | CGUACGG |
| SEQ ID NO: 6940 | CGUACGU |
| SEQ ID NO: 6941 | CGUACUA |
| SEQ ID NO: 6942 | CGUACUC |
| SEQ ID NO: 6943 | CGUACUG |
| SEQ ID NO: 6944 | CGUACUU |
| SEQ ID NO: 6945 | CGUAGAA |
| SEQ ID NO: 6946 | CGUAGAC |
| SEQ ID NO: 6947 | CGUAGAG |
| SEQ ID NO: 6948 | CGUAGAU |
| SEQ ID NO: 6949 | CGUAGCA |
| SEQ ID NO: 6950 | CGUAGCC |
| SEQ ID NO: 6951 | CGUAGCG |
| SEQ ID NO: 6952 | CGUAGCU |
| SEQ ID NO: 6953 | CGUAGGA |
| SEQ ID NO: 6954 | CGUAGGC |
| SEQ ID NO: 6955 | CGUAGGG |
| SEQ ID NO: 6956 | CGUAGGU |
| SEQ ID NO: 6957 | CGUAGUA |
| SEQ ID NO: 6958 | CGUAGUC |
| SEQ ID NO: 6959 | CGUAGUG |
| SEQ ID NO: 6960 | CGUAGUU |
| SEQ ID NO: 6961 | CGUAUAA |
| SEQ ID NO: 6962 | CGUAUAC |
| SEQ ID NO: 6963 | CGUAUAG |
| SEQ ID NO: 6964 | CGUAUAU |
| SEQ ID NO: 6965 | CGUAUCA |
| SEQ ID NO: 6966 | CGUAUCC |
| SEQ ID NO: 6967 | CGUAUCG |
| SEQ ID NO: 6968 | CGUAUCU |
| SEQ ID NO: 6969 | CGUAUGA |
| SEQ ID NO: 6970 | CGUAUGC |
| SEQ ID NO: 6971 | CGUAUGG |
| SEQ ID NO: 6972 | CGUAUGU |
| SEQ ID NO: 6973 | CGUAUUA |
| SEQ ID NO: 6974 | CGUAUUC |
| SEQ ID NO: 6975 | CGUAUUG |
| SEQ ID NO: 6976 | CGUAUUU |
| SEQ ID NO: 6977 | CGUCAAA |
| SEQ ID NO: 6978 | CGUCAAC |
| SEQ ID NO: 6979 | CGUCAAG |
| SEQ ID NO: 6980 | CGUCAAU |
| SEQ ID NO: 6981 | CGUCACA |
| SEQ ID NO: 6982 | CGUCACC |
| SEQ ID NO: 6983 | CGUCACG |
| SEQ ID NO: 6984 | CGUCACU |
| SEQ ID NO: 6985 | CGUCAGA |
| SEQ ID NO: 6986 | CGUCAGC |
| SEQ ID NO: 6987 | CGUCAGG |
| SEQ ID NO: 6988 | CGUCAGU |
| SEQ ID NO: 6989 | CGUCAUA |
| SEQ ID NO: 6990 | CGUCAUC |
| SEQ ID NO: 6991 | CGUCAUG |
| SEQ ID NO: 6992 | CGUCAUU |
| SEQ ID NO: 6993 | CGUCCAA |
| SEQ ID NO: 6994 | CGUCCAC |
| SEQ ID NO: 6995 | CGUCCAG |
| SEQ ID NO: 6996 | CGUCCAU |
| SEQ ID NO: 6997 | CGUCCCA |
| SEQ ID NO: 6998 | CGUCCCC |
| SEQ ID NO: 6999 | CGUCCCG |
| SEQ ID NO: 7000 | CGUCCCU |
| SEQ ID NO: 7001 | CGUCCGA |
| SEQ ID NO: 7002 | CGUCCGC |
| SEQ ID NO: 7003 | CGUCCGG |
| SEQ ID NO: 7004 | CGUCCGU |
| SEQ ID NO: 7005 | CGUCCUA |
| SEQ ID NO: 7006 | CGUCCUC |
| SEQ ID NO: 7007 | CGUCCUG |
| SEQ ID NO: 7008 | CGUCCUU |
| SEQ ID NO: 7009 | CGUCGAA |
| SEQ ID NO: 7010 | CGUCGAC |
| SEQ ID NO: 7011 | CGUCGAG |
| SEQ ID NO: 7012 | CGUCGAU |
| SEQ ID NO: 7013 | CGUCGCA |
| SEQ ID NO: 7014 | CGUCGCC |
| SEQ ID NO: 7015 | CGUCGCG |
| SEQ ID NO: 7016 | CGUCGCU |
| SEQ ID NO: 7017 | CGUCGGA |
| SEQ ID NO: 7018 | CGUCGGC |
| SEQ ID NO: 7019 | CGUCGGG |
| SEQ ID NO: 7020 | CGUCGGU |
| SEQ ID NO: 7021 | CGUCGUA |
| SEQ ID NO: 7022 | CGUCGUC |
| SEQ ID NO: 7023 | CGUCGUG |
| SEQ ID NO: 7024 | CGUCGUU |
| SEQ ID NO: 7025 | CGUCUAA |
| SEQ ID NO: 7026 | CGUCUAC |
| SEQ ID NO: 7027 | CGUCUAG |
| SEQ ID NO: 7028 | CGUCUAU |
| SEQ ID NO: 7029 | CGUCUCA |
| SEQ ID NO: 7030 | CGUCUCC |
| SEQ ID NO: 7031 | CGUCUCG |
| SEQ ID NO: 7032 | CGUCUCU |
| SEQ ID NO: 7033 | CGUCUGA |
| SEQ ID NO: 7034 | CGUCUGC |
| SEQ ID NO: 7035 | CGUCUGG |
| SEQ ID NO: 7036 | CGUCUGU |
| SEQ ID NO: 7037 | CGUCUUA |
| SEQ ID NO: 7038 | CGUCUUC |

Table 24

| | | | | | | | | | | | |
|---|---|---|---|---|---|---|---|---|---|---|---|
| SEQ ID NO: 7039 | CGUCUUG | SEQ ID NO: 7090 | CGUGUAC | SEQ ID NO: 7141 | CGUUGCA | SEQ ID NO: 7192 | CUAACCU | SEQ ID NO: 7243 | CUACAGG | SEQ ID NO: 7294 | CUACUUC |
| SEQ ID NO: 7040 | CGUCUUU | SEQ ID NO: 7091 | CGUGUAG | SEQ ID NO: 7142 | CGUUGCC | SEQ ID NO: 7193 | CUAACGA | SEQ ID NO: 7244 | CUACAGU | SEQ ID NO: 7295 | CUACUUG |
| SEQ ID NO: 7041 | CGUGAAA | SEQ ID NO: 7092 | CGUGUAU | SEQ ID NO: 7143 | CGUUGCG | SEQ ID NO: 7194 | CUAACGC | SEQ ID NO: 7245 | CUACAUA | SEQ ID NO: 7296 | CUACUUU |
| SEQ ID NO: 7042 | CGUGAAC | SEQ ID NO: 7093 | CGUGUCA | SEQ ID NO: 7144 | CGUUGCU | SEQ ID NO: 7195 | CUAACGG | SEQ ID NO: 7246 | CUACAUC | SEQ ID NO: 7297 | CUAGAAA |
| SEQ ID NO: 7043 | CGUGAAG | SEQ ID NO: 7094 | CGUGUCC | SEQ ID NO: 7145 | CGUUGGA | SEQ ID NO: 7196 | CUAACGU | SEQ ID NO: 7247 | CUACAUG | SEQ ID NO: 7298 | CUAGAAC |
| SEQ ID NO: 7044 | CGUGAAU | SEQ ID NO: 7095 | CGUGUCG | SEQ ID NO: 7146 | CGUUGGC | SEQ ID NO: 7197 | CUAACUA | SEQ ID NO: 7248 | CUACAUU | SEQ ID NO: 7299 | CUAGAAG |
| SEQ ID NO: 7045 | CGUGACA | SEQ ID NO: 7096 | CGUGUCU | SEQ ID NO: 7147 | CGUUGGG | SEQ ID NO: 7198 | CUAACUC | SEQ ID NO: 7249 | CUACCAA | SEQ ID NO: 7300 | CUAGAAU |
| SEQ ID NO: 7046 | CGUGACC | SEQ ID NO: 7097 | CGUGUGA | SEQ ID NO: 7148 | CGUUGGU | SEQ ID NO: 7199 | CUAACUG | SEQ ID NO: 7250 | CUACCAC | SEQ ID NO: 7301 | CUAGACA |
| SEQ ID NO: 7047 | CGUGACG | SEQ ID NO: 7098 | CGUGUGC | SEQ ID NO: 7149 | CGUUGUA | SEQ ID NO: 7200 | CUAACUU | SEQ ID NO: 7251 | CUACCAG | SEQ ID NO: 7302 | CUAGACC |
| SEQ ID NO: 7048 | CGUGACU | SEQ ID NO: 7099 | CGUGUGG | SEQ ID NO: 7150 | CGUUGUC | SEQ ID NO: 7201 | CUAAGAA | SEQ ID NO: 7252 | CUACCAU | SEQ ID NO: 7303 | CUAGACG |
| SEQ ID NO: 7049 | CGUGAGA | SEQ ID NO: 7100 | CGUGUGU | SEQ ID NO: 7151 | CGUUGUG | SEQ ID NO: 7202 | CUAAGAC | SEQ ID NO: 7253 | CUACCCA | SEQ ID NO: 7304 | CUAGACU |
| SEQ ID NO: 7050 | CGUGAGC | SEQ ID NO: 7101 | CGUGUUA | SEQ ID NO: 7152 | CGUUGUU | SEQ ID NO: 7203 | CUAAGAG | SEQ ID NO: 7254 | CUACCCC | SEQ ID NO: 7305 | CUAGAGA |
| SEQ ID NO: 7051 | CGUGAGG | SEQ ID NO: 7102 | CGUGUUC | SEQ ID NO: 7153 | CGUUUAA | SEQ ID NO: 7204 | CUAAGAU | SEQ ID NO: 7255 | CUACCCG | SEQ ID NO: 7306 | CUAGAGC |
| SEQ ID NO: 7052 | CGUGAGU | SEQ ID NO: 7103 | CGUGUUG | SEQ ID NO: 7154 | CGUUUAC | SEQ ID NO: 7205 | CUAAGCA | SEQ ID NO: 7256 | CUACCCU | SEQ ID NO: 7307 | CUAGAGG |
| SEQ ID NO: 7053 | CGUGAUA | SEQ ID NO: 7104 | CGUGUUU | SEQ ID NO: 7155 | CGUUUAG | SEQ ID NO: 7206 | CUAAGCC | SEQ ID NO: 7257 | CUACCGA | SEQ ID NO: 7308 | CUAGAGU |
| SEQ ID NO: 7054 | CGUGAUC | SEQ ID NO: 7105 | CGUUAAA | SEQ ID NO: 7156 | CGUUUAU | SEQ ID NO: 7207 | CUAAGCG | SEQ ID NO: 7258 | CUACCGC | SEQ ID NO: 7309 | CUAGAUA |
| SEQ ID NO: 7055 | CGUGAUG | SEQ ID NO: 7106 | CGUUAAC | SEQ ID NO: 7157 | CGUUUCA | SEQ ID NO: 7208 | CUAAGCU | SEQ ID NO: 7259 | CUACCGG | SEQ ID NO: 7310 | CUAGAUC |
| SEQ ID NO: 7056 | CGUGAUU | SEQ ID NO: 7107 | CGUUAAG | SEQ ID NO: 7158 | CGUUUCC | SEQ ID NO: 7209 | CUAAGGA | SEQ ID NO: 7260 | CUACCGU | SEQ ID NO: 7311 | CUAGAUG |
| SEQ ID NO: 7057 | CGUGCAA | SEQ ID NO: 7108 | CGUUAAU | SEQ ID NO: 7159 | CGUUUCG | SEQ ID NO: 7210 | CUAAGGC | SEQ ID NO: 7261 | CUACCUA | SEQ ID NO: 7312 | CUAGAUU |
| SEQ ID NO: 7058 | CGUGCAC | SEQ ID NO: 7109 | CGUUACA | SEQ ID NO: 7160 | CGUUUCU | SEQ ID NO: 7211 | CUAAGGG | SEQ ID NO: 7262 | CUACCUC | SEQ ID NO: 7313 | CUAGCAA |
| SEQ ID NO: 7059 | CGUGCAG | SEQ ID NO: 7110 | CGUUACC | SEQ ID NO: 7161 | CGUUUGA | SEQ ID NO: 7212 | CUAAGGU | SEQ ID NO: 7263 | CUACCUG | SEQ ID NO: 7314 | CUAGCAC |
| SEQ ID NO: 7060 | CGUGCAU | SEQ ID NO: 7111 | CGUUACG | SEQ ID NO: 7162 | CGUUUGC | SEQ ID NO: 7213 | CUAAGUA | SEQ ID NO: 7264 | CUACCUU | SEQ ID NO: 7315 | CUAGCAG |
| SEQ ID NO: 7061 | CGUGCCA | SEQ ID NO: 7112 | CGUUACU | SEQ ID NO: 7163 | CGUUUGG | SEQ ID NO: 7214 | CUAAGUC | SEQ ID NO: 7265 | CUACGAA | SEQ ID NO: 7316 | CUAGCAU |
| SEQ ID NO: 7062 | CGUGCCC | SEQ ID NO: 7113 | CGUUAGA | SEQ ID NO: 7164 | CGUUUGU | SEQ ID NO: 7215 | CUAAGUG | SEQ ID NO: 7266 | CUACGAC | SEQ ID NO: 7317 | CUAGCCA |
| SEQ ID NO: 7063 | CGUGCCG | SEQ ID NO: 7114 | CGUUAGC | SEQ ID NO: 7165 | CGUUUUA | SEQ ID NO: 7216 | CUAAGUU | SEQ ID NO: 7267 | CUACGAG | SEQ ID NO: 7318 | CUAGCCC |
| SEQ ID NO: 7064 | CGUGCCU | SEQ ID NO: 7115 | CGUUAGG | SEQ ID NO: 7166 | CGUUUUC | SEQ ID NO: 7217 | CUAAUAA | SEQ ID NO: 7268 | CUACGAU | SEQ ID NO: 7319 | CUAGCCG |
| SEQ ID NO: 7065 | CGUGCGA | SEQ ID NO: 7116 | CGUUAGU | SEQ ID NO: 7167 | CGUUUUG | SEQ ID NO: 7218 | CUAAUAC | SEQ ID NO: 7269 | CUACGCA | SEQ ID NO: 7320 | CUAGCCU |
| SEQ ID NO: 7066 | CGUGCGC | SEQ ID NO: 7117 | CGUUAUA | SEQ ID NO: 7168 | CGUUUUU | SEQ ID NO: 7219 | CUAAUAG | SEQ ID NO: 7270 | CUACGCC | SEQ ID NO: 7321 | CUAGCGA |
| SEQ ID NO: 7067 | CGUGCGG | SEQ ID NO: 7118 | CGUUAUC | SEQ ID NO: 7169 | CUAAAAA | SEQ ID NO: 7220 | CUAAUAU | SEQ ID NO: 7271 | CUACGCG | SEQ ID NO: 7322 | CUAGCGC |
| SEQ ID NO: 7068 | CGUGCGU | SEQ ID NO: 7119 | CGUUAUG | SEQ ID NO: 7170 | CUAAAAC | SEQ ID NO: 7221 | CUAAUCA | SEQ ID NO: 7272 | CUACGCU | SEQ ID NO: 7323 | CUAGCGG |
| SEQ ID NO: 7069 | CGUGCUA | SEQ ID NO: 7120 | CGUUAUU | SEQ ID NO: 7171 | CUAAAAG | SEQ ID NO: 7222 | CUAAUCC | SEQ ID NO: 7273 | CUACGGA | SEQ ID NO: 7324 | CUAGCGU |
| SEQ ID NO: 7070 | CGUGCUC | SEQ ID NO: 7121 | CGUUCAA | SEQ ID NO: 7172 | CUAAAAU | SEQ ID NO: 7223 | CUAAUCG | SEQ ID NO: 7274 | CUACGGC | SEQ ID NO: 7325 | CUAGCUA |
| SEQ ID NO: 7071 | CGUGCUG | SEQ ID NO: 7122 | CGUUCAC | SEQ ID NO: 7173 | CUAAACA | SEQ ID NO: 7224 | CUAAUCU | SEQ ID NO: 7275 | CUACGGG | SEQ ID NO: 7326 | CUAGCUC |
| SEQ ID NO: 7072 | CGUGCUU | SEQ ID NO: 7123 | CGUUCAG | SEQ ID NO: 7174 | CUAAACC | SEQ ID NO: 7225 | CUAAUGA | SEQ ID NO: 7276 | CUACGGU | SEQ ID NO: 7327 | CUAGCUG |
| SEQ ID NO: 7073 | CGUGGAA | SEQ ID NO: 7124 | CGUUCAU | SEQ ID NO: 7175 | CUAAACG | SEQ ID NO: 7226 | CUAAUGC | SEQ ID NO: 7277 | CUACGUA | SEQ ID NO: 7328 | CUAGCUU |
| SEQ ID NO: 7074 | CGUGGAC | SEQ ID NO: 7125 | CGUUCCA | SEQ ID NO: 7176 | CUAAACU | SEQ ID NO: 7227 | CUAAUGG | SEQ ID NO: 7278 | CUACGUC | SEQ ID NO: 7329 | CUAGGAA |
| SEQ ID NO: 7075 | CGUGGAG | SEQ ID NO: 7126 | CGUUCCC | SEQ ID NO: 7177 | CUAAAGA | SEQ ID NO: 7228 | CUAAUGU | SEQ ID NO: 7279 | CUACGUG | SEQ ID NO: 7330 | CUAGGAC |
| SEQ ID NO: 7076 | CGUGGAU | SEQ ID NO: 7127 | CGUUCCG | SEQ ID NO: 7178 | CUAAAGC | SEQ ID NO: 7229 | CUAAUUA | SEQ ID NO: 7280 | CUACGUU | SEQ ID NO: 7331 | CUAGGAG |
| SEQ ID NO: 7077 | CGUGGCA | SEQ ID NO: 7128 | CGUUCCU | SEQ ID NO: 7179 | CUAAAGG | SEQ ID NO: 7230 | CUAAUUC | SEQ ID NO: 7281 | CUACUAA | SEQ ID NO: 7332 | CUAGGAU |
| SEQ ID NO: 7078 | CGUGGCC | SEQ ID NO: 7129 | CGUUCGA | SEQ ID NO: 7180 | CUAAAGU | SEQ ID NO: 7231 | CUAAUUG | SEQ ID NO: 7282 | CUACUAC | SEQ ID NO: 7333 | CUAGGCA |
| SEQ ID NO: 7079 | CGUGGCG | SEQ ID NO: 7130 | CGUUCGC | SEQ ID NO: 7181 | CUAAAUA | SEQ ID NO: 7232 | CUAAUUU | SEQ ID NO: 7283 | CUACUAG | SEQ ID NO: 7334 | CUAGGCC |
| SEQ ID NO: 7080 | CGUGGCU | SEQ ID NO: 7131 | CGUUCGG | SEQ ID NO: 7182 | CUAAAUC | SEQ ID NO: 7233 | CUACAAA | SEQ ID NO: 7284 | CUACUAU | SEQ ID NO: 7335 | CUAGGCG |
| SEQ ID NO: 7081 | CGUGGGA | SEQ ID NO: 7132 | CGUUCGU | SEQ ID NO: 7183 | CUAAAUG | SEQ ID NO: 7234 | CUACAAC | SEQ ID NO: 7285 | CUACUCA | SEQ ID NO: 7336 | CUAGGCU |
| SEQ ID NO: 7082 | CGUGGGC | SEQ ID NO: 7133 | CGUUCUA | SEQ ID NO: 7184 | CUAAAUU | SEQ ID NO: 7235 | CUACAAG | SEQ ID NO: 7286 | CUACUCC | SEQ ID NO: 7337 | CUAGGGA |
| SEQ ID NO: 7083 | CGUGGGG | SEQ ID NO: 7134 | CGUUCUC | SEQ ID NO: 7185 | CUAACAA | SEQ ID NO: 7236 | CUACAAU | SEQ ID NO: 7287 | CUACUCG | SEQ ID NO: 7338 | CUAGGGC |
| SEQ ID NO: 7084 | CGUGGGU | SEQ ID NO: 7135 | CGUUCUG | SEQ ID NO: 7186 | CUAACAC | SEQ ID NO: 7237 | CUACACA | SEQ ID NO: 7288 | CUACUCU | SEQ ID NO: 7339 | CUAGGGG |
| SEQ ID NO: 7085 | CGUGGUA | SEQ ID NO: 7136 | CGUUCUU | SEQ ID NO: 7187 | CUAACAG | SEQ ID NO: 7238 | CUACACC | SEQ ID NO: 7289 | CUACUGA | SEQ ID NO: 7340 | CUAGGGU |
| SEQ ID NO: 7086 | CGUGGUC | SEQ ID NO: 7137 | CGUUGAA | SEQ ID NO: 7188 | CUAACAU | SEQ ID NO: 7239 | CUACACG | SEQ ID NO: 7290 | CUACUGC | SEQ ID NO: 7341 | CUAGGUA |
| SEQ ID NO: 7087 | CGUGGUG | SEQ ID NO: 7138 | CGUUGAC | SEQ ID NO: 7189 | CUAACCA | SEQ ID NO: 7240 | CUACACU | SEQ ID NO: 7291 | CUACUGG | SEQ ID NO: 7342 | CUAGGUC |
| SEQ ID NO: 7088 | CGUGGUU | SEQ ID NO: 7139 | CGUUGAG | SEQ ID NO: 7190 | CUAACCC | SEQ ID NO: 7241 | CUACAGA | SEQ ID NO: 7292 | CUACUGU | SEQ ID NO: 7343 | CUAGGUG |
| SEQ ID NO: 7089 | CGUGUAA | SEQ ID NO: 7140 | CGUUGAU | SEQ ID NO: 7191 | CUAACCG | SEQ ID NO: 7242 | CUACAGC | SEQ ID NO: 7293 | CUACUUA | SEQ ID NO: 7344 | CUAGGUU |

Table 25

| | | | | | | | | | | | |
|---|---|---|---|---|---|---|---|---|---|---|---|
| SEQ ID NO: 7345 | CUAGUAA | SEQ ID NO: 7396 | CUAUGAU | SEQ ID NO: 7447 | CUCACCG | SEQ ID NO: 7498 | CUCCAGC | SEQ ID NO: 7549 | CUCCUUA | SEQ ID NO: 7600 | CUCGGUU |
| SEQ ID NO: 7346 | CUAGUAC | SEQ ID NO: 7397 | CUAUGCA | SEQ ID NO: 7448 | CUCACCU | SEQ ID NO: 7499 | CUCCAGG | SEQ ID NO: 7550 | CUCCUUC | SEQ ID NO: 7601 | CUCGUAA |
| SEQ ID NO: 7347 | CUAGUAG | SEQ ID NO: 7398 | CUAUGCC | SEQ ID NO: 7449 | CUCACGA | SEQ ID NO: 7500 | CUCCAGU | SEQ ID NO: 7551 | CUCCUUG | SEQ ID NO: 7602 | CUCGUAC |
| SEQ ID NO: 7348 | CUAGUAU | SEQ ID NO: 7399 | CUAUGCG | SEQ ID NO: 7450 | CUCACGC | SEQ ID NO: 7501 | CUCCAUA | SEQ ID NO: 7552 | CUCCUUU | SEQ ID NO: 7603 | CUCGUAG |
| SEQ ID NO: 7349 | CUAGUCA | SEQ ID NO: 7400 | CUAUGCU | SEQ ID NO: 7451 | CUCACGG | SEQ ID NO: 7502 | CUCCAUC | SEQ ID NO: 7553 | CUCGAAA | SEQ ID NO: 7604 | CUCGUAU |
| SEQ ID NO: 7350 | CUAGUCC | SEQ ID NO: 7401 | CUAUGGA | SEQ ID NO: 7452 | CUCACGU | SEQ ID NO: 7503 | CUCCAUG | SEQ ID NO: 7554 | CUCGAAC | SEQ ID NO: 7605 | CUCGUCA |
| SEQ ID NO: 7351 | CUAGUCG | SEQ ID NO: 7402 | CUAUGGC | SEQ ID NO: 7453 | CUCACUA | SEQ ID NO: 7504 | CUCCAUU | SEQ ID NO: 7555 | CUCGAAG | SEQ ID NO: 7606 | CUCGUCC |
| SEQ ID NO: 7352 | CUAGUCU | SEQ ID NO: 7403 | CUAUGGG | SEQ ID NO: 7454 | CUCACUC | SEQ ID NO: 7505 | CUCCCAA | SEQ ID NO: 7556 | CUCGAAU | SEQ ID NO: 7607 | CUCGUCG |
| SEQ ID NO: 7353 | CUAGUGA | SEQ ID NO: 7404 | CUAUGGU | SEQ ID NO: 7455 | CUCACUG | SEQ ID NO: 7506 | CUCCCAC | SEQ ID NO: 7557 | CUCGACA | SEQ ID NO: 7608 | CUCGUCU |
| SEQ ID NO: 7354 | CUAGUGC | SEQ ID NO: 7405 | CUAUGUA | SEQ ID NO: 7456 | CUCACUU | SEQ ID NO: 7507 | CUCCCAG | SEQ ID NO: 7558 | CUCGACC | SEQ ID NO: 7609 | CUCGUGA |
| SEQ ID NO: 7355 | CUAGUGG | SEQ ID NO: 7406 | CUAUGUC | SEQ ID NO: 7457 | CUCAGAA | SEQ ID NO: 7508 | CUCCCAU | SEQ ID NO: 7559 | CUCGACG | SEQ ID NO: 7610 | CUCGUGC |
| SEQ ID NO: 7356 | CUAGUGU | SEQ ID NO: 7407 | CUAUGUG | SEQ ID NO: 7458 | CUCAGAC | SEQ ID NO: 7509 | CUCCCCA | SEQ ID NO: 7560 | CUCGACU | SEQ ID NO: 7611 | CUCGUGG |
| SEQ ID NO: 7357 | CUAGUUA | SEQ ID NO: 7408 | CUAUGUU | SEQ ID NO: 7459 | CUCAGAG | SEQ ID NO: 7510 | CUCCCCC | SEQ ID NO: 7561 | CUCGAGA | SEQ ID NO: 7612 | CUCGUGU |
| SEQ ID NO: 7358 | CUAGUUC | SEQ ID NO: 7409 | CUAUUAA | SEQ ID NO: 7460 | CUCAGAU | SEQ ID NO: 7511 | CUCCCCG | SEQ ID NO: 7562 | CUCGAGC | SEQ ID NO: 7613 | CUCGUUA |
| SEQ ID NO: 7359 | CUAGUUG | SEQ ID NO: 7410 | CUAUUAC | SEQ ID NO: 7461 | CUCAGCA | SEQ ID NO: 7512 | CUCCCCU | SEQ ID NO: 7563 | CUCGAGG | SEQ ID NO: 7614 | CUCGUUC |
| SEQ ID NO: 7360 | CUAGUUU | SEQ ID NO: 7411 | CUAUUAG | SEQ ID NO: 7462 | CUCAGCC | SEQ ID NO: 7513 | CUCCCGA | SEQ ID NO: 7564 | CUCGAGU | SEQ ID NO: 7615 | CUCGUUG |
| SEQ ID NO: 7361 | CUAUAAA | SEQ ID NO: 7412 | CUAUUAU | SEQ ID NO: 7463 | CUCAGCG | SEQ ID NO: 7514 | CUCCCGC | SEQ ID NO: 7565 | CUCGAUA | SEQ ID NO: 7616 | CUCGUUU |
| SEQ ID NO: 7362 | CUAUAAC | SEQ ID NO: 7413 | CUAUUCA | SEQ ID NO: 7464 | CUCAGCU | SEQ ID NO: 7515 | CUCCCGG | SEQ ID NO: 7566 | CUCGAUC | SEQ ID NO: 7617 | CUCUAAA |
| SEQ ID NO: 7363 | CUAUAAG | SEQ ID NO: 7414 | CUAUUCC | SEQ ID NO: 7465 | CUCAGGA | SEQ ID NO: 7516 | CUCCCGU | SEQ ID NO: 7567 | CUCGAUG | SEQ ID NO: 7618 | CUCUAAC |
| SEQ ID NO: 7364 | CUAUAAU | SEQ ID NO: 7415 | CUAUUCG | SEQ ID NO: 7466 | CUCAGGC | SEQ ID NO: 7517 | CUCCCUA | SEQ ID NO: 7568 | CUCGAUU | SEQ ID NO: 7619 | CUCUAAG |
| SEQ ID NO: 7365 | CUAUACA | SEQ ID NO: 7416 | CUAUUCU | SEQ ID NO: 7467 | CUCAGGG | SEQ ID NO: 7518 | CUCCCUC | SEQ ID NO: 7569 | CUCGCAA | SEQ ID NO: 7620 | CUCUAAU |
| SEQ ID NO: 7366 | CUAUACC | SEQ ID NO: 7417 | CUAUUGA | SEQ ID NO: 7468 | CUCAGGU | SEQ ID NO: 7519 | CUCCCUG | SEQ ID NO: 7570 | CUCGCAC | SEQ ID NO: 7621 | CUCUACA |
| SEQ ID NO: 7367 | CUAUACG | SEQ ID NO: 7418 | CUAUUGC | SEQ ID NO: 7469 | CUCAGUA | SEQ ID NO: 7520 | CUCCCUU | SEQ ID NO: 7571 | CUCGCAG | SEQ ID NO: 7622 | CUCUACC |
| SEQ ID NO: 7368 | CUAUACU | SEQ ID NO: 7419 | CUAUUGG | SEQ ID NO: 7470 | CUCAGUC | SEQ ID NO: 7521 | CUCCGAA | SEQ ID NO: 7572 | CUCGCAU | SEQ ID NO: 7623 | CUCUACG |
| SEQ ID NO: 7369 | CUAUAGA | SEQ ID NO: 7420 | CUAUUGU | SEQ ID NO: 7471 | CUCAGUG | SEQ ID NO: 7522 | CUCCGAC | SEQ ID NO: 7573 | CUCGCCA | SEQ ID NO: 7624 | CUCUACU |
| SEQ ID NO: 7370 | CUAUAGC | SEQ ID NO: 7421 | CUAUUUA | SEQ ID NO: 7472 | CUCAGUU | SEQ ID NO: 7523 | CUCCGAG | SEQ ID NO: 7574 | CUCGCCC | SEQ ID NO: 7625 | CUCUAGA |
| SEQ ID NO: 7371 | CUAUAGG | SEQ ID NO: 7422 | CUAUUUC | SEQ ID NO: 7473 | CUCAUAA | SEQ ID NO: 7524 | CUCCGAU | SEQ ID NO: 7575 | CUCGCCG | SEQ ID NO: 7626 | CUCUAGC |
| SEQ ID NO: 7372 | CUAUAGU | SEQ ID NO: 7423 | CUAUUUG | SEQ ID NO: 7474 | CUCAUAC | SEQ ID NO: 7525 | CUCCGCA | SEQ ID NO: 7576 | CUCGCCU | SEQ ID NO: 7627 | CUCUAGG |
| SEQ ID NO: 7373 | CUAUAUA | SEQ ID NO: 7424 | CUAUUUU | SEQ ID NO: 7475 | CUCAUAG | SEQ ID NO: 7526 | CUCCGCC | SEQ ID NO: 7577 | CUCGCGA | SEQ ID NO: 7628 | CUCUAGU |
| SEQ ID NO: 7374 | CUAUAUC | SEQ ID NO: 7425 | CUCAAAA | SEQ ID NO: 7476 | CUCAUAU | SEQ ID NO: 7527 | CUCCGCG | SEQ ID NO: 7578 | CUCGCGC | SEQ ID NO: 7629 | CUCUAUA |
| SEQ ID NO: 7375 | CUAUAUG | SEQ ID NO: 7426 | CUCAAAC | SEQ ID NO: 7477 | CUCAUCA | SEQ ID NO: 7528 | CUCCGCU | SEQ ID NO: 7579 | CUCGCGG | SEQ ID NO: 7630 | CUCUAUC |
| SEQ ID NO: 7376 | CUAUAUU | SEQ ID NO: 7427 | CUCAAAG | SEQ ID NO: 7478 | CUCAUCC | SEQ ID NO: 7529 | CUCCGGA | SEQ ID NO: 7580 | CUCGCGU | SEQ ID NO: 7631 | CUCUAUG |
| SEQ ID NO: 7377 | CUAUCAA | SEQ ID NO: 7428 | CUCAAAU | SEQ ID NO: 7479 | CUCAUCG | SEQ ID NO: 7530 | CUCCGGC | SEQ ID NO: 7581 | CUCGCUA | SEQ ID NO: 7632 | CUCUAUU |
| SEQ ID NO: 7378 | CUAUCAC | SEQ ID NO: 7429 | CUCAACA | SEQ ID NO: 7480 | CUCAUCU | SEQ ID NO: 7531 | CUCCGGG | SEQ ID NO: 7582 | CUCGCUC | SEQ ID NO: 7633 | CUCUCAA |
| SEQ ID NO: 7379 | CUAUCAG | SEQ ID NO: 7430 | CUCAACC | SEQ ID NO: 7481 | CUCAUGA | SEQ ID NO: 7532 | CUCCGGU | SEQ ID NO: 7583 | CUCGCUG | SEQ ID NO: 7634 | CUCUCAC |
| SEQ ID NO: 7380 | CUAUCAU | SEQ ID NO: 7431 | CUCAACG | SEQ ID NO: 7482 | CUCAUGC | SEQ ID NO: 7533 | CUCCGUA | SEQ ID NO: 7584 | CUCGCUU | SEQ ID NO: 7635 | CUCUCAG |
| SEQ ID NO: 7381 | CUAUCCA | SEQ ID NO: 7432 | CUCAACU | SEQ ID NO: 7483 | CUCAUGG | SEQ ID NO: 7534 | CUCCGUC | SEQ ID NO: 7585 | CUCGGAA | SEQ ID NO: 7636 | CUCUCAU |
| SEQ ID NO: 7382 | CUAUCCC | SEQ ID NO: 7433 | CUCAAGA | SEQ ID NO: 7484 | CUCAUGU | SEQ ID NO: 7535 | CUCCGUG | SEQ ID NO: 7586 | CUCGGAC | SEQ ID NO: 7637 | CUCUCCA |
| SEQ ID NO: 7383 | CUAUCCG | SEQ ID NO: 7434 | CUCAAGC | SEQ ID NO: 7485 | CUCAUUA | SEQ ID NO: 7536 | CUCCGUU | SEQ ID NO: 7587 | CUCGGAG | SEQ ID NO: 7638 | CUCUCCC |
| SEQ ID NO: 7384 | CUAUCCU | SEQ ID NO: 7435 | CUCAAGG | SEQ ID NO: 7486 | CUCAUUC | SEQ ID NO: 7537 | CUCCUAA | SEQ ID NO: 7588 | CUCGGAU | SEQ ID NO: 7639 | CUCUCCG |
| SEQ ID NO: 7385 | CUAUCGA | SEQ ID NO: 7436 | CUCAAGU | SEQ ID NO: 7487 | CUCAUUG | SEQ ID NO: 7538 | CUCCUAC | SEQ ID NO: 7589 | CUCGGCA | SEQ ID NO: 7640 | CUCUCCU |
| SEQ ID NO: 7386 | CUAUCGC | SEQ ID NO: 7437 | CUCAAUA | SEQ ID NO: 7488 | CUCAUUU | SEQ ID NO: 7539 | CUCCUAG | SEQ ID NO: 7590 | CUCGGCC | SEQ ID NO: 7641 | CUCUCGA |
| SEQ ID NO: 7387 | CUAUCGG | SEQ ID NO: 7438 | CUCAAUC | SEQ ID NO: 7489 | CUCCAAA | SEQ ID NO: 7540 | CUCCUAU | SEQ ID NO: 7591 | CUCGGCG | SEQ ID NO: 7642 | CUCUCGC |
| SEQ ID NO: 7388 | CUAUCGU | SEQ ID NO: 7439 | CUCAAUG | SEQ ID NO: 7490 | CUCCAAC | SEQ ID NO: 7541 | CUCCUCA | SEQ ID NO: 7592 | CUCGGCU | SEQ ID NO: 7643 | CUCUCGG |
| SEQ ID NO: 7389 | CUAUCUA | SEQ ID NO: 7440 | CUCAAUU | SEQ ID NO: 7491 | CUCCAAG | SEQ ID NO: 7542 | CUCCUCC | SEQ ID NO: 7593 | CUCGGGA | SEQ ID NO: 7644 | CUCUCGU |
| SEQ ID NO: 7390 | CUAUCUC | SEQ ID NO: 7441 | CUCACAA | SEQ ID NO: 7492 | CUCCAAU | SEQ ID NO: 7543 | CUCCUCG | SEQ ID NO: 7594 | CUCGGGC | SEQ ID NO: 7645 | CUCUCUA |
| SEQ ID NO: 7391 | CUAUCUG | SEQ ID NO: 7442 | CUCACAC | SEQ ID NO: 7493 | CUCCACA | SEQ ID NO: 7544 | CUCCUCU | SEQ ID NO: 7595 | CUCGGGG | SEQ ID NO: 7646 | CUCUCUC |
| SEQ ID NO: 7392 | CUAUCUU | SEQ ID NO: 7443 | CUCACAG | SEQ ID NO: 7494 | CUCCACC | SEQ ID NO: 7545 | CUCCUGA | SEQ ID NO: 7596 | CUCGGGU | SEQ ID NO: 7647 | CUCUCUG |
| SEQ ID NO: 7393 | CUAUGAA | SEQ ID NO: 7444 | CUCACAU | SEQ ID NO: 7495 | CUCCACG | SEQ ID NO: 7546 | CUCCUGC | SEQ ID NO: 7597 | CUCGGUA | SEQ ID NO: 7648 | CUCUCUU |
| SEQ ID NO: 7394 | CUAUGAC | SEQ ID NO: 7445 | CUCACCA | SEQ ID NO: 7496 | CUCCACU | SEQ ID NO: 7547 | CUCCUGG | SEQ ID NO: 7598 | CUCGGUC | SEQ ID NO: 7649 | CUCUGAA |
| SEQ ID NO: 7395 | CUAUGAG | SEQ ID NO: 7446 | CUCACCC | SEQ ID NO: 7497 | CUCCAGA | SEQ ID NO: 7548 | CUCCUGU | SEQ ID NO: 7599 | CUCGGUG | SEQ ID NO: 7650 | CUCUGAC |

Table 26

| | | | | | | | | | | | |
|---|---|---|---|---|---|---|---|---|---|---|---|
| SEQ ID NO: 7651 | CUCUGAG | SEQ ID NO: 7702 | CUGACCC | SEQ ID NO: 7753 | CUGCAGA | SEQ ID NO: 7804 | CUGCUGU | SEQ ID NO: 7855 | CUGGGUG | SEQ ID NO: 7906 | CUGUGAC |
| SEQ ID NO: 7652 | CUCUGAU | SEQ ID NO: 7703 | CUGACCG | SEQ ID NO: 7754 | CUGCAGC | SEQ ID NO: 7805 | CUGCUUA | SEQ ID NO: 7856 | CUGGGUU | SEQ ID NO: 7907 | CUGUGAG |
| SEQ ID NO: 7653 | CUCUGCA | SEQ ID NO: 7704 | CUGACCU | SEQ ID NO: 7755 | CUGCAGG | SEQ ID NO: 7806 | CUGCUUC | SEQ ID NO: 7857 | CUGGUAA | SEQ ID NO: 7908 | CUGUGAU |
| SEQ ID NO: 7654 | CUCUGCC | SEQ ID NO: 7705 | CUGACGA | SEQ ID NO: 7756 | CUGCAGU | SEQ ID NO: 7807 | CUGCUUG | SEQ ID NO: 7858 | CUGGUAC | SEQ ID NO: 7909 | CUGUGCA |
| SEQ ID NO: 7655 | CUCUGCG | SEQ ID NO: 7706 | CUGACGC | SEQ ID NO: 7757 | CUGCAUA | SEQ ID NO: 7808 | CUGCUUU | SEQ ID NO: 7859 | CUGGUAG | SEQ ID NO: 7910 | CUGUGCC |
| SEQ ID NO: 7656 | CUCUGCU | SEQ ID NO: 7707 | CUGACGG | SEQ ID NO: 7758 | CUGCAUC | SEQ ID NO: 7809 | CUGGAAA | SEQ ID NO: 7860 | CUGGUAU | SEQ ID NO: 7911 | CUGUGCG |
| SEQ ID NO: 7657 | CUCUGGA | SEQ ID NO: 7708 | CUGACGU | SEQ ID NO: 7759 | CUGCAUG | SEQ ID NO: 7810 | CUGGAAC | SEQ ID NO: 7861 | CUGGUCA | SEQ ID NO: 7912 | CUGUGCU |
| SEQ ID NO: 7658 | CUCUGGC | SEQ ID NO: 7709 | CUGACUA | SEQ ID NO: 7760 | CUGCAUU | SEQ ID NO: 7811 | CUGGAAG | SEQ ID NO: 7862 | CUGGUCC | SEQ ID NO: 7913 | CUGUGGA |
| SEQ ID NO: 7659 | CUCUGGG | SEQ ID NO: 7710 | CUGACUC | SEQ ID NO: 7761 | CUGCCAA | SEQ ID NO: 7812 | CUGGAAU | SEQ ID NO: 7863 | CUGGUCG | SEQ ID NO: 7914 | CUGUGGC |
| SEQ ID NO: 7660 | CUCUGGU | SEQ ID NO: 7711 | CUGACUG | SEQ ID NO: 7762 | CUGCCAC | SEQ ID NO: 7813 | CUGGACA | SEQ ID NO: 7864 | CUGGUCU | SEQ ID NO: 7915 | CUGUGGG |
| SEQ ID NO: 7661 | CUCUGUA | SEQ ID NO: 7712 | CUGACUU | SEQ ID NO: 7763 | CUGCCAG | SEQ ID NO: 7814 | CUGGACC | SEQ ID NO: 7865 | CUGGUGA | SEQ ID NO: 7916 | CUGUGGU |
| SEQ ID NO: 7662 | CUCUGUC | SEQ ID NO: 7713 | CUGAGAA | SEQ ID NO: 7764 | CUGCCAU | SEQ ID NO: 7815 | CUGGACG | SEQ ID NO: 7866 | CUGGUGC | SEQ ID NO: 7917 | CUGUGUA |
| SEQ ID NO: 7663 | CUCUGUG | SEQ ID NO: 7714 | CUGAGAC | SEQ ID NO: 7765 | CUGCCCA | SEQ ID NO: 7816 | CUGGACU | SEQ ID NO: 7867 | CUGGUGG | SEQ ID NO: 7918 | CUGUGUC |
| SEQ ID NO: 7664 | CUCUGUU | SEQ ID NO: 7715 | CUGAGAG | SEQ ID NO: 7766 | CUGCCCC | SEQ ID NO: 7817 | CUGGAGA | SEQ ID NO: 7868 | CUGGUGU | SEQ ID NO: 7919 | CUGUGUG |
| SEQ ID NO: 7665 | CUCUUAA | SEQ ID NO: 7716 | CUGAGAU | SEQ ID NO: 7767 | CUGCCCG | SEQ ID NO: 7818 | CUGGAGC | SEQ ID NO: 7869 | CUGGUUA | SEQ ID NO: 7920 | CUGUGUU |
| SEQ ID NO: 7666 | CUCUUAC | SEQ ID NO: 7717 | CUGAGCA | SEQ ID NO: 7768 | CUGCCCU | SEQ ID NO: 7819 | CUGGAGG | SEQ ID NO: 7870 | CUGGUUC | SEQ ID NO: 7921 | CUGUUAA |
| SEQ ID NO: 7667 | CUCUUAG | SEQ ID NO: 7718 | CUGAGCC | SEQ ID NO: 7769 | CUGCCGA | SEQ ID NO: 7820 | CUGGAGU | SEQ ID NO: 7871 | CUGGUUG | SEQ ID NO: 7922 | CUGUUAC |
| SEQ ID NO: 7668 | CUCUUAU | SEQ ID NO: 7719 | CUGAGCG | SEQ ID NO: 7770 | CUGCCGC | SEQ ID NO: 7821 | CUGGAUA | SEQ ID NO: 7872 | CUGGUUU | SEQ ID NO: 7923 | CUGUUAG |
| SEQ ID NO: 7669 | CUCUUCA | SEQ ID NO: 7720 | CUGAGCU | SEQ ID NO: 7771 | CUGCCGG | SEQ ID NO: 7822 | CUGGAUC | SEQ ID NO: 7873 | CUGUAAA | SEQ ID NO: 7924 | CUGUUAU |
| SEQ ID NO: 7670 | CUCUUCC | SEQ ID NO: 7721 | CUGAGGA | SEQ ID NO: 7772 | CUGCCGU | SEQ ID NO: 7823 | CUGGAUG | SEQ ID NO: 7874 | CUGUAAC | SEQ ID NO: 7925 | CUGUUCA |
| SEQ ID NO: 7671 | CUCUUCG | SEQ ID NO: 7722 | CUGAGGC | SEQ ID NO: 7773 | CUGCCUA | SEQ ID NO: 7824 | CUGGAUU | SEQ ID NO: 7875 | CUGUAAG | SEQ ID NO: 7926 | CUGUUCC |
| SEQ ID NO: 7672 | CUCUUCU | SEQ ID NO: 7723 | CUGAGGG | SEQ ID NO: 7774 | CUGCCUC | SEQ ID NO: 7825 | CUGGCAA | SEQ ID NO: 7876 | CUGUAAU | SEQ ID NO: 7927 | CUGUUCG |
| SEQ ID NO: 7673 | CUCUUGA | SEQ ID NO: 7724 | CUGAGGU | SEQ ID NO: 7775 | CUGCCUG | SEQ ID NO: 7826 | CUGGCAC | SEQ ID NO: 7877 | CUGUACA | SEQ ID NO: 7928 | CUGUUCU |
| SEQ ID NO: 7674 | CUCUUGC | SEQ ID NO: 7725 | CUGAGUA | SEQ ID NO: 7776 | CUGCCUU | SEQ ID NO: 7827 | CUGGCAG | SEQ ID NO: 7878 | CUGUACC | SEQ ID NO: 7929 | CUGUUGA |
| SEQ ID NO: 7675 | CUCUUGG | SEQ ID NO: 7726 | CUGAGUC | SEQ ID NO: 7777 | CUGCGAA | SEQ ID NO: 7828 | CUGGCAU | SEQ ID NO: 7879 | CUGUACG | SEQ ID NO: 7930 | CUGUUGC |
| SEQ ID NO: 7676 | CUCUUGU | SEQ ID NO: 7727 | CUGAGUG | SEQ ID NO: 7778 | CUGCGAC | SEQ ID NO: 7829 | CUGGCCA | SEQ ID NO: 7880 | CUGUACU | SEQ ID NO: 7931 | CUGUUGG |
| SEQ ID NO: 7677 | CUCUUUA | SEQ ID NO: 7728 | CUGAGUU | SEQ ID NO: 7779 | CUGCGAG | SEQ ID NO: 7830 | CUGGCCC | SEQ ID NO: 7881 | CUGUAGA | SEQ ID NO: 7932 | CUGUUGU |
| SEQ ID NO: 7678 | CUCUUUC | SEQ ID NO: 7729 | CUGAUAA | SEQ ID NO: 7780 | CUGCGAU | SEQ ID NO: 7831 | CUGGCCG | SEQ ID NO: 7882 | CUGUAGC | SEQ ID NO: 7933 | CUGUUUA |
| SEQ ID NO: 7679 | CUCUUUG | SEQ ID NO: 7730 | CUGAUAC | SEQ ID NO: 7781 | CUGCGCA | SEQ ID NO: 7832 | CUGGCCU | SEQ ID NO: 7883 | CUGUAGG | SEQ ID NO: 7934 | CUGUUUC |
| SEQ ID NO: 7680 | CUCUUUU | SEQ ID NO: 7731 | CUGAUAG | SEQ ID NO: 7782 | CUGCGCC | SEQ ID NO: 7833 | CUGGCGA | SEQ ID NO: 7884 | CUGUAGU | SEQ ID NO: 7935 | CUGUUUG |
| SEQ ID NO: 7681 | CUGAAAA | SEQ ID NO: 7732 | CUGAUAU | SEQ ID NO: 7783 | CUGCGCG | SEQ ID NO: 7834 | CUGGCGC | SEQ ID NO: 7885 | CUGUAUA | SEQ ID NO: 7936 | CUGUUUU |
| SEQ ID NO: 7682 | CUGAAAC | SEQ ID NO: 7733 | CUGAUCA | SEQ ID NO: 7784 | CUGCGCU | SEQ ID NO: 7835 | CUGGCGG | SEQ ID NO: 7886 | CUGUAUC | SEQ ID NO: 7937 | CUUAAAA |
| SEQ ID NO: 7683 | CUGAAAG | SEQ ID NO: 7734 | CUGAUCC | SEQ ID NO: 7785 | CUGCGGA | SEQ ID NO: 7836 | CUGGCGU | SEQ ID NO: 7887 | CUGUAUG | SEQ ID NO: 7938 | CUUAAAC |
| SEQ ID NO: 7684 | CUGAAAU | SEQ ID NO: 7735 | CUGAUCG | SEQ ID NO: 7786 | CUGCGGC | SEQ ID NO: 7837 | CUGGCUA | SEQ ID NO: 7888 | CUGUAUU | SEQ ID NO: 7939 | CUUAAAG |
| SEQ ID NO: 7685 | CUGAACA | SEQ ID NO: 7736 | CUGAUCU | SEQ ID NO: 7787 | CUGCGGG | SEQ ID NO: 7838 | CUGGCUC | SEQ ID NO: 7889 | CUGUCAA | SEQ ID NO: 7940 | CUUAAAU |
| SEQ ID NO: 7686 | CUGAACC | SEQ ID NO: 7737 | CUGAUGA | SEQ ID NO: 7788 | CUGCGGU | SEQ ID NO: 7839 | CUGGCUG | SEQ ID NO: 7890 | CUGUCAC | SEQ ID NO: 7941 | CUUAACA |
| SEQ ID NO: 7687 | CUGAACG | SEQ ID NO: 7738 | CUGAUGC | SEQ ID NO: 7789 | CUGCGUA | SEQ ID NO: 7840 | CUGGCUU | SEQ ID NO: 7891 | CUGUCAG | SEQ ID NO: 7942 | CUUAACC |
| SEQ ID NO: 7688 | CUGAACU | SEQ ID NO: 7739 | CUGAUGG | SEQ ID NO: 7790 | CUGCGUC | SEQ ID NO: 7841 | CUGGGAA | SEQ ID NO: 7892 | CUGUCAU | SEQ ID NO: 7943 | CUUAACG |
| SEQ ID NO: 7689 | CUGAAGA | SEQ ID NO: 7740 | CUGAUGU | SEQ ID NO: 7791 | CUGCGUG | SEQ ID NO: 7842 | CUGGGAC | SEQ ID NO: 7893 | CUGUCCA | SEQ ID NO: 7944 | CUUAACU |
| SEQ ID NO: 7690 | CUGAAGC | SEQ ID NO: 7741 | CUGAUUA | SEQ ID NO: 7792 | CUGCGUU | SEQ ID NO: 7843 | CUGGGAG | SEQ ID NO: 7894 | CUGUCCC | SEQ ID NO: 7945 | CUUAAGA |
| SEQ ID NO: 7691 | CUGAAGG | SEQ ID NO: 7742 | CUGAUUC | SEQ ID NO: 7793 | CUGCUAA | SEQ ID NO: 7844 | CUGGGAU | SEQ ID NO: 7895 | CUGUCCG | SEQ ID NO: 7946 | CUUAAGC |
| SEQ ID NO: 7692 | CUGAAGU | SEQ ID NO: 7743 | CUGAUUG | SEQ ID NO: 7794 | CUGCUAC | SEQ ID NO: 7845 | CUGGGCA | SEQ ID NO: 7896 | CUGUCCU | SEQ ID NO: 7947 | CUUAAGG |
| SEQ ID NO: 7693 | CUGAAUA | SEQ ID NO: 7744 | CUGAUUU | SEQ ID NO: 7795 | CUGCUAG | SEQ ID NO: 7846 | CUGGGCC | SEQ ID NO: 7897 | CUGUCGA | SEQ ID NO: 7948 | CUUAAGU |
| SEQ ID NO: 7694 | CUGAAUC | SEQ ID NO: 7745 | CUGCAAA | SEQ ID NO: 7796 | CUGCUAU | SEQ ID NO: 7847 | CUGGGCG | SEQ ID NO: 7898 | CUGUCGC | SEQ ID NO: 7949 | CUUAAUA |
| SEQ ID NO: 7695 | CUGAAUG | SEQ ID NO: 7746 | CUGCAAC | SEQ ID NO: 7797 | CUGCUCA | SEQ ID NO: 7848 | CUGGGCU | SEQ ID NO: 7899 | CUGUCGG | SEQ ID NO: 7950 | CUUAAUC |
| SEQ ID NO: 7696 | CUGAAUU | SEQ ID NO: 7747 | CUGCAAG | SEQ ID NO: 7798 | CUGCUCC | SEQ ID NO: 7849 | CUGGGGA | SEQ ID NO: 7900 | CUGUCGU | SEQ ID NO: 7951 | CUUAAUG |
| SEQ ID NO: 7697 | CUGACAA | SEQ ID NO: 7748 | CUGCAAU | SEQ ID NO: 7799 | CUGCUCG | SEQ ID NO: 7850 | CUGGGGC | SEQ ID NO: 7901 | CUGUCUA | SEQ ID NO: 7952 | CUUAAUU |
| SEQ ID NO: 7698 | CUGACAC | SEQ ID NO: 7749 | CUGCACA | SEQ ID NO: 7800 | CUGCUCU | SEQ ID NO: 7851 | CUGGGGG | SEQ ID NO: 7902 | CUGUCUC | SEQ ID NO: 7953 | CUUACAA |
| SEQ ID NO: 7699 | CUGACAG | SEQ ID NO: 7750 | CUGCACC | SEQ ID NO: 7801 | CUGCUGA | SEQ ID NO: 7852 | CUGGGGU | SEQ ID NO: 7903 | CUGUCUG | SEQ ID NO: 7954 | CUUACAC |
| SEQ ID NO: 7700 | CUGACAU | SEQ ID NO: 7751 | CUGCACG | SEQ ID NO: 7802 | CUGCUGC | SEQ ID NO: 7853 | CUGGGUA | SEQ ID NO: 7904 | CUGUCUU | SEQ ID NO: 7955 | CUUACAG |
| SEQ ID NO: 7701 | CUGACCA | SEQ ID NO: 7752 | CUGCACU | SEQ ID NO: 7803 | CUGCUGG | SEQ ID NO: 7854 | CUGGGUC | SEQ ID NO: 7905 | CUGUGAA | SEQ ID NO: 7956 | CUUACAU |

Table 27

| | |
|---|---|
| SEQ ID NO: 7957 | CUUACCA |
| SEQ ID NO: 7958 | CUUACCC |
| SEQ ID NO: 7959 | CUUACCG |
| SEQ ID NO: 7960 | CUUACCU |
| SEQ ID NO: 7961 | CUUACGA |
| SEQ ID NO: 7962 | CUUACGC |
| SEQ ID NO: 7963 | CUUACGG |
| SEQ ID NO: 7964 | CUUACGU |
| SEQ ID NO: 7965 | CUUACUA |
| SEQ ID NO: 7966 | CUUACUC |
| SEQ ID NO: 7967 | CUUACUG |
| SEQ ID NO: 7968 | CUUACUU |
| SEQ ID NO: 7969 | CUUAGAA |
| SEQ ID NO: 7970 | CUUAGAC |
| SEQ ID NO: 7971 | CUUAGAG |
| SEQ ID NO: 7972 | CUUAGAU |
| SEQ ID NO: 7973 | CUUAGCA |
| SEQ ID NO: 7974 | CUUAGCC |
| SEQ ID NO: 7975 | CUUAGCG |
| SEQ ID NO: 7976 | CUUAGCU |
| SEQ ID NO: 7977 | CUUAGGA |
| SEQ ID NO: 7978 | CUUAGGC |
| SEQ ID NO: 7979 | CUUAGGG |
| SEQ ID NO: 7980 | CUUAGGU |
| SEQ ID NO: 7981 | CUUAGUA |
| SEQ ID NO: 7982 | CUUAGUC |
| SEQ ID NO: 7983 | CUUAGUG |
| SEQ ID NO: 7984 | CUUAGUU |
| SEQ ID NO: 7985 | CUUAUAA |
| SEQ ID NO: 7986 | CUUAUAC |
| SEQ ID NO: 7987 | CUUAUAG |
| SEQ ID NO: 7988 | CUUAUAU |
| SEQ ID NO: 7989 | CUUAUCA |
| SEQ ID NO: 7990 | CUUAUCC |
| SEQ ID NO: 7991 | CUUAUCG |
| SEQ ID NO: 7992 | CUUAUCU |
| SEQ ID NO: 7993 | CUUAUGA |
| SEQ ID NO: 7994 | CUUAUGC |
| SEQ ID NO: 7995 | CUUAUGG |
| SEQ ID NO: 7996 | CUUAUGU |
| SEQ ID NO: 7997 | CUUAUUA |
| SEQ ID NO: 7998 | CUUAUUC |
| SEQ ID NO: 7999 | CUUAUUG |
| SEQ ID NO: 8000 | CUUAUUU |
| SEQ ID NO: 8001 | CUUCAAA |
| SEQ ID NO: 8002 | CUUCAAC |
| SEQ ID NO: 8003 | CUUCAAG |
| SEQ ID NO: 8004 | CUUCAAU |
| SEQ ID NO: 8005 | CUUCACA |
| SEQ ID NO: 8006 | CUUCACC |
| SEQ ID NO: 8007 | CUUCACG |

| | |
|---|---|
| SEQ ID NO: 8008 | CUUCACU |
| SEQ ID NO: 8009 | CUUCAGA |
| SEQ ID NO: 8010 | CUUCAGC |
| SEQ ID NO: 8011 | CUUCAGG |
| SEQ ID NO: 8012 | CUUCAGU |
| SEQ ID NO: 8013 | CUUCAUA |
| SEQ ID NO: 8014 | CUUCAUC |
| SEQ ID NO: 8015 | CUUCAUG |
| SEQ ID NO: 8016 | CUUCAUU |
| SEQ ID NO: 8017 | CUUCCAA |
| SEQ ID NO: 8018 | CUUCCAC |
| SEQ ID NO: 8019 | CUUCCAG |
| SEQ ID NO: 8020 | CUUCCAU |
| SEQ ID NO: 8021 | CUUCCCA |
| SEQ ID NO: 8022 | CUUCCCC |
| SEQ ID NO: 8023 | CUUCCCG |
| SEQ ID NO: 8024 | CUUCCCU |
| SEQ ID NO: 8025 | CUUCCGA |
| SEQ ID NO: 8026 | CUUCCGC |
| SEQ ID NO: 8027 | CUUCCGG |
| SEQ ID NO: 8028 | CUUCCGU |
| SEQ ID NO: 8029 | CUUCCUA |
| SEQ ID NO: 8030 | CUUCCUC |
| SEQ ID NO: 8031 | CUUCCUG |
| SEQ ID NO: 8032 | CUUCCUU |
| SEQ ID NO: 8033 | CUUCGAA |
| SEQ ID NO: 8034 | CUUCGAC |
| SEQ ID NO: 8035 | CUUCGAG |
| SEQ ID NO: 8036 | CUUCGAU |
| SEQ ID NO: 8037 | CUUCGCA |
| SEQ ID NO: 8038 | CUUCGCC |
| SEQ ID NO: 8039 | CUUCGCG |
| SEQ ID NO: 8040 | CUUCGCU |
| SEQ ID NO: 8041 | CUUCGGA |
| SEQ ID NO: 8042 | CUUCGGC |
| SEQ ID NO: 8043 | CUUCGGG |
| SEQ ID NO: 8044 | CUUCGGU |
| SEQ ID NO: 8045 | CUUCGUA |
| SEQ ID NO: 8046 | CUUCGUC |
| SEQ ID NO: 8047 | CUUCGUG |
| SEQ ID NO: 8048 | CUUCGUU |
| SEQ ID NO: 8049 | CUUCUAA |
| SEQ ID NO: 8050 | CUUCUAC |
| SEQ ID NO: 8051 | CUUCUAG |
| SEQ ID NO: 8052 | CUUCUAU |
| SEQ ID NO: 8053 | CUUCUCA |
| SEQ ID NO: 8054 | CUUCUCC |
| SEQ ID NO: 8055 | CUUCUCG |
| SEQ ID NO: 8056 | CUUCUCU |
| SEQ ID NO: 8057 | CUUCUGA |
| SEQ ID NO: 8058 | CUUCUGC |

| | |
|---|---|
| SEQ ID NO: 8059 | CUUCUGG |
| SEQ ID NO: 8060 | CUUCUGU |
| SEQ ID NO: 8061 | CUUCUUA |
| SEQ ID NO: 8062 | CUUCUUC |
| SEQ ID NO: 8063 | CUUCUUG |
| SEQ ID NO: 8064 | CUUCUUU |
| SEQ ID NO: 8065 | CUUGAAA |
| SEQ ID NO: 8066 | CUUGAAC |
| SEQ ID NO: 8067 | CUUGAAG |
| SEQ ID NO: 8068 | CUUGAAU |
| SEQ ID NO: 8069 | CUUGACA |
| SEQ ID NO: 8070 | CUUGACC |
| SEQ ID NO: 8071 | CUUGACG |
| SEQ ID NO: 8072 | CUUGACU |
| SEQ ID NO: 8073 | CUUGAGA |
| SEQ ID NO: 8074 | CUUGAGC |
| SEQ ID NO: 8075 | CUUGAGG |
| SEQ ID NO: 8076 | CUUGAGU |
| SEQ ID NO: 8077 | CUUGAUA |
| SEQ ID NO: 8078 | CUUGAUC |
| SEQ ID NO: 8079 | CUUGAUG |
| SEQ ID NO: 8080 | CUUGAUU |
| SEQ ID NO: 8081 | CUUGCAA |
| SEQ ID NO: 8082 | CUUGCAC |
| SEQ ID NO: 8083 | CUUGCAG |
| SEQ ID NO: 8084 | CUUGCAU |
| SEQ ID NO: 8085 | CUUGCCA |
| SEQ ID NO: 8086 | CUUGCCC |
| SEQ ID NO: 8087 | CUUGCCG |
| SEQ ID NO: 8088 | CUUGCCU |
| SEQ ID NO: 8089 | CUUGCGA |
| SEQ ID NO: 8090 | CUUGCGC |
| SEQ ID NO: 8091 | CUUGCGG |
| SEQ ID NO: 8092 | CUUGCGU |
| SEQ ID NO: 8093 | CUUGCUA |
| SEQ ID NO: 8094 | CUUGCUC |
| SEQ ID NO: 8095 | CUUGCUG |
| SEQ ID NO: 8096 | CUUGCUU |
| SEQ ID NO: 8097 | CUUGGAA |
| SEQ ID NO: 8098 | CUUGGAC |
| SEQ ID NO: 8099 | CUUGGAG |
| SEQ ID NO: 8100 | CUUGGAU |
| SEQ ID NO: 8101 | CUUGGCA |
| SEQ ID NO: 8102 | CUUGGCC |
| SEQ ID NO: 8103 | CUUGGCG |
| SEQ ID NO: 8104 | CUUGGCU |
| SEQ ID NO: 8105 | CUUGGGA |
| SEQ ID NO: 8106 | CUUGGGC |
| SEQ ID NO: 8107 | CUUGGGG |
| SEQ ID NO: 8108 | CUUGGGU |
| SEQ ID NO: 8109 | CUUGGUA |

| | |
|---|---|
| SEQ ID NO: 8110 | CUUGGUC |
| SEQ ID NO: 8111 | CUUGGUG |
| SEQ ID NO: 8112 | CUUGGUU |
| SEQ ID NO: 8113 | CUUGUAA |
| SEQ ID NO: 8114 | CUUGUAC |
| SEQ ID NO: 8115 | CUUGUAG |
| SEQ ID NO: 8116 | CUUGUAU |
| SEQ ID NO: 8117 | CUUGUCA |
| SEQ ID NO: 8118 | CUUGUCC |
| SEQ ID NO: 8119 | CUUGUCG |
| SEQ ID NO: 8120 | CUUGUCU |
| SEQ ID NO: 8121 | CUUGUGA |
| SEQ ID NO: 8122 | CUUGUGC |
| SEQ ID NO: 8123 | CUUGUGG |
| SEQ ID NO: 8124 | CUUGUGU |
| SEQ ID NO: 8125 | CUUGUUA |
| SEQ ID NO: 8126 | CUUGUUC |
| SEQ ID NO: 8127 | CUUGUUG |
| SEQ ID NO: 8128 | CUUGUUU |
| SEQ ID NO: 8129 | CUUUAAA |
| SEQ ID NO: 8130 | CUUUAAC |
| SEQ ID NO: 8131 | CUUUAAG |
| SEQ ID NO: 8132 | CUUUAAU |
| SEQ ID NO: 8133 | CUUUACA |
| SEQ ID NO: 8134 | CUUUACC |
| SEQ ID NO: 8135 | CUUUACG |
| SEQ ID NO: 8136 | CUUUACU |
| SEQ ID NO: 8137 | CUUUAGA |
| SEQ ID NO: 8138 | CUUUAGC |
| SEQ ID NO: 8139 | CUUUAGG |
| SEQ ID NO: 8140 | CUUUAGU |
| SEQ ID NO: 8141 | CUUUAUA |
| SEQ ID NO: 8142 | CUUUAUC |
| SEQ ID NO: 8143 | CUUUAUG |
| SEQ ID NO: 8144 | CUUUAUU |
| SEQ ID NO: 8145 | CUUUCAA |
| SEQ ID NO: 8146 | CUUUCAC |
| SEQ ID NO: 8147 | CUUUCAG |
| SEQ ID NO: 8148 | CUUUCAU |
| SEQ ID NO: 8149 | CUUUCCA |
| SEQ ID NO: 8150 | CUUUCCC |
| SEQ ID NO: 8151 | CUUUCCG |
| SEQ ID NO: 8152 | CUUUCCU |
| SEQ ID NO: 8153 | CUUUCGA |
| SEQ ID NO: 8154 | CUUUCGC |
| SEQ ID NO: 8155 | CUUUCGG |
| SEQ ID NO: 8156 | CUUUCGU |
| SEQ ID NO: 8157 | CUUUCUA |
| SEQ ID NO: 8158 | CUUUCUC |
| SEQ ID NO: 8159 | CUUUCUG |
| SEQ ID NO: 8160 | CUUUCUU |

| | |
|---|---|
| SEQ ID NO: 8161 | CUUUGAA |
| SEQ ID NO: 8162 | CUUUGAC |
| SEQ ID NO: 8163 | CUUUGAG |
| SEQ ID NO: 8164 | CUUUGAU |
| SEQ ID NO: 8165 | CUUUGCA |
| SEQ ID NO: 8166 | CUUUGCC |
| SEQ ID NO: 8167 | CUUUGCG |
| SEQ ID NO: 8168 | CUUUGCU |
| SEQ ID NO: 8169 | CUUUGGA |
| SEQ ID NO: 8170 | CUUUGGC |
| SEQ ID NO: 8171 | CUUUGGG |
| SEQ ID NO: 8172 | CUUUGGU |
| SEQ ID NO: 8173 | CUUUGUA |
| SEQ ID NO: 8174 | CUUUGUC |
| SEQ ID NO: 8175 | CUUUGUG |
| SEQ ID NO: 8176 | CUUUGUU |
| SEQ ID NO: 8177 | CUUUUAA |
| SEQ ID NO: 8178 | CUUUUAC |
| SEQ ID NO: 8179 | CUUUUAG |
| SEQ ID NO: 8180 | CUUUUAU |
| SEQ ID NO: 8181 | CUUUUCA |
| SEQ ID NO: 8182 | CUUUUCC |
| SEQ ID NO: 8183 | CUUUUCG |
| SEQ ID NO: 8184 | CUUUUCU |
| SEQ ID NO: 8185 | CUUUUGA |
| SEQ ID NO: 8186 | CUUUUGC |
| SEQ ID NO: 8187 | CUUUUGG |
| SEQ ID NO: 8188 | CUUUUGU |
| SEQ ID NO: 8189 | CUUUUUA |
| SEQ ID NO: 8190 | CUUUUUC |
| SEQ ID NO: 8191 | CUUUUUG |
| SEQ ID NO: 8192 | CUUUUUU |
| SEQ ID NO: 8193 | GAAAAAA |
| SEQ ID NO: 8194 | GAAAAAC |
| SEQ ID NO: 8195 | GAAAAAG |
| SEQ ID NO: 8196 | GAAAAAU |
| SEQ ID NO: 8197 | GAAAACA |
| SEQ ID NO: 8198 | GAAAACC |
| SEQ ID NO: 8199 | GAAAACG |
| SEQ ID NO: 8200 | GAAAACU |
| SEQ ID NO: 8201 | GAAAAGA |
| SEQ ID NO: 8202 | GAAAAGC |
| SEQ ID NO: 8203 | GAAAAGG |
| SEQ ID NO: 8204 | GAAAAGU |
| SEQ ID NO: 8205 | GAAAAUA |
| SEQ ID NO: 8206 | GAAAAUC |
| SEQ ID NO: 8207 | GAAAAUG |
| SEQ ID NO: 8208 | GAAAAUU |
| SEQ ID NO: 8209 | GAAACAA |
| SEQ ID NO: 8210 | GAAACAC |
| SEQ ID NO: 8211 | GAAACAG |

| | |
|---|---|
| SEQ ID NO: 8212 | GAAACAU |
| SEQ ID NO: 8213 | GAAACCA |
| SEQ ID NO: 8214 | GAAACCC |
| SEQ ID NO: 8215 | GAAACCG |
| SEQ ID NO: 8216 | GAAACCU |
| SEQ ID NO: 8217 | GAAACGA |
| SEQ ID NO: 8218 | GAAACGC |
| SEQ ID NO: 8219 | GAAACGG |
| SEQ ID NO: 8220 | GAAACGU |
| SEQ ID NO: 8221 | GAAACUA |
| SEQ ID NO: 8222 | GAAACUC |
| SEQ ID NO: 8223 | GAAACUG |
| SEQ ID NO: 8224 | GAAACUU |
| SEQ ID NO: 8225 | GAAAGAA |
| SEQ ID NO: 8226 | GAAAGAC |
| SEQ ID NO: 8227 | GAAAGAG |
| SEQ ID NO: 8228 | GAAAGAU |
| SEQ ID NO: 8229 | GAAAGCA |
| SEQ ID NO: 8230 | GAAAGCC |
| SEQ ID NO: 8231 | GAAAGCG |
| SEQ ID NO: 8232 | GAAAGCU |
| SEQ ID NO: 8233 | GAAAGGA |
| SEQ ID NO: 8234 | GAAAGGC |
| SEQ ID NO: 8235 | GAAAGGG |
| SEQ ID NO: 8236 | GAAAGGU |
| SEQ ID NO: 8237 | GAAAGUA |
| SEQ ID NO: 8238 | GAAAGUC |
| SEQ ID NO: 8239 | GAAAGUG |
| SEQ ID NO: 8240 | GAAAGUU |
| SEQ ID NO: 8241 | GAAAUAA |
| SEQ ID NO: 8242 | GAAAUAC |
| SEQ ID NO: 8243 | GAAAUAG |
| SEQ ID NO: 8244 | GAAAUAU |
| SEQ ID NO: 8245 | GAAAUCA |
| SEQ ID NO: 8246 | GAAAUCC |
| SEQ ID NO: 8247 | GAAAUCG |
| SEQ ID NO: 8248 | GAAAUCU |
| SEQ ID NO: 8249 | GAAAUGA |
| SEQ ID NO: 8250 | GAAAUGC |
| SEQ ID NO: 8251 | GAAAUGG |
| SEQ ID NO: 8252 | GAAAUGU |
| SEQ ID NO: 8253 | GAAAUUA |
| SEQ ID NO: 8254 | GAAAUUC |
| SEQ ID NO: 8255 | GAAAUUG |
| SEQ ID NO: 8256 | GAAAUUU |
| SEQ ID NO: 8257 | GAACAAA |
| SEQ ID NO: 8258 | GAACAAC |
| SEQ ID NO: 8259 | GAACAAG |
| SEQ ID NO: 8260 | GAACAAU |
| SEQ ID NO: 8261 | GAACACA |
| SEQ ID NO: 8262 | GAACACC |

Table 28

| | |
|---|---|
| SEQ ID NO: 8263 | GAACACG |
| SEQ ID NO: 8264 | GAACACU |
| SEQ ID NO: 8265 | GAACAGA |
| SEQ ID NO: 8266 | GAACAGC |
| SEQ ID NO: 8267 | GAACAGG |
| SEQ ID NO: 8268 | GAACAGU |
| SEQ ID NO: 8269 | GAACAUA |
| SEQ ID NO: 8270 | GAACAUC |
| SEQ ID NO: 8271 | GAACAUG |
| SEQ ID NO: 8272 | GAACAUU |
| SEQ ID NO: 8273 | GAACCAA |
| SEQ ID NO: 8274 | GAACCAC |
| SEQ ID NO: 8275 | GAACCAG |
| SEQ ID NO: 8276 | GAACCAU |
| SEQ ID NO: 8277 | GAACCCA |
| SEQ ID NO: 8278 | GAACCCC |
| SEQ ID NO: 8279 | GAACCCG |
| SEQ ID NO: 8280 | GAACCCU |
| SEQ ID NO: 8281 | GAACCGA |
| SEQ ID NO: 8282 | GAACCGC |
| SEQ ID NO: 8283 | GAACCGG |
| SEQ ID NO: 8284 | GAACCGU |
| SEQ ID NO: 8285 | GAACCUA |
| SEQ ID NO: 8286 | GAACCUC |
| SEQ ID NO: 8287 | GAACCUG |
| SEQ ID NO: 8288 | GAACCUU |
| SEQ ID NO: 8289 | GAACGAA |
| SEQ ID NO: 8290 | GAACGAC |
| SEQ ID NO: 8291 | GAACGAG |
| SEQ ID NO: 8292 | GAACGAU |
| SEQ ID NO: 8293 | GAACGCA |
| SEQ ID NO: 8294 | GAACGCC |
| SEQ ID NO: 8295 | GAACGCG |
| SEQ ID NO: 8296 | GAACGCU |
| SEQ ID NO: 8297 | GAACGGA |
| SEQ ID NO: 8298 | GAACGGC |
| SEQ ID NO: 8299 | GAACGGG |
| SEQ ID NO: 8300 | GAACGGU |
| SEQ ID NO: 8301 | GAACGUA |
| SEQ ID NO: 8302 | GAACGUC |
| SEQ ID NO: 8303 | GAACGUG |
| SEQ ID NO: 8304 | GAACGUU |
| SEQ ID NO: 8305 | GAACUAA |
| SEQ ID NO: 8306 | GAACUAC |
| SEQ ID NO: 8307 | GAACUAG |
| SEQ ID NO: 8308 | GAACUAU |
| SEQ ID NO: 8309 | GAACUCA |
| SEQ ID NO: 8310 | GAACUCC |
| SEQ ID NO: 8311 | GAACUCG |
| SEQ ID NO: 8312 | GAACUCU |
| SEQ ID NO: 8313 | GAACUGA |
| SEQ ID NO: 8314 | GAACUGC |
| SEQ ID NO: 8315 | GAACUGG |
| SEQ ID NO: 8316 | GAACUGU |
| SEQ ID NO: 8317 | GAACUUA |
| SEQ ID NO: 8318 | GAACUUC |
| SEQ ID NO: 8319 | GAACUUG |
| SEQ ID NO: 8320 | GAACUUU |
| SEQ ID NO: 8321 | GAAGAAA |
| SEQ ID NO: 8322 | GAAGAAC |
| SEQ ID NO: 8323 | GAAGAAG |
| SEQ ID NO: 8324 | GAAGAAU |
| SEQ ID NO: 8325 | GAAGACA |
| SEQ ID NO: 8326 | GAAGACC |
| SEQ ID NO: 8327 | GAAGACG |
| SEQ ID NO: 8328 | GAAGACU |
| SEQ ID NO: 8329 | GAAGAGA |
| SEQ ID NO: 8330 | GAAGAGC |
| SEQ ID NO: 8331 | GAAGAGG |
| SEQ ID NO: 8332 | GAAGAGU |
| SEQ ID NO: 8333 | GAAGAUA |
| SEQ ID NO: 8334 | GAAGAUC |
| SEQ ID NO: 8335 | GAAGAUG |
| SEQ ID NO: 8336 | GAAGAUU |
| SEQ ID NO: 8337 | GAAGCAA |
| SEQ ID NO: 8338 | GAAGCAC |
| SEQ ID NO: 8339 | GAAGCAG |
| SEQ ID NO: 8340 | GAAGCAU |
| SEQ ID NO: 8341 | GAAGCCA |
| SEQ ID NO: 8342 | GAAGCCC |
| SEQ ID NO: 8343 | GAAGCCG |
| SEQ ID NO: 8344 | GAAGCCU |
| SEQ ID NO: 8345 | GAAGCGA |
| SEQ ID NO: 8346 | GAAGCGC |
| SEQ ID NO: 8347 | GAAGCGG |
| SEQ ID NO: 8348 | GAAGCGU |
| SEQ ID NO: 8349 | GAAGCUA |
| SEQ ID NO: 8350 | GAAGCUC |
| SEQ ID NO: 8351 | GAAGCUG |
| SEQ ID NO: 8352 | GAAGCUU |
| SEQ ID NO: 8353 | GAAGGAA |
| SEQ ID NO: 8354 | GAAGGAC |
| SEQ ID NO: 8355 | GAAGGAG |
| SEQ ID NO: 8356 | GAAGGAU |
| SEQ ID NO: 8357 | GAAGGCA |
| SEQ ID NO: 8358 | GAAGGCC |
| SEQ ID NO: 8359 | GAAGGCG |
| SEQ ID NO: 8360 | GAAGGCU |
| SEQ ID NO: 8361 | GAAGGGA |
| SEQ ID NO: 8362 | GAAGGGC |
| SEQ ID NO: 8363 | GAAGGGG |
| SEQ ID NO: 8364 | GAAGGGU |
| SEQ ID NO: 8365 | GAAGGUA |
| SEQ ID NO: 8366 | GAAGGUC |
| SEQ ID NO: 8367 | GAAGGUG |
| SEQ ID NO: 8368 | GAAGGUU |
| SEQ ID NO: 8369 | GAAGUAA |
| SEQ ID NO: 8370 | GAAGUAC |
| SEQ ID NO: 8371 | GAAGUAG |
| SEQ ID NO: 8372 | GAAGUAU |
| SEQ ID NO: 8373 | GAAGUCA |
| SEQ ID NO: 8374 | GAAGUCC |
| SEQ ID NO: 8375 | GAAGUCG |
| SEQ ID NO: 8376 | GAAGUCU |
| SEQ ID NO: 8377 | GAAGUGA |
| SEQ ID NO: 8378 | GAAGUGC |
| SEQ ID NO: 8379 | GAAGUGG |
| SEQ ID NO: 8380 | GAAGUGU |
| SEQ ID NO: 8381 | GAAGUUA |
| SEQ ID NO: 8382 | GAAGUUC |
| SEQ ID NO: 8383 | GAAGUUG |
| SEQ ID NO: 8384 | GAAGUUU |
| SEQ ID NO: 8385 | GAAUAAA |
| SEQ ID NO: 8386 | GAAUAAC |
| SEQ ID NO: 8387 | GAAUAAG |
| SEQ ID NO: 8388 | GAAUAAU |
| SEQ ID NO: 8389 | GAAUACA |
| SEQ ID NO: 8390 | GAAUACC |
| SEQ ID NO: 8391 | GAAUACG |
| SEQ ID NO: 8392 | GAAUACU |
| SEQ ID NO: 8393 | GAAUAGA |
| SEQ ID NO: 8394 | GAAUAGC |
| SEQ ID NO: 8395 | GAAUAGG |
| SEQ ID NO: 8396 | GAAUAGU |
| SEQ ID NO: 8397 | GAAUAUA |
| SEQ ID NO: 8398 | GAAUAUC |
| SEQ ID NO: 8399 | GAAUAUG |
| SEQ ID NO: 8400 | GAAUAUU |
| SEQ ID NO: 8401 | GAAUCAA |
| SEQ ID NO: 8402 | GAAUCAC |
| SEQ ID NO: 8403 | GAAUCAG |
| SEQ ID NO: 8404 | GAAUCAU |
| SEQ ID NO: 8405 | GAAUCCA |
| SEQ ID NO: 8406 | GAAUCCC |
| SEQ ID NO: 8407 | GAAUCCG |
| SEQ ID NO: 8408 | GAAUCCU |
| SEQ ID NO: 8409 | GAAUCGA |
| SEQ ID NO: 8410 | GAAUCGC |
| SEQ ID NO: 8411 | GAAUCGG |
| SEQ ID NO: 8412 | GAAUCGU |
| SEQ ID NO: 8413 | GAAUCUA |
| SEQ ID NO: 8414 | GAAUCUC |
| SEQ ID NO: 8415 | GAAUCUG |
| SEQ ID NO: 8416 | GAAUCUU |
| SEQ ID NO: 8417 | GAAUGAA |
| SEQ ID NO: 8418 | GAAUGAC |
| SEQ ID NO: 8419 | GAAUGAG |
| SEQ ID NO: 8420 | GAAUGAU |
| SEQ ID NO: 8421 | GAAUGCA |
| SEQ ID NO: 8422 | GAAUGCC |
| SEQ ID NO: 8423 | GAAUGCG |
| SEQ ID NO: 8424 | GAAUGCU |
| SEQ ID NO: 8425 | GAAUGGA |
| SEQ ID NO: 8426 | GAAUGGC |
| SEQ ID NO: 8427 | GAAUGGG |
| SEQ ID NO: 8428 | GAAUGGU |
| SEQ ID NO: 8429 | GAAUGUA |
| SEQ ID NO: 8430 | GAAUGUC |
| SEQ ID NO: 8431 | GAAUGUG |
| SEQ ID NO: 8432 | GAAUGUU |
| SEQ ID NO: 8433 | GAAUUAA |
| SEQ ID NO: 8434 | GAAUUAC |
| SEQ ID NO: 8435 | GAAUUAG |
| SEQ ID NO: 8436 | GAAUUAU |
| SEQ ID NO: 8437 | GAAUUCA |
| SEQ ID NO: 8438 | GAAUUCC |
| SEQ ID NO: 8439 | GAAUUCG |
| SEQ ID NO: 8440 | GAAUUCU |
| SEQ ID NO: 8441 | GAAUUGA |
| SEQ ID NO: 8442 | GAAUUGC |
| SEQ ID NO: 8443 | GAAUUGG |
| SEQ ID NO: 8444 | GAAUUGU |
| SEQ ID NO: 8445 | GAAUUUA |
| SEQ ID NO: 8446 | GAAUUUC |
| SEQ ID NO: 8447 | GAAUUUG |
| SEQ ID NO: 8448 | GAAUUUU |
| SEQ ID NO: 8449 | GACAAAA |
| SEQ ID NO: 8450 | GACAAAC |
| SEQ ID NO: 8451 | GACAAAG |
| SEQ ID NO: 8452 | GACAAAU |
| SEQ ID NO: 8453 | GACAACA |
| SEQ ID NO: 8454 | GACAACC |
| SEQ ID NO: 8455 | GACAACG |
| SEQ ID NO: 8456 | GACAACU |
| SEQ ID NO: 8457 | GACAAGA |
| SEQ ID NO: 8458 | GACAAGC |
| SEQ ID NO: 8459 | GACAAGG |
| SEQ ID NO: 8460 | GACAAGU |
| SEQ ID NO: 8461 | GACAAUA |
| SEQ ID NO: 8462 | GACAAUC |
| SEQ ID NO: 8463 | GACAAUG |
| SEQ ID NO: 8464 | GACAAUU |
| SEQ ID NO: 8465 | GACACAA |
| SEQ ID NO: 8466 | GACACAC |
| SEQ ID NO: 8467 | GACACAG |
| SEQ ID NO: 8468 | GACACAU |
| SEQ ID NO: 8469 | GACACCA |
| SEQ ID NO: 8470 | GACACCC |
| SEQ ID NO: 8471 | GACACCG |
| SEQ ID NO: 8472 | GACACCU |
| SEQ ID NO: 8473 | GACACGA |
| SEQ ID NO: 8474 | GACACGC |
| SEQ ID NO: 8475 | GACACGG |
| SEQ ID NO: 8476 | GACACGU |
| SEQ ID NO: 8477 | GACACUA |
| SEQ ID NO: 8478 | GACACUC |
| SEQ ID NO: 8479 | GACACUG |
| SEQ ID NO: 8480 | GACACUU |
| SEQ ID NO: 8481 | GACAGAA |
| SEQ ID NO: 8482 | GACAGAC |
| SEQ ID NO: 8483 | GACAGAG |
| SEQ ID NO: 8484 | GACAGAU |
| SEQ ID NO: 8485 | GACAGCA |
| SEQ ID NO: 8486 | GACAGCC |
| SEQ ID NO: 8487 | GACAGCG |
| SEQ ID NO: 8488 | GACAGCU |
| SEQ ID NO: 8489 | GACAGGA |
| SEQ ID NO: 8490 | GACAGGC |
| SEQ ID NO: 8491 | GACAGGG |
| SEQ ID NO: 8492 | GACAGGU |
| SEQ ID NO: 8493 | GACAGUA |
| SEQ ID NO: 8494 | GACAGUC |
| SEQ ID NO: 8495 | GACAGUG |
| SEQ ID NO: 8496 | GACAGUU |
| SEQ ID NO: 8497 | GACAUAA |
| SEQ ID NO: 8498 | GACAUAC |
| SEQ ID NO: 8499 | GACAUAG |
| SEQ ID NO: 8500 | GACAUAU |
| SEQ ID NO: 8501 | GACAUCA |
| SEQ ID NO: 8502 | GACAUCC |
| SEQ ID NO: 8503 | GACAUCG |
| SEQ ID NO: 8504 | GACAUCU |
| SEQ ID NO: 8505 | GACAUGA |
| SEQ ID NO: 8506 | GACAUGC |
| SEQ ID NO: 8507 | GACAUGG |
| SEQ ID NO: 8508 | GACAUGU |
| SEQ ID NO: 8509 | GACAUUA |
| SEQ ID NO: 8510 | GACAUUC |
| SEQ ID NO: 8511 | GACAUUG |
| SEQ ID NO: 8512 | GACAUUU |
| SEQ ID NO: 8513 | GACCAAA |
| SEQ ID NO: 8514 | GACCAAC |
| SEQ ID NO: 8515 | GACCAAG |
| SEQ ID NO: 8516 | GACCAAU |
| SEQ ID NO: 8517 | GACCACA |
| SEQ ID NO: 8518 | GACCACC |
| SEQ ID NO: 8519 | GACCACG |
| SEQ ID NO: 8520 | GACCACU |
| SEQ ID NO: 8521 | GACCAGA |
| SEQ ID NO: 8522 | GACCAGC |
| SEQ ID NO: 8523 | GACCAGG |
| SEQ ID NO: 8524 | GACCAGU |
| SEQ ID NO: 8525 | GACCAUA |
| SEQ ID NO: 8526 | GACCAUC |
| SEQ ID NO: 8527 | GACCAUG |
| SEQ ID NO: 8528 | GACCAUU |
| SEQ ID NO: 8529 | GACCCAA |
| SEQ ID NO: 8530 | GACCCAC |
| SEQ ID NO: 8531 | GACCCAG |
| SEQ ID NO: 8532 | GACCCAU |
| SEQ ID NO: 8533 | GACCCCA |
| SEQ ID NO: 8534 | GACCCCC |
| SEQ ID NO: 8535 | GACCCCG |
| SEQ ID NO: 8536 | GACCCCU |
| SEQ ID NO: 8537 | GACCCGA |
| SEQ ID NO: 8538 | GACCCGC |
| SEQ ID NO: 8539 | GACCCGG |
| SEQ ID NO: 8540 | GACCCGU |
| SEQ ID NO: 8541 | GACCCUA |
| SEQ ID NO: 8542 | GACCCUC |
| SEQ ID NO: 8543 | GACCCUG |
| SEQ ID NO: 8544 | GACCCUU |
| SEQ ID NO: 8545 | GACCGAA |
| SEQ ID NO: 8546 | GACCGAC |
| SEQ ID NO: 8547 | GACCGAG |
| SEQ ID NO: 8548 | GACCGAU |
| SEQ ID NO: 8549 | GACCGCA |
| SEQ ID NO: 8550 | GACCGCC |
| SEQ ID NO: 8551 | GACCGCG |
| SEQ ID NO: 8552 | GACCGCU |
| SEQ ID NO: 8553 | GACCGGA |
| SEQ ID NO: 8554 | GACCGGC |
| SEQ ID NO: 8555 | GACCGGG |
| SEQ ID NO: 8556 | GACCGGU |
| SEQ ID NO: 8557 | GACCGUA |
| SEQ ID NO: 8558 | GACCGUC |
| SEQ ID NO: 8559 | GACCGUG |
| SEQ ID NO: 8560 | GACCGUU |
| SEQ ID NO: 8561 | GACCUAA |
| SEQ ID NO: 8562 | GACCUAC |
| SEQ ID NO: 8563 | GACCUAG |
| SEQ ID NO: 8564 | GACCUAU |
| SEQ ID NO: 8565 | GACCUCA |
| SEQ ID NO: 8566 | GACCUCC |
| SEQ ID NO: 8567 | GACCUCG |
| SEQ ID NO: 8568 | GACCUCU |

Table 29

| | | | | | | | | | | | |
|---|---|---|---|---|---|---|---|---|---|---|---|
| SEQ ID NO: 8569 | GACCUGA | SEQ ID NO: 8620 | GACGGGU | SEQ ID NO: 8671 | GACUCUG | SEQ ID NO: 8722 | GAGACAC | SEQ ID NO: 8773 | GAGCACA | SEQ ID NO: 8824 | GAGCUCU |
| SEQ ID NO: 8570 | GACCUGC | SEQ ID NO: 8621 | GACGGUA | SEQ ID NO: 8672 | GACUCUU | SEQ ID NO: 8723 | GAGACAG | SEQ ID NO: 8774 | GAGCACC | SEQ ID NO: 8825 | GAGCUGA |
| SEQ ID NO: 8571 | GACCUGG | SEQ ID NO: 8622 | GACGGUC | SEQ ID NO: 8673 | GACUGAA | SEQ ID NO: 8724 | GAGACAU | SEQ ID NO: 8775 | GAGCACG | SEQ ID NO: 8826 | GAGCUGC |
| SEQ ID NO: 8572 | GACCUGU | SEQ ID NO: 8623 | GACGGUG | SEQ ID NO: 8674 | GACUGAC | SEQ ID NO: 8725 | GAGACCA | SEQ ID NO: 8776 | GAGCACU | SEQ ID NO: 8827 | GAGCUGG |
| SEQ ID NO: 8573 | GACCUUA | SEQ ID NO: 8624 | GACGGUU | SEQ ID NO: 8675 | GACUGAG | SEQ ID NO: 8726 | GAGACCC | SEQ ID NO: 8777 | GAGCAGA | SEQ ID NO: 8828 | GAGCUGU |
| SEQ ID NO: 8574 | GACCUUC | SEQ ID NO: 8625 | GACGUAA | SEQ ID NO: 8676 | GACUGAU | SEQ ID NO: 8727 | GAGACCG | SEQ ID NO: 8778 | GAGCAGC | SEQ ID NO: 8829 | GAGCUUA |
| SEQ ID NO: 8575 | GACCUUG | SEQ ID NO: 8626 | GACGUAC | SEQ ID NO: 8677 | GACUGCA | SEQ ID NO: 8728 | GAGACCU | SEQ ID NO: 8779 | GAGCAGG | SEQ ID NO: 8830 | GAGCUUC |
| SEQ ID NO: 8576 | GACCUUU | SEQ ID NO: 8627 | GACGUAG | SEQ ID NO: 8678 | GACUGCC | SEQ ID NO: 8729 | GAGACGA | SEQ ID NO: 8780 | GAGCAGU | SEQ ID NO: 8831 | GAGCUUG |
| SEQ ID NO: 8577 | GACGAAA | SEQ ID NO: 8628 | GACGUAU | SEQ ID NO: 8679 | GACUGCG | SEQ ID NO: 8730 | GAGACGC | SEQ ID NO: 8781 | GAGCAUA | SEQ ID NO: 8832 | GAGCUUU |
| SEQ ID NO: 8578 | GACGAAC | SEQ ID NO: 8629 | GACGUCA | SEQ ID NO: 8680 | GACUGCU | SEQ ID NO: 8731 | GAGACGG | SEQ ID NO: 8782 | GAGCAUC | SEQ ID NO: 8833 | GAGGAAA |
| SEQ ID NO: 8579 | GACGAAG | SEQ ID NO: 8630 | GACGUCC | SEQ ID NO: 8681 | GACUGGA | SEQ ID NO: 8732 | GAGACGU | SEQ ID NO: 8783 | GAGCAUG | SEQ ID NO: 8834 | GAGGAAC |
| SEQ ID NO: 8580 | GACGAAU | SEQ ID NO: 8631 | GACGUCG | SEQ ID NO: 8682 | GACUGGC | SEQ ID NO: 8733 | GAGACUA | SEQ ID NO: 8784 | GAGCAUU | SEQ ID NO: 8835 | GAGGAAG |
| SEQ ID NO: 8581 | GACGACA | SEQ ID NO: 8632 | GACGUCU | SEQ ID NO: 8683 | GACUGGG | SEQ ID NO: 8734 | GAGACUC | SEQ ID NO: 8785 | GAGCCAA | SEQ ID NO: 8836 | GAGGAAU |
| SEQ ID NO: 8582 | GACGACC | SEQ ID NO: 8633 | GACGUGA | SEQ ID NO: 8684 | GACUGGU | SEQ ID NO: 8735 | GAGACUG | SEQ ID NO: 8786 | GAGCCAC | SEQ ID NO: 8837 | GAGGACA |
| SEQ ID NO: 8583 | GACGACG | SEQ ID NO: 8634 | GACGUGC | SEQ ID NO: 8685 | GACUGUA | SEQ ID NO: 8736 | GAGACUU | SEQ ID NO: 8787 | GAGCCAG | SEQ ID NO: 8838 | GAGGACC |
| SEQ ID NO: 8584 | GACGACU | SEQ ID NO: 8635 | GACGUGG | SEQ ID NO: 8686 | GACUGUC | SEQ ID NO: 8737 | GAGAGAA | SEQ ID NO: 8788 | GAGCCAU | SEQ ID NO: 8839 | GAGGACG |
| SEQ ID NO: 8585 | GACGAGA | SEQ ID NO: 8636 | GACGUGU | SEQ ID NO: 8687 | GACUGUG | SEQ ID NO: 8738 | GAGAGAC | SEQ ID NO: 8789 | GAGCCCA | SEQ ID NO: 8840 | GAGGACU |
| SEQ ID NO: 8586 | GACGAGC | SEQ ID NO: 8637 | GACGUUA | SEQ ID NO: 8688 | GACUGUU | SEQ ID NO: 8739 | GAGAGAG | SEQ ID NO: 8790 | GAGCCCC | SEQ ID NO: 8841 | GAGGAGA |
| SEQ ID NO: 8587 | GACGAGG | SEQ ID NO: 8638 | GACGUUC | SEQ ID NO: 8689 | GACUUAA | SEQ ID NO: 8740 | GAGAGAU | SEQ ID NO: 8791 | GAGCCCG | SEQ ID NO: 8842 | GAGGAGC |
| SEQ ID NO: 8588 | GACGAGU | SEQ ID NO: 8639 | GACGUUG | SEQ ID NO: 8690 | GACUUAC | SEQ ID NO: 8741 | GAGAGCA | SEQ ID NO: 8792 | GAGCCCU | SEQ ID NO: 8843 | GAGGAGG |
| SEQ ID NO: 8589 | GACGAUA | SEQ ID NO: 8640 | GACGUUU | SEQ ID NO: 8691 | GACUUAG | SEQ ID NO: 8742 | GAGAGCC | SEQ ID NO: 8793 | GAGCCGA | SEQ ID NO: 8844 | GAGGAGU |
| SEQ ID NO: 8590 | GACGAUC | SEQ ID NO: 8641 | GACUAAA | SEQ ID NO: 8692 | GACUUAU | SEQ ID NO: 8743 | GAGAGCG | SEQ ID NO: 8794 | GAGCCGC | SEQ ID NO: 8845 | GAGGAUA |
| SEQ ID NO: 8591 | GACGAUG | SEQ ID NO: 8642 | GACUAAC | SEQ ID NO: 8693 | GACUUCA | SEQ ID NO: 8744 | GAGAGCU | SEQ ID NO: 8795 | GAGCCGG | SEQ ID NO: 8846 | GAGGAUC |
| SEQ ID NO: 8592 | GACGAUU | SEQ ID NO: 8643 | GACUAAG | SEQ ID NO: 8694 | GACUUCC | SEQ ID NO: 8745 | GAGAGGA | SEQ ID NO: 8796 | GAGCCGU | SEQ ID NO: 8847 | GAGGAUG |
| SEQ ID NO: 8593 | GACGCAA | SEQ ID NO: 8644 | GACUAAU | SEQ ID NO: 8695 | GACUUCG | SEQ ID NO: 8746 | GAGAGGC | SEQ ID NO: 8797 | GAGCCUA | SEQ ID NO: 8848 | GAGGAUU |
| SEQ ID NO: 8594 | GACGCAC | SEQ ID NO: 8645 | GACUACA | SEQ ID NO: 8696 | GACUUCU | SEQ ID NO: 8747 | GAGAGGG | SEQ ID NO: 8798 | GAGCCUC | SEQ ID NO: 8849 | GAGGCAA |
| SEQ ID NO: 8595 | GACGCAG | SEQ ID NO: 8646 | GACUACC | SEQ ID NO: 8697 | GACUUGA | SEQ ID NO: 8748 | GAGAGGU | SEQ ID NO: 8799 | GAGCCUG | SEQ ID NO: 8850 | GAGGCAC |
| SEQ ID NO: 8596 | GACGCAU | SEQ ID NO: 8647 | GACUACG | SEQ ID NO: 8698 | GACUUGC | SEQ ID NO: 8749 | GAGAGUA | SEQ ID NO: 8800 | GAGCCUU | SEQ ID NO: 8851 | GAGGCAG |
| SEQ ID NO: 8597 | GACGCCA | SEQ ID NO: 8648 | GACUACU | SEQ ID NO: 8699 | GACUUGG | SEQ ID NO: 8750 | GAGAGUC | SEQ ID NO: 8801 | GAGCGAA | SEQ ID NO: 8852 | GAGGCAU |
| SEQ ID NO: 8598 | GACGCCC | SEQ ID NO: 8649 | GACUAGA | SEQ ID NO: 8700 | GACUUGU | SEQ ID NO: 8751 | GAGAGUG | SEQ ID NO: 8802 | GAGCGAC | SEQ ID NO: 8853 | GAGGCCA |
| SEQ ID NO: 8599 | GACGCCG | SEQ ID NO: 8650 | GACUAGC | SEQ ID NO: 8701 | GACUUUA | SEQ ID NO: 8752 | GAGAGUU | SEQ ID NO: 8803 | GAGCGAG | SEQ ID NO: 8854 | GAGGCCC |
| SEQ ID NO: 8600 | GACGCCU | SEQ ID NO: 8651 | GACUAGG | SEQ ID NO: 8702 | GACUUUC | SEQ ID NO: 8753 | GAGAUAA | SEQ ID NO: 8804 | GAGCGAU | SEQ ID NO: 8855 | GAGGCCG |
| SEQ ID NO: 8601 | GACGCGA | SEQ ID NO: 8652 | GACUAGU | SEQ ID NO: 8703 | GACUUUG | SEQ ID NO: 8754 | GAGAUAC | SEQ ID NO: 8805 | GAGCGCA | SEQ ID NO: 8856 | GAGGCCU |
| SEQ ID NO: 8602 | GACGCGC | SEQ ID NO: 8653 | GACUAUA | SEQ ID NO: 8704 | GACUUUU | SEQ ID NO: 8755 | GAGAUAG | SEQ ID NO: 8806 | GAGCGCC | SEQ ID NO: 8857 | GAGGCGA |
| SEQ ID NO: 8603 | GACGCGG | SEQ ID NO: 8654 | GACUAUC | SEQ ID NO: 8705 | GAGAAAA | SEQ ID NO: 8756 | GAGAUAU | SEQ ID NO: 8807 | GAGCGCG | SEQ ID NO: 8858 | GAGGCGC |
| SEQ ID NO: 8604 | GACGCGU | SEQ ID NO: 8655 | GACUAUG | SEQ ID NO: 8706 | GAGAAAC | SEQ ID NO: 8757 | GAGAUCA | SEQ ID NO: 8808 | GAGCGCU | SEQ ID NO: 8859 | GAGGCGG |
| SEQ ID NO: 8605 | GACGCUA | SEQ ID NO: 8656 | GACUAUU | SEQ ID NO: 8707 | GAGAAAG | SEQ ID NO: 8758 | GAGAUCC | SEQ ID NO: 8809 | GAGCGGA | SEQ ID NO: 8860 | GAGGCGU |
| SEQ ID NO: 8606 | GACGCUC | SEQ ID NO: 8657 | GACUCAA | SEQ ID NO: 8708 | GAGAAAU | SEQ ID NO: 8759 | GAGAUCG | SEQ ID NO: 8810 | GAGCGGC | SEQ ID NO: 8861 | GAGGCUA |
| SEQ ID NO: 8607 | GACGCUG | SEQ ID NO: 8658 | GACUCAC | SEQ ID NO: 8709 | GAGAACA | SEQ ID NO: 8760 | GAGAUCU | SEQ ID NO: 8811 | GAGCGGG | SEQ ID NO: 8862 | GAGGCUC |
| SEQ ID NO: 8608 | GACGCUU | SEQ ID NO: 8659 | GACUCAG | SEQ ID NO: 8710 | GAGAACC | SEQ ID NO: 8761 | GAGAUGA | SEQ ID NO: 8812 | GAGCGGU | SEQ ID NO: 8863 | GAGGCUG |
| SEQ ID NO: 8609 | GACGGAA | SEQ ID NO: 8660 | GACUCAU | SEQ ID NO: 8711 | GAGAACG | SEQ ID NO: 8762 | GAGAUGC | SEQ ID NO: 8813 | GAGCGUA | SEQ ID NO: 8864 | GAGGCUU |
| SEQ ID NO: 8610 | GACGGAC | SEQ ID NO: 8661 | GACUCCA | SEQ ID NO: 8712 | GAGAACU | SEQ ID NO: 8763 | GAGAUGG | SEQ ID NO: 8814 | GAGCGUC | SEQ ID NO: 8865 | GAGGGAA |
| SEQ ID NO: 8611 | GACGGAG | SEQ ID NO: 8662 | GACUCCC | SEQ ID NO: 8713 | GAGAAGA | SEQ ID NO: 8764 | GAGAUGU | SEQ ID NO: 8815 | GAGCGUG | SEQ ID NO: 8866 | GAGGGAC |
| SEQ ID NO: 8612 | GACGGAU | SEQ ID NO: 8663 | GACUCCG | SEQ ID NO: 8714 | GAGAAGC | SEQ ID NO: 8765 | GAGAUUA | SEQ ID NO: 8816 | GAGCGUU | SEQ ID NO: 8867 | GAGGGAG |
| SEQ ID NO: 8613 | GACGGCA | SEQ ID NO: 8664 | GACUCCU | SEQ ID NO: 8715 | GAGAAGG | SEQ ID NO: 8766 | GAGAUUC | SEQ ID NO: 8817 | GAGCUAA | SEQ ID NO: 8868 | GAGGGAU |
| SEQ ID NO: 8614 | GACGGCC | SEQ ID NO: 8665 | GACUCGA | SEQ ID NO: 8716 | GAGAAGU | SEQ ID NO: 8767 | GAGAUUG | SEQ ID NO: 8818 | GAGCUAC | SEQ ID NO: 8869 | GAGGGCA |
| SEQ ID NO: 8615 | GACGGCG | SEQ ID NO: 8666 | GACUCGC | SEQ ID NO: 8717 | GAGAAUA | SEQ ID NO: 8768 | GAGAUUU | SEQ ID NO: 8819 | GAGCUAG | SEQ ID NO: 8870 | GAGGGCC |
| SEQ ID NO: 8616 | GACGGCU | SEQ ID NO: 8667 | GACUCGG | SEQ ID NO: 8718 | GAGAAUC | SEQ ID NO: 8769 | GAGCAAA | SEQ ID NO: 8820 | GAGCUAU | SEQ ID NO: 8871 | GAGGGCG |
| SEQ ID NO: 8617 | GACGGGA | SEQ ID NO: 8668 | GACUCGU | SEQ ID NO: 8719 | GAGAAUG | SEQ ID NO: 8770 | GAGCAAC | SEQ ID NO: 8821 | GAGCUCA | SEQ ID NO: 8872 | GAGGGCU |
| SEQ ID NO: 8618 | GACGGGC | SEQ ID NO: 8669 | GACUCUA | SEQ ID NO: 8720 | GAGAAUU | SEQ ID NO: 8771 | GAGCAAG | SEQ ID NO: 8822 | GAGCUCC | SEQ ID NO: 8873 | GAGGGGA |
| SEQ ID NO: 8619 | GACGGGG | SEQ ID NO: 8670 | GACUCUC | SEQ ID NO: 8721 | GAGACAA | SEQ ID NO: 8772 | GAGCAAU | SEQ ID NO: 8823 | GAGCUCG | SEQ ID NO: 8874 | GAGGGGC |

Table 30

| | | | | | | | | | | | |
|---|---|---|---|---|---|---|---|---|---|---|---|
| SEQ ID NO: 8875 | GAGGGGG | SEQ ID NO: 8926 | GAGUCUC | SEQ ID NO: 8977 | GAUACAA | SEQ ID NO: 9028 | GAUCAAU | SEQ ID NO: 9079 | GAUCUCG | SEQ ID NO: 9130 | GAUGGGC |
| SEQ ID NO: 8876 | GAGGGGU | SEQ ID NO: 8927 | GAGUCUG | SEQ ID NO: 8978 | GAUACAC | SEQ ID NO: 9029 | GAUCACA | SEQ ID NO: 9080 | GAUCUCU | SEQ ID NO: 9131 | GAUGGGG |
| SEQ ID NO: 8877 | GAGGGUA | SEQ ID NO: 8928 | GAGUCUU | SEQ ID NO: 8979 | GAUACAG | SEQ ID NO: 9030 | GAUCACC | SEQ ID NO: 9081 | GAUCUGA | SEQ ID NO: 9132 | GAUGGGU |
| SEQ ID NO: 8878 | GAGGGUC | SEQ ID NO: 8929 | GAGUGAA | SEQ ID NO: 8980 | GAUACAU | SEQ ID NO: 9031 | GAUCACG | SEQ ID NO: 9082 | GAUCUGC | SEQ ID NO: 9133 | GAUGGUA |
| SEQ ID NO: 8879 | GAGGGUG | SEQ ID NO: 8930 | GAGUGAC | SEQ ID NO: 8981 | GAUACCA | SEQ ID NO: 9032 | GAUCACU | SEQ ID NO: 9083 | GAUCUGG | SEQ ID NO: 9134 | GAUGGUC |
| SEQ ID NO: 8880 | GAGGGUU | SEQ ID NO: 8931 | GAGUGAG | SEQ ID NO: 8982 | GAUACCC | SEQ ID NO: 9033 | GAUCAGA | SEQ ID NO: 9084 | GAUCUGU | SEQ ID NO: 9135 | GAUGGUG |
| SEQ ID NO: 8881 | GAGGUAA | SEQ ID NO: 8932 | GAGUGAU | SEQ ID NO: 8983 | GAUACCG | SEQ ID NO: 9034 | GAUCAGC | SEQ ID NO: 9085 | GAUCUUA | SEQ ID NO: 9136 | GAUGGUU |
| SEQ ID NO: 8882 | GAGGUAC | SEQ ID NO: 8933 | GAGUGCA | SEQ ID NO: 8984 | GAUACCU | SEQ ID NO: 9035 | GAUCAGG | SEQ ID NO: 9086 | GAUCUUC | SEQ ID NO: 9137 | GAUGUAA |
| SEQ ID NO: 8883 | GAGGUAG | SEQ ID NO: 8934 | GAGUGCC | SEQ ID NO: 8985 | GAUACGA | SEQ ID NO: 9036 | GAUCAGU | SEQ ID NO: 9087 | GAUCUUG | SEQ ID NO: 9138 | GAUGUAC |
| SEQ ID NO: 8884 | GAGGUAU | SEQ ID NO: 8935 | GAGUGCG | SEQ ID NO: 8986 | GAUACGC | SEQ ID NO: 9037 | GAUCAUA | SEQ ID NO: 9088 | GAUCUUU | SEQ ID NO: 9139 | GAUGUAG |
| SEQ ID NO: 8885 | GAGGUCA | SEQ ID NO: 8936 | GAGUGCU | SEQ ID NO: 8987 | GAUACGG | SEQ ID NO: 9038 | GAUCAUC | SEQ ID NO: 9089 | GAUGAAA | SEQ ID NO: 9140 | GAUGUAU |
| SEQ ID NO: 8886 | GAGGUCC | SEQ ID NO: 8937 | GAGUGGA | SEQ ID NO: 8988 | GAUACGU | SEQ ID NO: 9039 | GAUCAUG | SEQ ID NO: 9090 | GAUGAAC | SEQ ID NO: 9141 | GAUGUCA |
| SEQ ID NO: 8887 | GAGGUCG | SEQ ID NO: 8938 | GAGUGGC | SEQ ID NO: 8989 | GAUACUA | SEQ ID NO: 9040 | GAUCAUU | SEQ ID NO: 9091 | GAUGAAG | SEQ ID NO: 9142 | GAUGUCC |
| SEQ ID NO: 8888 | GAGGUCU | SEQ ID NO: 8939 | GAGUGGG | SEQ ID NO: 8990 | GAUACUC | SEQ ID NO: 9041 | GAUCCAA | SEQ ID NO: 9092 | GAUGAAU | SEQ ID NO: 9143 | GAUGUCG |
| SEQ ID NO: 8889 | GAGGUGA | SEQ ID NO: 8940 | GAGUGGU | SEQ ID NO: 8991 | GAUACUG | SEQ ID NO: 9042 | GAUCCAC | SEQ ID NO: 9093 | GAUGACA | SEQ ID NO: 9144 | GAUGUCU |
| SEQ ID NO: 8890 | GAGGUGC | SEQ ID NO: 8941 | GAGUGUA | SEQ ID NO: 8992 | GAUACUU | SEQ ID NO: 9043 | GAUCCAG | SEQ ID NO: 9094 | GAUGACC | SEQ ID NO: 9145 | GAUGUGA |
| SEQ ID NO: 8891 | GAGGUGG | SEQ ID NO: 8942 | GAGUGUC | SEQ ID NO: 8993 | GAUAGAA | SEQ ID NO: 9044 | GAUCCAU | SEQ ID NO: 9095 | GAUGACG | SEQ ID NO: 9146 | GAUGUGC |
| SEQ ID NO: 8892 | GAGGUGU | SEQ ID NO: 8943 | GAGUGUG | SEQ ID NO: 8994 | GAUAGAC | SEQ ID NO: 9045 | GAUCCCA | SEQ ID NO: 9096 | GAUGACU | SEQ ID NO: 9147 | GAUGUGG |
| SEQ ID NO: 8893 | GAGGUUA | SEQ ID NO: 8944 | GAGUGUU | SEQ ID NO: 8995 | GAUAGAG | SEQ ID NO: 9046 | GAUCCCC | SEQ ID NO: 9097 | GAUGAGA | SEQ ID NO: 9148 | GAUGUGU |
| SEQ ID NO: 8894 | GAGGUUC | SEQ ID NO: 8945 | GAGUUAA | SEQ ID NO: 8996 | GAUAGAU | SEQ ID NO: 9047 | GAUCCCG | SEQ ID NO: 9098 | GAUGAGC | SEQ ID NO: 9149 | GAUGUUA |
| SEQ ID NO: 8895 | GAGGUUG | SEQ ID NO: 8946 | GAGUUAC | SEQ ID NO: 8997 | GAUAGCA | SEQ ID NO: 9048 | GAUCCCU | SEQ ID NO: 9099 | GAUGAGG | SEQ ID NO: 9150 | GAUGUUC |
| SEQ ID NO: 8896 | GAGGUUU | SEQ ID NO: 8947 | GAGUUAG | SEQ ID NO: 8998 | GAUAGCC | SEQ ID NO: 9049 | GAUCCGA | SEQ ID NO: 9100 | GAUGAGU | SEQ ID NO: 9151 | GAUGUUG |
| SEQ ID NO: 8897 | GAGUAAA | SEQ ID NO: 8948 | GAGUUAU | SEQ ID NO: 8999 | GAUAGCG | SEQ ID NO: 9050 | GAUCCGC | SEQ ID NO: 9101 | GAUGAUA | SEQ ID NO: 9152 | GAUGUUU |
| SEQ ID NO: 8898 | GAGUAAC | SEQ ID NO: 8949 | GAGUUCA | SEQ ID NO: 9000 | GAUAGCU | SEQ ID NO: 9051 | GAUCCGG | SEQ ID NO: 9102 | GAUGAUC | SEQ ID NO: 9153 | GAUUAAA |
| SEQ ID NO: 8899 | GAGUAAG | SEQ ID NO: 8950 | GAGUUCC | SEQ ID NO: 9001 | GAUAGGA | SEQ ID NO: 9052 | GAUCCGU | SEQ ID NO: 9103 | GAUGAUG | SEQ ID NO: 9154 | GAUUAAC |
| SEQ ID NO: 8900 | GAGUAAU | SEQ ID NO: 8951 | GAGUUCG | SEQ ID NO: 9002 | GAUAGGC | SEQ ID NO: 9053 | GAUCCUA | SEQ ID NO: 9104 | GAUGAUU | SEQ ID NO: 9155 | GAUUAAG |
| SEQ ID NO: 8901 | GAGUACA | SEQ ID NO: 8952 | GAGUUCU | SEQ ID NO: 9003 | GAUAGGG | SEQ ID NO: 9054 | GAUCCUC | SEQ ID NO: 9105 | GAUGCAA | SEQ ID NO: 9156 | GAUUAAU |
| SEQ ID NO: 8902 | GAGUACC | SEQ ID NO: 8953 | GAGUUGA | SEQ ID NO: 9004 | GAUAGGU | SEQ ID NO: 9055 | GAUCCUG | SEQ ID NO: 9106 | GAUGCAC | SEQ ID NO: 9157 | GAUUACA |
| SEQ ID NO: 8903 | GAGUACG | SEQ ID NO: 8954 | GAGUUGC | SEQ ID NO: 9005 | GAUAGUA | SEQ ID NO: 9056 | GAUCCUU | SEQ ID NO: 9107 | GAUGCAG | SEQ ID NO: 9158 | GAUUACC |
| SEQ ID NO: 8904 | GAGUACU | SEQ ID NO: 8955 | GAGUUGG | SEQ ID NO: 9006 | GAUAGUC | SEQ ID NO: 9057 | GAUCGAA | SEQ ID NO: 9108 | GAUGCAU | SEQ ID NO: 9159 | GAUUACG |
| SEQ ID NO: 8905 | GAGUAGA | SEQ ID NO: 8956 | GAGUUGU | SEQ ID NO: 9007 | GAUAGUG | SEQ ID NO: 9058 | GAUCGAC | SEQ ID NO: 9109 | GAUGCCA | SEQ ID NO: 9160 | GAUUACU |
| SEQ ID NO: 8906 | GAGUAGC | SEQ ID NO: 8957 | GAGUUUA | SEQ ID NO: 9008 | GAUAGUU | SEQ ID NO: 9059 | GAUCGAG | SEQ ID NO: 9110 | GAUGCCC | SEQ ID NO: 9161 | GAUUAGA |
| SEQ ID NO: 8907 | GAGUAGG | SEQ ID NO: 8958 | GAGUUUC | SEQ ID NO: 9009 | GAUAUAA | SEQ ID NO: 9060 | GAUCGAU | SEQ ID NO: 9111 | GAUGCCG | SEQ ID NO: 9162 | GAUUAGC |
| SEQ ID NO: 8908 | GAGUAGU | SEQ ID NO: 8959 | GAGUUUG | SEQ ID NO: 9010 | GAUAUAC | SEQ ID NO: 9061 | GAUCGCA | SEQ ID NO: 9112 | GAUGCCU | SEQ ID NO: 9163 | GAUUAGG |
| SEQ ID NO: 8909 | GAGUAUA | SEQ ID NO: 8960 | GAGUUUU | SEQ ID NO: 9011 | GAUAUAG | SEQ ID NO: 9062 | GAUCGCC | SEQ ID NO: 9113 | GAUGCGA | SEQ ID NO: 9164 | GAUUAGU |
| SEQ ID NO: 8910 | GAGUAUC | SEQ ID NO: 8961 | GAUAAAA | SEQ ID NO: 9012 | GAUAUAU | SEQ ID NO: 9063 | GAUCGCG | SEQ ID NO: 9114 | GAUGCGC | SEQ ID NO: 9165 | GAUUAUA |
| SEQ ID NO: 8911 | GAGUAUG | SEQ ID NO: 8962 | GAUAAAC | SEQ ID NO: 9013 | GAUAUCA | SEQ ID NO: 9064 | GAUCGCU | SEQ ID NO: 9115 | GAUGCGG | SEQ ID NO: 9166 | GAUUAUC |
| SEQ ID NO: 8912 | GAGUAUU | SEQ ID NO: 8963 | GAUAAAG | SEQ ID NO: 9014 | GAUAUCC | SEQ ID NO: 9065 | GAUCGGA | SEQ ID NO: 9116 | GAUGCGU | SEQ ID NO: 9167 | GAUUAUG |
| SEQ ID NO: 8913 | GAGUCAA | SEQ ID NO: 8964 | GAUAAAU | SEQ ID NO: 9015 | GAUAUCG | SEQ ID NO: 9066 | GAUCGGC | SEQ ID NO: 9117 | GAUGCUA | SEQ ID NO: 9168 | GAUUAUU |
| SEQ ID NO: 8914 | GAGUCAC | SEQ ID NO: 8965 | GAUAACA | SEQ ID NO: 9016 | GAUAUCU | SEQ ID NO: 9067 | GAUCGGG | SEQ ID NO: 9118 | GAUGCUC | SEQ ID NO: 9169 | GAUUCAA |
| SEQ ID NO: 8915 | GAGUCAG | SEQ ID NO: 8966 | GAUAACC | SEQ ID NO: 9017 | GAUAUGA | SEQ ID NO: 9068 | GAUCGGU | SEQ ID NO: 9119 | GAUGCUG | SEQ ID NO: 9170 | GAUUCAC |
| SEQ ID NO: 8916 | GAGUCAU | SEQ ID NO: 8967 | GAUAACG | SEQ ID NO: 9018 | GAUAUGC | SEQ ID NO: 9069 | GAUCGUA | SEQ ID NO: 9120 | GAUGCUU | SEQ ID NO: 9171 | GAUUCAG |
| SEQ ID NO: 8917 | GAGUCCA | SEQ ID NO: 8968 | GAUAACU | SEQ ID NO: 9019 | GAUAUGG | SEQ ID NO: 9070 | GAUCGUC | SEQ ID NO: 9121 | GAUGGAA | SEQ ID NO: 9172 | GAUUCAU |
| SEQ ID NO: 8918 | GAGUCCC | SEQ ID NO: 8969 | GAUAAGA | SEQ ID NO: 9020 | GAUAUGU | SEQ ID NO: 9071 | GAUCGUG | SEQ ID NO: 9122 | GAUGGAC | SEQ ID NO: 9173 | GAUUCCA |
| SEQ ID NO: 8919 | GAGUCCG | SEQ ID NO: 8970 | GAUAAGC | SEQ ID NO: 9021 | GAUAUUA | SEQ ID NO: 9072 | GAUCGUU | SEQ ID NO: 9123 | GAUGGAG | SEQ ID NO: 9174 | GAUUCCC |
| SEQ ID NO: 8920 | GAGUCCU | SEQ ID NO: 8971 | GAUAAGG | SEQ ID NO: 9022 | GAUAUUC | SEQ ID NO: 9073 | GAUCUAA | SEQ ID NO: 9124 | GAUGGAU | SEQ ID NO: 9175 | GAUUCCG |
| SEQ ID NO: 8921 | GAGUCGA | SEQ ID NO: 8972 | GAUAAGU | SEQ ID NO: 9023 | GAUAUUG | SEQ ID NO: 9074 | GAUCUAC | SEQ ID NO: 9125 | GAUGGCA | SEQ ID NO: 9176 | GAUUCCU |
| SEQ ID NO: 8922 | GAGUCGC | SEQ ID NO: 8973 | GAUAAUA | SEQ ID NO: 9024 | GAUAUUU | SEQ ID NO: 9075 | GAUCUAG | SEQ ID NO: 9126 | GAUGGCC | SEQ ID NO: 9177 | GAUUCGA |
| SEQ ID NO: 8923 | GAGUCGG | SEQ ID NO: 8974 | GAUAAUC | SEQ ID NO: 9025 | GAUCAAA | SEQ ID NO: 9076 | GAUCUAU | SEQ ID NO: 9127 | GAUGGCG | SEQ ID NO: 9178 | GAUUCGC |
| SEQ ID NO: 8924 | GAGUCGU | SEQ ID NO: 8975 | GAUAAUG | SEQ ID NO: 9026 | GAUCAAC | SEQ ID NO: 9077 | GAUCUCA | SEQ ID NO: 9128 | GAUGGCU | SEQ ID NO: 9179 | GAUUCGG |
| SEQ ID NO: 8925 | GAGUCUA | SEQ ID NO: 8976 | GAUAAUU | SEQ ID NO: 9027 | GAUCAAG | SEQ ID NO: 9078 | GAUCUCC | SEQ ID NO: 9129 | GAUGGGA | SEQ ID NO: 9180 | GAUUCGU |

Table 31

| | |
|---|---|
| SEQ ID NO: 9181 | GAUUCUA |
| SEQ ID NO: 9182 | GAUUCUC |
| SEQ ID NO: 9183 | GAUUCUG |
| SEQ ID NO: 9184 | GAUUCUU |
| SEQ ID NO: 9185 | GAUUGAA |
| SEQ ID NO: 9186 | GAUUGAC |
| SEQ ID NO: 9187 | GAUUGAG |
| SEQ ID NO: 9188 | GAUUGAU |
| SEQ ID NO: 9189 | GAUUGCA |
| SEQ ID NO: 9190 | GAUUGCC |
| SEQ ID NO: 9191 | GAUUGCG |
| SEQ ID NO: 9192 | GAUUGCU |
| SEQ ID NO: 9193 | GAUUGGA |
| SEQ ID NO: 9194 | GAUUGGC |
| SEQ ID NO: 9195 | GAUUGGG |
| SEQ ID NO: 9196 | GAUUGGU |
| SEQ ID NO: 9197 | GAUUGUA |
| SEQ ID NO: 9198 | GAUUGUC |
| SEQ ID NO: 9199 | GAUUGUG |
| SEQ ID NO: 9200 | GAUUGUU |
| SEQ ID NO: 9201 | GAUUUAA |
| SEQ ID NO: 9202 | GAUUUAC |
| SEQ ID NO: 9203 | GAUUUAG |
| SEQ ID NO: 9204 | GAUUUAU |
| SEQ ID NO: 9205 | GAUUUCA |
| SEQ ID NO: 9206 | GAUUUCC |
| SEQ ID NO: 9207 | GAUUUCG |
| SEQ ID NO: 9208 | GAUUUCU |
| SEQ ID NO: 9209 | GAUUUGA |
| SEQ ID NO: 9210 | GAUUUGC |
| SEQ ID NO: 9211 | GAUUUGG |
| SEQ ID NO: 9212 | GAUUUGU |
| SEQ ID NO: 9213 | GAUUUUA |
| SEQ ID NO: 9214 | GAUUUUC |
| SEQ ID NO: 9215 | GAUUUUG |
| SEQ ID NO: 9216 | GAUUUUU |
| SEQ ID NO: 9217 | GCAAAAA |
| SEQ ID NO: 9218 | GCAAAAC |
| SEQ ID NO: 9219 | GCAAAAG |
| SEQ ID NO: 9220 | GCAAAAU |
| SEQ ID NO: 9221 | GCAAACA |
| SEQ ID NO: 9222 | GCAAACC |
| SEQ ID NO: 9223 | GCAAACG |
| SEQ ID NO: 9224 | GCAAACU |
| SEQ ID NO: 9225 | GCAAAGA |
| SEQ ID NO: 9226 | GCAAAGC |
| SEQ ID NO: 9227 | GCAAAGG |
| SEQ ID NO: 9228 | GCAAAGU |
| SEQ ID NO: 9229 | GCAAAUA |
| SEQ ID NO: 9230 | GCAAAUC |
| SEQ ID NO: 9231 | GCAAAUG |

| | |
|---|---|
| SEQ ID NO: 9232 | GCAAAUU |
| SEQ ID NO: 9233 | GCAACAA |
| SEQ ID NO: 9234 | GCAACAC |
| SEQ ID NO: 9235 | GCAACAG |
| SEQ ID NO: 9236 | GCAACAU |
| SEQ ID NO: 9237 | GCAACCA |
| SEQ ID NO: 9238 | GCAACCC |
| SEQ ID NO: 9239 | GCAACCG |
| SEQ ID NO: 9240 | GCAACCU |
| SEQ ID NO: 9241 | GCAACGA |
| SEQ ID NO: 9242 | GCAACGC |
| SEQ ID NO: 9243 | GCAACGG |
| SEQ ID NO: 9244 | GCAACGU |
| SEQ ID NO: 9245 | GCAACUA |
| SEQ ID NO: 9246 | GCAACUC |
| SEQ ID NO: 9247 | GCAACUG |
| SEQ ID NO: 9248 | GCAACUU |
| SEQ ID NO: 9249 | GCAAGAA |
| SEQ ID NO: 9250 | GCAAGAC |
| SEQ ID NO: 9251 | GCAAGAG |
| SEQ ID NO: 9252 | GCAAGAU |
| SEQ ID NO: 9253 | GCAAGCA |
| SEQ ID NO: 9254 | GCAAGCC |
| SEQ ID NO: 9255 | GCAAGCG |
| SEQ ID NO: 9256 | GCAAGCU |
| SEQ ID NO: 9257 | GCAAGGA |
| SEQ ID NO: 9258 | GCAAGGC |
| SEQ ID NO: 9259 | GCAAGGG |
| SEQ ID NO: 9260 | GCAAGGU |
| SEQ ID NO: 9261 | GCAAGUA |
| SEQ ID NO: 9262 | GCAAGUC |
| SEQ ID NO: 9263 | GCAAGUG |
| SEQ ID NO: 9264 | GCAAGUU |
| SEQ ID NO: 9265 | GCAAUAA |
| SEQ ID NO: 9266 | GCAAUAC |
| SEQ ID NO: 9267 | GCAAUAG |
| SEQ ID NO: 9268 | GCAAUAU |
| SEQ ID NO: 9269 | GCAAUCA |
| SEQ ID NO: 9270 | GCAAUCC |
| SEQ ID NO: 9271 | GCAAUCG |
| SEQ ID NO: 9272 | GCAAUCU |
| SEQ ID NO: 9273 | GCAAUGA |
| SEQ ID NO: 9274 | GCAAUGC |
| SEQ ID NO: 9275 | GCAAUGG |
| SEQ ID NO: 9276 | GCAAUGU |
| SEQ ID NO: 9277 | GCAAUUA |
| SEQ ID NO: 9278 | GCAAUUC |
| SEQ ID NO: 9279 | GCAAUUG |
| SEQ ID NO: 9280 | GCAAUUU |
| SEQ ID NO: 9281 | GCACAAA |
| SEQ ID NO: 9282 | GCACAAC |

| | |
|---|---|
| SEQ ID NO: 9283 | GCACAAG |
| SEQ ID NO: 9284 | GCACAAU |
| SEQ ID NO: 9285 | GCACACA |
| SEQ ID NO: 9286 | GCACACC |
| SEQ ID NO: 9287 | GCACACG |
| SEQ ID NO: 9288 | GCACACU |
| SEQ ID NO: 9289 | GCACAGA |
| SEQ ID NO: 9290 | GCACAGC |
| SEQ ID NO: 9291 | GCACAGG |
| SEQ ID NO: 9292 | GCACAGU |
| SEQ ID NO: 9293 | GCACAUA |
| SEQ ID NO: 9294 | GCACAUC |
| SEQ ID NO: 9295 | GCACAUG |
| SEQ ID NO: 9296 | GCACAUU |
| SEQ ID NO: 9297 | GCACCAA |
| SEQ ID NO: 9298 | GCACCAC |
| SEQ ID NO: 9299 | GCACCAG |
| SEQ ID NO: 9300 | GCACCAU |
| SEQ ID NO: 9301 | GCACCCA |
| SEQ ID NO: 9302 | GCACCCC |
| SEQ ID NO: 9303 | GCACCCG |
| SEQ ID NO: 9304 | GCACCCU |
| SEQ ID NO: 9305 | GCACCGA |
| SEQ ID NO: 9306 | GCACCGC |
| SEQ ID NO: 9307 | GCACCGG |
| SEQ ID NO: 9308 | GCACCGU |
| SEQ ID NO: 9309 | GCACCUA |
| SEQ ID NO: 9310 | GCACCUC |
| SEQ ID NO: 9311 | GCACCUG |
| SEQ ID NO: 9312 | GCACCUU |
| SEQ ID NO: 9313 | GCACGAA |
| SEQ ID NO: 9314 | GCACGAC |
| SEQ ID NO: 9315 | GCACGAG |
| SEQ ID NO: 9316 | GCACGAU |
| SEQ ID NO: 9317 | GCACGCA |
| SEQ ID NO: 9318 | GCACGCC |
| SEQ ID NO: 9319 | GCACGCG |
| SEQ ID NO: 9320 | GCACGCU |
| SEQ ID NO: 9321 | GCACGGA |
| SEQ ID NO: 9322 | GCACGGC |
| SEQ ID NO: 9323 | GCACGGG |
| SEQ ID NO: 9324 | GCACGGU |
| SEQ ID NO: 9325 | GCACGUA |
| SEQ ID NO: 9326 | GCACGUC |
| SEQ ID NO: 9327 | GCACGUG |
| SEQ ID NO: 9328 | GCACGUU |
| SEQ ID NO: 9329 | GCACUAA |
| SEQ ID NO: 9330 | GCACUAC |
| SEQ ID NO: 9331 | GCACUAG |
| SEQ ID NO: 9332 | GCACUAU |
| SEQ ID NO: 9333 | GCACUCA |

| | |
|---|---|
| SEQ ID NO: 9334 | GCACUCC |
| SEQ ID NO: 9335 | GCACUCG |
| SEQ ID NO: 9336 | GCACUCU |
| SEQ ID NO: 9337 | GCACUGA |
| SEQ ID NO: 9338 | GCACUGC |
| SEQ ID NO: 9339 | GCACUGG |
| SEQ ID NO: 9340 | GCACUGU |
| SEQ ID NO: 9341 | GCACUUA |
| SEQ ID NO: 9342 | GCACUUC |
| SEQ ID NO: 9343 | GCACUUG |
| SEQ ID NO: 9344 | GCACUUU |
| SEQ ID NO: 9345 | GCAGAAA |
| SEQ ID NO: 9346 | GCAGAAC |
| SEQ ID NO: 9347 | GCAGAAG |
| SEQ ID NO: 9348 | GCAGAAU |
| SEQ ID NO: 9349 | GCAGACA |
| SEQ ID NO: 9350 | GCAGACC |
| SEQ ID NO: 9351 | GCAGACG |
| SEQ ID NO: 9352 | GCAGACU |
| SEQ ID NO: 9353 | GCAGAGA |
| SEQ ID NO: 9354 | GCAGAGC |
| SEQ ID NO: 9355 | GCAGAGG |
| SEQ ID NO: 9356 | GCAGAGU |
| SEQ ID NO: 9357 | GCAGAUA |
| SEQ ID NO: 9358 | GCAGAUC |
| SEQ ID NO: 9359 | GCAGAUG |
| SEQ ID NO: 9360 | GCAGAUU |
| SEQ ID NO: 9361 | GCAGCAA |
| SEQ ID NO: 9362 | GCAGCAC |
| SEQ ID NO: 9363 | GCAGCAG |
| SEQ ID NO: 9364 | GCAGCAU |
| SEQ ID NO: 9365 | GCAGCCA |
| SEQ ID NO: 9366 | GCAGCCC |
| SEQ ID NO: 9367 | GCAGCCG |
| SEQ ID NO: 9368 | GCAGCCU |
| SEQ ID NO: 9369 | GCAGCGA |
| SEQ ID NO: 9370 | GCAGCGC |
| SEQ ID NO: 9371 | GCAGCGG |
| SEQ ID NO: 9372 | GCAGCGU |
| SEQ ID NO: 9373 | GCAGCUA |
| SEQ ID NO: 9374 | GCAGCUC |
| SEQ ID NO: 9375 | GCAGCUG |
| SEQ ID NO: 9376 | GCAGCUU |
| SEQ ID NO: 9377 | GCAGGAA |
| SEQ ID NO: 9378 | GCAGGAC |
| SEQ ID NO: 9379 | GCAGGAG |
| SEQ ID NO: 9380 | GCAGGAU |
| SEQ ID NO: 9381 | GCAGGCA |
| SEQ ID NO: 9382 | GCAGGCC |
| SEQ ID NO: 9383 | GCAGGCG |
| SEQ ID NO: 9384 | GCAGGCU |

| | |
|---|---|
| SEQ ID NO: 9385 | GCAGGGA |
| SEQ ID NO: 9386 | GCAGGGC |
| SEQ ID NO: 9387 | GCAGGGG |
| SEQ ID NO: 9388 | GCAGGGU |
| SEQ ID NO: 9389 | GCAGGUA |
| SEQ ID NO: 9390 | GCAGGUC |
| SEQ ID NO: 9391 | GCAGGUG |
| SEQ ID NO: 9392 | GCAGGUU |
| SEQ ID NO: 9393 | GCAGUAA |
| SEQ ID NO: 9394 | GCAGUAC |
| SEQ ID NO: 9395 | GCAGUAG |
| SEQ ID NO: 9396 | GCAGUAU |
| SEQ ID NO: 9397 | GCAGUCA |
| SEQ ID NO: 9398 | GCAGUCC |
| SEQ ID NO: 9399 | GCAGUCG |
| SEQ ID NO: 9400 | GCAGUCU |
| SEQ ID NO: 9401 | GCAGUGA |
| SEQ ID NO: 9402 | GCAGUGC |
| SEQ ID NO: 9403 | GCAGUGG |
| SEQ ID NO: 9404 | GCAGUGU |
| SEQ ID NO: 9405 | GCAGUUA |
| SEQ ID NO: 9406 | GCAGUUC |
| SEQ ID NO: 9407 | GCAGUUG |
| SEQ ID NO: 9408 | GCAGUUU |
| SEQ ID NO: 9409 | GCAUAAA |
| SEQ ID NO: 9410 | GCAUAAC |
| SEQ ID NO: 9411 | GCAUAAG |
| SEQ ID NO: 9412 | GCAUAAU |
| SEQ ID NO: 9413 | GCAUACA |
| SEQ ID NO: 9414 | GCAUACC |
| SEQ ID NO: 9415 | GCAUACG |
| SEQ ID NO: 9416 | GCAUACU |
| SEQ ID NO: 9417 | GCAUAGA |
| SEQ ID NO: 9418 | GCAUAGC |
| SEQ ID NO: 9419 | GCAUAGG |
| SEQ ID NO: 9420 | GCAUAGU |
| SEQ ID NO: 9421 | GCAUAUA |
| SEQ ID NO: 9422 | GCAUAUC |
| SEQ ID NO: 9423 | GCAUAUG |
| SEQ ID NO: 9424 | GCAUAUU |
| SEQ ID NO: 9425 | GCAUCAA |
| SEQ ID NO: 9426 | GCAUCAC |
| SEQ ID NO: 9427 | GCAUCAG |
| SEQ ID NO: 9428 | GCAUCAU |
| SEQ ID NO: 9429 | GCAUCCA |
| SEQ ID NO: 9430 | GCAUCCC |
| SEQ ID NO: 9431 | GCAUCCG |
| SEQ ID NO: 9432 | GCAUCCU |
| SEQ ID NO: 9433 | GCAUCGA |
| SEQ ID NO: 9434 | GCAUCGC |
| SEQ ID NO: 9435 | GCAUCGG |

| | |
|---|---|
| SEQ ID NO: 9436 | GCAUCGU |
| SEQ ID NO: 9437 | GCAUCUA |
| SEQ ID NO: 9438 | GCAUCUC |
| SEQ ID NO: 9439 | GCAUCUG |
| SEQ ID NO: 9440 | GCAUCUU |
| SEQ ID NO: 9441 | GCAUGAA |
| SEQ ID NO: 9442 | GCAUGAC |
| SEQ ID NO: 9443 | GCAUGAG |
| SEQ ID NO: 9444 | GCAUGAU |
| SEQ ID NO: 9445 | GCAUGCA |
| SEQ ID NO: 9446 | GCAUGCC |
| SEQ ID NO: 9447 | GCAUGCG |
| SEQ ID NO: 9448 | GCAUGCU |
| SEQ ID NO: 9449 | GCAUGGA |
| SEQ ID NO: 9450 | GCAUGGC |
| SEQ ID NO: 9451 | GCAUGGG |
| SEQ ID NO: 9452 | GCAUGGU |
| SEQ ID NO: 9453 | GCAUGUA |
| SEQ ID NO: 9454 | GCAUGUC |
| SEQ ID NO: 9455 | GCAUGUG |
| SEQ ID NO: 9456 | GCAUGUU |
| SEQ ID NO: 9457 | GCAUUAA |
| SEQ ID NO: 9458 | GCAUUAC |
| SEQ ID NO: 9459 | GCAUUAG |
| SEQ ID NO: 9460 | GCAUUAU |
| SEQ ID NO: 9461 | GCAUUCA |
| SEQ ID NO: 9462 | GCAUUCC |
| SEQ ID NO: 9463 | GCAUUCG |
| SEQ ID NO: 9464 | GCAUUCU |
| SEQ ID NO: 9465 | GCAUUGA |
| SEQ ID NO: 9466 | GCAUUGC |
| SEQ ID NO: 9467 | GCAUUGG |
| SEQ ID NO: 9468 | GCAUUGU |
| SEQ ID NO: 9469 | GCAUUUA |
| SEQ ID NO: 9470 | GCAUUUC |
| SEQ ID NO: 9471 | GCAUUUG |
| SEQ ID NO: 9472 | GCAUUUU |
| SEQ ID NO: 9473 | GCCAAAA |
| SEQ ID NO: 9474 | GCCAAAC |
| SEQ ID NO: 9475 | GCCAAAG |
| SEQ ID NO: 9476 | GCCAAAU |
| SEQ ID NO: 9477 | GCCAACA |
| SEQ ID NO: 9478 | GCCAACC |
| SEQ ID NO: 9479 | GCCAACG |
| SEQ ID NO: 9480 | GCCAACU |
| SEQ ID NO: 9481 | GCCAAGA |
| SEQ ID NO: 9482 | GCCAAGC |
| SEQ ID NO: 9483 | GCCAAGG |
| SEQ ID NO: 9484 | GCCAAGU |
| SEQ ID NO: 9485 | GCCAAUA |
| SEQ ID NO: 9486 | GCCAAUC |

Table 32

| | | | | | | | | | | | |
|---|---|---|---|---|---|---|---|---|---|---|---|
| SEQ ID NO: 9487 | GCCAAUG | SEQ ID NO: 9538 | GCCCAAC | SEQ ID NO: 9589 | GCCCUCA | SEQ ID NO: 9640 | GCCGGCU | SEQ ID NO: 9691 | GCCUCGG | SEQ ID NO: 9742 | GCGAAUC |
| SEQ ID NO: 9488 | GCCAAUU | SEQ ID NO: 9539 | GCCCAAG | SEQ ID NO: 9590 | GCCCUCC | SEQ ID NO: 9641 | GCCGGGA | SEQ ID NO: 9692 | GCCUCGU | SEQ ID NO: 9743 | GCGAAUG |
| SEQ ID NO: 9489 | GCCACAA | SEQ ID NO: 9540 | GCCCAAU | SEQ ID NO: 9591 | GCCCUCG | SEQ ID NO: 9642 | GCCGGGC | SEQ ID NO: 9693 | GCCUCUA | SEQ ID NO: 9744 | GCGAAUU |
| SEQ ID NO: 9490 | GCCACAC | SEQ ID NO: 9541 | GCCCACA | SEQ ID NO: 9592 | GCCCUCU | SEQ ID NO: 9643 | GCCGGGG | SEQ ID NO: 9694 | GCCUCUC | SEQ ID NO: 9745 | GCGACAA |
| SEQ ID NO: 9491 | GCCACAG | SEQ ID NO: 9542 | GCCCACC | SEQ ID NO: 9593 | GCCCUGA | SEQ ID NO: 9644 | GCCGGGU | SEQ ID NO: 9695 | GCCUCUG | SEQ ID NO: 9746 | GCGACAC |
| SEQ ID NO: 9492 | GCCACAU | SEQ ID NO: 9543 | GCCCACG | SEQ ID NO: 9594 | GCCCUGC | SEQ ID NO: 9645 | GCCGGUA | SEQ ID NO: 9696 | GCCUCUU | SEQ ID NO: 9747 | GCGACAG |
| SEQ ID NO: 9493 | GCCACCA | SEQ ID NO: 9544 | GCCCACU | SEQ ID NO: 9595 | GCCCUGG | SEQ ID NO: 9646 | GCCGGUC | SEQ ID NO: 9697 | GCCUGAA | SEQ ID NO: 9748 | GCGACAU |
| SEQ ID NO: 9494 | GCCACCC | SEQ ID NO: 9545 | GCCCAGA | SEQ ID NO: 9596 | GCCCUGU | SEQ ID NO: 9647 | GCCGGUG | SEQ ID NO: 9698 | GCCUGAC | SEQ ID NO: 9749 | GCGACCA |
| SEQ ID NO: 9495 | GCCACCG | SEQ ID NO: 9546 | GCCCAGC | SEQ ID NO: 9597 | GCCCUUA | SEQ ID NO: 9648 | GCCGGUU | SEQ ID NO: 9699 | GCCUGAG | SEQ ID NO: 9750 | GCGACCC |
| SEQ ID NO: 9496 | GCCACCU | SEQ ID NO: 9547 | GCCCAGG | SEQ ID NO: 9598 | GCCCUUC | SEQ ID NO: 9649 | GCCGUAA | SEQ ID NO: 9700 | GCCUGAU | SEQ ID NO: 9751 | GCGACCG |
| SEQ ID NO: 9497 | GCCACGA | SEQ ID NO: 9548 | GCCCAGU | SEQ ID NO: 9599 | GCCCUUG | SEQ ID NO: 9650 | GCCGUAC | SEQ ID NO: 9701 | GCCUGCA | SEQ ID NO: 9752 | GCGACCU |
| SEQ ID NO: 9498 | GCCACGC | SEQ ID NO: 9549 | GCCCAUA | SEQ ID NO: 9600 | GCCCUUU | SEQ ID NO: 9651 | GCCGUAG | SEQ ID NO: 9702 | GCCUGCC | SEQ ID NO: 9753 | GCGACGA |
| SEQ ID NO: 9499 | GCCACGG | SEQ ID NO: 9550 | GCCCAUC | SEQ ID NO: 9601 | GCCGAAA | SEQ ID NO: 9652 | GCCGUAU | SEQ ID NO: 9703 | GCCUGCG | SEQ ID NO: 9754 | GCGACGC |
| SEQ ID NO: 9500 | GCCACGU | SEQ ID NO: 9551 | GCCCAUG | SEQ ID NO: 9602 | GCCGAAC | SEQ ID NO: 9653 | GCCGUCA | SEQ ID NO: 9704 | GCCUGCU | SEQ ID NO: 9755 | GCGACGG |
| SEQ ID NO: 9501 | GCCACUA | SEQ ID NO: 9552 | GCCCAUU | SEQ ID NO: 9603 | GCCGAAG | SEQ ID NO: 9654 | GCCGUCC | SEQ ID NO: 9705 | GCCUGGA | SEQ ID NO: 9756 | GCGACGU |
| SEQ ID NO: 9502 | GCCACUC | SEQ ID NO: 9553 | GCCCCAA | SEQ ID NO: 9604 | GCCGAAU | SEQ ID NO: 9655 | GCCGUCG | SEQ ID NO: 9706 | GCCUGGC | SEQ ID NO: 9757 | GCGACUA |
| SEQ ID NO: 9503 | GCCACUG | SEQ ID NO: 9554 | GCCCCAC | SEQ ID NO: 9605 | GCCGACA | SEQ ID NO: 9656 | GCCGUCU | SEQ ID NO: 9707 | GCCUGGG | SEQ ID NO: 9758 | GCGACUC |
| SEQ ID NO: 9504 | GCCACUU | SEQ ID NO: 9555 | GCCCCAG | SEQ ID NO: 9606 | GCCGACC | SEQ ID NO: 9657 | GCCGUGA | SEQ ID NO: 9708 | GCCUGGU | SEQ ID NO: 9759 | GCGACUG |
| SEQ ID NO: 9505 | GCCAGAA | SEQ ID NO: 9556 | GCCCCAU | SEQ ID NO: 9607 | GCCGACG | SEQ ID NO: 9658 | GCCGUGC | SEQ ID NO: 9709 | GCCUGUA | SEQ ID NO: 9760 | GCGACUU |
| SEQ ID NO: 9506 | GCCAGAC | SEQ ID NO: 9557 | GCCCCCA | SEQ ID NO: 9608 | GCCGACU | SEQ ID NO: 9659 | GCCGUGG | SEQ ID NO: 9710 | GCCUGUC | SEQ ID NO: 9761 | GCGAGAA |
| SEQ ID NO: 9507 | GCCAGAG | SEQ ID NO: 9558 | GCCCCCC | SEQ ID NO: 9609 | GCCGAGA | SEQ ID NO: 9660 | GCCGUGU | SEQ ID NO: 9711 | GCCUGUG | SEQ ID NO: 9762 | GCGAGAC |
| SEQ ID NO: 9508 | GCCAGAU | SEQ ID NO: 9559 | GCCCCCG | SEQ ID NO: 9610 | GCCGAGC | SEQ ID NO: 9661 | GCCGUUA | SEQ ID NO: 9712 | GCCUGUU | SEQ ID NO: 9763 | GCGAGAG |
| SEQ ID NO: 9509 | GCCAGCA | SEQ ID NO: 9560 | GCCCCCU | SEQ ID NO: 9611 | GCCGAGG | SEQ ID NO: 9662 | GCCGUUC | SEQ ID NO: 9713 | GCCUUAA | SEQ ID NO: 9764 | GCGAGAU |
| SEQ ID NO: 9510 | GCCAGCC | SEQ ID NO: 9561 | GCCCCGA | SEQ ID NO: 9612 | GCCGAGU | SEQ ID NO: 9663 | GCCGUUG | SEQ ID NO: 9714 | GCCUUAC | SEQ ID NO: 9765 | GCGAGCA |
| SEQ ID NO: 9511 | GCCAGCG | SEQ ID NO: 9562 | GCCCCGC | SEQ ID NO: 9613 | GCCGAUA | SEQ ID NO: 9664 | GCCGUUU | SEQ ID NO: 9715 | GCCUUAG | SEQ ID NO: 9766 | GCGAGCC |
| SEQ ID NO: 9512 | GCCAGCU | SEQ ID NO: 9563 | GCCCCGG | SEQ ID NO: 9614 | GCCGAUC | SEQ ID NO: 9665 | GCCUAAA | SEQ ID NO: 9716 | GCCUUAU | SEQ ID NO: 9767 | GCGAGCG |
| SEQ ID NO: 9513 | GCCAGGA | SEQ ID NO: 9564 | GCCCCGU | SEQ ID NO: 9615 | GCCGAUG | SEQ ID NO: 9666 | GCCUAAC | SEQ ID NO: 9717 | GCCUUCA | SEQ ID NO: 9768 | GCGAGCU |
| SEQ ID NO: 9514 | GCCAGGC | SEQ ID NO: 9565 | GCCCCUA | SEQ ID NO: 9616 | GCCGAUU | SEQ ID NO: 9667 | GCCUAAG | SEQ ID NO: 9718 | GCCUUCC | SEQ ID NO: 9769 | GCGAGGA |
| SEQ ID NO: 9515 | GCCAGGG | SEQ ID NO: 9566 | GCCCCUC | SEQ ID NO: 9617 | GCCGCAA | SEQ ID NO: 9668 | GCCUAAU | SEQ ID NO: 9719 | GCCUUCG | SEQ ID NO: 9770 | GCGAGGC |
| SEQ ID NO: 9516 | GCCAGGU | SEQ ID NO: 9567 | GCCCCUG | SEQ ID NO: 9618 | GCCGCAC | SEQ ID NO: 9669 | GCCUACA | SEQ ID NO: 9720 | GCCUUCU | SEQ ID NO: 9771 | GCGAGGG |
| SEQ ID NO: 9517 | GCCAGUA | SEQ ID NO: 9568 | GCCCCUU | SEQ ID NO: 9619 | GCCGCAG | SEQ ID NO: 9670 | GCCUACC | SEQ ID NO: 9721 | GCCUUGA | SEQ ID NO: 9772 | GCGAGGU |
| SEQ ID NO: 9518 | GCCAGUC | SEQ ID NO: 9569 | GCCCGAA | SEQ ID NO: 9620 | GCCGCAU | SEQ ID NO: 9671 | GCCUACG | SEQ ID NO: 9722 | GCCUUGC | SEQ ID NO: 9773 | GCGAGUA |
| SEQ ID NO: 9519 | GCCAGUG | SEQ ID NO: 9570 | GCCCGAC | SEQ ID NO: 9621 | GCCGCCA | SEQ ID NO: 9672 | GCCUACU | SEQ ID NO: 9723 | GCCUUGG | SEQ ID NO: 9774 | GCGAGUC |
| SEQ ID NO: 9520 | GCCAGUU | SEQ ID NO: 9571 | GCCCGAG | SEQ ID NO: 9622 | GCCGCCC | SEQ ID NO: 9673 | GCCUAGA | SEQ ID NO: 9724 | GCCUUGU | SEQ ID NO: 9775 | GCGAGUG |
| SEQ ID NO: 9521 | GCCAUAA | SEQ ID NO: 9572 | GCCCGAU | SEQ ID NO: 9623 | GCCGCCG | SEQ ID NO: 9674 | GCCUAGC | SEQ ID NO: 9725 | GCCUUUA | SEQ ID NO: 9776 | GCGAGUU |
| SEQ ID NO: 9522 | GCCAUAC | SEQ ID NO: 9573 | GCCCGCA | SEQ ID NO: 9624 | GCCGCCU | SEQ ID NO: 9675 | GCCUAGG | SEQ ID NO: 9726 | GCCUUUC | SEQ ID NO: 9777 | GCGAUAA |
| SEQ ID NO: 9523 | GCCAUAG | SEQ ID NO: 9574 | GCCCGCC | SEQ ID NO: 9625 | GCCGCGA | SEQ ID NO: 9676 | GCCUAGU | SEQ ID NO: 9727 | GCCUUUG | SEQ ID NO: 9778 | GCGAUAC |
| SEQ ID NO: 9524 | GCCAUAU | SEQ ID NO: 9575 | GCCCGCG | SEQ ID NO: 9626 | GCCGCGC | SEQ ID NO: 9677 | GCCUAUA | SEQ ID NO: 9728 | GCCUUUU | SEQ ID NO: 9779 | GCGAUAG |
| SEQ ID NO: 9525 | GCCAUCA | SEQ ID NO: 9576 | GCCCGCU | SEQ ID NO: 9627 | GCCGCGG | SEQ ID NO: 9678 | GCCUAUC | SEQ ID NO: 9729 | GCGAAAA | SEQ ID NO: 9780 | GCGAUAU |
| SEQ ID NO: 9526 | GCCAUCC | SEQ ID NO: 9577 | GCCCGGA | SEQ ID NO: 9628 | GCCGCGU | SEQ ID NO: 9679 | GCCUAUG | SEQ ID NO: 9730 | GCGAAAC | SEQ ID NO: 9781 | GCGAUCA |
| SEQ ID NO: 9527 | GCCAUCG | SEQ ID NO: 9578 | GCCCGGC | SEQ ID NO: 9629 | GCCGCUA | SEQ ID NO: 9680 | GCCUAUU | SEQ ID NO: 9731 | GCGAAAG | SEQ ID NO: 9782 | GCGAUCC |
| SEQ ID NO: 9528 | GCCAUCU | SEQ ID NO: 9579 | GCCCGGG | SEQ ID NO: 9630 | GCCGCUC | SEQ ID NO: 9681 | GCCUCAA | SEQ ID NO: 9732 | GCGAAAU | SEQ ID NO: 9783 | GCGAUCG |
| SEQ ID NO: 9529 | GCCAUGA | SEQ ID NO: 9580 | GCCCGGU | SEQ ID NO: 9631 | GCCGCUG | SEQ ID NO: 9682 | GCCUCAC | SEQ ID NO: 9733 | GCGAACA | SEQ ID NO: 9784 | GCGAUCU |
| SEQ ID NO: 9530 | GCCAUGC | SEQ ID NO: 9581 | GCCCGUA | SEQ ID NO: 9632 | GCCGCUU | SEQ ID NO: 9683 | GCCUCAG | SEQ ID NO: 9734 | GCGAACC | SEQ ID NO: 9785 | GCGAUGA |
| SEQ ID NO: 9531 | GCCAUGG | SEQ ID NO: 9582 | GCCCGUC | SEQ ID NO: 9633 | GCCGGAA | SEQ ID NO: 9684 | GCCUCAU | SEQ ID NO: 9735 | GCGAACG | SEQ ID NO: 9786 | GCGAUGC |
| SEQ ID NO: 9532 | GCCAUGU | SEQ ID NO: 9583 | GCCCGUG | SEQ ID NO: 9634 | GCCGGAC | SEQ ID NO: 9685 | GCCUCCA | SEQ ID NO: 9736 | GCGAACU | SEQ ID NO: 9787 | GCGAUGG |
| SEQ ID NO: 9533 | GCCAUUA | SEQ ID NO: 9584 | GCCCGUU | SEQ ID NO: 9635 | GCCGGAG | SEQ ID NO: 9686 | GCCUCCC | SEQ ID NO: 9737 | GCGAAGA | SEQ ID NO: 9788 | GCGAUGU |
| SEQ ID NO: 9534 | GCCAUUC | SEQ ID NO: 9585 | GCCCUAA | SEQ ID NO: 9636 | GCCGGAU | SEQ ID NO: 9687 | GCCUCCG | SEQ ID NO: 9738 | GCGAAGC | SEQ ID NO: 9789 | GCGAUUA |
| SEQ ID NO: 9535 | GCCAUUG | SEQ ID NO: 9586 | GCCCUAC | SEQ ID NO: 9637 | GCCGGCA | SEQ ID NO: 9688 | GCCUCCU | SEQ ID NO: 9739 | GCGAAGG | SEQ ID NO: 9790 | GCGAUUC |
| SEQ ID NO: 9536 | GCCAUUU | SEQ ID NO: 9587 | GCCCUAG | SEQ ID NO: 9638 | GCCGGCC | SEQ ID NO: 9689 | GCCUCGA | SEQ ID NO: 9740 | GCGAAGU | SEQ ID NO: 9791 | GCGAUUG |
| SEQ ID NO: 9537 | GCCCAAA | SEQ ID NO: 9588 | GCCCUAU | SEQ ID NO: 9639 | GCCGGCG | SEQ ID NO: 9690 | GCCUCGC | SEQ ID NO: 9741 | GCGAAUA | SEQ ID NO: 9792 | GCGAUUU |

Table 33

| | | | | | | | | | | | |
|---|---|---|---|---|---|---|---|---|---|---|---|
| SEQ ID NO: 9793 | GCGCAAA | SEQ ID NO: 9844 | GCGCUAU | SEQ ID NO: 9895 | GCGGGCG | SEQ ID NO: 9946 | GCGUCGC | SEQ ID NO: 9997 | GCUAAUA | SEQ ID NO: 10048 | GCUAUUU |
| SEQ ID NO: 9794 | GCGCAAC | SEQ ID NO: 9845 | GCGCUCA | SEQ ID NO: 9896 | GCGGGCU | SEQ ID NO: 9947 | GCGUCGG | SEQ ID NO: 9998 | GCUAAUC | SEQ ID NO: 10049 | GCUCAAA |
| SEQ ID NO: 9795 | GCGCAAG | SEQ ID NO: 9846 | GCGCUCC | SEQ ID NO: 9897 | GCGGGGA | SEQ ID NO: 9948 | GCGUCGU | SEQ ID NO: 9999 | GCUAAUG | SEQ ID NO: 10050 | GCUCAAC |
| SEQ ID NO: 9796 | GCGCAAU | SEQ ID NO: 9847 | GCGCUCG | SEQ ID NO: 9898 | GCGGGGC | SEQ ID NO: 9949 | GCGUCUA | SEQ ID NO: 10000 | GCUAAUU | SEQ ID NO: 10051 | GCUCAAG |
| SEQ ID NO: 9797 | GCGCACA | SEQ ID NO: 9848 | GCGCUCU | SEQ ID NO: 9899 | GCGGGGG | SEQ ID NO: 9950 | GCGUCUC | SEQ ID NO: 10001 | GCUACAA | SEQ ID NO: 10052 | GCUCAAU |
| SEQ ID NO: 9798 | GCGCACC | SEQ ID NO: 9849 | GCGCUGA | SEQ ID NO: 9900 | GCGGGGU | SEQ ID NO: 9951 | GCGUCUG | SEQ ID NO: 10002 | GCUACAC | SEQ ID NO: 10053 | GCUCACA |
| SEQ ID NO: 9799 | GCGCACG | SEQ ID NO: 9850 | GCGCUGC | SEQ ID NO: 9901 | GCGGGUA | SEQ ID NO: 9952 | GCGUCUU | SEQ ID NO: 10003 | GCUACAG | SEQ ID NO: 10054 | GCUCACC |
| SEQ ID NO: 9800 | GCGCACU | SEQ ID NO: 9851 | GCGCUGG | SEQ ID NO: 9902 | GCGGGUC | SEQ ID NO: 9953 | GCGUGAA | SEQ ID NO: 10004 | GCUACAU | SEQ ID NO: 10055 | GCUCACG |
| SEQ ID NO: 9801 | GCGCAGA | SEQ ID NO: 9852 | GCGCUGU | SEQ ID NO: 9903 | GCGGGUG | SEQ ID NO: 9954 | GCGUGAC | SEQ ID NO: 10005 | GCUACCA | SEQ ID NO: 10056 | GCUCACU |
| SEQ ID NO: 9802 | GCGCAGC | SEQ ID NO: 9853 | GCGCUUA | SEQ ID NO: 9904 | GCGGGUU | SEQ ID NO: 9955 | GCGUGAG | SEQ ID NO: 10006 | GCUACCC | SEQ ID NO: 10057 | GCUCAGA |
| SEQ ID NO: 9803 | GCGCAGG | SEQ ID NO: 9854 | GCGCUUC | SEQ ID NO: 9905 | GCGGUAA | SEQ ID NO: 9956 | GCGUGAU | SEQ ID NO: 10007 | GCUACCG | SEQ ID NO: 10058 | GCUCAGC |
| SEQ ID NO: 9804 | GCGCAGU | SEQ ID NO: 9855 | GCGCUUG | SEQ ID NO: 9906 | GCGGUAC | SEQ ID NO: 9957 | GCGUGCA | SEQ ID NO: 10008 | GCUACCU | SEQ ID NO: 10059 | GCUCAGG |
| SEQ ID NO: 9805 | GCGCAUA | SEQ ID NO: 9856 | GCGCUUU | SEQ ID NO: 9907 | GCGGUAG | SEQ ID NO: 9958 | GCGUGCC | SEQ ID NO: 10009 | GCUACGA | SEQ ID NO: 10060 | GCUCAGU |
| SEQ ID NO: 9806 | GCGCAUC | SEQ ID NO: 9857 | GCGGAAA | SEQ ID NO: 9908 | GCGGUAU | SEQ ID NO: 9959 | GCGUGCG | SEQ ID NO: 10010 | GCUACGC | SEQ ID NO: 10061 | GCUCAUA |
| SEQ ID NO: 9807 | GCGCAUG | SEQ ID NO: 9858 | GCGGAAC | SEQ ID NO: 9909 | GCGGUCA | SEQ ID NO: 9960 | GCGUGCU | SEQ ID NO: 10011 | GCUACGG | SEQ ID NO: 10062 | GCUCAUC |
| SEQ ID NO: 9808 | GCGCAUU | SEQ ID NO: 9859 | GCGGAAG | SEQ ID NO: 9910 | GCGGUCC | SEQ ID NO: 9961 | GCGUGGA | SEQ ID NO: 10012 | GCUACGU | SEQ ID NO: 10063 | GCUCAUG |
| SEQ ID NO: 9809 | GCGCCAA | SEQ ID NO: 9860 | GCGGAAU | SEQ ID NO: 9911 | GCGGUCG | SEQ ID NO: 9962 | GCGUGGC | SEQ ID NO: 10013 | GCUACUA | SEQ ID NO: 10064 | GCUCAUU |
| SEQ ID NO: 9810 | GCGCCAC | SEQ ID NO: 9861 | GCGGACA | SEQ ID NO: 9912 | GCGGUCU | SEQ ID NO: 9963 | GCGUGGG | SEQ ID NO: 10014 | GCUACUC | SEQ ID NO: 10065 | GCUCCAA |
| SEQ ID NO: 9811 | GCGCCAG | SEQ ID NO: 9862 | GCGGACC | SEQ ID NO: 9913 | GCGGUGA | SEQ ID NO: 9964 | GCGUGGU | SEQ ID NO: 10015 | GCUACUG | SEQ ID NO: 10066 | GCUCCAC |
| SEQ ID NO: 9812 | GCGCCAU | SEQ ID NO: 9863 | GCGGACG | SEQ ID NO: 9914 | GCGGUGC | SEQ ID NO: 9965 | GCGUGUA | SEQ ID NO: 10016 | GCUACUU | SEQ ID NO: 10067 | GCUCCAG |
| SEQ ID NO: 9813 | GCGCCCA | SEQ ID NO: 9864 | GCGGACU | SEQ ID NO: 9915 | GCGGUGG | SEQ ID NO: 9966 | GCGUGUC | SEQ ID NO: 10017 | GCUAGAA | SEQ ID NO: 10068 | GCUCCAU |
| SEQ ID NO: 9814 | GCGCCCC | SEQ ID NO: 9865 | GCGGAGA | SEQ ID NO: 9916 | GCGGUGU | SEQ ID NO: 9967 | GCGUGUG | SEQ ID NO: 10018 | GCUAGAC | SEQ ID NO: 10069 | GCUCCCA |
| SEQ ID NO: 9815 | GCGCCCG | SEQ ID NO: 9866 | GCGGAGC | SEQ ID NO: 9917 | GCGGUUA | SEQ ID NO: 9968 | GCGUGUU | SEQ ID NO: 10019 | GCUAGAG | SEQ ID NO: 10070 | GCUCCCC |
| SEQ ID NO: 9816 | GCGCCCU | SEQ ID NO: 9867 | GCGGAGG | SEQ ID NO: 9918 | GCGGUUC | SEQ ID NO: 9969 | GCGUUAA | SEQ ID NO: 10020 | GCUAGAU | SEQ ID NO: 10071 | GCUCCCG |
| SEQ ID NO: 9817 | GCGCCGA | SEQ ID NO: 9868 | GCGGAGU | SEQ ID NO: 9919 | GCGGUUG | SEQ ID NO: 9970 | GCGUUAC | SEQ ID NO: 10021 | GCUAGCA | SEQ ID NO: 10072 | GCUCCCU |
| SEQ ID NO: 9818 | GCGCCGC | SEQ ID NO: 9869 | GCGGAUA | SEQ ID NO: 9920 | GCGGUUU | SEQ ID NO: 9971 | GCGUUAG | SEQ ID NO: 10022 | GCUAGCC | SEQ ID NO: 10073 | GCUCCGA |
| SEQ ID NO: 9819 | GCGCCGG | SEQ ID NO: 9870 | GCGGAUC | SEQ ID NO: 9921 | GCGUAAA | SEQ ID NO: 9972 | GCGUUAU | SEQ ID NO: 10023 | GCUAGCG | SEQ ID NO: 10074 | GCUCCGC |
| SEQ ID NO: 9820 | GCGCCGU | SEQ ID NO: 9871 | GCGGAUG | SEQ ID NO: 9922 | GCGUAAC | SEQ ID NO: 9973 | GCGUUCA | SEQ ID NO: 10024 | GCUAGCU | SEQ ID NO: 10075 | GCUCCGG |
| SEQ ID NO: 9821 | GCGCCUA | SEQ ID NO: 9872 | GCGGAUU | SEQ ID NO: 9923 | GCGUAAG | SEQ ID NO: 9974 | GCGUUCC | SEQ ID NO: 10025 | GCUAGGA | SEQ ID NO: 10076 | GCUCCGU |
| SEQ ID NO: 9822 | GCGCCUC | SEQ ID NO: 9873 | GCGGCAA | SEQ ID NO: 9924 | GCGUAAU | SEQ ID NO: 9975 | GCGUUCG | SEQ ID NO: 10026 | GCUAGGC | SEQ ID NO: 10077 | GCUCCUA |
| SEQ ID NO: 9823 | GCGCCUG | SEQ ID NO: 9874 | GCGGCAC | SEQ ID NO: 9925 | GCGUACA | SEQ ID NO: 9976 | GCGUUCU | SEQ ID NO: 10027 | GCUAGGG | SEQ ID NO: 10078 | GCUCCUC |
| SEQ ID NO: 9824 | GCGCCUU | SEQ ID NO: 9875 | GCGGCAG | SEQ ID NO: 9926 | GCGUACC | SEQ ID NO: 9977 | GCGUUGA | SEQ ID NO: 10028 | GCUAGGU | SEQ ID NO: 10079 | GCUCCUG |
| SEQ ID NO: 9825 | GCGCGAA | SEQ ID NO: 9876 | GCGGCAU | SEQ ID NO: 9927 | GCGUACG | SEQ ID NO: 9978 | GCGUUGC | SEQ ID NO: 10029 | GCUAGUA | SEQ ID NO: 10080 | GCUCCUU |
| SEQ ID NO: 9826 | GCGCGAC | SEQ ID NO: 9877 | GCGGCCA | SEQ ID NO: 9928 | GCGUACU | SEQ ID NO: 9979 | GCGUUGG | SEQ ID NO: 10030 | GCUAGUC | SEQ ID NO: 10081 | GCUCGAA |
| SEQ ID NO: 9827 | GCGCGAG | SEQ ID NO: 9878 | GCGGCCC | SEQ ID NO: 9929 | GCGUAGA | SEQ ID NO: 9980 | GCGUUGU | SEQ ID NO: 10031 | GCUAGUG | SEQ ID NO: 10082 | GCUCGAC |
| SEQ ID NO: 9828 | GCGCGAU | SEQ ID NO: 9879 | GCGGCCG | SEQ ID NO: 9930 | GCGUAGC | SEQ ID NO: 9981 | GCGUUUA | SEQ ID NO: 10032 | GCUAGUU | SEQ ID NO: 10083 | GCUCGAG |
| SEQ ID NO: 9829 | GCGCGCA | SEQ ID NO: 9880 | GCGGCCU | SEQ ID NO: 9931 | GCGUAGG | SEQ ID NO: 9982 | GCGUUUC | SEQ ID NO: 10033 | GCUAUAA | SEQ ID NO: 10084 | GCUCGAU |
| SEQ ID NO: 9830 | GCGCGCC | SEQ ID NO: 9881 | GCGGCGA | SEQ ID NO: 9932 | GCGUAGU | SEQ ID NO: 9983 | GCGUUUG | SEQ ID NO: 10034 | GCUAUAC | SEQ ID NO: 10085 | GCUCGCA |
| SEQ ID NO: 9831 | GCGCGCG | SEQ ID NO: 9882 | GCGGCGC | SEQ ID NO: 9933 | GCGUAUA | SEQ ID NO: 9984 | GCGUUUU | SEQ ID NO: 10035 | GCUAUAG | SEQ ID NO: 10086 | GCUCGCC |
| SEQ ID NO: 9832 | GCGCGCU | SEQ ID NO: 9883 | GCGGCGG | SEQ ID NO: 9934 | GCGUAUC | SEQ ID NO: 9985 | GCUAAAA | SEQ ID NO: 10036 | GCUAUAU | SEQ ID NO: 10087 | GCUCGCG |
| SEQ ID NO: 9833 | GCGCGGA | SEQ ID NO: 9884 | GCGGCGU | SEQ ID NO: 9935 | GCGUAUG | SEQ ID NO: 9986 | GCUAAAC | SEQ ID NO: 10037 | GCUAUCA | SEQ ID NO: 10088 | GCUCGCU |
| SEQ ID NO: 9834 | GCGCGGC | SEQ ID NO: 9885 | GCGGCUA | SEQ ID NO: 9936 | GCGUAUU | SEQ ID NO: 9987 | GCUAAAG | SEQ ID NO: 10038 | GCUAUCC | SEQ ID NO: 10089 | GCUCGGA |
| SEQ ID NO: 9835 | GCGCGGG | SEQ ID NO: 9886 | GCGGCUC | SEQ ID NO: 9937 | GCGUCAA | SEQ ID NO: 9988 | GCUAAAU | SEQ ID NO: 10039 | GCUAUCG | SEQ ID NO: 10090 | GCUCGGC |
| SEQ ID NO: 9836 | GCGCGGU | SEQ ID NO: 9887 | GCGGCUG | SEQ ID NO: 9938 | GCGUCAC | SEQ ID NO: 9989 | GCUAACA | SEQ ID NO: 10040 | GCUAUCU | SEQ ID NO: 10091 | GCUCGGG |
| SEQ ID NO: 9837 | GCGCGUA | SEQ ID NO: 9888 | GCGGCUU | SEQ ID NO: 9939 | GCGUCAG | SEQ ID NO: 9990 | GCUAACC | SEQ ID NO: 10041 | GCUAUGA | SEQ ID NO: 10092 | GCUCGGU |
| SEQ ID NO: 9838 | GCGCGUC | SEQ ID NO: 9889 | GCGGGAA | SEQ ID NO: 9940 | GCGUCAU | SEQ ID NO: 9991 | GCUAACG | SEQ ID NO: 10042 | GCUAUGC | SEQ ID NO: 10093 | GCUCGUA |
| SEQ ID NO: 9839 | GCGCGUG | SEQ ID NO: 9890 | GCGGGAC | SEQ ID NO: 9941 | GCGUCCA | SEQ ID NO: 9992 | GCUAACU | SEQ ID NO: 10043 | GCUAUGG | SEQ ID NO: 10094 | GCUCGUC |
| SEQ ID NO: 9840 | GCGCGUU | SEQ ID NO: 9891 | GCGGGAG | SEQ ID NO: 9942 | GCGUCCC | SEQ ID NO: 9993 | GCUAAGA | SEQ ID NO: 10044 | GCUAUGU | SEQ ID NO: 10095 | GCUCGUG |
| SEQ ID NO: 9841 | GCGCUAA | SEQ ID NO: 9892 | GCGGGAU | SEQ ID NO: 9943 | GCGUCCG | SEQ ID NO: 9994 | GCUAAGC | SEQ ID NO: 10045 | GCUAUUA | SEQ ID NO: 10096 | GCUCGUU |
| SEQ ID NO: 9842 | GCGCUAC | SEQ ID NO: 9893 | GCGGGCA | SEQ ID NO: 9944 | GCGUCCU | SEQ ID NO: 9995 | GCUAAGG | SEQ ID NO: 10046 | GCUAUUC | SEQ ID NO: 10097 | GCUCUAA |
| SEQ ID NO: 9843 | GCGCUAG | SEQ ID NO: 9894 | GCGGGCC | SEQ ID NO: 9945 | GCGUCGA | SEQ ID NO: 9996 | GCUAAGU | SEQ ID NO: 10047 | GCUAUUG | SEQ ID NO: 10098 | GCUCUAC |

Table 34

| | | | | | | | | | | | |
|---|---|---|---|---|---|---|---|---|---|---|---|
| SEQ ID NO: 10099 | GCUCUAG | SEQ ID NO: 10150 | GCUGGCC | SEQ ID NO: 10201 | GCUUCGA | SEQ ID NO: 10252 | GGAAAGU | SEQ ID NO: 10303 | GGAAUUG | SEQ ID NO: 10354 | GGACUAC |
| SEQ ID NO: 10100 | GCUCUAU | SEQ ID NO: 10151 | GCUGGCG | SEQ ID NO: 10202 | GCUUCGC | SEQ ID NO: 10253 | GGAAAUA | SEQ ID NO: 10304 | GGAAUUU | SEQ ID NO: 10355 | GGACUAG |
| SEQ ID NO: 10101 | GCUCUCA | SEQ ID NO: 10152 | GCUGGCU | SEQ ID NO: 10203 | GCUUCGG | SEQ ID NO: 10254 | GGAAAUC | SEQ ID NO: 10305 | GGACAAA | SEQ ID NO: 10356 | GGACUAU |
| SEQ ID NO: 10102 | GCUCUCC | SEQ ID NO: 10153 | GCUGGGA | SEQ ID NO: 10204 | GCUUCGU | SEQ ID NO: 10255 | GGAAAUG | SEQ ID NO: 10306 | GGACAAC | SEQ ID NO: 10357 | GGACUCA |
| SEQ ID NO: 10103 | GCUCUCG | SEQ ID NO: 10154 | GCUGGGC | SEQ ID NO: 10205 | GCUUCUA | SEQ ID NO: 10256 | GGAAAUU | SEQ ID NO: 10307 | GGACAAG | SEQ ID NO: 10358 | GGACUCC |
| SEQ ID NO: 10104 | GCUCUCU | SEQ ID NO: 10155 | GCUGGGG | SEQ ID NO: 10206 | GCUUCUC | SEQ ID NO: 10257 | GGAACAA | SEQ ID NO: 10308 | GGACAAU | SEQ ID NO: 10359 | GGACUCG |
| SEQ ID NO: 10105 | GCUCUGA | SEQ ID NO: 10156 | GCUGGGU | SEQ ID NO: 10207 | GCUUCUG | SEQ ID NO: 10258 | GGAACAC | SEQ ID NO: 10309 | GGACACA | SEQ ID NO: 10360 | GGACUCU |
| SEQ ID NO: 10106 | GCUCUGC | SEQ ID NO: 10157 | GCUGGUA | SEQ ID NO: 10208 | GCUUCUU | SEQ ID NO: 10259 | GGAACAG | SEQ ID NO: 10310 | GGACACC | SEQ ID NO: 10361 | GGACUGA |
| SEQ ID NO: 10107 | GCUCUGG | SEQ ID NO: 10158 | GCUGGUC | SEQ ID NO: 10209 | GCUUGAA | SEQ ID NO: 10260 | GGAACAU | SEQ ID NO: 10311 | GGACACG | SEQ ID NO: 10362 | GGACUGC |
| SEQ ID NO: 10108 | GCUCUGU | SEQ ID NO: 10159 | GCUGGUG | SEQ ID NO: 10210 | GCUUGAC | SEQ ID NO: 10261 | GGAACCA | SEQ ID NO: 10312 | GGACACU | SEQ ID NO: 10363 | GGACUGG |
| SEQ ID NO: 10109 | GCUCUUA | SEQ ID NO: 10160 | GCUGGUU | SEQ ID NO: 10211 | GCUUGAG | SEQ ID NO: 10262 | GGAACCC | SEQ ID NO: 10313 | GGACAGA | SEQ ID NO: 10364 | GGACUGU |
| SEQ ID NO: 10110 | GCUCUUC | SEQ ID NO: 10161 | GCUGUAA | SEQ ID NO: 10212 | GCUUGAU | SEQ ID NO: 10263 | GGAACCG | SEQ ID NO: 10314 | GGACAGC | SEQ ID NO: 10365 | GGACUUA |
| SEQ ID NO: 10111 | GCUCUUG | SEQ ID NO: 10162 | GCUGUAC | SEQ ID NO: 10213 | GCUUGCA | SEQ ID NO: 10264 | GGAACCU | SEQ ID NO: 10315 | GGACAGG | SEQ ID NO: 10366 | GGACUUC |
| SEQ ID NO: 10112 | GCUCUUU | SEQ ID NO: 10163 | GCUGUAG | SEQ ID NO: 10214 | GCUUGCC | SEQ ID NO: 10265 | GGAACGA | SEQ ID NO: 10316 | GGACAGU | SEQ ID NO: 10367 | GGACUUG |
| SEQ ID NO: 10113 | GCUGAAA | SEQ ID NO: 10164 | GCUGUAU | SEQ ID NO: 10215 | GCUUGCG | SEQ ID NO: 10266 | GGAACGC | SEQ ID NO: 10317 | GGACAUA | SEQ ID NO: 10368 | GGACUUU |
| SEQ ID NO: 10114 | GCUGAAC | SEQ ID NO: 10165 | GCUGUCA | SEQ ID NO: 10216 | GCUUGCU | SEQ ID NO: 10267 | GGAACGG | SEQ ID NO: 10318 | GGACAUC | SEQ ID NO: 10369 | GGAGAAA |
| SEQ ID NO: 10115 | GCUGAAG | SEQ ID NO: 10166 | GCUGUCC | SEQ ID NO: 10217 | GCUUGGA | SEQ ID NO: 10268 | GGAACGU | SEQ ID NO: 10319 | GGACAUG | SEQ ID NO: 10370 | GGAGAAC |
| SEQ ID NO: 10116 | GCUGAAU | SEQ ID NO: 10167 | GCUGUCG | SEQ ID NO: 10218 | GCUUGGC | SEQ ID NO: 10269 | GGAACUA | SEQ ID NO: 10320 | GGACAUU | SEQ ID NO: 10371 | GGAGAAG |
| SEQ ID NO: 10117 | GCUGACA | SEQ ID NO: 10168 | GCUGUCU | SEQ ID NO: 10219 | GCUUGGG | SEQ ID NO: 10270 | GGAACUC | SEQ ID NO: 10321 | GGACCAA | SEQ ID NO: 10372 | GGAGAAU |
| SEQ ID NO: 10118 | GCUGACC | SEQ ID NO: 10169 | GCUGUGA | SEQ ID NO: 10220 | GCUUGGU | SEQ ID NO: 10271 | GGAACUG | SEQ ID NO: 10322 | GGACCAC | SEQ ID NO: 10373 | GGAGACA |
| SEQ ID NO: 10119 | GCUGACG | SEQ ID NO: 10170 | GCUGUGC | SEQ ID NO: 10221 | GCUUGUA | SEQ ID NO: 10272 | GGAACUU | SEQ ID NO: 10323 | GGACCAG | SEQ ID NO: 10374 | GGAGACC |
| SEQ ID NO: 10120 | GCUGACU | SEQ ID NO: 10171 | GCUGUGG | SEQ ID NO: 10222 | GCUUGUC | SEQ ID NO: 10273 | GGAAGAA | SEQ ID NO: 10324 | GGACCAU | SEQ ID NO: 10375 | GGAGACG |
| SEQ ID NO: 10121 | GCUGAGA | SEQ ID NO: 10172 | GCUGUGU | SEQ ID NO: 10223 | GCUUGUG | SEQ ID NO: 10274 | GGAAGAC | SEQ ID NO: 10325 | GGACCCA | SEQ ID NO: 10376 | GGAGACU |
| SEQ ID NO: 10122 | GCUGAGC | SEQ ID NO: 10173 | GCUGUUA | SEQ ID NO: 10224 | GCUUGUU | SEQ ID NO: 10275 | GGAAGAG | SEQ ID NO: 10326 | GGACCCC | SEQ ID NO: 10377 | GGAGAGA |
| SEQ ID NO: 10123 | GCUGAGG | SEQ ID NO: 10174 | GCUGUUC | SEQ ID NO: 10225 | GCUUUAA | SEQ ID NO: 10276 | GGAAGAU | SEQ ID NO: 10327 | GGACCCG | SEQ ID NO: 10378 | GGAGAGC |
| SEQ ID NO: 10124 | GCUGAGU | SEQ ID NO: 10175 | GCUGUUG | SEQ ID NO: 10226 | GCUUUAC | SEQ ID NO: 10277 | GGAAGCA | SEQ ID NO: 10328 | GGACCCU | SEQ ID NO: 10379 | GGAGAGG |
| SEQ ID NO: 10125 | GCUGAUA | SEQ ID NO: 10176 | GCUGUUU | SEQ ID NO: 10227 | GCUUUAG | SEQ ID NO: 10278 | GGAAGCC | SEQ ID NO: 10329 | GGACCGA | SEQ ID NO: 10380 | GGAGAGU |
| SEQ ID NO: 10126 | GCUGAUC | SEQ ID NO: 10177 | GCUUAAA | SEQ ID NO: 10228 | GCUUUAU | SEQ ID NO: 10279 | GGAAGCG | SEQ ID NO: 10330 | GGACCGC | SEQ ID NO: 10381 | GGAGAUA |
| SEQ ID NO: 10127 | GCUGAUG | SEQ ID NO: 10178 | GCUUAAC | SEQ ID NO: 10229 | GCUUUCA | SEQ ID NO: 10280 | GGAAGCU | SEQ ID NO: 10331 | GGACCGG | SEQ ID NO: 10382 | GGAGAUC |
| SEQ ID NO: 10128 | GCUGAUU | SEQ ID NO: 10179 | GCUUAAG | SEQ ID NO: 10230 | GCUUUCC | SEQ ID NO: 10281 | GGAAGGA | SEQ ID NO: 10332 | GGACCGU | SEQ ID NO: 10383 | GGAGAUG |
| SEQ ID NO: 10129 | GCUGCAA | SEQ ID NO: 10180 | GCUUAAU | SEQ ID NO: 10231 | GCUUUCG | SEQ ID NO: 10282 | GGAAGGC | SEQ ID NO: 10333 | GGACCUA | SEQ ID NO: 10384 | GGAGAUU |
| SEQ ID NO: 10130 | GCUGCAC | SEQ ID NO: 10181 | GCUUACA | SEQ ID NO: 10232 | GCUUUCU | SEQ ID NO: 10283 | GGAAGGG | SEQ ID NO: 10334 | GGACCUC | SEQ ID NO: 10385 | GGAGCAA |
| SEQ ID NO: 10131 | GCUGCAG | SEQ ID NO: 10182 | GCUUACC | SEQ ID NO: 10233 | GCUUUGA | SEQ ID NO: 10284 | GGAAGGU | SEQ ID NO: 10335 | GGACCUG | SEQ ID NO: 10386 | GGAGCAC |
| SEQ ID NO: 10132 | GCUGCAU | SEQ ID NO: 10183 | GCUUACG | SEQ ID NO: 10234 | GCUUUGC | SEQ ID NO: 10285 | GGAAGUA | SEQ ID NO: 10336 | GGACCUU | SEQ ID NO: 10387 | GGAGCAG |
| SEQ ID NO: 10133 | GCUGCCA | SEQ ID NO: 10184 | GCUUACU | SEQ ID NO: 10235 | GCUUUGG | SEQ ID NO: 10286 | GGAAGUC | SEQ ID NO: 10337 | GGACGAA | SEQ ID NO: 10388 | GGAGCAU |
| SEQ ID NO: 10134 | GCUGCCC | SEQ ID NO: 10185 | GCUUAGA | SEQ ID NO: 10236 | GCUUUGU | SEQ ID NO: 10287 | GGAAGUG | SEQ ID NO: 10338 | GGACGAC | SEQ ID NO: 10389 | GGAGCCA |
| SEQ ID NO: 10135 | GCUGCCG | SEQ ID NO: 10186 | GCUUAGC | SEQ ID NO: 10237 | GCUUUUA | SEQ ID NO: 10288 | GGAAGUU | SEQ ID NO: 10339 | GGACGAG | SEQ ID NO: 10390 | GGAGCCC |
| SEQ ID NO: 10136 | GCUGCCU | SEQ ID NO: 10187 | GCUUAGG | SEQ ID NO: 10238 | GCUUUUC | SEQ ID NO: 10289 | GGAAUAA | SEQ ID NO: 10340 | GGACGAU | SEQ ID NO: 10391 | GGAGCCG |
| SEQ ID NO: 10137 | GCUGCGA | SEQ ID NO: 10188 | GCUUAGU | SEQ ID NO: 10239 | GCUUUUG | SEQ ID NO: 10290 | GGAAUAC | SEQ ID NO: 10341 | GGACGCA | SEQ ID NO: 10392 | GGAGCCU |
| SEQ ID NO: 10138 | GCUGCGC | SEQ ID NO: 10189 | GCUUAUA | SEQ ID NO: 10240 | GCUUUUU | SEQ ID NO: 10291 | GGAAUAG | SEQ ID NO: 10342 | GGACGCC | SEQ ID NO: 10393 | GGAGCGA |
| SEQ ID NO: 10139 | GCUGCGG | SEQ ID NO: 10190 | GCUUAUC | SEQ ID NO: 10241 | GGAAAAA | SEQ ID NO: 10292 | GGAAUAU | SEQ ID NO: 10343 | GGACGCG | SEQ ID NO: 10394 | GGAGCGC |
| SEQ ID NO: 10140 | GCUGCGU | SEQ ID NO: 10191 | GCUUAUG | SEQ ID NO: 10242 | GGAAAAC | SEQ ID NO: 10293 | GGAAUCA | SEQ ID NO: 10344 | GGACGCU | SEQ ID NO: 10395 | GGAGCGG |
| SEQ ID NO: 10141 | GCUGCUA | SEQ ID NO: 10192 | GCUUAUU | SEQ ID NO: 10243 | GGAAAAG | SEQ ID NO: 10294 | GGAAUCC | SEQ ID NO: 10345 | GGACGGA | SEQ ID NO: 10396 | GGAGCGU |
| SEQ ID NO: 10142 | GCUGCUC | SEQ ID NO: 10193 | GCUUCAA | SEQ ID NO: 10244 | GGAAAAU | SEQ ID NO: 10295 | GGAAUCG | SEQ ID NO: 10346 | GGACGGC | SEQ ID NO: 10397 | GGAGCUA |
| SEQ ID NO: 10143 | GCUGCUG | SEQ ID NO: 10194 | GCUUCAC | SEQ ID NO: 10245 | GGAAACA | SEQ ID NO: 10296 | GGAAUCU | SEQ ID NO: 10347 | GGACGGG | SEQ ID NO: 10398 | GGAGCUC |
| SEQ ID NO: 10144 | GCUGCUU | SEQ ID NO: 10195 | GCUUCAG | SEQ ID NO: 10246 | GGAAACC | SEQ ID NO: 10297 | GGAAUGA | SEQ ID NO: 10348 | GGACGGU | SEQ ID NO: 10399 | GGAGCUG |
| SEQ ID NO: 10145 | GCUGGAA | SEQ ID NO: 10196 | GCUUCAU | SEQ ID NO: 10247 | GGAAACG | SEQ ID NO: 10298 | GGAAUGC | SEQ ID NO: 10349 | GGACGUA | SEQ ID NO: 10400 | GGAGCUU |
| SEQ ID NO: 10146 | GCUGGAC | SEQ ID NO: 10197 | GCUUCCA | SEQ ID NO: 10248 | GGAAACU | SEQ ID NO: 10299 | GGAAUGG | SEQ ID NO: 10350 | GGACGUC | SEQ ID NO: 10401 | GGAGGAA |
| SEQ ID NO: 10147 | GCUGGAG | SEQ ID NO: 10198 | GCUUCCC | SEQ ID NO: 10249 | GGAAAGA | SEQ ID NO: 10300 | GGAAUGU | SEQ ID NO: 10351 | GGACGUG | SEQ ID NO: 10402 | GGAGGAC |
| SEQ ID NO: 10148 | GCUGGAU | SEQ ID NO: 10199 | GCUUCCG | SEQ ID NO: 10250 | GGAAAGC | SEQ ID NO: 10301 | GGAAUUA | SEQ ID NO: 10352 | GGACGUU | SEQ ID NO: 10403 | GGAGGAG |
| SEQ ID NO: 10149 | GCUGGCA | SEQ ID NO: 10200 | GCUUCCU | SEQ ID NO: 10251 | GGAAAGG | SEQ ID NO: 10302 | GGAAUUC | SEQ ID NO: 10353 | GGACUAA | SEQ ID NO: 10404 | GGAGGAU |

Table 35

| | | | | | | | | | | | |
|---|---|---|---|---|---|---|---|---|---|---|---|
| SEQ ID NO: 10405 | GGAGGCA | SEQ ID NO: 10456 | GGAUCCU | SEQ ID NO: 10507 | GGCAAGG | SEQ ID NO: 10558 | GGCAUUC | SEQ ID NO: 10609 | GGCCUAA | SEQ ID NO: 10660 | GGCGGAU |
| SEQ ID NO: 10406 | GGAGGCC | SEQ ID NO: 10457 | GGAUCGA | SEQ ID NO: 10508 | GGCAAGU | SEQ ID NO: 10559 | GGCAUUG | SEQ ID NO: 10610 | GGCCUAC | SEQ ID NO: 10661 | GGCGGCA |
| SEQ ID NO: 10407 | GGAGGCG | SEQ ID NO: 10458 | GGAUCGC | SEQ ID NO: 10509 | GGCAAUA | SEQ ID NO: 10560 | GGCAUUU | SEQ ID NO: 10611 | GGCCUAG | SEQ ID NO: 10662 | GGCGGCC |
| SEQ ID NO: 10408 | GGAGGCU | SEQ ID NO: 10459 | GGAUCGG | SEQ ID NO: 10510 | GGCAAUC | SEQ ID NO: 10561 | GGCCAAA | SEQ ID NO: 10612 | GGCCUAU | SEQ ID NO: 10663 | GGCGGCG |
| SEQ ID NO: 10409 | GGAGGGA | SEQ ID NO: 10460 | GGAUCGU | SEQ ID NO: 10511 | GGCAAUG | SEQ ID NO: 10562 | GGCCAAC | SEQ ID NO: 10613 | GGCCUCA | SEQ ID NO: 10664 | GGCGGCU |
| SEQ ID NO: 10410 | GGAGGGC | SEQ ID NO: 10461 | GGAUCUA | SEQ ID NO: 10512 | GGCAAUU | SEQ ID NO: 10563 | GGCCAAG | SEQ ID NO: 10614 | GGCCUCC | SEQ ID NO: 10665 | GGCGGGA |
| SEQ ID NO: 10411 | GGAGGGG | SEQ ID NO: 10462 | GGAUCUC | SEQ ID NO: 10513 | GGCACAA | SEQ ID NO: 10564 | GGCCAAU | SEQ ID NO: 10615 | GGCCUCG | SEQ ID NO: 10666 | GGCGGGC |
| SEQ ID NO: 10412 | GGAGGGU | SEQ ID NO: 10463 | GGAUCUG | SEQ ID NO: 10514 | GGCACAC | SEQ ID NO: 10565 | GGCCACA | SEQ ID NO: 10616 | GGCCUCU | SEQ ID NO: 10667 | GGCGGGG |
| SEQ ID NO: 10413 | GGAGGUA | SEQ ID NO: 10464 | GGAUCUU | SEQ ID NO: 10515 | GGCACAG | SEQ ID NO: 10566 | GGCCACC | SEQ ID NO: 10617 | GGCCUGA | SEQ ID NO: 10668 | GGCGGGU |
| SEQ ID NO: 10414 | GGAGGUC | SEQ ID NO: 10465 | GGAUGAA | SEQ ID NO: 10516 | GGCACAU | SEQ ID NO: 10567 | GGCCACG | SEQ ID NO: 10618 | GGCCUGC | SEQ ID NO: 10669 | GGCGGUA |
| SEQ ID NO: 10415 | GGAGGUG | SEQ ID NO: 10466 | GGAUGAC | SEQ ID NO: 10517 | GGCACCA | SEQ ID NO: 10568 | GGCCACU | SEQ ID NO: 10619 | GGCCUGG | SEQ ID NO: 10670 | GGCGGUC |
| SEQ ID NO: 10416 | GGAGGUU | SEQ ID NO: 10467 | GGAUGAG | SEQ ID NO: 10518 | GGCACCC | SEQ ID NO: 10569 | GGCCAGA | SEQ ID NO: 10620 | GGCCUGU | SEQ ID NO: 10671 | GGCGGUG |
| SEQ ID NO: 10417 | GGAGUAA | SEQ ID NO: 10468 | GGAUGAU | SEQ ID NO: 10519 | GGCACCG | SEQ ID NO: 10570 | GGCCAGC | SEQ ID NO: 10621 | GGCCUUA | SEQ ID NO: 10672 | GGCGGUU |
| SEQ ID NO: 10418 | GGAGUAC | SEQ ID NO: 10469 | GGAUGCA | SEQ ID NO: 10520 | GGCACCU | SEQ ID NO: 10571 | GGCCAGG | SEQ ID NO: 10622 | GGCCUUC | SEQ ID NO: 10673 | GGCGUAA |
| SEQ ID NO: 10419 | GGAGUAG | SEQ ID NO: 10470 | GGAUGCC | SEQ ID NO: 10521 | GGCACGA | SEQ ID NO: 10572 | GGCCAGU | SEQ ID NO: 10623 | GGCCUUG | SEQ ID NO: 10674 | GGCGUAC |
| SEQ ID NO: 10420 | GGAGUAU | SEQ ID NO: 10471 | GGAUGCG | SEQ ID NO: 10522 | GGCACGC | SEQ ID NO: 10573 | GGCCAUA | SEQ ID NO: 10624 | GGCCUUU | SEQ ID NO: 10675 | GGCGUAG |
| SEQ ID NO: 10421 | GGAGUCA | SEQ ID NO: 10472 | GGAUGCU | SEQ ID NO: 10523 | GGCACGG | SEQ ID NO: 10574 | GGCCAUC | SEQ ID NO: 10625 | GGCGAAA | SEQ ID NO: 10676 | GGCGUAU |
| SEQ ID NO: 10422 | GGAGUCC | SEQ ID NO: 10473 | GGAUGGA | SEQ ID NO: 10524 | GGCACGU | SEQ ID NO: 10575 | GGCCAUG | SEQ ID NO: 10626 | GGCGAAC | SEQ ID NO: 10677 | GGCGUCA |
| SEQ ID NO: 10423 | GGAGUCG | SEQ ID NO: 10474 | GGAUGGC | SEQ ID NO: 10525 | GGCACUA | SEQ ID NO: 10576 | GGCCAUU | SEQ ID NO: 10627 | GGCGAAG | SEQ ID NO: 10678 | GGCGUCC |
| SEQ ID NO: 10424 | GGAGUCU | SEQ ID NO: 10475 | GGAUGGG | SEQ ID NO: 10526 | GGCACUC | SEQ ID NO: 10577 | GGCCCAA | SEQ ID NO: 10628 | GGCGAAU | SEQ ID NO: 10679 | GGCGUCG |
| SEQ ID NO: 10425 | GGAGUGA | SEQ ID NO: 10476 | GGAUGGU | SEQ ID NO: 10527 | GGCACUG | SEQ ID NO: 10578 | GGCCCAC | SEQ ID NO: 10629 | GGCGACA | SEQ ID NO: 10680 | GGCGUCU |
| SEQ ID NO: 10426 | GGAGUGC | SEQ ID NO: 10477 | GGAUGUA | SEQ ID NO: 10528 | GGCACUU | SEQ ID NO: 10579 | GGCCCAG | SEQ ID NO: 10630 | GGCGACC | SEQ ID NO: 10681 | GGCGUGA |
| SEQ ID NO: 10427 | GGAGUGG | SEQ ID NO: 10478 | GGAUGUC | SEQ ID NO: 10529 | GGCAGAA | SEQ ID NO: 10580 | GGCCCAU | SEQ ID NO: 10631 | GGCGACG | SEQ ID NO: 10682 | GGCGUGC |
| SEQ ID NO: 10428 | GGAGUGU | SEQ ID NO: 10479 | GGAUGUG | SEQ ID NO: 10530 | GGCAGAC | SEQ ID NO: 10581 | GGCCCCA | SEQ ID NO: 10632 | GGCGACU | SEQ ID NO: 10683 | GGCGUGG |
| SEQ ID NO: 10429 | GGAGUUA | SEQ ID NO: 10480 | GGAUGUU | SEQ ID NO: 10531 | GGCAGAG | SEQ ID NO: 10582 | GGCCCCC | SEQ ID NO: 10633 | GGCGAGA | SEQ ID NO: 10684 | GGCGUGU |
| SEQ ID NO: 10430 | GGAGUUC | SEQ ID NO: 10481 | GGAUUAA | SEQ ID NO: 10532 | GGCAGAU | SEQ ID NO: 10583 | GGCCCCG | SEQ ID NO: 10634 | GGCGAGC | SEQ ID NO: 10685 | GGCGUUA |
| SEQ ID NO: 10431 | GGAGUUG | SEQ ID NO: 10482 | GGAUUAC | SEQ ID NO: 10533 | GGCAGCA | SEQ ID NO: 10584 | GGCCCCU | SEQ ID NO: 10635 | GGCGAGG | SEQ ID NO: 10686 | GGCGUUC |
| SEQ ID NO: 10432 | GGAGUUU | SEQ ID NO: 10483 | GGAUUAG | SEQ ID NO: 10534 | GGCAGCC | SEQ ID NO: 10585 | GGCCCGA | SEQ ID NO: 10636 | GGCGAGU | SEQ ID NO: 10687 | GGCGUUG |
| SEQ ID NO: 10433 | GGAUAAA | SEQ ID NO: 10484 | GGAUUAU | SEQ ID NO: 10535 | GGCAGCG | SEQ ID NO: 10586 | GGCCCGC | SEQ ID NO: 10637 | GGCGAUA | SEQ ID NO: 10688 | GGCGUUU |
| SEQ ID NO: 10434 | GGAUAAC | SEQ ID NO: 10485 | GGAUUCA | SEQ ID NO: 10536 | GGCAGCU | SEQ ID NO: 10587 | GGCCCGG | SEQ ID NO: 10638 | GGCGAUC | SEQ ID NO: 10689 | GGCUAAA |
| SEQ ID NO: 10435 | GGAUAAG | SEQ ID NO: 10486 | GGAUUCC | SEQ ID NO: 10537 | GGCAGGA | SEQ ID NO: 10588 | GGCCCGU | SEQ ID NO: 10639 | GGCGAUG | SEQ ID NO: 10690 | GGCUAAC |
| SEQ ID NO: 10436 | GGAUAAU | SEQ ID NO: 10487 | GGAUUCG | SEQ ID NO: 10538 | GGCAGGC | SEQ ID NO: 10589 | GGCCCUA | SEQ ID NO: 10640 | GGCGAUU | SEQ ID NO: 10691 | GGCUAAG |
| SEQ ID NO: 10437 | GGAUACA | SEQ ID NO: 10488 | GGAUUCU | SEQ ID NO: 10539 | GGCAGGG | SEQ ID NO: 10590 | GGCCCUC | SEQ ID NO: 10641 | GGCGCAA | SEQ ID NO: 10692 | GGCUAAU |
| SEQ ID NO: 10438 | GGAUACC | SEQ ID NO: 10489 | GGAUUGA | SEQ ID NO: 10540 | GGCAGGU | SEQ ID NO: 10591 | GGCCCUG | SEQ ID NO: 10642 | GGCGCAC | SEQ ID NO: 10693 | GGCUACA |
| SEQ ID NO: 10439 | GGAUACG | SEQ ID NO: 10490 | GGAUUGC | SEQ ID NO: 10541 | GGCAGUA | SEQ ID NO: 10592 | GGCCCUU | SEQ ID NO: 10643 | GGCGCAG | SEQ ID NO: 10694 | GGCUACC |
| SEQ ID NO: 10440 | GGAUACU | SEQ ID NO: 10491 | GGAUUGG | SEQ ID NO: 10542 | GGCAGUC | SEQ ID NO: 10593 | GGCCGAA | SEQ ID NO: 10644 | GGCGCAU | SEQ ID NO: 10695 | GGCUACG |
| SEQ ID NO: 10441 | GGAUAGA | SEQ ID NO: 10492 | GGAUUGU | SEQ ID NO: 10543 | GGCAGUG | SEQ ID NO: 10594 | GGCCGAC | SEQ ID NO: 10645 | GGCGCCA | SEQ ID NO: 10696 | GGCUACU |
| SEQ ID NO: 10442 | GGAUAGC | SEQ ID NO: 10493 | GGAUUUA | SEQ ID NO: 10544 | GGCAGUU | SEQ ID NO: 10595 | GGCCGAG | SEQ ID NO: 10646 | GGCGCCC | SEQ ID NO: 10697 | GGCUAGA |
| SEQ ID NO: 10443 | GGAUAGG | SEQ ID NO: 10494 | GGAUUUC | SEQ ID NO: 10545 | GGCAUAA | SEQ ID NO: 10596 | GGCCGAU | SEQ ID NO: 10647 | GGCGCCG | SEQ ID NO: 10698 | GGCUAGC |
| SEQ ID NO: 10444 | GGAUAGU | SEQ ID NO: 10495 | GGAUUUG | SEQ ID NO: 10546 | GGCAUAC | SEQ ID NO: 10597 | GGCCGCA | SEQ ID NO: 10648 | GGCGCCU | SEQ ID NO: 10699 | GGCUAGG |
| SEQ ID NO: 10445 | GGAUAUA | SEQ ID NO: 10496 | GGAUUUU | SEQ ID NO: 10547 | GGCAUAG | SEQ ID NO: 10598 | GGCCGCC | SEQ ID NO: 10649 | GGCGCGA | SEQ ID NO: 10700 | GGCUAGU |
| SEQ ID NO: 10446 | GGAUAUC | SEQ ID NO: 10497 | GGCAAAA | SEQ ID NO: 10548 | GGCAUAU | SEQ ID NO: 10599 | GGCCGCG | SEQ ID NO: 10650 | GGCGCGC | SEQ ID NO: 10701 | GGCUAUA |
| SEQ ID NO: 10447 | GGAUAUG | SEQ ID NO: 10498 | GGCAAAC | SEQ ID NO: 10549 | GGCAUCA | SEQ ID NO: 10600 | GGCCGCU | SEQ ID NO: 10651 | GGCGCGG | SEQ ID NO: 10702 | GGCUAUC |
| SEQ ID NO: 10448 | GGAUAUU | SEQ ID NO: 10499 | GGCAAAG | SEQ ID NO: 10550 | GGCAUCC | SEQ ID NO: 10601 | GGCCGGA | SEQ ID NO: 10652 | GGCGCGU | SEQ ID NO: 10703 | GGCUAUG |
| SEQ ID NO: 10449 | GGAUCAA | SEQ ID NO: 10500 | GGCAAAU | SEQ ID NO: 10551 | GGCAUCG | SEQ ID NO: 10602 | GGCCGGC | SEQ ID NO: 10653 | GGCGCUA | SEQ ID NO: 10704 | GGCUAUU |
| SEQ ID NO: 10450 | GGAUCAC | SEQ ID NO: 10501 | GGCAACA | SEQ ID NO: 10552 | GGCAUCU | SEQ ID NO: 10603 | GGCCGGG | SEQ ID NO: 10654 | GGCGCUC | SEQ ID NO: 10705 | GGCUCAA |
| SEQ ID NO: 10451 | GGAUCAG | SEQ ID NO: 10502 | GGCAACC | SEQ ID NO: 10553 | GGCAUGA | SEQ ID NO: 10604 | GGCCGGU | SEQ ID NO: 10655 | GGCGCUG | SEQ ID NO: 10706 | GGCUCAC |
| SEQ ID NO: 10452 | GGAUCAU | SEQ ID NO: 10503 | GGCAACG | SEQ ID NO: 10554 | GGCAUGC | SEQ ID NO: 10605 | GGCCGUA | SEQ ID NO: 10656 | GGCGCUU | SEQ ID NO: 10707 | GGCUCAG |
| SEQ ID NO: 10453 | GGAUCCA | SEQ ID NO: 10504 | GGCAACU | SEQ ID NO: 10555 | GGCAUGG | SEQ ID NO: 10606 | GGCCGUC | SEQ ID NO: 10657 | GGCGGAA | SEQ ID NO: 10708 | GGCUCAU |
| SEQ ID NO: 10454 | GGAUCCC | SEQ ID NO: 10505 | GGCAAGA | SEQ ID NO: 10556 | GGCAUGU | SEQ ID NO: 10607 | GGCCGUG | SEQ ID NO: 10658 | GGCGGAC | SEQ ID NO: 10709 | GGCUCCA |
| SEQ ID NO: 10455 | GGAUCCG | SEQ ID NO: 10506 | GGCAAGC | SEQ ID NO: 10557 | GGCAUUA | SEQ ID NO: 10608 | GGCCGUU | SEQ ID NO: 10659 | GGCGGAG | SEQ ID NO: 10710 | GGCUCCC |

Table 36

| | | | | | | | | | | | |
|---|---|---|---|---|---|---|---|---|---|---|---|
| SEQ ID NO: 10711 | GGCUCCG | SEQ ID NO: 10762 | GGGAAGC | SEQ ID NO: 10813 | GGGAUUA | SEQ ID NO: 10864 | GGGCGUU | SEQ ID NO: 10915 | GGGGGAG | SEQ ID NO: 10966 | GGGUCCC |
| SEQ ID NO: 10712 | GGCUCCU | SEQ ID NO: 10763 | GGGAAGG | SEQ ID NO: 10814 | GGGAUUC | SEQ ID NO: 10865 | GGGCUAA | SEQ ID NO: 10916 | GGGGGAU | SEQ ID NO: 10967 | GGGUCCG |
| SEQ ID NO: 10713 | GGCUCGA | SEQ ID NO: 10764 | GGGAAGU | SEQ ID NO: 10815 | GGGAUUG | SEQ ID NO: 10866 | GGGCUAC | SEQ ID NO: 10917 | GGGGGCA | SEQ ID NO: 10968 | GGGUCCU |
| SEQ ID NO: 10714 | GGCUCGC | SEQ ID NO: 10765 | GGGAAUA | SEQ ID NO: 10816 | GGGAUUU | SEQ ID NO: 10867 | GGGCUAG | SEQ ID NO: 10918 | GGGGGCC | SEQ ID NO: 10969 | GGGUCGA |
| SEQ ID NO: 10715 | GGCUCGG | SEQ ID NO: 10766 | GGGAAUC | SEQ ID NO: 10817 | GGGCAAA | SEQ ID NO: 10868 | GGGCUAU | SEQ ID NO: 10919 | GGGGGCG | SEQ ID NO: 10970 | GGGUCGC |
| SEQ ID NO: 10716 | GGCUCGU | SEQ ID NO: 10767 | GGGAAUG | SEQ ID NO: 10818 | GGGCAAC | SEQ ID NO: 10869 | GGGCUCA | SEQ ID NO: 10920 | GGGGGCU | SEQ ID NO: 10971 | GGGUCGG |
| SEQ ID NO: 10717 | GGCUCUA | SEQ ID NO: 10768 | GGGAAUU | SEQ ID NO: 10819 | GGGCAAG | SEQ ID NO: 10870 | GGGCUCC | SEQ ID NO: 10921 | GGGGGGA | SEQ ID NO: 10972 | GGGUCGU |
| SEQ ID NO: 10718 | GGCUCUC | SEQ ID NO: 10769 | GGGACAA | SEQ ID NO: 10820 | GGGCAAU | SEQ ID NO: 10871 | GGGCUCG | SEQ ID NO: 10922 | GGGGGGC | SEQ ID NO: 10973 | GGGUCUA |
| SEQ ID NO: 10719 | GGCUCUG | SEQ ID NO: 10770 | GGGACAC | SEQ ID NO: 10821 | GGGCACA | SEQ ID NO: 10872 | GGGCUCU | SEQ ID NO: 10923 | GGGGGGG | SEQ ID NO: 10974 | GGGUCUC |
| SEQ ID NO: 10720 | GGCUCUU | SEQ ID NO: 10771 | GGGACAG | SEQ ID NO: 10822 | GGGCACC | SEQ ID NO: 10873 | GGGCUGA | SEQ ID NO: 10924 | GGGGGGU | SEQ ID NO: 10975 | GGGUCUG |
| SEQ ID NO: 10721 | GGCUGAA | SEQ ID NO: 10772 | GGGACAU | SEQ ID NO: 10823 | GGGCACG | SEQ ID NO: 10874 | GGGCUGC | SEQ ID NO: 10925 | GGGGGUA | SEQ ID NO: 10976 | GGGUCUU |
| SEQ ID NO: 10722 | GGCUGAC | SEQ ID NO: 10773 | GGGACCA | SEQ ID NO: 10824 | GGGCACU | SEQ ID NO: 10875 | GGGCUGG | SEQ ID NO: 10926 | GGGGGUC | SEQ ID NO: 10977 | GGGUGAA |
| SEQ ID NO: 10723 | GGCUGAG | SEQ ID NO: 10774 | GGGACCC | SEQ ID NO: 10825 | GGGCAGA | SEQ ID NO: 10876 | GGGCUGU | SEQ ID NO: 10927 | GGGGGUG | SEQ ID NO: 10978 | GGGUGAC |
| SEQ ID NO: 10724 | GGCUGAU | SEQ ID NO: 10775 | GGGACCG | SEQ ID NO: 10826 | GGGCAGC | SEQ ID NO: 10877 | GGGCUUA | SEQ ID NO: 10928 | GGGGGUU | SEQ ID NO: 10979 | GGGUGAG |
| SEQ ID NO: 10725 | GGCUGCA | SEQ ID NO: 10776 | GGGACCU | SEQ ID NO: 10827 | GGGCAGG | SEQ ID NO: 10878 | GGGCUUC | SEQ ID NO: 10929 | GGGGUAA | SEQ ID NO: 10980 | GGGUGAU |
| SEQ ID NO: 10726 | GGCUGCC | SEQ ID NO: 10777 | GGGACGA | SEQ ID NO: 10828 | GGGCAGU | SEQ ID NO: 10879 | GGGCUUG | SEQ ID NO: 10930 | GGGGUAC | SEQ ID NO: 10981 | GGGUGCA |
| SEQ ID NO: 10727 | GGCUGCG | SEQ ID NO: 10778 | GGGACGC | SEQ ID NO: 10829 | GGGCAUA | SEQ ID NO: 10880 | GGGCUUU | SEQ ID NO: 10931 | GGGGUAG | SEQ ID NO: 10982 | GGGUGCC |
| SEQ ID NO: 10728 | GGCUGCU | SEQ ID NO: 10779 | GGGACGG | SEQ ID NO: 10830 | GGGCAUC | SEQ ID NO: 10881 | GGGGAAA | SEQ ID NO: 10932 | GGGGUAU | SEQ ID NO: 10983 | GGGUGCG |
| SEQ ID NO: 10729 | GGCUGGA | SEQ ID NO: 10780 | GGGACGU | SEQ ID NO: 10831 | GGGCAUG | SEQ ID NO: 10882 | GGGGAAC | SEQ ID NO: 10933 | GGGGUCA | SEQ ID NO: 10984 | GGGUGCU |
| SEQ ID NO: 10730 | GGCUGGC | SEQ ID NO: 10781 | GGGACUA | SEQ ID NO: 10832 | GGGCAUU | SEQ ID NO: 10883 | GGGGAAG | SEQ ID NO: 10934 | GGGGUCC | SEQ ID NO: 10985 | GGGUGGA |
| SEQ ID NO: 10731 | GGCUGGG | SEQ ID NO: 10782 | GGGACUC | SEQ ID NO: 10833 | GGGCCAA | SEQ ID NO: 10884 | GGGGAAU | SEQ ID NO: 10935 | GGGGUCG | SEQ ID NO: 10986 | GGGUGGC |
| SEQ ID NO: 10732 | GGCUGGU | SEQ ID NO: 10783 | GGGACUG | SEQ ID NO: 10834 | GGGCCAC | SEQ ID NO: 10885 | GGGGACA | SEQ ID NO: 10936 | GGGGUCU | SEQ ID NO: 10987 | GGGUGGG |
| SEQ ID NO: 10733 | GGCUGUA | SEQ ID NO: 10784 | GGGACUU | SEQ ID NO: 10835 | GGGCCAG | SEQ ID NO: 10886 | GGGGACC | SEQ ID NO: 10937 | GGGGUGA | SEQ ID NO: 10988 | GGGUGGU |
| SEQ ID NO: 10734 | GGCUGUC | SEQ ID NO: 10785 | GGGAGAA | SEQ ID NO: 10836 | GGGCCAU | SEQ ID NO: 10887 | GGGGACG | SEQ ID NO: 10938 | GGGGUGC | SEQ ID NO: 10989 | GGGUGUA |
| SEQ ID NO: 10735 | GGCUGUG | SEQ ID NO: 10786 | GGGAGAC | SEQ ID NO: 10837 | GGGCCCA | SEQ ID NO: 10888 | GGGGACU | SEQ ID NO: 10939 | GGGGUGG | SEQ ID NO: 10990 | GGGUGUC |
| SEQ ID NO: 10736 | GGCUGUU | SEQ ID NO: 10787 | GGGAGAG | SEQ ID NO: 10838 | GGGCCCC | SEQ ID NO: 10889 | GGGGAGA | SEQ ID NO: 10940 | GGGGUGU | SEQ ID NO: 10991 | GGGUGUG |
| SEQ ID NO: 10737 | GGCUUAA | SEQ ID NO: 10788 | GGGAGAU | SEQ ID NO: 10839 | GGGCCCG | SEQ ID NO: 10890 | GGGGAGC | SEQ ID NO: 10941 | GGGGUUA | SEQ ID NO: 10992 | GGGUGUU |
| SEQ ID NO: 10738 | GGCUUAC | SEQ ID NO: 10789 | GGGAGCA | SEQ ID NO: 10840 | GGGCCCU | SEQ ID NO: 10891 | GGGGAGG | SEQ ID NO: 10942 | GGGGUUC | SEQ ID NO: 10993 | GGGUUAA |
| SEQ ID NO: 10739 | GGCUUAG | SEQ ID NO: 10790 | GGGAGCC | SEQ ID NO: 10841 | GGGCCGA | SEQ ID NO: 10892 | GGGGAGU | SEQ ID NO: 10943 | GGGGUUG | SEQ ID NO: 10994 | GGGUUAC |
| SEQ ID NO: 10740 | GGCUUAU | SEQ ID NO: 10791 | GGGAGCG | SEQ ID NO: 10842 | GGGCCGC | SEQ ID NO: 10893 | GGGGAUA | SEQ ID NO: 10944 | GGGGUUU | SEQ ID NO: 10995 | GGGUUAG |
| SEQ ID NO: 10741 | GGCUUCA | SEQ ID NO: 10792 | GGGAGCU | SEQ ID NO: 10843 | GGGCCGG | SEQ ID NO: 10894 | GGGGAUC | SEQ ID NO: 10945 | GGGUAAA | SEQ ID NO: 10996 | GGGUUAU |
| SEQ ID NO: 10742 | GGCUUCC | SEQ ID NO: 10793 | GGGAGGA | SEQ ID NO: 10844 | GGGCCGU | SEQ ID NO: 10895 | GGGGAUG | SEQ ID NO: 10946 | GGGUAAC | SEQ ID NO: 10997 | GGGUUCA |
| SEQ ID NO: 10743 | GGCUUCG | SEQ ID NO: 10794 | GGGAGGC | SEQ ID NO: 10845 | GGGCCUA | SEQ ID NO: 10896 | GGGGAUU | SEQ ID NO: 10947 | GGGUAAG | SEQ ID NO: 10998 | GGGUUCC |
| SEQ ID NO: 10744 | GGCUUCU | SEQ ID NO: 10795 | GGGAGGG | SEQ ID NO: 10846 | GGGCCUC | SEQ ID NO: 10897 | GGGGCAA | SEQ ID NO: 10948 | GGGUAAU | SEQ ID NO: 10999 | GGGUUCG |
| SEQ ID NO: 10745 | GGCUUGA | SEQ ID NO: 10796 | GGGAGGU | SEQ ID NO: 10847 | GGGCCUG | SEQ ID NO: 10898 | GGGGCAC | SEQ ID NO: 10949 | GGGUACA | SEQ ID NO: 11000 | GGGUUCU |
| SEQ ID NO: 10746 | GGCUUGC | SEQ ID NO: 10797 | GGGAGUA | SEQ ID NO: 10848 | GGGCCUU | SEQ ID NO: 10899 | GGGGCAG | SEQ ID NO: 10950 | GGGUACC | SEQ ID NO: 11001 | GGGUUGA |
| SEQ ID NO: 10747 | GGCUUGG | SEQ ID NO: 10798 | GGGAGUC | SEQ ID NO: 10849 | GGGCGAA | SEQ ID NO: 10900 | GGGGCAU | SEQ ID NO: 10951 | GGGUACG | SEQ ID NO: 11002 | GGGUUGC |
| SEQ ID NO: 10748 | GGCUUGU | SEQ ID NO: 10799 | GGGAGUG | SEQ ID NO: 10850 | GGGCGAC | SEQ ID NO: 10901 | GGGGCCA | SEQ ID NO: 10952 | GGGUACU | SEQ ID NO: 11003 | GGGUUGG |
| SEQ ID NO: 10749 | GGCUUUA | SEQ ID NO: 10800 | GGGAGUU | SEQ ID NO: 10851 | GGGCGAG | SEQ ID NO: 10902 | GGGGCCC | SEQ ID NO: 10953 | GGGUAGA | SEQ ID NO: 11004 | GGGUUGU |
| SEQ ID NO: 10750 | GGCUUUC | SEQ ID NO: 10801 | GGGAUAA | SEQ ID NO: 10852 | GGGCGAU | SEQ ID NO: 10903 | GGGGCCG | SEQ ID NO: 10954 | GGGUAGC | SEQ ID NO: 11005 | GGGUUUA |
| SEQ ID NO: 10751 | GGCUUUG | SEQ ID NO: 10802 | GGGAUAC | SEQ ID NO: 10853 | GGGCGCA | SEQ ID NO: 10904 | GGGGCCU | SEQ ID NO: 10955 | GGGUAGG | SEQ ID NO: 11006 | GGGUUUC |
| SEQ ID NO: 10752 | GGCUUUU | SEQ ID NO: 10803 | GGGAUAG | SEQ ID NO: 10854 | GGGCGCC | SEQ ID NO: 10905 | GGGGCGA | SEQ ID NO: 10956 | GGGUAGU | SEQ ID NO: 11007 | GGGUUUG |
| SEQ ID NO: 10753 | GGGAAAA | SEQ ID NO: 10804 | GGGAUAU | SEQ ID NO: 10855 | GGGCGCG | SEQ ID NO: 10906 | GGGGCGC | SEQ ID NO: 10957 | GGGUAUA | SEQ ID NO: 11008 | GGGUUUU |
| SEQ ID NO: 10754 | GGGAAAC | SEQ ID NO: 10805 | GGGAUCA | SEQ ID NO: 10856 | GGGCGCU | SEQ ID NO: 10907 | GGGGCGG | SEQ ID NO: 10958 | GGGUAUC | SEQ ID NO: 11009 | GGUAAAA |
| SEQ ID NO: 10755 | GGGAAAG | SEQ ID NO: 10806 | GGGAUCC | SEQ ID NO: 10857 | GGGCGGA | SEQ ID NO: 10908 | GGGGCGU | SEQ ID NO: 10959 | GGGUAUG | SEQ ID NO: 11010 | GGUAAAC |
| SEQ ID NO: 10756 | GGGAAAU | SEQ ID NO: 10807 | GGGAUCG | SEQ ID NO: 10858 | GGGCGGC | SEQ ID NO: 10909 | GGGGCUA | SEQ ID NO: 10960 | GGGUAUU | SEQ ID NO: 11011 | GGUAAAG |
| SEQ ID NO: 10757 | GGGAACA | SEQ ID NO: 10808 | GGGAUCU | SEQ ID NO: 10859 | GGGCGGG | SEQ ID NO: 10910 | GGGGCUC | SEQ ID NO: 10961 | GGGUCAA | SEQ ID NO: 11012 | GGUAAAU |
| SEQ ID NO: 10758 | GGGAACC | SEQ ID NO: 10809 | GGGAUGA | SEQ ID NO: 10860 | GGGCGGU | SEQ ID NO: 10911 | GGGGCUG | SEQ ID NO: 10962 | GGGUCAC | SEQ ID NO: 11013 | GGUAACA |
| SEQ ID NO: 10759 | GGGAACG | SEQ ID NO: 10810 | GGGAUGC | SEQ ID NO: 10861 | GGGCGUA | SEQ ID NO: 10912 | GGGGCUU | SEQ ID NO: 10963 | GGGUCAG | SEQ ID NO: 11014 | GGUAACC |
| SEQ ID NO: 10760 | GGGAACU | SEQ ID NO: 10811 | GGGAUGG | SEQ ID NO: 10862 | GGGCGUC | SEQ ID NO: 10913 | GGGGGAA | SEQ ID NO: 10964 | GGGUCAU | SEQ ID NO: 11015 | GGUAACG |
| SEQ ID NO: 10761 | GGGAAGA | SEQ ID NO: 10812 | GGGAUGU | SEQ ID NO: 10863 | GGGCGUG | SEQ ID NO: 10914 | GGGGGAC | SEQ ID NO: 10965 | GGGUCCA | SEQ ID NO: 11016 | GGUAACU |

Table 37

| | | | | | | | | | | | |
|---|---|---|---|---|---|---|---|---|---|---|---|
| SEQ ID NO: 11017 | GGUAAGA | SEQ ID NO: 11068 | GGUAUGU | SEQ ID NO: 11119 | GGUCGUG | SEQ ID NO: 11170 | GGUGGAC | SEQ ID NO: 11221 | GGUUCCA | SEQ ID NO: 11272 | GUAAACU |
| SEQ ID NO: 11018 | GGUAAGC | SEQ ID NO: 11069 | GGUAUUA | SEQ ID NO: 11120 | GGUCGUU | SEQ ID NO: 11171 | GGUGGAG | SEQ ID NO: 11222 | GGUUCCC | SEQ ID NO: 11273 | GUAAAGA |
| SEQ ID NO: 11019 | GGUAAGG | SEQ ID NO: 11070 | GGUAUUC | SEQ ID NO: 11121 | GGUCUAA | SEQ ID NO: 11172 | GGUGGAU | SEQ ID NO: 11223 | GGUUCCG | SEQ ID NO: 11274 | GUAAAGC |
| SEQ ID NO: 11020 | GGUAAGU | SEQ ID NO: 11071 | GGUAUUG | SEQ ID NO: 11122 | GGUCUAC | SEQ ID NO: 11173 | GGUGGCA | SEQ ID NO: 11224 | GGUUCCU | SEQ ID NO: 11275 | GUAAAGG |
| SEQ ID NO: 11021 | GGUAAUA | SEQ ID NO: 11072 | GGUAUUU | SEQ ID NO: 11123 | GGUCUAG | SEQ ID NO: 11174 | GGUGGCC | SEQ ID NO: 11225 | GGUUCGA | SEQ ID NO: 11276 | GUAAAGU |
| SEQ ID NO: 11022 | GGUAAUC | SEQ ID NO: 11073 | GGUCAAA | SEQ ID NO: 11124 | GGUCUAU | SEQ ID NO: 11175 | GGUGGCG | SEQ ID NO: 11226 | GGUUCGC | SEQ ID NO: 11277 | GUAAAUA |
| SEQ ID NO: 11023 | GGUAAUG | SEQ ID NO: 11074 | GGUCAAC | SEQ ID NO: 11125 | GGUCUCA | SEQ ID NO: 11176 | GGUGGCU | SEQ ID NO: 11227 | GGUUCGG | SEQ ID NO: 11278 | GUAAAUC |
| SEQ ID NO: 11024 | GGUAAUU | SEQ ID NO: 11075 | GGUCAAG | SEQ ID NO: 11126 | GGUCUCC | SEQ ID NO: 11177 | GGUGGGA | SEQ ID NO: 11228 | GGUUCGU | SEQ ID NO: 11279 | GUAAAUG |
| SEQ ID NO: 11025 | GGUACAA | SEQ ID NO: 11076 | GGUCAAU | SEQ ID NO: 11127 | GGUCUCG | SEQ ID NO: 11178 | GGUGGGC | SEQ ID NO: 11229 | GGUUCUA | SEQ ID NO: 11280 | GUAAAUU |
| SEQ ID NO: 11026 | GGUACAC | SEQ ID NO: 11077 | GGUCACA | SEQ ID NO: 11128 | GGUCUCU | SEQ ID NO: 11179 | GGUGGGG | SEQ ID NO: 11230 | GGUUCUC | SEQ ID NO: 11281 | GUAACAA |
| SEQ ID NO: 11027 | GGUACAG | SEQ ID NO: 11078 | GGUCACC | SEQ ID NO: 11129 | GGUCUGA | SEQ ID NO: 11180 | GGUGGGU | SEQ ID NO: 11231 | GGUUCUG | SEQ ID NO: 11282 | GUAACAC |
| SEQ ID NO: 11028 | GGUACAU | SEQ ID NO: 11079 | GGUCACG | SEQ ID NO: 11130 | GGUCUGC | SEQ ID NO: 11181 | GGUGGUA | SEQ ID NO: 11232 | GGUUCUU | SEQ ID NO: 11283 | GUAACAG |
| SEQ ID NO: 11029 | GGUACCA | SEQ ID NO: 11080 | GGUCACU | SEQ ID NO: 11131 | GGUCUGG | SEQ ID NO: 11182 | GGUGGUC | SEQ ID NO: 11233 | GGUUGAA | SEQ ID NO: 11284 | GUAACAU |
| SEQ ID NO: 11030 | GGUACCC | SEQ ID NO: 11081 | GGUCAGA | SEQ ID NO: 11132 | GGUCUGU | SEQ ID NO: 11183 | GGUGGUG | SEQ ID NO: 11234 | GGUUGAC | SEQ ID NO: 11285 | GUAACCA |
| SEQ ID NO: 11031 | GGUACCG | SEQ ID NO: 11082 | GGUCAGC | SEQ ID NO: 11133 | GGUCUUA | SEQ ID NO: 11184 | GGUGGUU | SEQ ID NO: 11235 | GGUUGAG | SEQ ID NO: 11286 | GUAACCC |
| SEQ ID NO: 11032 | GGUACCU | SEQ ID NO: 11083 | GGUCAGG | SEQ ID NO: 11134 | GGUCUUC | SEQ ID NO: 11185 | GGUGUAA | SEQ ID NO: 11236 | GGUUGAU | SEQ ID NO: 11287 | GUAACCG |
| SEQ ID NO: 11033 | GGUACGA | SEQ ID NO: 11084 | GGUCAGU | SEQ ID NO: 11135 | GGUCUUG | SEQ ID NO: 11186 | GGUGUAC | SEQ ID NO: 11237 | GGUUGCA | SEQ ID NO: 11288 | GUAACCU |
| SEQ ID NO: 11034 | GGUACGC | SEQ ID NO: 11085 | GGUCAUA | SEQ ID NO: 11136 | GGUCUUU | SEQ ID NO: 11187 | GGUGUAG | SEQ ID NO: 11238 | GGUUGCC | SEQ ID NO: 11289 | GUAACGA |
| SEQ ID NO: 11035 | GGUACGG | SEQ ID NO: 11086 | GGUCAUC | SEQ ID NO: 11137 | GGUGAAA | SEQ ID NO: 11188 | GGUGUAU | SEQ ID NO: 11239 | GGUUGCG | SEQ ID NO: 11290 | GUAACGC |
| SEQ ID NO: 11036 | GGUACGU | SEQ ID NO: 11087 | GGUCAUG | SEQ ID NO: 11138 | GGUGAAC | SEQ ID NO: 11189 | GGUGUCA | SEQ ID NO: 11240 | GGUUGCU | SEQ ID NO: 11291 | GUAACGG |
| SEQ ID NO: 11037 | GGUACUA | SEQ ID NO: 11088 | GGUCAUU | SEQ ID NO: 11139 | GGUGAAG | SEQ ID NO: 11190 | GGUGUCC | SEQ ID NO: 11241 | GGUUGGA | SEQ ID NO: 11292 | GUAACGU |
| SEQ ID NO: 11038 | GGUACUC | SEQ ID NO: 11089 | GGUCCAA | SEQ ID NO: 11140 | GGUGAAU | SEQ ID NO: 11191 | GGUGUCG | SEQ ID NO: 11242 | GGUUGGC | SEQ ID NO: 11293 | GUAACUA |
| SEQ ID NO: 11039 | GGUACUG | SEQ ID NO: 11090 | GGUCCAC | SEQ ID NO: 11141 | GGUGACA | SEQ ID NO: 11192 | GGUGUCU | SEQ ID NO: 11243 | GGUUGGG | SEQ ID NO: 11294 | GUAACUC |
| SEQ ID NO: 11040 | GGUACUU | SEQ ID NO: 11091 | GGUCCAG | SEQ ID NO: 11142 | GGUGACC | SEQ ID NO: 11193 | GGUGUGA | SEQ ID NO: 11244 | GGUUGGU | SEQ ID NO: 11295 | GUAACUG |
| SEQ ID NO: 11041 | GGUAGAA | SEQ ID NO: 11092 | GGUCCAU | SEQ ID NO: 11143 | GGUGACG | SEQ ID NO: 11194 | GGUGUGC | SEQ ID NO: 11245 | GGUUGUA | SEQ ID NO: 11296 | GUAACUU |
| SEQ ID NO: 11042 | GGUAGAC | SEQ ID NO: 11093 | GGUCCCA | SEQ ID NO: 11144 | GGUGACU | SEQ ID NO: 11195 | GGUGUGG | SEQ ID NO: 11246 | GGUUGUC | SEQ ID NO: 11297 | GUAAGAA |
| SEQ ID NO: 11043 | GGUAGAG | SEQ ID NO: 11094 | GGUCCCC | SEQ ID NO: 11145 | GGUGAGA | SEQ ID NO: 11196 | GGUGUGU | SEQ ID NO: 11247 | GGUUGUG | SEQ ID NO: 11298 | GUAAGAC |
| SEQ ID NO: 11044 | GGUAGAU | SEQ ID NO: 11095 | GGUCCCG | SEQ ID NO: 11146 | GGUGAGC | SEQ ID NO: 11197 | GGUGUUA | SEQ ID NO: 11248 | GGUUGUU | SEQ ID NO: 11299 | GUAAGAG |
| SEQ ID NO: 11045 | GGUAGCA | SEQ ID NO: 11096 | GGUCCCU | SEQ ID NO: 11147 | GGUGAGG | SEQ ID NO: 11198 | GGUGUUC | SEQ ID NO: 11249 | GGUUUAA | SEQ ID NO: 11300 | GUAAGAU |
| SEQ ID NO: 11046 | GGUAGCC | SEQ ID NO: 11097 | GGUCCGA | SEQ ID NO: 11148 | GGUGAGU | SEQ ID NO: 11199 | GGUGUUG | SEQ ID NO: 11250 | GGUUUAC | SEQ ID NO: 11301 | GUAAGCA |
| SEQ ID NO: 11047 | GGUAGCG | SEQ ID NO: 11098 | GGUCCGC | SEQ ID NO: 11149 | GGUGAUA | SEQ ID NO: 11200 | GGUGUUU | SEQ ID NO: 11251 | GGUUUAG | SEQ ID NO: 11302 | GUAAGCC |
| SEQ ID NO: 11048 | GGUAGCU | SEQ ID NO: 11099 | GGUCCGG | SEQ ID NO: 11150 | GGUGAUC | SEQ ID NO: 11201 | GGUUAAA | SEQ ID NO: 11252 | GGUUUAU | SEQ ID NO: 11303 | GUAAGCG |
| SEQ ID NO: 11049 | GGUAGGA | SEQ ID NO: 11100 | GGUCCGU | SEQ ID NO: 11151 | GGUGAUG | SEQ ID NO: 11202 | GGUUAAC | SEQ ID NO: 11253 | GGUUUCA | SEQ ID NO: 11304 | GUAAGCU |
| SEQ ID NO: 11050 | GGUAGGC | SEQ ID NO: 11101 | GGUCCUA | SEQ ID NO: 11152 | GGUGAUU | SEQ ID NO: 11203 | GGUUAAG | SEQ ID NO: 11254 | GGUUUCC | SEQ ID NO: 11305 | GUAAGGA |
| SEQ ID NO: 11051 | GGUAGGG | SEQ ID NO: 11102 | GGUCCUC | SEQ ID NO: 11153 | GGUGCAA | SEQ ID NO: 11204 | GGUUAAU | SEQ ID NO: 11255 | GGUUUCG | SEQ ID NO: 11306 | GUAAGGC |
| SEQ ID NO: 11052 | GGUAGGU | SEQ ID NO: 11103 | GGUCCUG | SEQ ID NO: 11154 | GGUGCAC | SEQ ID NO: 11205 | GGUUACA | SEQ ID NO: 11256 | GGUUUCU | SEQ ID NO: 11307 | GUAAGGG |
| SEQ ID NO: 11053 | GGUAGUA | SEQ ID NO: 11104 | GGUCCUU | SEQ ID NO: 11155 | GGUGCAG | SEQ ID NO: 11206 | GGUUACC | SEQ ID NO: 11257 | GGUUUGA | SEQ ID NO: 11308 | GUAAGGU |
| SEQ ID NO: 11054 | GGUAGUC | SEQ ID NO: 11105 | GGUCGAA | SEQ ID NO: 11156 | GGUGCAU | SEQ ID NO: 11207 | GGUUACG | SEQ ID NO: 11258 | GGUUUGC | SEQ ID NO: 11309 | GUAAGUA |
| SEQ ID NO: 11055 | GGUAGUG | SEQ ID NO: 11106 | GGUCGAC | SEQ ID NO: 11157 | GGUGCCA | SEQ ID NO: 11208 | GGUUACU | SEQ ID NO: 11259 | GGUUUGG | SEQ ID NO: 11310 | GUAAGUC |
| SEQ ID NO: 11056 | GGUAGUU | SEQ ID NO: 11107 | GGUCGAG | SEQ ID NO: 11158 | GGUGCCC | SEQ ID NO: 11209 | GGUUAGA | SEQ ID NO: 11260 | GGUUUGU | SEQ ID NO: 11311 | GUAAGUG |
| SEQ ID NO: 11057 | GGUAUAA | SEQ ID NO: 11108 | GGUCGAU | SEQ ID NO: 11159 | GGUGCCG | SEQ ID NO: 11210 | GGUUAGC | SEQ ID NO: 11261 | GGUUUUA | SEQ ID NO: 11312 | GUAAGUU |
| SEQ ID NO: 11058 | GGUAUAC | SEQ ID NO: 11109 | GGUCGCA | SEQ ID NO: 11160 | GGUGCCU | SEQ ID NO: 11211 | GGUUAGG | SEQ ID NO: 11262 | GGUUUUC | SEQ ID NO: 11313 | GUAAUAA |
| SEQ ID NO: 11059 | GGUAUAG | SEQ ID NO: 11110 | GGUCGCC | SEQ ID NO: 11161 | GGUGCGA | SEQ ID NO: 11212 | GGUUAGU | SEQ ID NO: 11263 | GGUUUUG | SEQ ID NO: 11314 | GUAAUAC |
| SEQ ID NO: 11060 | GGUAUAU | SEQ ID NO: 11111 | GGUCGCG | SEQ ID NO: 11162 | GGUGCGC | SEQ ID NO: 11213 | GGUUAUA | SEQ ID NO: 11264 | GGUUUUU | SEQ ID NO: 11315 | GUAAUAG |
| SEQ ID NO: 11061 | GGUAUCA | SEQ ID NO: 11112 | GGUCGCU | SEQ ID NO: 11163 | GGUGCGG | SEQ ID NO: 11214 | GGUUAUC | SEQ ID NO: 11265 | GUAAAAA | SEQ ID NO: 11316 | GUAAUAU |
| SEQ ID NO: 11062 | GGUAUCC | SEQ ID NO: 11113 | GGUCGGA | SEQ ID NO: 11164 | GGUGCGU | SEQ ID NO: 11215 | GGUUAUG | SEQ ID NO: 11266 | GUAAAAC | SEQ ID NO: 11317 | GUAAUCA |
| SEQ ID NO: 11063 | GGUAUCG | SEQ ID NO: 11114 | GGUCGGC | SEQ ID NO: 11165 | GGUGCUA | SEQ ID NO: 11216 | GGUUAUU | SEQ ID NO: 11267 | GUAAAAG | SEQ ID NO: 11318 | GUAAUCC |
| SEQ ID NO: 11064 | GGUAUCU | SEQ ID NO: 11115 | GGUCGGG | SEQ ID NO: 11166 | GGUGCUC | SEQ ID NO: 11217 | GGUUCAA | SEQ ID NO: 11268 | GUAAAAU | SEQ ID NO: 11319 | GUAAUCG |
| SEQ ID NO: 11065 | GGUAUGA | SEQ ID NO: 11116 | GGUCGGU | SEQ ID NO: 11167 | GGUGCUG | SEQ ID NO: 11218 | GGUUCAC | SEQ ID NO: 11269 | GUAAACA | SEQ ID NO: 11320 | GUAAUCU |
| SEQ ID NO: 11066 | GGUAUGC | SEQ ID NO: 11117 | GGUCGUA | SEQ ID NO: 11168 | GGUGCUU | SEQ ID NO: 11219 | GGUUCAG | SEQ ID NO: 11270 | GUAAACC | SEQ ID NO: 11321 | GUAAUGA |
| SEQ ID NO: 11067 | GGUAUGG | SEQ ID NO: 11118 | GGUCGUC | SEQ ID NO: 11169 | GGUGGAA | SEQ ID NO: 11220 | GGUUCAU | SEQ ID NO: 11271 | GUAAACG | SEQ ID NO: 11322 | GUAAUGC |

Table 38

| | | | | | | | | | | | |
|---|---|---|---|---|---|---|---|---|---|---|---|
| SEQ ID NO: 11323 | GUAAUGG | SEQ ID NO: 11374 | GUACGUC | SEQ ID NO: 11425 | GUAGGAA | SEQ ID NO: 11476 | GUAUCAU | SEQ ID NO: 11527 | GUCAACG | SEQ ID NO: 11578 | GUCAUGC |
| SEQ ID NO: 11324 | GUAAUGU | SEQ ID NO: 11375 | GUACGUG | SEQ ID NO: 11426 | GUAGGAC | SEQ ID NO: 11477 | GUAUCCA | SEQ ID NO: 11528 | GUCAACU | SEQ ID NO: 11579 | GUCAUGG |
| SEQ ID NO: 11325 | GUAAUUA | SEQ ID NO: 11376 | GUACGUU | SEQ ID NO: 11427 | GUAGGAG | SEQ ID NO: 11478 | GUAUCCC | SEQ ID NO: 11529 | GUCAAGA | SEQ ID NO: 11580 | GUCAUGU |
| SEQ ID NO: 11326 | GUAAUUC | SEQ ID NO: 11377 | GUACUAA | SEQ ID NO: 11428 | GUAGGAU | SEQ ID NO: 11479 | GUAUCCG | SEQ ID NO: 11530 | GUCAAGC | SEQ ID NO: 11581 | GUCAUUA |
| SEQ ID NO: 11327 | GUAAUUG | SEQ ID NO: 11378 | GUACUAC | SEQ ID NO: 11429 | GUAGGCA | SEQ ID NO: 11480 | GUAUCCU | SEQ ID NO: 11531 | GUCAAGG | SEQ ID NO: 11582 | GUCAUUC |
| SEQ ID NO: 11328 | GUAAUUU | SEQ ID NO: 11379 | GUACUAG | SEQ ID NO: 11430 | GUAGGCC | SEQ ID NO: 11481 | GUAUCGA | SEQ ID NO: 11532 | GUCAAGU | SEQ ID NO: 11583 | GUCAUUG |
| SEQ ID NO: 11329 | GUACAAA | SEQ ID NO: 11380 | GUACUAU | SEQ ID NO: 11431 | GUAGGCG | SEQ ID NO: 11482 | GUAUCGC | SEQ ID NO: 11533 | GUCAAUA | SEQ ID NO: 11584 | GUCAUUU |
| SEQ ID NO: 11330 | GUACAAC | SEQ ID NO: 11381 | GUACUCA | SEQ ID NO: 11432 | GUAGGCU | SEQ ID NO: 11483 | GUAUCGG | SEQ ID NO: 11534 | GUCAAUC | SEQ ID NO: 11585 | GUCCAAA |
| SEQ ID NO: 11331 | GUACAAG | SEQ ID NO: 11382 | GUACUCC | SEQ ID NO: 11433 | GUAGGGA | SEQ ID NO: 11484 | GUAUCGU | SEQ ID NO: 11535 | GUCAAUG | SEQ ID NO: 11586 | GUCCAAC |
| SEQ ID NO: 11332 | GUACAAU | SEQ ID NO: 11383 | GUACUCG | SEQ ID NO: 11434 | GUAGGGC | SEQ ID NO: 11485 | GUAUCUA | SEQ ID NO: 11536 | GUCAAUU | SEQ ID NO: 11587 | GUCCAAG |
| SEQ ID NO: 11333 | GUACACA | SEQ ID NO: 11384 | GUACUCU | SEQ ID NO: 11435 | GUAGGGG | SEQ ID NO: 11486 | GUAUCUC | SEQ ID NO: 11537 | GUCACAA | SEQ ID NO: 11588 | GUCCAAU |
| SEQ ID NO: 11334 | GUACACC | SEQ ID NO: 11385 | GUACUGA | SEQ ID NO: 11436 | GUAGGGU | SEQ ID NO: 11487 | GUAUCUG | SEQ ID NO: 11538 | GUCACAC | SEQ ID NO: 11589 | GUCCACA |
| SEQ ID NO: 11335 | GUACACG | SEQ ID NO: 11386 | GUACUGC | SEQ ID NO: 11437 | GUAGGUA | SEQ ID NO: 11488 | GUAUCUU | SEQ ID NO: 11539 | GUCACAG | SEQ ID NO: 11590 | GUCCACC |
| SEQ ID NO: 11336 | GUACACU | SEQ ID NO: 11387 | GUACUGG | SEQ ID NO: 11438 | GUAGGUC | SEQ ID NO: 11489 | GUAUGAA | SEQ ID NO: 11540 | GUCACAU | SEQ ID NO: 11591 | GUCCACG |
| SEQ ID NO: 11337 | GUACAGA | SEQ ID NO: 11388 | GUACUGU | SEQ ID NO: 11439 | GUAGGUG | SEQ ID NO: 11490 | GUAUGAC | SEQ ID NO: 11541 | GUCACCA | SEQ ID NO: 11592 | GUCCACU |
| SEQ ID NO: 11338 | GUACAGC | SEQ ID NO: 11389 | GUACUUA | SEQ ID NO: 11440 | GUAGGUU | SEQ ID NO: 11491 | GUAUGAG | SEQ ID NO: 11542 | GUCACCC | SEQ ID NO: 11593 | GUCCAGA |
| SEQ ID NO: 11339 | GUACAGG | SEQ ID NO: 11390 | GUACUUC | SEQ ID NO: 11441 | GUAGUAA | SEQ ID NO: 11492 | GUAUGAU | SEQ ID NO: 11543 | GUCACCG | SEQ ID NO: 11594 | GUCCAGC |
| SEQ ID NO: 11340 | GUACAGU | SEQ ID NO: 11391 | GUACUUG | SEQ ID NO: 11442 | GUAGUAC | SEQ ID NO: 11493 | GUAUGCA | SEQ ID NO: 11544 | GUCACCU | SEQ ID NO: 11595 | GUCCAGG |
| SEQ ID NO: 11341 | GUACAUA | SEQ ID NO: 11392 | GUACUUU | SEQ ID NO: 11443 | GUAGUAG | SEQ ID NO: 11494 | GUAUGCC | SEQ ID NO: 11545 | GUCACGA | SEQ ID NO: 11596 | GUCCAGU |
| SEQ ID NO: 11342 | GUACAUC | SEQ ID NO: 11393 | GUAGAAA | SEQ ID NO: 11444 | GUAGUAU | SEQ ID NO: 11495 | GUAUGCG | SEQ ID NO: 11546 | GUCACGC | SEQ ID NO: 11597 | GUCCAUA |
| SEQ ID NO: 11343 | GUACAUG | SEQ ID NO: 11394 | GUAGAAC | SEQ ID NO: 11445 | GUAGUCA | SEQ ID NO: 11496 | GUAUGCU | SEQ ID NO: 11547 | GUCACGG | SEQ ID NO: 11598 | GUCCAUC |
| SEQ ID NO: 11344 | GUACAUU | SEQ ID NO: 11395 | GUAGAAG | SEQ ID NO: 11446 | GUAGUCC | SEQ ID NO: 11497 | GUAUGGA | SEQ ID NO: 11548 | GUCACGU | SEQ ID NO: 11599 | GUCCAUG |
| SEQ ID NO: 11345 | GUACCAA | SEQ ID NO: 11396 | GUAGAAU | SEQ ID NO: 11447 | GUAGUCG | SEQ ID NO: 11498 | GUAUGGC | SEQ ID NO: 11549 | GUCACUA | SEQ ID NO: 11600 | GUCCAUU |
| SEQ ID NO: 11346 | GUACCAC | SEQ ID NO: 11397 | GUAGACA | SEQ ID NO: 11448 | GUAGUCU | SEQ ID NO: 11499 | GUAUGGG | SEQ ID NO: 11550 | GUCACUC | SEQ ID NO: 11601 | GUCCCAA |
| SEQ ID NO: 11347 | GUACCAG | SEQ ID NO: 11398 | GUAGACC | SEQ ID NO: 11449 | GUAGUGA | SEQ ID NO: 11500 | GUAUGGU | SEQ ID NO: 11551 | GUCACUG | SEQ ID NO: 11602 | GUCCCAC |
| SEQ ID NO: 11348 | GUACCAU | SEQ ID NO: 11399 | GUAGACG | SEQ ID NO: 11450 | GUAGUGC | SEQ ID NO: 11501 | GUAUGUA | SEQ ID NO: 11552 | GUCACUU | SEQ ID NO: 11603 | GUCCCAG |
| SEQ ID NO: 11349 | GUACCCA | SEQ ID NO: 11400 | GUAGACU | SEQ ID NO: 11451 | GUAGUGG | SEQ ID NO: 11502 | GUAUGUC | SEQ ID NO: 11553 | GUCAGAA | SEQ ID NO: 11604 | GUCCCAU |
| SEQ ID NO: 11350 | GUACCCC | SEQ ID NO: 11401 | GUAGAGA | SEQ ID NO: 11452 | GUAGUGU | SEQ ID NO: 11503 | GUAUGUG | SEQ ID NO: 11554 | GUCAGAC | SEQ ID NO: 11605 | GUCCCCA |
| SEQ ID NO: 11351 | GUACCCG | SEQ ID NO: 11402 | GUAGAGC | SEQ ID NO: 11453 | GUAGUUA | SEQ ID NO: 11504 | GUAUGUU | SEQ ID NO: 11555 | GUCAGAG | SEQ ID NO: 11606 | GUCCCCC |
| SEQ ID NO: 11352 | GUACCCU | SEQ ID NO: 11403 | GUAGAGG | SEQ ID NO: 11454 | GUAGUUC | SEQ ID NO: 11505 | GUAUUAA | SEQ ID NO: 11556 | GUCAGAU | SEQ ID NO: 11607 | GUCCCCG |
| SEQ ID NO: 11353 | GUACCGA | SEQ ID NO: 11404 | GUAGAGU | SEQ ID NO: 11455 | GUAGUUG | SEQ ID NO: 11506 | GUAUUAC | SEQ ID NO: 11557 | GUCAGCA | SEQ ID NO: 11608 | GUCCCCU |
| SEQ ID NO: 11354 | GUACCGC | SEQ ID NO: 11405 | GUAGAUA | SEQ ID NO: 11456 | GUAGUUU | SEQ ID NO: 11507 | GUAUUAG | SEQ ID NO: 11558 | GUCAGCC | SEQ ID NO: 11609 | GUCCCGA |
| SEQ ID NO: 11355 | GUACCGG | SEQ ID NO: 11406 | GUAGAUC | SEQ ID NO: 11457 | GUAUAAA | SEQ ID NO: 11508 | GUAUUAU | SEQ ID NO: 11559 | GUCAGCG | SEQ ID NO: 11610 | GUCCCGC |
| SEQ ID NO: 11356 | GUACCGU | SEQ ID NO: 11407 | GUAGAUG | SEQ ID NO: 11458 | GUAUAAC | SEQ ID NO: 11509 | GUAUUCA | SEQ ID NO: 11560 | GUCAGCU | SEQ ID NO: 11611 | GUCCCGG |
| SEQ ID NO: 11357 | GUACCUA | SEQ ID NO: 11408 | GUAGAUU | SEQ ID NO: 11459 | GUAUAAG | SEQ ID NO: 11510 | GUAUUCC | SEQ ID NO: 11561 | GUCAGGA | SEQ ID NO: 11612 | GUCCCGU |
| SEQ ID NO: 11358 | GUACCUC | SEQ ID NO: 11409 | GUAGCAA | SEQ ID NO: 11460 | GUAUAAU | SEQ ID NO: 11511 | GUAUUCG | SEQ ID NO: 11562 | GUCAGGC | SEQ ID NO: 11613 | GUCCCUA |
| SEQ ID NO: 11359 | GUACCUG | SEQ ID NO: 11410 | GUAGCAC | SEQ ID NO: 11461 | GUAUACA | SEQ ID NO: 11512 | GUAUUCU | SEQ ID NO: 11563 | GUCAGGG | SEQ ID NO: 11614 | GUCCCUC |
| SEQ ID NO: 11360 | GUACCUU | SEQ ID NO: 11411 | GUAGCAG | SEQ ID NO: 11462 | GUAUACC | SEQ ID NO: 11513 | GUAUUGA | SEQ ID NO: 11564 | GUCAGGU | SEQ ID NO: 11615 | GUCCCUG |
| SEQ ID NO: 11361 | GUACGAA | SEQ ID NO: 11412 | GUAGCAU | SEQ ID NO: 11463 | GUAUACG | SEQ ID NO: 11514 | GUAUUGC | SEQ ID NO: 11565 | GUCAGUA | SEQ ID NO: 11616 | GUCCCUU |
| SEQ ID NO: 11362 | GUACGAC | SEQ ID NO: 11413 | GUAGCCA | SEQ ID NO: 11464 | GUAUACU | SEQ ID NO: 11515 | GUAUUGG | SEQ ID NO: 11566 | GUCAGUC | SEQ ID NO: 11617 | GUCCGAA |
| SEQ ID NO: 11363 | GUACGAG | SEQ ID NO: 11414 | GUAGCCC | SEQ ID NO: 11465 | GUAUAGA | SEQ ID NO: 11516 | GUAUUGU | SEQ ID NO: 11567 | GUCAGUG | SEQ ID NO: 11618 | GUCCGAC |
| SEQ ID NO: 11364 | GUACGAU | SEQ ID NO: 11415 | GUAGCCG | SEQ ID NO: 11466 | GUAUAGC | SEQ ID NO: 11517 | GUAUUUA | SEQ ID NO: 11568 | GUCAGUU | SEQ ID NO: 11619 | GUCCGAG |
| SEQ ID NO: 11365 | GUACGCA | SEQ ID NO: 11416 | GUAGCCU | SEQ ID NO: 11467 | GUAUAGG | SEQ ID NO: 11518 | GUAUUUC | SEQ ID NO: 11569 | GUCAUAA | SEQ ID NO: 11620 | GUCCGAU |
| SEQ ID NO: 11366 | GUACGCC | SEQ ID NO: 11417 | GUAGCGA | SEQ ID NO: 11468 | GUAUAGU | SEQ ID NO: 11519 | GUAUUUG | SEQ ID NO: 11570 | GUCAUAC | SEQ ID NO: 11621 | GUCCGCA |
| SEQ ID NO: 11367 | GUACGCG | SEQ ID NO: 11418 | GUAGCGC | SEQ ID NO: 11469 | GUAUAUA | SEQ ID NO: 11520 | GUAUUUU | SEQ ID NO: 11571 | GUCAUAG | SEQ ID NO: 11622 | GUCCGCC |
| SEQ ID NO: 11368 | GUACGCU | SEQ ID NO: 11419 | GUAGCGG | SEQ ID NO: 11470 | GUAUAUC | SEQ ID NO: 11521 | GUCAAAA | SEQ ID NO: 11572 | GUCAUAU | SEQ ID NO: 11623 | GUCCGCG |
| SEQ ID NO: 11369 | GUACGGA | SEQ ID NO: 11420 | GUAGCGU | SEQ ID NO: 11471 | GUAUAUG | SEQ ID NO: 11522 | GUCAAAC | SEQ ID NO: 11573 | GUCAUCA | SEQ ID NO: 11624 | GUCCGCU |
| SEQ ID NO: 11370 | GUACGGC | SEQ ID NO: 11421 | GUAGCUA | SEQ ID NO: 11472 | GUAUAUU | SEQ ID NO: 11523 | GUCAAAG | SEQ ID NO: 11574 | GUCAUCC | SEQ ID NO: 11625 | GUCCGGA |
| SEQ ID NO: 11371 | GUACGGG | SEQ ID NO: 11422 | GUAGCUC | SEQ ID NO: 11473 | GUAUCAA | SEQ ID NO: 11524 | GUCAAAU | SEQ ID NO: 11575 | GUCAUCG | SEQ ID NO: 11626 | GUCCGGC |
| SEQ ID NO: 11372 | GUACGGU | SEQ ID NO: 11423 | GUAGCUG | SEQ ID NO: 11474 | GUAUCAC | SEQ ID NO: 11525 | GUCAACA | SEQ ID NO: 11576 | GUCAUCU | SEQ ID NO: 11627 | GUCCGGG |
| SEQ ID NO: 11373 | GUACGUA | SEQ ID NO: 11424 | GUAGCUU | SEQ ID NO: 11475 | GUAUCAG | SEQ ID NO: 11526 | GUCAACC | SEQ ID NO: 11577 | GUCAUGA | SEQ ID NO: 11628 | GUCCGGU |

Table 39

| | | | | | | | | | | | |
|---|---|---|---|---|---|---|---|---|---|---|---|
| SEQ ID NO: 11629 | GUCCGUA | SEQ ID NO: 11680 | GUCGCUU | SEQ ID NO: 11731 | GUCUCAG | SEQ ID NO: 11782 | GUGAACC | SEQ ID NO: 11833 | GUGAUGA | SEQ ID NO: 11884 | GUGCGGU |
| SEQ ID NO: 11630 | GUCCGUC | SEQ ID NO: 11681 | GUCGGAA | SEQ ID NO: 11732 | GUCUCAU | SEQ ID NO: 11783 | GUGAACG | SEQ ID NO: 11834 | GUGAUGC | SEQ ID NO: 11885 | GUGCGUA |
| SEQ ID NO: 11631 | GUCCGUG | SEQ ID NO: 11682 | GUCGGAC | SEQ ID NO: 11733 | GUCUCCA | SEQ ID NO: 11784 | GUGAACU | SEQ ID NO: 11835 | GUGAUGG | SEQ ID NO: 11886 | GUGCGUC |
| SEQ ID NO: 11632 | GUCCGUU | SEQ ID NO: 11683 | GUCGGAG | SEQ ID NO: 11734 | GUCUCCC | SEQ ID NO: 11785 | GUGAAGA | SEQ ID NO: 11836 | GUGAUGU | SEQ ID NO: 11887 | GUGCGUG |
| SEQ ID NO: 11633 | GUCCUAA | SEQ ID NO: 11684 | GUCGGAU | SEQ ID NO: 11735 | GUCUCCG | SEQ ID NO: 11786 | GUGAAGC | SEQ ID NO: 11837 | GUGAUUA | SEQ ID NO: 11888 | GUGCGUU |
| SEQ ID NO: 11634 | GUCCUAC | SEQ ID NO: 11685 | GUCGGCA | SEQ ID NO: 11736 | GUCUCCU | SEQ ID NO: 11787 | GUGAAGG | SEQ ID NO: 11838 | GUGAUUC | SEQ ID NO: 11889 | GUGCUAA |
| SEQ ID NO: 11635 | GUCCUAG | SEQ ID NO: 11686 | GUCGGCC | SEQ ID NO: 11737 | GUCUCGA | SEQ ID NO: 11788 | GUGAAGU | SEQ ID NO: 11839 | GUGAUUG | SEQ ID NO: 11890 | GUGCUAC |
| SEQ ID NO: 11636 | GUCCUAU | SEQ ID NO: 11687 | GUCGGCG | SEQ ID NO: 11738 | GUCUCGC | SEQ ID NO: 11789 | GUGAAUA | SEQ ID NO: 11840 | GUGAUUU | SEQ ID NO: 11891 | GUGCUAG |
| SEQ ID NO: 11637 | GUCCUCA | SEQ ID NO: 11688 | GUCGGCU | SEQ ID NO: 11739 | GUCUCGG | SEQ ID NO: 11790 | GUGAAUC | SEQ ID NO: 11841 | GUGCAAA | SEQ ID NO: 11892 | GUGCUAU |
| SEQ ID NO: 11638 | GUCCUCC | SEQ ID NO: 11689 | GUCGGGA | SEQ ID NO: 11740 | GUCUCGU | SEQ ID NO: 11791 | GUGAAUG | SEQ ID NO: 11842 | GUGCAAC | SEQ ID NO: 11893 | GUGCUCA |
| SEQ ID NO: 11639 | GUCCUCG | SEQ ID NO: 11690 | GUCGGGC | SEQ ID NO: 11741 | GUCUCUA | SEQ ID NO: 11792 | GUGAAUU | SEQ ID NO: 11843 | GUGCAAG | SEQ ID NO: 11894 | GUGCUCC |
| SEQ ID NO: 11640 | GUCCUCU | SEQ ID NO: 11691 | GUCGGGG | SEQ ID NO: 11742 | GUCUCUC | SEQ ID NO: 11793 | GUGACAA | SEQ ID NO: 11844 | GUGCAAU | SEQ ID NO: 11895 | GUGCUCG |
| SEQ ID NO: 11641 | GUCCUGA | SEQ ID NO: 11692 | GUCGGGU | SEQ ID NO: 11743 | GUCUCUG | SEQ ID NO: 11794 | GUGACAC | SEQ ID NO: 11845 | GUGCACA | SEQ ID NO: 11896 | GUGCUCU |
| SEQ ID NO: 11642 | GUCCUGC | SEQ ID NO: 11693 | GUCGGUA | SEQ ID NO: 11744 | GUCUCUU | SEQ ID NO: 11795 | GUGACAG | SEQ ID NO: 11846 | GUGCACC | SEQ ID NO: 11897 | GUGCUGA |
| SEQ ID NO: 11643 | GUCCUGG | SEQ ID NO: 11694 | GUCGGUC | SEQ ID NO: 11745 | GUCUGAA | SEQ ID NO: 11796 | GUGACAU | SEQ ID NO: 11847 | GUGCACG | SEQ ID NO: 11898 | GUGCUGC |
| SEQ ID NO: 11644 | GUCCUGU | SEQ ID NO: 11695 | GUCGGUG | SEQ ID NO: 11746 | GUCUGAC | SEQ ID NO: 11797 | GUGACCA | SEQ ID NO: 11848 | GUGCACU | SEQ ID NO: 11899 | GUGCUGG |
| SEQ ID NO: 11645 | GUCCUUA | SEQ ID NO: 11696 | GUCGGUU | SEQ ID NO: 11747 | GUCUGAG | SEQ ID NO: 11798 | GUGACCC | SEQ ID NO: 11849 | GUGCAGA | SEQ ID NO: 11900 | GUGCUGU |
| SEQ ID NO: 11646 | GUCCUUC | SEQ ID NO: 11697 | GUCGUAA | SEQ ID NO: 11748 | GUCUGAU | SEQ ID NO: 11799 | GUGACCG | SEQ ID NO: 11850 | GUGCAGC | SEQ ID NO: 11901 | GUGCUUA |
| SEQ ID NO: 11647 | GUCCUUG | SEQ ID NO: 11698 | GUCGUAC | SEQ ID NO: 11749 | GUCUGCA | SEQ ID NO: 11800 | GUGACCU | SEQ ID NO: 11851 | GUGCAGG | SEQ ID NO: 11902 | GUGCUUC |
| SEQ ID NO: 11648 | GUCCUUU | SEQ ID NO: 11699 | GUCGUAG | SEQ ID NO: 11750 | GUCUGCC | SEQ ID NO: 11801 | GUGACGA | SEQ ID NO: 11852 | GUGCAGU | SEQ ID NO: 11903 | GUGCUUG |
| SEQ ID NO: 11649 | GUCGAAA | SEQ ID NO: 11700 | GUCGUAU | SEQ ID NO: 11751 | GUCUGCG | SEQ ID NO: 11802 | GUGACGC | SEQ ID NO: 11853 | GUGCAUA | SEQ ID NO: 11904 | GUGCUUU |
| SEQ ID NO: 11650 | GUCGAAC | SEQ ID NO: 11701 | GUCGUCA | SEQ ID NO: 11752 | GUCUGCU | SEQ ID NO: 11803 | GUGACGG | SEQ ID NO: 11854 | GUGCAUC | SEQ ID NO: 11905 | GUGGAAA |
| SEQ ID NO: 11651 | GUCGAAG | SEQ ID NO: 11702 | GUCGUCC | SEQ ID NO: 11753 | GUCUGGA | SEQ ID NO: 11804 | GUGACGU | SEQ ID NO: 11855 | GUGCAUG | SEQ ID NO: 11906 | GUGGAAC |
| SEQ ID NO: 11652 | GUCGAAU | SEQ ID NO: 11703 | GUCGUCG | SEQ ID NO: 11754 | GUCUGGC | SEQ ID NO: 11805 | GUGACUA | SEQ ID NO: 11856 | GUGCAUU | SEQ ID NO: 11907 | GUGGAAG |
| SEQ ID NO: 11653 | GUCGACA | SEQ ID NO: 11704 | GUCGUCU | SEQ ID NO: 11755 | GUCUGGG | SEQ ID NO: 11806 | GUGACUC | SEQ ID NO: 11857 | GUGCCAA | SEQ ID NO: 11908 | GUGGAAU |
| SEQ ID NO: 11654 | GUCGACC | SEQ ID NO: 11705 | GUCGUGA | SEQ ID NO: 11756 | GUCUGGU | SEQ ID NO: 11807 | GUGACUG | SEQ ID NO: 11858 | GUGCCAC | SEQ ID NO: 11909 | GUGGACA |
| SEQ ID NO: 11655 | GUCGACG | SEQ ID NO: 11706 | GUCGUGC | SEQ ID NO: 11757 | GUCUGUA | SEQ ID NO: 11808 | GUGACUU | SEQ ID NO: 11859 | GUGCCAG | SEQ ID NO: 11910 | GUGGACC |
| SEQ ID NO: 11656 | GUCGACU | SEQ ID NO: 11707 | GUCGUGG | SEQ ID NO: 11758 | GUCUGUC | SEQ ID NO: 11809 | GUGAGAA | SEQ ID NO: 11860 | GUGCCAU | SEQ ID NO: 11911 | GUGGACG |
| SEQ ID NO: 11657 | GUCGAGA | SEQ ID NO: 11708 | GUCGUGU | SEQ ID NO: 11759 | GUCUGUG | SEQ ID NO: 11810 | GUGAGAC | SEQ ID NO: 11861 | GUGCCCA | SEQ ID NO: 11912 | GUGGACU |
| SEQ ID NO: 11658 | GUCGAGC | SEQ ID NO: 11709 | GUCGUUA | SEQ ID NO: 11760 | GUCUGUU | SEQ ID NO: 11811 | GUGAGAG | SEQ ID NO: 11862 | GUGCCCC | SEQ ID NO: 11913 | GUGGAGA |
| SEQ ID NO: 11659 | GUCGAGG | SEQ ID NO: 11710 | GUCGUUC | SEQ ID NO: 11761 | GUCUUAA | SEQ ID NO: 11812 | GUGAGAU | SEQ ID NO: 11863 | GUGCCCG | SEQ ID NO: 11914 | GUGGAGC |
| SEQ ID NO: 11660 | GUCGAGU | SEQ ID NO: 11711 | GUCGUUG | SEQ ID NO: 11762 | GUCUUAC | SEQ ID NO: 11813 | GUGAGCA | SEQ ID NO: 11864 | GUGCCCU | SEQ ID NO: 11915 | GUGGAGG |
| SEQ ID NO: 11661 | GUCGAUA | SEQ ID NO: 11712 | GUCGUUU | SEQ ID NO: 11763 | GUCUUAG | SEQ ID NO: 11814 | GUGAGCC | SEQ ID NO: 11865 | GUGCCGA | SEQ ID NO: 11916 | GUGGAGU |
| SEQ ID NO: 11662 | GUCGAUC | SEQ ID NO: 11713 | GUCUAAA | SEQ ID NO: 11764 | GUCUUAU | SEQ ID NO: 11815 | GUGAGCG | SEQ ID NO: 11866 | GUGCCGC | SEQ ID NO: 11917 | GUGGAUA |
| SEQ ID NO: 11663 | GUCGAUG | SEQ ID NO: 11714 | GUCUAAC | SEQ ID NO: 11765 | GUCUUCA | SEQ ID NO: 11816 | GUGAGCU | SEQ ID NO: 11867 | GUGCCGG | SEQ ID NO: 11918 | GUGGAUC |
| SEQ ID NO: 11664 | GUCGAUU | SEQ ID NO: 11715 | GUCUAAG | SEQ ID NO: 11766 | GUCUUCC | SEQ ID NO: 11817 | GUGAGGA | SEQ ID NO: 11868 | GUGCCGU | SEQ ID NO: 11919 | GUGGAUG |
| SEQ ID NO: 11665 | GUCGCAA | SEQ ID NO: 11716 | GUCUAAU | SEQ ID NO: 11767 | GUCUUCG | SEQ ID NO: 11818 | GUGAGGC | SEQ ID NO: 11869 | GUGCCUA | SEQ ID NO: 11920 | GUGGAUU |
| SEQ ID NO: 11666 | GUCGCAC | SEQ ID NO: 11717 | GUCUACA | SEQ ID NO: 11768 | GUCUUCU | SEQ ID NO: 11819 | GUGAGGG | SEQ ID NO: 11870 | GUGCCUC | SEQ ID NO: 11921 | GUGGCAA |
| SEQ ID NO: 11667 | GUCGCAG | SEQ ID NO: 11718 | GUCUACC | SEQ ID NO: 11769 | GUCUUGA | SEQ ID NO: 11820 | GUGAGGU | SEQ ID NO: 11871 | GUGCCUG | SEQ ID NO: 11922 | GUGGCAC |
| SEQ ID NO: 11668 | GUCGCAU | SEQ ID NO: 11719 | GUCUACG | SEQ ID NO: 11770 | GUCUUGC | SEQ ID NO: 11821 | GUGAGUA | SEQ ID NO: 11872 | GUGCCUU | SEQ ID NO: 11923 | GUGGCAG |
| SEQ ID NO: 11669 | GUCGCCA | SEQ ID NO: 11720 | GUCUACU | SEQ ID NO: 11771 | GUCUUGG | SEQ ID NO: 11822 | GUGAGUC | SEQ ID NO: 11873 | GUGCGAA | SEQ ID NO: 11924 | GUGGCAU |
| SEQ ID NO: 11670 | GUCGCCC | SEQ ID NO: 11721 | GUCUAGA | SEQ ID NO: 11772 | GUCUUGU | SEQ ID NO: 11823 | GUGAGUG | SEQ ID NO: 11874 | GUGCGAC | SEQ ID NO: 11925 | GUGGCCA |
| SEQ ID NO: 11671 | GUCGCCG | SEQ ID NO: 11722 | GUCUAGC | SEQ ID NO: 11773 | GUCUUUA | SEQ ID NO: 11824 | GUGAGUU | SEQ ID NO: 11875 | GUGCGAG | SEQ ID NO: 11926 | GUGGCCC |
| SEQ ID NO: 11672 | GUCGCCU | SEQ ID NO: 11723 | GUCUAGG | SEQ ID NO: 11774 | GUCUUUC | SEQ ID NO: 11825 | GUGAUAA | SEQ ID NO: 11876 | GUGCGAU | SEQ ID NO: 11927 | GUGGCCG |
| SEQ ID NO: 11673 | GUCGCGA | SEQ ID NO: 11724 | GUCUAGU | SEQ ID NO: 11775 | GUCUUUG | SEQ ID NO: 11826 | GUGAUAC | SEQ ID NO: 11877 | GUGCGCA | SEQ ID NO: 11928 | GUGGCCU |
| SEQ ID NO: 11674 | GUCGCGC | SEQ ID NO: 11725 | GUCUAUA | SEQ ID NO: 11776 | GUCUUUU | SEQ ID NO: 11827 | GUGAUAG | SEQ ID NO: 11878 | GUGCGCC | SEQ ID NO: 11929 | GUGGCGA |
| SEQ ID NO: 11675 | GUCGCGG | SEQ ID NO: 11726 | GUCUAUC | SEQ ID NO: 11777 | GUGAAAA | SEQ ID NO: 11828 | GUGAUAU | SEQ ID NO: 11879 | GUGCGCG | SEQ ID NO: 11930 | GUGGCGC |
| SEQ ID NO: 11676 | GUCGCGU | SEQ ID NO: 11727 | GUCUAUG | SEQ ID NO: 11778 | GUGAAAC | SEQ ID NO: 11829 | GUGAUCA | SEQ ID NO: 11880 | GUGCGCU | SEQ ID NO: 11931 | GUGGCGG |
| SEQ ID NO: 11677 | GUCGCUA | SEQ ID NO: 11728 | GUCUAUU | SEQ ID NO: 11779 | GUGAAAG | SEQ ID NO: 11830 | GUGAUCC | SEQ ID NO: 11881 | GUGCGGA | SEQ ID NO: 11932 | GUGGCGU |
| SEQ ID NO: 11678 | GUCGCUC | SEQ ID NO: 11729 | GUCUCAA | SEQ ID NO: 11780 | GUGAAAU | SEQ ID NO: 11831 | GUGAUCG | SEQ ID NO: 11882 | GUGCGGC | SEQ ID NO: 11933 | GUGGCUA |
| SEQ ID NO: 11679 | GUCGCUG | SEQ ID NO: 11730 | GUCUCAC | SEQ ID NO: 11781 | GUGAACA | SEQ ID NO: 11832 | GUGAUCU | SEQ ID NO: 11883 | GUGCGGG | SEQ ID NO: 11934 | GUGGCUC |

Table 40

| | |
|---|---|
| SEQ ID NO: 11935 | GUGGCUG |
| SEQ ID NO: 11936 | GUGGCUU |
| SEQ ID NO: 11937 | GUGGGAA |
| SEQ ID NO: 11938 | GUGGGAC |
| SEQ ID NO: 11939 | GUGGGAG |
| SEQ ID NO: 11940 | GUGGGAU |
| SEQ ID NO: 11941 | GUGGGCA |
| SEQ ID NO: 11942 | GUGGGCC |
| SEQ ID NO: 11943 | GUGGGCG |
| SEQ ID NO: 11944 | GUGGGCU |
| SEQ ID NO: 11945 | GUGGGGA |
| SEQ ID NO: 11946 | GUGGGGC |
| SEQ ID NO: 11947 | GUGGGGG |
| SEQ ID NO: 11948 | GUGGGGU |
| SEQ ID NO: 11949 | GUGGGUA |
| SEQ ID NO: 11950 | GUGGGUC |
| SEQ ID NO: 11951 | GUGGGUG |
| SEQ ID NO: 11952 | GUGGGUU |
| SEQ ID NO: 11953 | GUGGUAA |
| SEQ ID NO: 11954 | GUGGUAC |
| SEQ ID NO: 11955 | GUGGUAG |
| SEQ ID NO: 11956 | GUGGUAU |
| SEQ ID NO: 11957 | GUGGUCA |
| SEQ ID NO: 11958 | GUGGUCC |
| SEQ ID NO: 11959 | GUGGUCG |
| SEQ ID NO: 11960 | GUGGUCU |
| SEQ ID NO: 11961 | GUGGUGA |
| SEQ ID NO: 11962 | GUGGUGC |
| SEQ ID NO: 11963 | GUGGUGG |
| SEQ ID NO: 11964 | GUGGUGU |
| SEQ ID NO: 11965 | GUGGUUA |
| SEQ ID NO: 11966 | GUGGUUC |
| SEQ ID NO: 11967 | GUGGUUG |
| SEQ ID NO: 11968 | GUGGUUU |
| SEQ ID NO: 11969 | GUGUAAA |
| SEQ ID NO: 11970 | GUGUAAC |
| SEQ ID NO: 11971 | GUGUAAG |
| SEQ ID NO: 11972 | GUGUAAU |
| SEQ ID NO: 11973 | GUGUACA |
| SEQ ID NO: 11974 | GUGUACC |
| SEQ ID NO: 11975 | GUGUACG |
| SEQ ID NO: 11976 | GUGUACU |
| SEQ ID NO: 11977 | GUGUAGA |
| SEQ ID NO: 11978 | GUGUAGC |
| SEQ ID NO: 11979 | GUGUAGG |
| SEQ ID NO: 11980 | GUGUAGU |
| SEQ ID NO: 11981 | GUGUAUA |
| SEQ ID NO: 11982 | GUGUAUC |
| SEQ ID NO: 11983 | GUGUAUG |
| SEQ ID NO: 11984 | GUGUAUU |
| SEQ ID NO: 11985 | GUGUCAA |
| SEQ ID NO: 11986 | GUGUCAC |
| SEQ ID NO: 11987 | GUGUCAG |
| SEQ ID NO: 11988 | GUGUCAU |
| SEQ ID NO: 11989 | GUGUCCA |
| SEQ ID NO: 11990 | GUGUCCC |
| SEQ ID NO: 11991 | GUGUCCG |
| SEQ ID NO: 11992 | GUGUCCU |
| SEQ ID NO: 11993 | GUGUCGA |
| SEQ ID NO: 11994 | GUGUCGC |
| SEQ ID NO: 11995 | GUGUCGG |
| SEQ ID NO: 11996 | GUGUCGU |
| SEQ ID NO: 11997 | GUGUCUA |
| SEQ ID NO: 11998 | GUGUCUC |
| SEQ ID NO: 11999 | GUGUCUG |
| SEQ ID NO: 12000 | GUGUCUU |
| SEQ ID NO: 12001 | GUGUGAA |
| SEQ ID NO: 12002 | GUGUGAC |
| SEQ ID NO: 12003 | GUGUGAG |
| SEQ ID NO: 12004 | GUGUGAU |
| SEQ ID NO: 12005 | GUGUGCA |
| SEQ ID NO: 12006 | GUGUGCC |
| SEQ ID NO: 12007 | GUGUGCG |
| SEQ ID NO: 12008 | GUGUGCU |
| SEQ ID NO: 12009 | GUGUGGA |
| SEQ ID NO: 12010 | GUGUGGC |
| SEQ ID NO: 12011 | GUGUGGG |
| SEQ ID NO: 12012 | GUGUGGU |
| SEQ ID NO: 12013 | GUGUGUA |
| SEQ ID NO: 12014 | GUGUGUC |
| SEQ ID NO: 12015 | GUGUGUG |
| SEQ ID NO: 12016 | GUGUGUU |
| SEQ ID NO: 12017 | GUGUUAA |
| SEQ ID NO: 12018 | GUGUUAC |
| SEQ ID NO: 12019 | GUGUUAG |
| SEQ ID NO: 12020 | GUGUUAU |
| SEQ ID NO: 12021 | GUGUUCA |
| SEQ ID NO: 12022 | GUGUUCC |
| SEQ ID NO: 12023 | GUGUUCG |
| SEQ ID NO: 12024 | GUGUUCU |
| SEQ ID NO: 12025 | GUGUUGA |
| SEQ ID NO: 12026 | GUGUUGC |
| SEQ ID NO: 12027 | GUGUUGG |
| SEQ ID NO: 12028 | GUGUUGU |
| SEQ ID NO: 12029 | GUGUUUA |
| SEQ ID NO: 12030 | GUGUUUC |
| SEQ ID NO: 12031 | GUGUUUG |
| SEQ ID NO: 12032 | GUGUUUU |
| SEQ ID NO: 12033 | GUUAAAA |
| SEQ ID NO: 12034 | GUUAAAC |
| SEQ ID NO: 12035 | GUUAAAG |
| SEQ ID NO: 12036 | GUUAAAU |
| SEQ ID NO: 12037 | GUUAACA |
| SEQ ID NO: 12038 | GUUAACC |
| SEQ ID NO: 12039 | GUUAACG |
| SEQ ID NO: 12040 | GUUAACU |
| SEQ ID NO: 12041 | GUUAAGA |
| SEQ ID NO: 12042 | GUUAAGC |
| SEQ ID NO: 12043 | GUUAAGG |
| SEQ ID NO: 12044 | GUUAAGU |
| SEQ ID NO: 12045 | GUUAAUA |
| SEQ ID NO: 12046 | GUUAAUC |
| SEQ ID NO: 12047 | GUUAAUG |
| SEQ ID NO: 12048 | GUUAAUU |
| SEQ ID NO: 12049 | GUUACAA |
| SEQ ID NO: 12050 | GUUACAC |
| SEQ ID NO: 12051 | GUUACAG |
| SEQ ID NO: 12052 | GUUACAU |
| SEQ ID NO: 12053 | GUUACCA |
| SEQ ID NO: 12054 | GUUACCC |
| SEQ ID NO: 12055 | GUUACCG |
| SEQ ID NO: 12056 | GUUACCU |
| SEQ ID NO: 12057 | GUUACGA |
| SEQ ID NO: 12058 | GUUACGC |
| SEQ ID NO: 12059 | GUUACGG |
| SEQ ID NO: 12060 | GUUACGU |
| SEQ ID NO: 12061 | GUUACUA |
| SEQ ID NO: 12062 | GUUACUC |
| SEQ ID NO: 12063 | GUUACUG |
| SEQ ID NO: 12064 | GUUACUU |
| SEQ ID NO: 12065 | GUUAGAA |
| SEQ ID NO: 12066 | GUUAGAC |
| SEQ ID NO: 12067 | GUUAGAG |
| SEQ ID NO: 12068 | GUUAGAU |
| SEQ ID NO: 12069 | GUUAGCA |
| SEQ ID NO: 12070 | GUUAGCC |
| SEQ ID NO: 12071 | GUUAGCG |
| SEQ ID NO: 12072 | GUUAGCU |
| SEQ ID NO: 12073 | GUUAGGA |
| SEQ ID NO: 12074 | GUUAGGC |
| SEQ ID NO: 12075 | GUUAGGG |
| SEQ ID NO: 12076 | GUUAGGU |
| SEQ ID NO: 12077 | GUUAGUA |
| SEQ ID NO: 12078 | GUUAGUC |
| SEQ ID NO: 12079 | GUUAGUG |
| SEQ ID NO: 12080 | GUUAGUU |
| SEQ ID NO: 12081 | GUUAUAA |
| SEQ ID NO: 12082 | GUUAUAC |
| SEQ ID NO: 12083 | GUUAUAG |
| SEQ ID NO: 12084 | GUUAUAU |
| SEQ ID NO: 12085 | GUUAUCA |
| SEQ ID NO: 12086 | GUUAUCC |
| SEQ ID NO: 12087 | GUUAUCG |
| SEQ ID NO: 12088 | GUUAUCU |
| SEQ ID NO: 12089 | GUUAUGA |
| SEQ ID NO: 12090 | GUUAUGC |
| SEQ ID NO: 12091 | GUUAUGG |
| SEQ ID NO: 12092 | GUUAUGU |
| SEQ ID NO: 12093 | GUUAUUA |
| SEQ ID NO: 12094 | GUUAUUC |
| SEQ ID NO: 12095 | GUUAUUG |
| SEQ ID NO: 12096 | GUUAUUU |
| SEQ ID NO: 12097 | GUUCAAA |
| SEQ ID NO: 12098 | GUUCAAC |
| SEQ ID NO: 12099 | GUUCAAG |
| SEQ ID NO: 12100 | GUUCAAU |
| SEQ ID NO: 12101 | GUUCACA |
| SEQ ID NO: 12102 | GUUCACC |
| SEQ ID NO: 12103 | GUUCACG |
| SEQ ID NO: 12104 | GUUCACU |
| SEQ ID NO: 12105 | GUUCAGA |
| SEQ ID NO: 12106 | GUUCAGC |
| SEQ ID NO: 12107 | GUUCAGG |
| SEQ ID NO: 12108 | GUUCAGU |
| SEQ ID NO: 12109 | GUUCAUA |
| SEQ ID NO: 12110 | GUUCAUC |
| SEQ ID NO: 12111 | GUUCAUG |
| SEQ ID NO: 12112 | GUUCAUU |
| SEQ ID NO: 12113 | GUUCCAA |
| SEQ ID NO: 12114 | GUUCCAC |
| SEQ ID NO: 12115 | GUUCCAG |
| SEQ ID NO: 12116 | GUUCCAU |
| SEQ ID NO: 12117 | GUUCCCA |
| SEQ ID NO: 12118 | GUUCCCC |
| SEQ ID NO: 12119 | GUUCCCG |
| SEQ ID NO: 12120 | GUUCCCU |
| SEQ ID NO: 12121 | GUUCCGA |
| SEQ ID NO: 12122 | GUUCCGC |
| SEQ ID NO: 12123 | GUUCCGG |
| SEQ ID NO: 12124 | GUUCCGU |
| SEQ ID NO: 12125 | GUUCCUA |
| SEQ ID NO: 12126 | GUUCCUC |
| SEQ ID NO: 12127 | GUUCCUG |
| SEQ ID NO: 12128 | GUUCCUU |
| SEQ ID NO: 12129 | GUUCGAA |
| SEQ ID NO: 12130 | GUUCGAC |
| SEQ ID NO: 12131 | GUUCGAG |
| SEQ ID NO: 12132 | GUUCGAU |
| SEQ ID NO: 12133 | GUUCGCA |
| SEQ ID NO: 12134 | GUUCGCC |
| SEQ ID NO: 12135 | GUUCGCG |
| SEQ ID NO: 12136 | GUUCGCU |
| SEQ ID NO: 12137 | GUUCGGA |
| SEQ ID NO: 12138 | GUUCGGC |
| SEQ ID NO: 12139 | GUUCGGG |
| SEQ ID NO: 12140 | GUUCGGU |
| SEQ ID NO: 12141 | GUUCGUA |
| SEQ ID NO: 12142 | GUUCGUC |
| SEQ ID NO: 12143 | GUUCGUG |
| SEQ ID NO: 12144 | GUUCGUU |
| SEQ ID NO: 12145 | GUUCUAA |
| SEQ ID NO: 12146 | GUUCUAC |
| SEQ ID NO: 12147 | GUUCUAG |
| SEQ ID NO: 12148 | GUUCUAU |
| SEQ ID NO: 12149 | GUUCUCA |
| SEQ ID NO: 12150 | GUUCUCC |
| SEQ ID NO: 12151 | GUUCUCG |
| SEQ ID NO: 12152 | GUUCUCU |
| SEQ ID NO: 12153 | GUUCUGA |
| SEQ ID NO: 12154 | GUUCUGC |
| SEQ ID NO: 12155 | GUUCUGG |
| SEQ ID NO: 12156 | GUUCUGU |
| SEQ ID NO: 12157 | GUUCUUA |
| SEQ ID NO: 12158 | GUUCUUC |
| SEQ ID NO: 12159 | GUUCUUG |
| SEQ ID NO: 12160 | GUUCUUU |
| SEQ ID NO: 12161 | GUUGAAA |
| SEQ ID NO: 12162 | GUUGAAC |
| SEQ ID NO: 12163 | GUUGAAG |
| SEQ ID NO: 12164 | GUUGAAU |
| SEQ ID NO: 12165 | GUUGACA |
| SEQ ID NO: 12166 | GUUGACC |
| SEQ ID NO: 12167 | GUUGACG |
| SEQ ID NO: 12168 | GUUGACU |
| SEQ ID NO: 12169 | GUUGAGA |
| SEQ ID NO: 12170 | GUUGAGC |
| SEQ ID NO: 12171 | GUUGAGG |
| SEQ ID NO: 12172 | GUUGAGU |
| SEQ ID NO: 12173 | GUUGAUA |
| SEQ ID NO: 12174 | GUUGAUC |
| SEQ ID NO: 12175 | GUUGAUG |
| SEQ ID NO: 12176 | GUUGAUU |
| SEQ ID NO: 12177 | GUUGCAA |
| SEQ ID NO: 12178 | GUUGCAC |
| SEQ ID NO: 12179 | GUUGCAG |
| SEQ ID NO: 12180 | GUUGCAU |
| SEQ ID NO: 12181 | GUUGCCA |
| SEQ ID NO: 12182 | GUUGCCC |
| SEQ ID NO: 12183 | GUUGCCG |
| SEQ ID NO: 12184 | GUUGCCU |
| SEQ ID NO: 12185 | GUUGCGA |
| SEQ ID NO: 12186 | GUUGCGC |
| SEQ ID NO: 12187 | GUUGCGG |
| SEQ ID NO: 12188 | GUUGCGU |
| SEQ ID NO: 12189 | GUUGCUA |
| SEQ ID NO: 12190 | GUUGCUC |
| SEQ ID NO: 12191 | GUUGCUG |
| SEQ ID NO: 12192 | GUUGCUU |
| SEQ ID NO: 12193 | GUUGGAA |
| SEQ ID NO: 12194 | GUUGGAC |
| SEQ ID NO: 12195 | GUUGGAG |
| SEQ ID NO: 12196 | GUUGGAU |
| SEQ ID NO: 12197 | GUUGGCA |
| SEQ ID NO: 12198 | GUUGGCC |
| SEQ ID NO: 12199 | GUUGGCG |
| SEQ ID NO: 12200 | GUUGGCU |
| SEQ ID NO: 12201 | GUUGGGA |
| SEQ ID NO: 12202 | GUUGGGC |
| SEQ ID NO: 12203 | GUUGGGG |
| SEQ ID NO: 12204 | GUUGGGU |
| SEQ ID NO: 12205 | GUUGGUA |
| SEQ ID NO: 12206 | GUUGGUC |
| SEQ ID NO: 12207 | GUUGGUG |
| SEQ ID NO: 12208 | GUUGGUU |
| SEQ ID NO: 12209 | GUUGUAA |
| SEQ ID NO: 12210 | GUUGUAC |
| SEQ ID NO: 12211 | GUUGUAG |
| SEQ ID NO: 12212 | GUUGUAU |
| SEQ ID NO: 12213 | GUUGUCA |
| SEQ ID NO: 12214 | GUUGUCC |
| SEQ ID NO: 12215 | GUUGUCG |
| SEQ ID NO: 12216 | GUUGUCU |
| SEQ ID NO: 12217 | GUUGUGA |
| SEQ ID NO: 12218 | GUUGUGC |
| SEQ ID NO: 12219 | GUUGUGG |
| SEQ ID NO: 12220 | GUUGUGU |
| SEQ ID NO: 12221 | GUUGUUA |
| SEQ ID NO: 12222 | GUUGUUC |
| SEQ ID NO: 12223 | GUUGUUG |
| SEQ ID NO: 12224 | GUUGUUU |
| SEQ ID NO: 12225 | GUUUAAA |
| SEQ ID NO: 12226 | GUUUAAC |
| SEQ ID NO: 12227 | GUUUAAG |
| SEQ ID NO: 12228 | GUUUAAU |
| SEQ ID NO: 12229 | GUUUACA |
| SEQ ID NO: 12230 | GUUUACC |
| SEQ ID NO: 12231 | GUUUACG |
| SEQ ID NO: 12232 | GUUUACU |
| SEQ ID NO: 12233 | GUUUAGA |
| SEQ ID NO: 12234 | GUUUAGC |
| SEQ ID NO: 12235 | GUUUAGG |
| SEQ ID NO: 12236 | GUUUAGU |
| SEQ ID NO: 12237 | GUUUAUA |
| SEQ ID NO: 12238 | GUUUAUC |
| SEQ ID NO: 12239 | GUUUAUG |
| SEQ ID NO: 12240 | GUUUAUU |

Table 41

| | | | | | | | | | | | |
|---|---|---|---|---|---|---|---|---|---|---|---|
| SEQ ID NO: 12241 | GUUUCAA | SEQ ID NO: 12292 | UAAAAAU | SEQ ID NO: 12343 | UAAAUCG | SEQ ID NO: 12394 | UAACGGC | SEQ ID NO: 12445 | UAAGCUA | SEQ ID NO: 12496 | UAAUAUU |
| SEQ ID NO: 12242 | GUUUCAC | SEQ ID NO: 12293 | UAAAACA | SEQ ID NO: 12344 | UAAAUCU | SEQ ID NO: 12395 | UAACGGG | SEQ ID NO: 12446 | UAAGCUC | SEQ ID NO: 12497 | UAAUCAA |
| SEQ ID NO: 12243 | GUUUCAG | SEQ ID NO: 12294 | UAAAACC | SEQ ID NO: 12345 | UAAAUGA | SEQ ID NO: 12396 | UAACGGU | SEQ ID NO: 12447 | UAAGCUG | SEQ ID NO: 12498 | UAAUCAC |
| SEQ ID NO: 12244 | GUUUCAU | SEQ ID NO: 12295 | UAAAACG | SEQ ID NO: 12346 | UAAAUGC | SEQ ID NO: 12397 | UAACGUA | SEQ ID NO: 12448 | UAAGCUU | SEQ ID NO: 12499 | UAAUCAG |
| SEQ ID NO: 12245 | GUUUCCA | SEQ ID NO: 12296 | UAAAACU | SEQ ID NO: 12347 | UAAAUGG | SEQ ID NO: 12398 | UAACGUC | SEQ ID NO: 12449 | UAAGGAA | SEQ ID NO: 12500 | UAAUCAU |
| SEQ ID NO: 12246 | GUUUCCC | SEQ ID NO: 12297 | UAAAAGA | SEQ ID NO: 12348 | UAAAUGU | SEQ ID NO: 12399 | UAACGUG | SEQ ID NO: 12450 | UAAGGAC | SEQ ID NO: 12501 | UAAUCCA |
| SEQ ID NO: 12247 | GUUUCCG | SEQ ID NO: 12298 | UAAAAGC | SEQ ID NO: 12349 | UAAAUUA | SEQ ID NO: 12400 | UAACGUU | SEQ ID NO: 12451 | UAAGGAG | SEQ ID NO: 12502 | UAAUCCC |
| SEQ ID NO: 12248 | GUUUCCU | SEQ ID NO: 12299 | UAAAAGG | SEQ ID NO: 12350 | UAAAUUC | SEQ ID NO: 12401 | UAACUAA | SEQ ID NO: 12452 | UAAGGAU | SEQ ID NO: 12503 | UAAUCCG |
| SEQ ID NO: 12249 | GUUUCGA | SEQ ID NO: 12300 | UAAAAGU | SEQ ID NO: 12351 | UAAAUUG | SEQ ID NO: 12402 | UAACUAC | SEQ ID NO: 12453 | UAAGGCA | SEQ ID NO: 12504 | UAAUCCU |
| SEQ ID NO: 12250 | GUUUCGC | SEQ ID NO: 12301 | UAAAAUA | SEQ ID NO: 12352 | UAAAUUU | SEQ ID NO: 12403 | UAACUAG | SEQ ID NO: 12454 | UAAGGCC | SEQ ID NO: 12505 | UAAUCGA |
| SEQ ID NO: 12251 | GUUUCGG | SEQ ID NO: 12302 | UAAAAUC | SEQ ID NO: 12353 | UAACAAA | SEQ ID NO: 12404 | UAACUAU | SEQ ID NO: 12455 | UAAGGCG | SEQ ID NO: 12506 | UAAUCGC |
| SEQ ID NO: 12252 | GUUUCGU | SEQ ID NO: 12303 | UAAAAUG | SEQ ID NO: 12354 | UAACAAC | SEQ ID NO: 12405 | UAACUCA | SEQ ID NO: 12456 | UAAGGCU | SEQ ID NO: 12507 | UAAUCGG |
| SEQ ID NO: 12253 | GUUUCUA | SEQ ID NO: 12304 | UAAAAUU | SEQ ID NO: 12355 | UAACAAG | SEQ ID NO: 12406 | UAACUCC | SEQ ID NO: 12457 | UAAGGGA | SEQ ID NO: 12508 | UAAUCGU |
| SEQ ID NO: 12254 | GUUUCUC | SEQ ID NO: 12305 | UAAACAA | SEQ ID NO: 12356 | UAACAAU | SEQ ID NO: 12407 | UAACUCG | SEQ ID NO: 12458 | UAAGGGC | SEQ ID NO: 12509 | UAAUCUA |
| SEQ ID NO: 12255 | GUUUCUG | SEQ ID NO: 12306 | UAAACAC | SEQ ID NO: 12357 | UAACACA | SEQ ID NO: 12408 | UAACUCU | SEQ ID NO: 12459 | UAAGGGG | SEQ ID NO: 12510 | UAAUCUC |
| SEQ ID NO: 12256 | GUUUCUU | SEQ ID NO: 12307 | UAAACAG | SEQ ID NO: 12358 | UAACACC | SEQ ID NO: 12409 | UAACUGA | SEQ ID NO: 12460 | UAAGGGU | SEQ ID NO: 12511 | UAAUCUG |
| SEQ ID NO: 12257 | GUUUGAA | SEQ ID NO: 12308 | UAAACAU | SEQ ID NO: 12359 | UAACACG | SEQ ID NO: 12410 | UAACUGC | SEQ ID NO: 12461 | UAAGGUA | SEQ ID NO: 12512 | UAAUCUU |
| SEQ ID NO: 12258 | GUUUGAC | SEQ ID NO: 12309 | UAAACCA | SEQ ID NO: 12360 | UAACACU | SEQ ID NO: 12411 | UAACUGG | SEQ ID NO: 12462 | UAAGGUC | SEQ ID NO: 12513 | UAAUGAA |
| SEQ ID NO: 12259 | GUUUGAG | SEQ ID NO: 12310 | UAAACCC | SEQ ID NO: 12361 | UAACAGA | SEQ ID NO: 12412 | UAACUGU | SEQ ID NO: 12463 | UAAGGUG | SEQ ID NO: 12514 | UAAUGAC |
| SEQ ID NO: 12260 | GUUUGAU | SEQ ID NO: 12311 | UAAACCG | SEQ ID NO: 12362 | UAACAGC | SEQ ID NO: 12413 | UAACUUA | SEQ ID NO: 12464 | UAAGGUU | SEQ ID NO: 12515 | UAAUGAG |
| SEQ ID NO: 12261 | GUUUGCA | SEQ ID NO: 12312 | UAAACCU | SEQ ID NO: 12363 | UAACAGG | SEQ ID NO: 12414 | UAACUUC | SEQ ID NO: 12465 | UAAGUAA | SEQ ID NO: 12516 | UAAUGAU |
| SEQ ID NO: 12262 | GUUUGCC | SEQ ID NO: 12313 | UAAACGA | SEQ ID NO: 12364 | UAACAGU | SEQ ID NO: 12415 | UAACUUG | SEQ ID NO: 12466 | UAAGUAC | SEQ ID NO: 12517 | UAAUGCA |
| SEQ ID NO: 12263 | GUUUGCG | SEQ ID NO: 12314 | UAAACGC | SEQ ID NO: 12365 | UAACAUA | SEQ ID NO: 12416 | UAACUUU | SEQ ID NO: 12467 | UAAGUAG | SEQ ID NO: 12518 | UAAUGCC |
| SEQ ID NO: 12264 | GUUUGCU | SEQ ID NO: 12315 | UAAACGG | SEQ ID NO: 12366 | UAACAUC | SEQ ID NO: 12417 | UAAGAAA | SEQ ID NO: 12468 | UAAGUAU | SEQ ID NO: 12519 | UAAUGCG |
| SEQ ID NO: 12265 | GUUUGGA | SEQ ID NO: 12316 | UAAACGU | SEQ ID NO: 12367 | UAACAUG | SEQ ID NO: 12418 | UAAGAAC | SEQ ID NO: 12469 | UAAGUCA | SEQ ID NO: 12520 | UAAUGCU |
| SEQ ID NO: 12266 | GUUUGGC | SEQ ID NO: 12317 | UAAACUA | SEQ ID NO: 12368 | UAACAUU | SEQ ID NO: 12419 | UAAGAAG | SEQ ID NO: 12470 | UAAGUCC | SEQ ID NO: 12521 | UAAUGGA |
| SEQ ID NO: 12267 | GUUUGGG | SEQ ID NO: 12318 | UAAACUC | SEQ ID NO: 12369 | UAACCAA | SEQ ID NO: 12420 | UAAGAAU | SEQ ID NO: 12471 | UAAGUCG | SEQ ID NO: 12522 | UAAUGGC |
| SEQ ID NO: 12268 | GUUUGGU | SEQ ID NO: 12319 | UAAACUG | SEQ ID NO: 12370 | UAACCAC | SEQ ID NO: 12421 | UAAGACA | SEQ ID NO: 12472 | UAAGUCU | SEQ ID NO: 12523 | UAAUGGG |
| SEQ ID NO: 12269 | GUUUGUA | SEQ ID NO: 12320 | UAAACUU | SEQ ID NO: 12371 | UAACCAG | SEQ ID NO: 12422 | UAAGACC | SEQ ID NO: 12473 | UAAGUGA | SEQ ID NO: 12524 | UAAUGGU |
| SEQ ID NO: 12270 | GUUUGUC | SEQ ID NO: 12321 | UAAAGAA | SEQ ID NO: 12372 | UAACCAU | SEQ ID NO: 12423 | UAAGACG | SEQ ID NO: 12474 | UAAGUGC | SEQ ID NO: 12525 | UAAUGUA |
| SEQ ID NO: 12271 | GUUUGUG | SEQ ID NO: 12322 | UAAAGAC | SEQ ID NO: 12373 | UAACCCA | SEQ ID NO: 12424 | UAAGACU | SEQ ID NO: 12475 | UAAGUGG | SEQ ID NO: 12526 | UAAUGUC |
| SEQ ID NO: 12272 | GUUUGUU | SEQ ID NO: 12323 | UAAAGAG | SEQ ID NO: 12374 | UAACCCC | SEQ ID NO: 12425 | UAAGAGA | SEQ ID NO: 12476 | UAAGUGU | SEQ ID NO: 12527 | UAAUGUG |
| SEQ ID NO: 12273 | GUUUUAA | SEQ ID NO: 12324 | UAAAGAU | SEQ ID NO: 12375 | UAACCCG | SEQ ID NO: 12426 | UAAGAGC | SEQ ID NO: 12477 | UAAGUUA | SEQ ID NO: 12528 | UAAUGUU |
| SEQ ID NO: 12274 | GUUUUAC | SEQ ID NO: 12325 | UAAAGCA | SEQ ID NO: 12376 | UAACCCU | SEQ ID NO: 12427 | UAAGAGG | SEQ ID NO: 12478 | UAAGUUC | SEQ ID NO: 12529 | UAAUUAA |
| SEQ ID NO: 12275 | GUUUUAG | SEQ ID NO: 12326 | UAAAGCC | SEQ ID NO: 12377 | UAACCGA | SEQ ID NO: 12428 | UAAGAGU | SEQ ID NO: 12479 | UAAGUUG | SEQ ID NO: 12530 | UAAUUAC |
| SEQ ID NO: 12276 | GUUUUAU | SEQ ID NO: 12327 | UAAAGCG | SEQ ID NO: 12378 | UAACCGC | SEQ ID NO: 12429 | UAAGAUA | SEQ ID NO: 12480 | UAAGUUU | SEQ ID NO: 12531 | UAAUUAG |
| SEQ ID NO: 12277 | GUUUUCA | SEQ ID NO: 12328 | UAAAGCU | SEQ ID NO: 12379 | UAACCGG | SEQ ID NO: 12430 | UAAGAUC | SEQ ID NO: 12481 | UAAUAAA | SEQ ID NO: 12532 | UAAUUAU |
| SEQ ID NO: 12278 | GUUUUCC | SEQ ID NO: 12329 | UAAAGGA | SEQ ID NO: 12380 | UAACCGU | SEQ ID NO: 12431 | UAAGAUG | SEQ ID NO: 12482 | UAAUAAC | SEQ ID NO: 12533 | UAAUUCA |
| SEQ ID NO: 12279 | GUUUUCG | SEQ ID NO: 12330 | UAAAGGC | SEQ ID NO: 12381 | UAACCUA | SEQ ID NO: 12432 | UAAGAUU | SEQ ID NO: 12483 | UAAUAAG | SEQ ID NO: 12534 | UAAUUCC |
| SEQ ID NO: 12280 | GUUUUCU | SEQ ID NO: 12331 | UAAAGGG | SEQ ID NO: 12382 | UAACCUC | SEQ ID NO: 12433 | UAAGCAA | SEQ ID NO: 12484 | UAAUAAU | SEQ ID NO: 12535 | UAAUUCG |
| SEQ ID NO: 12281 | GUUUUGA | SEQ ID NO: 12332 | UAAAGGU | SEQ ID NO: 12383 | UAACCUG | SEQ ID NO: 12434 | UAAGCAC | SEQ ID NO: 12485 | UAAUACA | SEQ ID NO: 12536 | UAAUUCU |
| SEQ ID NO: 12282 | GUUUUGC | SEQ ID NO: 12333 | UAAAGUA | SEQ ID NO: 12384 | UAACCUU | SEQ ID NO: 12435 | UAAGCAG | SEQ ID NO: 12486 | UAAUACC | SEQ ID NO: 12537 | UAAUUGA |
| SEQ ID NO: 12283 | GUUUUGG | SEQ ID NO: 12334 | UAAAGUC | SEQ ID NO: 12385 | UAACGAA | SEQ ID NO: 12436 | UAAGCAU | SEQ ID NO: 12487 | UAAUACG | SEQ ID NO: 12538 | UAAUUGC |
| SEQ ID NO: 12284 | GUUUUGU | SEQ ID NO: 12335 | UAAAGUG | SEQ ID NO: 12386 | UAACGAC | SEQ ID NO: 12437 | UAAGCCA | SEQ ID NO: 12488 | UAAUACU | SEQ ID NO: 12539 | UAAUUGG |
| SEQ ID NO: 12285 | GUUUUUA | SEQ ID NO: 12336 | UAAAGUU | SEQ ID NO: 12387 | UAACGAG | SEQ ID NO: 12438 | UAAGCCC | SEQ ID NO: 12489 | UAAUAGA | SEQ ID NO: 12540 | UAAUUGU |
| SEQ ID NO: 12286 | GUUUUUC | SEQ ID NO: 12337 | UAAAUAA | SEQ ID NO: 12388 | UAACGAU | SEQ ID NO: 12439 | UAAGCCG | SEQ ID NO: 12490 | UAAUAGC | SEQ ID NO: 12541 | UAAUUUA |
| SEQ ID NO: 12287 | GUUUUUG | SEQ ID NO: 12338 | UAAAUAC | SEQ ID NO: 12389 | UAACGCA | SEQ ID NO: 12440 | UAAGCCU | SEQ ID NO: 12491 | UAAUAGG | SEQ ID NO: 12542 | UAAUUUC |
| SEQ ID NO: 12288 | GUUUUUU | SEQ ID NO: 12339 | UAAAUAG | SEQ ID NO: 12390 | UAACGCC | SEQ ID NO: 12441 | UAAGCGA | SEQ ID NO: 12492 | UAAUAGU | SEQ ID NO: 12543 | UAAUUUG |
| SEQ ID NO: 12289 | UAAAAAA | SEQ ID NO: 12340 | UAAAUAU | SEQ ID NO: 12391 | UAACGCG | SEQ ID NO: 12442 | UAAGCGC | SEQ ID NO: 12493 | UAAUAUA | SEQ ID NO: 12544 | UAAUUUU |
| SEQ ID NO: 12290 | UAAAAAC | SEQ ID NO: 12341 | UAAAUCA | SEQ ID NO: 12392 | UAACGCU | SEQ ID NO: 12443 | UAAGCGG | SEQ ID NO: 12494 | UAAUAUC | SEQ ID NO: 12545 | UACAAAA |
| SEQ ID NO: 12291 | UAAAAAG | SEQ ID NO: 12342 | UAAAUCC | SEQ ID NO: 12393 | UAACGGA | SEQ ID NO: 12444 | UAAGCGU | SEQ ID NO: 12495 | UAAUAUG | SEQ ID NO: 12546 | UACAAAC |

Table 42

| | |
|---|---|
| SEQ ID NO: 12547 | UACAAAG |
| SEQ ID NO: 12548 | UACAAAU |
| SEQ ID NO: 12549 | UACAACA |
| SEQ ID NO: 12550 | UACAACC |
| SEQ ID NO: 12551 | UACAACG |
| SEQ ID NO: 12552 | UACAACU |
| SEQ ID NO: 12553 | UACAAGA |
| SEQ ID NO: 12554 | UACAAGC |
| SEQ ID NO: 12555 | UACAAGG |
| SEQ ID NO: 12556 | UACAAGU |
| SEQ ID NO: 12557 | UACAAUA |
| SEQ ID NO: 12558 | UACAAUC |
| SEQ ID NO: 12559 | UACAAUG |
| SEQ ID NO: 12560 | UACAAUU |
| SEQ ID NO: 12561 | UACACAA |
| SEQ ID NO: 12562 | UACACAC |
| SEQ ID NO: 12563 | UACACAG |
| SEQ ID NO: 12564 | UACACAU |
| SEQ ID NO: 12565 | UACACCA |
| SEQ ID NO: 12566 | UACACCC |
| SEQ ID NO: 12567 | UACACCG |
| SEQ ID NO: 12568 | UACACCU |
| SEQ ID NO: 12569 | UACACGA |
| SEQ ID NO: 12570 | UACACGC |
| SEQ ID NO: 12571 | UACACGG |
| SEQ ID NO: 12572 | UACACGU |
| SEQ ID NO: 12573 | UACACUA |
| SEQ ID NO: 12574 | UACACUC |
| SEQ ID NO: 12575 | UACACUG |
| SEQ ID NO: 12576 | UACACUU |
| SEQ ID NO: 12577 | UACAGAA |
| SEQ ID NO: 12578 | UACAGAC |
| SEQ ID NO: 12579 | UACAGAG |
| SEQ ID NO: 12580 | UACAGAU |
| SEQ ID NO: 12581 | UACAGCA |
| SEQ ID NO: 12582 | UACAGCC |
| SEQ ID NO: 12583 | UACAGCG |
| SEQ ID NO: 12584 | UACAGCU |
| SEQ ID NO: 12585 | UACAGGA |
| SEQ ID NO: 12586 | UACAGGC |
| SEQ ID NO: 12587 | UACAGGG |
| SEQ ID NO: 12588 | UACAGGU |
| SEQ ID NO: 12589 | UACAGUA |
| SEQ ID NO: 12590 | UACAGUC |
| SEQ ID NO: 12591 | UACAGUG |
| SEQ ID NO: 12592 | UACAGUU |
| SEQ ID NO: 12593 | UACAUAA |
| SEQ ID NO: 12594 | UACAUAC |
| SEQ ID NO: 12595 | UACAUAG |
| SEQ ID NO: 12596 | UACAUAU |
| SEQ ID NO: 12597 | UACAUCA |

| | |
|---|---|
| SEQ ID NO: 12598 | UACAUCC |
| SEQ ID NO: 12599 | UACAUCG |
| SEQ ID NO: 12600 | UACAUCU |
| SEQ ID NO: 12601 | UACAUGA |
| SEQ ID NO: 12602 | UACAUGC |
| SEQ ID NO: 12603 | UACAUGG |
| SEQ ID NO: 12604 | UACAUGU |
| SEQ ID NO: 12605 | UACAUUA |
| SEQ ID NO: 12606 | UACAUUC |
| SEQ ID NO: 12607 | UACAUUG |
| SEQ ID NO: 12608 | UACAUUU |
| SEQ ID NO: 12609 | UACCAAA |
| SEQ ID NO: 12610 | UACCAAC |
| SEQ ID NO: 12611 | UACCAAG |
| SEQ ID NO: 12612 | UACCAAU |
| SEQ ID NO: 12613 | UACCACA |
| SEQ ID NO: 12614 | UACCACC |
| SEQ ID NO: 12615 | UACCACG |
| SEQ ID NO: 12616 | UACCACU |
| SEQ ID NO: 12617 | UACCAGA |
| SEQ ID NO: 12618 | UACCAGC |
| SEQ ID NO: 12619 | UACCAGG |
| SEQ ID NO: 12620 | UACCAGU |
| SEQ ID NO: 12621 | UACCAUA |
| SEQ ID NO: 12622 | UACCAUC |
| SEQ ID NO: 12623 | UACCAUG |
| SEQ ID NO: 12624 | UACCAUU |
| SEQ ID NO: 12625 | UACCCAA |
| SEQ ID NO: 12626 | UACCCAC |
| SEQ ID NO: 12627 | UACCCAG |
| SEQ ID NO: 12628 | UACCCAU |
| SEQ ID NO: 12629 | UACCCCA |
| SEQ ID NO: 12630 | UACCCCC |
| SEQ ID NO: 12631 | UACCCCG |
| SEQ ID NO: 12632 | UACCCCU |
| SEQ ID NO: 12633 | UACCCGA |
| SEQ ID NO: 12634 | UACCCGC |
| SEQ ID NO: 12635 | UACCCGG |
| SEQ ID NO: 12636 | UACCCGU |
| SEQ ID NO: 12637 | UACCCUA |
| SEQ ID NO: 12638 | UACCCUC |
| SEQ ID NO: 12639 | UACCCUG |
| SEQ ID NO: 12640 | UACCCUU |
| SEQ ID NO: 12641 | UACCGAA |
| SEQ ID NO: 12642 | UACCGAC |
| SEQ ID NO: 12643 | UACCGAG |
| SEQ ID NO: 12644 | UACCGAU |
| SEQ ID NO: 12645 | UACCGCA |
| SEQ ID NO: 12646 | UACCGCC |
| SEQ ID NO: 12647 | UACCGCG |
| SEQ ID NO: 12648 | UACCGCU |

| | |
|---|---|
| SEQ ID NO: 12649 | UACCGGA |
| SEQ ID NO: 12650 | UACCGGC |
| SEQ ID NO: 12651 | UACCGGG |
| SEQ ID NO: 12652 | UACCGGU |
| SEQ ID NO: 12653 | UACCGUA |
| SEQ ID NO: 12654 | UACCGUC |
| SEQ ID NO: 12655 | UACCGUG |
| SEQ ID NO: 12656 | UACCGUU |
| SEQ ID NO: 12657 | UACCUAA |
| SEQ ID NO: 12658 | UACCUAC |
| SEQ ID NO: 12659 | UACCUAG |
| SEQ ID NO: 12660 | UACCUAU |
| SEQ ID NO: 12661 | UACCUCA |
| SEQ ID NO: 12662 | UACCUCC |
| SEQ ID NO: 12663 | UACCUCG |
| SEQ ID NO: 12664 | UACCUCU |
| SEQ ID NO: 12665 | UACCUGA |
| SEQ ID NO: 12666 | UACCUGC |
| SEQ ID NO: 12667 | UACCUGG |
| SEQ ID NO: 12668 | UACCUGU |
| SEQ ID NO: 12669 | UACCUUA |
| SEQ ID NO: 12670 | UACCUUC |
| SEQ ID NO: 12671 | UACCUUG |
| SEQ ID NO: 12672 | UACCUUU |
| SEQ ID NO: 12673 | UACGAAA |
| SEQ ID NO: 12674 | UACGAAC |
| SEQ ID NO: 12675 | UACGAAG |
| SEQ ID NO: 12676 | UACGAAU |
| SEQ ID NO: 12677 | UACGACA |
| SEQ ID NO: 12678 | UACGACC |
| SEQ ID NO: 12679 | UACGACG |
| SEQ ID NO: 12680 | UACGACU |
| SEQ ID NO: 12681 | UACGAGA |
| SEQ ID NO: 12682 | UACGAGC |
| SEQ ID NO: 12683 | UACGAGG |
| SEQ ID NO: 12684 | UACGAGU |
| SEQ ID NO: 12685 | UACGAUA |
| SEQ ID NO: 12686 | UACGAUC |
| SEQ ID NO: 12687 | UACGAUG |
| SEQ ID NO: 12688 | UACGAUU |
| SEQ ID NO: 12689 | UACGCAA |
| SEQ ID NO: 12690 | UACGCAC |
| SEQ ID NO: 12691 | UACGCAG |
| SEQ ID NO: 12692 | UACGCAU |
| SEQ ID NO: 12693 | UACGCCA |
| SEQ ID NO: 12694 | UACGCCC |
| SEQ ID NO: 12695 | UACGCCG |
| SEQ ID NO: 12696 | UACGCCU |
| SEQ ID NO: 12697 | UACGCGA |
| SEQ ID NO: 12698 | UACGCGC |
| SEQ ID NO: 12699 | UACGCGG |

| | |
|---|---|
| SEQ ID NO: 12700 | UACGCGU |
| SEQ ID NO: 12701 | UACGCUA |
| SEQ ID NO: 12702 | UACGCUC |
| SEQ ID NO: 12703 | UACGCUG |
| SEQ ID NO: 12704 | UACGCUU |
| SEQ ID NO: 12705 | UACGGAA |
| SEQ ID NO: 12706 | UACGGAC |
| SEQ ID NO: 12707 | UACGGAG |
| SEQ ID NO: 12708 | UACGGAU |
| SEQ ID NO: 12709 | UACGGCA |
| SEQ ID NO: 12710 | UACGGCC |
| SEQ ID NO: 12711 | UACGGCG |
| SEQ ID NO: 12712 | UACGGCU |
| SEQ ID NO: 12713 | UACGGGA |
| SEQ ID NO: 12714 | UACGGGC |
| SEQ ID NO: 12715 | UACGGGG |
| SEQ ID NO: 12716 | UACGGGU |
| SEQ ID NO: 12717 | UACGGUA |
| SEQ ID NO: 12718 | UACGGUC |
| SEQ ID NO: 12719 | UACGGUG |
| SEQ ID NO: 12720 | UACGGUU |
| SEQ ID NO: 12721 | UACGUAA |
| SEQ ID NO: 12722 | UACGUAC |
| SEQ ID NO: 12723 | UACGUAG |
| SEQ ID NO: 12724 | UACGUAU |
| SEQ ID NO: 12725 | UACGUCA |
| SEQ ID NO: 12726 | UACGUCC |
| SEQ ID NO: 12727 | UACGUCG |
| SEQ ID NO: 12728 | UACGUCU |
| SEQ ID NO: 12729 | UACGUGA |
| SEQ ID NO: 12730 | UACGUGC |
| SEQ ID NO: 12731 | UACGUGG |
| SEQ ID NO: 12732 | UACGUGU |
| SEQ ID NO: 12733 | UACGUUA |
| SEQ ID NO: 12734 | UACGUUC |
| SEQ ID NO: 12735 | UACGUUG |
| SEQ ID NO: 12736 | UACGUUU |
| SEQ ID NO: 12737 | UACUAAA |
| SEQ ID NO: 12738 | UACUAAC |
| SEQ ID NO: 12739 | UACUAAG |
| SEQ ID NO: 12740 | UACUAAU |
| SEQ ID NO: 12741 | UACUACA |
| SEQ ID NO: 12742 | UACUACC |
| SEQ ID NO: 12743 | UACUACG |
| SEQ ID NO: 12744 | UACUACU |
| SEQ ID NO: 12745 | UACUAGA |
| SEQ ID NO: 12746 | UACUAGC |
| SEQ ID NO: 12747 | UACUAGG |
| SEQ ID NO: 12748 | UACUAGU |
| SEQ ID NO: 12749 | UACUAUA |
| SEQ ID NO: 12750 | UACUAUC |

| | |
|---|---|
| SEQ ID NO: 12751 | UACUAUG |
| SEQ ID NO: 12752 | UACUAUU |
| SEQ ID NO: 12753 | UACUCAA |
| SEQ ID NO: 12754 | UACUCAC |
| SEQ ID NO: 12755 | UACUCAG |
| SEQ ID NO: 12756 | UACUCAU |
| SEQ ID NO: 12757 | UACUCCA |
| SEQ ID NO: 12758 | UACUCCC |
| SEQ ID NO: 12759 | UACUCCG |
| SEQ ID NO: 12760 | UACUCCU |
| SEQ ID NO: 12761 | UACUCGA |
| SEQ ID NO: 12762 | UACUCGC |
| SEQ ID NO: 12763 | UACUCGG |
| SEQ ID NO: 12764 | UACUCGU |
| SEQ ID NO: 12765 | UACUCUA |
| SEQ ID NO: 12766 | UACUCUC |
| SEQ ID NO: 12767 | UACUCUG |
| SEQ ID NO: 12768 | UACUCUU |
| SEQ ID NO: 12769 | UACUGAA |
| SEQ ID NO: 12770 | UACUGAC |
| SEQ ID NO: 12771 | UACUGAG |
| SEQ ID NO: 12772 | UACUGAU |
| SEQ ID NO: 12773 | UACUGCA |
| SEQ ID NO: 12774 | UACUGCC |
| SEQ ID NO: 12775 | UACUGCG |
| SEQ ID NO: 12776 | UACUGCU |
| SEQ ID NO: 12777 | UACUGGA |
| SEQ ID NO: 12778 | UACUGGC |
| SEQ ID NO: 12779 | UACUGGG |
| SEQ ID NO: 12780 | UACUGGU |
| SEQ ID NO: 12781 | UACUGUA |
| SEQ ID NO: 12782 | UACUGUC |
| SEQ ID NO: 12783 | UACUGUG |
| SEQ ID NO: 12784 | UACUGUU |
| SEQ ID NO: 12785 | UACUUAA |
| SEQ ID NO: 12786 | UACUUAC |
| SEQ ID NO: 12787 | UACUUAG |
| SEQ ID NO: 12788 | UACUUAU |
| SEQ ID NO: 12789 | UACUUCA |
| SEQ ID NO: 12790 | UACUUCC |
| SEQ ID NO: 12791 | UACUUCG |
| SEQ ID NO: 12792 | UACUUCU |
| SEQ ID NO: 12793 | UACUUGA |
| SEQ ID NO: 12794 | UACUUGC |
| SEQ ID NO: 12795 | UACUUGG |
| SEQ ID NO: 12796 | UACUUGU |
| SEQ ID NO: 12797 | UACUUUA |
| SEQ ID NO: 12798 | UACUUUC |
| SEQ ID NO: 12799 | UACUUUG |
| SEQ ID NO: 12800 | UACUUUU |
| SEQ ID NO: 12801 | UAGAAAA |

| | |
|---|---|
| SEQ ID NO: 12802 | UAGAAAC |
| SEQ ID NO: 12803 | UAGAAAG |
| SEQ ID NO: 12804 | UAGAAAU |
| SEQ ID NO: 12805 | UAGAACA |
| SEQ ID NO: 12806 | UAGAACC |
| SEQ ID NO: 12807 | UAGAACG |
| SEQ ID NO: 12808 | UAGAACU |
| SEQ ID NO: 12809 | UAGAAGA |
| SEQ ID NO: 12810 | UAGAAGC |
| SEQ ID NO: 12811 | UAGAAGG |
| SEQ ID NO: 12812 | UAGAAGU |
| SEQ ID NO: 12813 | UAGAAUA |
| SEQ ID NO: 12814 | UAGAAUC |
| SEQ ID NO: 12815 | UAGAAUG |
| SEQ ID NO: 12816 | UAGAAUU |
| SEQ ID NO: 12817 | UAGACAA |
| SEQ ID NO: 12818 | UAGACAC |
| SEQ ID NO: 12819 | UAGACAG |
| SEQ ID NO: 12820 | UAGACAU |
| SEQ ID NO: 12821 | UAGACCA |
| SEQ ID NO: 12822 | UAGACCC |
| SEQ ID NO: 12823 | UAGACCG |
| SEQ ID NO: 12824 | UAGACCU |
| SEQ ID NO: 12825 | UAGACGA |
| SEQ ID NO: 12826 | UAGACGC |
| SEQ ID NO: 12827 | UAGACGG |
| SEQ ID NO: 12828 | UAGACGU |
| SEQ ID NO: 12829 | UAGACUA |
| SEQ ID NO: 12830 | UAGACUC |
| SEQ ID NO: 12831 | UAGACUG |
| SEQ ID NO: 12832 | UAGACUU |
| SEQ ID NO: 12833 | UAGAGAA |
| SEQ ID NO: 12834 | UAGAGAC |
| SEQ ID NO: 12835 | UAGAGAG |
| SEQ ID NO: 12836 | UAGAGAU |
| SEQ ID NO: 12837 | UAGAGCA |
| SEQ ID NO: 12838 | UAGAGCC |
| SEQ ID NO: 12839 | UAGAGCG |
| SEQ ID NO: 12840 | UAGAGCU |
| SEQ ID NO: 12841 | UAGAGGA |
| SEQ ID NO: 12842 | UAGAGGC |
| SEQ ID NO: 12843 | UAGAGGG |
| SEQ ID NO: 12844 | UAGAGGU |
| SEQ ID NO: 12845 | UAGAGUA |
| SEQ ID NO: 12846 | UAGAGUC |
| SEQ ID NO: 12847 | UAGAGUG |
| SEQ ID NO: 12848 | UAGAGUU |
| SEQ ID NO: 12849 | UAGAUAA |
| SEQ ID NO: 12850 | UAGAUAC |
| SEQ ID NO: 12851 | UAGAUAG |
| SEQ ID NO: 12852 | UAGAUAU |

Table 43

| | | | | | | | | | | | |
|---|---|---|---|---|---|---|---|---|---|---|---|
| SEQ ID NO: 12853 | UAGAUCA | SEQ ID NO: 12904 | UAGCGCU | SEQ ID NO: 12955 | UAGGCGG | SEQ ID NO: 13006 | UAGUAUC | SEQ ID NO: 13057 | UAUAAAA | SEQ ID NO: 13108 | UAUAUAU |
| SEQ ID NO: 12854 | UAGAUCC | SEQ ID NO: 12905 | UAGCGGA | SEQ ID NO: 12956 | UAGGCGU | SEQ ID NO: 13007 | UAGUAUG | SEQ ID NO: 13058 | UAUAAAC | SEQ ID NO: 13109 | UAUAUCA |
| SEQ ID NO: 12855 | UAGAUCG | SEQ ID NO: 12906 | UAGCGGC | SEQ ID NO: 12957 | UAGGCUA | SEQ ID NO: 13008 | UAGUAUU | SEQ ID NO: 13059 | UAUAAAG | SEQ ID NO: 13110 | UAUAUCC |
| SEQ ID NO: 12856 | UAGAUCU | SEQ ID NO: 12907 | UAGCGGG | SEQ ID NO: 12958 | UAGGCUC | SEQ ID NO: 13009 | UAGUCAA | SEQ ID NO: 13060 | UAUAAAU | SEQ ID NO: 13111 | UAUAUCG |
| SEQ ID NO: 12857 | UAGAUGA | SEQ ID NO: 12908 | UAGCGGU | SEQ ID NO: 12959 | UAGGCUG | SEQ ID NO: 13010 | UAGUCAC | SEQ ID NO: 13061 | UAUAACA | SEQ ID NO: 13112 | UAUAUCU |
| SEQ ID NO: 12858 | UAGAUGC | SEQ ID NO: 12909 | UAGCGUA | SEQ ID NO: 12960 | UAGGCUU | SEQ ID NO: 13011 | UAGUCAG | SEQ ID NO: 13062 | UAUAACC | SEQ ID NO: 13113 | UAUAUGA |
| SEQ ID NO: 12859 | UAGAUGG | SEQ ID NO: 12910 | UAGCGUC | SEQ ID NO: 12961 | UAGGGAA | SEQ ID NO: 13012 | UAGUCAU | SEQ ID NO: 13063 | UAUAACG | SEQ ID NO: 13114 | UAUAUGC |
| SEQ ID NO: 12860 | UAGAUGU | SEQ ID NO: 12911 | UAGCGUG | SEQ ID NO: 12962 | UAGGGAC | SEQ ID NO: 13013 | UAGUCCA | SEQ ID NO: 13064 | UAUAACU | SEQ ID NO: 13115 | UAUAUGG |
| SEQ ID NO: 12861 | UAGAUUA | SEQ ID NO: 12912 | UAGCGUU | SEQ ID NO: 12963 | UAGGGAG | SEQ ID NO: 13014 | UAGUCCC | SEQ ID NO: 13065 | UAUAAGA | SEQ ID NO: 13116 | UAUAUGU |
| SEQ ID NO: 12862 | UAGAUUC | SEQ ID NO: 12913 | UAGCUAA | SEQ ID NO: 12964 | UAGGGAU | SEQ ID NO: 13015 | UAGUCCG | SEQ ID NO: 13066 | UAUAAGC | SEQ ID NO: 13117 | UAUAUUA |
| SEQ ID NO: 12863 | UAGAUUG | SEQ ID NO: 12914 | UAGCUAC | SEQ ID NO: 12965 | UAGGGCA | SEQ ID NO: 13016 | UAGUCCU | SEQ ID NO: 13067 | UAUAAGG | SEQ ID NO: 13118 | UAUAUUC |
| SEQ ID NO: 12864 | UAGAUUU | SEQ ID NO: 12915 | UAGCUAG | SEQ ID NO: 12966 | UAGGGCC | SEQ ID NO: 13017 | UAGUCGA | SEQ ID NO: 13068 | UAUAAGU | SEQ ID NO: 13119 | UAUAUUG |
| SEQ ID NO: 12865 | UAGCAAA | SEQ ID NO: 12916 | UAGCUAU | SEQ ID NO: 12967 | UAGGGCG | SEQ ID NO: 13018 | UAGUCGC | SEQ ID NO: 13069 | UAUAAUA | SEQ ID NO: 13120 | UAUAUUU |
| SEQ ID NO: 12866 | UAGCAAC | SEQ ID NO: 12917 | UAGCUCA | SEQ ID NO: 12968 | UAGGGCU | SEQ ID NO: 13019 | UAGUCGG | SEQ ID NO: 13070 | UAUAAUC | SEQ ID NO: 13121 | UAUCAAA |
| SEQ ID NO: 12867 | UAGCAAG | SEQ ID NO: 12918 | UAGCUCC | SEQ ID NO: 12969 | UAGGGGA | SEQ ID NO: 13020 | UAGUCGU | SEQ ID NO: 13071 | UAUAAUG | SEQ ID NO: 13122 | UAUCAAC |
| SEQ ID NO: 12868 | UAGCAAU | SEQ ID NO: 12919 | UAGCUCG | SEQ ID NO: 12970 | UAGGGGC | SEQ ID NO: 13021 | UAGUCUA | SEQ ID NO: 13072 | UAUAAUU | SEQ ID NO: 13123 | UAUCAAG |
| SEQ ID NO: 12869 | UAGCACA | SEQ ID NO: 12920 | UAGCUCU | SEQ ID NO: 12971 | UAGGGGG | SEQ ID NO: 13022 | UAGUCUC | SEQ ID NO: 13073 | UAUACAA | SEQ ID NO: 13124 | UAUCAAU |
| SEQ ID NO: 12870 | UAGCACC | SEQ ID NO: 12921 | UAGCUGA | SEQ ID NO: 12972 | UAGGGGU | SEQ ID NO: 13023 | UAGUCUG | SEQ ID NO: 13074 | UAUACAC | SEQ ID NO: 13125 | UAUCACA |
| SEQ ID NO: 12871 | UAGCACG | SEQ ID NO: 12922 | UAGCUGC | SEQ ID NO: 12973 | UAGGGUA | SEQ ID NO: 13024 | UAGUCUU | SEQ ID NO: 13075 | UAUACAG | SEQ ID NO: 13126 | UAUCACC |
| SEQ ID NO: 12872 | UAGCACU | SEQ ID NO: 12923 | UAGCUGG | SEQ ID NO: 12974 | UAGGGUC | SEQ ID NO: 13025 | UAGUGAA | SEQ ID NO: 13076 | UAUACAU | SEQ ID NO: 13127 | UAUCACG |
| SEQ ID NO: 12873 | UAGCAGA | SEQ ID NO: 12924 | UAGCUGU | SEQ ID NO: 12975 | UAGGGUG | SEQ ID NO: 13026 | UAGUGAC | SEQ ID NO: 13077 | UAUACCA | SEQ ID NO: 13128 | UAUCACU |
| SEQ ID NO: 12874 | UAGCAGC | SEQ ID NO: 12925 | UAGCUUA | SEQ ID NO: 12976 | UAGGGUU | SEQ ID NO: 13027 | UAGUGAG | SEQ ID NO: 13078 | UAUACCC | SEQ ID NO: 13129 | UAUCAGA |
| SEQ ID NO: 12875 | UAGCAGG | SEQ ID NO: 12926 | UAGCUUC | SEQ ID NO: 12977 | UAGGUAA | SEQ ID NO: 13028 | UAGUGAU | SEQ ID NO: 13079 | UAUACCG | SEQ ID NO: 13130 | UAUCAGC |
| SEQ ID NO: 12876 | UAGCAGU | SEQ ID NO: 12927 | UAGCUUG | SEQ ID NO: 12978 | UAGGUAC | SEQ ID NO: 13029 | UAGUGCA | SEQ ID NO: 13080 | UAUACCU | SEQ ID NO: 13131 | UAUCAGG |
| SEQ ID NO: 12877 | UAGCAUA | SEQ ID NO: 12928 | UAGCUUU | SEQ ID NO: 12979 | UAGGUAG | SEQ ID NO: 13030 | UAGUGCC | SEQ ID NO: 13081 | UAUACGA | SEQ ID NO: 13132 | UAUCAGU |
| SEQ ID NO: 12878 | UAGCAUC | SEQ ID NO: 12929 | UAGGAAA | SEQ ID NO: 12980 | UAGGUAU | SEQ ID NO: 13031 | UAGUGCG | SEQ ID NO: 13082 | UAUACGC | SEQ ID NO: 13133 | UAUCAUA |
| SEQ ID NO: 12879 | UAGCAUG | SEQ ID NO: 12930 | UAGGAAC | SEQ ID NO: 12981 | UAGGUCA | SEQ ID NO: 13032 | UAGUGCU | SEQ ID NO: 13083 | UAUACGG | SEQ ID NO: 13134 | UAUCAUC |
| SEQ ID NO: 12880 | UAGCAUU | SEQ ID NO: 12931 | UAGGAAG | SEQ ID NO: 12982 | UAGGUCC | SEQ ID NO: 13033 | UAGUGGA | SEQ ID NO: 13084 | UAUACGU | SEQ ID NO: 13135 | UAUCAUG |
| SEQ ID NO: 12881 | UAGCCAA | SEQ ID NO: 12932 | UAGGAAU | SEQ ID NO: 12983 | UAGGUCG | SEQ ID NO: 13034 | UAGUGGC | SEQ ID NO: 13085 | UAUACUA | SEQ ID NO: 13136 | UAUCAUU |
| SEQ ID NO: 12882 | UAGCCAC | SEQ ID NO: 12933 | UAGGACA | SEQ ID NO: 12984 | UAGGUCU | SEQ ID NO: 13035 | UAGUGGG | SEQ ID NO: 13086 | UAUACUC | SEQ ID NO: 13137 | UAUCCAA |
| SEQ ID NO: 12883 | UAGCCAG | SEQ ID NO: 12934 | UAGGACC | SEQ ID NO: 12985 | UAGGUGA | SEQ ID NO: 13036 | UAGUGGU | SEQ ID NO: 13087 | UAUACUG | SEQ ID NO: 13138 | UAUCCAC |
| SEQ ID NO: 12884 | UAGCCAU | SEQ ID NO: 12935 | UAGGACG | SEQ ID NO: 12986 | UAGGUGC | SEQ ID NO: 13037 | UAGUGUA | SEQ ID NO: 13088 | UAUACUU | SEQ ID NO: 13139 | UAUCCAG |
| SEQ ID NO: 12885 | UAGCCCA | SEQ ID NO: 12936 | UAGGACU | SEQ ID NO: 12987 | UAGGUGG | SEQ ID NO: 13038 | UAGUGUC | SEQ ID NO: 13089 | UAUAGAA | SEQ ID NO: 13140 | UAUCCAU |
| SEQ ID NO: 12886 | UAGCCCC | SEQ ID NO: 12937 | UAGGAGA | SEQ ID NO: 12988 | UAGGUGU | SEQ ID NO: 13039 | UAGUGUG | SEQ ID NO: 13090 | UAUAGAC | SEQ ID NO: 13141 | UAUCCCA |
| SEQ ID NO: 12887 | UAGCCCG | SEQ ID NO: 12938 | UAGGAGC | SEQ ID NO: 12989 | UAGGUUA | SEQ ID NO: 13040 | UAGUGUU | SEQ ID NO: 13091 | UAUAGAG | SEQ ID NO: 13142 | UAUCCCC |
| SEQ ID NO: 12888 | UAGCCCU | SEQ ID NO: 12939 | UAGGAGG | SEQ ID NO: 12990 | UAGGUUC | SEQ ID NO: 13041 | UAGUUAA | SEQ ID NO: 13092 | UAUAGAU | SEQ ID NO: 13143 | UAUCCCG |
| SEQ ID NO: 12889 | UAGCCGA | SEQ ID NO: 12940 | UAGGAGU | SEQ ID NO: 12991 | UAGGUUG | SEQ ID NO: 13042 | UAGUUAC | SEQ ID NO: 13093 | UAUAGCA | SEQ ID NO: 13144 | UAUCCCU |
| SEQ ID NO: 12890 | UAGCCGC | SEQ ID NO: 12941 | UAGGAUA | SEQ ID NO: 12992 | UAGGUUU | SEQ ID NO: 13043 | UAGUUAG | SEQ ID NO: 13094 | UAUAGCC | SEQ ID NO: 13145 | UAUCCGA |
| SEQ ID NO: 12891 | UAGCCGG | SEQ ID NO: 12942 | UAGGAUC | SEQ ID NO: 12993 | UAGUAAA | SEQ ID NO: 13044 | UAGUUAU | SEQ ID NO: 13095 | UAUAGCG | SEQ ID NO: 13146 | UAUCCGC |
| SEQ ID NO: 12892 | UAGCCGU | SEQ ID NO: 12943 | UAGGAUG | SEQ ID NO: 12994 | UAGUAAC | SEQ ID NO: 13045 | UAGUUCA | SEQ ID NO: 13096 | UAUAGCU | SEQ ID NO: 13147 | UAUCCGG |
| SEQ ID NO: 12893 | UAGCCUA | SEQ ID NO: 12944 | UAGGAUU | SEQ ID NO: 12995 | UAGUAAG | SEQ ID NO: 13046 | UAGUUCC | SEQ ID NO: 13097 | UAUAGGA | SEQ ID NO: 13148 | UAUCCGU |
| SEQ ID NO: 12894 | UAGCCUC | SEQ ID NO: 12945 | UAGGCAA | SEQ ID NO: 12996 | UAGUAAU | SEQ ID NO: 13047 | UAGUUCG | SEQ ID NO: 13098 | UAUAGGC | SEQ ID NO: 13149 | UAUCCUA |
| SEQ ID NO: 12895 | UAGCCUG | SEQ ID NO: 12946 | UAGGCAC | SEQ ID NO: 12997 | UAGUACA | SEQ ID NO: 13048 | UAGUUCU | SEQ ID NO: 13099 | UAUAGGG | SEQ ID NO: 13150 | UAUCCUC |
| SEQ ID NO: 12896 | UAGCCUU | SEQ ID NO: 12947 | UAGGCAG | SEQ ID NO: 12998 | UAGUACC | SEQ ID NO: 13049 | UAGUUGA | SEQ ID NO: 13100 | UAUAGGU | SEQ ID NO: 13151 | UAUCCUG |
| SEQ ID NO: 12897 | UAGCGAA | SEQ ID NO: 12948 | UAGGCAU | SEQ ID NO: 12999 | UAGUACG | SEQ ID NO: 13050 | UAGUUGC | SEQ ID NO: 13101 | UAUAGUA | SEQ ID NO: 13152 | UAUCCUU |
| SEQ ID NO: 12898 | UAGCGAC | SEQ ID NO: 12949 | UAGGCCA | SEQ ID NO: 13000 | UAGUACU | SEQ ID NO: 13051 | UAGUUGG | SEQ ID NO: 13102 | UAUAGUC | SEQ ID NO: 13153 | UAUCGAA |
| SEQ ID NO: 12899 | UAGCGAG | SEQ ID NO: 12950 | UAGGCCC | SEQ ID NO: 13001 | UAGUAGA | SEQ ID NO: 13052 | UAGUUGU | SEQ ID NO: 13103 | UAUAGUG | SEQ ID NO: 13154 | UAUCGAC |
| SEQ ID NO: 12900 | UAGCGAU | SEQ ID NO: 12951 | UAGGCCG | SEQ ID NO: 13002 | UAGUAGC | SEQ ID NO: 13053 | UAGUUUA | SEQ ID NO: 13104 | UAUAGUU | SEQ ID NO: 13155 | UAUCGAG |
| SEQ ID NO: 12901 | UAGCGCA | SEQ ID NO: 12952 | UAGGCCU | SEQ ID NO: 13003 | UAGUAGG | SEQ ID NO: 13054 | UAGUUUC | SEQ ID NO: 13105 | UAUAUAA | SEQ ID NO: 13156 | UAUCGAU |
| SEQ ID NO: 12902 | UAGCGCC | SEQ ID NO: 12953 | UAGGCGA | SEQ ID NO: 13004 | UAGUAGU | SEQ ID NO: 13055 | UAGUUUG | SEQ ID NO: 13106 | UAUAUAC | SEQ ID NO: 13157 | UAUCGCA |
| SEQ ID NO: 12903 | UAGCGCG | SEQ ID NO: 12954 | UAGGCGC | SEQ ID NO: 13005 | UAGUAUA | SEQ ID NO: 13056 | UAGUUUU | SEQ ID NO: 13107 | UAUAUAG | SEQ ID NO: 13158 | UAUCGCC |

Table 44

| | |
|---|---|
| SEQ ID NO: 13159 | UAUCGCG |
| SEQ ID NO: 13160 | UAUCGCU |
| SEQ ID NO: 13161 | UAUCGGA |
| SEQ ID NO: 13162 | UAUCGGC |
| SEQ ID NO: 13163 | UAUCGGG |
| SEQ ID NO: 13164 | UAUCGGU |
| SEQ ID NO: 13165 | UAUCGUA |
| SEQ ID NO: 13166 | UAUCGUC |
| SEQ ID NO: 13167 | UAUCGUG |
| SEQ ID NO: 13168 | UAUCGUU |
| SEQ ID NO: 13169 | UAUCUAA |
| SEQ ID NO: 13170 | UAUCUAC |
| SEQ ID NO: 13171 | UAUCUAG |
| SEQ ID NO: 13172 | UAUCUAU |
| SEQ ID NO: 13173 | UAUCUCA |
| SEQ ID NO: 13174 | UAUCUCC |
| SEQ ID NO: 13175 | UAUCUCG |
| SEQ ID NO: 13176 | UAUCUCU |
| SEQ ID NO: 13177 | UAUCUGA |
| SEQ ID NO: 13178 | UAUCUGC |
| SEQ ID NO: 13179 | UAUCUGG |
| SEQ ID NO: 13180 | UAUCUGU |
| SEQ ID NO: 13181 | UAUCUUA |
| SEQ ID NO: 13182 | UAUCUUC |
| SEQ ID NO: 13183 | UAUCUUG |
| SEQ ID NO: 13184 | UAUCUUU |
| SEQ ID NO: 13185 | UAUGAAA |
| SEQ ID NO: 13186 | UAUGAAC |
| SEQ ID NO: 13187 | UAUGAAG |
| SEQ ID NO: 13188 | UAUGAAU |
| SEQ ID NO: 13189 | UAUGACA |
| SEQ ID NO: 13190 | UAUGACC |
| SEQ ID NO: 13191 | UAUGACG |
| SEQ ID NO: 13192 | UAUGACU |
| SEQ ID NO: 13193 | UAUGAGA |
| SEQ ID NO: 13194 | UAUGAGC |
| SEQ ID NO: 13195 | UAUGAGG |
| SEQ ID NO: 13196 | UAUGAGU |
| SEQ ID NO: 13197 | UAUGAUA |
| SEQ ID NO: 13198 | UAUGAUC |
| SEQ ID NO: 13199 | UAUGAUG |
| SEQ ID NO: 13200 | UAUGAUU |
| SEQ ID NO: 13201 | UAUGCAA |
| SEQ ID NO: 13202 | UAUGCAC |
| SEQ ID NO: 13203 | UAUGCAG |
| SEQ ID NO: 13204 | UAUGCAU |
| SEQ ID NO: 13205 | UAUGCCA |
| SEQ ID NO: 13206 | UAUGCCC |
| SEQ ID NO: 13207 | UAUGCCG |
| SEQ ID NO: 13208 | UAUGCCU |
| SEQ ID NO: 13209 | UAUGCGA |
| SEQ ID NO: 13210 | UAUGCGC |
| SEQ ID NO: 13211 | UAUGCGG |
| SEQ ID NO: 13212 | UAUGCGU |
| SEQ ID NO: 13213 | UAUGCUA |
| SEQ ID NO: 13214 | UAUGCUC |
| SEQ ID NO: 13215 | UAUGCUG |
| SEQ ID NO: 13216 | UAUGCUU |
| SEQ ID NO: 13217 | UAUGGAA |
| SEQ ID NO: 13218 | UAUGGAC |
| SEQ ID NO: 13219 | UAUGGAG |
| SEQ ID NO: 13220 | UAUGGAU |
| SEQ ID NO: 13221 | UAUGGCA |
| SEQ ID NO: 13222 | UAUGGCC |
| SEQ ID NO: 13223 | UAUGGCG |
| SEQ ID NO: 13224 | UAUGGCU |
| SEQ ID NO: 13225 | UAUGGGA |
| SEQ ID NO: 13226 | UAUGGGC |
| SEQ ID NO: 13227 | UAUGGGG |
| SEQ ID NO: 13228 | UAUGGGU |
| SEQ ID NO: 13229 | UAUGGUA |
| SEQ ID NO: 13230 | UAUGGUC |
| SEQ ID NO: 13231 | UAUGGUG |
| SEQ ID NO: 13232 | UAUGGUU |
| SEQ ID NO: 13233 | UAUGUAA |
| SEQ ID NO: 13234 | UAUGUAC |
| SEQ ID NO: 13235 | UAUGUAG |
| SEQ ID NO: 13236 | UAUGUAU |
| SEQ ID NO: 13237 | UAUGUCA |
| SEQ ID NO: 13238 | UAUGUCC |
| SEQ ID NO: 13239 | UAUGUCG |
| SEQ ID NO: 13240 | UAUGUCU |
| SEQ ID NO: 13241 | UAUGUGA |
| SEQ ID NO: 13242 | UAUGUGC |
| SEQ ID NO: 13243 | UAUGUGG |
| SEQ ID NO: 13244 | UAUGUGU |
| SEQ ID NO: 13245 | UAUGUUA |
| SEQ ID NO: 13246 | UAUGUUC |
| SEQ ID NO: 13247 | UAUGUUG |
| SEQ ID NO: 13248 | UAUGUUU |
| SEQ ID NO: 13249 | UAUUAAA |
| SEQ ID NO: 13250 | UAUUAAC |
| SEQ ID NO: 13251 | UAUUAAG |
| SEQ ID NO: 13252 | UAUUAAU |
| SEQ ID NO: 13253 | UAUUACA |
| SEQ ID NO: 13254 | UAUUACC |
| SEQ ID NO: 13255 | UAUUACG |
| SEQ ID NO: 13256 | UAUUACU |
| SEQ ID NO: 13257 | UAUUAGA |
| SEQ ID NO: 13258 | UAUUAGC |
| SEQ ID NO: 13259 | UAUUAGG |
| SEQ ID NO: 13260 | UAUUAGU |
| SEQ ID NO: 13261 | UAUUAUA |
| SEQ ID NO: 13262 | UAUUAUC |
| SEQ ID NO: 13263 | UAUUAUG |
| SEQ ID NO: 13264 | UAUUAUU |
| SEQ ID NO: 13265 | UAUUCAA |
| SEQ ID NO: 13266 | UAUUCAC |
| SEQ ID NO: 13267 | UAUUCAG |
| SEQ ID NO: 13268 | UAUUCAU |
| SEQ ID NO: 13269 | UAUUCCA |
| SEQ ID NO: 13270 | UAUUCCC |
| SEQ ID NO: 13271 | UAUUCCG |
| SEQ ID NO: 13272 | UAUUCCU |
| SEQ ID NO: 13273 | UAUUCGA |
| SEQ ID NO: 13274 | UAUUCGC |
| SEQ ID NO: 13275 | UAUUCGG |
| SEQ ID NO: 13276 | UAUUCGU |
| SEQ ID NO: 13277 | UAUUCUA |
| SEQ ID NO: 13278 | UAUUCUC |
| SEQ ID NO: 13279 | UAUUCUG |
| SEQ ID NO: 13280 | UAUUCUU |
| SEQ ID NO: 13281 | UAUUGAA |
| SEQ ID NO: 13282 | UAUUGAC |
| SEQ ID NO: 13283 | UAUUGAG |
| SEQ ID NO: 13284 | UAUUGAU |
| SEQ ID NO: 13285 | UAUUGCA |
| SEQ ID NO: 13286 | UAUUGCC |
| SEQ ID NO: 13287 | UAUUGCG |
| SEQ ID NO: 13288 | UAUUGCU |
| SEQ ID NO: 13289 | UAUUGGA |
| SEQ ID NO: 13290 | UAUUGGC |
| SEQ ID NO: 13291 | UAUUGGG |
| SEQ ID NO: 13292 | UAUUGGU |
| SEQ ID NO: 13293 | UAUUGUA |
| SEQ ID NO: 13294 | UAUUGUC |
| SEQ ID NO: 13295 | UAUUGUG |
| SEQ ID NO: 13296 | UAUUGUU |
| SEQ ID NO: 13297 | UAUUUAA |
| SEQ ID NO: 13298 | UAUUUAC |
| SEQ ID NO: 13299 | UAUUUAG |
| SEQ ID NO: 13300 | UAUUUAU |
| SEQ ID NO: 13301 | UAUUUCA |
| SEQ ID NO: 13302 | UAUUUCC |
| SEQ ID NO: 13303 | UAUUUCG |
| SEQ ID NO: 13304 | UAUUUCU |
| SEQ ID NO: 13305 | UAUUUGA |
| SEQ ID NO: 13306 | UAUUUGC |
| SEQ ID NO: 13307 | UAUUUGG |
| SEQ ID NO: 13308 | UAUUUGU |
| SEQ ID NO: 13309 | UAUUUUA |
| SEQ ID NO: 13310 | UAUUUUC |
| SEQ ID NO: 13311 | UAUUUUG |
| SEQ ID NO: 13312 | UAUUUUU |
| SEQ ID NO: 13313 | UCAAAAA |
| SEQ ID NO: 13314 | UCAAAAC |
| SEQ ID NO: 13315 | UCAAAAG |
| SEQ ID NO: 13316 | UCAAAAU |
| SEQ ID NO: 13317 | UCAAACA |
| SEQ ID NO: 13318 | UCAAACC |
| SEQ ID NO: 13319 | UCAAACG |
| SEQ ID NO: 13320 | UCAAACU |
| SEQ ID NO: 13321 | UCAAAGA |
| SEQ ID NO: 13322 | UCAAAGC |
| SEQ ID NO: 13323 | UCAAAGG |
| SEQ ID NO: 13324 | UCAAAGU |
| SEQ ID NO: 13325 | UCAAAUA |
| SEQ ID NO: 13326 | UCAAAUC |
| SEQ ID NO: 13327 | UCAAAUG |
| SEQ ID NO: 13328 | UCAAAUU |
| SEQ ID NO: 13329 | UCAACAA |
| SEQ ID NO: 13330 | UCAACAC |
| SEQ ID NO: 13331 | UCAACAG |
| SEQ ID NO: 13332 | UCAACAU |
| SEQ ID NO: 13333 | UCAACCA |
| SEQ ID NO: 13334 | UCAACCC |
| SEQ ID NO: 13335 | UCAACCG |
| SEQ ID NO: 13336 | UCAACCU |
| SEQ ID NO: 13337 | UCAACGA |
| SEQ ID NO: 13338 | UCAACGC |
| SEQ ID NO: 13339 | UCAACGG |
| SEQ ID NO: 13340 | UCAACGU |
| SEQ ID NO: 13341 | UCAACUA |
| SEQ ID NO: 13342 | UCAACUC |
| SEQ ID NO: 13343 | UCAACUG |
| SEQ ID NO: 13344 | UCAACUU |
| SEQ ID NO: 13345 | UCAAGAA |
| SEQ ID NO: 13346 | UCAAGAC |
| SEQ ID NO: 13347 | UCAAGAG |
| SEQ ID NO: 13348 | UCAAGAU |
| SEQ ID NO: 13349 | UCAAGCA |
| SEQ ID NO: 13350 | UCAAGCC |
| SEQ ID NO: 13351 | UCAAGCG |
| SEQ ID NO: 13352 | UCAAGCU |
| SEQ ID NO: 13353 | UCAAGGA |
| SEQ ID NO: 13354 | UCAAGGC |
| SEQ ID NO: 13355 | UCAAGGG |
| SEQ ID NO: 13356 | UCAAGGU |
| SEQ ID NO: 13357 | UCAAGUA |
| SEQ ID NO: 13358 | UCAAGUC |
| SEQ ID NO: 13359 | UCAAGUG |
| SEQ ID NO: 13360 | UCAAGUU |
| SEQ ID NO: 13361 | UCAAUAA |
| SEQ ID NO: 13362 | UCAAUAC |
| SEQ ID NO: 13363 | UCAAUAG |
| SEQ ID NO: 13364 | UCAAUAU |
| SEQ ID NO: 13365 | UCAAUCA |
| SEQ ID NO: 13366 | UCAAUCC |
| SEQ ID NO: 13367 | UCAAUCG |
| SEQ ID NO: 13368 | UCAAUCU |
| SEQ ID NO: 13369 | UCAAUGA |
| SEQ ID NO: 13370 | UCAAUGC |
| SEQ ID NO: 13371 | UCAAUGG |
| SEQ ID NO: 13372 | UCAAUGU |
| SEQ ID NO: 13373 | UCAAUUA |
| SEQ ID NO: 13374 | UCAAUUC |
| SEQ ID NO: 13375 | UCAAUUG |
| SEQ ID NO: 13376 | UCAAUUU |
| SEQ ID NO: 13377 | UCACAAA |
| SEQ ID NO: 13378 | UCACAAC |
| SEQ ID NO: 13379 | UCACAAG |
| SEQ ID NO: 13380 | UCACAAU |
| SEQ ID NO: 13381 | UCACACA |
| SEQ ID NO: 13382 | UCACACC |
| SEQ ID NO: 13383 | UCACACG |
| SEQ ID NO: 13384 | UCACACU |
| SEQ ID NO: 13385 | UCACAGA |
| SEQ ID NO: 13386 | UCACAGC |
| SEQ ID NO: 13387 | UCACAGG |
| SEQ ID NO: 13388 | UCACAGU |
| SEQ ID NO: 13389 | UCACAUA |
| SEQ ID NO: 13390 | UCACAUC |
| SEQ ID NO: 13391 | UCACAUG |
| SEQ ID NO: 13392 | UCACAUU |
| SEQ ID NO: 13393 | UCACCAA |
| SEQ ID NO: 13394 | UCACCAC |
| SEQ ID NO: 13395 | UCACCAG |
| SEQ ID NO: 13396 | UCACCAU |
| SEQ ID NO: 13397 | UCACCCA |
| SEQ ID NO: 13398 | UCACCCC |
| SEQ ID NO: 13399 | UCACCCG |
| SEQ ID NO: 13400 | UCACCCU |
| SEQ ID NO: 13401 | UCACCGA |
| SEQ ID NO: 13402 | UCACCGC |
| SEQ ID NO: 13403 | UCACCGG |
| SEQ ID NO: 13404 | UCACCGU |
| SEQ ID NO: 13405 | UCACCUA |
| SEQ ID NO: 13406 | UCACCUC |
| SEQ ID NO: 13407 | UCACCUG |
| SEQ ID NO: 13408 | UCACCUU |
| SEQ ID NO: 13409 | UCACGAA |
| SEQ ID NO: 13410 | UCACGAC |
| SEQ ID NO: 13411 | UCACGAG |
| SEQ ID NO: 13412 | UCACGAU |
| SEQ ID NO: 13413 | UCACGCA |
| SEQ ID NO: 13414 | UCACGCC |
| SEQ ID NO: 13415 | UCACGCG |
| SEQ ID NO: 13416 | UCACGCU |
| SEQ ID NO: 13417 | UCACGGA |
| SEQ ID NO: 13418 | UCACGGC |
| SEQ ID NO: 13419 | UCACGGG |
| SEQ ID NO: 13420 | UCACGGU |
| SEQ ID NO: 13421 | UCACGUA |
| SEQ ID NO: 13422 | UCACGUC |
| SEQ ID NO: 13423 | UCACGUG |
| SEQ ID NO: 13424 | UCACGUU |
| SEQ ID NO: 13425 | UCACUAA |
| SEQ ID NO: 13426 | UCACUAC |
| SEQ ID NO: 13427 | UCACUAG |
| SEQ ID NO: 13428 | UCACUAU |
| SEQ ID NO: 13429 | UCACUCA |
| SEQ ID NO: 13430 | UCACUCC |
| SEQ ID NO: 13431 | UCACUCG |
| SEQ ID NO: 13432 | UCACUCU |
| SEQ ID NO: 13433 | UCACUGA |
| SEQ ID NO: 13434 | UCACUGC |
| SEQ ID NO: 13435 | UCACUGG |
| SEQ ID NO: 13436 | UCACUGU |
| SEQ ID NO: 13437 | UCACUUA |
| SEQ ID NO: 13438 | UCACUUC |
| SEQ ID NO: 13439 | UCACUUG |
| SEQ ID NO: 13440 | UCACUUU |
| SEQ ID NO: 13441 | UCAGAAA |
| SEQ ID NO: 13442 | UCAGAAC |
| SEQ ID NO: 13443 | UCAGAAG |
| SEQ ID NO: 13444 | UCAGAAU |
| SEQ ID NO: 13445 | UCAGACA |
| SEQ ID NO: 13446 | UCAGACC |
| SEQ ID NO: 13447 | UCAGACG |
| SEQ ID NO: 13448 | UCAGACU |
| SEQ ID NO: 13449 | UCAGAGA |
| SEQ ID NO: 13450 | UCAGAGC |
| SEQ ID NO: 13451 | UCAGAGG |
| SEQ ID NO: 13452 | UCAGAGU |
| SEQ ID NO: 13453 | UCAGAUA |
| SEQ ID NO: 13454 | UCAGAUC |
| SEQ ID NO: 13455 | UCAGAUG |
| SEQ ID NO: 13456 | UCAGAUU |
| SEQ ID NO: 13457 | UCAGCAA |
| SEQ ID NO: 13458 | UCAGCAC |
| SEQ ID NO: 13459 | UCAGCAG |
| SEQ ID NO: 13460 | UCAGCAU |
| SEQ ID NO: 13461 | UCAGCCA |
| SEQ ID NO: 13462 | UCAGCCC |
| SEQ ID NO: 13463 | UCAGCCG |
| SEQ ID NO: 13464 | UCAGCCU |

Table 45

| | | | | | | | | | | | |
|---|---|---|---|---|---|---|---|---|---|---|---|
| SEQ ID NO: 13465 | UCAGCGA | SEQ ID NO: 13516 | UCAUAGU | SEQ ID NO: 13567 | UCAUUUG | SEQ ID NO: 13618 | UCCAUAC | SEQ ID NO: 13669 | UCCCGCA | SEQ ID NO: 13720 | UCCGCCU |
| SEQ ID NO: 13466 | UCAGCGC | SEQ ID NO: 13517 | UCAUAUA | SEQ ID NO: 13568 | UCAUUUU | SEQ ID NO: 13619 | UCCAUAG | SEQ ID NO: 13670 | UCCCGCC | SEQ ID NO: 13721 | UCCGCGA |
| SEQ ID NO: 13467 | UCAGCGG | SEQ ID NO: 13518 | UCAUAUC | SEQ ID NO: 13569 | UCCAAAA | SEQ ID NO: 13620 | UCCAUAU | SEQ ID NO: 13671 | UCCCGCG | SEQ ID NO: 13722 | UCCGCGC |
| SEQ ID NO: 13468 | UCAGCGU | SEQ ID NO: 13519 | UCAUAUG | SEQ ID NO: 13570 | UCCAAAC | SEQ ID NO: 13621 | UCCAUCA | SEQ ID NO: 13672 | UCCCGCU | SEQ ID NO: 13723 | UCCGCGG |
| SEQ ID NO: 13469 | UCAGCUA | SEQ ID NO: 13520 | UCAUAUU | SEQ ID NO: 13571 | UCCAAAG | SEQ ID NO: 13622 | UCCAUCC | SEQ ID NO: 13673 | UCCCGGA | SEQ ID NO: 13724 | UCCGCGU |
| SEQ ID NO: 13470 | UCAGCUC | SEQ ID NO: 13521 | UCAUCAA | SEQ ID NO: 13572 | UCCAAAU | SEQ ID NO: 13623 | UCCAUCG | SEQ ID NO: 13674 | UCCCGGC | SEQ ID NO: 13725 | UCCGCUA |
| SEQ ID NO: 13471 | UCAGCUG | SEQ ID NO: 13522 | UCAUCAC | SEQ ID NO: 13573 | UCCAACA | SEQ ID NO: 13624 | UCCAUCU | SEQ ID NO: 13675 | UCCCGGG | SEQ ID NO: 13726 | UCCGCUC |
| SEQ ID NO: 13472 | UCAGCUU | SEQ ID NO: 13523 | UCAUCAG | SEQ ID NO: 13574 | UCCAACC | SEQ ID NO: 13625 | UCCAUGA | SEQ ID NO: 13676 | UCCCGGU | SEQ ID NO: 13727 | UCCGCUG |
| SEQ ID NO: 13473 | UCAGGAA | SEQ ID NO: 13524 | UCAUCAU | SEQ ID NO: 13575 | UCCAACG | SEQ ID NO: 13626 | UCCAUGC | SEQ ID NO: 13677 | UCCCGUA | SEQ ID NO: 13728 | UCCGCUU |
| SEQ ID NO: 13474 | UCAGGAC | SEQ ID NO: 13525 | UCAUCCA | SEQ ID NO: 13576 | UCCAACU | SEQ ID NO: 13627 | UCCAUGG | SEQ ID NO: 13678 | UCCCGUC | SEQ ID NO: 13729 | UCCGGAA |
| SEQ ID NO: 13475 | UCAGGAG | SEQ ID NO: 13526 | UCAUCCC | SEQ ID NO: 13577 | UCCAAGA | SEQ ID NO: 13628 | UCCAUGU | SEQ ID NO: 13679 | UCCCGUG | SEQ ID NO: 13730 | UCCGGAC |
| SEQ ID NO: 13476 | UCAGGAU | SEQ ID NO: 13527 | UCAUCCG | SEQ ID NO: 13578 | UCCAAGC | SEQ ID NO: 13629 | UCCAUUA | SEQ ID NO: 13680 | UCCCGUU | SEQ ID NO: 13731 | UCCGGAG |
| SEQ ID NO: 13477 | UCAGGCA | SEQ ID NO: 13528 | UCAUCCU | SEQ ID NO: 13579 | UCCAAGG | SEQ ID NO: 13630 | UCCAUUC | SEQ ID NO: 13681 | UCCCUAA | SEQ ID NO: 13732 | UCCGGAU |
| SEQ ID NO: 13478 | UCAGGCC | SEQ ID NO: 13529 | UCAUCGA | SEQ ID NO: 13580 | UCCAAGU | SEQ ID NO: 13631 | UCCAUUG | SEQ ID NO: 13682 | UCCCUAC | SEQ ID NO: 13733 | UCCGGCA |
| SEQ ID NO: 13479 | UCAGGCG | SEQ ID NO: 13530 | UCAUCGC | SEQ ID NO: 13581 | UCCAAUA | SEQ ID NO: 13632 | UCCAUUU | SEQ ID NO: 13683 | UCCCUAG | SEQ ID NO: 13734 | UCCGGCC |
| SEQ ID NO: 13480 | UCAGGCU | SEQ ID NO: 13531 | UCAUCGG | SEQ ID NO: 13582 | UCCAAUC | SEQ ID NO: 13633 | UCCCAAA | SEQ ID NO: 13684 | UCCCUAU | SEQ ID NO: 13735 | UCCGGCG |
| SEQ ID NO: 13481 | UCAGGGA | SEQ ID NO: 13532 | UCAUCGU | SEQ ID NO: 13583 | UCCAAUG | SEQ ID NO: 13634 | UCCCAAC | SEQ ID NO: 13685 | UCCCUCA | SEQ ID NO: 13736 | UCCGGCU |
| SEQ ID NO: 13482 | UCAGGGC | SEQ ID NO: 13533 | UCAUCUA | SEQ ID NO: 13584 | UCCAAUU | SEQ ID NO: 13635 | UCCCAAG | SEQ ID NO: 13686 | UCCCUCC | SEQ ID NO: 13737 | UCCGGGA |
| SEQ ID NO: 13483 | UCAGGGG | SEQ ID NO: 13534 | UCAUCUC | SEQ ID NO: 13585 | UCCACAA | SEQ ID NO: 13636 | UCCCAAU | SEQ ID NO: 13687 | UCCCUCG | SEQ ID NO: 13738 | UCCGGGC |
| SEQ ID NO: 13484 | UCAGGGU | SEQ ID NO: 13535 | UCAUCUG | SEQ ID NO: 13586 | UCCACAC | SEQ ID NO: 13637 | UCCCACA | SEQ ID NO: 13688 | UCCCUCU | SEQ ID NO: 13739 | UCCGGGG |
| SEQ ID NO: 13485 | UCAGGUA | SEQ ID NO: 13536 | UCAUCUU | SEQ ID NO: 13587 | UCCACAG | SEQ ID NO: 13638 | UCCCACC | SEQ ID NO: 13689 | UCCCUGA | SEQ ID NO: 13740 | UCCGGGU |
| SEQ ID NO: 13486 | UCAGGUC | SEQ ID NO: 13537 | UCAUGAA | SEQ ID NO: 13588 | UCCACAU | SEQ ID NO: 13639 | UCCCACG | SEQ ID NO: 13690 | UCCCUGC | SEQ ID NO: 13741 | UCCGGUA |
| SEQ ID NO: 13487 | UCAGGUG | SEQ ID NO: 13538 | UCAUGAC | SEQ ID NO: 13589 | UCCACCA | SEQ ID NO: 13640 | UCCCACU | SEQ ID NO: 13691 | UCCCUGG | SEQ ID NO: 13742 | UCCGGUC |
| SEQ ID NO: 13488 | UCAGGUU | SEQ ID NO: 13539 | UCAUGAG | SEQ ID NO: 13590 | UCCACCC | SEQ ID NO: 13641 | UCCCAGA | SEQ ID NO: 13692 | UCCCUGU | SEQ ID NO: 13743 | UCCGGUG |
| SEQ ID NO: 13489 | UCAGUAA | SEQ ID NO: 13540 | UCAUGAU | SEQ ID NO: 13591 | UCCACCG | SEQ ID NO: 13642 | UCCCAGC | SEQ ID NO: 13693 | UCCCUUA | SEQ ID NO: 13744 | UCCGGUU |
| SEQ ID NO: 13490 | UCAGUAC | SEQ ID NO: 13541 | UCAUGCA | SEQ ID NO: 13592 | UCCACCU | SEQ ID NO: 13643 | UCCCAGG | SEQ ID NO: 13694 | UCCCUUC | SEQ ID NO: 13745 | UCCGUAA |
| SEQ ID NO: 13491 | UCAGUAG | SEQ ID NO: 13542 | UCAUGCC | SEQ ID NO: 13593 | UCCACGA | SEQ ID NO: 13644 | UCCCAGU | SEQ ID NO: 13695 | UCCCUUG | SEQ ID NO: 13746 | UCCGUAC |
| SEQ ID NO: 13492 | UCAGUAU | SEQ ID NO: 13543 | UCAUGCG | SEQ ID NO: 13594 | UCCACGC | SEQ ID NO: 13645 | UCCCAUA | SEQ ID NO: 13696 | UCCCUUU | SEQ ID NO: 13747 | UCCGUAG |
| SEQ ID NO: 13493 | UCAGUCA | SEQ ID NO: 13544 | UCAUGCU | SEQ ID NO: 13595 | UCCACGG | SEQ ID NO: 13646 | UCCCAUC | SEQ ID NO: 13697 | UCCGAAA | SEQ ID NO: 13748 | UCCGUAU |
| SEQ ID NO: 13494 | UCAGUCC | SEQ ID NO: 13545 | UCAUGGA | SEQ ID NO: 13596 | UCCACGU | SEQ ID NO: 13647 | UCCCAUG | SEQ ID NO: 13698 | UCCGAAC | SEQ ID NO: 13749 | UCCGUCA |
| SEQ ID NO: 13495 | UCAGUCG | SEQ ID NO: 13546 | UCAUGGC | SEQ ID NO: 13597 | UCCACUA | SEQ ID NO: 13648 | UCCCAUU | SEQ ID NO: 13699 | UCCGAAG | SEQ ID NO: 13750 | UCCGUCC |
| SEQ ID NO: 13496 | UCAGUCU | SEQ ID NO: 13547 | UCAUGGG | SEQ ID NO: 13598 | UCCACUC | SEQ ID NO: 13649 | UCCCCAA | SEQ ID NO: 13700 | UCCGAAU | SEQ ID NO: 13751 | UCCGUCG |
| SEQ ID NO: 13497 | UCAGUGA | SEQ ID NO: 13548 | UCAUGGU | SEQ ID NO: 13599 | UCCACUG | SEQ ID NO: 13650 | UCCCCAC | SEQ ID NO: 13701 | UCCGACA | SEQ ID NO: 13752 | UCCGUCU |
| SEQ ID NO: 13498 | UCAGUGC | SEQ ID NO: 13549 | UCAUGUA | SEQ ID NO: 13600 | UCCACUU | SEQ ID NO: 13651 | UCCCCAG | SEQ ID NO: 13702 | UCCGACC | SEQ ID NO: 13753 | UCCGUGA |
| SEQ ID NO: 13499 | UCAGUGG | SEQ ID NO: 13550 | UCAUGUC | SEQ ID NO: 13601 | UCCAGAA | SEQ ID NO: 13652 | UCCCCAU | SEQ ID NO: 13703 | UCCGACG | SEQ ID NO: 13754 | UCCGUGC |
| SEQ ID NO: 13500 | UCAGUGU | SEQ ID NO: 13551 | UCAUGUG | SEQ ID NO: 13602 | UCCAGAC | SEQ ID NO: 13653 | UCCCCCA | SEQ ID NO: 13704 | UCCGACU | SEQ ID NO: 13755 | UCCGUGG |
| SEQ ID NO: 13501 | UCAGUUA | SEQ ID NO: 13552 | UCAUGUU | SEQ ID NO: 13603 | UCCAGAG | SEQ ID NO: 13654 | UCCCCCC | SEQ ID NO: 13705 | UCCGAGA | SEQ ID NO: 13756 | UCCGUGU |
| SEQ ID NO: 13502 | UCAGUUC | SEQ ID NO: 13553 | UCAUUAA | SEQ ID NO: 13604 | UCCAGAU | SEQ ID NO: 13655 | UCCCCCG | SEQ ID NO: 13706 | UCCGAGC | SEQ ID NO: 13757 | UCCGUUA |
| SEQ ID NO: 13503 | UCAGUUG | SEQ ID NO: 13554 | UCAUUAC | SEQ ID NO: 13605 | UCCAGCA | SEQ ID NO: 13656 | UCCCCCU | SEQ ID NO: 13707 | UCCGAGG | SEQ ID NO: 13758 | UCCGUUC |
| SEQ ID NO: 13504 | UCAGUUU | SEQ ID NO: 13555 | UCAUUAG | SEQ ID NO: 13606 | UCCAGCC | SEQ ID NO: 13657 | UCCCCGA | SEQ ID NO: 13708 | UCCGAGU | SEQ ID NO: 13759 | UCCGUUG |
| SEQ ID NO: 13505 | UCAUAAA | SEQ ID NO: 13556 | UCAUUAU | SEQ ID NO: 13607 | UCCAGCG | SEQ ID NO: 13658 | UCCCCGC | SEQ ID NO: 13709 | UCCGAUA | SEQ ID NO: 13760 | UCCGUUU |
| SEQ ID NO: 13506 | UCAUAAC | SEQ ID NO: 13557 | UCAUUCA | SEQ ID NO: 13608 | UCCAGCU | SEQ ID NO: 13659 | UCCCCGG | SEQ ID NO: 13710 | UCCGAUC | SEQ ID NO: 13761 | UCCUAAA |
| SEQ ID NO: 13507 | UCAUAAG | SEQ ID NO: 13558 | UCAUUCC | SEQ ID NO: 13609 | UCCAGGA | SEQ ID NO: 13660 | UCCCCGU | SEQ ID NO: 13711 | UCCGAUG | SEQ ID NO: 13762 | UCCUAAC |
| SEQ ID NO: 13508 | UCAUAAU | SEQ ID NO: 13559 | UCAUUCG | SEQ ID NO: 13610 | UCCAGGC | SEQ ID NO: 13661 | UCCCCUA | SEQ ID NO: 13712 | UCCGAUU | SEQ ID NO: 13763 | UCCUAAG |
| SEQ ID NO: 13509 | UCAUACA | SEQ ID NO: 13560 | UCAUUCU | SEQ ID NO: 13611 | UCCAGGG | SEQ ID NO: 13662 | UCCCCUC | SEQ ID NO: 13713 | UCCGCAA | SEQ ID NO: 13764 | UCCUAAU |
| SEQ ID NO: 13510 | UCAUACC | SEQ ID NO: 13561 | UCAUUGA | SEQ ID NO: 13612 | UCCAGGU | SEQ ID NO: 13663 | UCCCCUG | SEQ ID NO: 13714 | UCCGCAC | SEQ ID NO: 13765 | UCCUACA |
| SEQ ID NO: 13511 | UCAUACG | SEQ ID NO: 13562 | UCAUUGC | SEQ ID NO: 13613 | UCCAGUA | SEQ ID NO: 13664 | UCCCCUU | SEQ ID NO: 13715 | UCCGCAG | SEQ ID NO: 13766 | UCCUACC |
| SEQ ID NO: 13512 | UCAUACU | SEQ ID NO: 13563 | UCAUUGG | SEQ ID NO: 13614 | UCCAGUC | SEQ ID NO: 13665 | UCCCGAA | SEQ ID NO: 13716 | UCCGCAU | SEQ ID NO: 13767 | UCCUACG |
| SEQ ID NO: 13513 | UCAUAGA | SEQ ID NO: 13564 | UCAUUGU | SEQ ID NO: 13615 | UCCAGUG | SEQ ID NO: 13666 | UCCCGAC | SEQ ID NO: 13717 | UCCGCCA | SEQ ID NO: 13768 | UCCUACU |
| SEQ ID NO: 13514 | UCAUAGC | SEQ ID NO: 13565 | UCAUUUA | SEQ ID NO: 13616 | UCCAGUU | SEQ ID NO: 13667 | UCCCGAG | SEQ ID NO: 13718 | UCCGCCC | SEQ ID NO: 13769 | UCCUAGA |
| SEQ ID NO: 13515 | UCAUAGG | SEQ ID NO: 13566 | UCAUUUC | SEQ ID NO: 13617 | UCCAUAA | SEQ ID NO: 13668 | UCCCGAU | SEQ ID NO: 13719 | UCCGCCG | SEQ ID NO: 13770 | UCCUAGC |

Table 46

| | |
|---|---|
| SEQ ID NO: 13771 | UCCUAGG |
| SEQ ID NO: 13772 | UCCUAGU |
| SEQ ID NO: 13773 | UCCUAUA |
| SEQ ID NO: 13774 | UCCUAUC |
| SEQ ID NO: 13775 | UCCUAUG |
| SEQ ID NO: 13776 | UCCUAUU |
| SEQ ID NO: 13777 | UCCUCAA |
| SEQ ID NO: 13778 | UCCUCAC |
| SEQ ID NO: 13779 | UCCUCAG |
| SEQ ID NO: 13780 | UCCUCAU |
| SEQ ID NO: 13781 | UCCUCCA |
| SEQ ID NO: 13782 | UCCUCCC |
| SEQ ID NO: 13783 | UCCUCCG |
| SEQ ID NO: 13784 | UCCUCCU |
| SEQ ID NO: 13785 | UCCUCGA |
| SEQ ID NO: 13786 | UCCUCGC |
| SEQ ID NO: 13787 | UCCUCGG |
| SEQ ID NO: 13788 | UCCUCGU |
| SEQ ID NO: 13789 | UCCUCUA |
| SEQ ID NO: 13790 | UCCUCUC |
| SEQ ID NO: 13791 | UCCUCUG |
| SEQ ID NO: 13792 | UCCUCUU |
| SEQ ID NO: 13793 | UCCUGAA |
| SEQ ID NO: 13794 | UCCUGAC |
| SEQ ID NO: 13795 | UCCUGAG |
| SEQ ID NO: 13796 | UCCUGAU |
| SEQ ID NO: 13797 | UCCUGCA |
| SEQ ID NO: 13798 | UCCUGCC |
| SEQ ID NO: 13799 | UCCUGCG |
| SEQ ID NO: 13800 | UCCUGCU |
| SEQ ID NO: 13801 | UCCUGGA |
| SEQ ID NO: 13802 | UCCUGGC |
| SEQ ID NO: 13803 | UCCUGGG |
| SEQ ID NO: 13804 | UCCUGGU |
| SEQ ID NO: 13805 | UCCUGUA |
| SEQ ID NO: 13806 | UCCUGUC |
| SEQ ID NO: 13807 | UCCUGUG |
| SEQ ID NO: 13808 | UCCUGUU |
| SEQ ID NO: 13809 | UCCUUAA |
| SEQ ID NO: 13810 | UCCUUAC |
| SEQ ID NO: 13811 | UCCUUAG |
| SEQ ID NO: 13812 | UCCUUAU |
| SEQ ID NO: 13813 | UCCUUCA |
| SEQ ID NO: 13814 | UCCUUCC |
| SEQ ID NO: 13815 | UCCUUCG |
| SEQ ID NO: 13816 | UCCUUCU |
| SEQ ID NO: 13817 | UCCUUGA |
| SEQ ID NO: 13818 | UCCUUGC |
| SEQ ID NO: 13819 | UCCUUGG |
| SEQ ID NO: 13820 | UCCUUGU |
| SEQ ID NO: 13821 | UCCUUUA |
| SEQ ID NO: 13822 | UCCUUUC |
| SEQ ID NO: 13823 | UCCUUUG |
| SEQ ID NO: 13824 | UCCUUUU |
| SEQ ID NO: 13825 | UCGAAAA |
| SEQ ID NO: 13826 | UCGAAAC |
| SEQ ID NO: 13827 | UCGAAAG |
| SEQ ID NO: 13828 | UCGAAAU |
| SEQ ID NO: 13829 | UCGAACA |
| SEQ ID NO: 13830 | UCGAACC |
| SEQ ID NO: 13831 | UCGAACG |
| SEQ ID NO: 13832 | UCGAACU |
| SEQ ID NO: 13833 | UCGAAGA |
| SEQ ID NO: 13834 | UCGAAGC |
| SEQ ID NO: 13835 | UCGAAGG |
| SEQ ID NO: 13836 | UCGAAGU |
| SEQ ID NO: 13837 | UCGAAUA |
| SEQ ID NO: 13838 | UCGAAUC |
| SEQ ID NO: 13839 | UCGAAUG |
| SEQ ID NO: 13840 | UCGAAUU |
| SEQ ID NO: 13841 | UCGACAA |
| SEQ ID NO: 13842 | UCGACAC |
| SEQ ID NO: 13843 | UCGACAG |
| SEQ ID NO: 13844 | UCGACAU |
| SEQ ID NO: 13845 | UCGACCA |
| SEQ ID NO: 13846 | UCGACCC |
| SEQ ID NO: 13847 | UCGACCG |
| SEQ ID NO: 13848 | UCGACCU |
| SEQ ID NO: 13849 | UCGACGA |
| SEQ ID NO: 13850 | UCGACGC |
| SEQ ID NO: 13851 | UCGACGG |
| SEQ ID NO: 13852 | UCGACGU |
| SEQ ID NO: 13853 | UCGACUA |
| SEQ ID NO: 13854 | UCGACUC |
| SEQ ID NO: 13855 | UCGACUG |
| SEQ ID NO: 13856 | UCGACUU |
| SEQ ID NO: 13857 | UCGAGAA |
| SEQ ID NO: 13858 | UCGAGAC |
| SEQ ID NO: 13859 | UCGAGAG |
| SEQ ID NO: 13860 | UCGAGAU |
| SEQ ID NO: 13861 | UCGAGCA |
| SEQ ID NO: 13862 | UCGAGCC |
| SEQ ID NO: 13863 | UCGAGCG |
| SEQ ID NO: 13864 | UCGAGCU |
| SEQ ID NO: 13865 | UCGAGGA |
| SEQ ID NO: 13866 | UCGAGGC |
| SEQ ID NO: 13867 | UCGAGGG |
| SEQ ID NO: 13868 | UCGAGGU |
| SEQ ID NO: 13869 | UCGAGUA |
| SEQ ID NO: 13870 | UCGAGUC |
| SEQ ID NO: 13871 | UCGAGUG |
| SEQ ID NO: 13872 | UCGAGUU |
| SEQ ID NO: 13873 | UCGAUAA |
| SEQ ID NO: 13874 | UCGAUAC |
| SEQ ID NO: 13875 | UCGAUAG |
| SEQ ID NO: 13876 | UCGAUAU |
| SEQ ID NO: 13877 | UCGAUCA |
| SEQ ID NO: 13878 | UCGAUCC |
| SEQ ID NO: 13879 | UCGAUCG |
| SEQ ID NO: 13880 | UCGAUCU |
| SEQ ID NO: 13881 | UCGAUGA |
| SEQ ID NO: 13882 | UCGAUGC |
| SEQ ID NO: 13883 | UCGAUGG |
| SEQ ID NO: 13884 | UCGAUGU |
| SEQ ID NO: 13885 | UCGAUUA |
| SEQ ID NO: 13886 | UCGAUUC |
| SEQ ID NO: 13887 | UCGAUUG |
| SEQ ID NO: 13888 | UCGAUUU |
| SEQ ID NO: 13889 | UCGCAAA |
| SEQ ID NO: 13890 | UCGCAAC |
| SEQ ID NO: 13891 | UCGCAAG |
| SEQ ID NO: 13892 | UCGCAAU |
| SEQ ID NO: 13893 | UCGCACA |
| SEQ ID NO: 13894 | UCGCACC |
| SEQ ID NO: 13895 | UCGCACG |
| SEQ ID NO: 13896 | UCGCACU |
| SEQ ID NO: 13897 | UCGCAGA |
| SEQ ID NO: 13898 | UCGCAGC |
| SEQ ID NO: 13899 | UCGCAGG |
| SEQ ID NO: 13900 | UCGCAGU |
| SEQ ID NO: 13901 | UCGCAUA |
| SEQ ID NO: 13902 | UCGCAUC |
| SEQ ID NO: 13903 | UCGCAUG |
| SEQ ID NO: 13904 | UCGCAUU |
| SEQ ID NO: 13905 | UCGCCAA |
| SEQ ID NO: 13906 | UCGCCAC |
| SEQ ID NO: 13907 | UCGCCAG |
| SEQ ID NO: 13908 | UCGCCAU |
| SEQ ID NO: 13909 | UCGCCCA |
| SEQ ID NO: 13910 | UCGCCCC |
| SEQ ID NO: 13911 | UCGCCCG |
| SEQ ID NO: 13912 | UCGCCCU |
| SEQ ID NO: 13913 | UCGCCGA |
| SEQ ID NO: 13914 | UCGCCGC |
| SEQ ID NO: 13915 | UCGCCGG |
| SEQ ID NO: 13916 | UCGCCGU |
| SEQ ID NO: 13917 | UCGCCUA |
| SEQ ID NO: 13918 | UCGCCUC |
| SEQ ID NO: 13919 | UCGCCUG |
| SEQ ID NO: 13920 | UCGCCUU |
| SEQ ID NO: 13921 | UCGCGAA |
| SEQ ID NO: 13922 | UCGCGAC |
| SEQ ID NO: 13923 | UCGCGAG |
| SEQ ID NO: 13924 | UCGCGAU |
| SEQ ID NO: 13925 | UCGCGCA |
| SEQ ID NO: 13926 | UCGCGCC |
| SEQ ID NO: 13927 | UCGCGCG |
| SEQ ID NO: 13928 | UCGCGCU |
| SEQ ID NO: 13929 | UCGCGGA |
| SEQ ID NO: 13930 | UCGCGGC |
| SEQ ID NO: 13931 | UCGCGGG |
| SEQ ID NO: 13932 | UCGCGGU |
| SEQ ID NO: 13933 | UCGCGUA |
| SEQ ID NO: 13934 | UCGCGUC |
| SEQ ID NO: 13935 | UCGCGUG |
| SEQ ID NO: 13936 | UCGCGUU |
| SEQ ID NO: 13937 | UCGCUAA |
| SEQ ID NO: 13938 | UCGCUAC |
| SEQ ID NO: 13939 | UCGCUAG |
| SEQ ID NO: 13940 | UCGCUAU |
| SEQ ID NO: 13941 | UCGCUCA |
| SEQ ID NO: 13942 | UCGCUCC |
| SEQ ID NO: 13943 | UCGCUCG |
| SEQ ID NO: 13944 | UCGCUCU |
| SEQ ID NO: 13945 | UCGCUGA |
| SEQ ID NO: 13946 | UCGCUGC |
| SEQ ID NO: 13947 | UCGCUGG |
| SEQ ID NO: 13948 | UCGCUGU |
| SEQ ID NO: 13949 | UCGCUUA |
| SEQ ID NO: 13950 | UCGCUUC |
| SEQ ID NO: 13951 | UCGCUUG |
| SEQ ID NO: 13952 | UCGCUUU |
| SEQ ID NO: 13953 | UCGGAAA |
| SEQ ID NO: 13954 | UCGGAAC |
| SEQ ID NO: 13955 | UCGGAAG |
| SEQ ID NO: 13956 | UCGGAAU |
| SEQ ID NO: 13957 | UCGGACA |
| SEQ ID NO: 13958 | UCGGACC |
| SEQ ID NO: 13959 | UCGGACG |
| SEQ ID NO: 13960 | UCGGACU |
| SEQ ID NO: 13961 | UCGGAGA |
| SEQ ID NO: 13962 | UCGGAGC |
| SEQ ID NO: 13963 | UCGGAGG |
| SEQ ID NO: 13964 | UCGGAGU |
| SEQ ID NO: 13965 | UCGGAUA |
| SEQ ID NO: 13966 | UCGGAUC |
| SEQ ID NO: 13967 | UCGGAUG |
| SEQ ID NO: 13968 | UCGGAUU |
| SEQ ID NO: 13969 | UCGGCAA |
| SEQ ID NO: 13970 | UCGGCAC |
| SEQ ID NO: 13971 | UCGGCAG |
| SEQ ID NO: 13972 | UCGGCAU |
| SEQ ID NO: 13973 | UCGGCCA |
| SEQ ID NO: 13974 | UCGGCCC |
| SEQ ID NO: 13975 | UCGGCCG |
| SEQ ID NO: 13976 | UCGGCCU |
| SEQ ID NO: 13977 | UCGGCGA |
| SEQ ID NO: 13978 | UCGGCGC |
| SEQ ID NO: 13979 | UCGGCGG |
| SEQ ID NO: 13980 | UCGGCGU |
| SEQ ID NO: 13981 | UCGGCUA |
| SEQ ID NO: 13982 | UCGGCUC |
| SEQ ID NO: 13983 | UCGGCUG |
| SEQ ID NO: 13984 | UCGGCUU |
| SEQ ID NO: 13985 | UCGGGAA |
| SEQ ID NO: 13986 | UCGGGAC |
| SEQ ID NO: 13987 | UCGGGAG |
| SEQ ID NO: 13988 | UCGGGAU |
| SEQ ID NO: 13989 | UCGGGCA |
| SEQ ID NO: 13990 | UCGGGCC |
| SEQ ID NO: 13991 | UCGGGCG |
| SEQ ID NO: 13992 | UCGGGCU |
| SEQ ID NO: 13993 | UCGGGGA |
| SEQ ID NO: 13994 | UCGGGGC |
| SEQ ID NO: 13995 | UCGGGGG |
| SEQ ID NO: 13996 | UCGGGGU |
| SEQ ID NO: 13997 | UCGGGUA |
| SEQ ID NO: 13998 | UCGGGUC |
| SEQ ID NO: 13999 | UCGGGUG |
| SEQ ID NO: 14000 | UCGGGUU |
| SEQ ID NO: 14001 | UCGGUAA |
| SEQ ID NO: 14002 | UCGGUAC |
| SEQ ID NO: 14003 | UCGGUAG |
| SEQ ID NO: 14004 | UCGGUAU |
| SEQ ID NO: 14005 | UCGGUCA |
| SEQ ID NO: 14006 | UCGGUCC |
| SEQ ID NO: 14007 | UCGGUCG |
| SEQ ID NO: 14008 | UCGGUCU |
| SEQ ID NO: 14009 | UCGGUGA |
| SEQ ID NO: 14010 | UCGGUGC |
| SEQ ID NO: 14011 | UCGGUGG |
| SEQ ID NO: 14012 | UCGGUGU |
| SEQ ID NO: 14013 | UCGGUUA |
| SEQ ID NO: 14014 | UCGGUUC |
| SEQ ID NO: 14015 | UCGGUUG |
| SEQ ID NO: 14016 | UCGGUUU |
| SEQ ID NO: 14017 | UCGUAAA |
| SEQ ID NO: 14018 | UCGUAAC |
| SEQ ID NO: 14019 | UCGUAAG |
| SEQ ID NO: 14020 | UCGUAAU |
| SEQ ID NO: 14021 | UCGUACA |
| SEQ ID NO: 14022 | UCGUACC |
| SEQ ID NO: 14023 | UCGUACG |
| SEQ ID NO: 14024 | UCGUACU |
| SEQ ID NO: 14025 | UCGUAGA |
| SEQ ID NO: 14026 | UCGUAGC |
| SEQ ID NO: 14027 | UCGUAGG |
| SEQ ID NO: 14028 | UCGUAGU |
| SEQ ID NO: 14029 | UCGUAUA |
| SEQ ID NO: 14030 | UCGUAUC |
| SEQ ID NO: 14031 | UCGUAUG |
| SEQ ID NO: 14032 | UCGUAUU |
| SEQ ID NO: 14033 | UCGUCAA |
| SEQ ID NO: 14034 | UCGUCAC |
| SEQ ID NO: 14035 | UCGUCAG |
| SEQ ID NO: 14036 | UCGUCAU |
| SEQ ID NO: 14037 | UCGUCCA |
| SEQ ID NO: 14038 | UCGUCCC |
| SEQ ID NO: 14039 | UCGUCCG |
| SEQ ID NO: 14040 | UCGUCCU |
| SEQ ID NO: 14041 | UCGUCGA |
| SEQ ID NO: 14042 | UCGUCGC |
| SEQ ID NO: 14043 | UCGUCGG |
| SEQ ID NO: 14044 | UCGUCGU |
| SEQ ID NO: 14045 | UCGUCUA |
| SEQ ID NO: 14046 | UCGUCUC |
| SEQ ID NO: 14047 | UCGUCUG |
| SEQ ID NO: 14048 | UCGUCUU |
| SEQ ID NO: 14049 | UCGUGAA |
| SEQ ID NO: 14050 | UCGUGAC |
| SEQ ID NO: 14051 | UCGUGAG |
| SEQ ID NO: 14052 | UCGUGAU |
| SEQ ID NO: 14053 | UCGUGCA |
| SEQ ID NO: 14054 | UCGUGCC |
| SEQ ID NO: 14055 | UCGUGCG |
| SEQ ID NO: 14056 | UCGUGCU |
| SEQ ID NO: 14057 | UCGUGGA |
| SEQ ID NO: 14058 | UCGUGGC |
| SEQ ID NO: 14059 | UCGUGGG |
| SEQ ID NO: 14060 | UCGUGGU |
| SEQ ID NO: 14061 | UCGUGUA |
| SEQ ID NO: 14062 | UCGUGUC |
| SEQ ID NO: 14063 | UCGUGUG |
| SEQ ID NO: 14064 | UCGUGUU |
| SEQ ID NO: 14065 | UCGUUAA |
| SEQ ID NO: 14066 | UCGUUAC |
| SEQ ID NO: 14067 | UCGUUAG |
| SEQ ID NO: 14068 | UCGUUAU |
| SEQ ID NO: 14069 | UCGUUCA |
| SEQ ID NO: 14070 | UCGUUCC |
| SEQ ID NO: 14071 | UCGUUCG |
| SEQ ID NO: 14072 | UCGUUCU |
| SEQ ID NO: 14073 | UCGUUGA |
| SEQ ID NO: 14074 | UCGUUGC |
| SEQ ID NO: 14075 | UCGUUGG |
| SEQ ID NO: 14076 | UCGUUGU |

Table 47

| | |
|---|---|
| SEQ ID NO: 14077 | UCGUUUA |
| SEQ ID NO: 14078 | UCGUUUC |
| SEQ ID NO: 14079 | UCGUUUG |
| SEQ ID NO: 14080 | UCGUUUU |
| SEQ ID NO: 14081 | UCUAAAA |
| SEQ ID NO: 14082 | UCUAAAC |
| SEQ ID NO: 14083 | UCUAAAG |
| SEQ ID NO: 14084 | UCUAAAU |
| SEQ ID NO: 14085 | UCUAACA |
| SEQ ID NO: 14086 | UCUAACC |
| SEQ ID NO: 14087 | UCUAACG |
| SEQ ID NO: 14088 | UCUAACU |
| SEQ ID NO: 14089 | UCUAAGA |
| SEQ ID NO: 14090 | UCUAAGC |
| SEQ ID NO: 14091 | UCUAAGG |
| SEQ ID NO: 14092 | UCUAAGU |
| SEQ ID NO: 14093 | UCUAAUA |
| SEQ ID NO: 14094 | UCUAAUC |
| SEQ ID NO: 14095 | UCUAAUG |
| SEQ ID NO: 14096 | UCUAAUU |
| SEQ ID NO: 14097 | UCUACAA |
| SEQ ID NO: 14098 | UCUACAC |
| SEQ ID NO: 14099 | UCUACAG |
| SEQ ID NO: 14100 | UCUACAU |
| SEQ ID NO: 14101 | UCUACCA |
| SEQ ID NO: 14102 | UCUACCC |
| SEQ ID NO: 14103 | UCUACCG |
| SEQ ID NO: 14104 | UCUACCU |
| SEQ ID NO: 14105 | UCUACGA |
| SEQ ID NO: 14106 | UCUACGC |
| SEQ ID NO: 14107 | UCUACGG |
| SEQ ID NO: 14108 | UCUACGU |
| SEQ ID NO: 14109 | UCUACUA |
| SEQ ID NO: 14110 | UCUACUC |
| SEQ ID NO: 14111 | UCUACUG |
| SEQ ID NO: 14112 | UCUACUU |
| SEQ ID NO: 14113 | UCUAGAA |
| SEQ ID NO: 14114 | UCUAGAC |
| SEQ ID NO: 14115 | UCUAGAG |
| SEQ ID NO: 14116 | UCUAGAU |
| SEQ ID NO: 14117 | UCUAGCA |
| SEQ ID NO: 14118 | UCUAGCC |
| SEQ ID NO: 14119 | UCUAGCG |
| SEQ ID NO: 14120 | UCUAGCU |
| SEQ ID NO: 14121 | UCUAGGA |
| SEQ ID NO: 14122 | UCUAGGC |
| SEQ ID NO: 14123 | UCUAGGG |
| SEQ ID NO: 14124 | UCUAGGU |
| SEQ ID NO: 14125 | UCUAGUA |
| SEQ ID NO: 14126 | UCUAGUC |
| SEQ ID NO: 14127 | UCUAGUG |
| SEQ ID NO: 14128 | UCUAGUU |
| SEQ ID NO: 14129 | UCUAUAA |
| SEQ ID NO: 14130 | UCUAUAC |
| SEQ ID NO: 14131 | UCUAUAG |
| SEQ ID NO: 14132 | UCUAUAU |
| SEQ ID NO: 14133 | UCUAUCA |
| SEQ ID NO: 14134 | UCUAUCC |
| SEQ ID NO: 14135 | UCUAUCG |
| SEQ ID NO: 14136 | UCUAUCU |
| SEQ ID NO: 14137 | UCUAUGA |
| SEQ ID NO: 14138 | UCUAUGC |
| SEQ ID NO: 14139 | UCUAUGG |
| SEQ ID NO: 14140 | UCUAUGU |
| SEQ ID NO: 14141 | UCUAUUA |
| SEQ ID NO: 14142 | UCUAUUC |
| SEQ ID NO: 14143 | UCUAUUG |
| SEQ ID NO: 14144 | UCUAUUU |
| SEQ ID NO: 14145 | UCUCAAA |
| SEQ ID NO: 14146 | UCUCAAC |
| SEQ ID NO: 14147 | UCUCAAG |
| SEQ ID NO: 14148 | UCUCAAU |
| SEQ ID NO: 14149 | UCUCACA |
| SEQ ID NO: 14150 | UCUCACC |
| SEQ ID NO: 14151 | UCUCACG |
| SEQ ID NO: 14152 | UCUCACU |
| SEQ ID NO: 14153 | UCUCAGA |
| SEQ ID NO: 14154 | UCUCAGC |
| SEQ ID NO: 14155 | UCUCAGG |
| SEQ ID NO: 14156 | UCUCAGU |
| SEQ ID NO: 14157 | UCUCAUA |
| SEQ ID NO: 14158 | UCUCAUC |
| SEQ ID NO: 14159 | UCUCAUG |
| SEQ ID NO: 14160 | UCUCAUU |
| SEQ ID NO: 14161 | UCUCCAA |
| SEQ ID NO: 14162 | UCUCCAC |
| SEQ ID NO: 14163 | UCUCCAG |
| SEQ ID NO: 14164 | UCUCCAU |
| SEQ ID NO: 14165 | UCUCCCA |
| SEQ ID NO: 14166 | UCUCCCC |
| SEQ ID NO: 14167 | UCUCCCG |
| SEQ ID NO: 14168 | UCUCCCU |
| SEQ ID NO: 14169 | UCUCCGA |
| SEQ ID NO: 14170 | UCUCCGC |
| SEQ ID NO: 14171 | UCUCCGG |
| SEQ ID NO: 14172 | UCUCCGU |
| SEQ ID NO: 14173 | UCUCCUA |
| SEQ ID NO: 14174 | UCUCCUC |
| SEQ ID NO: 14175 | UCUCCUG |
| SEQ ID NO: 14176 | UCUCCUU |
| SEQ ID NO: 14177 | UCUCGAA |
| SEQ ID NO: 14178 | UCUCGAC |
| SEQ ID NO: 14179 | UCUCGAG |
| SEQ ID NO: 14180 | UCUCGAU |
| SEQ ID NO: 14181 | UCUCGCA |
| SEQ ID NO: 14182 | UCUCGCC |
| SEQ ID NO: 14183 | UCUCGCG |
| SEQ ID NO: 14184 | UCUCGCU |
| SEQ ID NO: 14185 | UCUCGGA |
| SEQ ID NO: 14186 | UCUCGGC |
| SEQ ID NO: 14187 | UCUCGGG |
| SEQ ID NO: 14188 | UCUCGGU |
| SEQ ID NO: 14189 | UCUCGUA |
| SEQ ID NO: 14190 | UCUCGUC |
| SEQ ID NO: 14191 | UCUCGUG |
| SEQ ID NO: 14192 | UCUCGUU |
| SEQ ID NO: 14193 | UCUCUAA |
| SEQ ID NO: 14194 | UCUCUAC |
| SEQ ID NO: 14195 | UCUCUAG |
| SEQ ID NO: 14196 | UCUCUAU |
| SEQ ID NO: 14197 | UCUCUCA |
| SEQ ID NO: 14198 | UCUCUCC |
| SEQ ID NO: 14199 | UCUCUCG |
| SEQ ID NO: 14200 | UCUCUCU |
| SEQ ID NO: 14201 | UCUCUGA |
| SEQ ID NO: 14202 | UCUCUGC |
| SEQ ID NO: 14203 | UCUCUGG |
| SEQ ID NO: 14204 | UCUCUGU |
| SEQ ID NO: 14205 | UCUCUUA |
| SEQ ID NO: 14206 | UCUCUUC |
| SEQ ID NO: 14207 | UCUCUUG |
| SEQ ID NO: 14208 | UCUCUUU |
| SEQ ID NO: 14209 | UCUGAAA |
| SEQ ID NO: 14210 | UCUGAAC |
| SEQ ID NO: 14211 | UCUGAAG |
| SEQ ID NO: 14212 | UCUGAAU |
| SEQ ID NO: 14213 | UCUGACA |
| SEQ ID NO: 14214 | UCUGACC |
| SEQ ID NO: 14215 | UCUGACG |
| SEQ ID NO: 14216 | UCUGACU |
| SEQ ID NO: 14217 | UCUGAGA |
| SEQ ID NO: 14218 | UCUGAGC |
| SEQ ID NO: 14219 | UCUGAGG |
| SEQ ID NO: 14220 | UCUGAGU |
| SEQ ID NO: 14221 | UCUGAUA |
| SEQ ID NO: 14222 | UCUGAUC |
| SEQ ID NO: 14223 | UCUGAUG |
| SEQ ID NO: 14224 | UCUGAUU |
| SEQ ID NO: 14225 | UCUGCAA |
| SEQ ID NO: 14226 | UCUGCAC |
| SEQ ID NO: 14227 | UCUGCAG |
| SEQ ID NO: 14228 | UCUGCAU |
| SEQ ID NO: 14229 | UCUGCCA |
| SEQ ID NO: 14230 | UCUGCCC |
| SEQ ID NO: 14231 | UCUGCCG |
| SEQ ID NO: 14232 | UCUGCCU |
| SEQ ID NO: 14233 | UCUGCGA |
| SEQ ID NO: 14234 | UCUGCGC |
| SEQ ID NO: 14235 | UCUGCGG |
| SEQ ID NO: 14236 | UCUGCGU |
| SEQ ID NO: 14237 | UCUGCUA |
| SEQ ID NO: 14238 | UCUGCUC |
| SEQ ID NO: 14239 | UCUGCUG |
| SEQ ID NO: 14240 | UCUGCUU |
| SEQ ID NO: 14241 | UCUGGAA |
| SEQ ID NO: 14242 | UCUGGAC |
| SEQ ID NO: 14243 | UCUGGAG |
| SEQ ID NO: 14244 | UCUGGAU |
| SEQ ID NO: 14245 | UCUGGCA |
| SEQ ID NO: 14246 | UCUGGCC |
| SEQ ID NO: 14247 | UCUGGCG |
| SEQ ID NO: 14248 | UCUGGCU |
| SEQ ID NO: 14249 | UCUGGGA |
| SEQ ID NO: 14250 | UCUGGGC |
| SEQ ID NO: 14251 | UCUGGGG |
| SEQ ID NO: 14252 | UCUGGGU |
| SEQ ID NO: 14253 | UCUGGUA |
| SEQ ID NO: 14254 | UCUGGUC |
| SEQ ID NO: 14255 | UCUGGUG |
| SEQ ID NO: 14256 | UCUGGUU |
| SEQ ID NO: 14257 | UCUGUAA |
| SEQ ID NO: 14258 | UCUGUAC |
| SEQ ID NO: 14259 | UCUGUAG |
| SEQ ID NO: 14260 | UCUGUAU |
| SEQ ID NO: 14261 | UCUGUCA |
| SEQ ID NO: 14262 | UCUGUCC |
| SEQ ID NO: 14263 | UCUGUCG |
| SEQ ID NO: 14264 | UCUGUCU |
| SEQ ID NO: 14265 | UCUGUGA |
| SEQ ID NO: 14266 | UCUGUGC |
| SEQ ID NO: 14267 | UCUGUGG |
| SEQ ID NO: 14268 | UCUGUGU |
| SEQ ID NO: 14269 | UCUGUUA |
| SEQ ID NO: 14270 | UCUGUUC |
| SEQ ID NO: 14271 | UCUGUUG |
| SEQ ID NO: 14272 | UCUGUUU |
| SEQ ID NO: 14273 | UCUUAAA |
| SEQ ID NO: 14274 | UCUUAAC |
| SEQ ID NO: 14275 | UCUUAAG |
| SEQ ID NO: 14276 | UCUUAAU |
| SEQ ID NO: 14277 | UCUUACA |
| SEQ ID NO: 14278 | UCUUACC |
| SEQ ID NO: 14279 | UCUUACG |
| SEQ ID NO: 14280 | UCUUACU |
| SEQ ID NO: 14281 | UCUUAGA |
| SEQ ID NO: 14282 | UCUUAGC |
| SEQ ID NO: 14283 | UCUUAGG |
| SEQ ID NO: 14284 | UCUUAGU |
| SEQ ID NO: 14285 | UCUUAUA |
| SEQ ID NO: 14286 | UCUUAUC |
| SEQ ID NO: 14287 | UCUUAUG |
| SEQ ID NO: 14288 | UCUUAUU |
| SEQ ID NO: 14289 | UCUUCAA |
| SEQ ID NO: 14290 | UCUUCAC |
| SEQ ID NO: 14291 | UCUUCAG |
| SEQ ID NO: 14292 | UCUUCAU |
| SEQ ID NO: 14293 | UCUUCCA |
| SEQ ID NO: 14294 | UCUUCCC |
| SEQ ID NO: 14295 | UCUUCCG |
| SEQ ID NO: 14296 | UCUUCCU |
| SEQ ID NO: 14297 | UCUUCGA |
| SEQ ID NO: 14298 | UCUUCGC |
| SEQ ID NO: 14299 | UCUUCGG |
| SEQ ID NO: 14300 | UCUUCGU |
| SEQ ID NO: 14301 | UCUUCUA |
| SEQ ID NO: 14302 | UCUUCUC |
| SEQ ID NO: 14303 | UCUUCUG |
| SEQ ID NO: 14304 | UCUUCUU |
| SEQ ID NO: 14305 | UCUUGAA |
| SEQ ID NO: 14306 | UCUUGAC |
| SEQ ID NO: 14307 | UCUUGAG |
| SEQ ID NO: 14308 | UCUUGAU |
| SEQ ID NO: 14309 | UCUUGCA |
| SEQ ID NO: 14310 | UCUUGCC |
| SEQ ID NO: 14311 | UCUUGCG |
| SEQ ID NO: 14312 | UCUUGCU |
| SEQ ID NO: 14313 | UCUUGGA |
| SEQ ID NO: 14314 | UCUUGGC |
| SEQ ID NO: 14315 | UCUUGGG |
| SEQ ID NO: 14316 | UCUUGGU |
| SEQ ID NO: 14317 | UCUUGUA |
| SEQ ID NO: 14318 | UCUUGUC |
| SEQ ID NO: 14319 | UCUUGUG |
| SEQ ID NO: 14320 | UCUUGUU |
| SEQ ID NO: 14321 | UCUUUAA |
| SEQ ID NO: 14322 | UCUUUAC |
| SEQ ID NO: 14323 | UCUUUAG |
| SEQ ID NO: 14324 | UCUUUAU |
| SEQ ID NO: 14325 | UCUUUCA |
| SEQ ID NO: 14326 | UCUUUCC |
| SEQ ID NO: 14327 | UCUUUCG |
| SEQ ID NO: 14328 | UCUUUCU |
| SEQ ID NO: 14329 | UCUUUGA |
| SEQ ID NO: 14330 | UCUUUGC |
| SEQ ID NO: 14331 | UCUUUGG |
| SEQ ID NO: 14332 | UCUUUGU |
| SEQ ID NO: 14333 | UCUUUUA |
| SEQ ID NO: 14334 | UCUUUUC |
| SEQ ID NO: 14335 | UCUUUUG |
| SEQ ID NO: 14336 | UCUUUUU |
| SEQ ID NO: 14337 | UGAAAAA |
| SEQ ID NO: 14338 | UGAAAAC |
| SEQ ID NO: 14339 | UGAAAAG |
| SEQ ID NO: 14340 | UGAAAAU |
| SEQ ID NO: 14341 | UGAAACA |
| SEQ ID NO: 14342 | UGAAACC |
| SEQ ID NO: 14343 | UGAAACG |
| SEQ ID NO: 14344 | UGAAACU |
| SEQ ID NO: 14345 | UGAAAGA |
| SEQ ID NO: 14346 | UGAAAGC |
| SEQ ID NO: 14347 | UGAAAGG |
| SEQ ID NO: 14348 | UGAAAGU |
| SEQ ID NO: 14349 | UGAAAUA |
| SEQ ID NO: 14350 | UGAAAUC |
| SEQ ID NO: 14351 | UGAAAUG |
| SEQ ID NO: 14352 | UGAAAUU |
| SEQ ID NO: 14353 | UGAACAA |
| SEQ ID NO: 14354 | UGAACAC |
| SEQ ID NO: 14355 | UGAACAG |
| SEQ ID NO: 14356 | UGAACAU |
| SEQ ID NO: 14357 | UGAACCA |
| SEQ ID NO: 14358 | UGAACCC |
| SEQ ID NO: 14359 | UGAACCG |
| SEQ ID NO: 14360 | UGAACCU |
| SEQ ID NO: 14361 | UGAACGA |
| SEQ ID NO: 14362 | UGAACGC |
| SEQ ID NO: 14363 | UGAACGG |
| SEQ ID NO: 14364 | UGAACGU |
| SEQ ID NO: 14365 | UGAACUA |
| SEQ ID NO: 14366 | UGAACUC |
| SEQ ID NO: 14367 | UGAACUG |
| SEQ ID NO: 14368 | UGAACUU |
| SEQ ID NO: 14369 | UGAAGAA |
| SEQ ID NO: 14370 | UGAAGAC |
| SEQ ID NO: 14371 | UGAAGAG |
| SEQ ID NO: 14372 | UGAAGAU |
| SEQ ID NO: 14373 | UGAAGCA |
| SEQ ID NO: 14374 | UGAAGCC |
| SEQ ID NO: 14375 | UGAAGCG |
| SEQ ID NO: 14376 | UGAAGCU |
| SEQ ID NO: 14377 | UGAAGGA |
| SEQ ID NO: 14378 | UGAAGGC |
| SEQ ID NO: 14379 | UGAAGGG |
| SEQ ID NO: 14380 | UGAAGGU |
| SEQ ID NO: 14381 | UGAAGUA |
| SEQ ID NO: 14382 | UGAAGUC |

Table 48

| | | | | | | | | | | | |
|---|---|---|---|---|---|---|---|---|---|---|---|
| SEQ ID NO: 14383 | UGAAGUG | SEQ ID NO: 14434 | UGACGAC | SEQ ID NO: 14485 | UGAGCCA | SEQ ID NO: 14536 | UGAUACU | SEQ ID NO: 14587 | UGAUUGG | SEQ ID NO: 14638 | UGCAGUC |
| SEQ ID NO: 14384 | UGAAGUU | SEQ ID NO: 14435 | UGACGAG | SEQ ID NO: 14486 | UGAGCCC | SEQ ID NO: 14537 | UGAUAGA | SEQ ID NO: 14588 | UGAUUGU | SEQ ID NO: 14639 | UGCAGUG |
| SEQ ID NO: 14385 | UGAAUAA | SEQ ID NO: 14436 | UGACGAU | SEQ ID NO: 14487 | UGAGCCG | SEQ ID NO: 14538 | UGAUAGC | SEQ ID NO: 14589 | UGAUUUA | SEQ ID NO: 14640 | UGCAGUU |
| SEQ ID NO: 14386 | UGAAUAC | SEQ ID NO: 14437 | UGACGCA | SEQ ID NO: 14488 | UGAGCCU | SEQ ID NO: 14539 | UGAUAGG | SEQ ID NO: 14590 | UGAUUUC | SEQ ID NO: 14641 | UGCAUAA |
| SEQ ID NO: 14387 | UGAAUAG | SEQ ID NO: 14438 | UGACGCC | SEQ ID NO: 14489 | UGAGCGA | SEQ ID NO: 14540 | UGAUAGU | SEQ ID NO: 14591 | UGAUUUG | SEQ ID NO: 14642 | UGCAUAC |
| SEQ ID NO: 14388 | UGAAUAU | SEQ ID NO: 14439 | UGACGCG | SEQ ID NO: 14490 | UGAGCGC | SEQ ID NO: 14541 | UGAUAUA | SEQ ID NO: 14592 | UGAUUUU | SEQ ID NO: 14643 | UGCAUAG |
| SEQ ID NO: 14389 | UGAAUCA | SEQ ID NO: 14440 | UGACGCU | SEQ ID NO: 14491 | UGAGCGG | SEQ ID NO: 14542 | UGAUAUC | SEQ ID NO: 14593 | UGCAAAA | SEQ ID NO: 14644 | UGCAUAU |
| SEQ ID NO: 14390 | UGAAUCC | SEQ ID NO: 14441 | UGACGGA | SEQ ID NO: 14492 | UGAGCGU | SEQ ID NO: 14543 | UGAUAUG | SEQ ID NO: 14594 | UGCAAAC | SEQ ID NO: 14645 | UGCAUCA |
| SEQ ID NO: 14391 | UGAAUCG | SEQ ID NO: 14442 | UGACGGC | SEQ ID NO: 14493 | UGAGCUA | SEQ ID NO: 14544 | UGAUAUU | SEQ ID NO: 14595 | UGCAAAG | SEQ ID NO: 14646 | UGCAUCC |
| SEQ ID NO: 14392 | UGAAUCU | SEQ ID NO: 14443 | UGACGGG | SEQ ID NO: 14494 | UGAGCUC | SEQ ID NO: 14545 | UGAUCAA | SEQ ID NO: 14596 | UGCAAAU | SEQ ID NO: 14647 | UGCAUCG |
| SEQ ID NO: 14393 | UGAAUGA | SEQ ID NO: 14444 | UGACGGU | SEQ ID NO: 14495 | UGAGCUG | SEQ ID NO: 14546 | UGAUCAC | SEQ ID NO: 14597 | UGCAACA | SEQ ID NO: 14648 | UGCAUCU |
| SEQ ID NO: 14394 | UGAAUGC | SEQ ID NO: 14445 | UGACGUA | SEQ ID NO: 14496 | UGAGCUU | SEQ ID NO: 14547 | UGAUCAG | SEQ ID NO: 14598 | UGCAACC | SEQ ID NO: 14649 | UGCAUGA |
| SEQ ID NO: 14395 | UGAAUGG | SEQ ID NO: 14446 | UGACGUC | SEQ ID NO: 14497 | UGAGGAA | SEQ ID NO: 14548 | UGAUCAU | SEQ ID NO: 14599 | UGCAACG | SEQ ID NO: 14650 | UGCAUGC |
| SEQ ID NO: 14396 | UGAAUGU | SEQ ID NO: 14447 | UGACGUG | SEQ ID NO: 14498 | UGAGGAC | SEQ ID NO: 14549 | UGAUCCA | SEQ ID NO: 14600 | UGCAACU | SEQ ID NO: 14651 | UGCAUGG |
| SEQ ID NO: 14397 | UGAAUUA | SEQ ID NO: 14448 | UGACGUU | SEQ ID NO: 14499 | UGAGGAG | SEQ ID NO: 14550 | UGAUCCC | SEQ ID NO: 14601 | UGCAAGA | SEQ ID NO: 14652 | UGCAUGU |
| SEQ ID NO: 14398 | UGAAUUC | SEQ ID NO: 14449 | UGACUAA | SEQ ID NO: 14500 | UGAGGAU | SEQ ID NO: 14551 | UGAUCCG | SEQ ID NO: 14602 | UGCAAGC | SEQ ID NO: 14653 | UGCAUUA |
| SEQ ID NO: 14399 | UGAAUUG | SEQ ID NO: 14450 | UGACUAC | SEQ ID NO: 14501 | UGAGGCA | SEQ ID NO: 14552 | UGAUCCU | SEQ ID NO: 14603 | UGCAAGG | SEQ ID NO: 14654 | UGCAUUC |
| SEQ ID NO: 14400 | UGAAUUU | SEQ ID NO: 14451 | UGACUAG | SEQ ID NO: 14502 | UGAGGCC | SEQ ID NO: 14553 | UGAUCGA | SEQ ID NO: 14604 | UGCAAGU | SEQ ID NO: 14655 | UGCAUUG |
| SEQ ID NO: 14401 | UGACAAA | SEQ ID NO: 14452 | UGACUAU | SEQ ID NO: 14503 | UGAGGCG | SEQ ID NO: 14554 | UGAUCGC | SEQ ID NO: 14605 | UGCAAUA | SEQ ID NO: 14656 | UGCAUUU |
| SEQ ID NO: 14402 | UGACAAC | SEQ ID NO: 14453 | UGACUCA | SEQ ID NO: 14504 | UGAGGCU | SEQ ID NO: 14555 | UGAUCGG | SEQ ID NO: 14606 | UGCAAUC | SEQ ID NO: 14657 | UGCCAAA |
| SEQ ID NO: 14403 | UGACAAG | SEQ ID NO: 14454 | UGACUCC | SEQ ID NO: 14505 | UGAGGGA | SEQ ID NO: 14556 | UGAUCGU | SEQ ID NO: 14607 | UGCAAUG | SEQ ID NO: 14658 | UGCCAAC |
| SEQ ID NO: 14404 | UGACAAU | SEQ ID NO: 14455 | UGACUCG | SEQ ID NO: 14506 | UGAGGGC | SEQ ID NO: 14557 | UGAUCUA | SEQ ID NO: 14608 | UGCAAUU | SEQ ID NO: 14659 | UGCCAAG |
| SEQ ID NO: 14405 | UGACACA | SEQ ID NO: 14456 | UGACUCU | SEQ ID NO: 14507 | UGAGGGG | SEQ ID NO: 14558 | UGAUCUC | SEQ ID NO: 14609 | UGCACAA | SEQ ID NO: 14660 | UGCCAAU |
| SEQ ID NO: 14406 | UGACACC | SEQ ID NO: 14457 | UGACUGA | SEQ ID NO: 14508 | UGAGGGU | SEQ ID NO: 14559 | UGAUCUG | SEQ ID NO: 14610 | UGCACAC | SEQ ID NO: 14661 | UGCCACA |
| SEQ ID NO: 14407 | UGACACG | SEQ ID NO: 14458 | UGACUGC | SEQ ID NO: 14509 | UGAGGUA | SEQ ID NO: 14560 | UGAUCUU | SEQ ID NO: 14611 | UGCACAG | SEQ ID NO: 14662 | UGCCACC |
| SEQ ID NO: 14408 | UGACACU | SEQ ID NO: 14459 | UGACUGG | SEQ ID NO: 14510 | UGAGGUC | SEQ ID NO: 14561 | UGAUGAA | SEQ ID NO: 14612 | UGCACAU | SEQ ID NO: 14663 | UGCCACG |
| SEQ ID NO: 14409 | UGACAGA | SEQ ID NO: 14460 | UGACUGU | SEQ ID NO: 14511 | UGAGGUG | SEQ ID NO: 14562 | UGAUGAC | SEQ ID NO: 14613 | UGCACCA | SEQ ID NO: 14664 | UGCCACU |
| SEQ ID NO: 14410 | UGACAGC | SEQ ID NO: 14461 | UGACUUA | SEQ ID NO: 14512 | UGAGGUU | SEQ ID NO: 14563 | UGAUGAG | SEQ ID NO: 14614 | UGCACCC | SEQ ID NO: 14665 | UGCCAGA |
| SEQ ID NO: 14411 | UGACAGG | SEQ ID NO: 14462 | UGACUUC | SEQ ID NO: 14513 | UGAGUAA | SEQ ID NO: 14564 | UGAUGAU | SEQ ID NO: 14615 | UGCACCG | SEQ ID NO: 14666 | UGCCAGC |
| SEQ ID NO: 14412 | UGACAGU | SEQ ID NO: 14463 | UGACUUG | SEQ ID NO: 14514 | UGAGUAC | SEQ ID NO: 14565 | UGAUGCA | SEQ ID NO: 14616 | UGCACCU | SEQ ID NO: 14667 | UGCCAGG |
| SEQ ID NO: 14413 | UGACAUA | SEQ ID NO: 14464 | UGACUUU | SEQ ID NO: 14515 | UGAGUAG | SEQ ID NO: 14566 | UGAUGCC | SEQ ID NO: 14617 | UGCACGA | SEQ ID NO: 14668 | UGCCAGU |
| SEQ ID NO: 14414 | UGACAUC | SEQ ID NO: 14465 | UGAGAAA | SEQ ID NO: 14516 | UGAGUAU | SEQ ID NO: 14567 | UGAUGCG | SEQ ID NO: 14618 | UGCACGC | SEQ ID NO: 14669 | UGCCAUA |
| SEQ ID NO: 14415 | UGACAUG | SEQ ID NO: 14466 | UGAGAAC | SEQ ID NO: 14517 | UGAGUCA | SEQ ID NO: 14568 | UGAUGCU | SEQ ID NO: 14619 | UGCACGG | SEQ ID NO: 14670 | UGCCAUC |
| SEQ ID NO: 14416 | UGACAUU | SEQ ID NO: 14467 | UGAGAAG | SEQ ID NO: 14518 | UGAGUCC | SEQ ID NO: 14569 | UGAUGGA | SEQ ID NO: 14620 | UGCACGU | SEQ ID NO: 14671 | UGCCAUG |
| SEQ ID NO: 14417 | UGACCAA | SEQ ID NO: 14468 | UGAGAAU | SEQ ID NO: 14519 | UGAGUCG | SEQ ID NO: 14570 | UGAUGGC | SEQ ID NO: 14621 | UGCACUA | SEQ ID NO: 14672 | UGCCAUU |
| SEQ ID NO: 14418 | UGACCAC | SEQ ID NO: 14469 | UGAGACA | SEQ ID NO: 14520 | UGAGUCU | SEQ ID NO: 14571 | UGAUGGG | SEQ ID NO: 14622 | UGCACUC | SEQ ID NO: 14673 | UGCCCAA |
| SEQ ID NO: 14419 | UGACCAG | SEQ ID NO: 14470 | UGAGACC | SEQ ID NO: 14521 | UGAGUGA | SEQ ID NO: 14572 | UGAUGGU | SEQ ID NO: 14623 | UGCACUG | SEQ ID NO: 14674 | UGCCCAC |
| SEQ ID NO: 14420 | UGACCAU | SEQ ID NO: 14471 | UGAGACG | SEQ ID NO: 14522 | UGAGUGC | SEQ ID NO: 14573 | UGAUGUA | SEQ ID NO: 14624 | UGCACUU | SEQ ID NO: 14675 | UGCCCAG |
| SEQ ID NO: 14421 | UGACCCA | SEQ ID NO: 14472 | UGAGACU | SEQ ID NO: 14523 | UGAGUGG | SEQ ID NO: 14574 | UGAUGUC | SEQ ID NO: 14625 | UGCAGAA | SEQ ID NO: 14676 | UGCCCAU |
| SEQ ID NO: 14422 | UGACCCC | SEQ ID NO: 14473 | UGAGAGA | SEQ ID NO: 14524 | UGAGUGU | SEQ ID NO: 14575 | UGAUGUG | SEQ ID NO: 14626 | UGCAGAC | SEQ ID NO: 14677 | UGCCCCA |
| SEQ ID NO: 14423 | UGACCCG | SEQ ID NO: 14474 | UGAGAGC | SEQ ID NO: 14525 | UGAGUUA | SEQ ID NO: 14576 | UGAUGUU | SEQ ID NO: 14627 | UGCAGAG | SEQ ID NO: 14678 | UGCCCCC |
| SEQ ID NO: 14424 | UGACCCU | SEQ ID NO: 14475 | UGAGAGG | SEQ ID NO: 14526 | UGAGUUC | SEQ ID NO: 14577 | UGAUUAA | SEQ ID NO: 14628 | UGCAGAU | SEQ ID NO: 14679 | UGCCCCG |
| SEQ ID NO: 14425 | UGACCGA | SEQ ID NO: 14476 | UGAGAGU | SEQ ID NO: 14527 | UGAGUUG | SEQ ID NO: 14578 | UGAUUAC | SEQ ID NO: 14629 | UGCAGCA | SEQ ID NO: 14680 | UGCCCCU |
| SEQ ID NO: 14426 | UGACCGC | SEQ ID NO: 14477 | UGAGAUA | SEQ ID NO: 14528 | UGAGUUU | SEQ ID NO: 14579 | UGAUUAG | SEQ ID NO: 14630 | UGCAGCC | SEQ ID NO: 14681 | UGCCCGA |
| SEQ ID NO: 14427 | UGACCGG | SEQ ID NO: 14478 | UGAGAUC | SEQ ID NO: 14529 | UGAUAAA | SEQ ID NO: 14580 | UGAUUAU | SEQ ID NO: 14631 | UGCAGCG | SEQ ID NO: 14682 | UGCCCGC |
| SEQ ID NO: 14428 | UGACCGU | SEQ ID NO: 14479 | UGAGAUG | SEQ ID NO: 14530 | UGAUAAC | SEQ ID NO: 14581 | UGAUUCA | SEQ ID NO: 14632 | UGCAGCU | SEQ ID NO: 14683 | UGCCCGG |
| SEQ ID NO: 14429 | UGACCUA | SEQ ID NO: 14480 | UGAGAUU | SEQ ID NO: 14531 | UGAUAAG | SEQ ID NO: 14582 | UGAUUCC | SEQ ID NO: 14633 | UGCAGGA | SEQ ID NO: 14684 | UGCCCGU |
| SEQ ID NO: 14430 | UGACCUC | SEQ ID NO: 14481 | UGAGCAA | SEQ ID NO: 14532 | UGAUAAU | SEQ ID NO: 14583 | UGAUUCG | SEQ ID NO: 14634 | UGCAGGC | SEQ ID NO: 14685 | UGCCCUA |
| SEQ ID NO: 14431 | UGACCUG | SEQ ID NO: 14482 | UGAGCAC | SEQ ID NO: 14533 | UGAUACA | SEQ ID NO: 14584 | UGAUUCU | SEQ ID NO: 14635 | UGCAGGG | SEQ ID NO: 14686 | UGCCCUC |
| SEQ ID NO: 14432 | UGACCUU | SEQ ID NO: 14483 | UGAGCAG | SEQ ID NO: 14534 | UGAUACC | SEQ ID NO: 14585 | UGAUUGA | SEQ ID NO: 14636 | UGCAGGU | SEQ ID NO: 14687 | UGCCCUG |
| SEQ ID NO: 14433 | UGACGAA | SEQ ID NO: 14484 | UGAGCAU | SEQ ID NO: 14535 | UGAUACG | SEQ ID NO: 14586 | UGAUUGC | SEQ ID NO: 14637 | UGCAGUA | SEQ ID NO: 14688 | UGCCCUU |

Table 49

| | |
|---|---|
| SEQ ID NO: 14689 | UGCCGAA |
| SEQ ID NO: 14690 | UGCCGAC |
| SEQ ID NO: 14691 | UGCCGAG |
| SEQ ID NO: 14692 | UGCCGAU |
| SEQ ID NO: 14693 | UGCCGCA |
| SEQ ID NO: 14694 | UGCCGCC |
| SEQ ID NO: 14695 | UGCCGCG |
| SEQ ID NO: 14696 | UGCCGCU |
| SEQ ID NO: 14697 | UGCCGGA |
| SEQ ID NO: 14698 | UGCCGGC |
| SEQ ID NO: 14699 | UGCCGGG |
| SEQ ID NO: 14700 | UGCCGGU |
| SEQ ID NO: 14701 | UGCCGUA |
| SEQ ID NO: 14702 | UGCCGUC |
| SEQ ID NO: 14703 | UGCCGUG |
| SEQ ID NO: 14704 | UGCCGUU |
| SEQ ID NO: 14705 | UGCCUAA |
| SEQ ID NO: 14706 | UGCCUAC |
| SEQ ID NO: 14707 | UGCCUAG |
| SEQ ID NO: 14708 | UGCCUAU |
| SEQ ID NO: 14709 | UGCCUCA |
| SEQ ID NO: 14710 | UGCCUCC |
| SEQ ID NO: 14711 | UGCCUCG |
| SEQ ID NO: 14712 | UGCCUCU |
| SEQ ID NO: 14713 | UGCCUGA |
| SEQ ID NO: 14714 | UGCCUGC |
| SEQ ID NO: 14715 | UGCCUGG |
| SEQ ID NO: 14716 | UGCCUGU |
| SEQ ID NO: 14717 | UGCCUUA |
| SEQ ID NO: 14718 | UGCCUUC |
| SEQ ID NO: 14719 | UGCCUUG |
| SEQ ID NO: 14720 | UGCCUUU |
| SEQ ID NO: 14721 | UGCGAAA |
| SEQ ID NO: 14722 | UGCGAAC |
| SEQ ID NO: 14723 | UGCGAAG |
| SEQ ID NO: 14724 | UGCGAAU |
| SEQ ID NO: 14725 | UGCGACA |
| SEQ ID NO: 14726 | UGCGACC |
| SEQ ID NO: 14727 | UGCGACG |
| SEQ ID NO: 14728 | UGCGACU |
| SEQ ID NO: 14729 | UGCGAGA |
| SEQ ID NO: 14730 | UGCGAGC |
| SEQ ID NO: 14731 | UGCGAGG |
| SEQ ID NO: 14732 | UGCGAGU |
| SEQ ID NO: 14733 | UGCGAUA |
| SEQ ID NO: 14734 | UGCGAUC |
| SEQ ID NO: 14735 | UGCGAUG |
| SEQ ID NO: 14736 | UGCGAUU |
| SEQ ID NO: 14737 | UGCGCAA |
| SEQ ID NO: 14738 | UGCGCAC |
| SEQ ID NO: 14739 | UGCGCAG |
| SEQ ID NO: 14740 | UGCGCAU |
| SEQ ID NO: 14741 | UGCGCCA |
| SEQ ID NO: 14742 | UGCGCCC |
| SEQ ID NO: 14743 | UGCGCCG |
| SEQ ID NO: 14744 | UGCGCCU |
| SEQ ID NO: 14745 | UGCGCGA |
| SEQ ID NO: 14746 | UGCGCGC |
| SEQ ID NO: 14747 | UGCGCGG |
| SEQ ID NO: 14748 | UGCGCGU |
| SEQ ID NO: 14749 | UGCGCUA |
| SEQ ID NO: 14750 | UGCGCUC |
| SEQ ID NO: 14751 | UGCGCUG |
| SEQ ID NO: 14752 | UGCGCUU |
| SEQ ID NO: 14753 | UGCGGAA |
| SEQ ID NO: 14754 | UGCGGAC |
| SEQ ID NO: 14755 | UGCGGAG |
| SEQ ID NO: 14756 | UGCGGAU |
| SEQ ID NO: 14757 | UGCGGCA |
| SEQ ID NO: 14758 | UGCGGCC |
| SEQ ID NO: 14759 | UGCGGCG |
| SEQ ID NO: 14760 | UGCGGCU |
| SEQ ID NO: 14761 | UGCGGGA |
| SEQ ID NO: 14762 | UGCGGGC |
| SEQ ID NO: 14763 | UGCGGGG |
| SEQ ID NO: 14764 | UGCGGGU |
| SEQ ID NO: 14765 | UGCGGUA |
| SEQ ID NO: 14766 | UGCGGUC |
| SEQ ID NO: 14767 | UGCGGUG |
| SEQ ID NO: 14768 | UGCGGUU |
| SEQ ID NO: 14769 | UGCGUAA |
| SEQ ID NO: 14770 | UGCGUAC |
| SEQ ID NO: 14771 | UGCGUAG |
| SEQ ID NO: 14772 | UGCGUAU |
| SEQ ID NO: 14773 | UGCGUCA |
| SEQ ID NO: 14774 | UGCGUCC |
| SEQ ID NO: 14775 | UGCGUCG |
| SEQ ID NO: 14776 | UGCGUCU |
| SEQ ID NO: 14777 | UGCGUGA |
| SEQ ID NO: 14778 | UGCGUGC |
| SEQ ID NO: 14779 | UGCGUGG |
| SEQ ID NO: 14780 | UGCGUGU |
| SEQ ID NO: 14781 | UGCGUUA |
| SEQ ID NO: 14782 | UGCGUUC |
| SEQ ID NO: 14783 | UGCGUUG |
| SEQ ID NO: 14784 | UGCGUUU |
| SEQ ID NO: 14785 | UGCUAAA |
| SEQ ID NO: 14786 | UGCUAAC |
| SEQ ID NO: 14787 | UGCUAAG |
| SEQ ID NO: 14788 | UGCUAAU |
| SEQ ID NO: 14789 | UGCUACA |
| SEQ ID NO: 14790 | UGCUACC |
| SEQ ID NO: 14791 | UGCUACG |
| SEQ ID NO: 14792 | UGCUACU |
| SEQ ID NO: 14793 | UGCUAGA |
| SEQ ID NO: 14794 | UGCUAGC |
| SEQ ID NO: 14795 | UGCUAGG |
| SEQ ID NO: 14796 | UGCUAGU |
| SEQ ID NO: 14797 | UGCUAUA |
| SEQ ID NO: 14798 | UGCUAUC |
| SEQ ID NO: 14799 | UGCUAUG |
| SEQ ID NO: 14800 | UGCUAUU |
| SEQ ID NO: 14801 | UGCUCAA |
| SEQ ID NO: 14802 | UGCUCAC |
| SEQ ID NO: 14803 | UGCUCAG |
| SEQ ID NO: 14804 | UGCUCAU |
| SEQ ID NO: 14805 | UGCUCCA |
| SEQ ID NO: 14806 | UGCUCCC |
| SEQ ID NO: 14807 | UGCUCCG |
| SEQ ID NO: 14808 | UGCUCCU |
| SEQ ID NO: 14809 | UGCUCGA |
| SEQ ID NO: 14810 | UGCUCGC |
| SEQ ID NO: 14811 | UGCUCGG |
| SEQ ID NO: 14812 | UGCUCGU |
| SEQ ID NO: 14813 | UGCUCUA |
| SEQ ID NO: 14814 | UGCUCUC |
| SEQ ID NO: 14815 | UGCUCUG |
| SEQ ID NO: 14816 | UGCUCUU |
| SEQ ID NO: 14817 | UGCUGAA |
| SEQ ID NO: 14818 | UGCUGAC |
| SEQ ID NO: 14819 | UGCUGAG |
| SEQ ID NO: 14820 | UGCUGAU |
| SEQ ID NO: 14821 | UGCUGCA |
| SEQ ID NO: 14822 | UGCUGCC |
| SEQ ID NO: 14823 | UGCUGCG |
| SEQ ID NO: 14824 | UGCUGCU |
| SEQ ID NO: 14825 | UGCUGGA |
| SEQ ID NO: 14826 | UGCUGGC |
| SEQ ID NO: 14827 | UGCUGGG |
| SEQ ID NO: 14828 | UGCUGGU |
| SEQ ID NO: 14829 | UGCUGUA |
| SEQ ID NO: 14830 | UGCUGUC |
| SEQ ID NO: 14831 | UGCUGUG |
| SEQ ID NO: 14832 | UGCUGUU |
| SEQ ID NO: 14833 | UGCUUAA |
| SEQ ID NO: 14834 | UGCUUAC |
| SEQ ID NO: 14835 | UGCUUAG |
| SEQ ID NO: 14836 | UGCUUAU |
| SEQ ID NO: 14837 | UGCUUCA |
| SEQ ID NO: 14838 | UGCUUCC |
| SEQ ID NO: 14839 | UGCUUCG |
| SEQ ID NO: 14840 | UGCUUCU |
| SEQ ID NO: 14841 | UGCUUGA |
| SEQ ID NO: 14842 | UGCUUGC |
| SEQ ID NO: 14843 | UGCUUGG |
| SEQ ID NO: 14844 | UGCUUGU |
| SEQ ID NO: 14845 | UGCUUUA |
| SEQ ID NO: 14846 | UGCUUUC |
| SEQ ID NO: 14847 | UGCUUUG |
| SEQ ID NO: 14848 | UGCUUUU |
| SEQ ID NO: 14849 | UGGAAAA |
| SEQ ID NO: 14850 | UGGAAAC |
| SEQ ID NO: 14851 | UGGAAAG |
| SEQ ID NO: 14852 | UGGAAAU |
| SEQ ID NO: 14853 | UGGAACA |
| SEQ ID NO: 14854 | UGGAACC |
| SEQ ID NO: 14855 | UGGAACG |
| SEQ ID NO: 14856 | UGGAACU |
| SEQ ID NO: 14857 | UGGAAGA |
| SEQ ID NO: 14858 | UGGAAGC |
| SEQ ID NO: 14859 | UGGAAGG |
| SEQ ID NO: 14860 | UGGAAGU |
| SEQ ID NO: 14861 | UGGAAUA |
| SEQ ID NO: 14862 | UGGAAUC |
| SEQ ID NO: 14863 | UGGAAUG |
| SEQ ID NO: 14864 | UGGAAUU |
| SEQ ID NO: 14865 | UGGACAA |
| SEQ ID NO: 14866 | UGGACAC |
| SEQ ID NO: 14867 | UGGACAG |
| SEQ ID NO: 14868 | UGGACAU |
| SEQ ID NO: 14869 | UGGACCA |
| SEQ ID NO: 14870 | UGGACCC |
| SEQ ID NO: 14871 | UGGACCG |
| SEQ ID NO: 14872 | UGGACCU |
| SEQ ID NO: 14873 | UGGACGA |
| SEQ ID NO: 14874 | UGGACGC |
| SEQ ID NO: 14875 | UGGACGG |
| SEQ ID NO: 14876 | UGGACGU |
| SEQ ID NO: 14877 | UGGACUA |
| SEQ ID NO: 14878 | UGGACUC |
| SEQ ID NO: 14879 | UGGACUG |
| SEQ ID NO: 14880 | UGGACUU |
| SEQ ID NO: 14881 | UGGAGAA |
| SEQ ID NO: 14882 | UGGAGAC |
| SEQ ID NO: 14883 | UGGAGAG |
| SEQ ID NO: 14884 | UGGAGAU |
| SEQ ID NO: 14885 | UGGAGCA |
| SEQ ID NO: 14886 | UGGAGCC |
| SEQ ID NO: 14887 | UGGAGCG |
| SEQ ID NO: 14888 | UGGAGCU |
| SEQ ID NO: 14889 | UGGAGGA |
| SEQ ID NO: 14890 | UGGAGGC |
| SEQ ID NO: 14891 | UGGAGGG |
| SEQ ID NO: 14892 | UGGAGGU |
| SEQ ID NO: 14893 | UGGAGUA |
| SEQ ID NO: 14894 | UGGAGUC |
| SEQ ID NO: 14895 | UGGAGUG |
| SEQ ID NO: 14896 | UGGAGUU |
| SEQ ID NO: 14897 | UGGAUAA |
| SEQ ID NO: 14898 | UGGAUAC |
| SEQ ID NO: 14899 | UGGAUAG |
| SEQ ID NO: 14900 | UGGAUAU |
| SEQ ID NO: 14901 | UGGAUCA |
| SEQ ID NO: 14902 | UGGAUCC |
| SEQ ID NO: 14903 | UGGAUCG |
| SEQ ID NO: 14904 | UGGAUCU |
| SEQ ID NO: 14905 | UGGAUGA |
| SEQ ID NO: 14906 | UGGAUGC |
| SEQ ID NO: 14907 | UGGAUGG |
| SEQ ID NO: 14908 | UGGAUGU |
| SEQ ID NO: 14909 | UGGAUUA |
| SEQ ID NO: 14910 | UGGAUUC |
| SEQ ID NO: 14911 | UGGAUUG |
| SEQ ID NO: 14912 | UGGAUUU |
| SEQ ID NO: 14913 | UGGCAAA |
| SEQ ID NO: 14914 | UGGCAAC |
| SEQ ID NO: 14915 | UGGCAAG |
| SEQ ID NO: 14916 | UGGCAAU |
| SEQ ID NO: 14917 | UGGCACA |
| SEQ ID NO: 14918 | UGGCACC |
| SEQ ID NO: 14919 | UGGCACG |
| SEQ ID NO: 14920 | UGGCACU |
| SEQ ID NO: 14921 | UGGCAGA |
| SEQ ID NO: 14922 | UGGCAGC |
| SEQ ID NO: 14923 | UGGCAGG |
| SEQ ID NO: 14924 | UGGCAGU |
| SEQ ID NO: 14925 | UGGCAUA |
| SEQ ID NO: 14926 | UGGCAUC |
| SEQ ID NO: 14927 | UGGCAUG |
| SEQ ID NO: 14928 | UGGCAUU |
| SEQ ID NO: 14929 | UGGCCAA |
| SEQ ID NO: 14930 | UGGCCAC |
| SEQ ID NO: 14931 | UGGCCAG |
| SEQ ID NO: 14932 | UGGCCAU |
| SEQ ID NO: 14933 | UGGCCCA |
| SEQ ID NO: 14934 | UGGCCCC |
| SEQ ID NO: 14935 | UGGCCCG |
| SEQ ID NO: 14936 | UGGCCCU |
| SEQ ID NO: 14937 | UGGCCGA |
| SEQ ID NO: 14938 | UGGCCGC |
| SEQ ID NO: 14939 | UGGCCGG |
| SEQ ID NO: 14940 | UGGCCGU |
| SEQ ID NO: 14941 | UGGCCUA |
| SEQ ID NO: 14942 | UGGCCUC |
| SEQ ID NO: 14943 | UGGCCUG |
| SEQ ID NO: 14944 | UGGCCUU |
| SEQ ID NO: 14945 | UGGCGAA |
| SEQ ID NO: 14946 | UGGCGAC |
| SEQ ID NO: 14947 | UGGCGAG |
| SEQ ID NO: 14948 | UGGCGAU |
| SEQ ID NO: 14949 | UGGCGCA |
| SEQ ID NO: 14950 | UGGCGCC |
| SEQ ID NO: 14951 | UGGCGCG |
| SEQ ID NO: 14952 | UGGCGCU |
| SEQ ID NO: 14953 | UGGCGGA |
| SEQ ID NO: 14954 | UGGCGGC |
| SEQ ID NO: 14955 | UGGCGGG |
| SEQ ID NO: 14956 | UGGCGGU |
| SEQ ID NO: 14957 | UGGCGUA |
| SEQ ID NO: 14958 | UGGCGUC |
| SEQ ID NO: 14959 | UGGCGUG |
| SEQ ID NO: 14960 | UGGCGUU |
| SEQ ID NO: 14961 | UGGCUAA |
| SEQ ID NO: 14962 | UGGCUAC |
| SEQ ID NO: 14963 | UGGCUAG |
| SEQ ID NO: 14964 | UGGCUAU |
| SEQ ID NO: 14965 | UGGCUCA |
| SEQ ID NO: 14966 | UGGCUCC |
| SEQ ID NO: 14967 | UGGCUCG |
| SEQ ID NO: 14968 | UGGCUCU |
| SEQ ID NO: 14969 | UGGCUGA |
| SEQ ID NO: 14970 | UGGCUGC |
| SEQ ID NO: 14971 | UGGCUGG |
| SEQ ID NO: 14972 | UGGCUGU |
| SEQ ID NO: 14973 | UGGCUUA |
| SEQ ID NO: 14974 | UGGCUUC |
| SEQ ID NO: 14975 | UGGCUUG |
| SEQ ID NO: 14976 | UGGCUUU |
| SEQ ID NO: 14977 | UGGGAAA |
| SEQ ID NO: 14978 | UGGGAAC |
| SEQ ID NO: 14979 | UGGGAAG |
| SEQ ID NO: 14980 | UGGGAAU |
| SEQ ID NO: 14981 | UGGGACA |
| SEQ ID NO: 14982 | UGGGACC |
| SEQ ID NO: 14983 | UGGGACG |
| SEQ ID NO: 14984 | UGGGACU |
| SEQ ID NO: 14985 | UGGGAGA |
| SEQ ID NO: 14986 | UGGGAGC |
| SEQ ID NO: 14987 | UGGGAGG |
| SEQ ID NO: 14988 | UGGGAGU |
| SEQ ID NO: 14989 | UGGGAUA |
| SEQ ID NO: 14990 | UGGGAUC |
| SEQ ID NO: 14991 | UGGGAUG |
| SEQ ID NO: 14992 | UGGGAUU |
| SEQ ID NO: 14993 | UGGGCAA |
| SEQ ID NO: 14994 | UGGGCAC |

Table 50

| | | | | | | | | | | | |
|---|---|---|---|---|---|---|---|---|---|---|---|
| SEQ ID NO: 14995 | UGGGCAG | SEQ ID NO: 15046 | UGGUACC | SEQ ID NO: 15097 | UGGUUGA | SEQ ID NO: 15148 | UGUAGGU | SEQ ID NO: 15199 | UGUCCUG | SEQ ID NO: 15250 | UGUGCAC |
| SEQ ID NO: 14996 | UGGGCAU | SEQ ID NO: 15047 | UGGUACG | SEQ ID NO: 15098 | UGGUUGC | SEQ ID NO: 15149 | UGUAGUA | SEQ ID NO: 15200 | UGUCCUU | SEQ ID NO: 15251 | UGUGCAG |
| SEQ ID NO: 14997 | UGGGCCA | SEQ ID NO: 15048 | UGGUACU | SEQ ID NO: 15099 | UGGUUGG | SEQ ID NO: 15150 | UGUAGUC | SEQ ID NO: 15201 | UGUCGAA | SEQ ID NO: 15252 | UGUGCAU |
| SEQ ID NO: 14998 | UGGGCCC | SEQ ID NO: 15049 | UGGUAGA | SEQ ID NO: 15100 | UGGUUGU | SEQ ID NO: 15151 | UGUAGUG | SEQ ID NO: 15202 | UGUCGAC | SEQ ID NO: 15253 | UGUGCCA |
| SEQ ID NO: 14999 | UGGGCCG | SEQ ID NO: 15050 | UGGUAGC | SEQ ID NO: 15101 | UGGUUUA | SEQ ID NO: 15152 | UGUAGUU | SEQ ID NO: 15203 | UGUCGAG | SEQ ID NO: 15254 | UGUGCCC |
| SEQ ID NO: 15000 | UGGGCCU | SEQ ID NO: 15051 | UGGUAGG | SEQ ID NO: 15102 | UGGUUUC | SEQ ID NO: 15153 | UGUAUAA | SEQ ID NO: 15204 | UGUCGAU | SEQ ID NO: 15255 | UGUGCCG |
| SEQ ID NO: 15001 | UGGGCGA | SEQ ID NO: 15052 | UGGUAGU | SEQ ID NO: 15103 | UGGUUUG | SEQ ID NO: 15154 | UGUAUAC | SEQ ID NO: 15205 | UGUCGCA | SEQ ID NO: 15256 | UGUGCCU |
| SEQ ID NO: 15002 | UGGGCGC | SEQ ID NO: 15053 | UGGUAUA | SEQ ID NO: 15104 | UGGUUUU | SEQ ID NO: 15155 | UGUAUAG | SEQ ID NO: 15206 | UGUCGCC | SEQ ID NO: 15257 | UGUGCGA |
| SEQ ID NO: 15003 | UGGGCGG | SEQ ID NO: 15054 | UGGUAUC | SEQ ID NO: 15105 | UGUAAAA | SEQ ID NO: 15156 | UGUAUAU | SEQ ID NO: 15207 | UGUCGCG | SEQ ID NO: 15258 | UGUGCGC |
| SEQ ID NO: 15004 | UGGGCGU | SEQ ID NO: 15055 | UGGUAUG | SEQ ID NO: 15106 | UGUAAAC | SEQ ID NO: 15157 | UGUAUCA | SEQ ID NO: 15208 | UGUCGCU | SEQ ID NO: 15259 | UGUGCGG |
| SEQ ID NO: 15005 | UGGGCUA | SEQ ID NO: 15056 | UGGUAUU | SEQ ID NO: 15107 | UGUAAAG | SEQ ID NO: 15158 | UGUAUCC | SEQ ID NO: 15209 | UGUCGGA | SEQ ID NO: 15260 | UGUGCGU |
| SEQ ID NO: 15006 | UGGGCUC | SEQ ID NO: 15057 | UGGUCAA | SEQ ID NO: 15108 | UGUAAAU | SEQ ID NO: 15159 | UGUAUCG | SEQ ID NO: 15210 | UGUCGGC | SEQ ID NO: 15261 | UGUGCUA |
| SEQ ID NO: 15007 | UGGGCUG | SEQ ID NO: 15058 | UGGUCAC | SEQ ID NO: 15109 | UGUAACA | SEQ ID NO: 15160 | UGUAUCU | SEQ ID NO: 15211 | UGUCGGG | SEQ ID NO: 15262 | UGUGCUC |
| SEQ ID NO: 15008 | UGGGCUU | SEQ ID NO: 15059 | UGGUCAG | SEQ ID NO: 15110 | UGUAACC | SEQ ID NO: 15161 | UGUAUGA | SEQ ID NO: 15212 | UGUCGGU | SEQ ID NO: 15263 | UGUGCUG |
| SEQ ID NO: 15009 | UGGGGAA | SEQ ID NO: 15060 | UGGUCAU | SEQ ID NO: 15111 | UGUAACG | SEQ ID NO: 15162 | UGUAUGC | SEQ ID NO: 15213 | UGUCGUA | SEQ ID NO: 15264 | UGUGCUU |
| SEQ ID NO: 15010 | UGGGGAC | SEQ ID NO: 15061 | UGGUCCA | SEQ ID NO: 15112 | UGUAACU | SEQ ID NO: 15163 | UGUAUGG | SEQ ID NO: 15214 | UGUCGUC | SEQ ID NO: 15265 | UGUGGAA |
| SEQ ID NO: 15011 | UGGGGAG | SEQ ID NO: 15062 | UGGUCCC | SEQ ID NO: 15113 | UGUAAGA | SEQ ID NO: 15164 | UGUAUGU | SEQ ID NO: 15215 | UGUCGUG | SEQ ID NO: 15266 | UGUGGAC |
| SEQ ID NO: 15012 | UGGGGAU | SEQ ID NO: 15063 | UGGUCCG | SEQ ID NO: 15114 | UGUAAGC | SEQ ID NO: 15165 | UGUAUUA | SEQ ID NO: 15216 | UGUCGUU | SEQ ID NO: 15267 | UGUGGAG |
| SEQ ID NO: 15013 | UGGGGCA | SEQ ID NO: 15064 | UGGUCCU | SEQ ID NO: 15115 | UGUAAGG | SEQ ID NO: 15166 | UGUAUUC | SEQ ID NO: 15217 | UGUCUAA | SEQ ID NO: 15268 | UGUGGAU |
| SEQ ID NO: 15014 | UGGGGCC | SEQ ID NO: 15065 | UGGUCGA | SEQ ID NO: 15116 | UGUAAGU | SEQ ID NO: 15167 | UGUAUUG | SEQ ID NO: 15218 | UGUCUAC | SEQ ID NO: 15269 | UGUGGCA |
| SEQ ID NO: 15015 | UGGGGCG | SEQ ID NO: 15066 | UGGUCGC | SEQ ID NO: 15117 | UGUAAUA | SEQ ID NO: 15168 | UGUAUUU | SEQ ID NO: 15219 | UGUCUAG | SEQ ID NO: 15270 | UGUGGCC |
| SEQ ID NO: 15016 | UGGGGCU | SEQ ID NO: 15067 | UGGUCGG | SEQ ID NO: 15118 | UGUAAUC | SEQ ID NO: 15169 | UGUCAAA | SEQ ID NO: 15220 | UGUCUAU | SEQ ID NO: 15271 | UGUGGCG |
| SEQ ID NO: 15017 | UGGGGGA | SEQ ID NO: 15068 | UGGUCGU | SEQ ID NO: 15119 | UGUAAUG | SEQ ID NO: 15170 | UGUCAAC | SEQ ID NO: 15221 | UGUCUCA | SEQ ID NO: 15272 | UGUGGCU |
| SEQ ID NO: 15018 | UGGGGGC | SEQ ID NO: 15069 | UGGUCUA | SEQ ID NO: 15120 | UGUAAUU | SEQ ID NO: 15171 | UGUCAAG | SEQ ID NO: 15222 | UGUCUCC | SEQ ID NO: 15273 | UGUGGGA |
| SEQ ID NO: 15019 | UGGGGGG | SEQ ID NO: 15070 | UGGUCUC | SEQ ID NO: 15121 | UGUACAA | SEQ ID NO: 15172 | UGUCAAU | SEQ ID NO: 15223 | UGUCUCG | SEQ ID NO: 15274 | UGUGGGC |
| SEQ ID NO: 15020 | UGGGGGU | SEQ ID NO: 15071 | UGGUCUG | SEQ ID NO: 15122 | UGUACAC | SEQ ID NO: 15173 | UGUCACA | SEQ ID NO: 15224 | UGUCUCU | SEQ ID NO: 15275 | UGUGGGG |
| SEQ ID NO: 15021 | UGGGGUA | SEQ ID NO: 15072 | UGGUCUU | SEQ ID NO: 15123 | UGUACAG | SEQ ID NO: 15174 | UGUCACC | SEQ ID NO: 15225 | UGUCUGA | SEQ ID NO: 15276 | UGUGGGU |
| SEQ ID NO: 15022 | UGGGGUC | SEQ ID NO: 15073 | UGGUGAA | SEQ ID NO: 15124 | UGUACAU | SEQ ID NO: 15175 | UGUCACG | SEQ ID NO: 15226 | UGUCUGC | SEQ ID NO: 15277 | UGUGGUA |
| SEQ ID NO: 15023 | UGGGGUG | SEQ ID NO: 15074 | UGGUGAC | SEQ ID NO: 15125 | UGUACCA | SEQ ID NO: 15176 | UGUCACU | SEQ ID NO: 15227 | UGUCUGG | SEQ ID NO: 15278 | UGUGGUC |
| SEQ ID NO: 15024 | UGGGGUU | SEQ ID NO: 15075 | UGGUGAG | SEQ ID NO: 15126 | UGUACCC | SEQ ID NO: 15177 | UGUCAGA | SEQ ID NO: 15228 | UGUCUGU | SEQ ID NO: 15279 | UGUGGUG |
| SEQ ID NO: 15025 | UGGGUAA | SEQ ID NO: 15076 | UGGUGAU | SEQ ID NO: 15127 | UGUACCG | SEQ ID NO: 15178 | UGUCAGC | SEQ ID NO: 15229 | UGUCUUA | SEQ ID NO: 15280 | UGUGGUU |
| SEQ ID NO: 15026 | UGGGUAC | SEQ ID NO: 15077 | UGGUGCA | SEQ ID NO: 15128 | UGUACCU | SEQ ID NO: 15179 | UGUCAGG | SEQ ID NO: 15230 | UGUCUUC | SEQ ID NO: 15281 | UGUGUAA |
| SEQ ID NO: 15027 | UGGGUAG | SEQ ID NO: 15078 | UGGUGCC | SEQ ID NO: 15129 | UGUACGA | SEQ ID NO: 15180 | UGUCAGU | SEQ ID NO: 15231 | UGUCUUG | SEQ ID NO: 15282 | UGUGUAC |
| SEQ ID NO: 15028 | UGGGUAU | SEQ ID NO: 15079 | UGGUGCG | SEQ ID NO: 15130 | UGUACGC | SEQ ID NO: 15181 | UGUCAUA | SEQ ID NO: 15232 | UGUCUUU | SEQ ID NO: 15283 | UGUGUAG |
| SEQ ID NO: 15029 | UGGGUCA | SEQ ID NO: 15080 | UGGUGCU | SEQ ID NO: 15131 | UGUACGG | SEQ ID NO: 15182 | UGUCAUC | SEQ ID NO: 15233 | UGUGAAA | SEQ ID NO: 15284 | UGUGUAU |
| SEQ ID NO: 15030 | UGGGUCC | SEQ ID NO: 15081 | UGGUGGA | SEQ ID NO: 15132 | UGUACGU | SEQ ID NO: 15183 | UGUCAUG | SEQ ID NO: 15234 | UGUGAAC | SEQ ID NO: 15285 | UGUGUCA |
| SEQ ID NO: 15031 | UGGGUCG | SEQ ID NO: 15082 | UGGUGGC | SEQ ID NO: 15133 | UGUACUA | SEQ ID NO: 15184 | UGUCAUU | SEQ ID NO: 15235 | UGUGAAG | SEQ ID NO: 15286 | UGUGUCC |
| SEQ ID NO: 15032 | UGGGUCU | SEQ ID NO: 15083 | UGGUGGG | SEQ ID NO: 15134 | UGUACUC | SEQ ID NO: 15185 | UGUCCAA | SEQ ID NO: 15236 | UGUGAAU | SEQ ID NO: 15287 | UGUGUCG |
| SEQ ID NO: 15033 | UGGGUGA | SEQ ID NO: 15084 | UGGUGGU | SEQ ID NO: 15135 | UGUACUG | SEQ ID NO: 15186 | UGUCCAC | SEQ ID NO: 15237 | UGUGACA | SEQ ID NO: 15288 | UGUGUCU |
| SEQ ID NO: 15034 | UGGGUGC | SEQ ID NO: 15085 | UGGUGUA | SEQ ID NO: 15136 | UGUACUU | SEQ ID NO: 15187 | UGUCCAG | SEQ ID NO: 15238 | UGUGACC | SEQ ID NO: 15289 | UGUGUGA |
| SEQ ID NO: 15035 | UGGGUGG | SEQ ID NO: 15086 | UGGUGUC | SEQ ID NO: 15137 | UGUAGAA | SEQ ID NO: 15188 | UGUCCAU | SEQ ID NO: 15239 | UGUGACG | SEQ ID NO: 15290 | UGUGUGC |
| SEQ ID NO: 15036 | UGGGUGU | SEQ ID NO: 15087 | UGGUGUG | SEQ ID NO: 15138 | UGUAGAC | SEQ ID NO: 15189 | UGUCCCA | SEQ ID NO: 15240 | UGUGACU | SEQ ID NO: 15291 | UGUGUGG |
| SEQ ID NO: 15037 | UGGGUUA | SEQ ID NO: 15088 | UGGUGUU | SEQ ID NO: 15139 | UGUAGAG | SEQ ID NO: 15190 | UGUCCCC | SEQ ID NO: 15241 | UGUGAGA | SEQ ID NO: 15292 | UGUGUGU |
| SEQ ID NO: 15038 | UGGGUUC | SEQ ID NO: 15089 | UGGUUAA | SEQ ID NO: 15140 | UGUAGAU | SEQ ID NO: 15191 | UGUCCCG | SEQ ID NO: 15242 | UGUGAGC | SEQ ID NO: 15293 | UGUGUUA |
| SEQ ID NO: 15039 | UGGGUUG | SEQ ID NO: 15090 | UGGUUAC | SEQ ID NO: 15141 | UGUAGCA | SEQ ID NO: 15192 | UGUCCCU | SEQ ID NO: 15243 | UGUGAGG | SEQ ID NO: 15294 | UGUGUUC |
| SEQ ID NO: 15040 | UGGGUUU | SEQ ID NO: 15091 | UGGUUAG | SEQ ID NO: 15142 | UGUAGCC | SEQ ID NO: 15193 | UGUCCGA | SEQ ID NO: 15244 | UGUGAGU | SEQ ID NO: 15295 | UGUGUUG |
| SEQ ID NO: 15041 | UGGUAAA | SEQ ID NO: 15092 | UGGUUAU | SEQ ID NO: 15143 | UGUAGCG | SEQ ID NO: 15194 | UGUCCGC | SEQ ID NO: 15245 | UGUGAUA | SEQ ID NO: 15296 | UGUGUUU |
| SEQ ID NO: 15042 | UGGUAAC | SEQ ID NO: 15093 | UGGUUCA | SEQ ID NO: 15144 | UGUAGCU | SEQ ID NO: 15195 | UGUCCGG | SEQ ID NO: 15246 | UGUGAUC | SEQ ID NO: 15297 | UGUUAAA |
| SEQ ID NO: 15043 | UGGUAAG | SEQ ID NO: 15094 | UGGUUCC | SEQ ID NO: 15145 | UGUAGGA | SEQ ID NO: 15196 | UGUCCGU | SEQ ID NO: 15247 | UGUGAUG | SEQ ID NO: 15298 | UGUUAAC |
| SEQ ID NO: 15044 | UGGUAAU | SEQ ID NO: 15095 | UGGUUCG | SEQ ID NO: 15146 | UGUAGGC | SEQ ID NO: 15197 | UGUCCUA | SEQ ID NO: 15248 | UGUGAUU | SEQ ID NO: 15299 | UGUUAAG |
| SEQ ID NO: 15045 | UGGUACA | SEQ ID NO: 15096 | UGGUUCU | SEQ ID NO: 15147 | UGUAGGG | SEQ ID NO: 15198 | UGUCCUC | SEQ ID NO: 15249 | UGUGCAA | SEQ ID NO: 15300 | UGUUAAU |

Table 51

| | | | | | | | | | | | |
|---|---|---|---|---|---|---|---|---|---|---|---|
| SEQ ID NO: 15301 | UGUUACA | SEQ ID NO: 15352 | UGUUUCU | SEQ ID NO: 15403 | UUAAGGG | SEQ ID NO: 15454 | UUACCUC | SEQ ID NO: 15505 | UUAGCAA | SEQ ID NO: 15556 | UUAUAAU |
| SEQ ID NO: 15302 | UGUUACC | SEQ ID NO: 15353 | UGUUUGA | SEQ ID NO: 15404 | UUAAGGU | SEQ ID NO: 15455 | UUACCUG | SEQ ID NO: 15506 | UUAGCAC | SEQ ID NO: 15557 | UUAUACA |
| SEQ ID NO: 15303 | UGUUACG | SEQ ID NO: 15354 | UGUUUGC | SEQ ID NO: 15405 | UUAAGUA | SEQ ID NO: 15456 | UUACCUU | SEQ ID NO: 15507 | UUAGCAG | SEQ ID NO: 15558 | UUAUACC |
| SEQ ID NO: 15304 | UGUUACU | SEQ ID NO: 15355 | UGUUUGG | SEQ ID NO: 15406 | UUAAGUC | SEQ ID NO: 15457 | UUACGAA | SEQ ID NO: 15508 | UUAGCAU | SEQ ID NO: 15559 | UUAUACG |
| SEQ ID NO: 15305 | UGUUAGA | SEQ ID NO: 15356 | UGUUUGU | SEQ ID NO: 15407 | UUAAGUG | SEQ ID NO: 15458 | UUACGAC | SEQ ID NO: 15509 | UUAGCCA | SEQ ID NO: 15560 | UUAUACU |
| SEQ ID NO: 15306 | UGUUAGC | SEQ ID NO: 15357 | UGUUUUA | SEQ ID NO: 15408 | UUAAGUU | SEQ ID NO: 15459 | UUACGAG | SEQ ID NO: 15510 | UUAGCCC | SEQ ID NO: 15561 | UUAUAGA |
| SEQ ID NO: 15307 | UGUUAGG | SEQ ID NO: 15358 | UGUUUUC | SEQ ID NO: 15409 | UUAAUAA | SEQ ID NO: 15460 | UUACGAU | SEQ ID NO: 15511 | UUAGCCG | SEQ ID NO: 15562 | UUAUAGC |
| SEQ ID NO: 15308 | UGUUAGU | SEQ ID NO: 15359 | UGUUUUG | SEQ ID NO: 15410 | UUAAUAC | SEQ ID NO: 15461 | UUACGCA | SEQ ID NO: 15512 | UUAGCCU | SEQ ID NO: 15563 | UUAUAGG |
| SEQ ID NO: 15309 | UGUUAUA | SEQ ID NO: 15360 | UGUUUUU | SEQ ID NO: 15411 | UUAAUAG | SEQ ID NO: 15462 | UUACGCC | SEQ ID NO: 15513 | UUAGCGA | SEQ ID NO: 15564 | UUAUAGU |
| SEQ ID NO: 15310 | UGUUAUC | SEQ ID NO: 15361 | UUAAAAA | SEQ ID NO: 15412 | UUAAUAU | SEQ ID NO: 15463 | UUACGCG | SEQ ID NO: 15514 | UUAGCGC | SEQ ID NO: 15565 | UUAUAUA |
| SEQ ID NO: 15311 | UGUUAUG | SEQ ID NO: 15362 | UUAAAAC | SEQ ID NO: 15413 | UUAAUCA | SEQ ID NO: 15464 | UUACGCU | SEQ ID NO: 15515 | UUAGCGG | SEQ ID NO: 15566 | UUAUAUC |
| SEQ ID NO: 15312 | UGUUAUU | SEQ ID NO: 15363 | UUAAAAG | SEQ ID NO: 15414 | UUAAUCC | SEQ ID NO: 15465 | UUACGGA | SEQ ID NO: 15516 | UUAGCGU | SEQ ID NO: 15567 | UUAUAUG |
| SEQ ID NO: 15313 | UGUUCAA | SEQ ID NO: 15364 | UUAAAAU | SEQ ID NO: 15415 | UUAAUCG | SEQ ID NO: 15466 | UUACGGC | SEQ ID NO: 15517 | UUAGCUA | SEQ ID NO: 15568 | UUAUAUU |
| SEQ ID NO: 15314 | UGUUCAC | SEQ ID NO: 15365 | UUAAACA | SEQ ID NO: 15416 | UUAAUCU | SEQ ID NO: 15467 | UUACGGG | SEQ ID NO: 15518 | UUAGCUC | SEQ ID NO: 15569 | UUAUCAA |
| SEQ ID NO: 15315 | UGUUCAG | SEQ ID NO: 15366 | UUAAACC | SEQ ID NO: 15417 | UUAAUGA | SEQ ID NO: 15468 | UUACGGU | SEQ ID NO: 15519 | UUAGCUG | SEQ ID NO: 15570 | UUAUCAC |
| SEQ ID NO: 15316 | UGUUCAU | SEQ ID NO: 15367 | UUAAACG | SEQ ID NO: 15418 | UUAAUGC | SEQ ID NO: 15469 | UUACGUA | SEQ ID NO: 15520 | UUAGCUU | SEQ ID NO: 15571 | UUAUCAG |
| SEQ ID NO: 15317 | UGUUCCA | SEQ ID NO: 15368 | UUAAACU | SEQ ID NO: 15419 | UUAAUGG | SEQ ID NO: 15470 | UUACGUC | SEQ ID NO: 15521 | UUAGGAA | SEQ ID NO: 15572 | UUAUCAU |
| SEQ ID NO: 15318 | UGUUCCC | SEQ ID NO: 15369 | UUAAAGA | SEQ ID NO: 15420 | UUAAUGU | SEQ ID NO: 15471 | UUACGUG | SEQ ID NO: 15522 | UUAGGAC | SEQ ID NO: 15573 | UUAUCCA |
| SEQ ID NO: 15319 | UGUUCCG | SEQ ID NO: 15370 | UUAAAGC | SEQ ID NO: 15421 | UUAAUUA | SEQ ID NO: 15472 | UUACGUU | SEQ ID NO: 15523 | UUAGGAG | SEQ ID NO: 15574 | UUAUCCC |
| SEQ ID NO: 15320 | UGUUCCU | SEQ ID NO: 15371 | UUAAAGG | SEQ ID NO: 15422 | UUAAUUC | SEQ ID NO: 15473 | UUACUAA | SEQ ID NO: 15524 | UUAGGAU | SEQ ID NO: 15575 | UUAUCCG |
| SEQ ID NO: 15321 | UGUUCGA | SEQ ID NO: 15372 | UUAAAGU | SEQ ID NO: 15423 | UUAAUUG | SEQ ID NO: 15474 | UUACUAC | SEQ ID NO: 15525 | UUAGGCA | SEQ ID NO: 15576 | UUAUCCU |
| SEQ ID NO: 15322 | UGUUCGC | SEQ ID NO: 15373 | UUAAAUA | SEQ ID NO: 15424 | UUAAUUU | SEQ ID NO: 15475 | UUACUAG | SEQ ID NO: 15526 | UUAGGCC | SEQ ID NO: 15577 | UUAUCGA |
| SEQ ID NO: 15323 | UGUUCGG | SEQ ID NO: 15374 | UUAAAUC | SEQ ID NO: 15425 | UUACAAA | SEQ ID NO: 15476 | UUACUAU | SEQ ID NO: 15527 | UUAGGCG | SEQ ID NO: 15578 | UUAUCGC |
| SEQ ID NO: 15324 | UGUUCGU | SEQ ID NO: 15375 | UUAAAUG | SEQ ID NO: 15426 | UUACAAC | SEQ ID NO: 15477 | UUACUCA | SEQ ID NO: 15528 | UUAGGCU | SEQ ID NO: 15579 | UUAUCGG |
| SEQ ID NO: 15325 | UGUUCUA | SEQ ID NO: 15376 | UUAAAUU | SEQ ID NO: 15427 | UUACAAG | SEQ ID NO: 15478 | UUACUCC | SEQ ID NO: 15529 | UUAGGGA | SEQ ID NO: 15580 | UUAUCGU |
| SEQ ID NO: 15326 | UGUUCUC | SEQ ID NO: 15377 | UUAACAA | SEQ ID NO: 15428 | UUACAAU | SEQ ID NO: 15479 | UUACUCG | SEQ ID NO: 15530 | UUAGGGC | SEQ ID NO: 15581 | UUAUCUA |
| SEQ ID NO: 15327 | UGUUCUG | SEQ ID NO: 15378 | UUAACAC | SEQ ID NO: 15429 | UUACACA | SEQ ID NO: 15480 | UUACUCU | SEQ ID NO: 15531 | UUAGGGG | SEQ ID NO: 15582 | UUAUCUC |
| SEQ ID NO: 15328 | UGUUCUU | SEQ ID NO: 15379 | UUAACAG | SEQ ID NO: 15430 | UUACACC | SEQ ID NO: 15481 | UUACUGA | SEQ ID NO: 15532 | UUAGGGU | SEQ ID NO: 15583 | UUAUCUG |
| SEQ ID NO: 15329 | UGUUGAA | SEQ ID NO: 15380 | UUAACAU | SEQ ID NO: 15431 | UUACACG | SEQ ID NO: 15482 | UUACUGC | SEQ ID NO: 15533 | UUAGGUA | SEQ ID NO: 15584 | UUAUCUU |
| SEQ ID NO: 15330 | UGUUGAC | SEQ ID NO: 15381 | UUAACCA | SEQ ID NO: 15432 | UUACACU | SEQ ID NO: 15483 | UUACUGG | SEQ ID NO: 15534 | UUAGGUC | SEQ ID NO: 15585 | UUAUGAA |
| SEQ ID NO: 15331 | UGUUGAG | SEQ ID NO: 15382 | UUAACCC | SEQ ID NO: 15433 | UUACAGA | SEQ ID NO: 15484 | UUACUGU | SEQ ID NO: 15535 | UUAGGUG | SEQ ID NO: 15586 | UUAUGAC |
| SEQ ID NO: 15332 | UGUUGAU | SEQ ID NO: 15383 | UUAACCG | SEQ ID NO: 15434 | UUACAGC | SEQ ID NO: 15485 | UUACUUA | SEQ ID NO: 15536 | UUAGGUU | SEQ ID NO: 15587 | UUAUGAG |
| SEQ ID NO: 15333 | UGUUGCA | SEQ ID NO: 15384 | UUAACCU | SEQ ID NO: 15435 | UUACAGG | SEQ ID NO: 15486 | UUACUUC | SEQ ID NO: 15537 | UUAGUAA | SEQ ID NO: 15588 | UUAUGAU |
| SEQ ID NO: 15334 | UGUUGCC | SEQ ID NO: 15385 | UUAACGA | SEQ ID NO: 15436 | UUACAGU | SEQ ID NO: 15487 | UUACUUG | SEQ ID NO: 15538 | UUAGUAC | SEQ ID NO: 15589 | UUAUGCA |
| SEQ ID NO: 15335 | UGUUGCG | SEQ ID NO: 15386 | UUAACGC | SEQ ID NO: 15437 | UUACAUA | SEQ ID NO: 15488 | UUACUUU | SEQ ID NO: 15539 | UUAGUAG | SEQ ID NO: 15590 | UUAUGCC |
| SEQ ID NO: 15336 | UGUUGCU | SEQ ID NO: 15387 | UUAACGG | SEQ ID NO: 15438 | UUACAUC | SEQ ID NO: 15489 | UUAGAAA | SEQ ID NO: 15540 | UUAGUAU | SEQ ID NO: 15591 | UUAUGCG |
| SEQ ID NO: 15337 | UGUUGGA | SEQ ID NO: 15388 | UUAACGU | SEQ ID NO: 15439 | UUACAUG | SEQ ID NO: 15490 | UUAGAAC | SEQ ID NO: 15541 | UUAGUCA | SEQ ID NO: 15592 | UUAUGCU |
| SEQ ID NO: 15338 | UGUUGGC | SEQ ID NO: 15389 | UUAACUA | SEQ ID NO: 15440 | UUACAUU | SEQ ID NO: 15491 | UUAGAAG | SEQ ID NO: 15542 | UUAGUCC | SEQ ID NO: 15593 | UUAUGGA |
| SEQ ID NO: 15339 | UGUUGGG | SEQ ID NO: 15390 | UUAACUC | SEQ ID NO: 15441 | UUACCAA | SEQ ID NO: 15492 | UUAGAAU | SEQ ID NO: 15543 | UUAGUCG | SEQ ID NO: 15594 | UUAUGGC |
| SEQ ID NO: 15340 | UGUUGGU | SEQ ID NO: 15391 | UUAACUG | SEQ ID NO: 15442 | UUACCAC | SEQ ID NO: 15493 | UUAGACA | SEQ ID NO: 15544 | UUAGUCU | SEQ ID NO: 15595 | UUAUGGG |
| SEQ ID NO: 15341 | UGUUGUA | SEQ ID NO: 15392 | UUAACUU | SEQ ID NO: 15443 | UUACCAG | SEQ ID NO: 15494 | UUAGACC | SEQ ID NO: 15545 | UUAGUGA | SEQ ID NO: 15596 | UUAUGGU |
| SEQ ID NO: 15342 | UGUUGUC | SEQ ID NO: 15393 | UUAAGAA | SEQ ID NO: 15444 | UUACCAU | SEQ ID NO: 15495 | UUAGACG | SEQ ID NO: 15546 | UUAGUGC | SEQ ID NO: 15597 | UUAUGUA |
| SEQ ID NO: 15343 | UGUUGUG | SEQ ID NO: 15394 | UUAAGAC | SEQ ID NO: 15445 | UUACCCA | SEQ ID NO: 15496 | UUAGACU | SEQ ID NO: 15547 | UUAGUGG | SEQ ID NO: 15598 | UUAUGUC |
| SEQ ID NO: 15344 | UGUUGUU | SEQ ID NO: 15395 | UUAAGAG | SEQ ID NO: 15446 | UUACCCC | SEQ ID NO: 15497 | UUAGAGA | SEQ ID NO: 15548 | UUAGUGU | SEQ ID NO: 15599 | UUAUGUG |
| SEQ ID NO: 15345 | UGUUUAA | SEQ ID NO: 15396 | UUAAGAU | SEQ ID NO: 15447 | UUACCCG | SEQ ID NO: 15498 | UUAGAGC | SEQ ID NO: 15549 | UUAGUUA | SEQ ID NO: 15600 | UUAUGUU |
| SEQ ID NO: 15346 | UGUUUAC | SEQ ID NO: 15397 | UUAAGCA | SEQ ID NO: 15448 | UUACCCU | SEQ ID NO: 15499 | UUAGAGG | SEQ ID NO: 15550 | UUAGUUC | SEQ ID NO: 15601 | UUAUUAA |
| SEQ ID NO: 15347 | UGUUUAG | SEQ ID NO: 15398 | UUAAGCC | SEQ ID NO: 15449 | UUACCGA | SEQ ID NO: 15500 | UUAGAGU | SEQ ID NO: 15551 | UUAGUUG | SEQ ID NO: 15602 | UUAUUAC |
| SEQ ID NO: 15348 | UGUUUAU | SEQ ID NO: 15399 | UUAAGCG | SEQ ID NO: 15450 | UUACCGC | SEQ ID NO: 15501 | UUAGAUA | SEQ ID NO: 15552 | UUAGUUU | SEQ ID NO: 15603 | UUAUUAG |
| SEQ ID NO: 15349 | UGUUUCA | SEQ ID NO: 15400 | UUAAGCU | SEQ ID NO: 15451 | UUACCGG | SEQ ID NO: 15502 | UUAGAUC | SEQ ID NO: 15553 | UUAUAAA | SEQ ID NO: 15604 | UUAUUAU |
| SEQ ID NO: 15350 | UGUUUCC | SEQ ID NO: 15401 | UUAAGGA | SEQ ID NO: 15452 | UUACCGU | SEQ ID NO: 15503 | UUAGAUG | SEQ ID NO: 15554 | UUAUAAC | SEQ ID NO: 15605 | UUAUUCA |
| SEQ ID NO: 15351 | UGUUUCG | SEQ ID NO: 15402 | UUAAGGC | SEQ ID NO: 15453 | UUACCUA | SEQ ID NO: 15504 | UUAGAUU | SEQ ID NO: 15555 | UUAUAAG | SEQ ID NO: 15606 | UUAUUCC |

Table 52

| | |
|---|---|
| SEQ ID NO: 15607 | UUAUUCG |
| SEQ ID NO: 15608 | UUAUUCU |
| SEQ ID NO: 15609 | UUAUUGA |
| SEQ ID NO: 15610 | UUAUUGC |
| SEQ ID NO: 15611 | UUAUUGG |
| SEQ ID NO: 15612 | UUAUUGU |
| SEQ ID NO: 15613 | UUAUUUA |
| SEQ ID NO: 15614 | UUAUUUC |
| SEQ ID NO: 15615 | UUAUUUG |
| SEQ ID NO: 15616 | UUAUUUU |
| SEQ ID NO: 15617 | UUCAAAA |
| SEQ ID NO: 15618 | UUCAAAC |
| SEQ ID NO: 15619 | UUCAAAG |
| SEQ ID NO: 15620 | UUCAAAU |
| SEQ ID NO: 15621 | UUCAACA |
| SEQ ID NO: 15622 | UUCAACC |
| SEQ ID NO: 15623 | UUCAACG |
| SEQ ID NO: 15624 | UUCAACU |
| SEQ ID NO: 15625 | UUCAAGA |
| SEQ ID NO: 15626 | UUCAAGC |
| SEQ ID NO: 15627 | UUCAAGG |
| SEQ ID NO: 15628 | UUCAAGU |
| SEQ ID NO: 15629 | UUCAAUA |
| SEQ ID NO: 15630 | UUCAAUC |
| SEQ ID NO: 15631 | UUCAAUG |
| SEQ ID NO: 15632 | UUCAAUU |
| SEQ ID NO: 15633 | UUCACAA |
| SEQ ID NO: 15634 | UUCACAC |
| SEQ ID NO: 15635 | UUCACAG |
| SEQ ID NO: 15636 | UUCACAU |
| SEQ ID NO: 15637 | UUCACCA |
| SEQ ID NO: 15638 | UUCACCC |
| SEQ ID NO: 15639 | UUCACCG |
| SEQ ID NO: 15640 | UUCACCU |
| SEQ ID NO: 15641 | UUCACGA |
| SEQ ID NO: 15642 | UUCACGC |
| SEQ ID NO: 15643 | UUCACGG |
| SEQ ID NO: 15644 | UUCACGU |
| SEQ ID NO: 15645 | UUCACUA |
| SEQ ID NO: 15646 | UUCACUC |
| SEQ ID NO: 15647 | UUCACUG |
| SEQ ID NO: 15648 | UUCACUU |
| SEQ ID NO: 15649 | UUCAGAA |
| SEQ ID NO: 15650 | UUCAGAC |
| SEQ ID NO: 15651 | UUCAGAG |
| SEQ ID NO: 15652 | UUCAGAU |
| SEQ ID NO: 15653 | UUCAGCA |
| SEQ ID NO: 15654 | UUCAGCC |
| SEQ ID NO: 15655 | UUCAGCG |
| SEQ ID NO: 15656 | UUCAGCU |
| SEQ ID NO: 15657 | UUCAGGA |
| SEQ ID NO: 15658 | UUCAGGC |
| SEQ ID NO: 15659 | UUCAGGG |
| SEQ ID NO: 15660 | UUCAGGU |
| SEQ ID NO: 15661 | UUCAGUA |
| SEQ ID NO: 15662 | UUCAGUC |
| SEQ ID NO: 15663 | UUCAGUG |
| SEQ ID NO: 15664 | UUCAGUU |
| SEQ ID NO: 15665 | UUCAUAA |
| SEQ ID NO: 15666 | UUCAUAC |
| SEQ ID NO: 15667 | UUCAUAG |
| SEQ ID NO: 15668 | UUCAUAU |
| SEQ ID NO: 15669 | UUCAUCA |
| SEQ ID NO: 15670 | UUCAUCC |
| SEQ ID NO: 15671 | UUCAUCG |
| SEQ ID NO: 15672 | UUCAUCU |
| SEQ ID NO: 15673 | UUCAUGA |
| SEQ ID NO: 15674 | UUCAUGC |
| SEQ ID NO: 15675 | UUCAUGG |
| SEQ ID NO: 15676 | UUCAUGU |
| SEQ ID NO: 15677 | UUCAUUA |
| SEQ ID NO: 15678 | UUCAUUC |
| SEQ ID NO: 15679 | UUCAUUG |
| SEQ ID NO: 15680 | UUCAUUU |
| SEQ ID NO: 15681 | UUCCAAA |
| SEQ ID NO: 15682 | UUCCAAC |
| SEQ ID NO: 15683 | UUCCAAG |
| SEQ ID NO: 15684 | UUCCAAU |
| SEQ ID NO: 15685 | UUCCACA |
| SEQ ID NO: 15686 | UUCCACC |
| SEQ ID NO: 15687 | UUCCACG |
| SEQ ID NO: 15688 | UUCCACU |
| SEQ ID NO: 15689 | UUCCAGA |
| SEQ ID NO: 15690 | UUCCAGC |
| SEQ ID NO: 15691 | UUCCAGG |
| SEQ ID NO: 15692 | UUCCAGU |
| SEQ ID NO: 15693 | UUCCAUA |
| SEQ ID NO: 15694 | UUCCAUC |
| SEQ ID NO: 15695 | UUCCAUG |
| SEQ ID NO: 15696 | UUCCAUU |
| SEQ ID NO: 15697 | UUCCCAA |
| SEQ ID NO: 15698 | UUCCCAC |
| SEQ ID NO: 15699 | UUCCCAG |
| SEQ ID NO: 15700 | UUCCCAU |
| SEQ ID NO: 15701 | UUCCCCA |
| SEQ ID NO: 15702 | UUCCCCC |
| SEQ ID NO: 15703 | UUCCCCG |
| SEQ ID NO: 15704 | UUCCCCU |
| SEQ ID NO: 15705 | UUCCCGA |
| SEQ ID NO: 15706 | UUCCCGC |
| SEQ ID NO: 15707 | UUCCCGG |
| SEQ ID NO: 15708 | UUCCCGU |
| SEQ ID NO: 15709 | UUCCCUA |
| SEQ ID NO: 15710 | UUCCCUC |
| SEQ ID NO: 15711 | UUCCCUG |
| SEQ ID NO: 15712 | UUCCCUU |
| SEQ ID NO: 15713 | UUCCGAA |
| SEQ ID NO: 15714 | UUCCGAC |
| SEQ ID NO: 15715 | UUCCGAG |
| SEQ ID NO: 15716 | UUCCGAU |
| SEQ ID NO: 15717 | UUCCGCA |
| SEQ ID NO: 15718 | UUCCGCC |
| SEQ ID NO: 15719 | UUCCGCG |
| SEQ ID NO: 15720 | UUCCGCU |
| SEQ ID NO: 15721 | UUCCGGA |
| SEQ ID NO: 15722 | UUCCGGC |
| SEQ ID NO: 15723 | UUCCGGG |
| SEQ ID NO: 15724 | UUCCGGU |
| SEQ ID NO: 15725 | UUCCGUA |
| SEQ ID NO: 15726 | UUCCGUC |
| SEQ ID NO: 15727 | UUCCGUG |
| SEQ ID NO: 15728 | UUCCGUU |
| SEQ ID NO: 15729 | UUCCUAA |
| SEQ ID NO: 15730 | UUCCUAC |
| SEQ ID NO: 15731 | UUCCUAG |
| SEQ ID NO: 15732 | UUCCUAU |
| SEQ ID NO: 15733 | UUCCUCA |
| SEQ ID NO: 15734 | UUCCUCC |
| SEQ ID NO: 15735 | UUCCUCG |
| SEQ ID NO: 15736 | UUCCUCU |
| SEQ ID NO: 15737 | UUCCUGA |
| SEQ ID NO: 15738 | UUCCUGC |
| SEQ ID NO: 15739 | UUCCUGG |
| SEQ ID NO: 15740 | UUCCUGU |
| SEQ ID NO: 15741 | UUCCUUA |
| SEQ ID NO: 15742 | UUCCUUC |
| SEQ ID NO: 15743 | UUCCUUG |
| SEQ ID NO: 15744 | UUCCUUU |
| SEQ ID NO: 15745 | UUCGAAA |
| SEQ ID NO: 15746 | UUCGAAC |
| SEQ ID NO: 15747 | UUCGAAG |
| SEQ ID NO: 15748 | UUCGAAU |
| SEQ ID NO: 15749 | UUCGACA |
| SEQ ID NO: 15750 | UUCGACC |
| SEQ ID NO: 15751 | UUCGACG |
| SEQ ID NO: 15752 | UUCGACU |
| SEQ ID NO: 15753 | UUCGAGA |
| SEQ ID NO: 15754 | UUCGAGC |
| SEQ ID NO: 15755 | UUCGAGG |
| SEQ ID NO: 15756 | UUCGAGU |
| SEQ ID NO: 15757 | UUCGAUA |
| SEQ ID NO: 15758 | UUCGAUC |
| SEQ ID NO: 15759 | UUCGAUG |
| SEQ ID NO: 15760 | UUCGAUU |
| SEQ ID NO: 15761 | UUCGCAA |
| SEQ ID NO: 15762 | UUCGCAC |
| SEQ ID NO: 15763 | UUCGCAG |
| SEQ ID NO: 15764 | UUCGCAU |
| SEQ ID NO: 15765 | UUCGCCA |
| SEQ ID NO: 15766 | UUCGCCC |
| SEQ ID NO: 15767 | UUCGCCG |
| SEQ ID NO: 15768 | UUCGCCU |
| SEQ ID NO: 15769 | UUCGCGA |
| SEQ ID NO: 15770 | UUCGCGC |
| SEQ ID NO: 15771 | UUCGCGG |
| SEQ ID NO: 15772 | UUCGCGU |
| SEQ ID NO: 15773 | UUCGCUA |
| SEQ ID NO: 15774 | UUCGCUC |
| SEQ ID NO: 15775 | UUCGCUG |
| SEQ ID NO: 15776 | UUCGCUU |
| SEQ ID NO: 15777 | UUCGGAA |
| SEQ ID NO: 15778 | UUCGGAC |
| SEQ ID NO: 15779 | UUCGGAG |
| SEQ ID NO: 15780 | UUCGGAU |
| SEQ ID NO: 15781 | UUCGGCA |
| SEQ ID NO: 15782 | UUCGGCC |
| SEQ ID NO: 15783 | UUCGGCG |
| SEQ ID NO: 15784 | UUCGGCU |
| SEQ ID NO: 15785 | UUCGGGA |
| SEQ ID NO: 15786 | UUCGGGC |
| SEQ ID NO: 15787 | UUCGGGG |
| SEQ ID NO: 15788 | UUCGGGU |
| SEQ ID NO: 15789 | UUCGGUA |
| SEQ ID NO: 15790 | UUCGGUC |
| SEQ ID NO: 15791 | UUCGGUG |
| SEQ ID NO: 15792 | UUCGGUU |
| SEQ ID NO: 15793 | UUCGUAA |
| SEQ ID NO: 15794 | UUCGUAC |
| SEQ ID NO: 15795 | UUCGUAG |
| SEQ ID NO: 15796 | UUCGUAU |
| SEQ ID NO: 15797 | UUCGUCA |
| SEQ ID NO: 15798 | UUCGUCC |
| SEQ ID NO: 15799 | UUCGUCG |
| SEQ ID NO: 15800 | UUCGUCU |
| SEQ ID NO: 15801 | UUCGUGA |
| SEQ ID NO: 15802 | UUCGUGC |
| SEQ ID NO: 15803 | UUCGUGG |
| SEQ ID NO: 15804 | UUCGUGU |
| SEQ ID NO: 15805 | UUCGUUA |
| SEQ ID NO: 15806 | UUCGUUC |
| SEQ ID NO: 15807 | UUCGUUG |
| SEQ ID NO: 15808 | UUCGUUU |
| SEQ ID NO: 15809 | UUCUAAA |
| SEQ ID NO: 15810 | UUCUAAC |
| SEQ ID NO: 15811 | UUCUAAG |
| SEQ ID NO: 15812 | UUCUAAU |
| SEQ ID NO: 15813 | UUCUACA |
| SEQ ID NO: 15814 | UUCUACC |
| SEQ ID NO: 15815 | UUCUACG |
| SEQ ID NO: 15816 | UUCUACU |
| SEQ ID NO: 15817 | UUCUAGA |
| SEQ ID NO: 15818 | UUCUAGC |
| SEQ ID NO: 15819 | UUCUAGG |
| SEQ ID NO: 15820 | UUCUAGU |
| SEQ ID NO: 15821 | UUCUAUA |
| SEQ ID NO: 15822 | UUCUAUC |
| SEQ ID NO: 15823 | UUCUAUG |
| SEQ ID NO: 15824 | UUCUAUU |
| SEQ ID NO: 15825 | UUCUCAA |
| SEQ ID NO: 15826 | UUCUCAC |
| SEQ ID NO: 15827 | UUCUCAG |
| SEQ ID NO: 15828 | UUCUCAU |
| SEQ ID NO: 15829 | UUCUCCA |
| SEQ ID NO: 15830 | UUCUCCC |
| SEQ ID NO: 15831 | UUCUCCG |
| SEQ ID NO: 15832 | UUCUCCU |
| SEQ ID NO: 15833 | UUCUCGA |
| SEQ ID NO: 15834 | UUCUCGC |
| SEQ ID NO: 15835 | UUCUCGG |
| SEQ ID NO: 15836 | UUCUCGU |
| SEQ ID NO: 15837 | UUCUCUA |
| SEQ ID NO: 15838 | UUCUCUC |
| SEQ ID NO: 15839 | UUCUCUG |
| SEQ ID NO: 15840 | UUCUCUU |
| SEQ ID NO: 15841 | UUCUGAA |
| SEQ ID NO: 15842 | UUCUGAC |
| SEQ ID NO: 15843 | UUCUGAG |
| SEQ ID NO: 15844 | UUCUGAU |
| SEQ ID NO: 15845 | UUCUGCA |
| SEQ ID NO: 15846 | UUCUGCC |
| SEQ ID NO: 15847 | UUCUGCG |
| SEQ ID NO: 15848 | UUCUGCU |
| SEQ ID NO: 15849 | UUCUGGA |
| SEQ ID NO: 15850 | UUCUGGC |
| SEQ ID NO: 15851 | UUCUGGG |
| SEQ ID NO: 15852 | UUCUGGU |
| SEQ ID NO: 15853 | UUCUGUA |
| SEQ ID NO: 15854 | UUCUGUC |
| SEQ ID NO: 15855 | UUCUGUG |
| SEQ ID NO: 15856 | UUCUGUU |
| SEQ ID NO: 15857 | UUCUUAA |
| SEQ ID NO: 15858 | UUCUUAC |
| SEQ ID NO: 15859 | UUCUUAG |
| SEQ ID NO: 15860 | UUCUUAU |
| SEQ ID NO: 15861 | UUCUUCA |
| SEQ ID NO: 15862 | UUCUUCC |
| SEQ ID NO: 15863 | UUCUUCG |
| SEQ ID NO: 15864 | UUCUUCU |
| SEQ ID NO: 15865 | UUCUUGA |
| SEQ ID NO: 15866 | UUCUUGC |
| SEQ ID NO: 15867 | UUCUUGG |
| SEQ ID NO: 15868 | UUCUUGU |
| SEQ ID NO: 15869 | UUCUUUA |
| SEQ ID NO: 15870 | UUCUUUC |
| SEQ ID NO: 15871 | UUCUUUG |
| SEQ ID NO: 15872 | UUCUUUU |
| SEQ ID NO: 15873 | UUGAAAA |
| SEQ ID NO: 15874 | UUGAAAC |
| SEQ ID NO: 15875 | UUGAAAG |
| SEQ ID NO: 15876 | UUGAAAU |
| SEQ ID NO: 15877 | UUGAACA |
| SEQ ID NO: 15878 | UUGAACC |
| SEQ ID NO: 15879 | UUGAACG |
| SEQ ID NO: 15880 | UUGAACU |
| SEQ ID NO: 15881 | UUGAAGA |
| SEQ ID NO: 15882 | UUGAAGC |
| SEQ ID NO: 15883 | UUGAAGG |
| SEQ ID NO: 15884 | UUGAAGU |
| SEQ ID NO: 15885 | UUGAAUA |
| SEQ ID NO: 15886 | UUGAAUC |
| SEQ ID NO: 15887 | UUGAAUG |
| SEQ ID NO: 15888 | UUGAAUU |
| SEQ ID NO: 15889 | UUGACAA |
| SEQ ID NO: 15890 | UUGACAC |
| SEQ ID NO: 15891 | UUGACAG |
| SEQ ID NO: 15892 | UUGACAU |
| SEQ ID NO: 15893 | UUGACCA |
| SEQ ID NO: 15894 | UUGACCC |
| SEQ ID NO: 15895 | UUGACCG |
| SEQ ID NO: 15896 | UUGACCU |
| SEQ ID NO: 15897 | UUGACGA |
| SEQ ID NO: 15898 | UUGACGC |
| SEQ ID NO: 15899 | UUGACGG |
| SEQ ID NO: 15900 | UUGACGU |
| SEQ ID NO: 15901 | UUGACUA |
| SEQ ID NO: 15902 | UUGACUC |
| SEQ ID NO: 15903 | UUGACUG |
| SEQ ID NO: 15904 | UUGACUU |
| SEQ ID NO: 15905 | UUGAGAA |
| SEQ ID NO: 15906 | UUGAGAC |
| SEQ ID NO: 15907 | UUGAGAG |
| SEQ ID NO: 15908 | UUGAGAU |
| SEQ ID NO: 15909 | UUGAGCA |
| SEQ ID NO: 15910 | UUGAGCC |
| SEQ ID NO: 15911 | UUGAGCG |
| SEQ ID NO: 15912 | UUGAGCU |

Table 53

| | | | | | | | | | | | |
|---|---|---|---|---|---|---|---|---|---|---|---|
| SEQ ID NO: 15913 | UUGAGGA | SEQ ID NO: 15964 | UUGCCGU | SEQ ID NO: 16015 | UUGGAUG | SEQ ID NO: 16066 | UUGUAAC | SEQ ID NO: 16117 | UUGUUCA | SEQ ID NO: 16168 | UUUAGCU |
| SEQ ID NO: 15914 | UUGAGGC | SEQ ID NO: 15965 | UUGCCUA | SEQ ID NO: 16016 | UUGGAUU | SEQ ID NO: 16067 | UUGUAAG | SEQ ID NO: 16118 | UUGUUCC | SEQ ID NO: 16169 | UUUAGGA |
| SEQ ID NO: 15915 | UUGAGGG | SEQ ID NO: 15966 | UUGCCUC | SEQ ID NO: 16017 | UUGGCAA | SEQ ID NO: 16068 | UUGUAAU | SEQ ID NO: 16119 | UUGUUCG | SEQ ID NO: 16170 | UUUAGGC |
| SEQ ID NO: 15916 | UUGAGGU | SEQ ID NO: 15967 | UUGCCUG | SEQ ID NO: 16018 | UUGGCAC | SEQ ID NO: 16069 | UUGUACA | SEQ ID NO: 16120 | UUGUUCU | SEQ ID NO: 16171 | UUUAGGG |
| SEQ ID NO: 15917 | UUGAGUA | SEQ ID NO: 15968 | UUGCCUU | SEQ ID NO: 16019 | UUGGCAG | SEQ ID NO: 16070 | UUGUACC | SEQ ID NO: 16121 | UUGUUGA | SEQ ID NO: 16172 | UUUAGGU |
| SEQ ID NO: 15918 | UUGAGUC | SEQ ID NO: 15969 | UUGCGAA | SEQ ID NO: 16020 | UUGGCAU | SEQ ID NO: 16071 | UUGUACG | SEQ ID NO: 16122 | UUGUUGC | SEQ ID NO: 16173 | UUUAGUA |
| SEQ ID NO: 15919 | UUGAGUG | SEQ ID NO: 15970 | UUGCGAC | SEQ ID NO: 16021 | UUGGCCA | SEQ ID NO: 16072 | UUGUACU | SEQ ID NO: 16123 | UUGUUGG | SEQ ID NO: 16174 | UUUAGUC |
| SEQ ID NO: 15920 | UUGAGUU | SEQ ID NO: 15971 | UUGCGAG | SEQ ID NO: 16022 | UUGGCCC | SEQ ID NO: 16073 | UUGUAGA | SEQ ID NO: 16124 | UUGUUGU | SEQ ID NO: 16175 | UUUAGUG |
| SEQ ID NO: 15921 | UUGAUAA | SEQ ID NO: 15972 | UUGCGAU | SEQ ID NO: 16023 | UUGGCCG | SEQ ID NO: 16074 | UUGUAGC | SEQ ID NO: 16125 | UUGUUUA | SEQ ID NO: 16176 | UUUAGUU |
| SEQ ID NO: 15922 | UUGAUAC | SEQ ID NO: 15973 | UUGCGCA | SEQ ID NO: 16024 | UUGGCCU | SEQ ID NO: 16075 | UUGUAGG | SEQ ID NO: 16126 | UUGUUUC | SEQ ID NO: 16177 | UUUAUAA |
| SEQ ID NO: 15923 | UUGAUAG | SEQ ID NO: 15974 | UUGCGCC | SEQ ID NO: 16025 | UUGGCGA | SEQ ID NO: 16076 | UUGUAGU | SEQ ID NO: 16127 | UUGUUUG | SEQ ID NO: 16178 | UUUAUAC |
| SEQ ID NO: 15924 | UUGAUAU | SEQ ID NO: 15975 | UUGCGCG | SEQ ID NO: 16026 | UUGGCGC | SEQ ID NO: 16077 | UUGUAUA | SEQ ID NO: 16128 | UUGUUUU | SEQ ID NO: 16179 | UUUAUAG |
| SEQ ID NO: 15925 | UUGAUCA | SEQ ID NO: 15976 | UUGCGCU | SEQ ID NO: 16027 | UUGGCGG | SEQ ID NO: 16078 | UUGUAUC | SEQ ID NO: 16129 | UUUAAAA | SEQ ID NO: 16180 | UUUAUAU |
| SEQ ID NO: 15926 | UUGAUCC | SEQ ID NO: 15977 | UUGCGGA | SEQ ID NO: 16028 | UUGGCGU | SEQ ID NO: 16079 | UUGUAUG | SEQ ID NO: 16130 | UUUAAAC | SEQ ID NO: 16181 | UUUAUCA |
| SEQ ID NO: 15927 | UUGAUCG | SEQ ID NO: 15978 | UUGCGGC | SEQ ID NO: 16029 | UUGGCUA | SEQ ID NO: 16080 | UUGUAUU | SEQ ID NO: 16131 | UUUAAAG | SEQ ID NO: 16182 | UUUAUCC |
| SEQ ID NO: 15928 | UUGAUCU | SEQ ID NO: 15979 | UUGCGGG | SEQ ID NO: 16030 | UUGGCUC | SEQ ID NO: 16081 | UUGUCAA | SEQ ID NO: 16132 | UUUAAAU | SEQ ID NO: 16183 | UUUAUCG |
| SEQ ID NO: 15929 | UUGAUGA | SEQ ID NO: 15980 | UUGCGGU | SEQ ID NO: 16031 | UUGGCUG | SEQ ID NO: 16082 | UUGUCAC | SEQ ID NO: 16133 | UUUAACA | SEQ ID NO: 16184 | UUUAUCU |
| SEQ ID NO: 15930 | UUGAUGC | SEQ ID NO: 15981 | UUGCGUA | SEQ ID NO: 16032 | UUGGCUU | SEQ ID NO: 16083 | UUGUCAG | SEQ ID NO: 16134 | UUUAACC | SEQ ID NO: 16185 | UUUAUGA |
| SEQ ID NO: 15931 | UUGAUGG | SEQ ID NO: 15982 | UUGCGUC | SEQ ID NO: 16033 | UUGGGAA | SEQ ID NO: 16084 | UUGUCAU | SEQ ID NO: 16135 | UUUAACG | SEQ ID NO: 16186 | UUUAUGC |
| SEQ ID NO: 15932 | UUGAUGU | SEQ ID NO: 15983 | UUGCGUG | SEQ ID NO: 16034 | UUGGGAC | SEQ ID NO: 16085 | UUGUCCA | SEQ ID NO: 16136 | UUUAACU | SEQ ID NO: 16187 | UUUAUGG |
| SEQ ID NO: 15933 | UUGAUUA | SEQ ID NO: 15984 | UUGCGUU | SEQ ID NO: 16035 | UUGGGAG | SEQ ID NO: 16086 | UUGUCCC | SEQ ID NO: 16137 | UUUAAGA | SEQ ID NO: 16188 | UUUAUGU |
| SEQ ID NO: 15934 | UUGAUUC | SEQ ID NO: 15985 | UUGCUAA | SEQ ID NO: 16036 | UUGGGAU | SEQ ID NO: 16087 | UUGUCCG | SEQ ID NO: 16138 | UUUAAGC | SEQ ID NO: 16189 | UUUAUUA |
| SEQ ID NO: 15935 | UUGAUUG | SEQ ID NO: 15986 | UUGCUAC | SEQ ID NO: 16037 | UUGGGCA | SEQ ID NO: 16088 | UUGUCCU | SEQ ID NO: 16139 | UUUAAGG | SEQ ID NO: 16190 | UUUAUUC |
| SEQ ID NO: 15936 | UUGAUUU | SEQ ID NO: 15987 | UUGCUAG | SEQ ID NO: 16038 | UUGGGCC | SEQ ID NO: 16089 | UUGUCGA | SEQ ID NO: 16140 | UUUAAGU | SEQ ID NO: 16191 | UUUAUUG |
| SEQ ID NO: 15937 | UUGCAAA | SEQ ID NO: 15988 | UUGCUAU | SEQ ID NO: 16039 | UUGGGCG | SEQ ID NO: 16090 | UUGUCGC | SEQ ID NO: 16141 | UUUAAUA | SEQ ID NO: 16192 | UUUAUUU |
| SEQ ID NO: 15938 | UUGCAAC | SEQ ID NO: 15989 | UUGCUCA | SEQ ID NO: 16040 | UUGGGCU | SEQ ID NO: 16091 | UUGUCGG | SEQ ID NO: 16142 | UUUAAUC | SEQ ID NO: 16193 | UUUCAAA |
| SEQ ID NO: 15939 | UUGCAAG | SEQ ID NO: 15990 | UUGCUCC | SEQ ID NO: 16041 | UUGGGGA | SEQ ID NO: 16092 | UUGUCGU | SEQ ID NO: 16143 | UUUAAUG | SEQ ID NO: 16194 | UUUCAAC |
| SEQ ID NO: 15940 | UUGCAAU | SEQ ID NO: 15991 | UUGCUCG | SEQ ID NO: 16042 | UUGGGGC | SEQ ID NO: 16093 | UUGUCUA | SEQ ID NO: 16144 | UUUAAUU | SEQ ID NO: 16195 | UUUCAAG |
| SEQ ID NO: 15941 | UUGCACA | SEQ ID NO: 15992 | UUGCUCU | SEQ ID NO: 16043 | UUGGGGG | SEQ ID NO: 16094 | UUGUCUC | SEQ ID NO: 16145 | UUUACAA | SEQ ID NO: 16196 | UUUCAAU |
| SEQ ID NO: 15942 | UUGCACC | SEQ ID NO: 15993 | UUGCUGA | SEQ ID NO: 16044 | UUGGGGU | SEQ ID NO: 16095 | UUGUCUG | SEQ ID NO: 16146 | UUUACAC | SEQ ID NO: 16197 | UUUCACA |
| SEQ ID NO: 15943 | UUGCACG | SEQ ID NO: 15994 | UUGCUGC | SEQ ID NO: 16045 | UUGGGUA | SEQ ID NO: 16096 | UUGUCUU | SEQ ID NO: 16147 | UUUACAG | SEQ ID NO: 16198 | UUUCACC |
| SEQ ID NO: 15944 | UUGCACU | SEQ ID NO: 15995 | UUGCUGG | SEQ ID NO: 16046 | UUGGGUC | SEQ ID NO: 16097 | UUGUGAA | SEQ ID NO: 16148 | UUUACAU | SEQ ID NO: 16199 | UUUCACG |
| SEQ ID NO: 15945 | UUGCAGA | SEQ ID NO: 15996 | UUGCUGU | SEQ ID NO: 16047 | UUGGGUG | SEQ ID NO: 16098 | UUGUGAC | SEQ ID NO: 16149 | UUUACCA | SEQ ID NO: 16200 | UUUCACU |
| SEQ ID NO: 15946 | UUGCAGC | SEQ ID NO: 15997 | UUGCUUA | SEQ ID NO: 16048 | UUGGGUU | SEQ ID NO: 16099 | UUGUGAG | SEQ ID NO: 16150 | UUUACCC | SEQ ID NO: 16201 | UUUCAGA |
| SEQ ID NO: 15947 | UUGCAGG | SEQ ID NO: 15998 | UUGCUUC | SEQ ID NO: 16049 | UUGGUAA | SEQ ID NO: 16100 | UUGUGAU | SEQ ID NO: 16151 | UUUACCG | SEQ ID NO: 16202 | UUUCAGC |
| SEQ ID NO: 15948 | UUGCAGU | SEQ ID NO: 15999 | UUGCUUG | SEQ ID NO: 16050 | UUGGUAC | SEQ ID NO: 16101 | UUGUGCA | SEQ ID NO: 16152 | UUUACCU | SEQ ID NO: 16203 | UUUCAGG |
| SEQ ID NO: 15949 | UUGCAUA | SEQ ID NO: 16000 | UUGCUUU | SEQ ID NO: 16051 | UUGGUAG | SEQ ID NO: 16102 | UUGUGCC | SEQ ID NO: 16153 | UUUACGA | SEQ ID NO: 16204 | UUUCAGU |
| SEQ ID NO: 15950 | UUGCAUC | SEQ ID NO: 16001 | UUGGAAA | SEQ ID NO: 16052 | UUGGUAU | SEQ ID NO: 16103 | UUGUGCG | SEQ ID NO: 16154 | UUUACGC | SEQ ID NO: 16205 | UUUCAUA |
| SEQ ID NO: 15951 | UUGCAUG | SEQ ID NO: 16002 | UUGGAAC | SEQ ID NO: 16053 | UUGGUCA | SEQ ID NO: 16104 | UUGUGCU | SEQ ID NO: 16155 | UUUACGG | SEQ ID NO: 16206 | UUUCAUC |
| SEQ ID NO: 15952 | UUGCAUU | SEQ ID NO: 16003 | UUGGAAG | SEQ ID NO: 16054 | UUGGUCC | SEQ ID NO: 16105 | UUGUGGA | SEQ ID NO: 16156 | UUUACGU | SEQ ID NO: 16207 | UUUCAUG |
| SEQ ID NO: 15953 | UUGCCAA | SEQ ID NO: 16004 | UUGGAAU | SEQ ID NO: 16055 | UUGGUCG | SEQ ID NO: 16106 | UUGUGGC | SEQ ID NO: 16157 | UUUACUA | SEQ ID NO: 16208 | UUUCAUU |
| SEQ ID NO: 15954 | UUGCCAC | SEQ ID NO: 16005 | UUGGACA | SEQ ID NO: 16056 | UUGGUCU | SEQ ID NO: 16107 | UUGUGGG | SEQ ID NO: 16158 | UUUACUC | SEQ ID NO: 16209 | UUUCCAA |
| SEQ ID NO: 15955 | UUGCCAG | SEQ ID NO: 16006 | UUGGACC | SEQ ID NO: 16057 | UUGGUGA | SEQ ID NO: 16108 | UUGUGGU | SEQ ID NO: 16159 | UUUACUG | SEQ ID NO: 16210 | UUUCCAC |
| SEQ ID NO: 15956 | UUGCCAU | SEQ ID NO: 16007 | UUGGACG | SEQ ID NO: 16058 | UUGGUGC | SEQ ID NO: 16109 | UUGUGUA | SEQ ID NO: 16160 | UUUACUU | SEQ ID NO: 16211 | UUUCCAG |
| SEQ ID NO: 15957 | UUGCCCA | SEQ ID NO: 16008 | UUGGACU | SEQ ID NO: 16059 | UUGGUGG | SEQ ID NO: 16110 | UUGUGUC | SEQ ID NO: 16161 | UUUAGAA | SEQ ID NO: 16212 | UUUCCAU |
| SEQ ID NO: 15958 | UUGCCCC | SEQ ID NO: 16009 | UUGGAGA | SEQ ID NO: 16060 | UUGGUGU | SEQ ID NO: 16111 | UUGUGUG | SEQ ID NO: 16162 | UUUAGAC | SEQ ID NO: 16213 | UUUCCCA |
| SEQ ID NO: 15959 | UUGCCCG | SEQ ID NO: 16010 | UUGGAGC | SEQ ID NO: 16061 | UUGGUUA | SEQ ID NO: 16112 | UUGUGUU | SEQ ID NO: 16163 | UUUAGAG | SEQ ID NO: 16214 | UUUCCCC |
| SEQ ID NO: 15960 | UUGCCCU | SEQ ID NO: 16011 | UUGGAGG | SEQ ID NO: 16062 | UUGGUUC | SEQ ID NO: 16113 | UUGUUAA | SEQ ID NO: 16164 | UUUAGAU | SEQ ID NO: 16215 | UUUCCCG |
| SEQ ID NO: 15961 | UUGCCGA | SEQ ID NO: 16012 | UUGGAGU | SEQ ID NO: 16063 | UUGGUUG | SEQ ID NO: 16114 | UUGUUAC | SEQ ID NO: 16165 | UUUAGCA | SEQ ID NO: 16216 | UUUCCCU |
| SEQ ID NO: 15962 | UUGCCGC | SEQ ID NO: 16013 | UUGGAUA | SEQ ID NO: 16064 | UUGGUUU | SEQ ID NO: 16115 | UUGUUAG | SEQ ID NO: 16166 | UUUAGCC | SEQ ID NO: 16217 | UUUCCGA |
| SEQ ID NO: 15963 | UUGCCGG | SEQ ID NO: 16014 | UUGGAUC | SEQ ID NO: 16065 | UUGUAAA | SEQ ID NO: 16116 | UUGUUAU | SEQ ID NO: 16167 | UUUAGCG | SEQ ID NO: 16218 | UUUCCGC |

Table 54

| | | | | | | | |
|---|---|---|---|---|---|---|---|
| SEQ ID NO: 16219 | UUUCCGG | SEQ ID NO: 16270 | UUUGAUC | SEQ ID NO: 16321 | UUUUAAA | SEQ ID NO: 16372 | UUUUUAU |
| SEQ ID NO: 16220 | UUUCCGU | SEQ ID NO: 16271 | UUUGAUG | SEQ ID NO: 16322 | UUUUAAC | SEQ ID NO: 16373 | UUUUUCA |
| SEQ ID NO: 16221 | UUUCCUA | SEQ ID NO: 16272 | UUUGAUU | SEQ ID NO: 16323 | UUUUAAG | SEQ ID NO: 16374 | UUUUUCC |
| SEQ ID NO: 16222 | UUUCCUC | SEQ ID NO: 16273 | UUUGCAA | SEQ ID NO: 16324 | UUUUAAU | SEQ ID NO: 16375 | UUUUUCG |
| SEQ ID NO: 16223 | UUUCCUG | SEQ ID NO: 16274 | UUUGCAC | SEQ ID NO: 16325 | UUUUACA | SEQ ID NO: 16376 | UUUUUCU |
| SEQ ID NO: 16224 | UUUCCUU | SEQ ID NO: 16275 | UUUGCAG | SEQ ID NO: 16326 | UUUUACC | SEQ ID NO: 16377 | UUUUUGA |
| SEQ ID NO: 16225 | UUUCGAA | SEQ ID NO: 16276 | UUUGCAU | SEQ ID NO: 16327 | UUUUACG | SEQ ID NO: 16378 | UUUUUGC |
| SEQ ID NO: 16226 | UUUCGAC | SEQ ID NO: 16277 | UUUGCCA | SEQ ID NO: 16328 | UUUUACU | SEQ ID NO: 16379 | UUUUUGG |
| SEQ ID NO: 16227 | UUUCGAG | SEQ ID NO: 16278 | UUUGCCC | SEQ ID NO: 16329 | UUUUAGA | SEQ ID NO: 16380 | UUUUUGU |
| SEQ ID NO: 16228 | UUUCGAU | SEQ ID NO: 16279 | UUUGCCG | SEQ ID NO: 16330 | UUUUAGC | SEQ ID NO: 16381 | UUUUUUA |
| SEQ ID NO: 16229 | UUUCGCA | SEQ ID NO: 16280 | UUUGCCU | SEQ ID NO: 16331 | UUUUAGG | SEQ ID NO: 16382 | UUUUUUC |
| SEQ ID NO: 16230 | UUUCGCC | SEQ ID NO: 16281 | UUUGCGA | SEQ ID NO: 16332 | UUUUAGU | SEQ ID NO: 16383 | UUUUUUG |
| SEQ ID NO: 16231 | UUUCGCG | SEQ ID NO: 16282 | UUUGCGC | SEQ ID NO: 16333 | UUUUAUA | SEQ ID NO: 16384 | UUUUUUU |
| SEQ ID NO: 16232 | UUUCGCU | SEQ ID NO: 16283 | UUUGCGG | SEQ ID NO: 16334 | UUUUAUC | | |
| SEQ ID NO: 16233 | UUUCGGA | SEQ ID NO: 16284 | UUUGCGU | SEQ ID NO: 16335 | UUUUAUG | | |
| SEQ ID NO: 16234 | UUUCGGC | SEQ ID NO: 16285 | UUUGCUA | SEQ ID NO: 16336 | UUUUAUU | | |
| SEQ ID NO: 16235 | UUUCGGG | SEQ ID NO: 16286 | UUUGCUC | SEQ ID NO: 16337 | UUUUCAA | | |
| SEQ ID NO: 16236 | UUUCGGU | SEQ ID NO: 16287 | UUUGCUG | SEQ ID NO: 16338 | UUUUCAC | | |
| SEQ ID NO: 16237 | UUUCGUA | SEQ ID NO: 16288 | UUUGCUU | SEQ ID NO: 16339 | UUUUCAG | | |
| SEQ ID NO: 16238 | UUUCGUC | SEQ ID NO: 16289 | UUUGGAA | SEQ ID NO: 16340 | UUUUCAU | | |
| SEQ ID NO: 16239 | UUUCGUG | SEQ ID NO: 16290 | UUUGGAC | SEQ ID NO: 16341 | UUUUCCA | | |
| SEQ ID NO: 16240 | UUUCGUU | SEQ ID NO: 16291 | UUUGGAG | SEQ ID NO: 16342 | UUUUCCC | | |
| SEQ ID NO: 16241 | UUUCUAA | SEQ ID NO: 16292 | UUUGGAU | SEQ ID NO: 16343 | UUUUCCG | | |
| SEQ ID NO: 16242 | UUUCUAC | SEQ ID NO: 16293 | UUUGGCA | SEQ ID NO: 16344 | UUUUCCU | | |
| SEQ ID NO: 16243 | UUUCUAG | SEQ ID NO: 16294 | UUUGGCC | SEQ ID NO: 16345 | UUUUCGA | | |
| SEQ ID NO: 16244 | UUUCUAU | SEQ ID NO: 16295 | UUUGGCG | SEQ ID NO: 16346 | UUUUCGC | | |
| SEQ ID NO: 16245 | UUUCUCA | SEQ ID NO: 16296 | UUUGGCU | SEQ ID NO: 16347 | UUUUCGG | | |
| SEQ ID NO: 16246 | UUUCUCC | SEQ ID NO: 16297 | UUUGGGA | SEQ ID NO: 16348 | UUUUCGU | | |
| SEQ ID NO: 16247 | UUUCUCG | SEQ ID NO: 16298 | UUUGGGC | SEQ ID NO: 16349 | UUUUCUA | | |
| SEQ ID NO: 16248 | UUUCUCU | SEQ ID NO: 16299 | UUUGGGG | SEQ ID NO: 16350 | UUUUCUC | | |
| SEQ ID NO: 16249 | UUUCUGA | SEQ ID NO: 16300 | UUUGGGU | SEQ ID NO: 16351 | UUUUCUG | | |
| SEQ ID NO: 16250 | UUUCUGC | SEQ ID NO: 16301 | UUUGGUA | SEQ ID NO: 16352 | UUUUCUU | | |
| SEQ ID NO: 16251 | UUUCUGG | SEQ ID NO: 16302 | UUUGGUC | SEQ ID NO: 16353 | UUUUGAA | | |
| SEQ ID NO: 16252 | UUUCUGU | SEQ ID NO: 16303 | UUUGGUG | SEQ ID NO: 16354 | UUUUGAC | | |
| SEQ ID NO: 16253 | UUUCUUA | SEQ ID NO: 16304 | UUUGGUU | SEQ ID NO: 16355 | UUUUGAG | | |
| SEQ ID NO: 16254 | UUUCUUC | SEQ ID NO: 16305 | UUUGUAA | SEQ ID NO: 16356 | UUUUGAU | | |
| SEQ ID NO: 16255 | UUUCUUG | SEQ ID NO: 16306 | UUUGUAC | SEQ ID NO: 16357 | UUUUGCA | | |
| SEQ ID NO: 16256 | UUUCUUU | SEQ ID NO: 16307 | UUUGUAG | SEQ ID NO: 16358 | UUUUGCC | | |
| SEQ ID NO: 16257 | UUUGAAA | SEQ ID NO: 16308 | UUUGUAU | SEQ ID NO: 16359 | UUUUGCG | | |
| SEQ ID NO: 16258 | UUUGAAC | SEQ ID NO: 16309 | UUUGUCA | SEQ ID NO: 16360 | UUUUGCU | | |
| SEQ ID NO: 16259 | UUUGAAG | SEQ ID NO: 16310 | UUUGUCC | SEQ ID NO: 16361 | UUUUGGA | | |
| SEQ ID NO: 16260 | UUUGAAU | SEQ ID NO: 16311 | UUUGUCG | SEQ ID NO: 16362 | UUUUGGC | | |
| SEQ ID NO: 16261 | UUUGACA | SEQ ID NO: 16312 | UUUGUCU | SEQ ID NO: 16363 | UUUUGGG | | |
| SEQ ID NO: 16262 | UUUGACC | SEQ ID NO: 16313 | UUUGUGA | SEQ ID NO: 16364 | UUUUGGU | | |
| SEQ ID NO: 16263 | UUUGACG | SEQ ID NO: 16314 | UUUGUGC | SEQ ID NO: 16365 | UUUUGUA | | |
| SEQ ID NO: 16264 | UUUGACU | SEQ ID NO: 16315 | UUUGUGG | SEQ ID NO: 16366 | UUUUGUC | | |
| SEQ ID NO: 16265 | UUUGAGA | SEQ ID NO: 16316 | UUUGUGU | SEQ ID NO: 16367 | UUUUGUG | | |
| SEQ ID NO: 16266 | UUUGAGC | SEQ ID NO: 16317 | UUUGUUA | SEQ ID NO: 16368 | UUUUGUU | | |
| SEQ ID NO: 16267 | UUUGAGG | SEQ ID NO: 16318 | UUUGUUC | SEQ ID NO: 16369 | UUUUUAA | | |
| SEQ ID NO: 16268 | UUUGAGU | SEQ ID NO: 16319 | UUUGUUG | SEQ ID NO: 16370 | UUUUUAC | | |
| SEQ ID NO: 16269 | UUUGAUA | SEQ ID NO: 16320 | UUUGUUU | SEQ ID NO: 16371 | UUUUUAG | | |

Structural analysis can be performed to identify the T-arm-like stem-loop structure formed by a target RNA in the target RNA, by performing assessments based on pattern matching and thermodynamic stability. In computations for structural analysis, a working hypothesis can be put in place that 1) the mRNA adopts a higher-order structure in a single strand on its own without protein involvement, 2) the mRNA adopts a higher-order structure on a fixed-length partial sequence of the part presumed to be flanked by ribosomes and ribosomes rather than on the full-length during protein synthesis by ribosome, and 3) the single primary-structure mRNA (or a fixed-length partial sequence as part thereof) is presumed to adopt multiple higher-order structures and they are in equilibrium (i.e., the existence probabilities are distributed according to the amount of energy). Calculation and evaluation of intrinsic partial structures can be based on this hypothesis. In addition, for the designing of sgASO, as tRNase $Z^L$ can recognize a higher-order structure formed by the sgASO and intrinsic structure, it can be hypothesized that for the intrinsic structure, 1) the loop is 10 bases or less, and 2) the stem is 5 base pairs. If structural analyses based on pattern matching and thermodynamic stability can address all possible structures comprehensively, analysis can be simplified by calculating only those structures that are likely to be important using general structure prediction software. For structural predictions, software such as vswindow, UNAFold, mfold can be utilized.

For structure prediction, the following references may be referred to:

- Markham, N. R. & Zuker, M. (2005) DINAMelt web server for nucleic acid melting prediction. Nucleic Acids Res., 33, W577-W581; and
- Markham, N. R. & Zuker, M. (2008) UNAFold: software for nucleic acid folding and hybridization. In Keith, J. M., editor, Bioinformatics, Volume II. Structure, Function and Applications, number 453 in Methods in Molecular Biology, chapter 1, pages 3-31. Humana Press, Totowa, NJ. ISBN 978-1-60327-428-9.).

For example, by setting the W-base width frame n from the 5' end of the mRNA in R base increments (nmax frames can be obtained from the transcript of interest); and in each frame n, it is hypothesized that the state within the cell within which the mRNA is placed is in equilibrium based on the predicted structure results and the respective energy levels, and thus the existence probabilities are calculated from each predicted structure result by the Maxwell-Boltzmann statistics. Here, for the prediction result of mth from the most stable structure among the structure prediction results of nth frame (in frame n, the number of the predicted structure result obtained is mmax(n)), the existence probability of that predicted structure can be j(n,m).

Here, defining the partial structure found in each of the structure prediction results, which consists of only one loop site and only one stem complementary to each other, as the stem-loop structure (the term "complementary to each other" for the stem loop does not mean that it does not constitute any other stem loop even in other prediction structure frames, but it means to never include a portion of another stem loop in a single prediction structure result). Here, the stem may contain a mismatch (bases that do not participate in base pairing in the stem are opposing) or a bulge (bases that do not participate in base pairing in the stem are not opposing), but both ends are base pairs. In addition, the stem loop enclosed within the stem loop is also considered as a separate stem loop (e.g., the loosened base pairs farthest from the loop of the stem are treated as separate stem loops from those that are not loosened).

These stem loops are called motif(x,p) when they begin with the absolute position x on the sequence rather than in the frame, and the property profiles of the constituting loops and stems (individual characteristics of the stem loops defined by the position of bases in the stem-constituting base pairs) are designated as p. Then, the existence probability within the frame n of motif(x,p) is set as partial existence probability P_local(x,p,n), and the value is the sum of j values for the structures in which the stem loop exists in the structure prediction results obtained in the n frame, i.e., Σj(n,m). In addition, when the result of summing from frame 1 to nmax for this P_local(x,p,n) is taken as ΣP_local(x,p,n), and the number of frames in the full-length sequence constituting the stem loop which could fall within the frame is set as n_all(x,p), the existence probability of motif(x,p) in the whole, P_global(x,p), is represented as ΣP_local(x,p,n)/n_all(x,p).

Namely, in frame n, the local existence probability P_local(x,p,n) of motif(x,p) is represented as:

$$\mathrm{P_local(x,p,n)} = \sum_{m=1}^{\mathrm{mmax(n)}} \begin{cases} 0 & \text{... if motif (x, p) is not found in the m} - \text{th structure of frame n} \\ \mathrm{j(n,m)} & \text{... if motif (x, p) is found in the m} - \text{th structure of frame n} \end{cases}$$

For motif(x,p), the existence probability in the whole P_global(x,p) is represented as:

$$\mathrm{P_global(x,p)} = \sum_{n=1}^{nmax} \frac{P_local(x,p,n)}{n_all}$$

In the target RNA, motif(x, p1) can be identified in which P_global(x, p) is 50% or more and p1 = "stem is 5 base pairs". Among them, motif(x, p2) can be further identified where p2 = "left-right evenness (within one difference between the 5' side sequence and 3' side sequence of the loop-flanking stem) and the length of the loop does not exceed 10".

A sgASO binding site can then be located at the seven bases immediately after each of the stem loops of motif (x,p2), and the following corrections can be processed: (1) the loop portion predicted to be three bases unwinds the next base pair to form a five-base loop; (2) when the number of base pairs in the mismatch or bulge of the stem is 2 or less, they are ignored in the counting of base pairs; and/or (3) when the number of base pairs in the mismatch or bulge of the stem is 3 or more, half of the number of base pairs in the mismatch or bulge is counted as base pairs. This processing identifies the 5th base pair of the stem loop structure, and the immediate (3' side) position can serve as the site of sgASO binding.

One embodiment of the present invention relates to a method of designing an oligonucleotide (sgASO) for cleaving a target RNA in a eukaryotic cell, comprising (1) identifying in the target RNA sequence a sequence in which at least one stem-loop structure is formed, (2) identifying a sequence of 6-10 bases adjacent to the stem loop structure on the 3' side as a sgASO binding sequence, and (3) identifying a sequence complementary to the binding sequence as a sgASO sequence.

Target transcripts

Target RNAs are transcript RNAs, particularly mRNAs, of genes of interest (target genes) that suppress or abolish their expression, but may be ncRNAs. Also, target RNAs include RNAs from the nuclear genome, RNAs from the mitochondrial genome, RNAs from the viral or bacterial genome, and such. Although the sgASO used in the present invention does not necessarily need to be fully complementary to the target RNA, it is typically fully complementary to the target RNA. Suppression of the expression of a target gene is reduction of the amount of protein transcriptionally synthesized from that gene to 25% or less, preferably 50% or less, more preferably 75% or less, and particularly preferably 95% or less. The target gene is a gene whose suppression or loss of expression is of industrial value, particularly a gene whose increased expression is the cause of a particular disease (hereafter referred to as a pathogenic gene). Examples of such pathogenic genes include, for example, the ras, erbb2, myc, apc, brca1, rb, Bcl-2, BGEF oncogenes, genes involved in hypertension such as renin, diabetes-related genes such as insulin, hyperlipidemia-related genes such as LDL receptor, obesity-related genes such as leptin, arteriosclerosis disease-related genes such as angiotensin, apolipoproteins and preserinins known to be responsible for dementia, and senescence-related genes. These genes and their mRNA sequences are known in public databases (e.g., NCBI nucleotides databases).

Pharmaceutical compositions

Although small-guide antisense nucleic acids can also be formulated alone, they are typically mixed with one or more pharmacologically acceptable carriers and preferably administered as pharmaceutical formulations manufactured by any method well known in the art of pharmaceutical sciences. Pharmaceutical compositions may include mixtures of multiple sgASOs with distinct sequences.

Targets for administration include humans or non-human animals, e.g., non-human mammals. The most effective route of administration is preferably used in the treatment and can be given orally or parenterally, such as intraoral, airway, rectal, subcutaneous, intramuscular, intravenous and transdermal, preferably intravenously.

Formulations suitable for oral administration include emulsions, syrups, capsules, tablets, powders, granules, and such. Liquid preparations such as emulsions and syrups can be manufactured using water, sucrose, sorbitol, saccharides such as fructose, glycols such as polyethylene glycol and propylene glycol, oils such as sesame oil, olive oil and soybean oil, antiseptics such as p-hydroxybenzoate esters, flavors such as strawberry flavor, peppermint, and such as additives. Capsules, tablets, powders, granules, and such can be manufactured using excipients such as lactose, glucose, sucrose, mannitol, etc.; excipients such as starch, sodium alginate, etc.; lubricants such as magnesium stearate, talc, etc.; binding agents such as polyvinyl alcohol, hydroxypropyl cellulose, gelatin, etc.; surfactants such as fatty acid esters; plasticizers such as glycerin, and such as additives.

Formulations suitable for parenteral administration include injections, suppositories, and sprays. Injections are prepared using a salt solution, a glucose solution, or a carrier consisting of a mixture of both. Suppositories are prepared using carriers such as cocoa fat, hydrogenated fat or carboxylic acid. Sprays are also prepared using carriers or such, which do not irritate the recipient's oral and respiratory mucosa and disperse the active ingredient as fine particles to facilitate absorption.

Examples of carriers include lactose, glycerin, liposomes, nano micelles, and such. Due to the nature of the nucleic acids used in the present invention and even the carriers used, formulations such as aerosols, dry powders, and such can be made. Ingredients listed as examples of excipient in oral formulations can also be added to these parenteral.

The dosage or frequency of administration varies depending on the intended therapeutic effect, administration method, treatment period, age, and body weight, but is usually between 10 μg/kg and 100 mg/kg/day for adults.

One embodiment of the present invention is a pharmaceutical composition for use in the treatment or prevention of a disease or a disorder in a patient, comprising an oligonucleotide (sgASO) of about 7 mers, wherein the oligonucleotide of about 7 mers is complementary to a target RNA associated with a disease or disorder, the target RNA is capable of forming at least one stem-loop structure, the oligonucleotide of about 7 mers is able to hybridize to a 3'-side region of the stem-loop structure to form at least one larger stem-loop structure in conjunction with the stem loop formed by the target RNA, and the formed structure is recognized by tRNaseZ$^L$ in the patient's body to cleave the target RNA. The patient may be a human or a non-human animal. It should be noted that when referred to herein as approximately 7 mers, it is interpreted to include 6 mers, 7 mers, and 8 mers.

SgASO is commonly, but not exclusively, designed to base-pair with the base directly 3' adjacent to the base that forms the root of the stem-loop structure formed by the target RNA (i.e., the terminal base pair opposite to the loop) and with the 3' terminal base of the sgASO. For example, it may be designed so that base pairs are formed between the 3'-terminal bases of the sgASO and the second to third bases counted from the root of the stem-loop structure formed by the target RNA.

In one embodiment of the present invention, the pharmaceutical compositions may comprise at least one sgASO of VIS-01 to VIS-07 disclosed in the embodiment of the present invention. In one embodiment of the present invention, the pharmaceutical composition may be a composition for the treatment or prevention of a cancer, or a composition for use in the treatment or prevention of a cancer.

One embodiment in the present invention relates to a pharmaceutical composition for the treatment or prevention of a cancer, or a pharmaceutical composition for use in the treatment or prevention of a cancer, comprising a sgASO comprising or consisting of any sequence selected from: 5'-GAAACUU-3'; 5'-CUGUCAA-3'; 5'-UCUUCAA-3'; 5'-UUAUCGU-3'; 5'-CUUAUAA-3'; 5'-GCGGGGG-3'; 5'-ACUCAAA-3'. The sgASO may have a phosphate group at one or both ends. Cancers for which the pharmaceutical composition may be used are those involving the Bcl-2 gene, e.g., lymphoma.

<u>Use in the manufacturing of pharmaceuticals and treatment methods</u>

One embodiment of the present invention is the use of an oligonucleotide (sgASO) of about 7 mers in the manufacture of a pharmaceutical for use in the treatment or prevention of a disease or disorder in a patient, wherein the oligonucleotide of about 7 mers is complementary to a target RNA associated with a disease or disorder, the target RNA is capable of forming at least one stem-loop structure, the oligonucleotide of about 7 mers hybridizes to a 3'-side region of the stem-loop structure to form at least one larger stem-loop structure in conjunction with the stem loop formed by the target RNA, and the formed structure is recognized by tRNaseZ$^L$ in the patient's body to cleave the target RNA.

Further, one embodiment of the present invention relates to a method for the treatment or prevention of a disease or disorder in a patient, comprising administering to the patient a sgASO of about 7 mers, wherein the oligonucleotide of about 7 mers is complementary to a target RNA associated with the disease or disorder, wherein the target RNA is capable of forming at least one stem loop structure, wherein the oligonucleotide of about 7 mers is capable of hybridizing to a 3'-side region of the stem-loop structure to form at least one larger stem loop structure in conjunction with the stem loop formed by the target RNA, wherein the formed structure is recognized by tRNaseZ$^L$ in the patient to cleave the target RNA.

One embodiment of the present invention relates to the use of a sgASO comprising or consisting of any sequence selected from: 5'-GAAACUU-3'; 5'-CUGUCAA-3'; 5'-UCUUCAA-3'; 5'-UUAUCGU-3'; 5'-CUUAUAA-3'; 5'-GCGGGGG-3'; 5'-ACUCAAA-3' in the manufacture of a medicament for the treatment or prevention of cancer.

Further, one embodiment of the present invention relates to a method for the treatment or prevention of cancer in a patient, comprising administering to the patient a sgASO comprising or consisting of any sequence selected from: 5'-GAAACUU-3'; 5'-CUGUCAA-3'; 5'-UCUUCAA-3'; 5'-UUAUCGU-3'; 5'-CUUAUAA-3'; 5'-GCGGGGG-3'; 5'-ACUCAAA-3'.

The present invention is specifically illustrated by demonstrating the examples below; however, the present invention is not particularly limited thereto.

<u>Overview</u>

Cancer was chosen as the target disease, and Bcl-2 downregulation was contemplated as the therapy. To do so, an attempt was made to create a nucleic acid drug targeting Bcl-2 mRNA. In the Structure Prediction and Structure Analysis section, the working hypothesis is that 1) the mRNA adopts a higher-order structure in a single strand on its own without protein involvement, and 2) the single primary-structure mRNA is presumed to adopt multiple higher-order structures and they are in equilibrium (i.e., the existence probabilities are distributed according to the amount of energy). Computation and evaluation of intrinsic partial structures are based on this hypothesis. In addition, for the designing of sgASO, as tRNase $Z^L$ can recognize a higher-order structure formed by the sgASO and intrinsic structure, it can be hypothesized that for the intrinsic structure, 1) the loop is 10 bases or less, and 2) the stem is 5 base pairs.

Examples

<Example 1: structure prediction and structure analysis>

Sequence information of the Bcl-2 mRNA (Accession NO. NM_000633.2) was retrieved from the NCBI databases. Based on the sequence data, a 300-base-wide frame was set at 3 nucleotides from the 5' end (The first frame ranges from base 1 (base 1) to base 300 at the 5' end and the second frame ranges from base 4 to base 303. The same applies hereafter. nmax frames are given). In each of the frames, structure predications based on pattern matching and thermodynamic stability were made for the sequence of the constituent 300 bases (see, e.g., Ref. Markham, N. R. & Zuker, M. (2005) DINAMelt web server for nucleic acid melting prediction. Nucleic Acids Res., 33, W577-W581; Markham, N. R. & Zuker, M. (2008) UNAFold: software for nucleic acid folding and hybridization. In Keith, J. M., editor, Bioinformatics, Volume II. Structure, Function and Applications, number 453 in Methods in Molecular Biology, chapter 1, pages 3-31. Humana Press, Totowa, NJ. ISBN 978-1-60327-428-9.). It is hypothesized that the state within the cell within which the mRNA is placed is in equilibrium based on the predicted structure results and the respective energy levels, and thus the existence probabilities are calculated from each predicted structure result. Here, for the prediction result of mth from the most stable structure among the structure prediction results of nth frame (in frame n, the number of the predicted structure result obtained is mmax (n)), the existence probability of that predicted structure can be j(n,m).

Here, defining the partial structure found in each of the structure prediction results, which consists of only one loop site and only one stem complementary to each other, as the stem-loop structure (the term "complementary to each other" for the stem loop does not mean that it does not constitute any other stem loop even in other prediction structure frames, but it means to never include a portion of another stem loop in a single prediction structure result). Here, the stem may contain a mismatch (bases that do not participate in base pairing in the stem are opposing) or a bulge (bases that do not participate in base pairing in the stem are not opposing), but both ends are base pairs. In addition, the stem loop enclosed within the stem loop is also considered as a separate stem loop (e.g., the loosened base pairs farthest from the loop of the stem are treated as separate stem loops from those that are not loosened).

These stem loops are called motif(x,p) when they begin with the absolute position x on the sequence rather than in the frame, and the property profiles of the constituting loops and stems (individual characteristics of the stem loops defined by the position of bases in the stem-constituting base pairs) are designated as p. Then, the existence probability within the frame n of motif(x,p) is set as partial existence probability P_local(x,p,n), and the value is the sum of j values for the structures in which the stem loop exists in the structure prediction results obtained in the n frame, i.e., Σj(n,m). In addition, when the result of summing from frame 1 to nmax for this P_local(x,p,n) is taken as ΣP_local(x,p,n), and the number of frames in the full-length sequence constituting the stem loop which could fall within the frame is set as n_all(x,p), the existence probability of motif(x,p) in the whole, P_global(x,p), is represented as ΣP_local(x,p,n)/n_all(x,p).

<Example 2: sgASO sequence design>

Twelve sites were found in 5' UTR and CDS and 3' UTR of Bcl-2 mRNA in search for motif(x, p1) with P_global(x, p) of 50% or more and p1 = "stem is 5 base pairs". Among them, there were seven motif (x, p2) in which p2 = "left-right evenness (within one difference between the 5' side sequence and 3' side sequence of the loop-flanking stem) and the length of the loop does not exceed 10."

A sgASO binding site can then be located at the seven bases immediately after each of the stem loops of motif(x,p2), and the following corrections can be processed: 1) the loop portion predicted to be three bases unwinds the next base pair to form a five-base loop; (2) when the number of base pairs in the mismatch or bulge of the stem is 2 or less, they are ignored in the counting of base pairs; and (3) when the number of base pairs in the mismatch or bulge of the stem is 3 or more, half of the number of base pairs in the mismatch or bulge is counted as base pairs. This processing identifies the 5th base pair, and the site of sgASO binding is immediately after that.

As a result, the sequences of binding sites where sgASO is expected to be effective are AAGUUUC, UUGACAG, UUGAAGA, ACGAUAA, UUAUAAG, CCCCCGC, and UUUGAGU; and the following 7-mer antisense oligos (VIS-01 to VIS-07) were synthesized (synthesis by Nippon Bio-Service Co., Ltd.), respectively.

| Target Sequence | Name | sgASO Sequence | Number |
|---|---|---|---|
| AAGUUUC | VIS-01 | 5'-pGAAACUUp -3' | 8224 |
| UUGACAG | VIS-02 | 5'-pCUGUCAAp -3' | 7889 |

(continued)

| Target Sequence | Name | sgASO Sequence | Number |
|---|---|---|---|
| UUGAAGA | VIS-03 | 5'-pUCUUCAAp -3' | 14289 |
| ACGAUAA | VIS-04 | 5'-pUUAUCGUp -3' | 15580 |
| UUAUAAG | VIS-05 | 5'-pCUUAUAAp -3' | 7985 |
| CCCCCGC | VIS-06 | 5"-pGCGGGGGp -3' | 9899 |
| UUUGAGU | VIS-07 | 5'-pACUCAAAp -3' | 1857 |

<Example 3: cell experiments>

The inhibition of Bcl-2 mRNA expression by the above sgASO (VIS-1 to VIS-7) was carried out using the human leukemic cell line HL60. All of the nucleic acid chains were phosphorylated at both ends, and the 2'OH moiety was O-methylated. One previously reported sgASO, Hep1 (Sequence: GGGCCAG; see Japanese Patent No. 5995849 and Cancer Letters 328 (2013) 362-368), was also used as a reference. HL60 cells were cultured in 24-well plates at a density of 10,000 cells/500μL in RPMI-1640 medium (containing 10% fetal bovine serum and 1% penicillin-streptomycin) at 37°C in a 5%$CO_2$ incubator. Each sgASO was added to the culture medium to reach a final concentration of 1 pM and then cultured for 48 hours. Forty-eight hours later, total RNA was extracted from HL60 cells using RNAisoPlus, and quantitative RT-PCR was performed. The level of GAPDH mRNA was used as an internal control, and the level of Bcl-2 mRNA was normalized. As a result, VIS-1 and VIS-5 were obtained as sgASO that showed a two-fold activity relative to that of Hep1, a previously reported sgASO (Fig. 1). The results revealed that the methods for designing and identifying sgASO according to the present invention are superior to the prior art.

The present specification shows the preferred embodiments of the present invention, and it is clear to those skilled in the art that such embodiments are provided simply for the purpose of exemplification. A skilled artisan may be able to make various transformations, and add modifications and substitutions without deviating from the present invention. It should be understood that the various alternative embodiments of invention described in the present specification may be used when practicing the present invention. Further, the contents described in all publications referred to in the present specification, including patents and patent application documents, should be construed as being incorporated the same as the contents clearly written in the present specification by their citation.

Industrial Applicability

The present inventors developed a method to efficiently regulate gene expression in cells using relatively short oligonucleotides by predicting the target RNA sequences subject to gene expression regulation based on the sequence information, calculating the existence probabilities from the predicted structure results obtained and their respective energy levels, and establishing a method for designing a sgASO from the existence probabilities and stem-loop structures. This technique is useful for the treatment or prevention of various diseases and disorders, as it can in principle be applied to the regulation of any gene expression.

## Claims

**1.** A method for cleaving a target RNA in a eukaryotic cell, comprising:

i) identifying a sequence in the target RNA sequence where at least one stem-loop structure is formed through hybridization of a complementary 6-mer to 10-mer oligonucleotide (sgASO) to the target RNA, and
ii) preparing the sgASO and contacting it with the target RNA in the eukaryotic cell,

wherein the stem-loop structure formed through sgASO hybridization is recognized by tRNaseZ$^L$ within the eukaryotic cell to cleave the target RNA.

**2.** The method according to claim 1, wherein the number of base pairs in the stem portion of the stem-loop structure formed through sgASO hybridization is 11 to 14.

**3.** The method according to claim 1 or 2, wherein the sgASO is a 7-mer.

**4.** The method according to any one of claims 1-3, wherein the loop portion in the stem-loop structure formed through sgASO hybridization is formed by the target RNA.

5. The method according to any one of claims 1-4, wherein the number of bases in the loop portion of the stem-loop structure formed through sgASO hybridization is 3 to 10.

6. The method according to any one of claims 1-5, wherein the stem portion of the stem-loop structure formed through sgASO hybridization does not contain a mismatch or bulge.

7. The method according to any one of claims 1-6, wherein the stem portion of the stem-loop structure formed through sgASO hybridization contains a mismatch or bulge.

8. The method according to claim 7, wherein in the counting of the number of base pairs in the stem portion, when the number of base pairs in the mismatch or bulge of the stem is 2 or less, the mismatch or bulge is considered to form a base pair and counted; and when the number of base pairs in the mismatch or bulge of the stem is 3 or more, half of the number of bases in the mismatch or bulge is counted as base pairs.

9. The method according to any one of claims 1-8, wherein the target RNA is mRNA or ncRNA.

10. The method according to any one of claims 1-9, wherein the sgASO comprises a modified nucleoside and/or a modified internucleoside linkage.

11. The method according to any one of claims 1-10, wherein the difference in the number of bases between the 5' side sequence and 3' side sequence of the stem portion formed by the target RNA is 1 or less.

12. The method according to any one of claims 1-11, wherein one or both of the terminal hydroxyl groups of the sgASO are modified.

13. The method according to any one of claims 1-12, wherein a phosphate group is added to one or both of the terminal hydroxyl groups of the sgASO.

14. The method according to any one of claims 1-13, wherein when the bases constituting the sgASO-bound region on the target RNA from the 5' end are N1, N2, N3, N4, N5, N6 and N7, and further the first base adjacent to the 3' end of the region to which the sgASO binds is N8, at least one of the conditions 1-3 below is met:

- condition 1: N8 is A or G;
- condition 2: N7 is C; and
- condition 3: N6 is A, C or G.

15. The method according to any one of claims 1-14, wherein the sgASO is an oligonucleotide consisting of any one sequence from SEQ ID NO.: 1 to SEQ ID NO.: 16384.

16. A pharmaceutical composition for use in the treatment or prevention of a disease or disorder in a patient, comprising an oligonucleotide of about 7 mers, wherein the oligonucleotide of about 7 mers is complementary to the target RNA associated with the disease or disorder, the target RNA can form at least one stem-loop structure, the oligonucleotide of about 7 mers can hybridize to the 3' side region of the stem loop structure to form at least one larger stem loop structure in conjunction with the stem loop formed by the target RNA, and wherein the formed structures are recognized by tRNaseZ$^L$ in the patient's body and the target mRNA is cleaved.

17. A pharmaceutical composition for the treatment or prevention of a cancer, comprising a sgASO comprising any of the sequences selected from:

5'-GAAACUU -3';
5'-CUGUCAA-3';
5'-UCUUCAA-3';
5'-UUAUCGU-3';
5'-CUUAUAA-3';
5'-GCGGGGG-3'; and
5'-ACUCAAA-3'.

**18.** A method for designing an oligonucleotide (sgASO) to cleave a target RNA in a eukaryotic cell, comprising:

i) identifying a sequence in the target RNA sequence in which at least one stem-loop structure is formed,
ii) identifying a sequence of 6 to 10 bases on the 3' side adjacent to the stem-loop structure as a sgASO-binding sequence, and
iii) identifying a sequence complementary to the binding sequence as a sgASO sequence.

**19.** The method for designing an oligonucleotide (sgASO) according to claim 18, wherein

i) the number of base pairs in the stem portion of the stem-loop structure formed by the target RNA is 4 to 8;
ii) the number of bases in the loop portion of the stem-loop structure is 3 to 10;
iii) in the case where the stem portion of the stem-loop structure contains a mismatch or bulge, in the counting of the number of base pairs in the stem portion, when the number of base pairs in the mismatch or bulge of the stem is 2 or less, the mismatch or bulge is considered to form a base pair and counted; and when the number of base pairs in the mismatch or bulge of the stem is 3 or more, half of the number of bases in the mismatch or bulge is counted as base pairs; and
iv) the difference in the number of bases between the 5'-side sequence and 3'-side sequence of the stem portion is 1 or less.

**20.** A method of designing a small guide antisense oligonucleotide (sgASO), comprising

(1) identifying a stem loop with a high existence probability on the target RNA,
(2) evaluating the stem loop, and
(3) setting the seven bases immediately 3' to the stem loop-forming base pair as the target sequence (sense strand) and identifying its antisense strand to be a sgASO sequence, wherein the step of (1) identifying a stem loop with a high existence probability on the target RNA comprises:

i) a step of predicting structure, comprising setting frame n having the width of W bases by an increment of R bases starting from the 5' end, wherein the number of the resulting frames is nmax, computing the base-pair pattern which can be obtained by pattern matching for the constituent base sequence of the W bases in each frame n, applying known thermodynamic stability calculations to the result, and giving ΔG for each base-pair pattern;
ii) a step of analyzing structure, comprising hypothesizing based on the resulting mmax(n) structures predicted in frame n and respective energy level, that the state inside the cell within which the RNA is placed is in equilibrium, calculating the existence probability of each resulting predicted structure according to the Maxwell-Boltzmann statistics, wherein the existence probability of each predicted structure result is j(n,m) for the mth predicted result from the most stable structure among the resulting predicted structures in frame n;
iii) a step of calculating local existence probability, comprising setting p as a property profile of a loop and stem (characteristics of the stem loop defined by the position of base in the stem-constituting base pair) formed beginning from the absolute position x on the sequence rather than in the frame, and defining the stem-loop as motif(x, p), and defining the existence probability in frame n of the motif(x,p) as partial existence probability P_local(x,p,n), and calculating the value as sum Σj(n,m) of the j values for the prediction results of structures in which the stem loop exists among all the resulting predicted structures obtained in the frame n, wherein the local existence probability P_local(x,p,n) of motif(x,p) in the frame n is represented below:

$$\mathrm{P_local}(x,p,n) = \sum_{m=1}^{\mathrm{mmax}(n)} \begin{cases} 0 & \text{... if motif } (x,p) \text{ is not found in the } m-\text{th structure of frame } n \\ j(n,m) & \text{... if motif } (x,p) \text{ is found in the } m-\text{th structure of frame } n \end{cases}$$

iv) a step of calculating existence probability, comprising giving the existence probability P_global(x,p) of motif(x,p) among the entirety as ΣP_local(x,p,n)/n_all(x,p) when ΣP_local(x,p,n) is the result of sum of P_local(x,p,n) from frame 1 to nmax, and the number of frames in which the full length of the sequence constituting the stem-loop is contained within the frame is n_all(x,p), wherein the existence probability of motif(x,p) among the entirety is represented as below, and

$$P_global(x,p) = \sum_{n=1}^{nmax} \frac{P_local(x,p,n)}{n_all}$$

v) a step of analyzing, comprising selecting a stem-loop based on the existence probability and property p, with respect to the obtained existence probability P_global(x,p) of motif(x, p).

Fig. 1

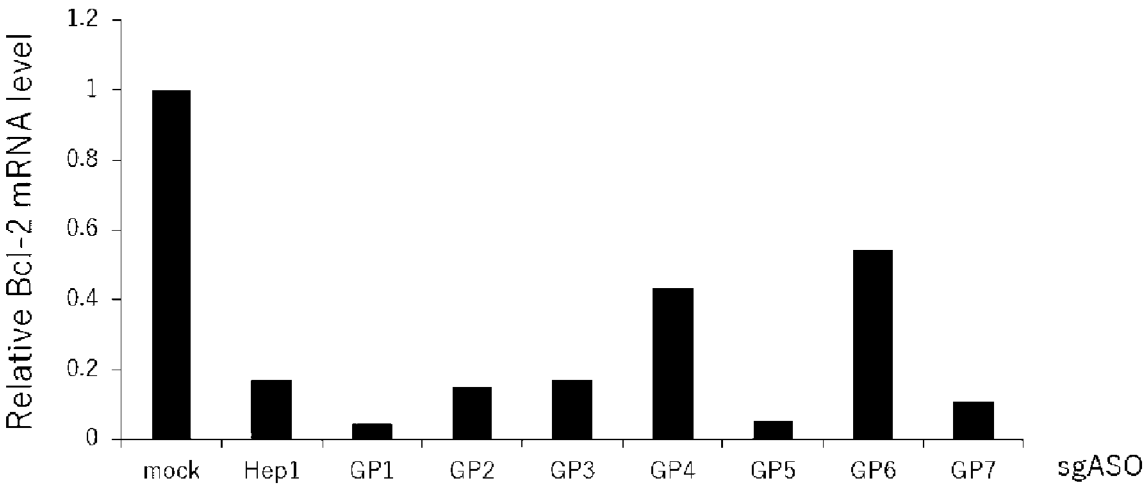
Relative Bcl-2 mRNA level
1.2
1
0.8
0.6
0.4
0.2
0
mock
Hep1
GP1
GP2
GP3
GP4
GP5
GP6
GP7
sgASO

**INTERNATIONAL SEARCH REPORT**

International application No.
PCT/JP2018/032122

A. CLASSIFICATION OF SUBJECT MATTER
Int.Cl. C12N15/113(2010.01)i, A61K31/7088(2006.01)i, A61P35/00(2006.01)i, A61P43/00(2006.01)i

According to International Patent Classification (IPC) or to both national classification and IPC

B. FIELDS SEARCHED

Minimum documentation searched (classification system followed by classification symbols)
Int.Cl. C12N15/00-15/90, C12Q1/68-1/6897

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/REGISTRY/MEDLINE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | HAINO, A. et al., "TRUE Gene Silencing: Screening of a Heptamer-type Small Guide RNA Library for Potential Cancer Therapeutic Agents", J. Vis. Exp., 2016, 112, e53879, in particular, pp. 1-2, 4, section "Discussion" | 1-7, 9-18<br>1-20 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 November 2018 (14.11.2018) | 27 November 2018 (27.11.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2018/032122

C (Continuation). DOCUMENTS CONSIDERED TO BE RELEVANT

| Category* | Citation of document, with indication, where appropriate, of the relevant passages | Relevant to claim No. |
|---|---|---|
| X<br>A | TAKAHASHI, M. et al., "Screening of a heptamer-type sgRNA library for potential therapeutic agents against hematological malignancies", Leuk. Res., 2014, 38 (7), pp. 808-815, in particular, abstract, page 810, right column, paragraph [0004], table S1, fig. 5 | 1-7, 9-17<br>8, 18-20 |
| X<br>A | WATANABE, N. et al., "Induction of apoptosis of leukemic cells by TRUE gene silencing using small guide RNAs targeting the WT1 mRNA", Leuk. Res., 2013, 37(5), pp. 580-585, in particular, abstract, page 581, left column, paragraph [0002], fig. 1 | 1-6, 9-17<br>7-8, 18-20 |
| X<br>A | WO 2013/031704 A1 (NIIGATA UNIVERSITY OF PHARMACY AND APPLIED LIFE SCIENCES) 07 March 2013, paragraphs [0015]-[0016] & US 2015/0025232 A1, paragraphs [0042]-[0043] | 16-17<br>1-15, 18-20 |
| X<br>A | WO 2013/022092 A1 (NIIGATA UNIVERSITY OF PHARMACY AND APPLIED LIFE SCIENCES) 14 February 2013, paragraphs [0013]-[0014] (Family: none) | 16-17<br>1-15, 18-20 |
| Y | WO 2013/161964 A1 (KIRIN HOLDINGS COMPANY, LIMITED) 31 October 2013, paragraph [0056] & US 2015/0292005 A1, paragraph [0161] | 1-20 |
| Y | JP 2005-341865 A (KYOTO UNIVERSITY) 15 December 2005, paragraph [0056] (Family: none) | 1-20 |
| Y | WO 2011/062166 A1 (TAKEDA PHARMACEUTICAL COMPANY LIMITED) 26 May 2011, examples (Family: none) | 1-20 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017166641 A
- JP 3660718 B
- JP 5959522 B
- JP 5995849 B
- JP 5194256 B
- JP 2015020994 A
- JP 2007000087 W
- JP 2016059398 W


### Non-patent literature cited in the description


- **CHERY, J.** RNA therapeutics: RNAi and antisense mechanisms and clinical applications. *Postdoc J.,* 2016, vol. 4 (7), 35-50
- **RUPAIMOOLE, R. ; SLACK,F.J.** MicroRNA therapeutics: towards a new era for the management of cancer and other diseases. *Nature Review Drug Discovery,* 2017, vol. 16, 203-221
- **TAMURA, M. ; NASHIMOTO, C. ; MIYAKE, N. ; DAIKUHARA,Y. ; OCHI,K. ; NASHIMOTO,M.** Intracellular mRNA cleavage by 3' tRNase under the direction of 2'-O-methylRNA heptamers. *Nucleic Acids Res.,* 2003, vol. 31, 4354-4360
- **HABU,Y. ; MIYANO-KUROSAKI,N. ; KITANO,M. ; ENDO,Y. ; YUKITA,M. ; OHIRA,S. ; TAKAKU,H. ; NASHIMOTO,M. ; TAKAKU,H.** Inhibition of HIV-1 gene expression by retroviral vector-mediated small-guide RNAs that direct specific RNA cleavage by tRNase ZL. *Nucleic Acids Res.,* 2005, vol. 33, 235-243
- **TAKAKO SANO ; MASAYUKI TAKAHASHI ; TADASUKE NOZAKI ; YOSHIAKI TAKAHASHI ; MASATO TAMURA ; MASAYUKI NASHIMOTO.** Expanding the utility of heptamer-type sgRNA for TRUE gene silencing. *Biochem. and Biophys. Res. Commun.,* 2011, vol. 416, 427-432
- **MASAYUKI TAKAHASHI ; REYAD A. ELBARBARY ; AIKO NAKASHIMA ; MAYUMI ABE ; NORIHIRO WATANABE ; MIWAKO NARITA ; MASUHIRO TAKAHASHI ; MASATO TAMURA ; TETSUO YOSHIDA ; MASAYUKI NASHIMOTO.** A naked RNA heptamer targeting the human Bcl-2 mRNA induces apoptosis of HL60 leukemia cells. *Cancer Letters,* 2013, vol. 328, 362-368
- **NORIHIRO WATANABE ; MIWAKO NARITA ; AKIE YAMAHIRA ; TOMOYO TANIGUCHI ; TATSUO FURUKAWA ; TETSUO YOSHIDA ; TATSUYA MIYAZAWA ; MASAYUKI NASHIMOTO ; MASUHIRO TAKAHASHI.** Induction of apoptosis of leukemic cells by TRUE gene silencing using small guide RNAs targeting the WT1 mRNA. *Leukemia Research,* 2013, vol. 37, 580-585
- **SATOSHI IIZUKA ; NOBUHIKO ORIDATE ; MASAYUKI NASHIMOTO ; SATOSHI FUKUDA ; MASATO TAMURA.** Growth inhibition of head and neck squamous cell carcinoma cells by sgRNA targeting the cyclin D1 mRNA based on TRUE gene silencing. *PLoS One,* 2014, vol. 9, e114121
- **NAKASHIMA,A. ; TAKAKU,H. ; SHIBATA,H.S. ; NEGISHI,Y. ; TAKAGI,M. ; TAMURA,M. ; NASHIMOTO,M.** Gene silencing by the tRNA maturase tRNase ZL under the direction of small guide RNA. *Gene Therapy,* 2007, vol. 14, 78-85
- **ELBARBARY,R.A. ; TAKAKU,H. ; TAMURA,M. ; NASHIMOTO,M.** Inhibition of vascular endothelial growth factor expression by TRUE gene silencing. *Biochem.and Biophys. Res. Commun.,* 2009, vol. 379, 924-927
- **NASHIMOTO, M.** Specific cleavage of target RNAs from HIV-1 with 5' half tRNA by mammalian tRNA 3' processing endoribonuclease. *RNA,* 1996, vol. 2, 2523-2524
- **SHIBATA, H.S. ; TAKAKU, H. ; TAKAGI, M. ; NASHIMOTO, M.** The T loops structure is dispensable for substrate recognition by tRNase ZL. *J. Biol. Chem.,* 2005, vol. 280, 22326-22334
- **NASHIMOTO, M. ; GEARY, S. ; TAMURA, M. ; KASPER, R.** RNA heptamers that directs RNA cleavage by mammalian tRNA 3' processing endoribonuclease. *Nucleic Acids Res.,* 1998, vol. 26, 2565-2571

- **TAKAKU, H. ; MINAGAWA, A. ; TAKAGI, M. ; NASHIMOTO, M.** A novel four-base-recognizing RNA cutter that can remove the single 3' terminal nucleotides from RNA molecules. *Nucleic Acids Res.,* 2004, vol. 32, e91
- **ELBARBARY, R.A. ; TAKAKU, H. ; UCHIUMI, N. ; TAMIYA, H. ; ABE, M. ; TAKAHASHI, M. ; NISHIDA, H. ; NASHIMOTO, M.** Modulation of gene expression by human cytosolic tRNase ZL through 5'-half-tRNA. *PLoS ONE,* 2009, vol. 4, e5908
- **ELBARBARY, R.A. ; TAKAKU, H. ; UCHIUMI, N. ; TAMIYA, H. ; ABE, M. ; NISHIDA, H. ; NASHIMOTO, M.** Human cytosolic tRNase ZL can downregulate gene expression through miRNA. *FEBS Lett.,* 2009, vol. 583, 3241-3246
- **LOKE, S.L. ; STEIN, C.A. ; ZHANG X. H. ; MORI, K. ; NAKANISHI, M. ; SUBASINGHE, C. ; COHEN, J.S. ; NECKERS, L.M.** Characterization of oligonucleotide transport into living cells. *Proc. Natl. Acad. Sci. USA,* 1989, vol. 86, 3474-3478
- **KHVOROVA ; WATTS.** *Nature Biotechnology,* 2017, vol. 35, 238-248
- *Nucleic Acid Research,* 2004, vol. 32, e175
- *Acc. Chem. Res.,* 1999, vol. 32, 624
- *J. Am. Chem. Soc.,* 2001, vol. 123, 4653
- *J. Am. Chem. Soc.,* 2000, vol. 122, 6900
- **MARKHAM, N. R. ; ZUKER, M.** DINAMelt web server for nucleic acid melting prediction. *Nucleic Acids Res.,* 2005, vol. 33, W577-W581
- UNAFold: software for nucleic acid folding and hybridization. **MARKHAM, N. R. ; ZUKER, M.** Bioinformatics. 2008, vol. II
- Structure, Function and Applications, number 453. Methods in Molecular Biology. Humana Press, vol. 1, 3-31
- *Cancer Letters,* 2013, vol. 328, 362-368